# EUROPEAN PATENT APPLICATION

(11) **EP 2 423 684 A2**
(43) Date of publication of application: **29.02.2012**
(21) Application number: 11156670.9
(22) Date of filing: 21.04.2007
(51) Int. Cl.: G01N 33/50, C07K 14/00

(54) **Differential expression profiling analysis of cell culture phenotypes and the uses thereof**

(30) Priority: 21.04.2006 US 794299 P; 25.01.2007 US 897412 P
(62) Divisional of application: 07835734.0
(71) Applicant: Wyeth LLC, Madison, NJ 07940 (US); Dublin City University, Dublin 9 (IE)
(72) Inventor: Anderson, Karin, Georgetown, MA 01833 (US); Barron, Niall, Co Dublin (IE); Charlebois, Timothy, Andover, MA 01810 (US); Clynes, Martin, 3, Dublin (IE); Di Nino, Dana L., North Andover, MA 01845 (US); Doolan, Padraig, Co Dublin (IE); Gammel, Patrick, Co Kildare (IE); Kopycinski, Kathleen, Somerville, MA 02144 (US); Leonard, Mark, Manchester, NH 03104 (US); McCarthy, Kevin M., North Andover, 01845 (US); Melville, Mark, Melrose, MA 02176 (US); Sinacore, Martin S., Andover, MA 01810 (US); Meleady, Paula, Co Meath (IE)
(74) Representative: Dörries, Hans Ulrich

(57) **Abstract**

The invention provides methods for systematically identifying genes and proteins and related pathways that maximize protein expression and secretion by expression profiling analysis. The present invention further provides methods for manipulating the identified genes and proteins to engineer improved cell lines.

## Description

### REFERENCE TO SEQUENCE LISTING

This application includes as part of the originally filed subject matter three compact discs, labeled "Copy 1," "Copy 2," and "Copy 3," each disc containing a Sequence Listing. The machine format of each compact disc is IBM-PC and the operating system of each compact disc is MS-Windows. Each of the compact discs includes a single text file, which is named "WYE-060pc.ST25.txt" (1,423 KB, created April 20, 2007). The contents of the compact discs labeled "Copy 1," "Copy 2," and "Copy 3" are hereby incorporated by reference herein in their entireties.

### FIELD OF THE INVENTION

The present invention relates to methods for identifying genes and proteins that are involved in conferring a particular cell phenotype by differential expression profiling analysis and the use of the genes and proteins in the optimization of cell line culture conditions and transgene expression.

### BACKGROUND OF THE INVENTION

Fundamental to the present-day study of biology is the ability to optimally culture and maintain cell lines. Cell lines not only provide an *in vitro* model for the study of biological systems and diseases, but are also used to produce organic reagents. Of particular importance is the use of genetically engineered prokaryotic or eukaryotic cell lines to generate mass quantities of recombinant proteins. A recombinant protein may be used in a biological study, or as a therapeutic compound for treating a particular ailment or disease.

The production of recombinant proteins for biopharmaceutical application typically requires vast numbers of cells and/or particular cell culture conditions that influence cell growth and/or expression. In some cases, production of recombinant proteins benefits from the introduction of chemical inducing agents (such as sodium butyrate or valeric acid) to the cell culture medium. Identifying the genes and related genetic pathways that respond to the culture conditions (or particular agents) that increase transgene expression may elucidate potential targets that can be manipulated to increase recombinant protein production and/or influence cell growth.

Research into optimizing recombinant protein production has been primarily devoted to examining gene regulation, cellular responses, cellular metabolism, and pathways activated in response to unfolded proteins. Currently, there is no available method that allows for the simultaneous monitoring of transgene expression and identification of the genetic pathways involved in transgene expression. For example, currently available methods for detecting transgene expression include those that measure only the presence and amount of known proteins (*e.g.*, Western blot analysis, enzyme-linked immunosorbent assay, and fluorescence-activated cell sorting), or the presence and amount of known messenger RNA (mRNA) transcripts (*e.g.*, Northern blot analysis and reverse transcription-polymerase chain reaction). These and similar methods are not only limited in the number of known proteins and/or mRNA transcripts that can be detected at one time, but they also require that the investigator know or "guess" what genes are involved in transgene expression prior to experimentation (so that the appropriate antibodies or oligonucleotide probes are used). Another limitation inherent in blot analyses and similar protocols is that proteins or mRNA that are the same size cannot be distinguished. Considering the vast number of genes contained within a single genome, identification of even a minority of genes involved in a genetic pathway using the methods described above is costly and time-consuming. Additionally, the requirement that the investigator have some idea regarding which genes are involved does not allow for the identification of genes and related pathways that were either previously undiscovered or unknown to be involved in the regulation of transgene expression.

Therefore, there is a need in the field of cell line engineering for a more systematic approach to identify genes and proteins (including previously undiscovered genes and proteins) and related genetic pathways that are involved (directly or indirectly) with a particular cell culture phenotype, *e.g.*, increased and efficient transgene expression. Discovery of these genes and/or related pathways will provide new targets that can be manipulated to improve the yield and quality of recombinant proteins and influence cell growth.

### SUMMARY OF THE INVENTION

The present invention solves these problems by providing differential expression profiling analysis of industrially relevant cell line phenotypes through the use of nucleic acid microarray and proteomics analysis methods. In particular, the present invention provides methods for systematically identifying genes and proteins and related pathways that maximize protein expression and secretion by expression profiling analysis. The present invention further provides methods for manipulating the identified genes and proteins to engineer improved cell lines.

Thus, in one aspect, the present invention features a method for identifying proteins regulating or indicative of a cell culture phenotype in a cell line. The method includes generating a protein expression profile of a sample derived from a test cell line; comparing the protein expression profile to a control profile derived from a control cell line; and identifying one or more differentially expressed proteins based on the comparison, wherein the test cell line has a cell culture phenotype distinct from that of the control cell line, and the one or more differentially expressed proteins are capable of regulating or indicating the cell culture phenotype. In a preferred embodiment, the cell line is a Chinese hamster ovary (CHO) cell line. In another embodiment, the protein expression profile is generated by fluorescent two-dimensional differential in-gel electrophoresis.

In some embodiments, the cell culture phenotype is a cell growth rate, a cellular productivity (such as a maximum cellular productivity or a sustained high cellular productivity), a peak cell density, a sustained cell viability, a rate of ammonia production or consumption, or a rate of lactate production or consumption. In one embodiment, the cell culture phenotype is a maximum cellular productivity. In another embodiment, the cell culture phenotype is a sustained cell viability. In yet another embodiment, the cell culture phenotype is a peak cell density. In still another embodiment, the cell culture phenotype is a cell growth rate.

The present invention provides a method for improving a cell line by modulating, *i.e.,* up-regulating or down-regulating, one or more proteins identified according to the method described above. As used herein, "up-regulating" includes providing an exogenous nucleic acid (*e.g.,* an over-expression construct) encoding a protein of interest or a variant retaining its activity (such as, for example, a mammalian homolog thereof, such as a primate or rodent homolog) or providing a factor or a molecule indirectly enhancing the protein or gene activity or expression level. As used herein, "down-regulating" includes knocking-out the gene encoding a protein of interest, providing an RNA interference construct, or providing an inhibitor or other factors indirectly inhibiting the protein or gene activity or expression level. In one particular embodiment, the present invention provides a method for improving a cell line by down-regulating one or more proteins identified according to the method described above by RNA interference.

In particular, the present invention provides a method for improving cellular productivity of a cell line including modulating, *i.e*., up-regulating or down-regulating, one or more proteins identified according to the method described above. In one embodiment, the present invention provides a method for improving cellular productivity of a cell line including modulating, *i.e*., up-regulating or down-regulating, one or more genes or proteins selected from Tables 2, 3, 9, 10, 11, and 12.

In one embodiment, the present invention provides a method for improving the cell growth rate of a cell line including modulating, *i.e.*, up-regulating or down-regulating, one or more proteins identified according to the method described above. In particular, the present invention provides a method for improving the cell growth rate of a cell line including modulating, *i.e*., up-regulating or down-regulating, one or more genes or proteins selected from Tables 4, 5, 6, 13, 14, 27 and 28.

In another embodiment, the present invention provides a method for increasing the peak cell density of a cell line including modulating, *i.e.*, up-regulating or down-regulating, one or more proteins identified according to the method described above. In particular, the present invention provides a method for increasing the peak cell density of a cell line including modulating, *i.e.*, up-regulating or down-regulating, one or more genes or proteins selected from Tables 8, 15, 16, and 17.

In another embodiment, the present invention provides a method for increasing the sustained cell viability of a cell line including modulating, *i.e.*, up-regulating or down-regulating, one or more proteins identified according to the method described above. In particular, the present invention provides a method for increasing the sustained cell viability of a cell line including modulating, *i.e.*, up-regulating or down-regulating, one or more genes or proteins selected from Tables 7, 18 and 19.

In another embodiment, the present invention provides a method for regulating the lactate production or consumption of a cell line including modulating, *i.e.*, up-regulating or down-regulating, one or more proteins identified according to the method described above. In particular, the present invention provides a method for regulating the lactate production or consumption of a cell line including modulating, *i.e*., up-regulating or down-regulating, one or more genes or proteins selected from Tables 7, 18 and 19.

In yet another embodiment, the present invention provides a method for improving a cell line by modulating, *i.e*., up-regulating or down-regulating, one or more genes or proteins identified according to the method described above. In particular, the present invention provides a method for improving a cell line by modulating, *i.e*., up-regulating or down-regulating, one or more genes or proteins selected from Tables 20, 24, 25 and 26.

In another aspect, the present invention provides a method for improving a cell line by modulating, *i.e*., up-regulating or down-regulating, at least two genes or proteins, wherein a first gene or protein affects a first cell culture phenotype and a second gene or protein affects a second, different cell culture phenotype, wherein the cell culture phenotypes are selected from the group consisting of a cell growth rate, a cellular productivity, a peak cell density, a sustained cell viability, a rate of ammonia production or consumption, or a rate of lactate production or consumption. In one embodiment, the method further including up-regulating or down-regulating a third gene or protein affecting a third cell culture phenotype different from the first and second cell culture phenotypes.

In yet another aspect, the present invention provides a method of assessing a cell culture phenotype of a cell line. The method including detecting, in a sample from the cell culture, an expression level of a protein identified according to any of the methods described above; and comparing the expression level to a reference level, wherein the comparison is indicative of the cell culture phenotype.

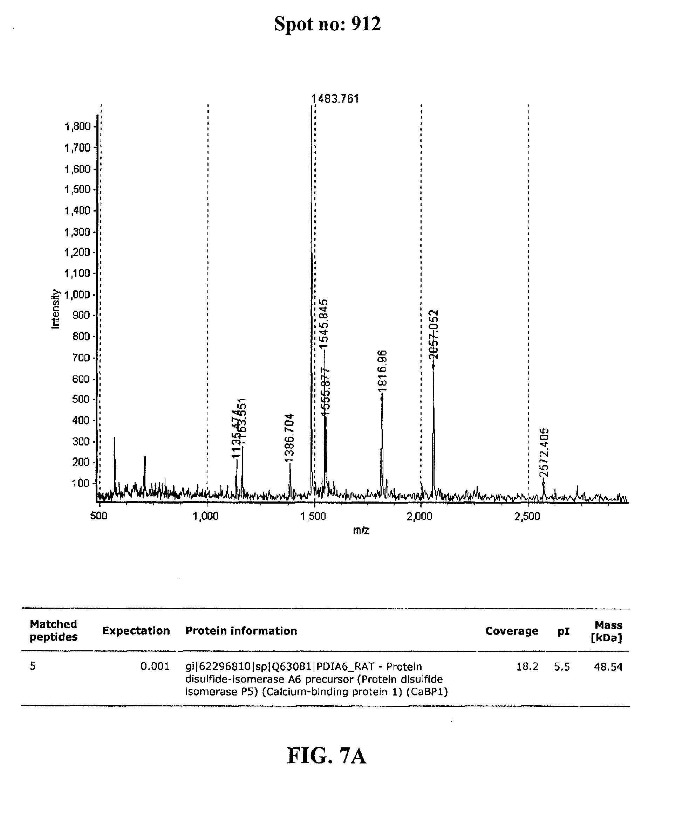

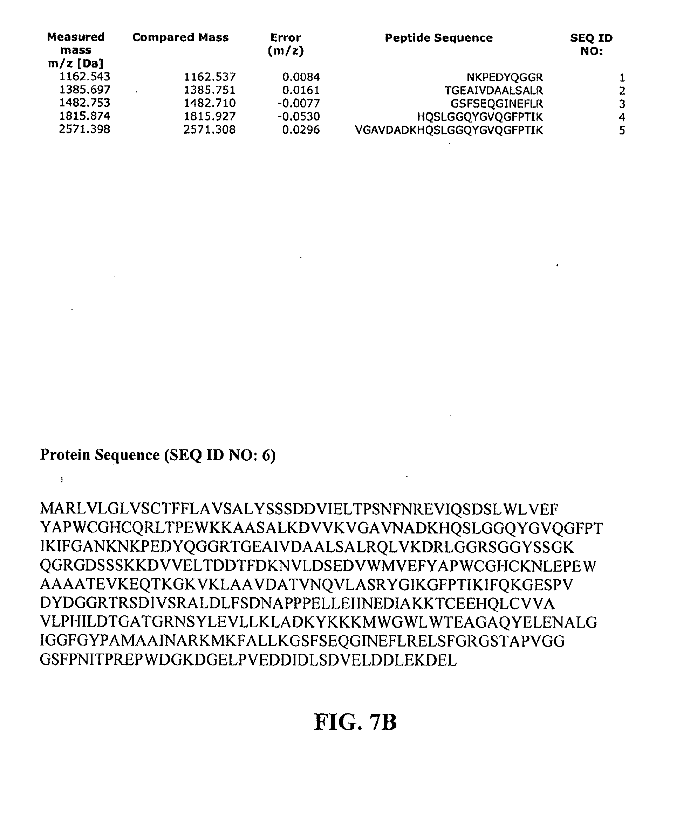

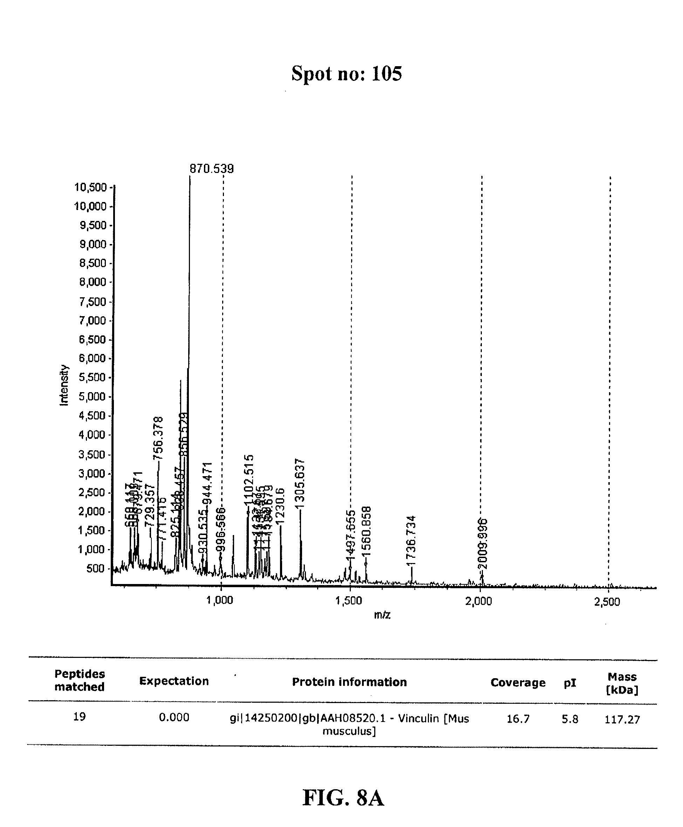

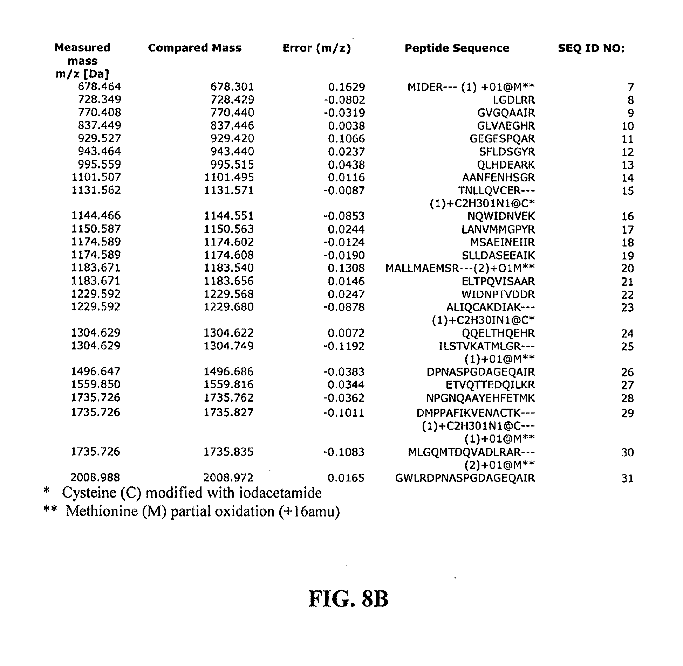

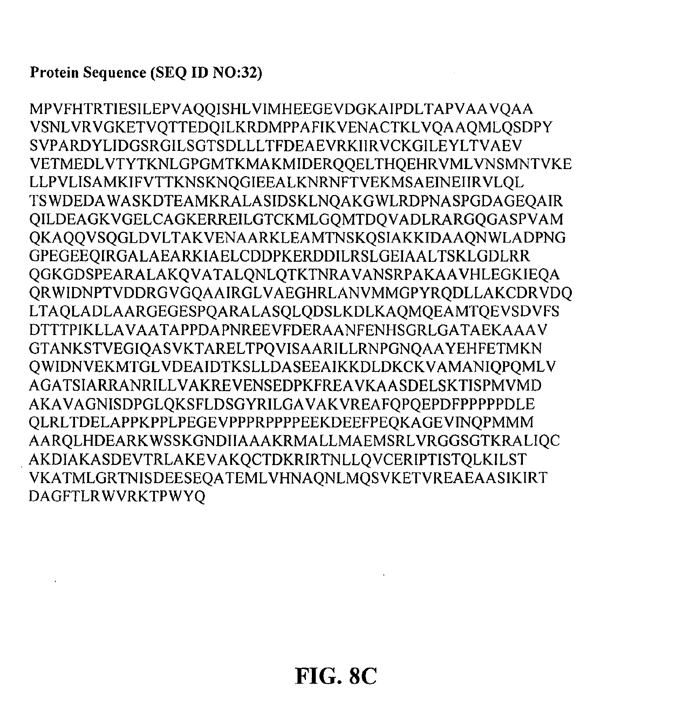

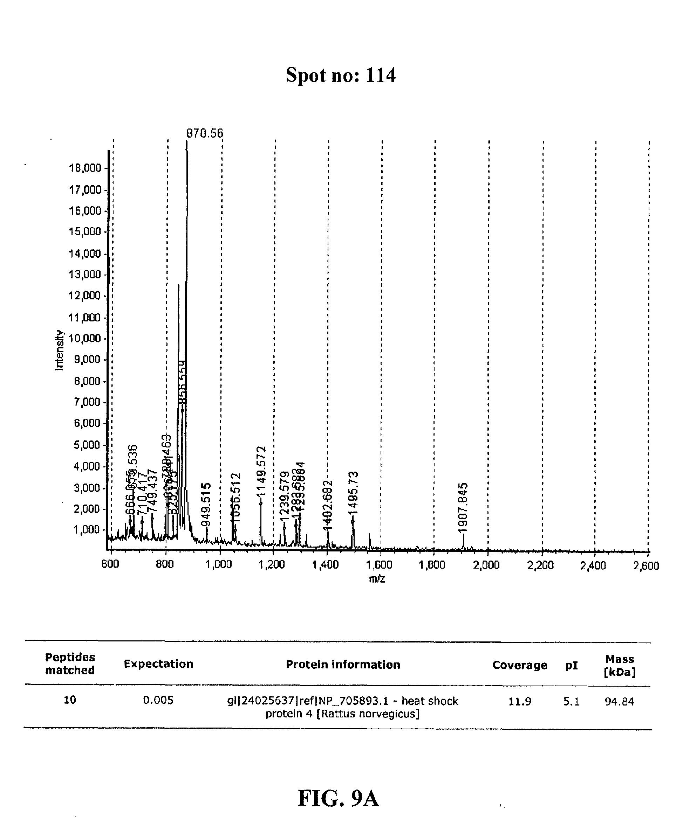

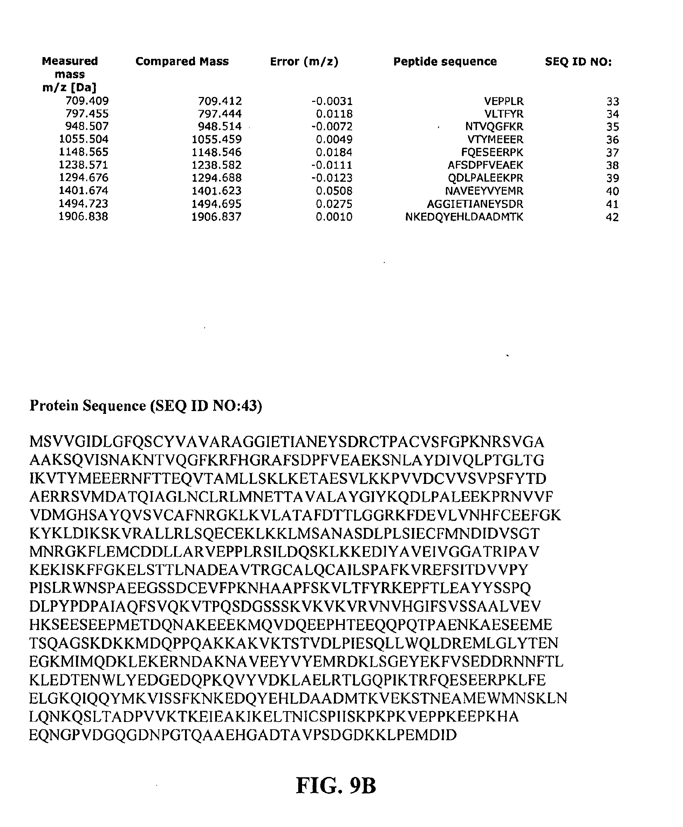

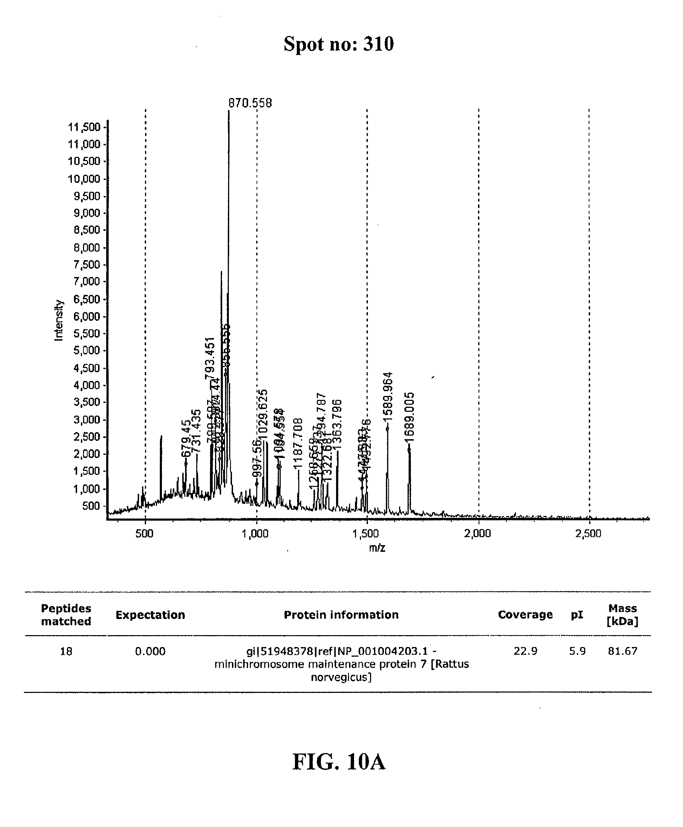

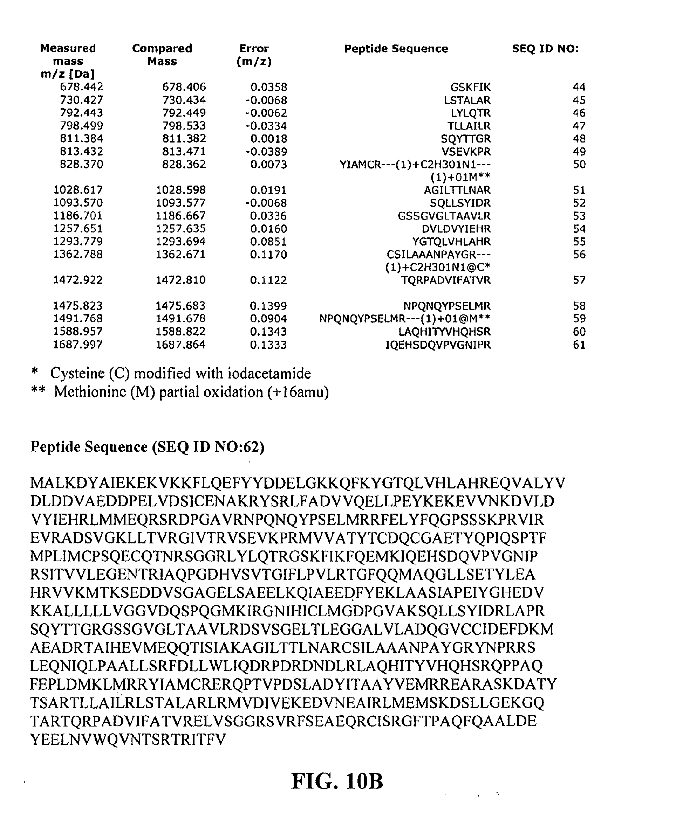

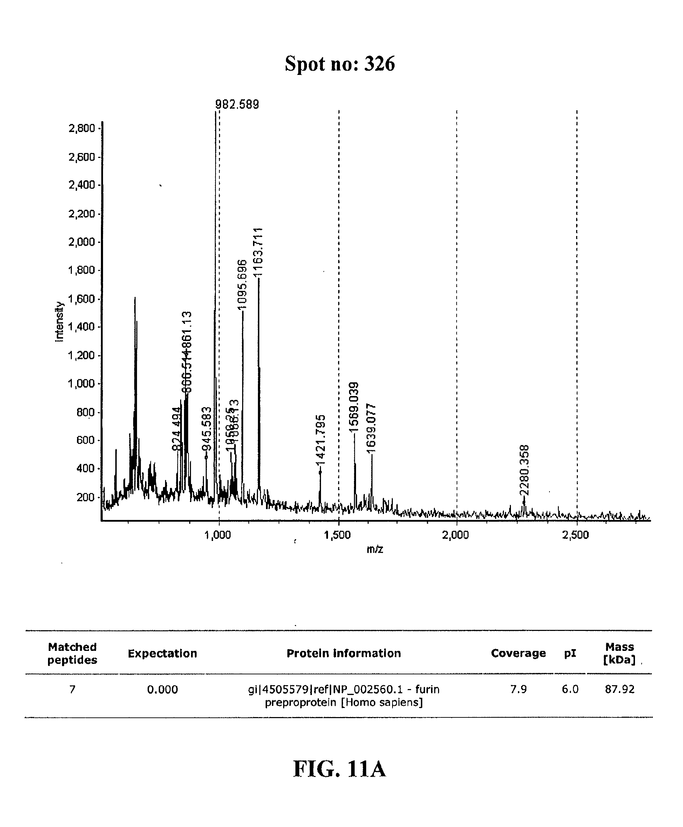

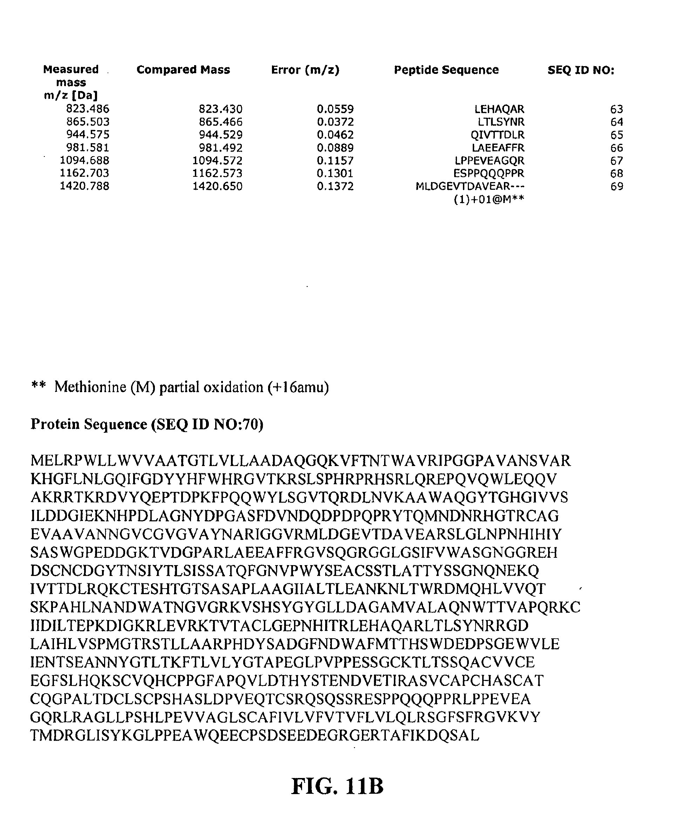

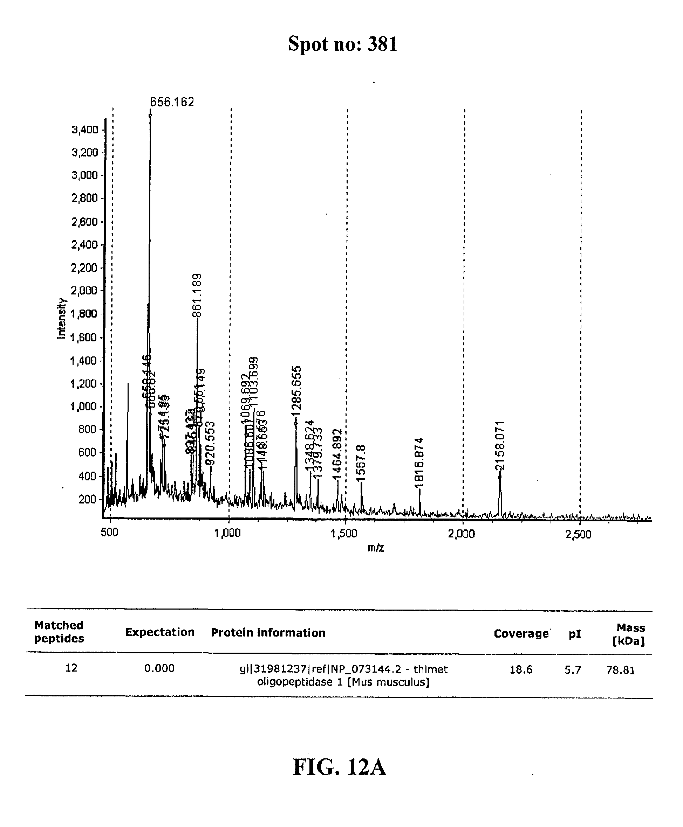

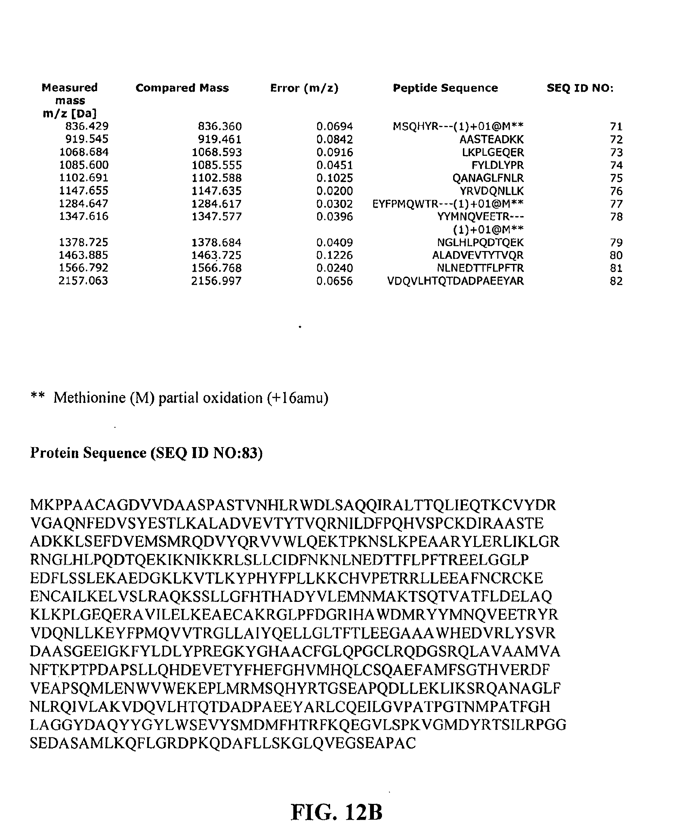

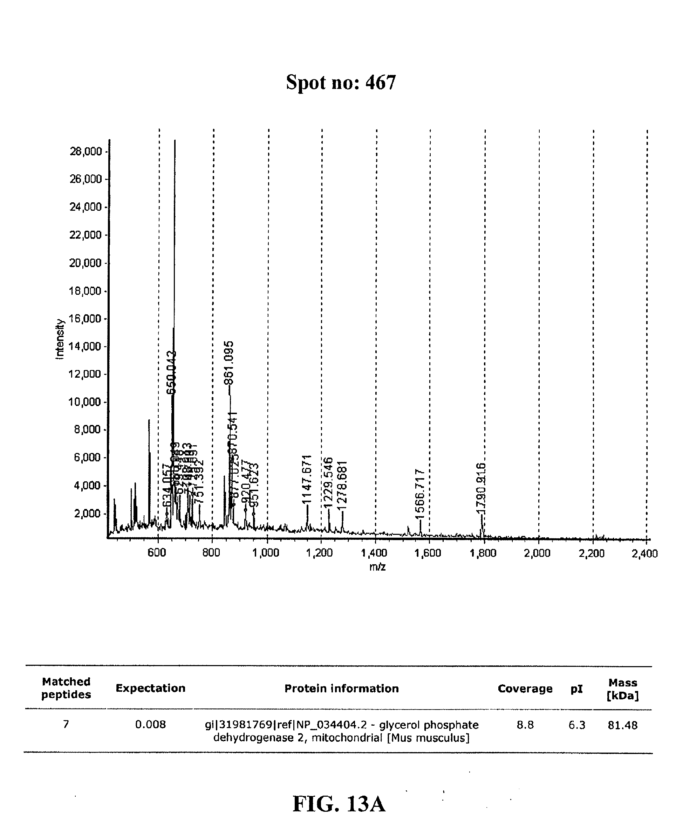

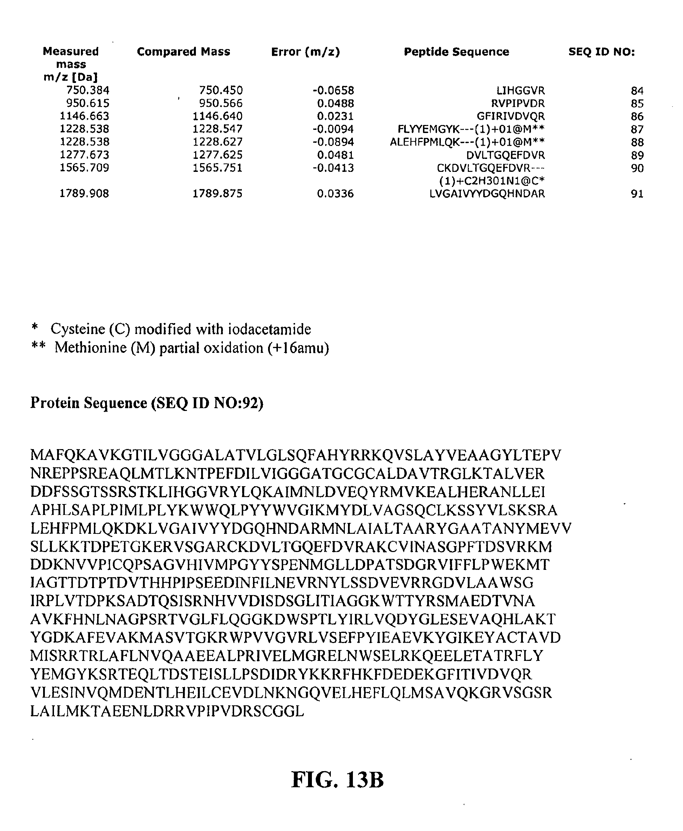

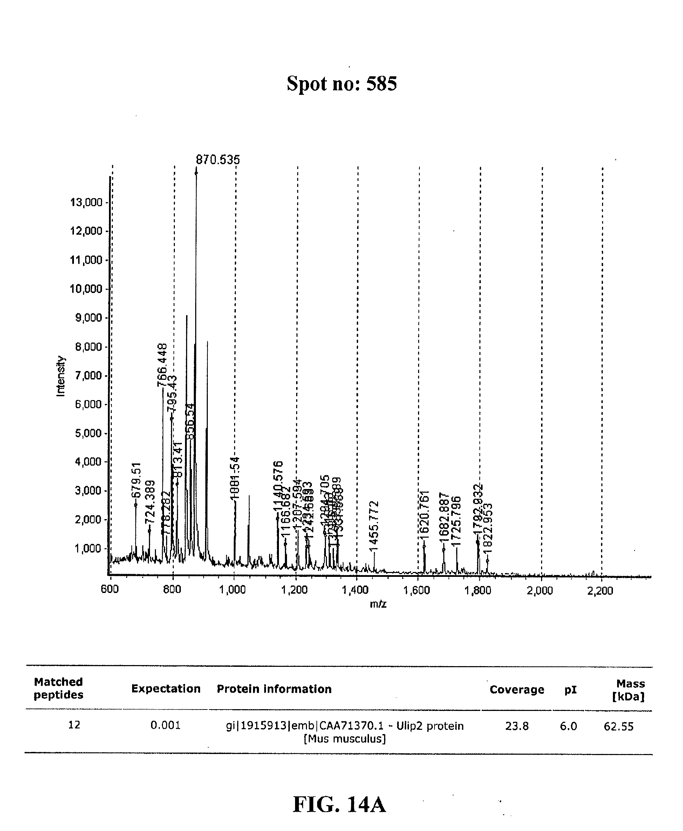

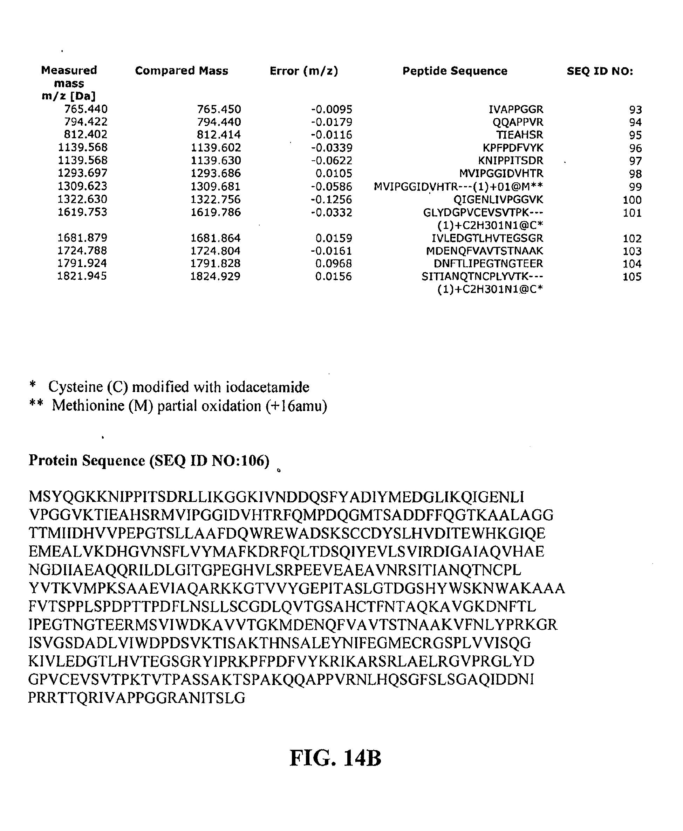

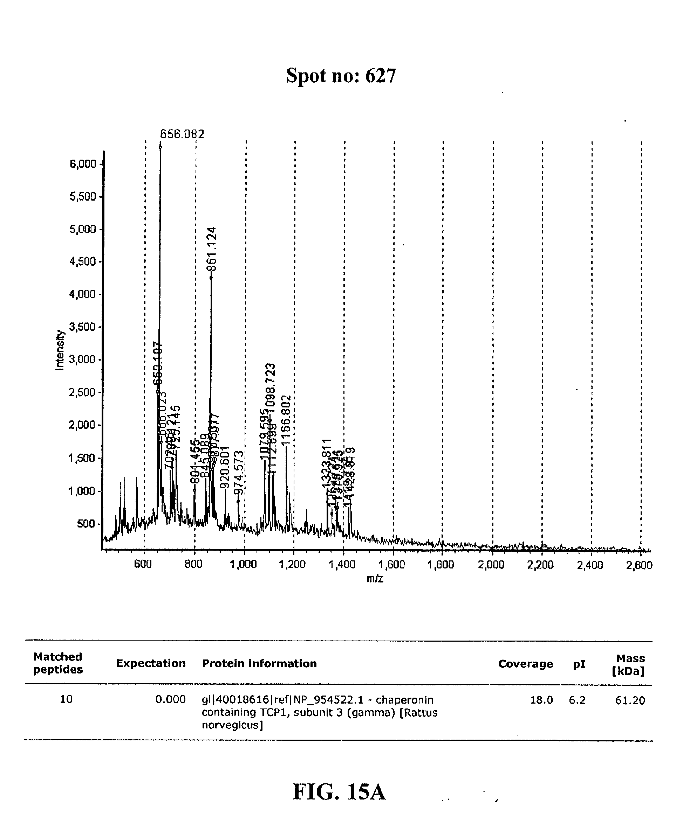

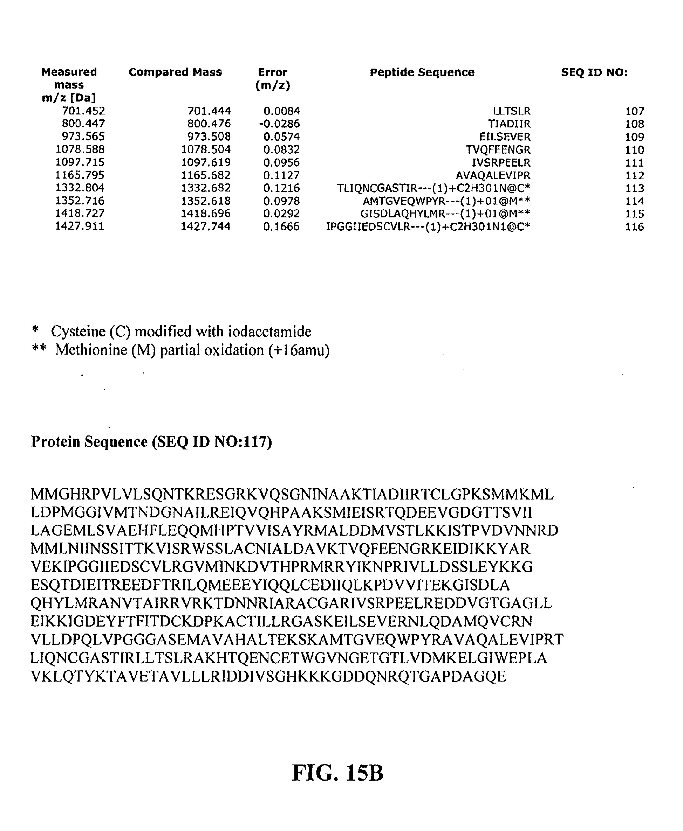

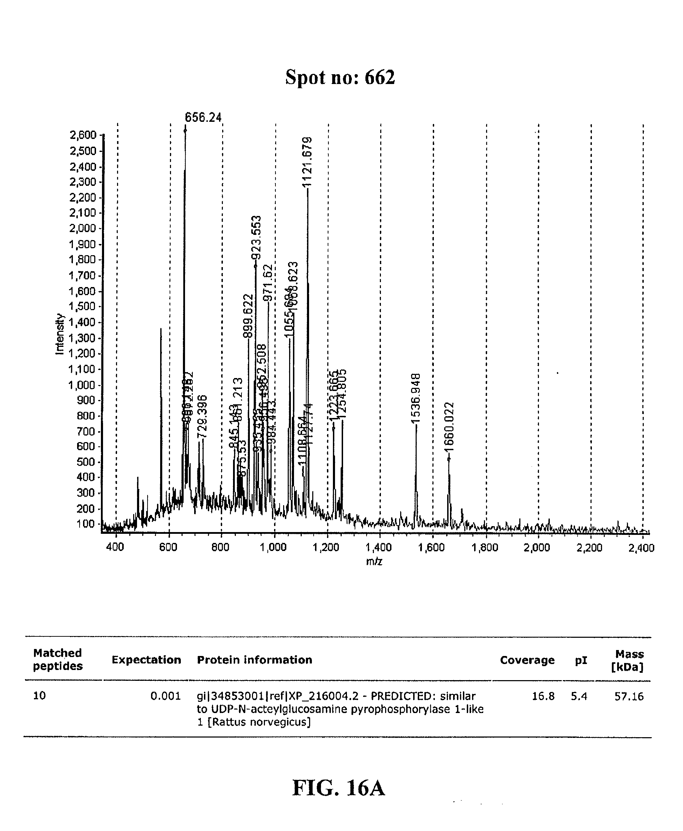

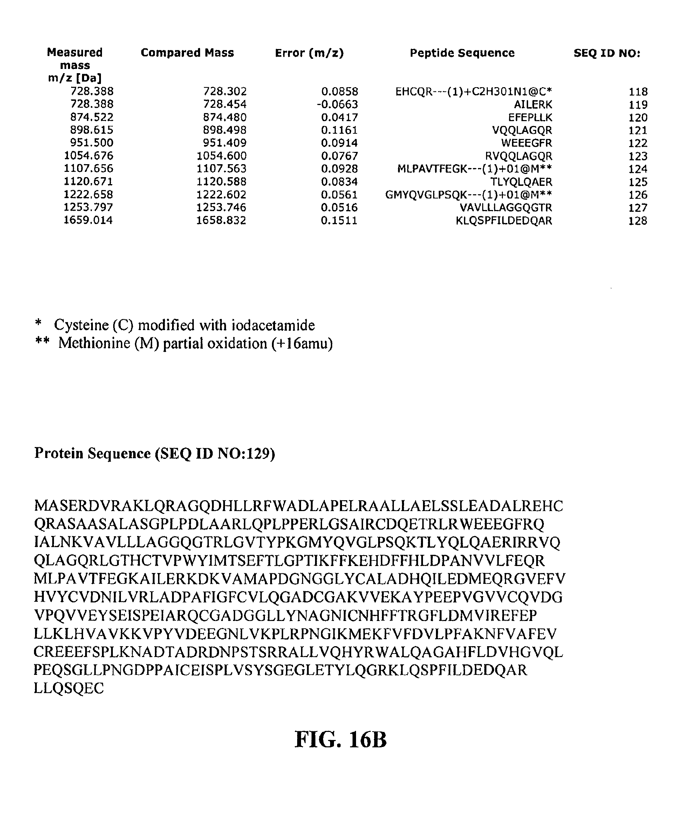

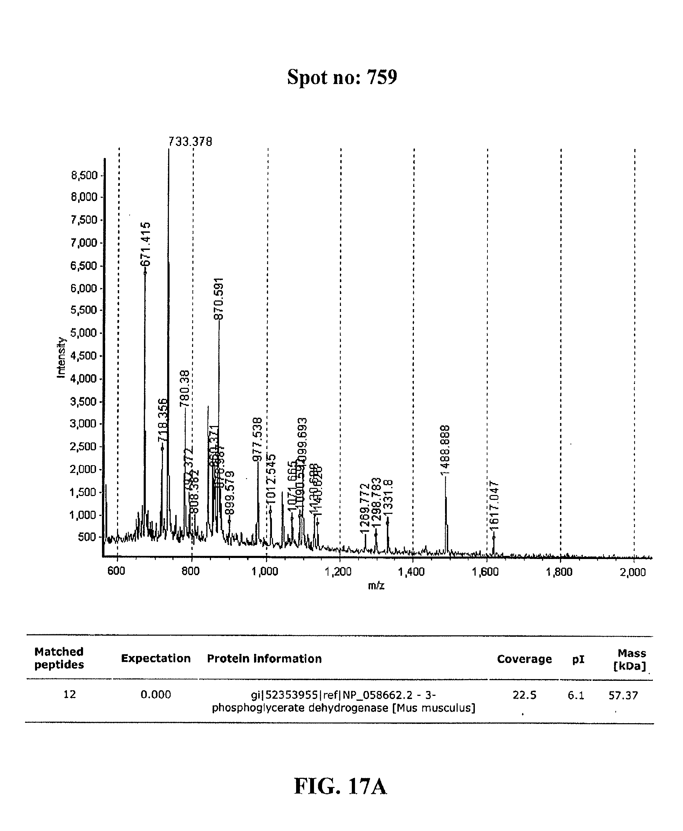

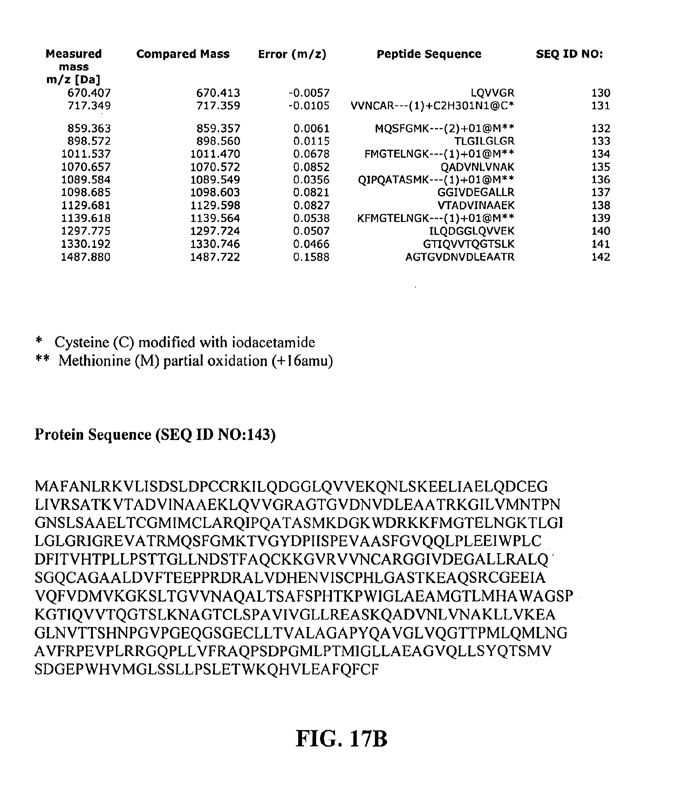

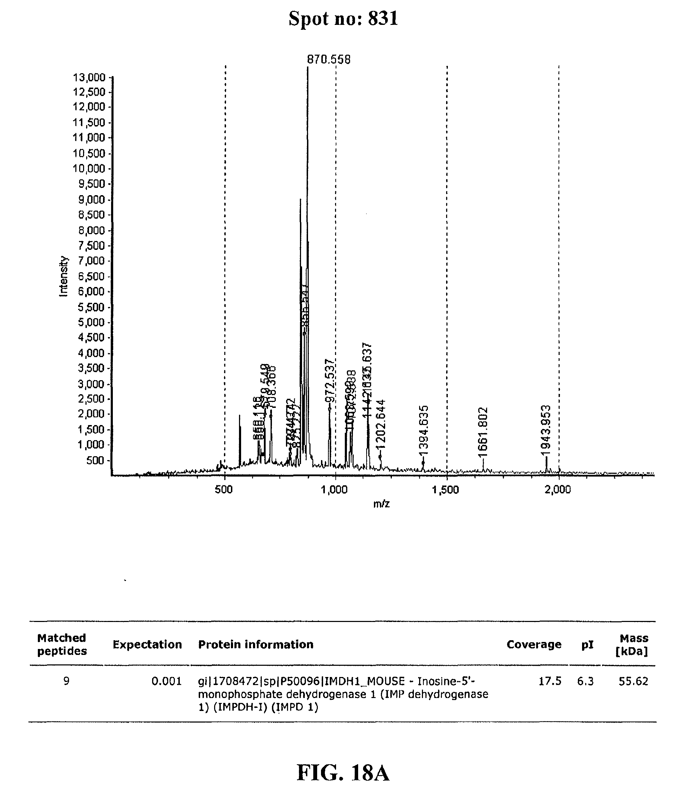

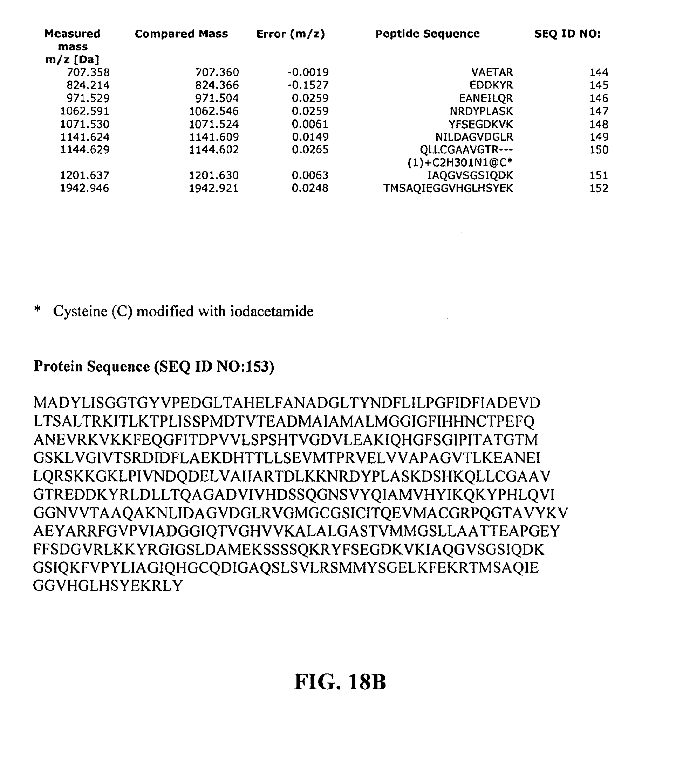

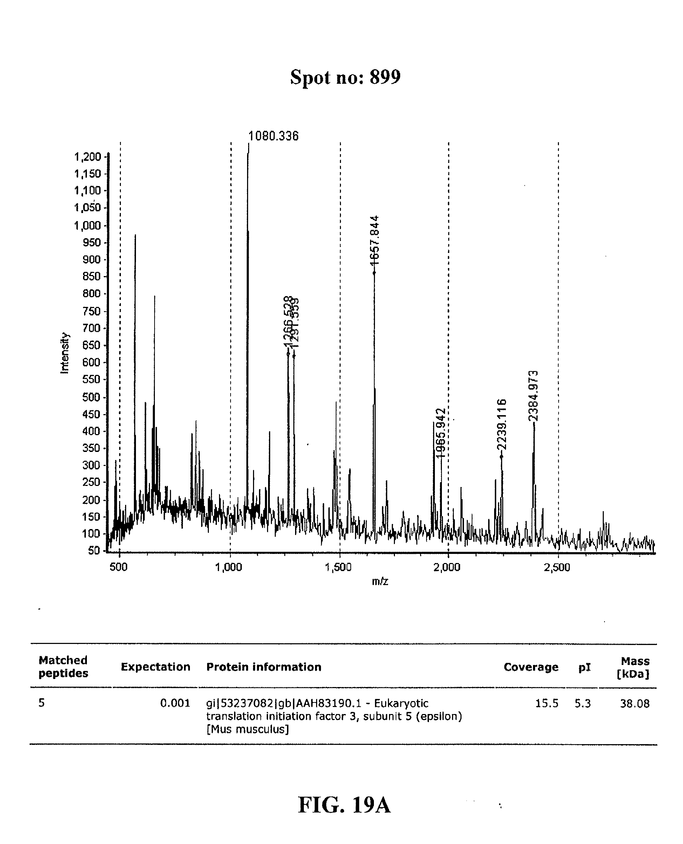

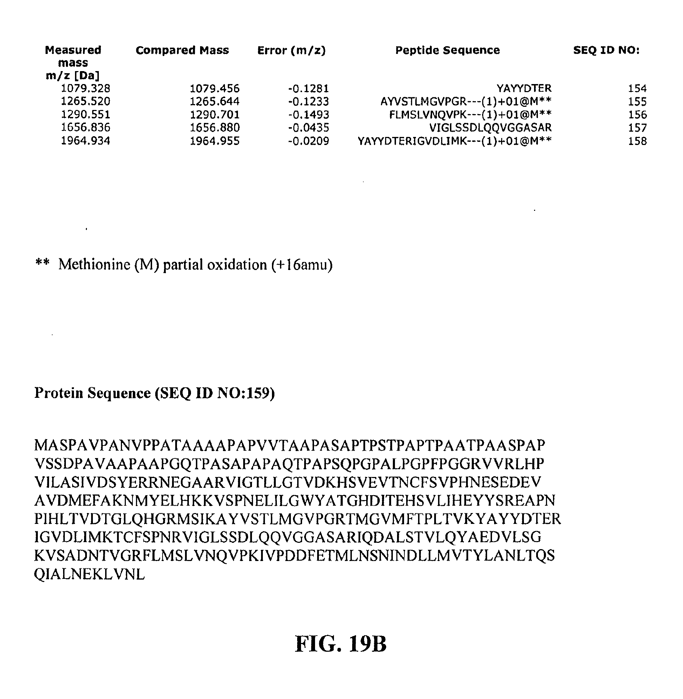

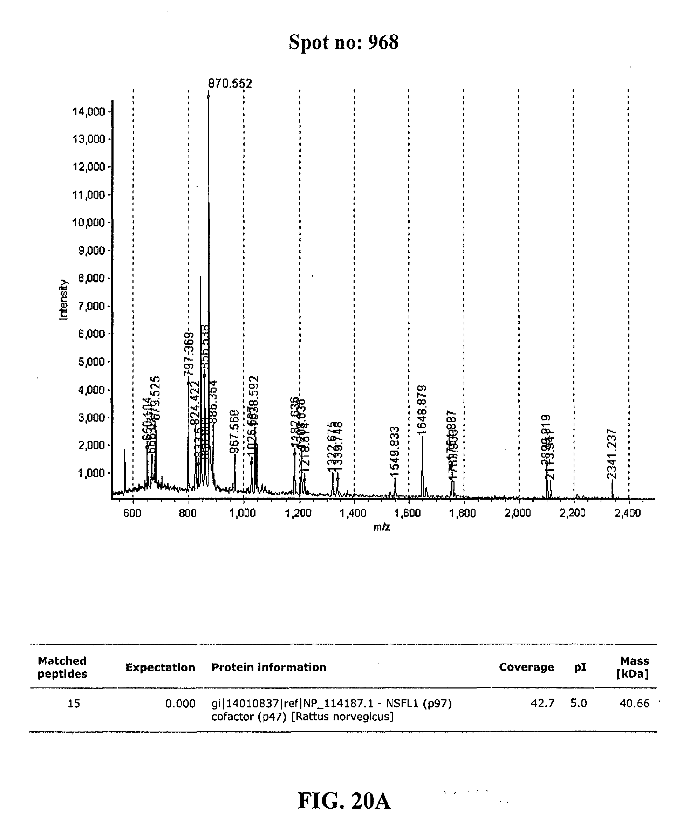

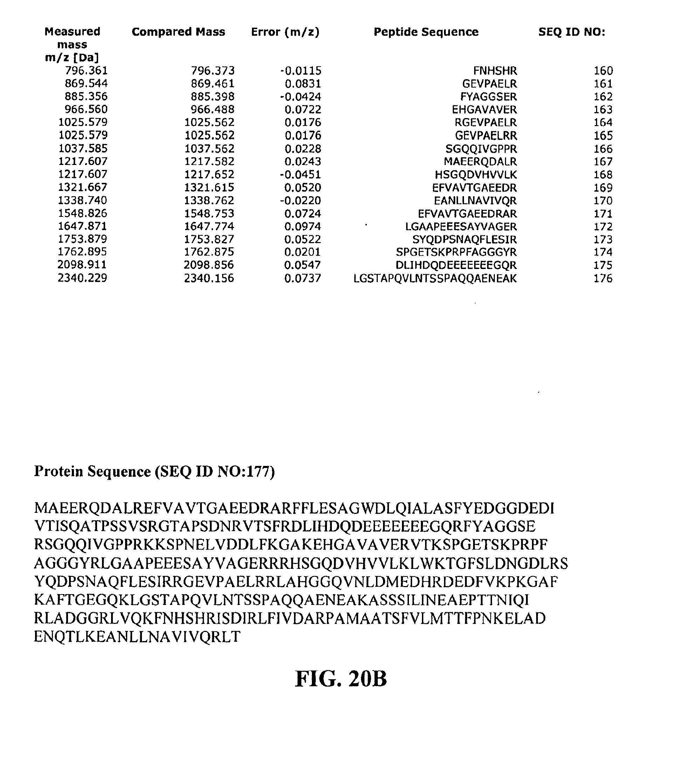

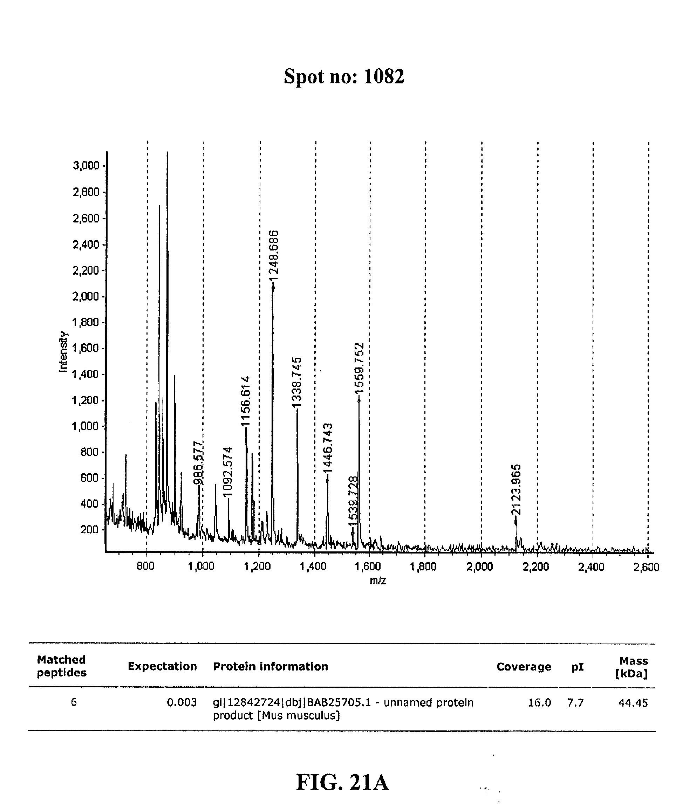

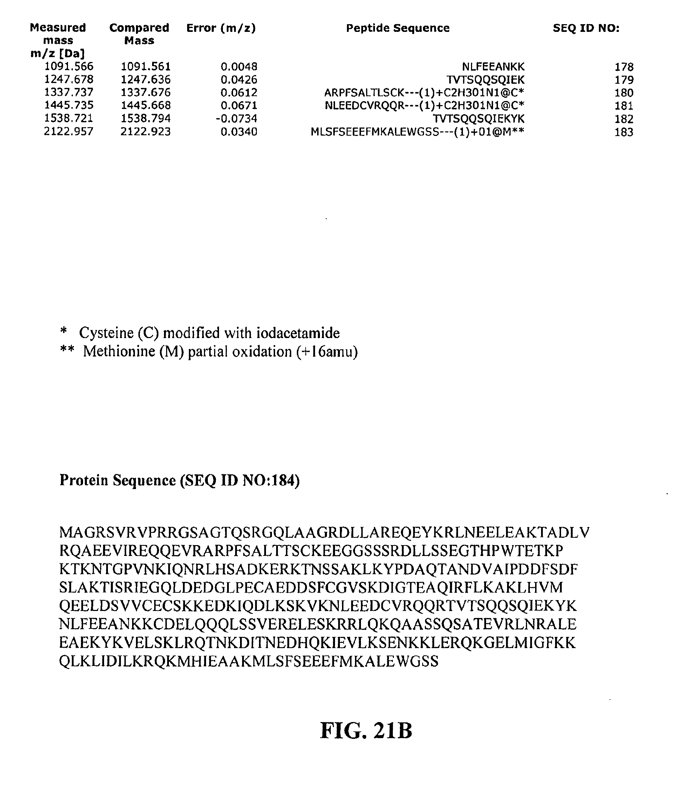

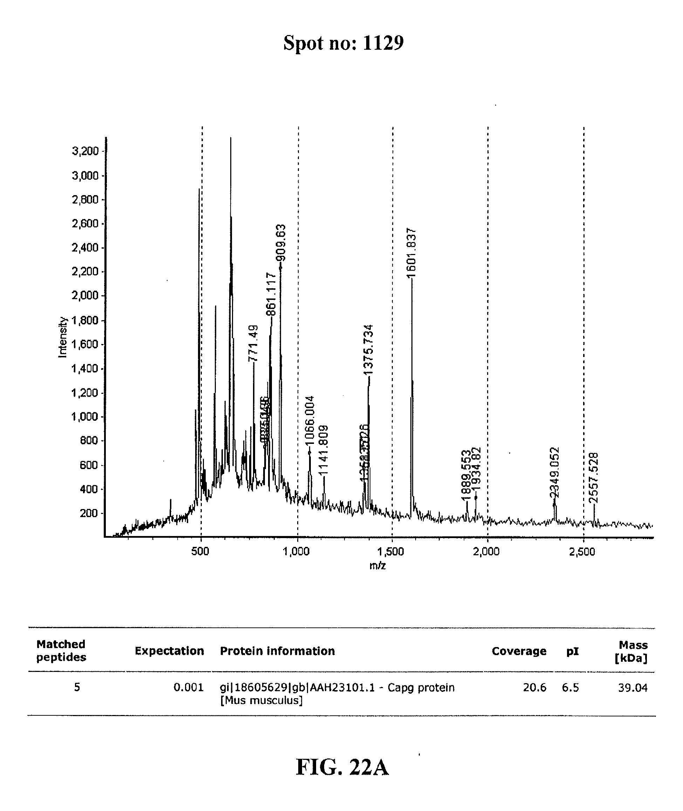

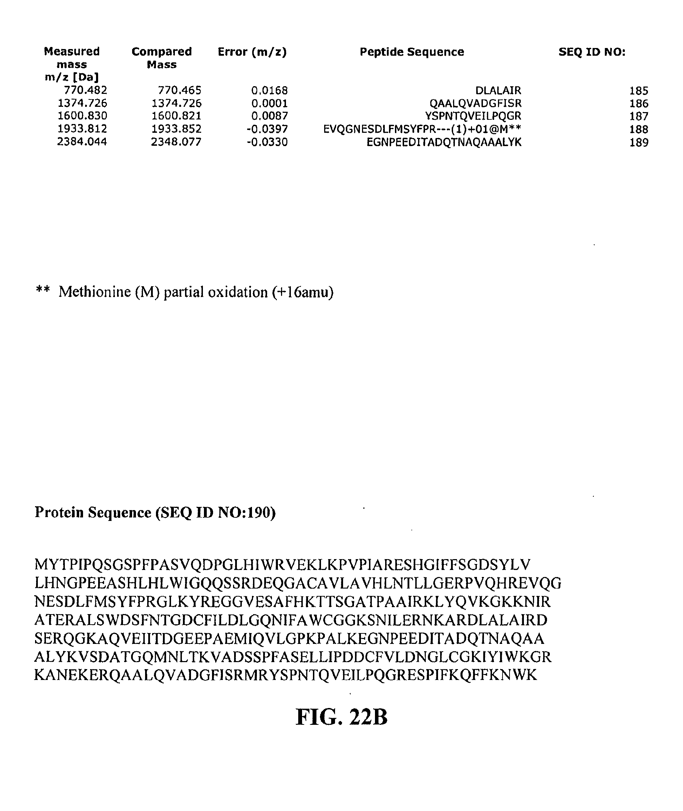

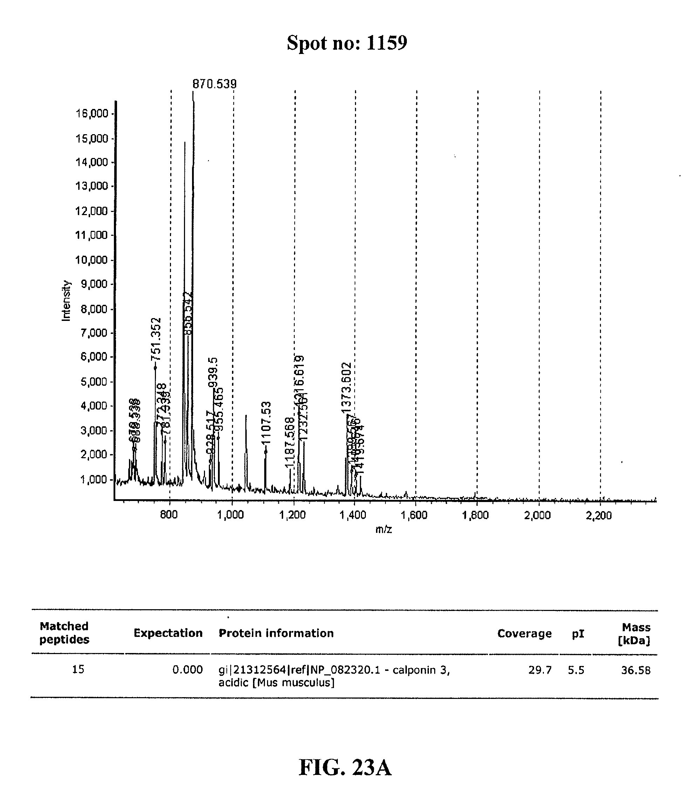

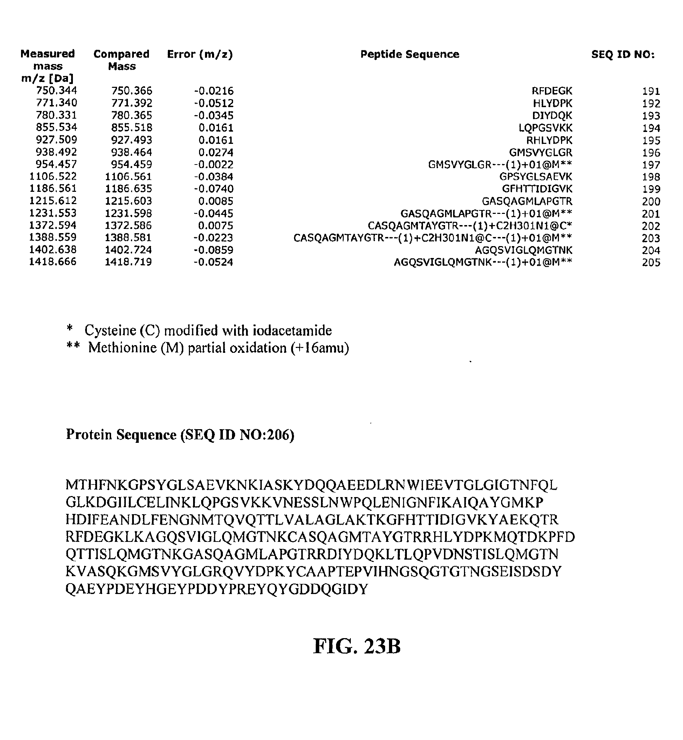

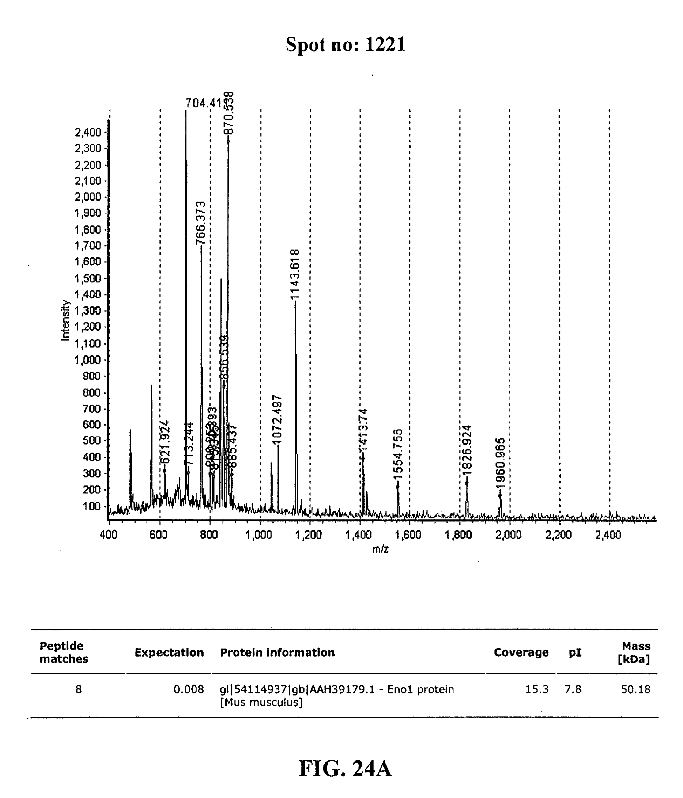

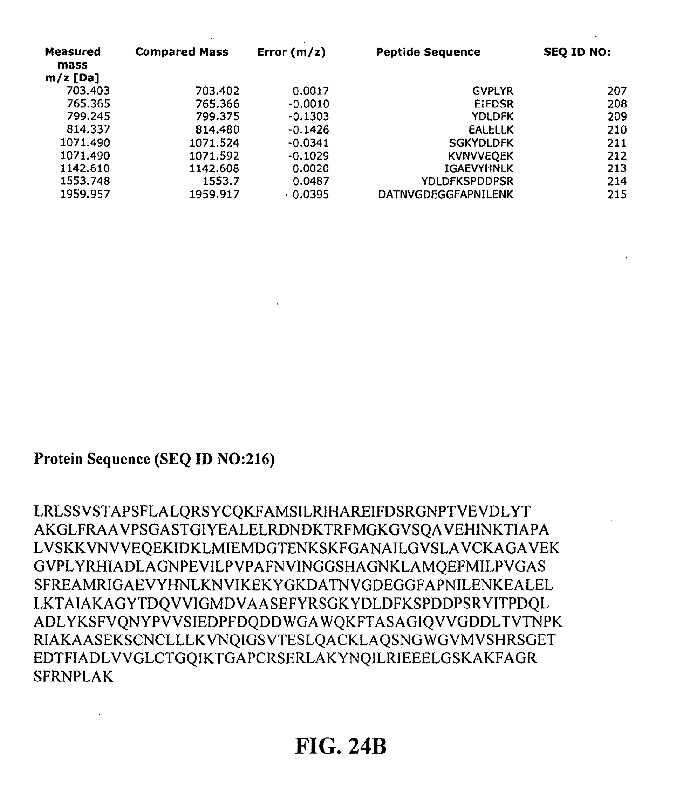

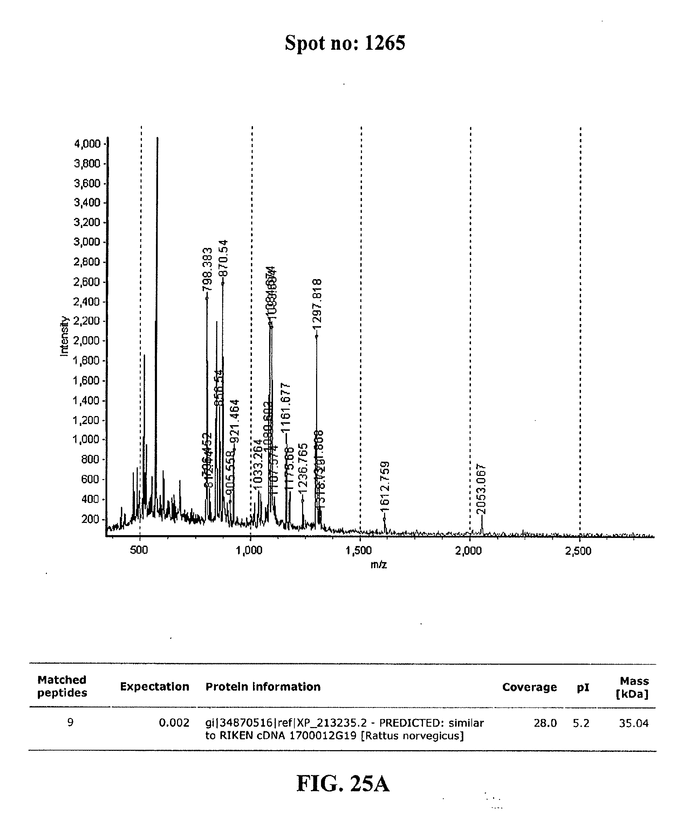

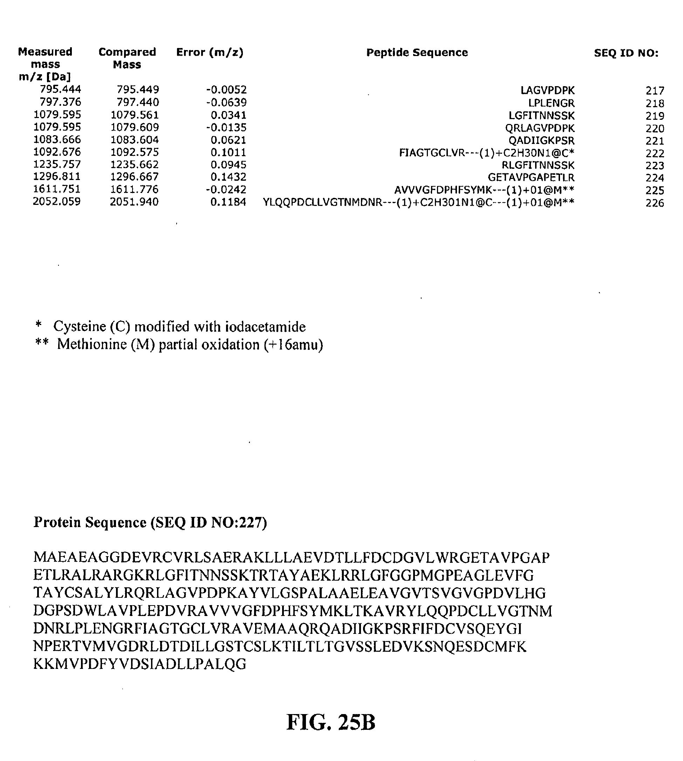

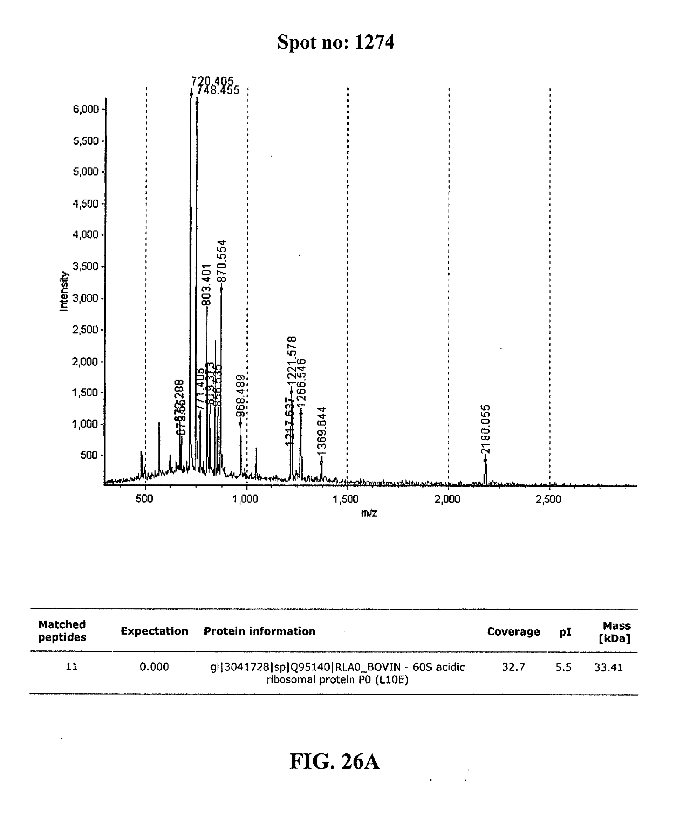

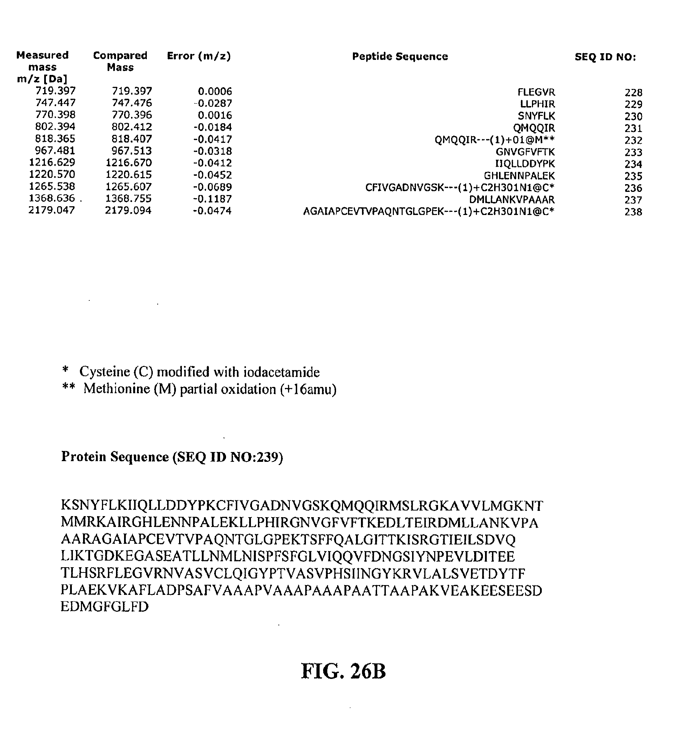

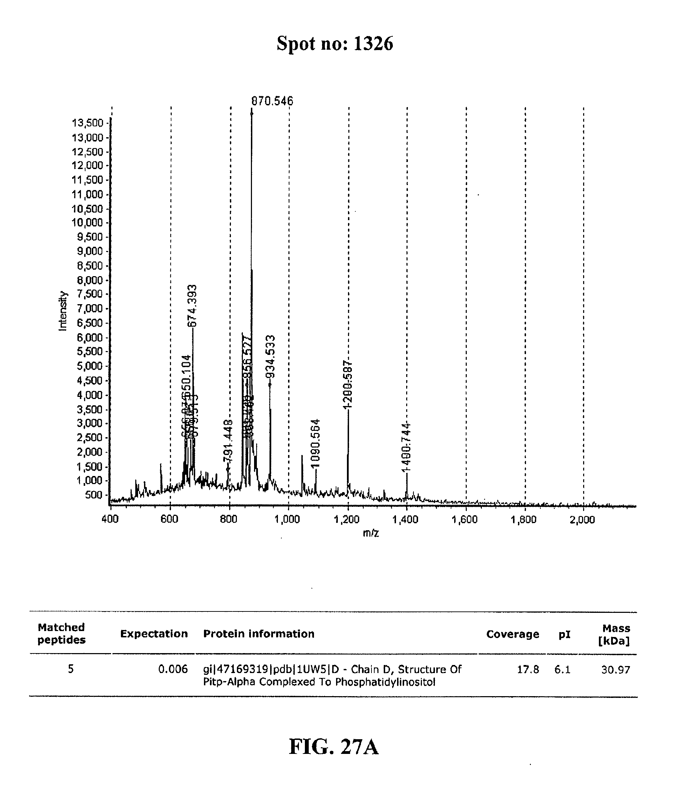

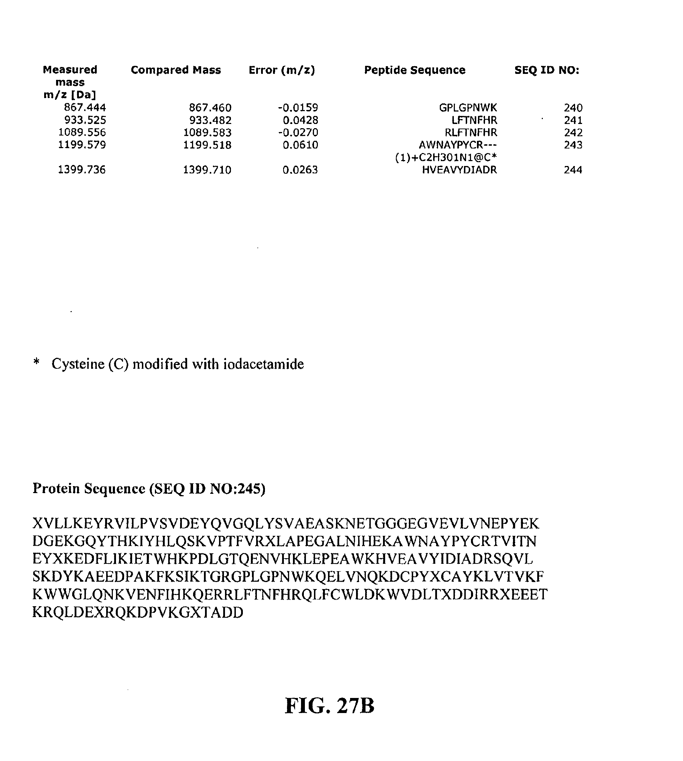

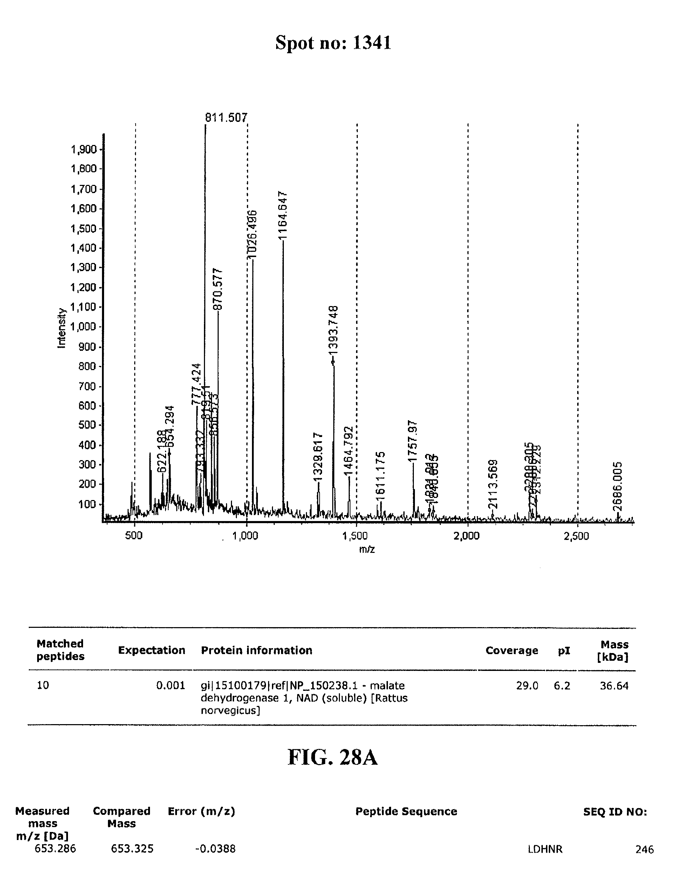

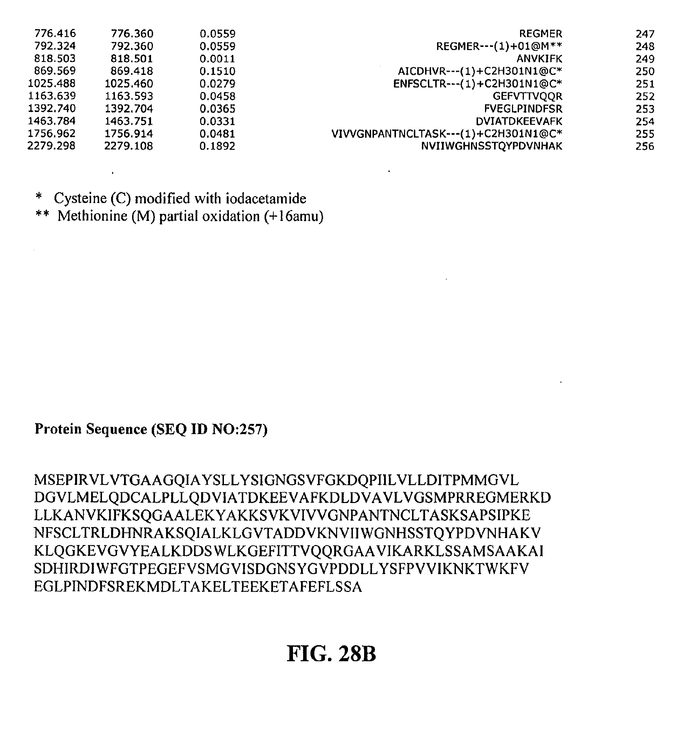

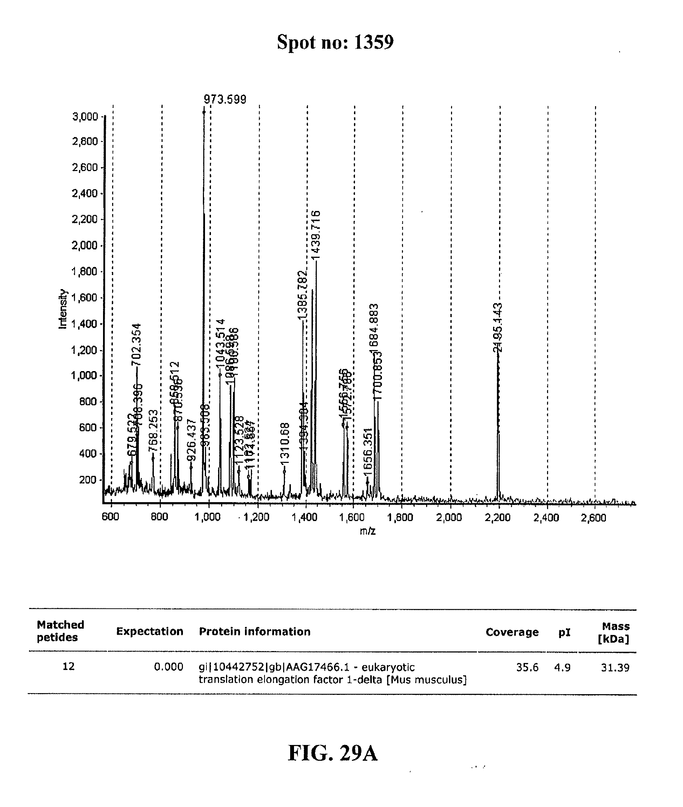

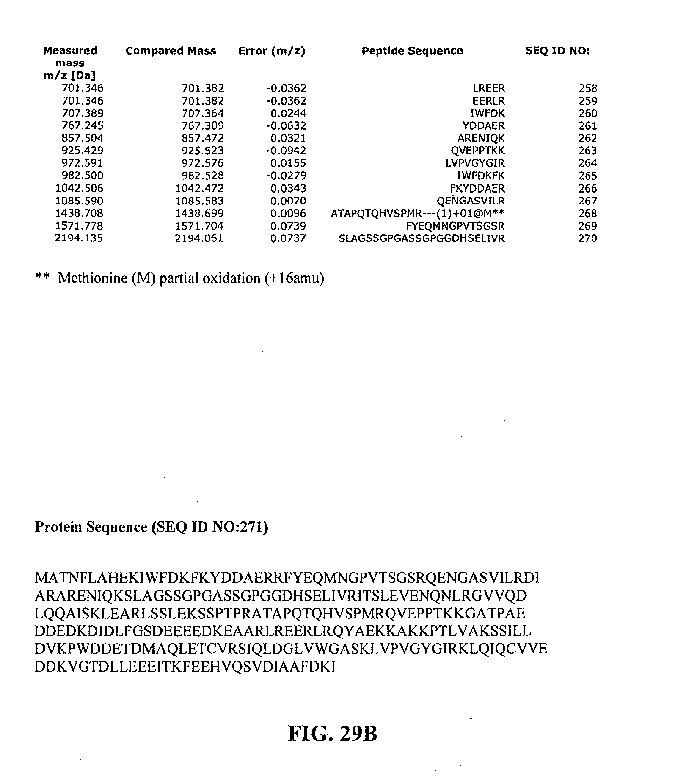

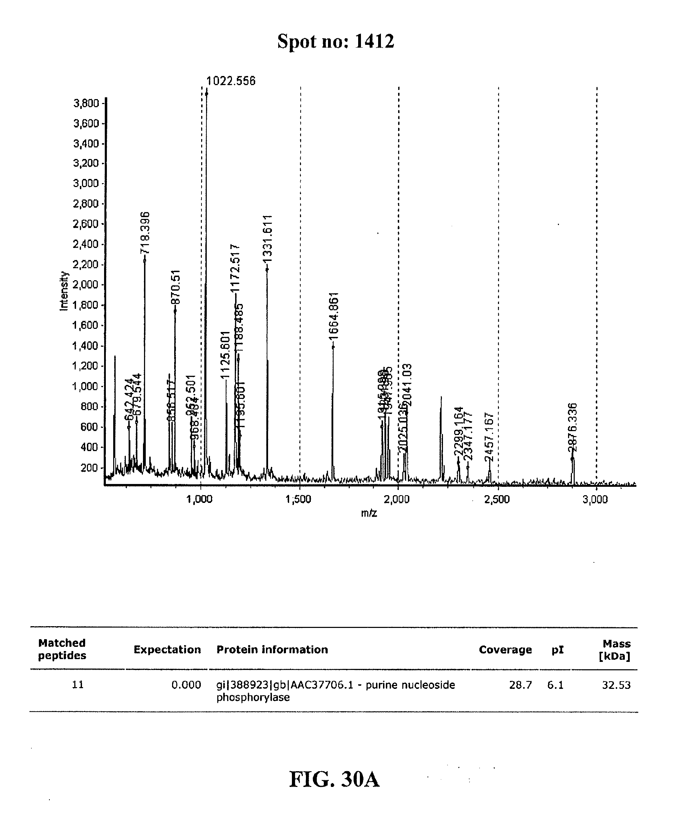

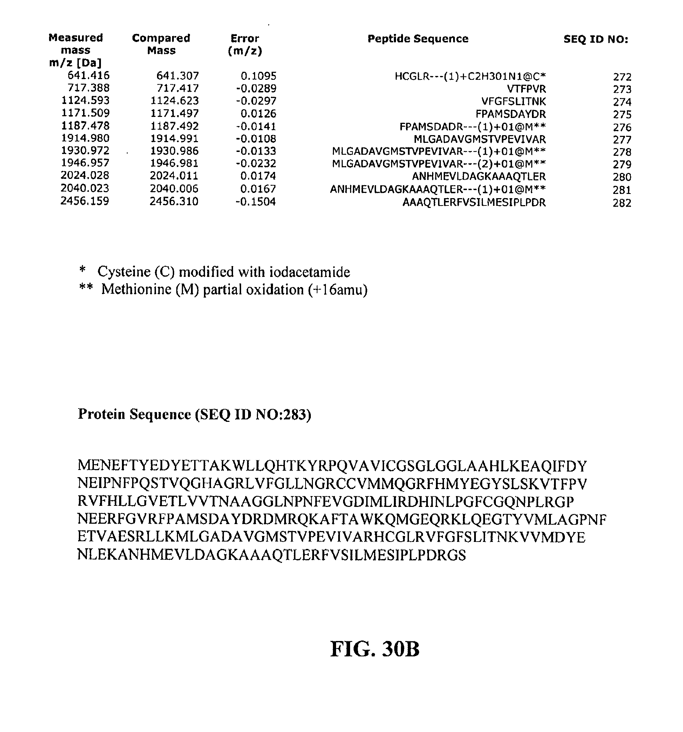

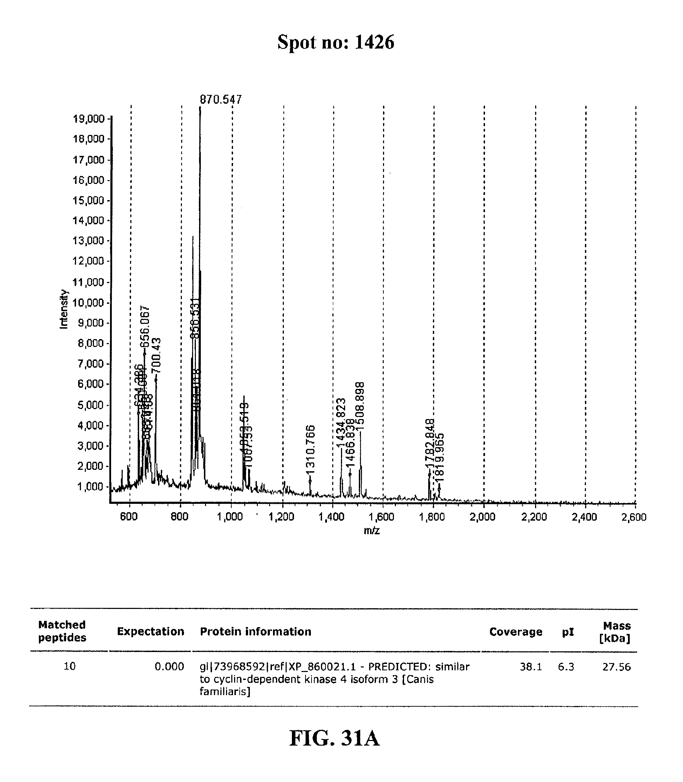

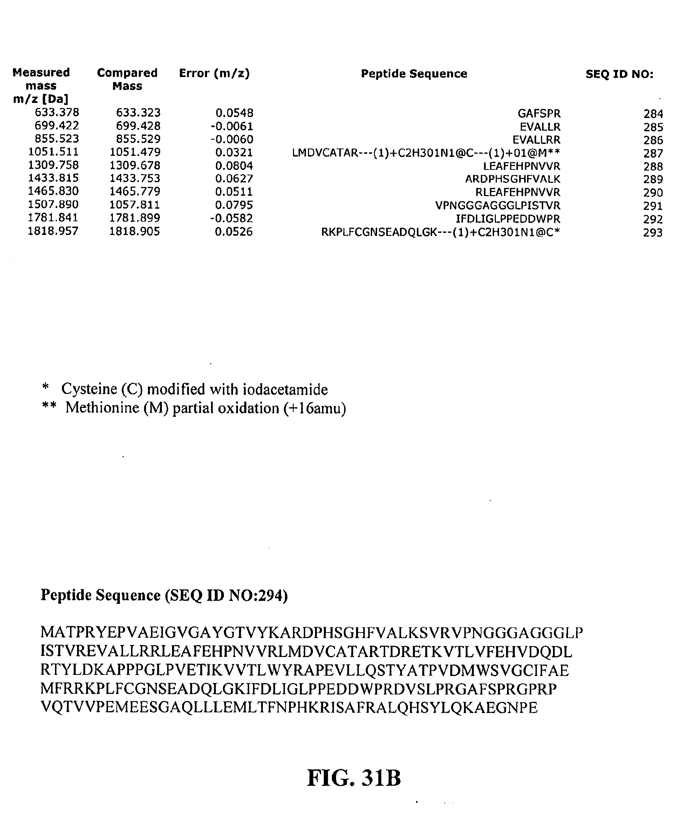

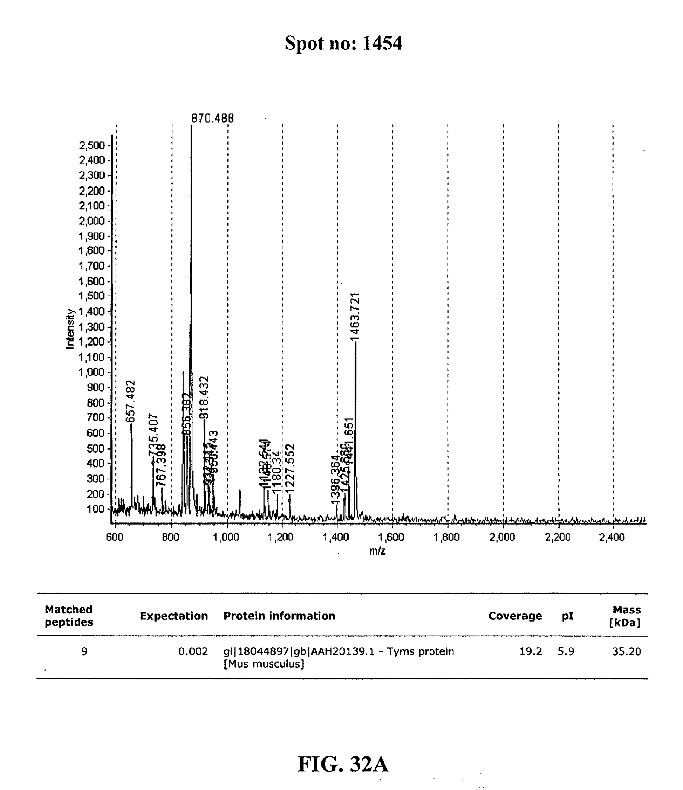

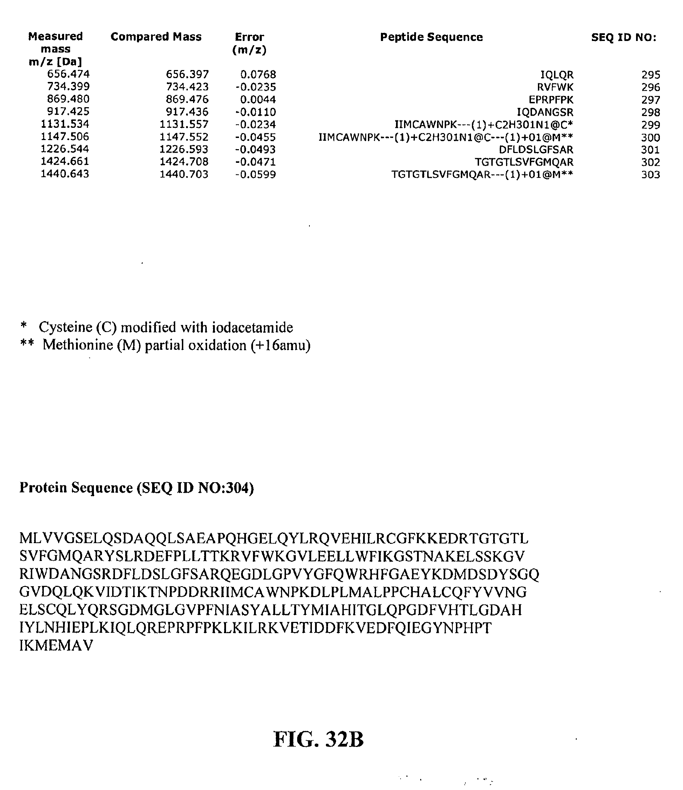

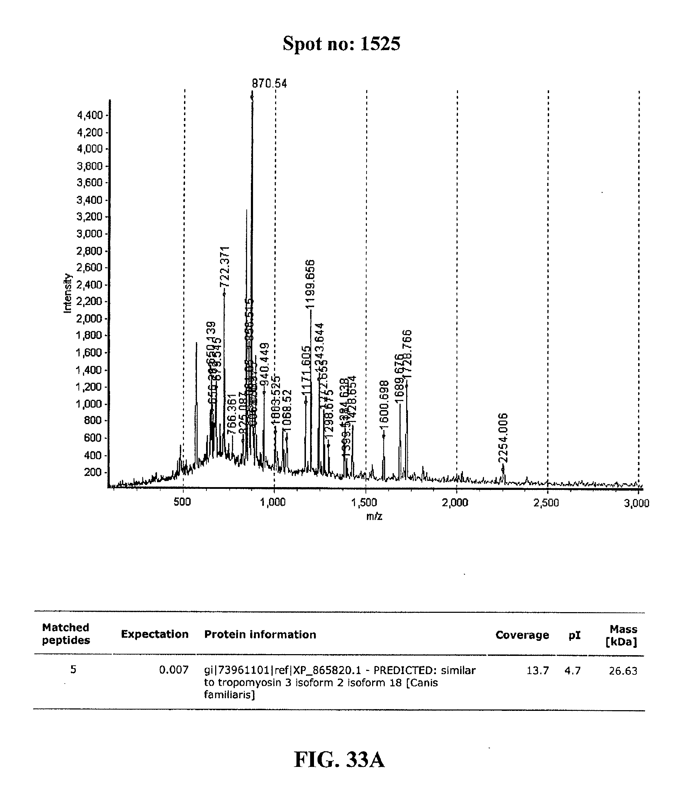

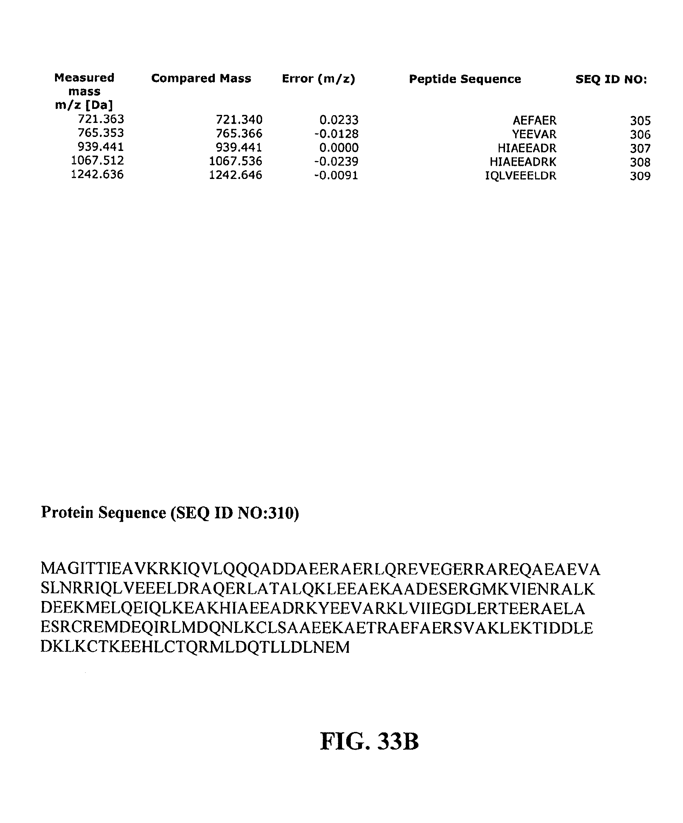

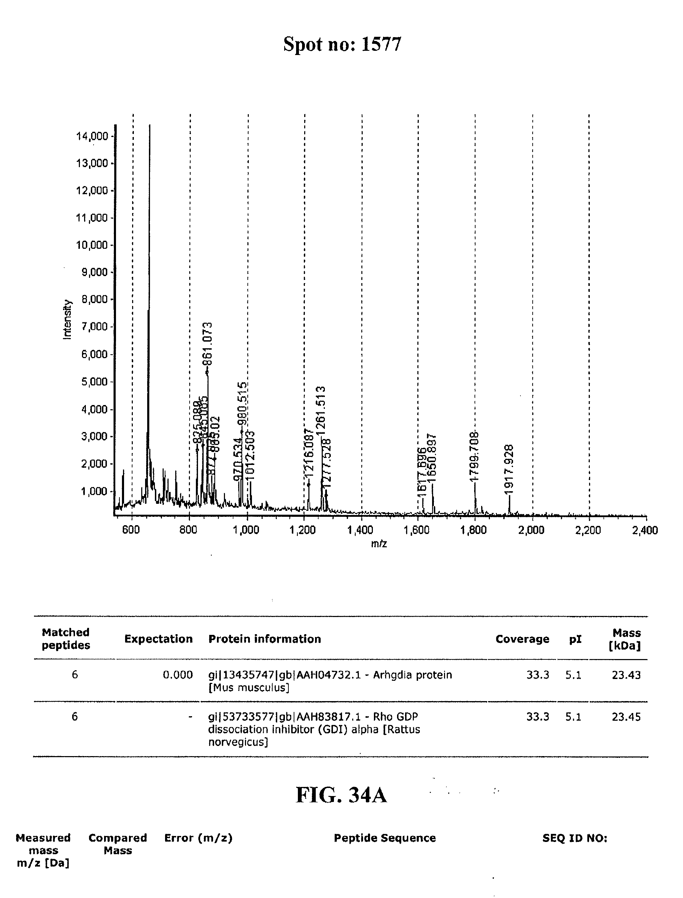

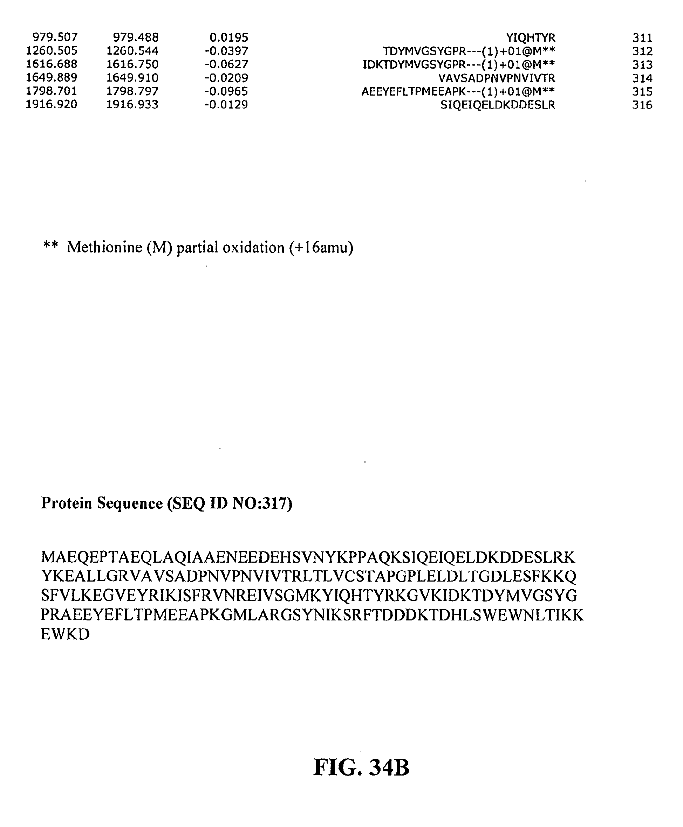

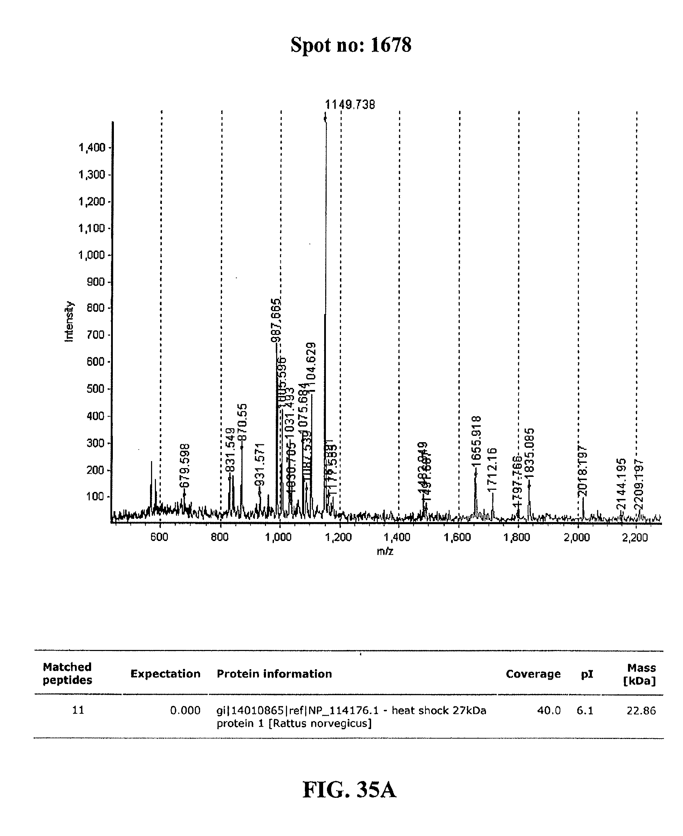

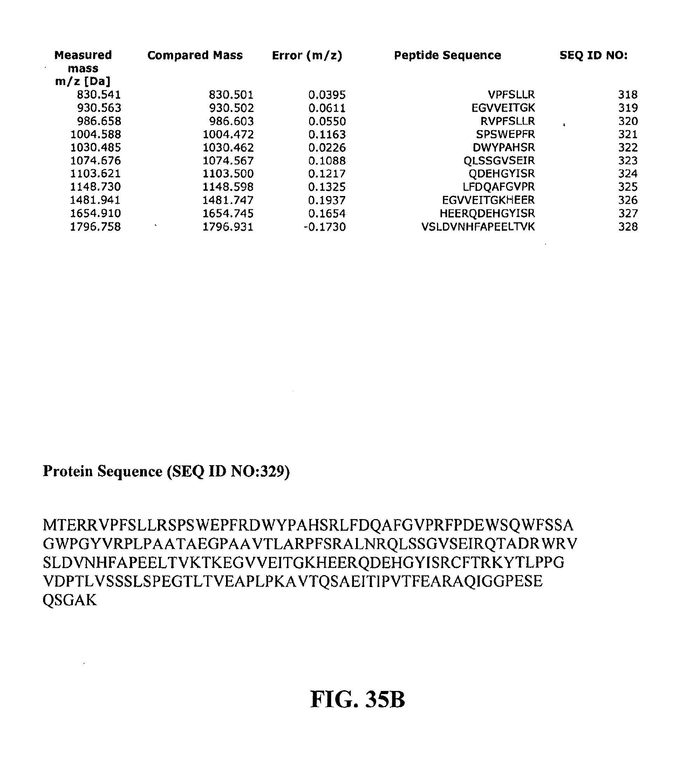

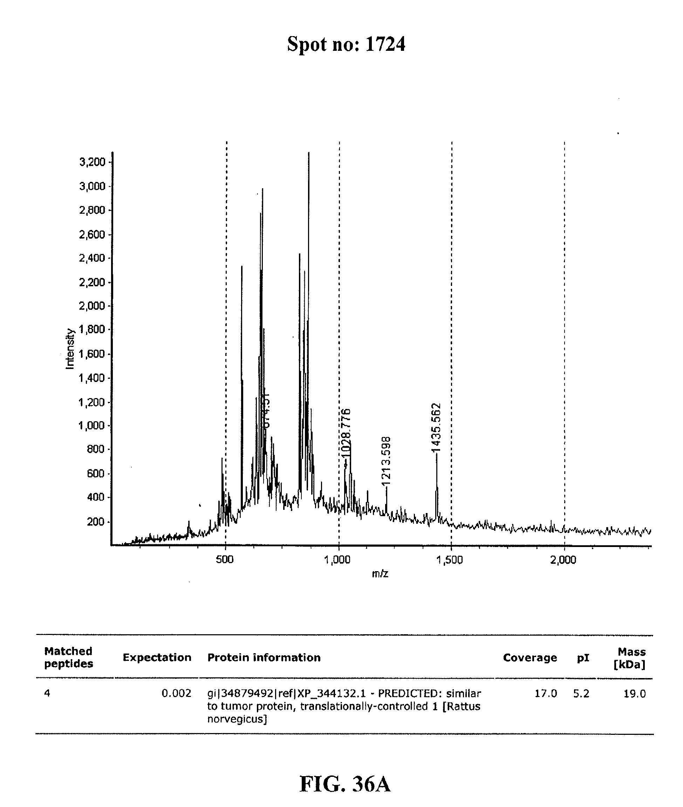

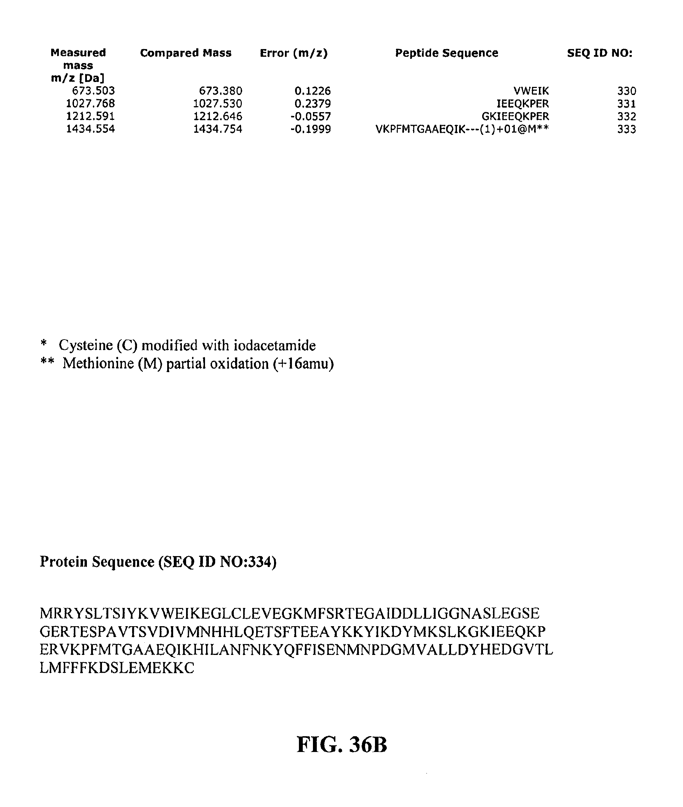

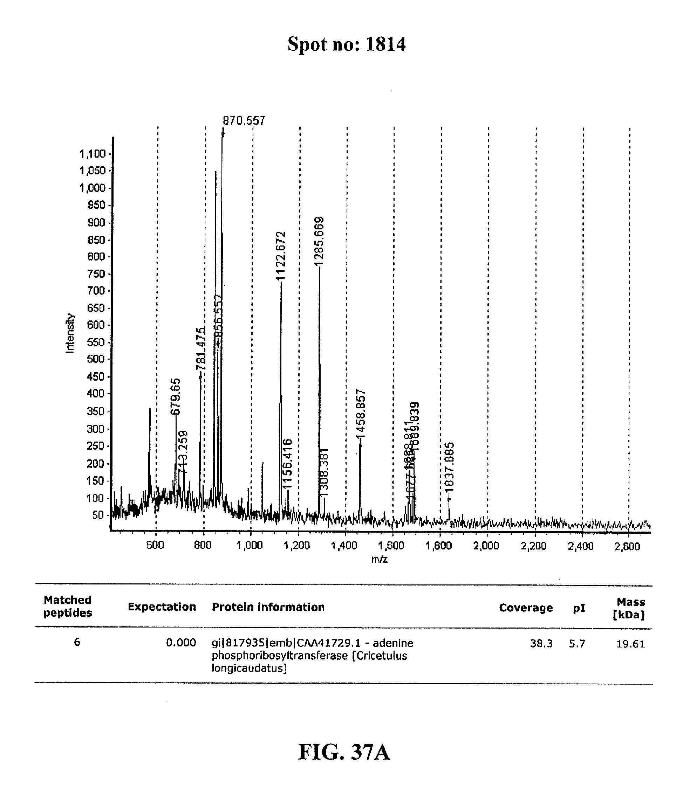

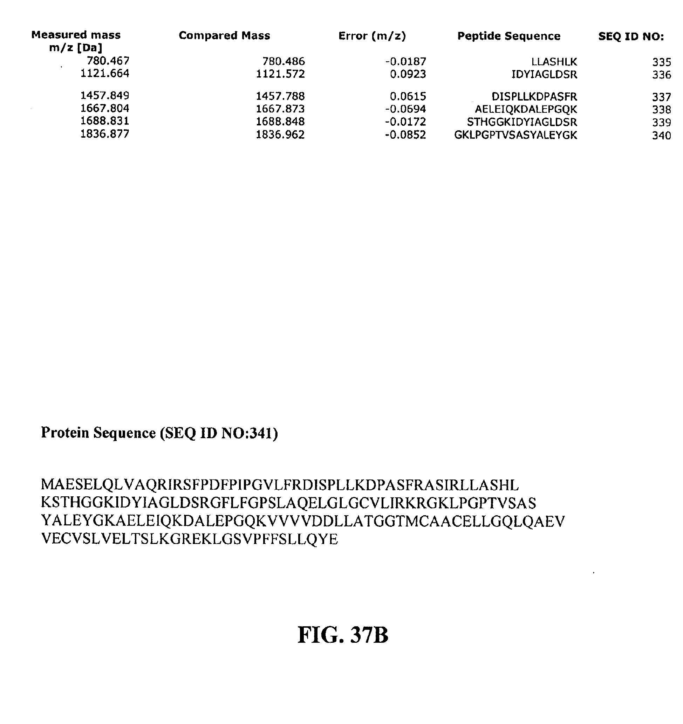

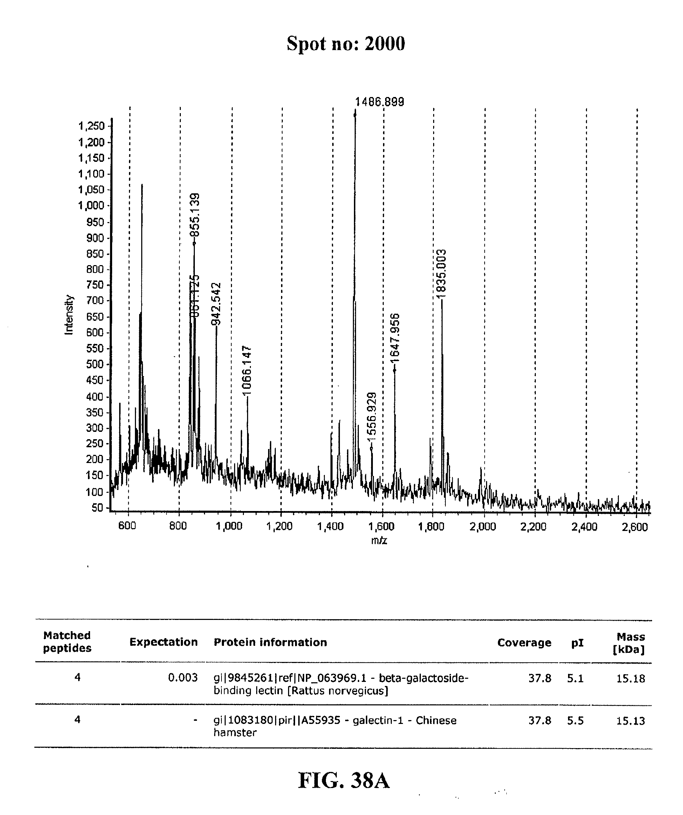

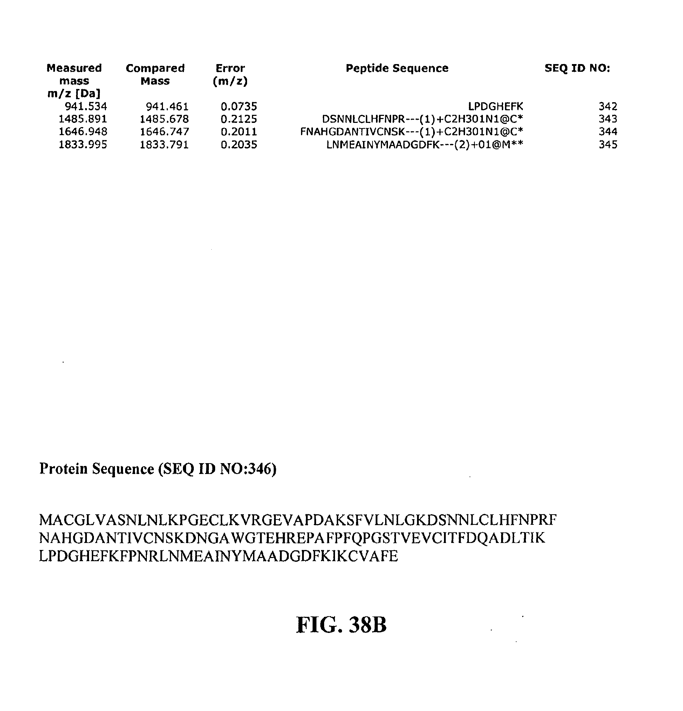

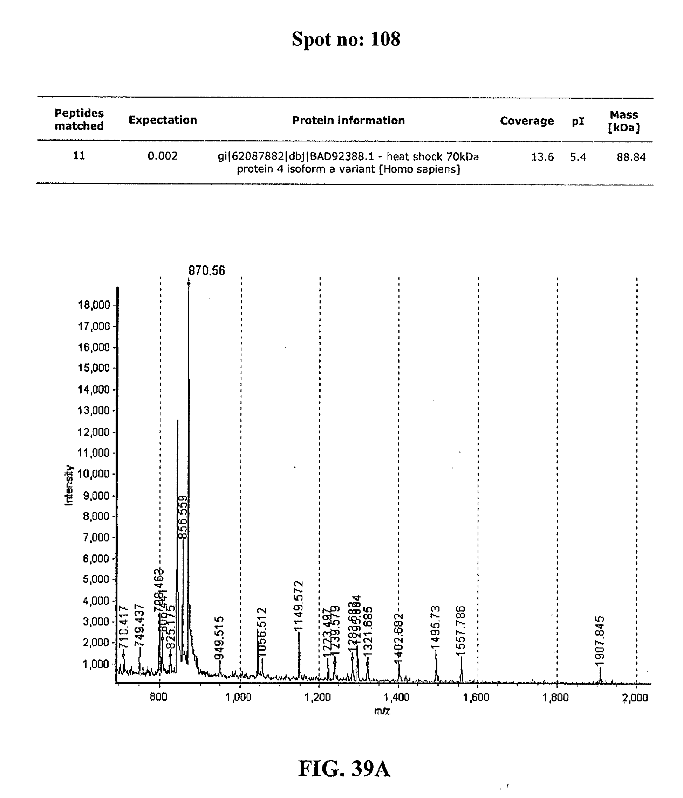

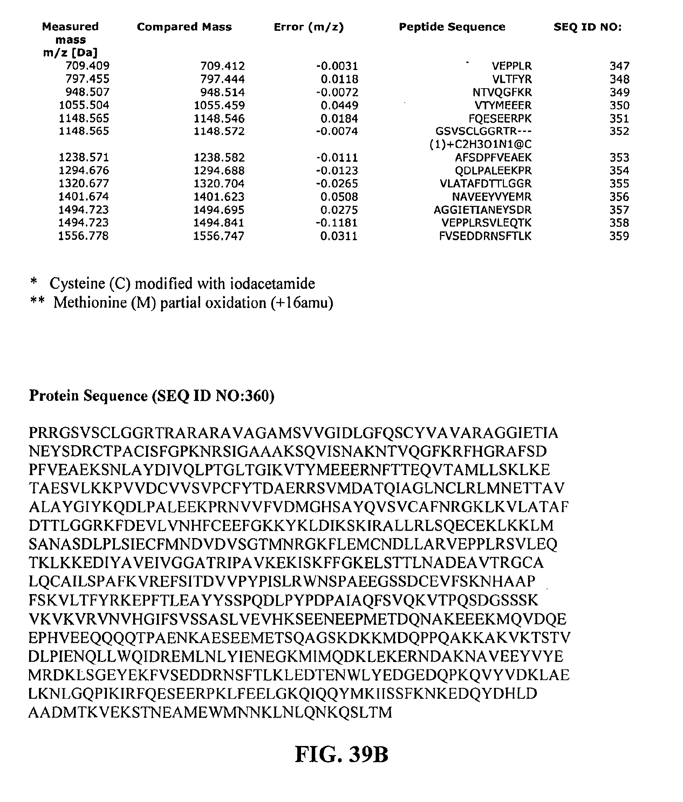

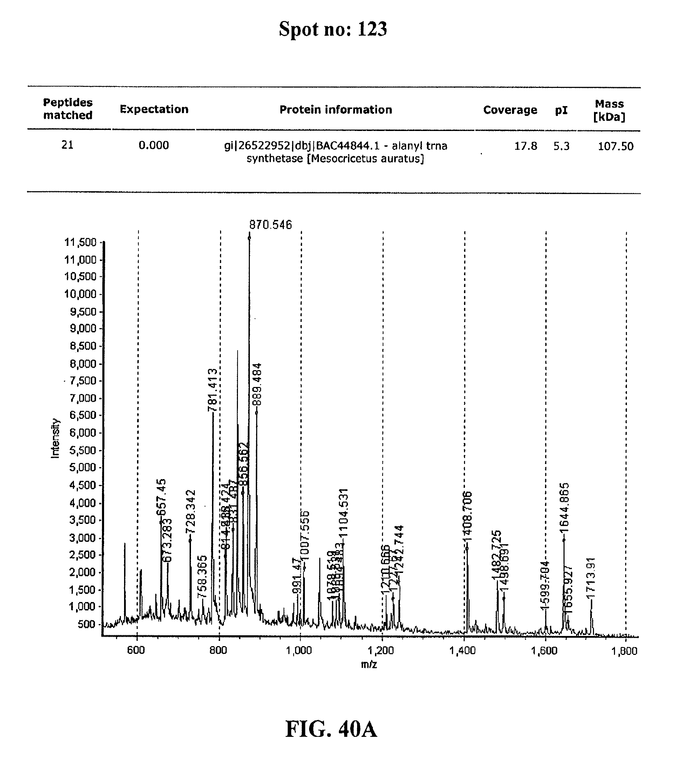

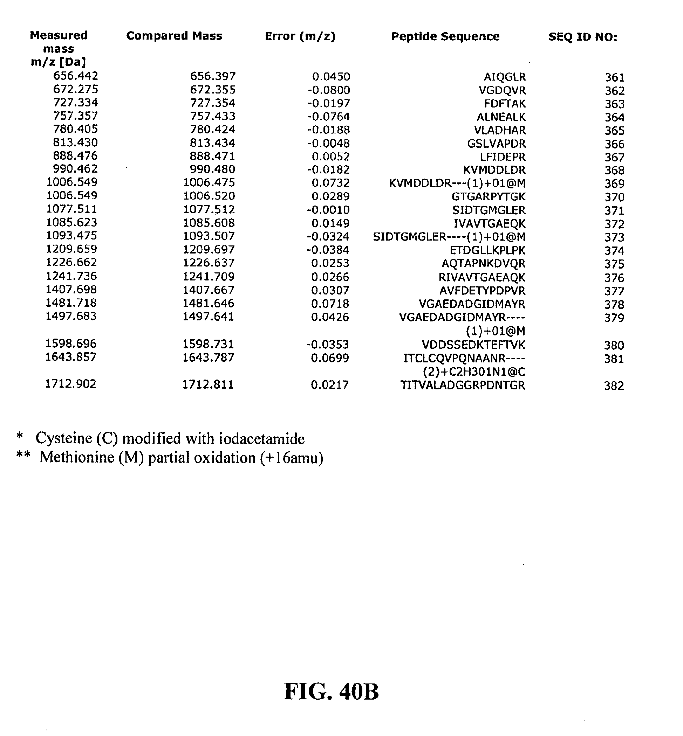

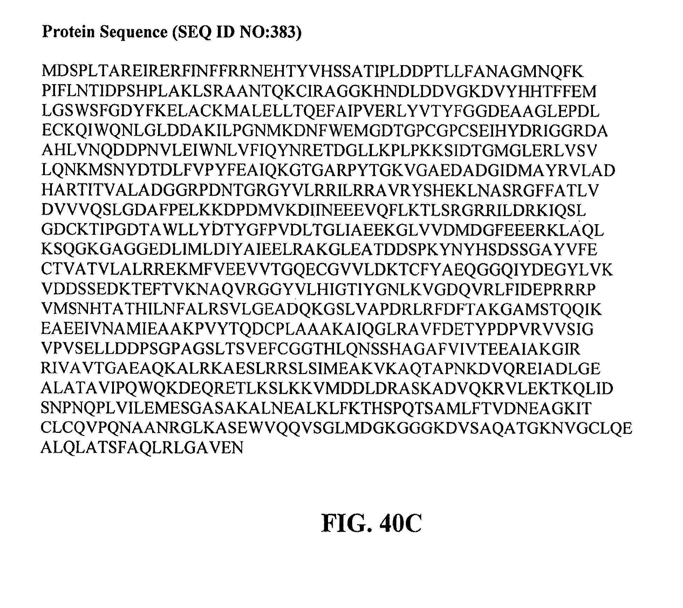

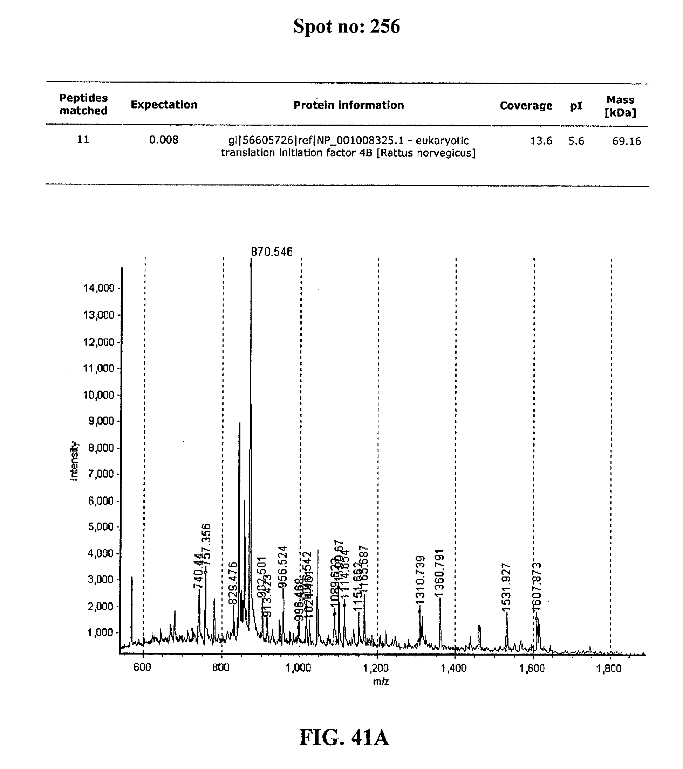

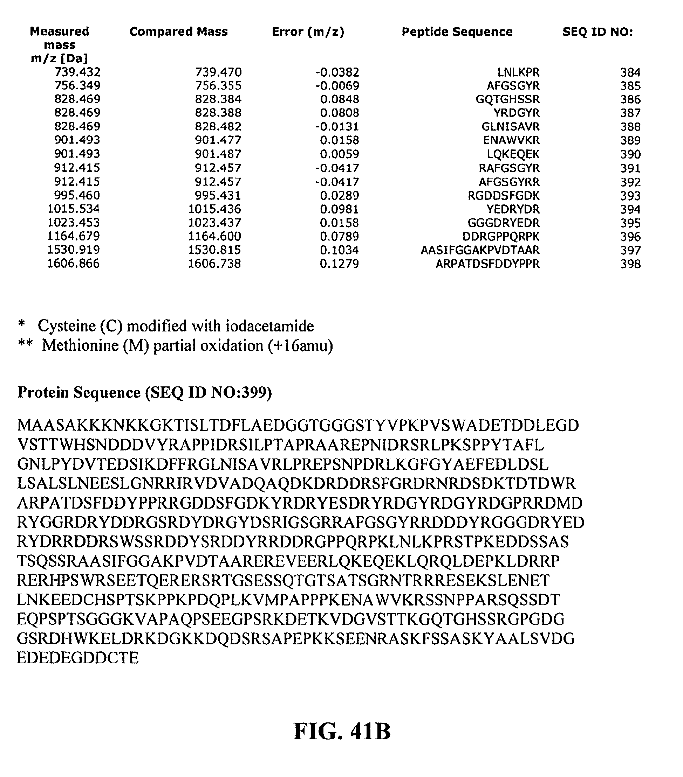

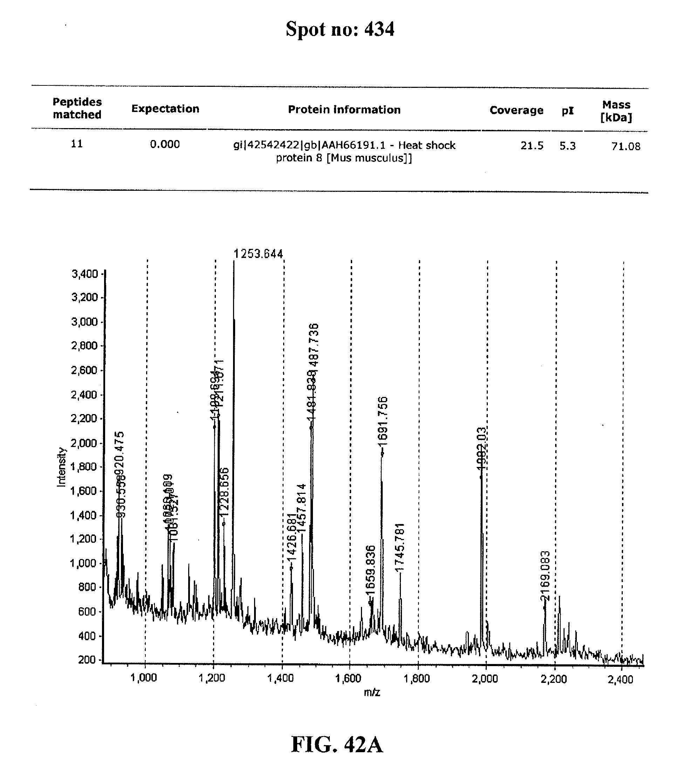

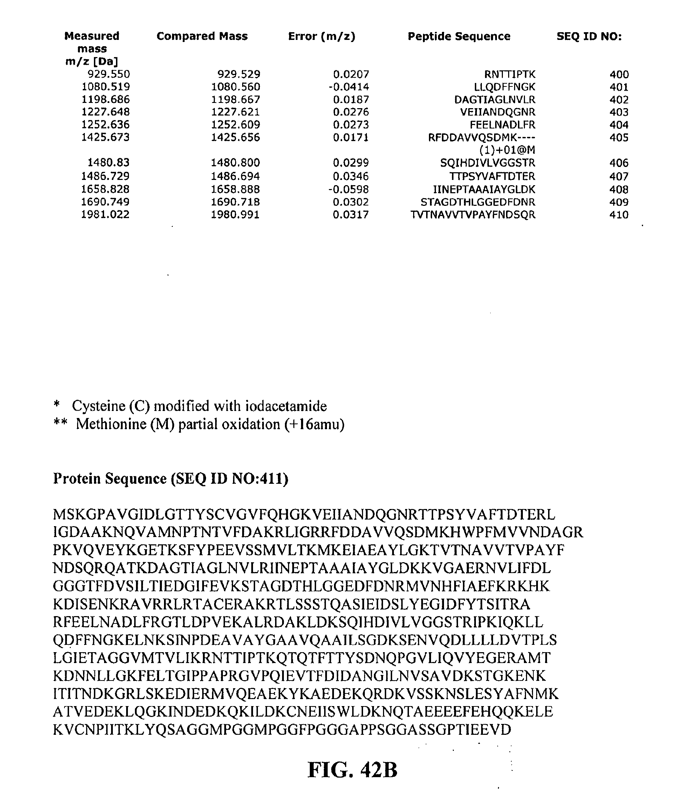

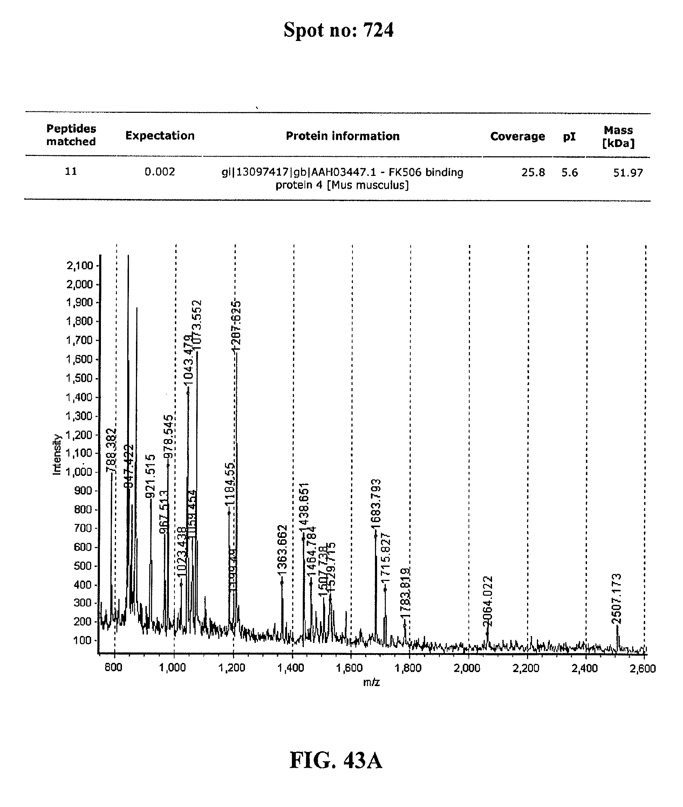

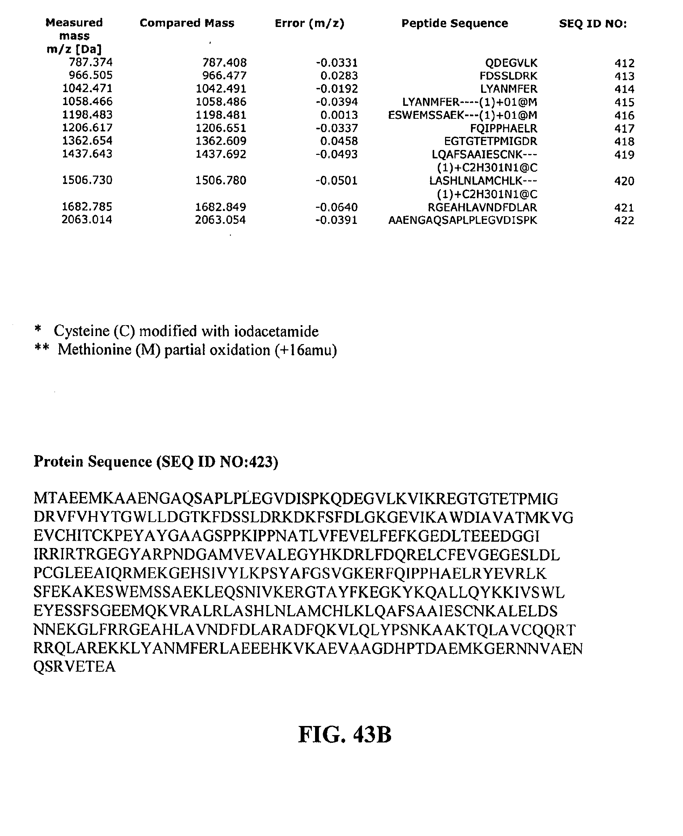

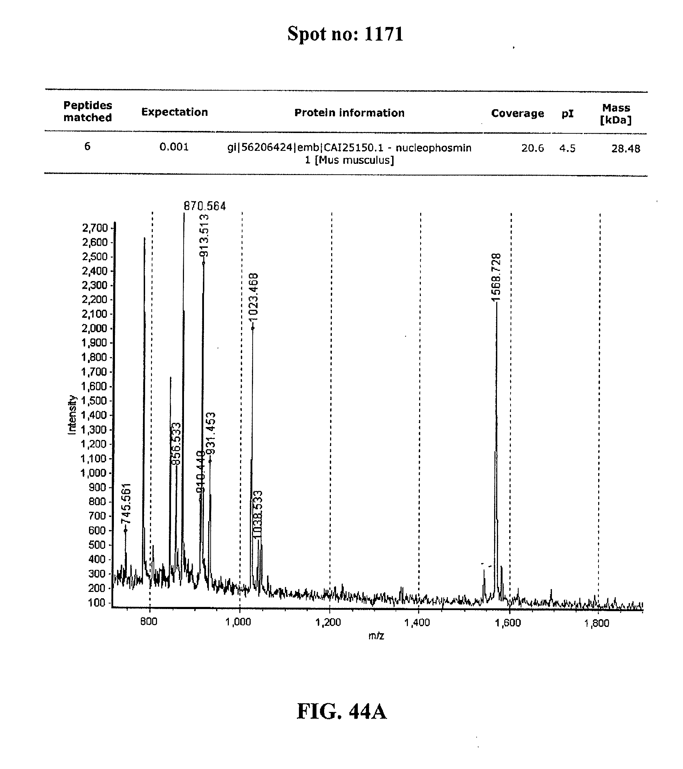

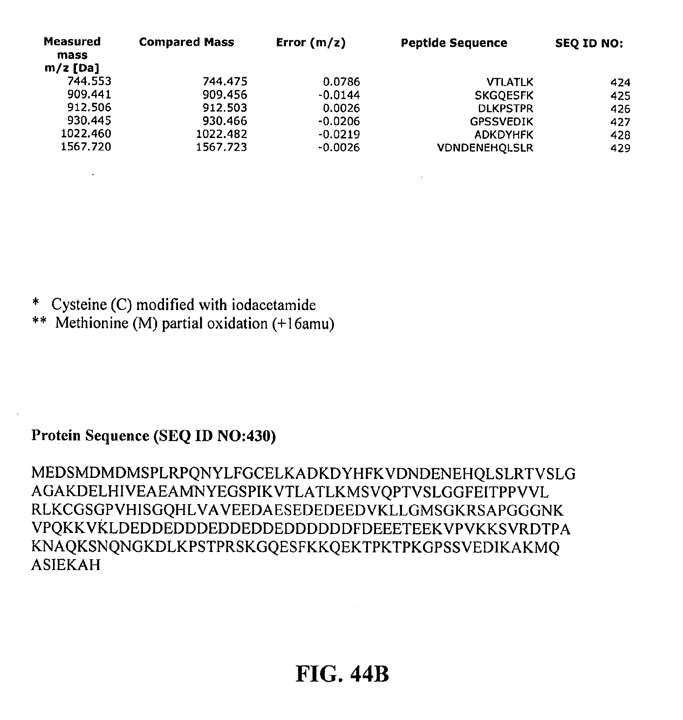

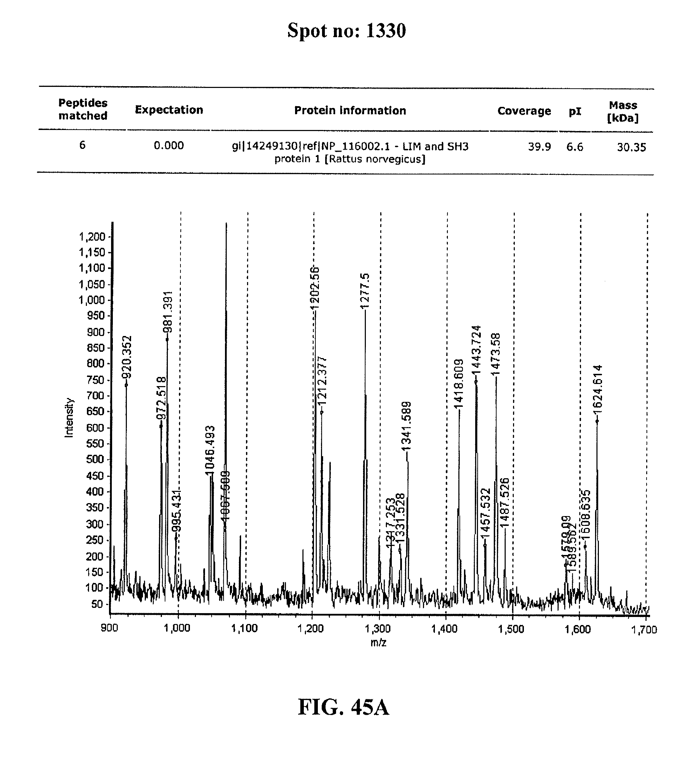

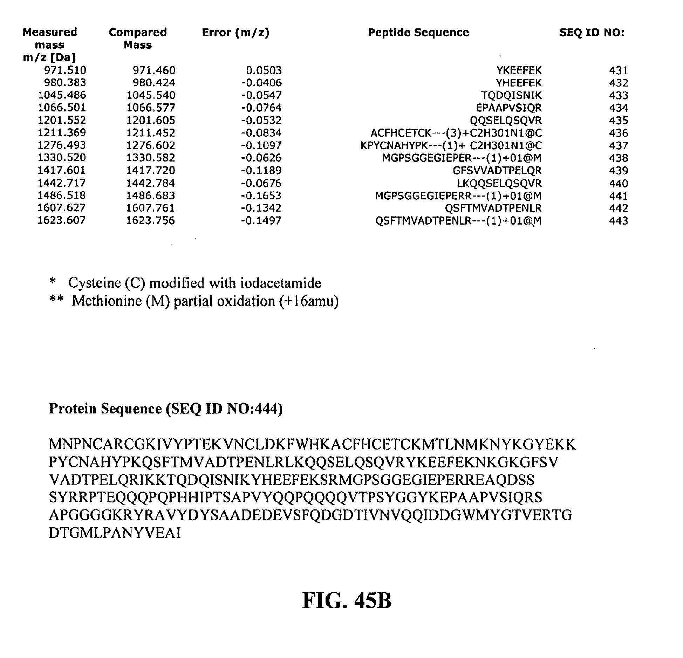

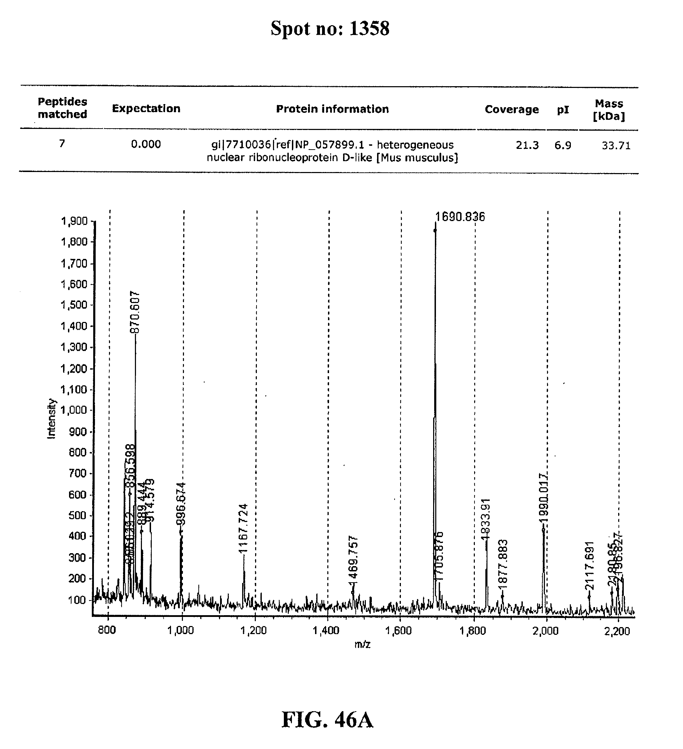

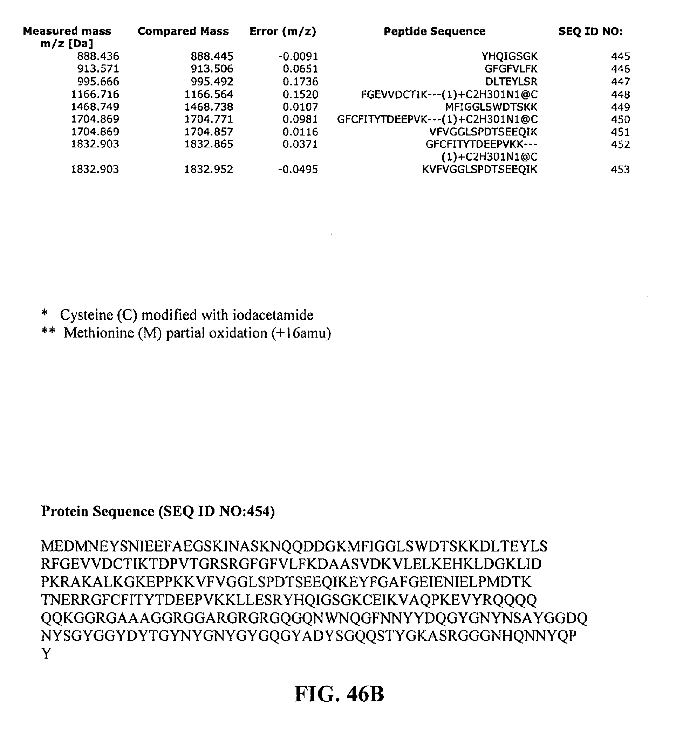

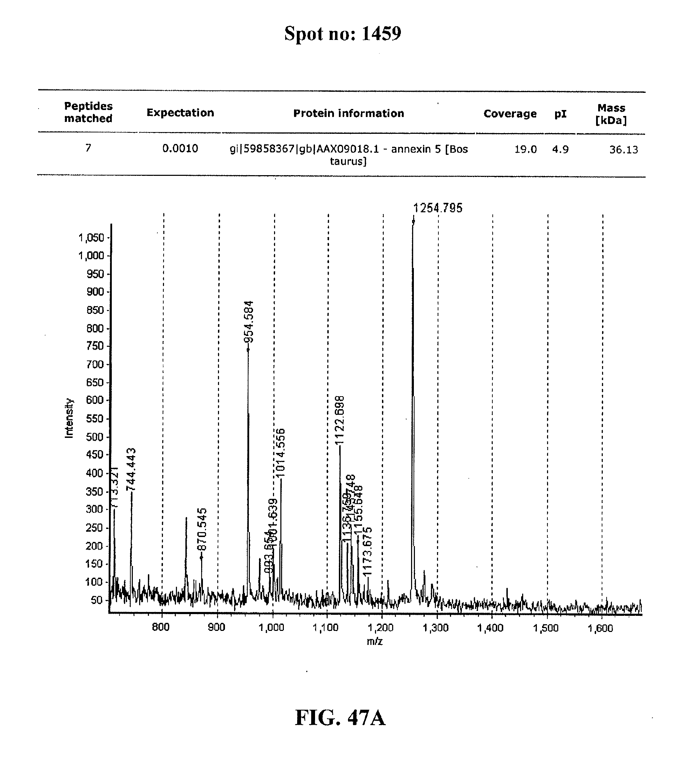

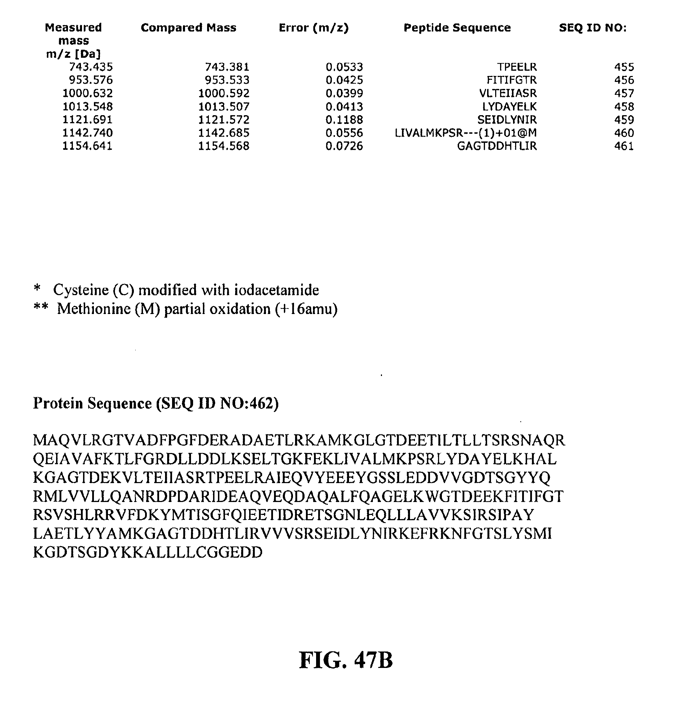

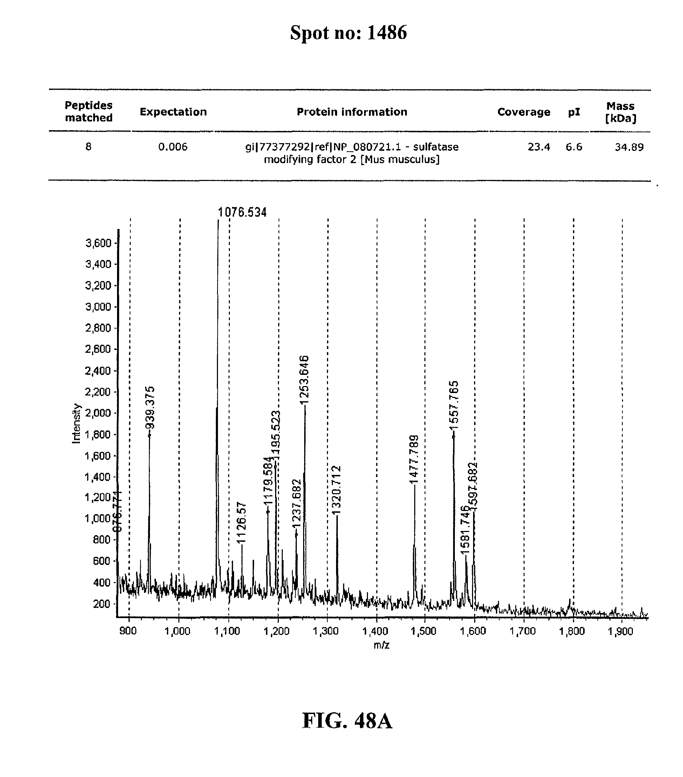

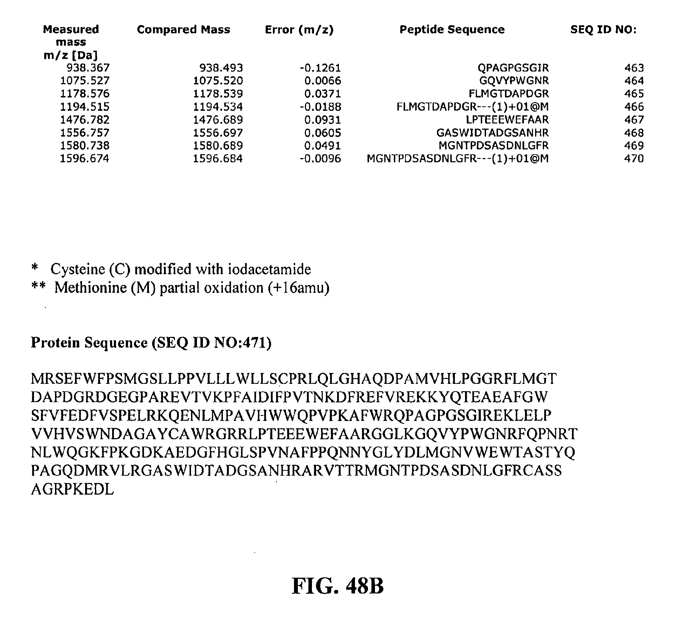

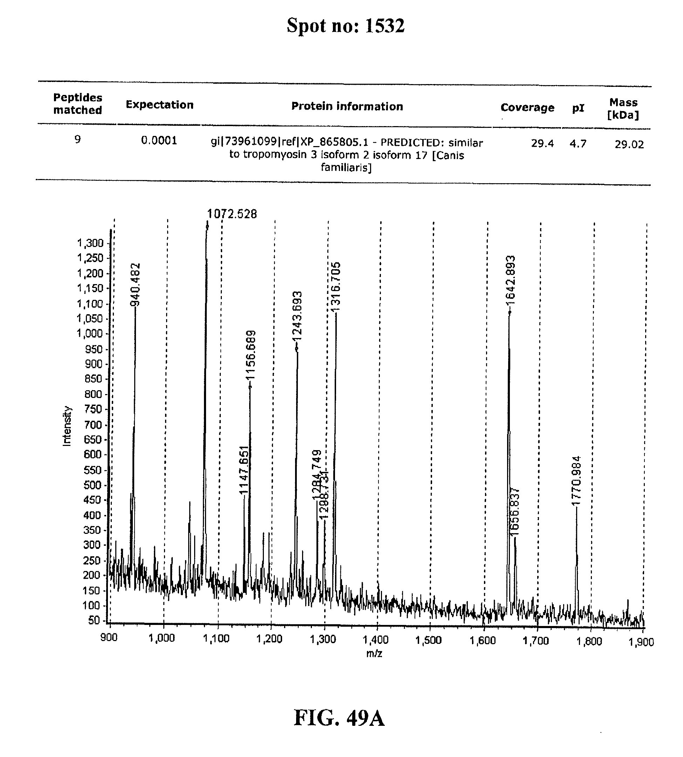

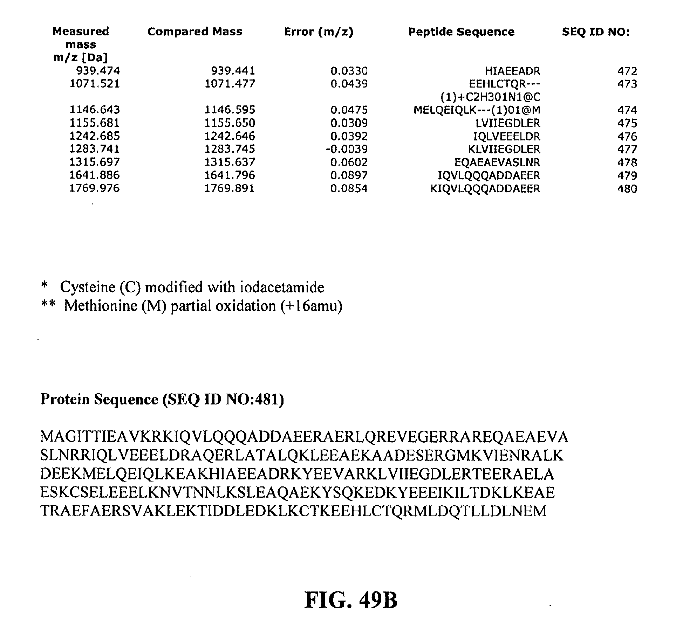

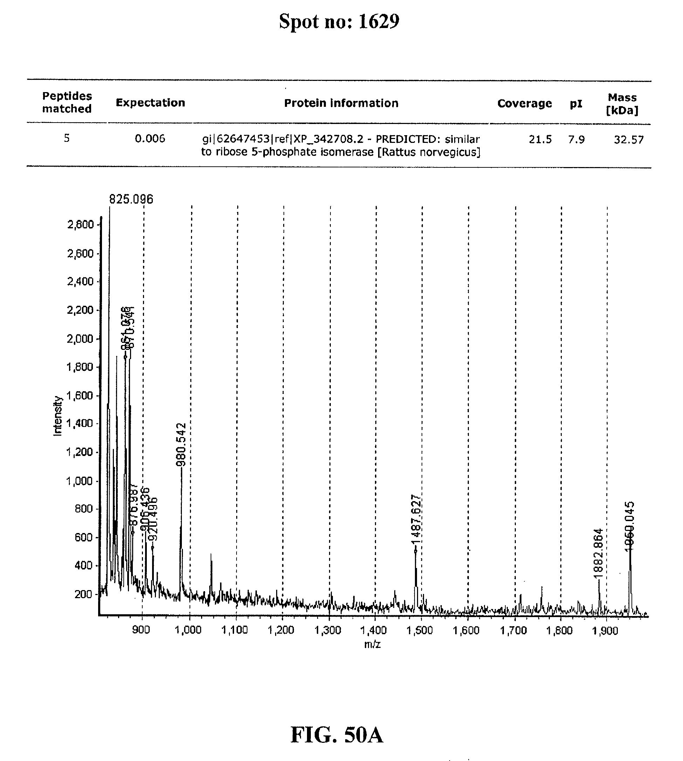

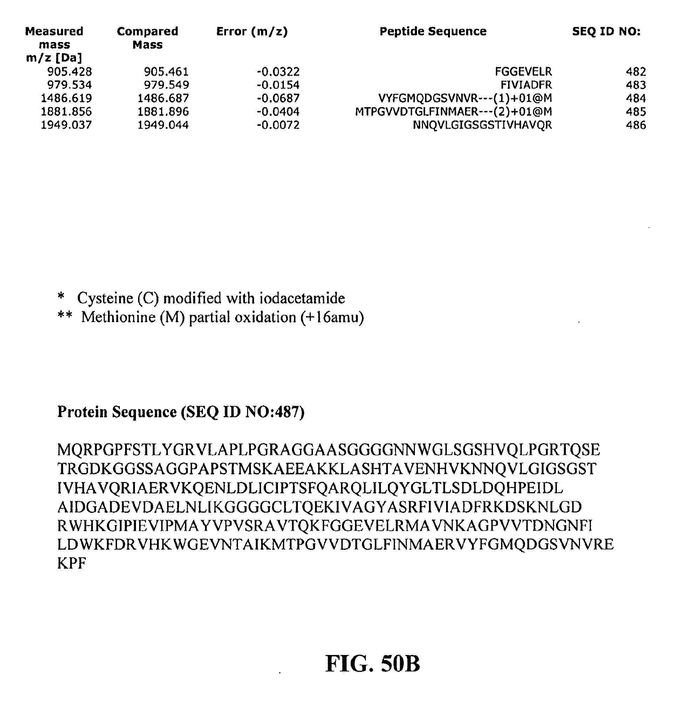

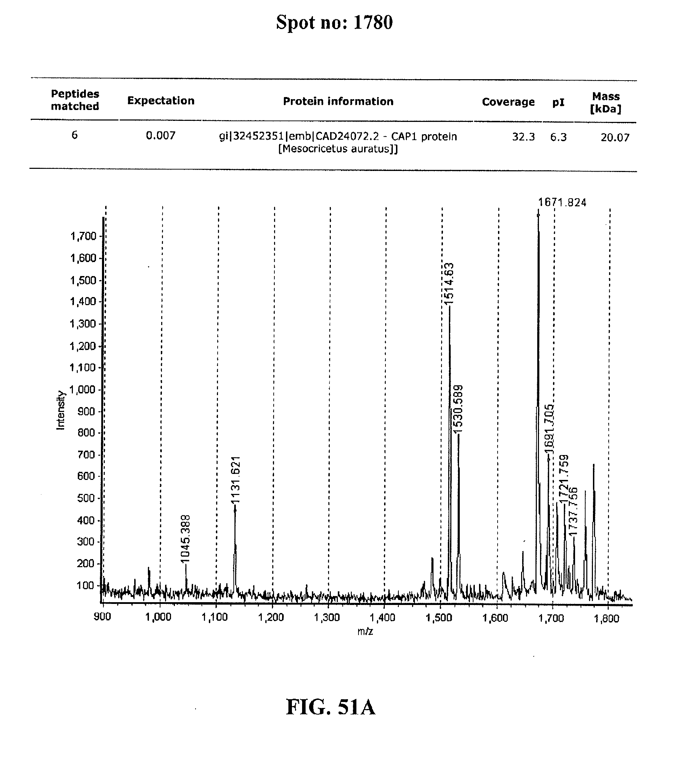

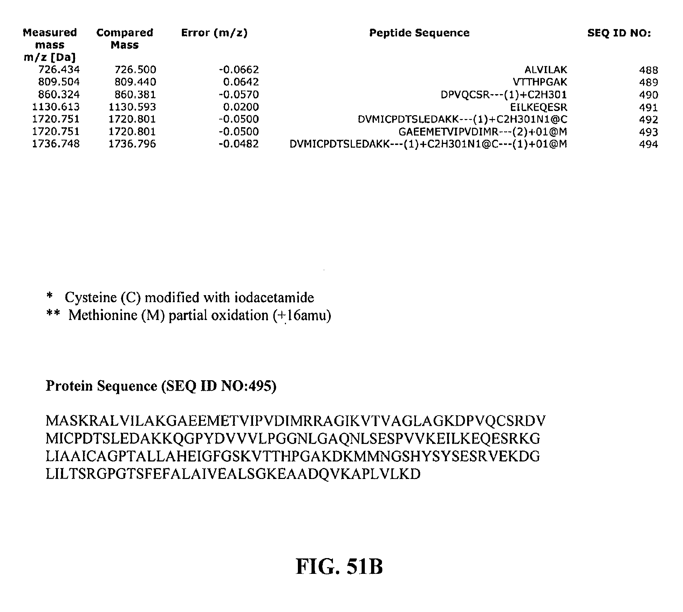

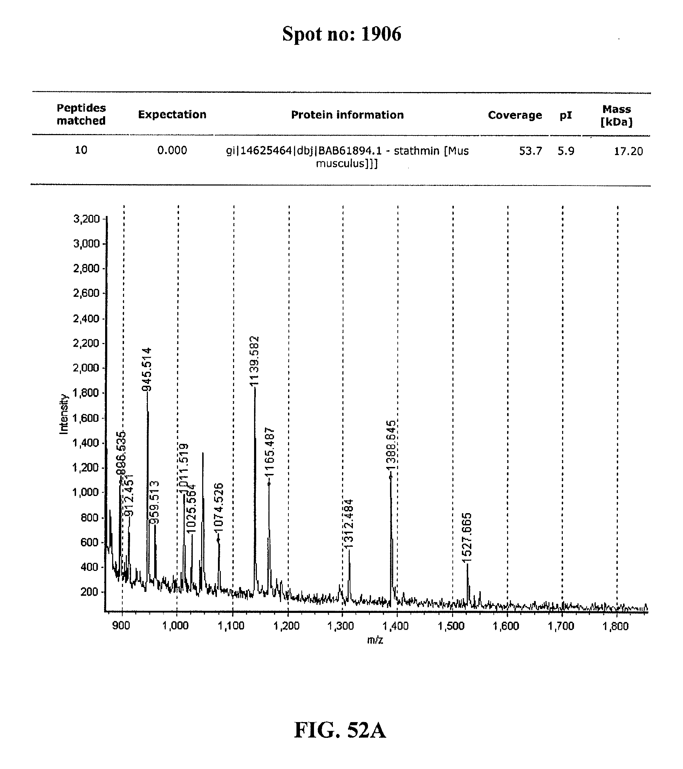

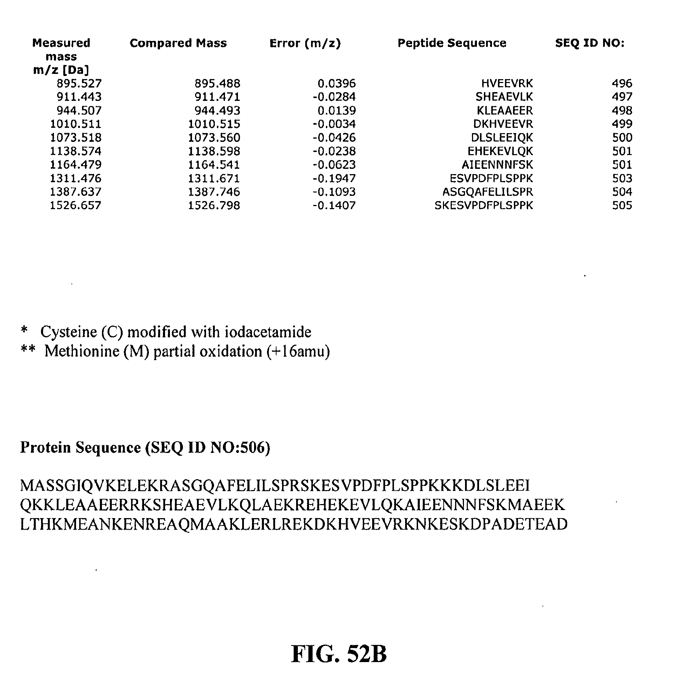

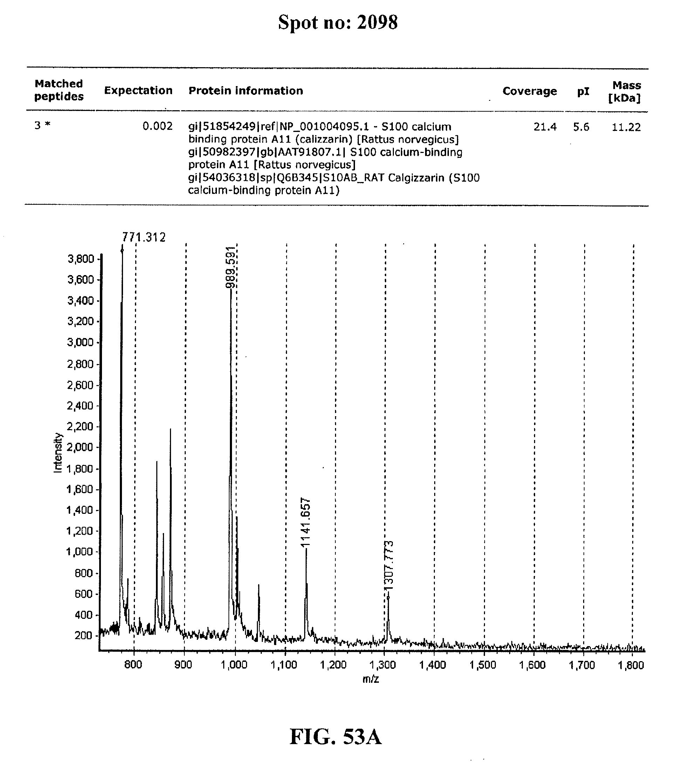

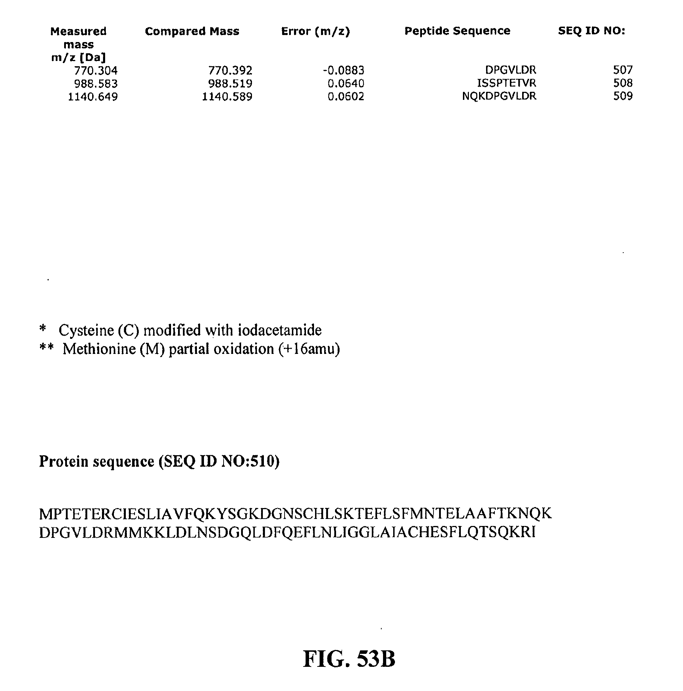

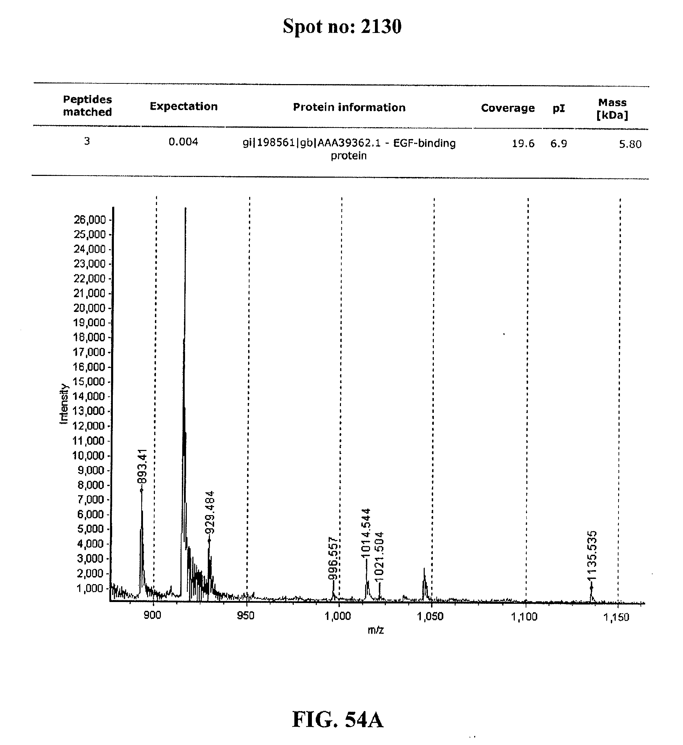

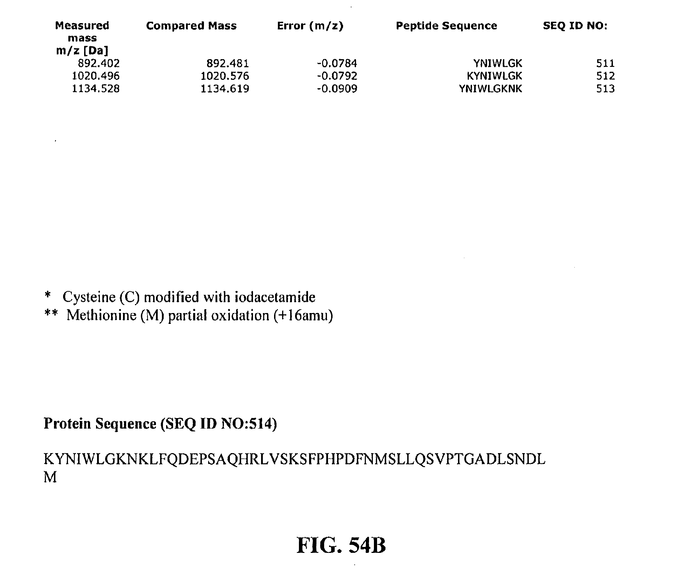

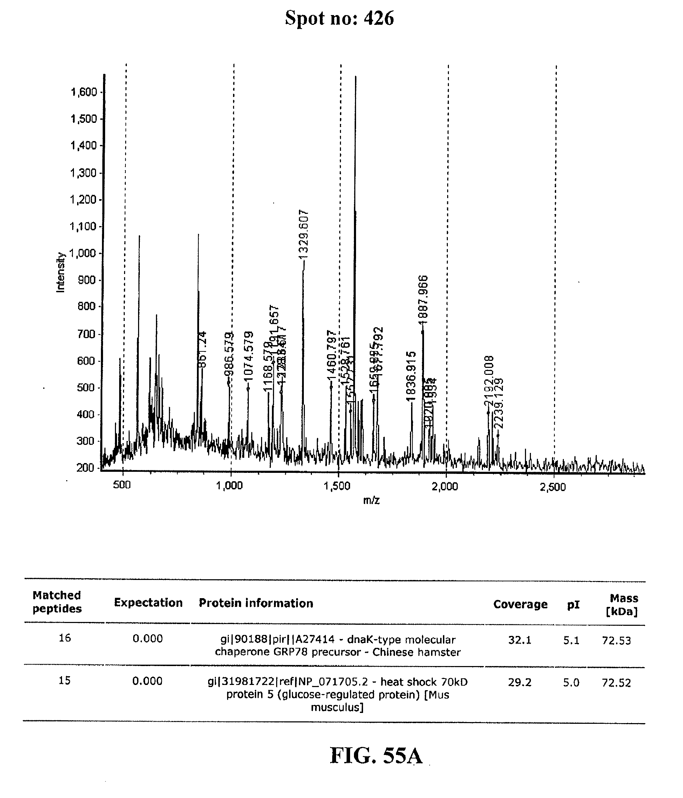

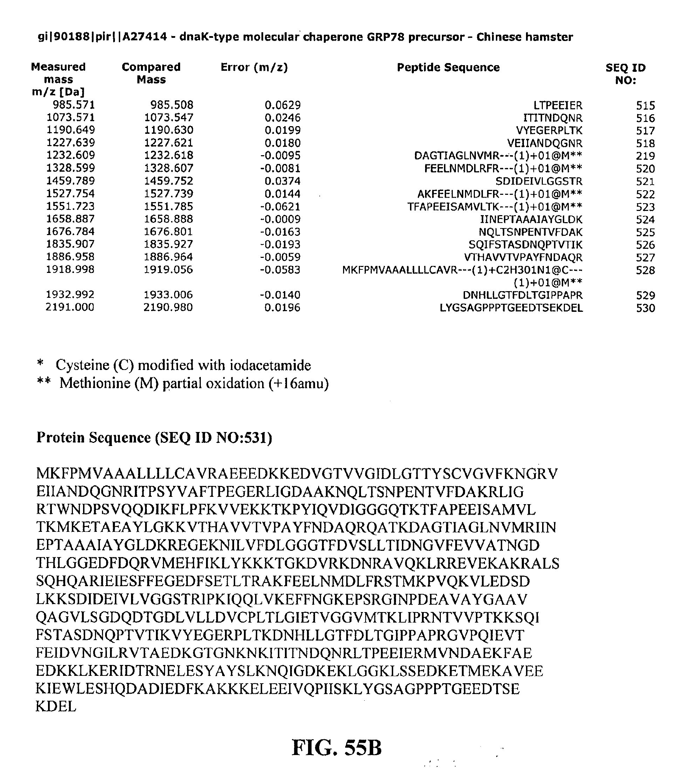

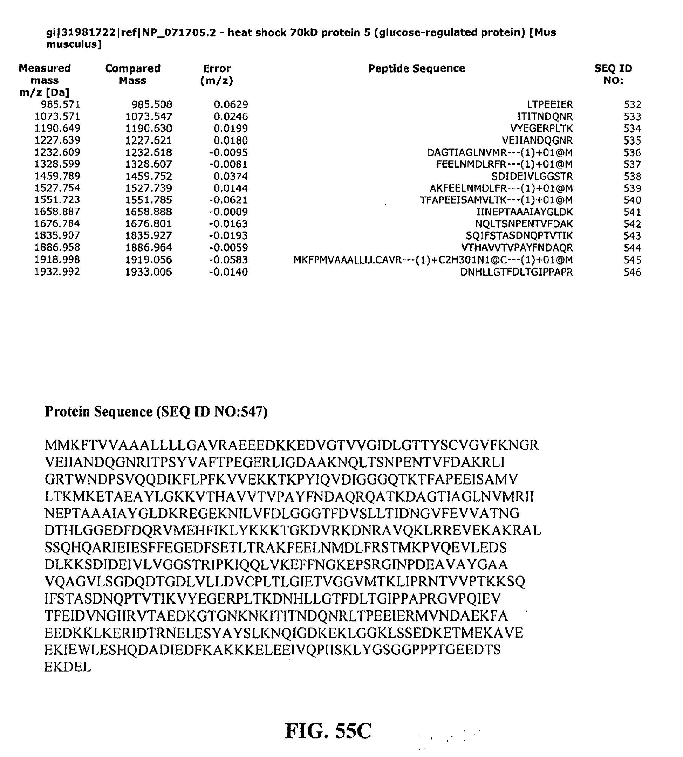

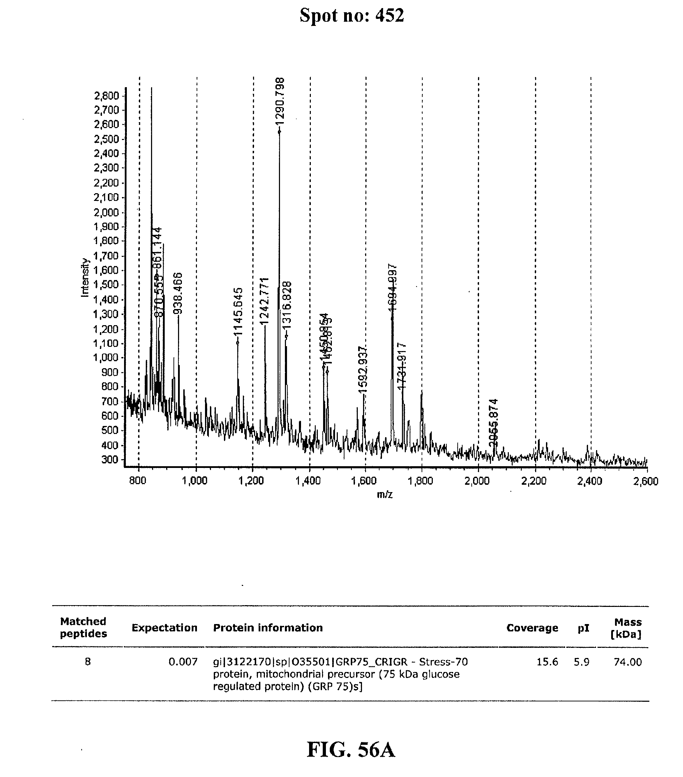

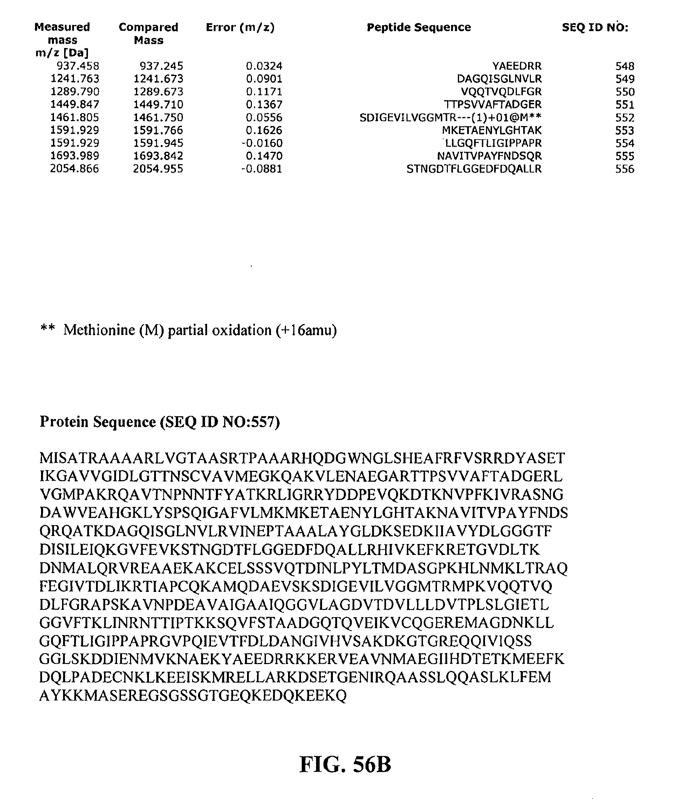

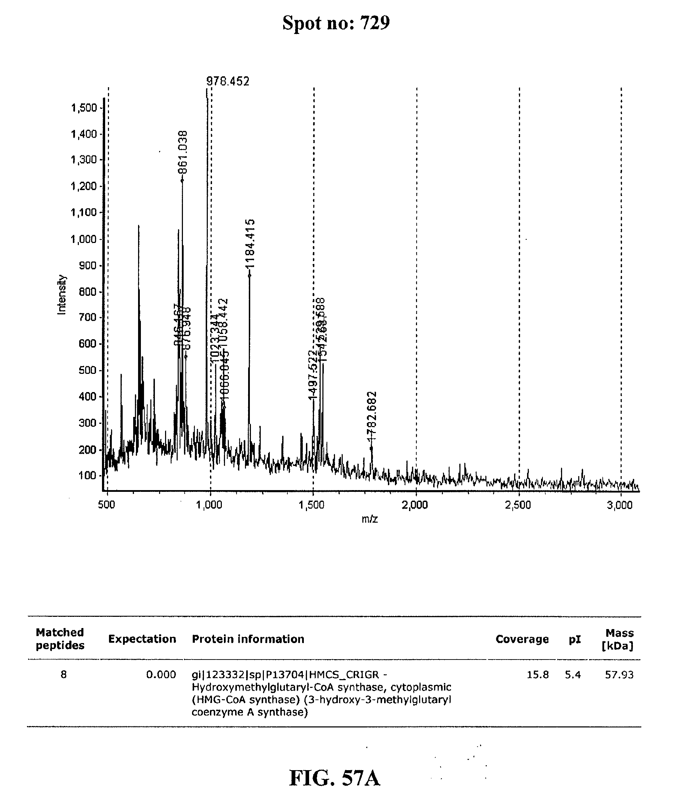

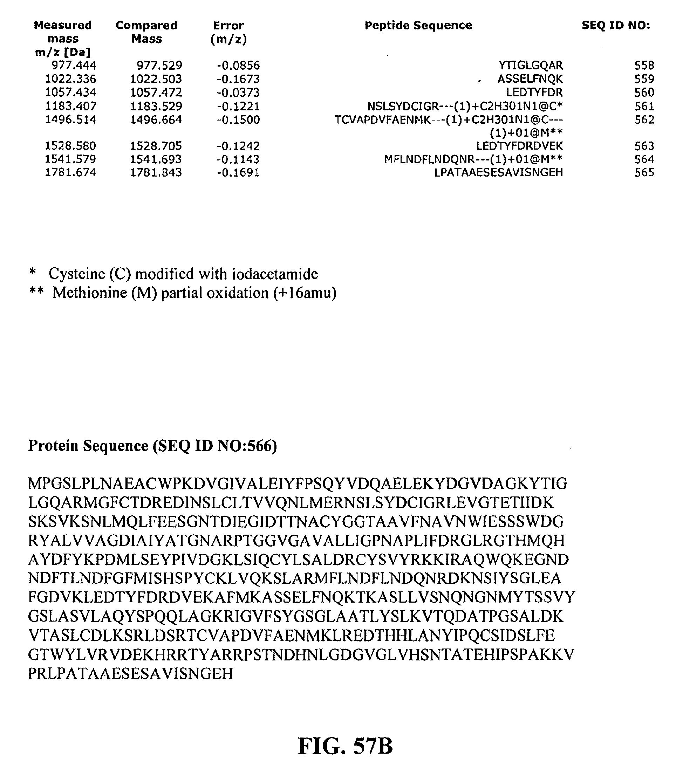

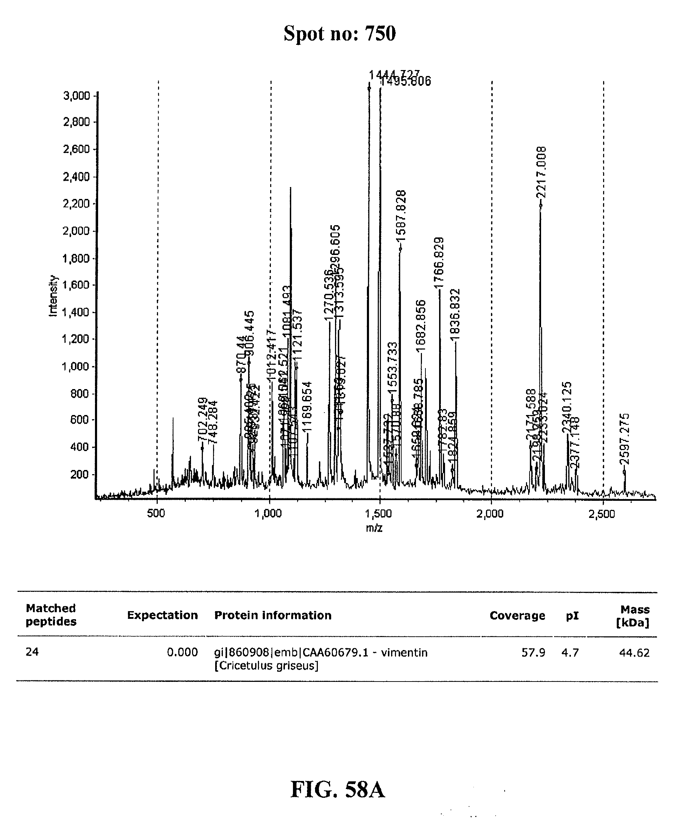

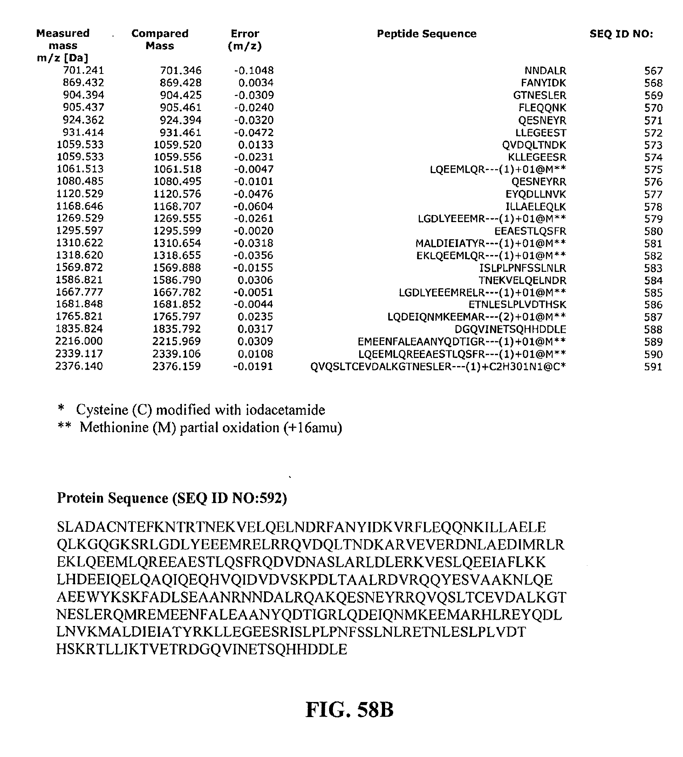

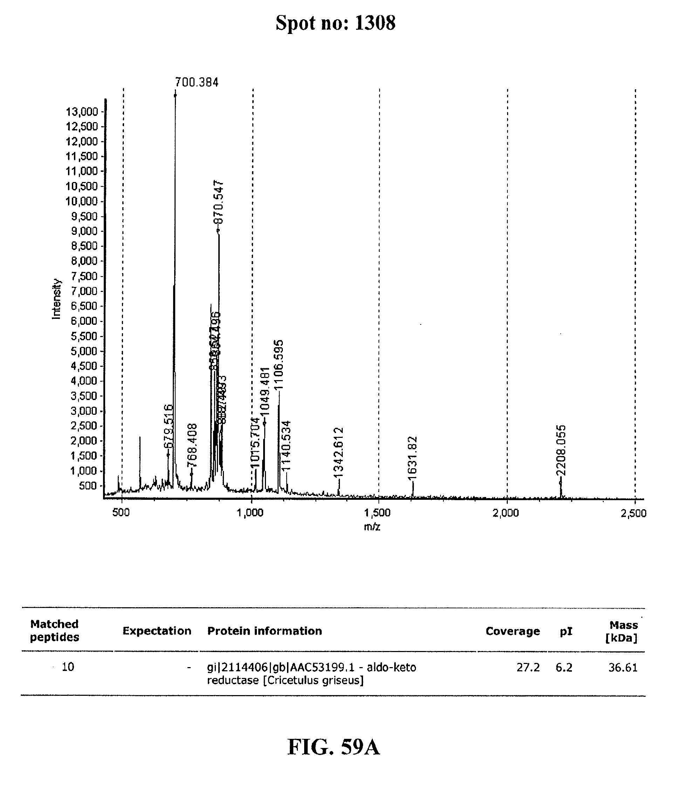

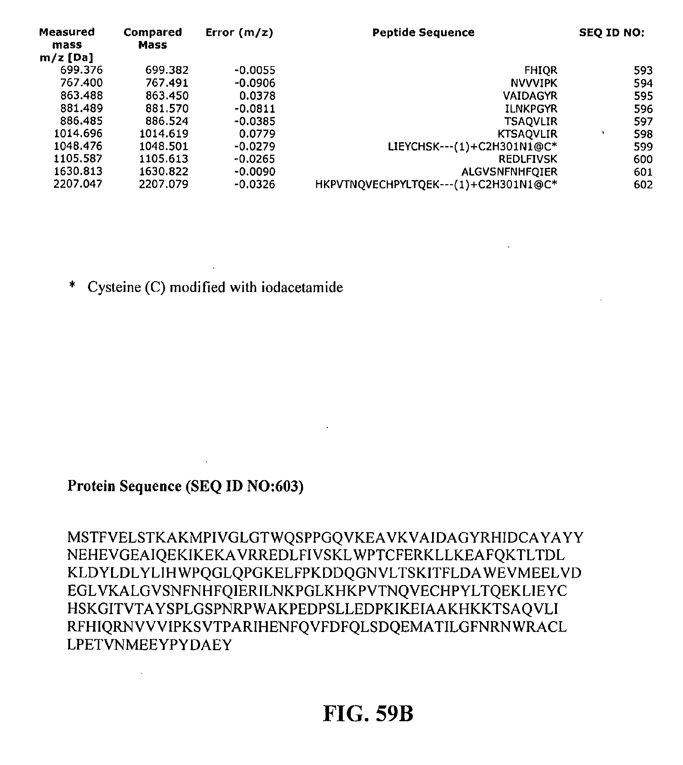

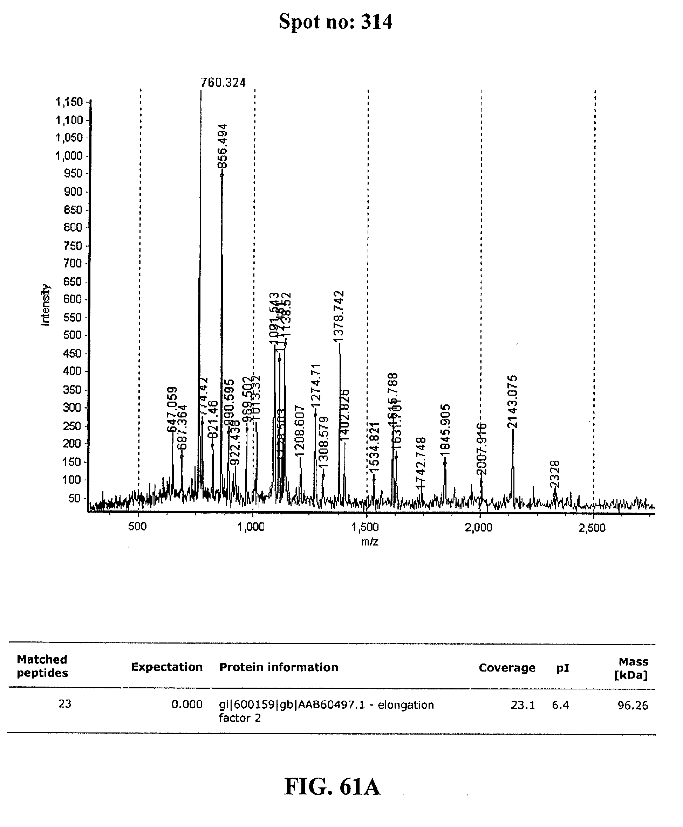

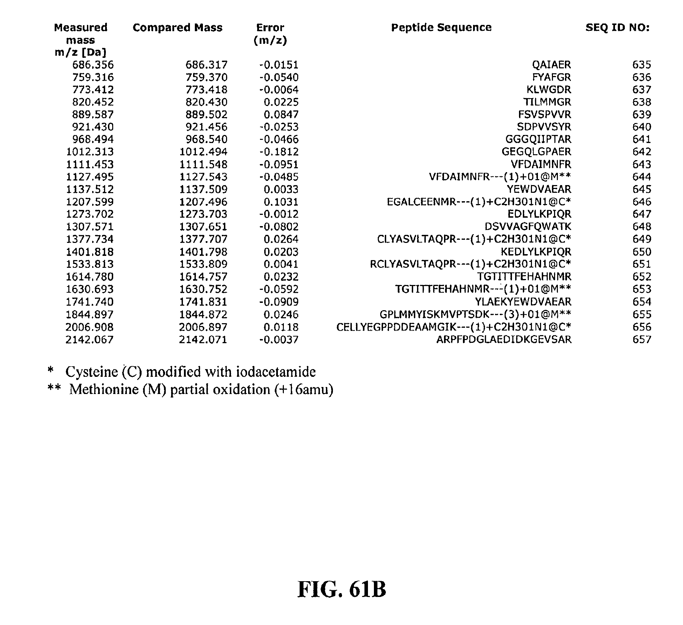

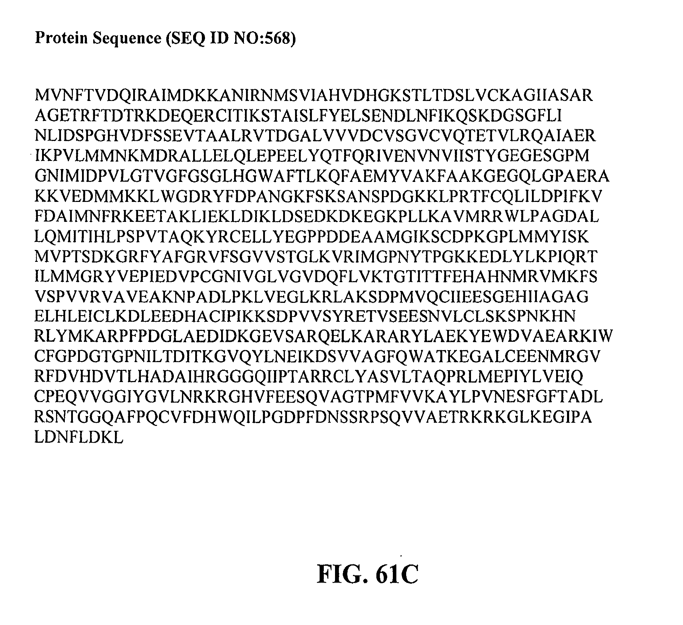

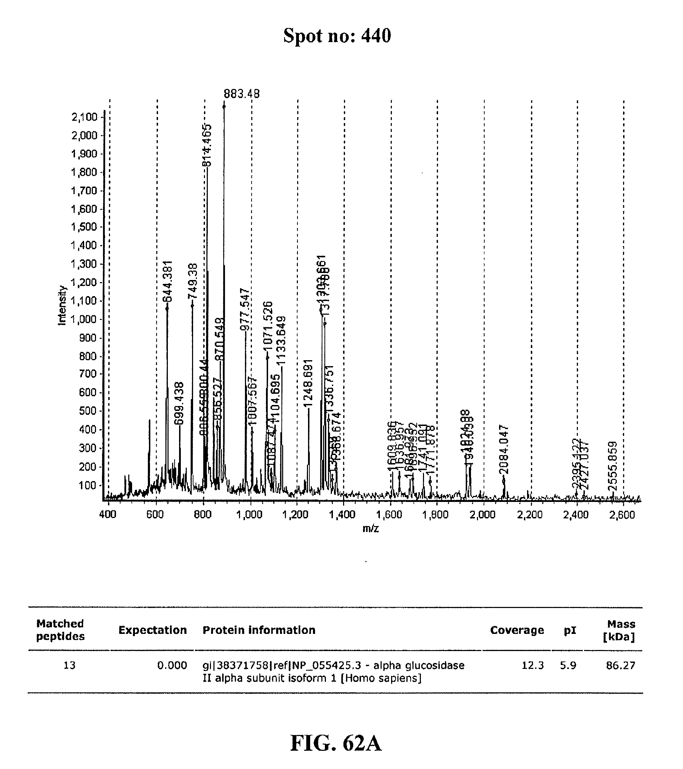

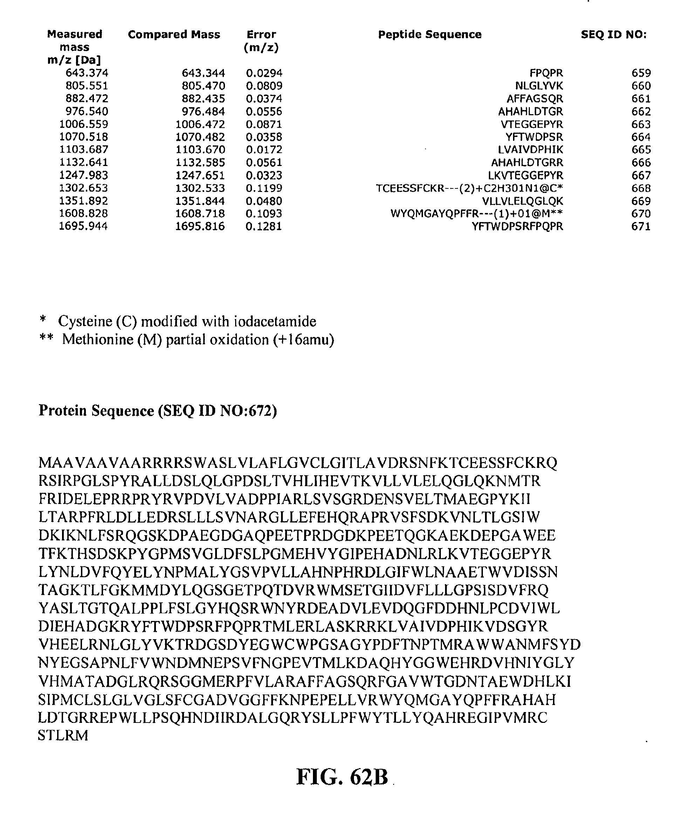

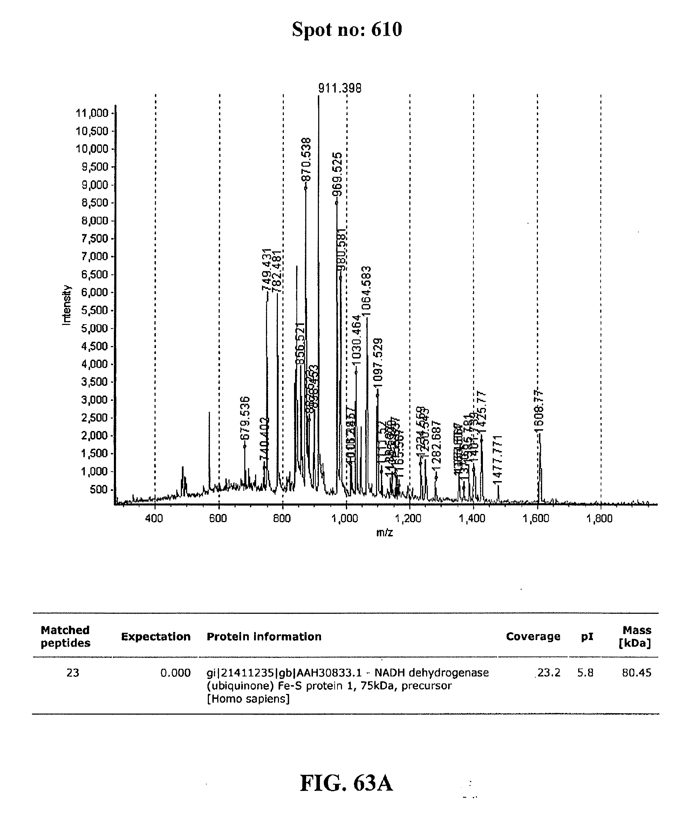

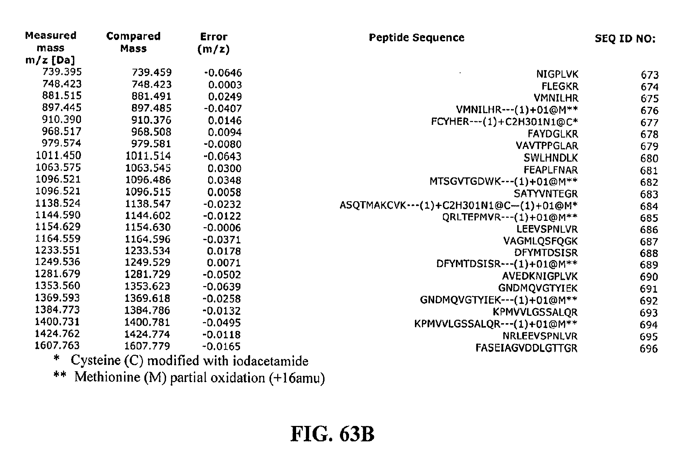

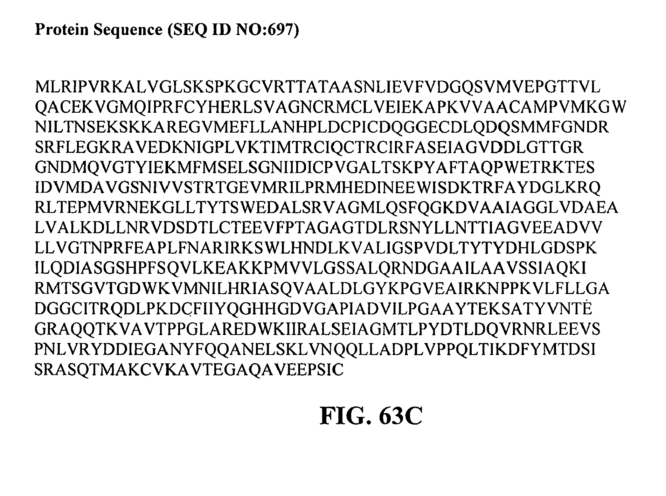

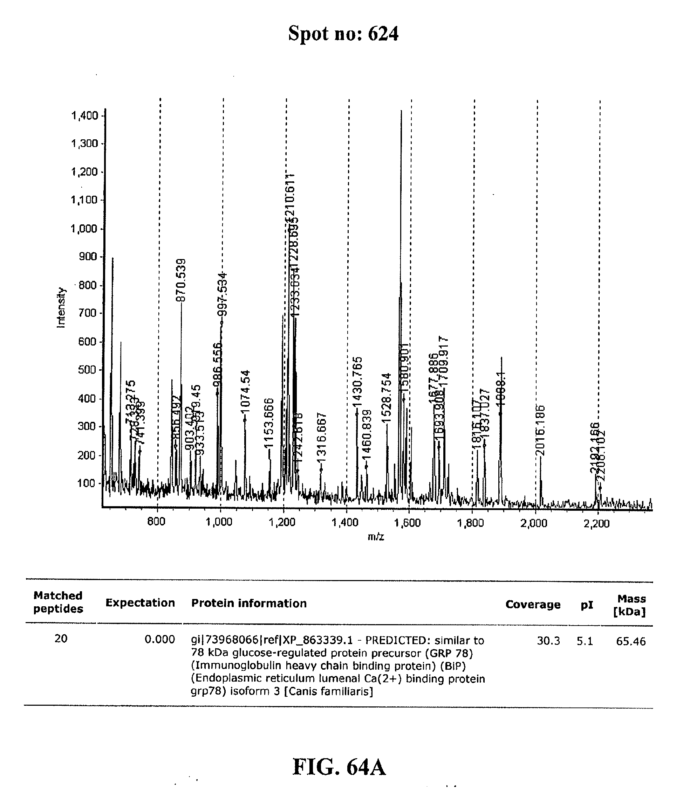

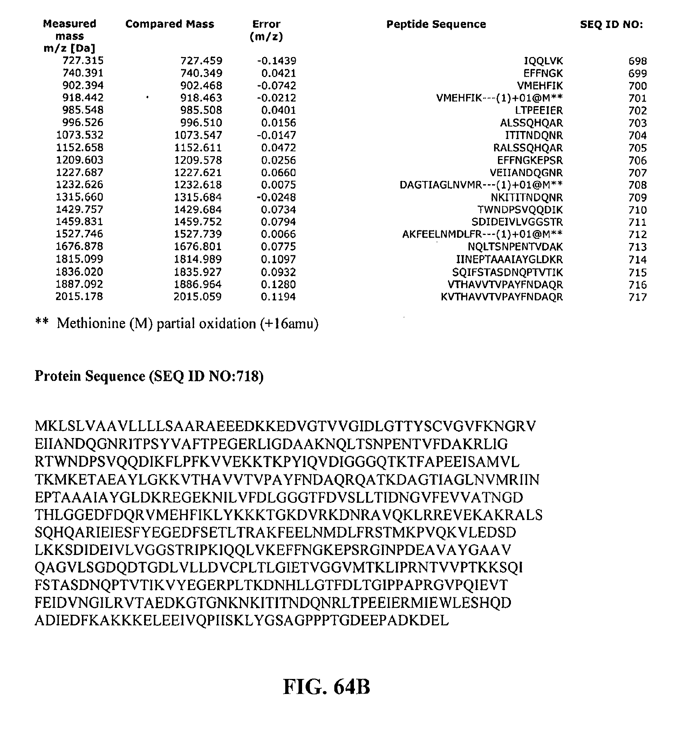

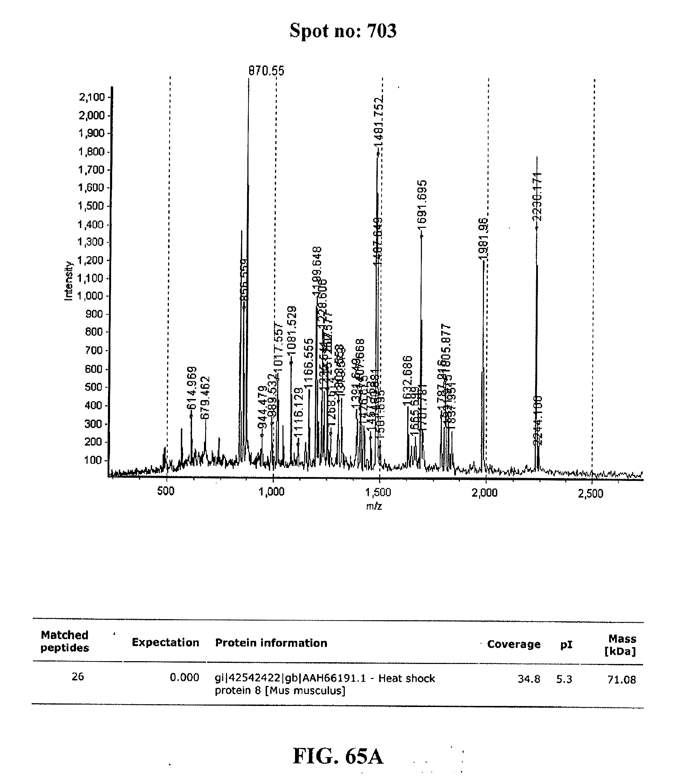

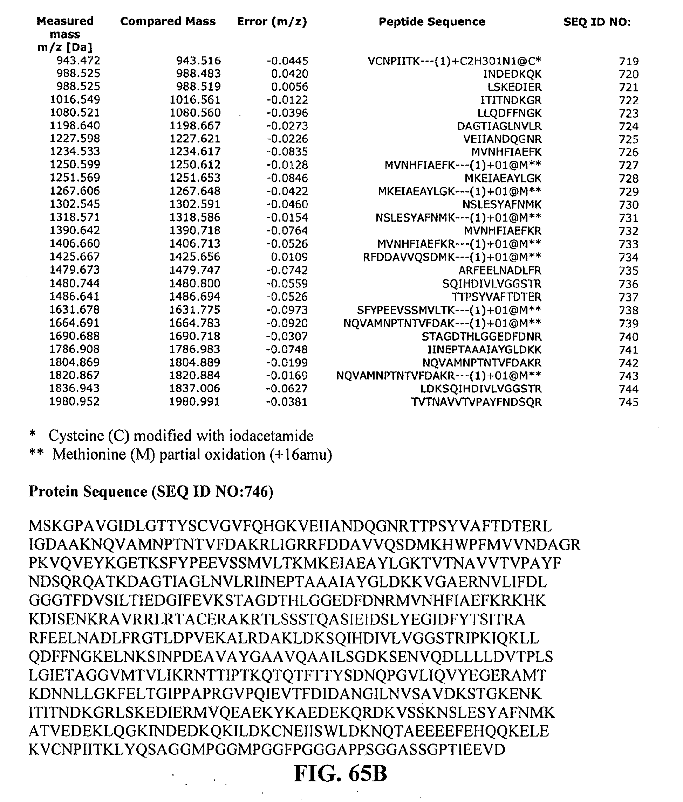

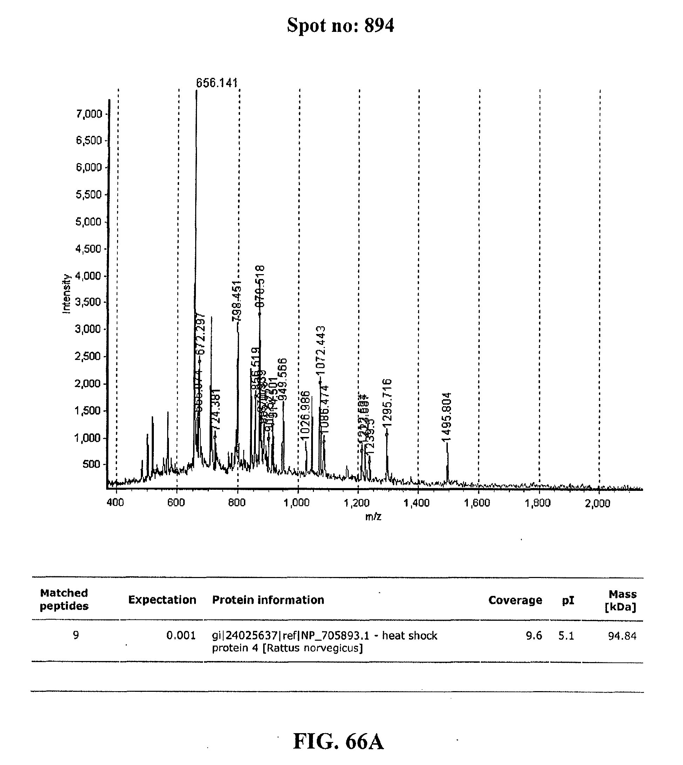

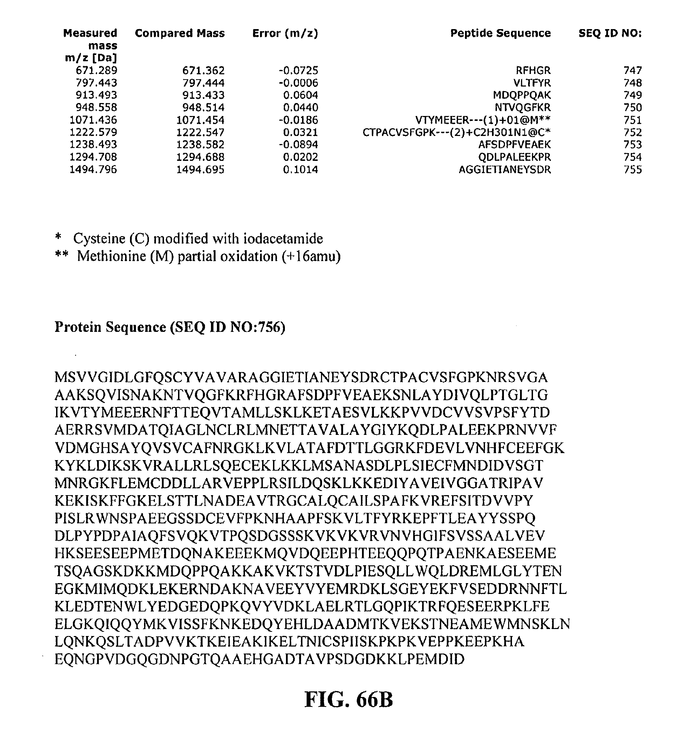

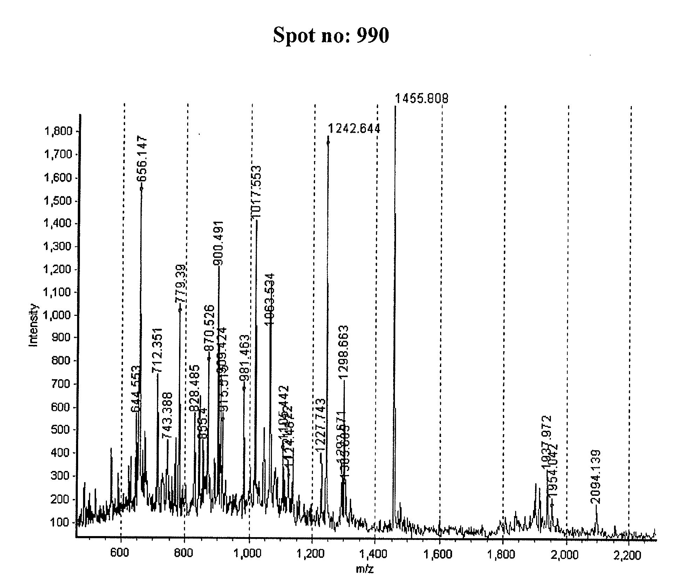

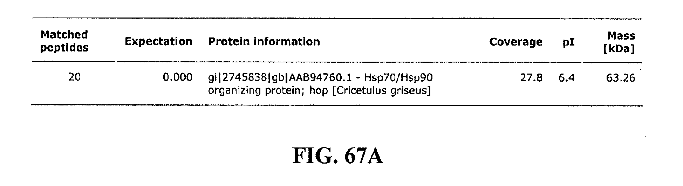

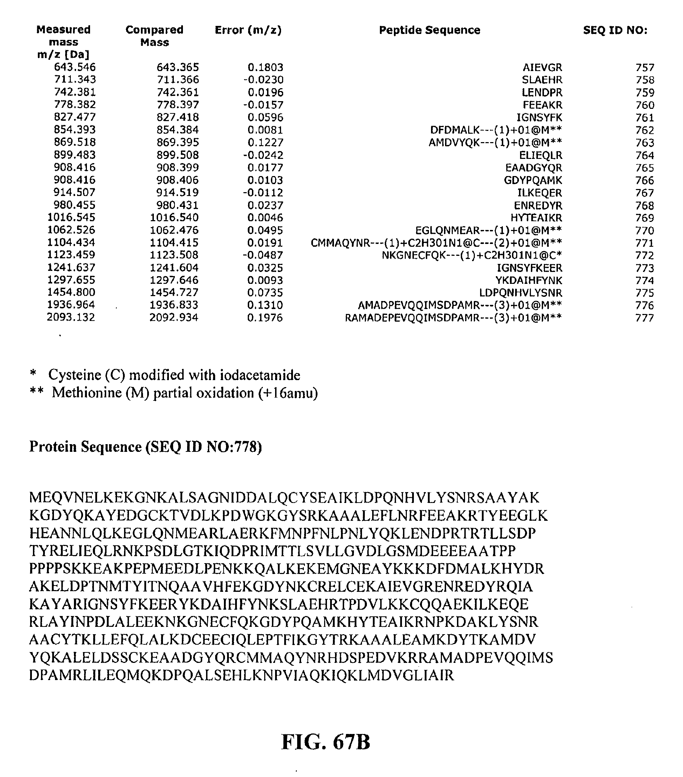

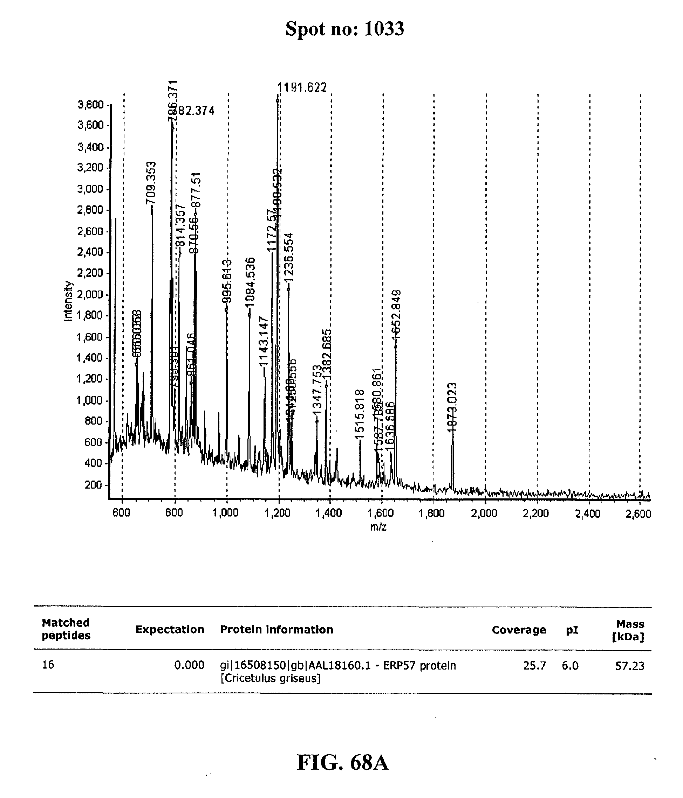

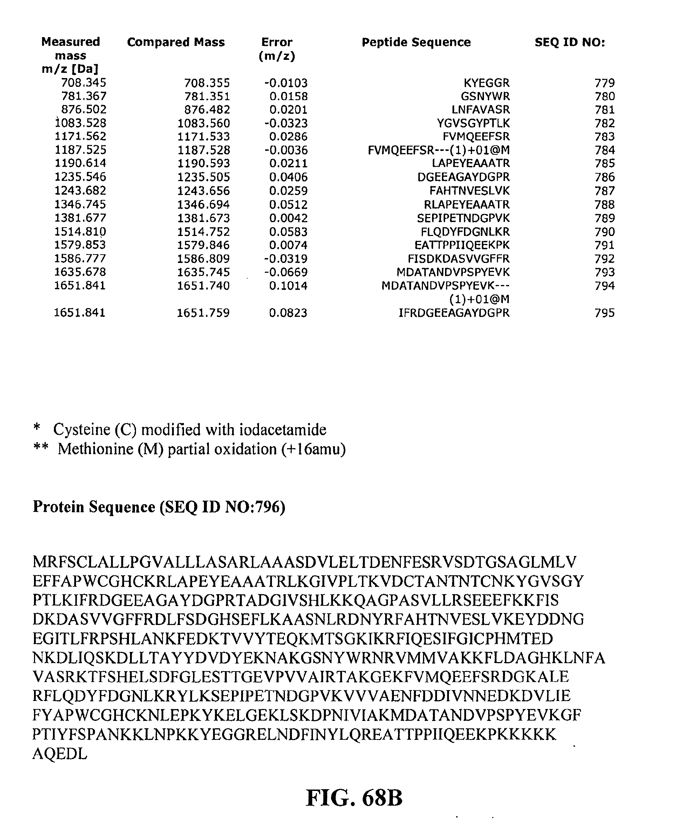

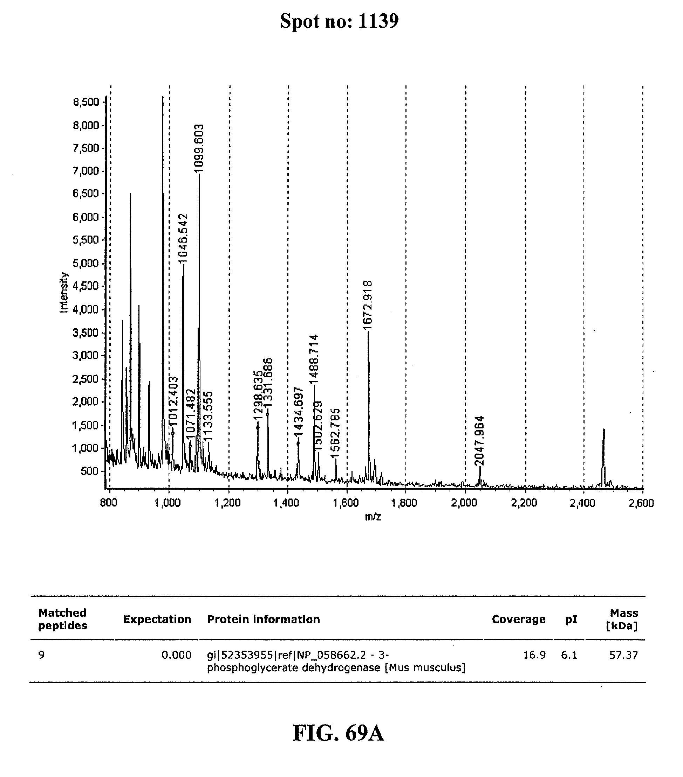

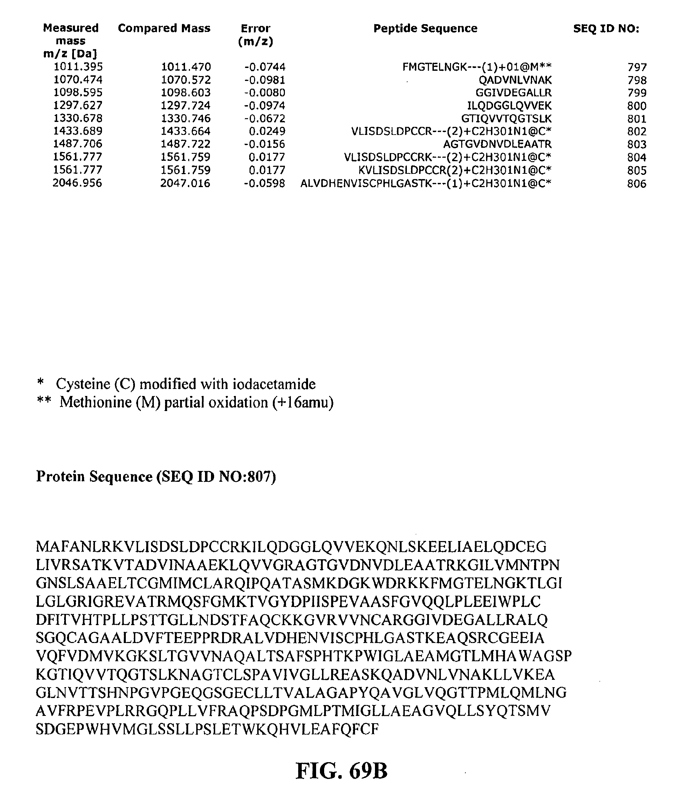

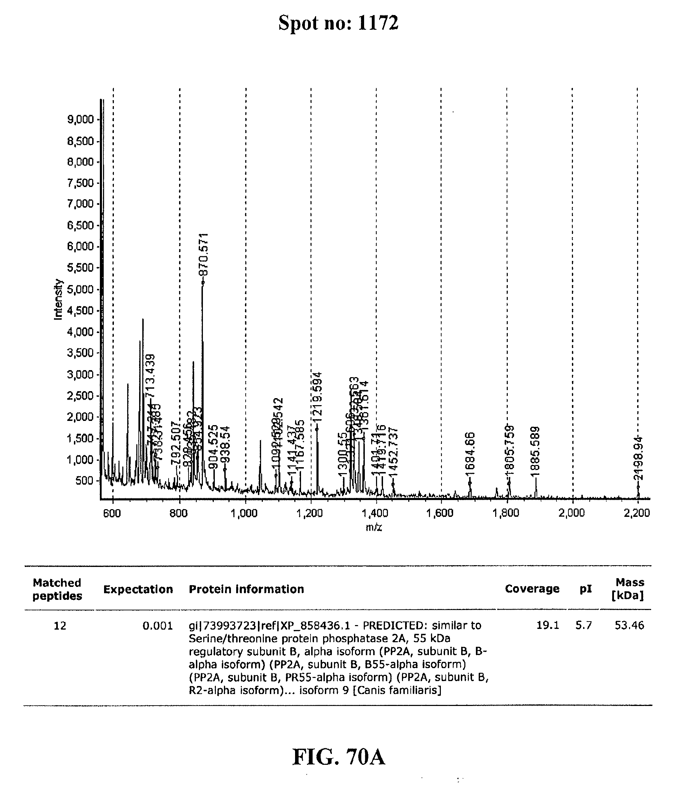

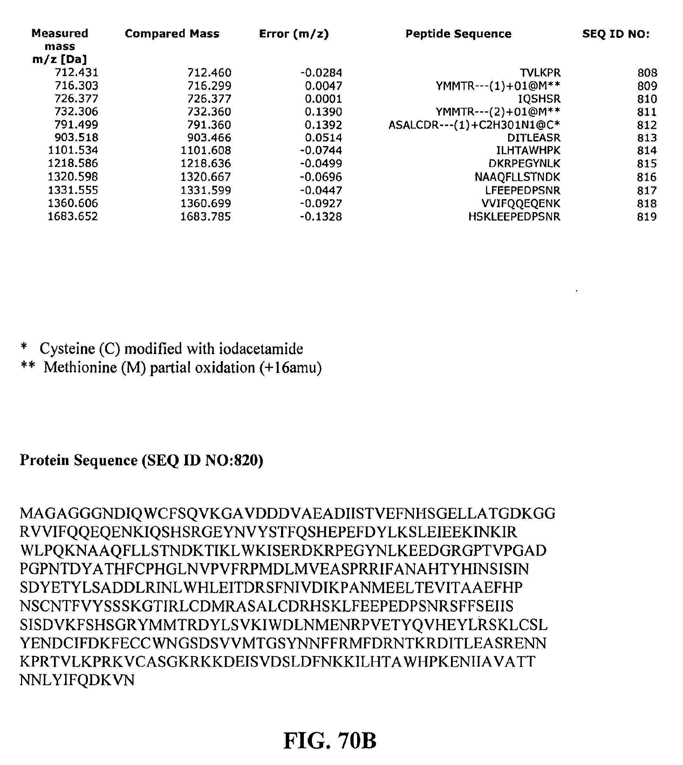

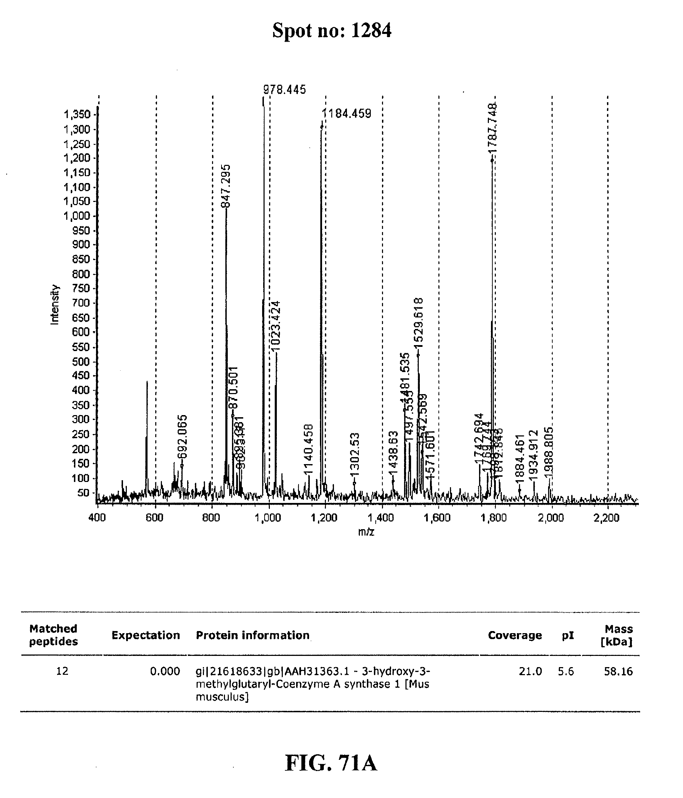

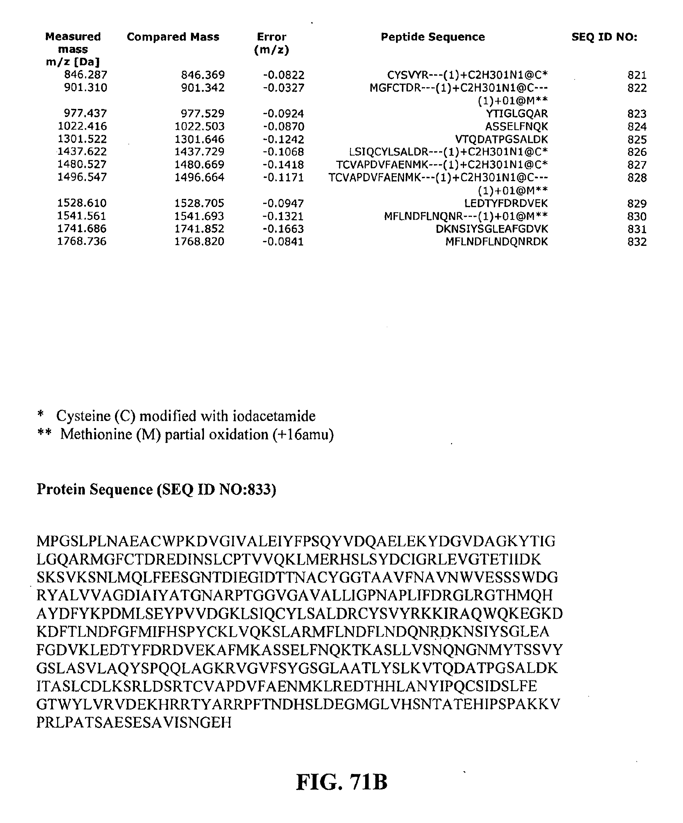

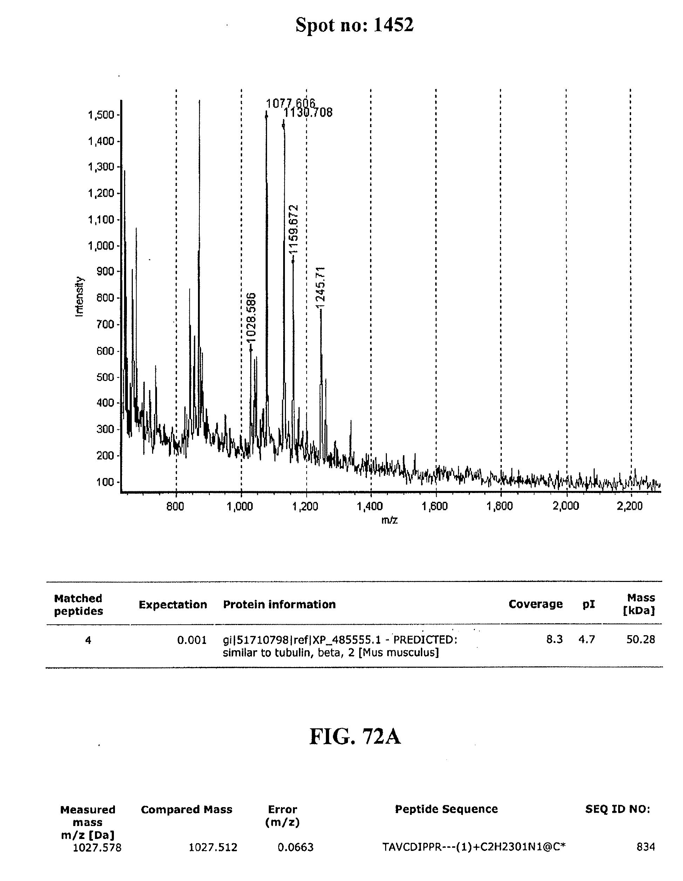

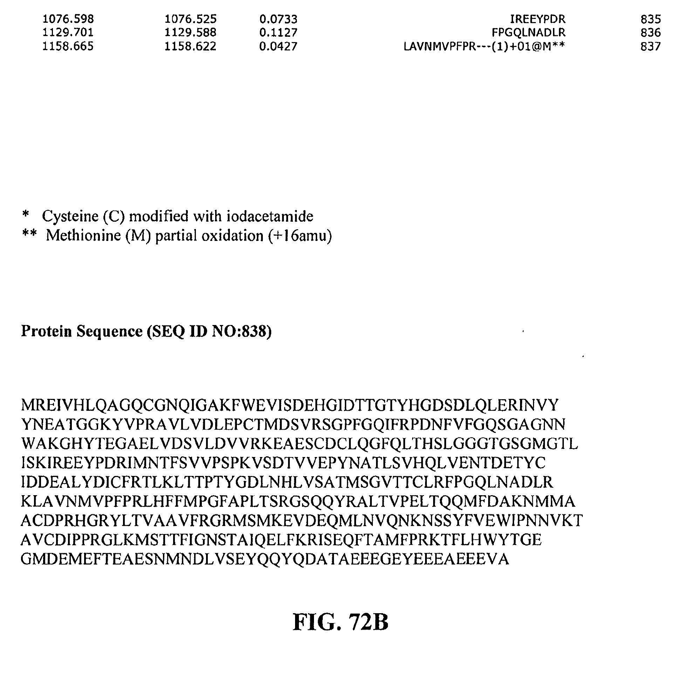

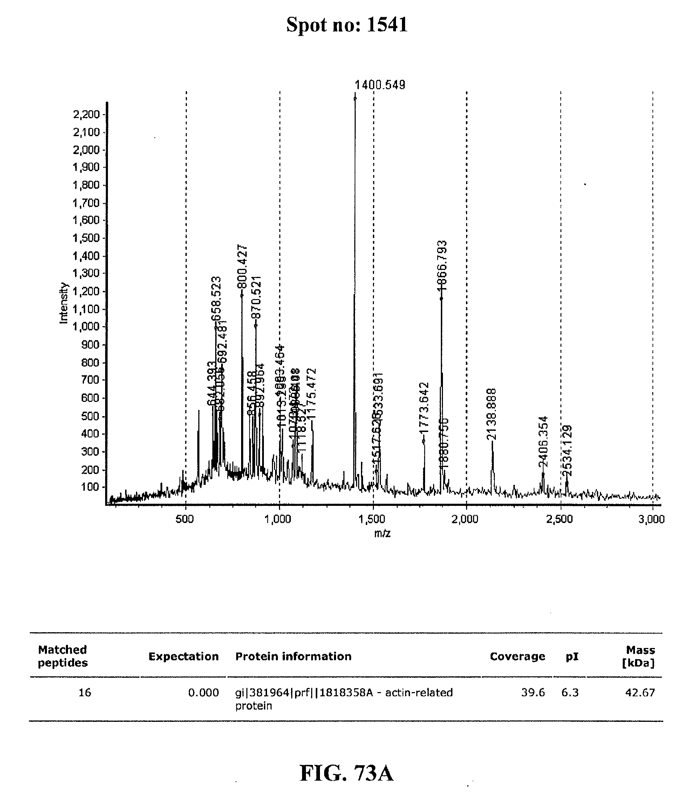

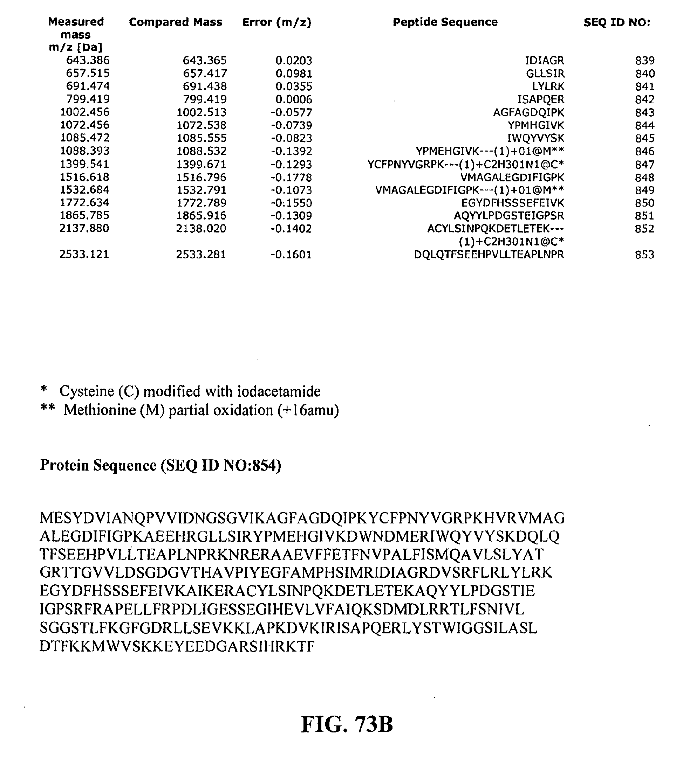

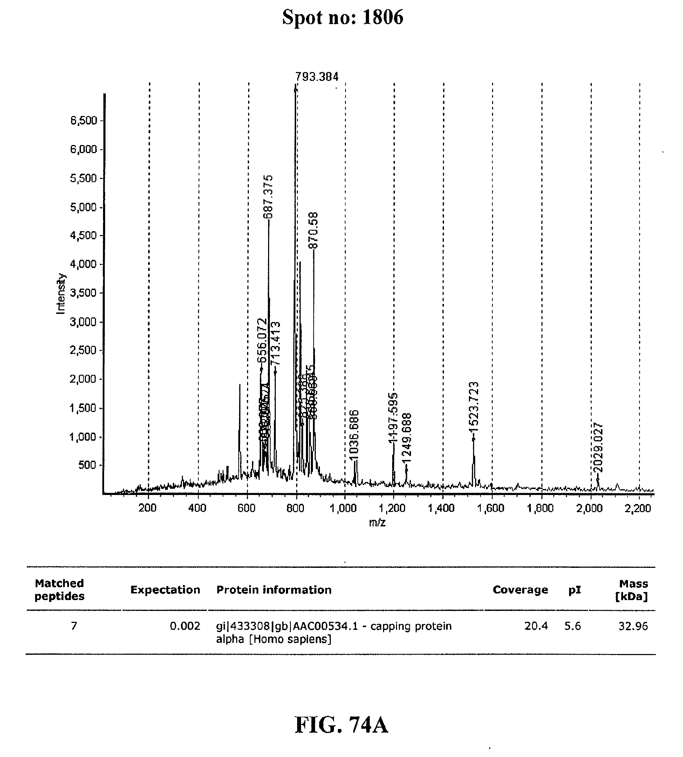

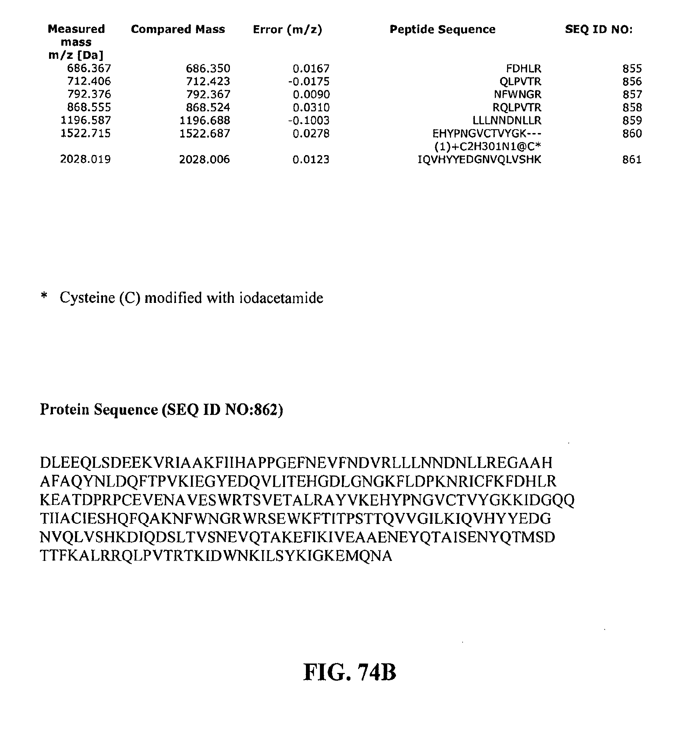

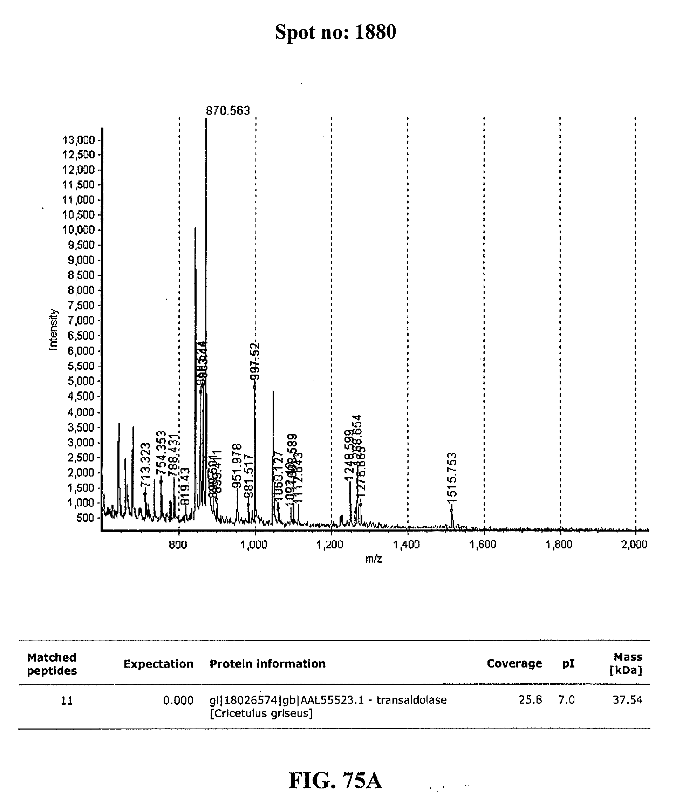

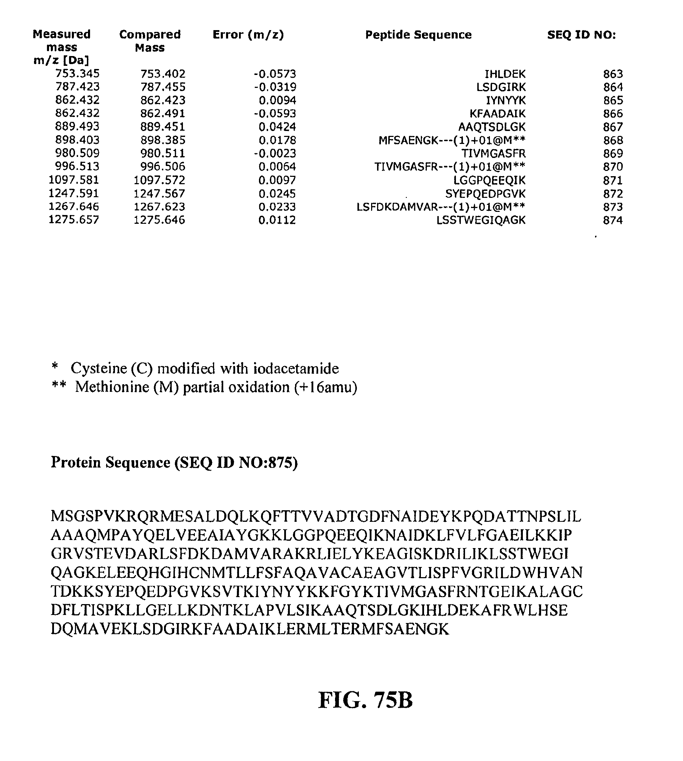

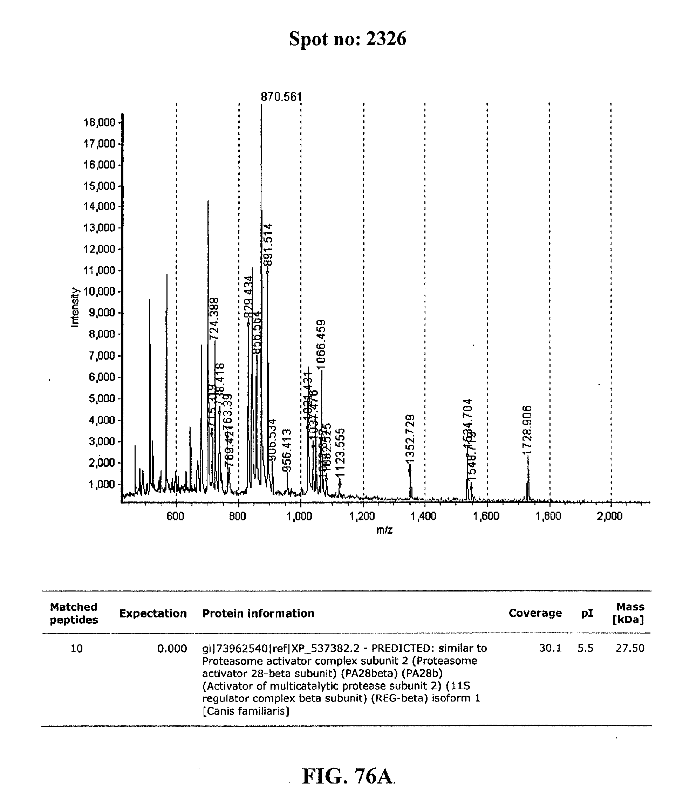

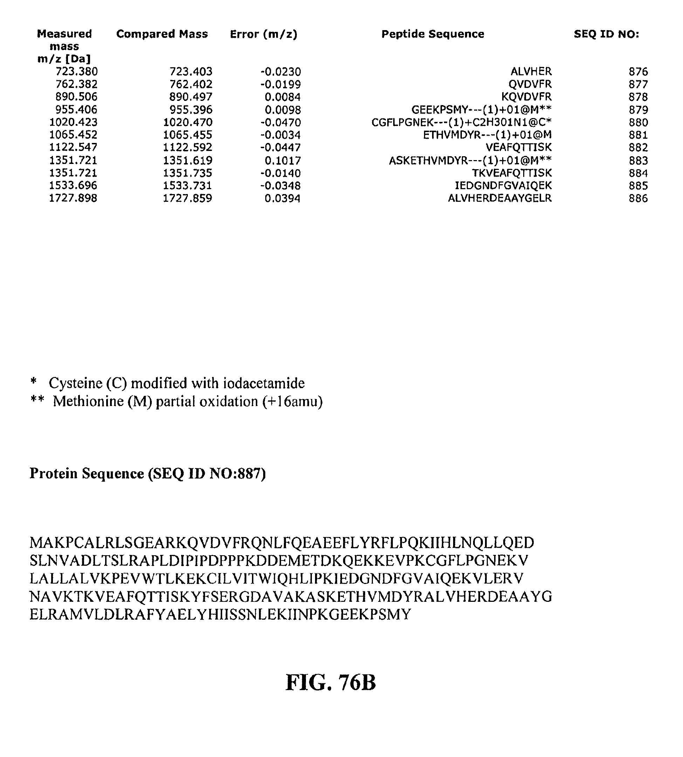

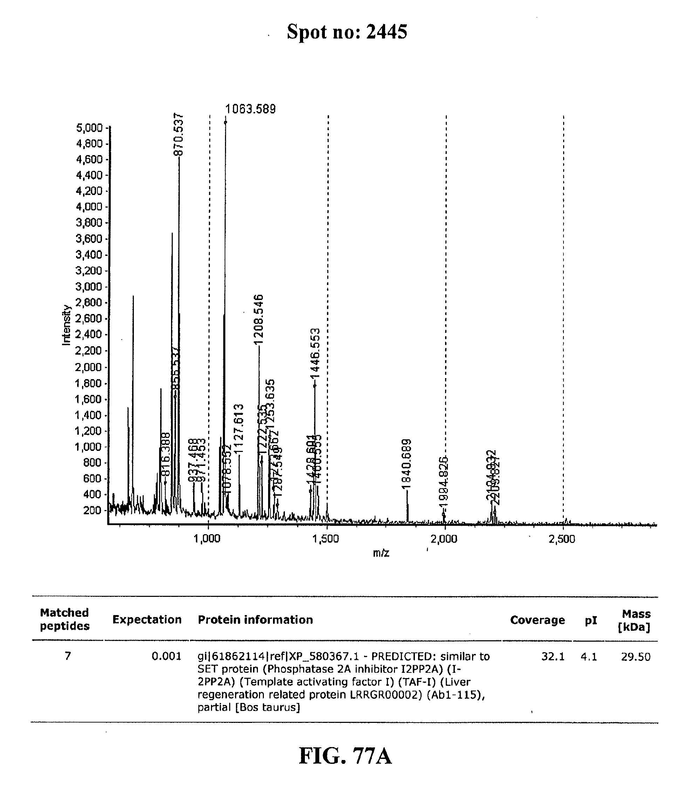

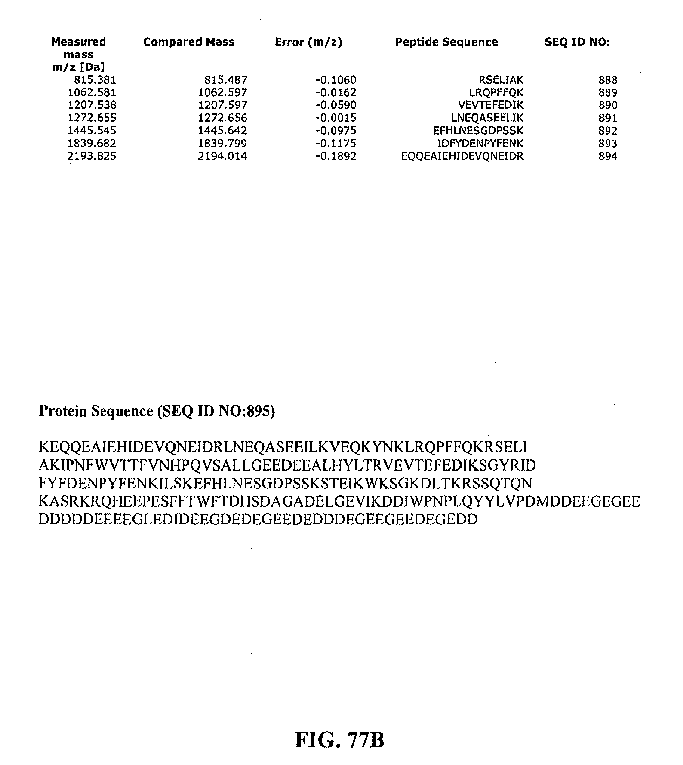

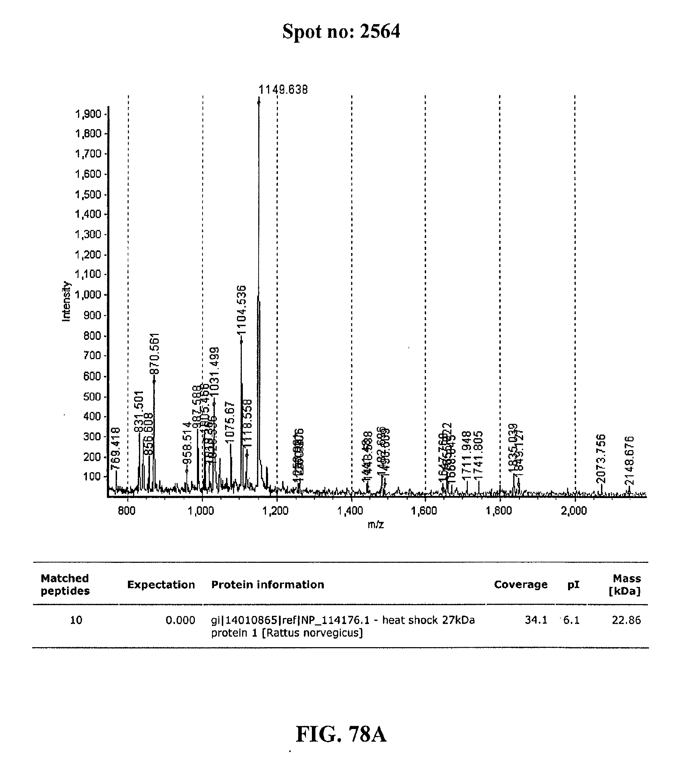

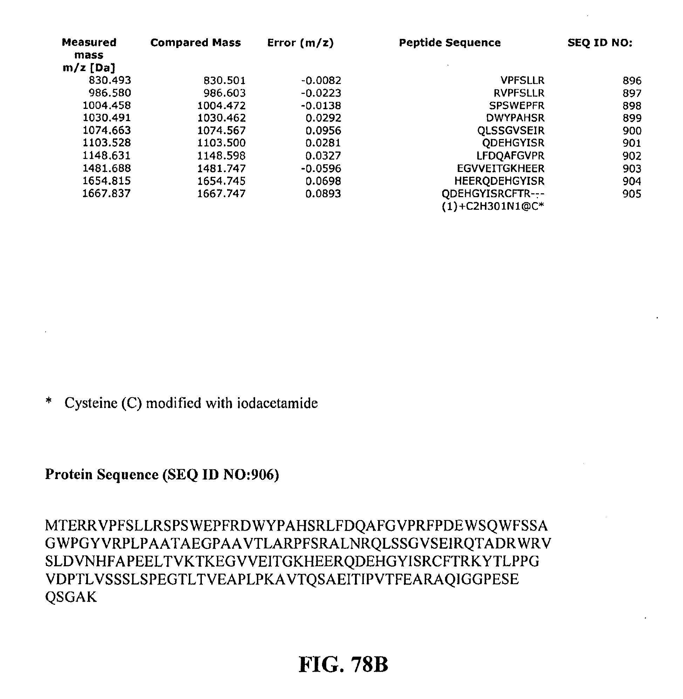

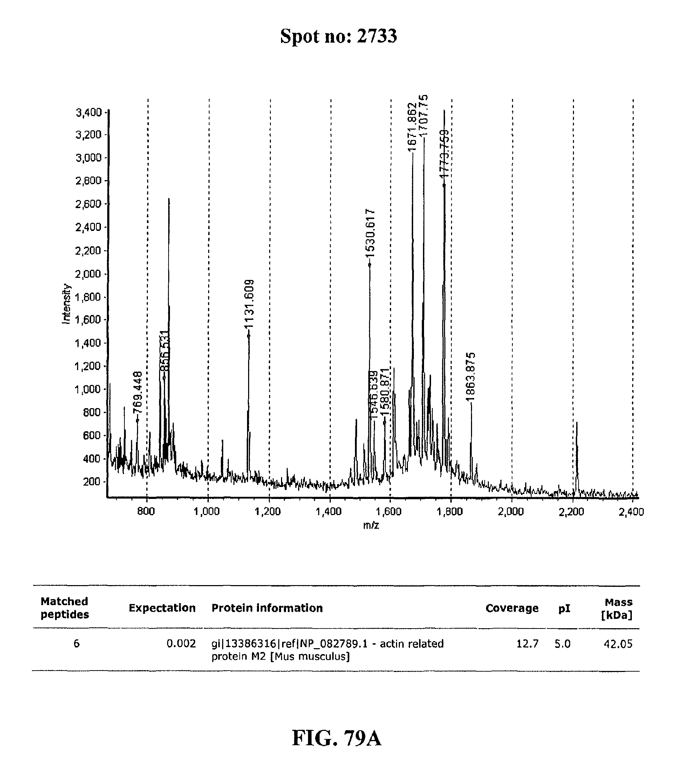

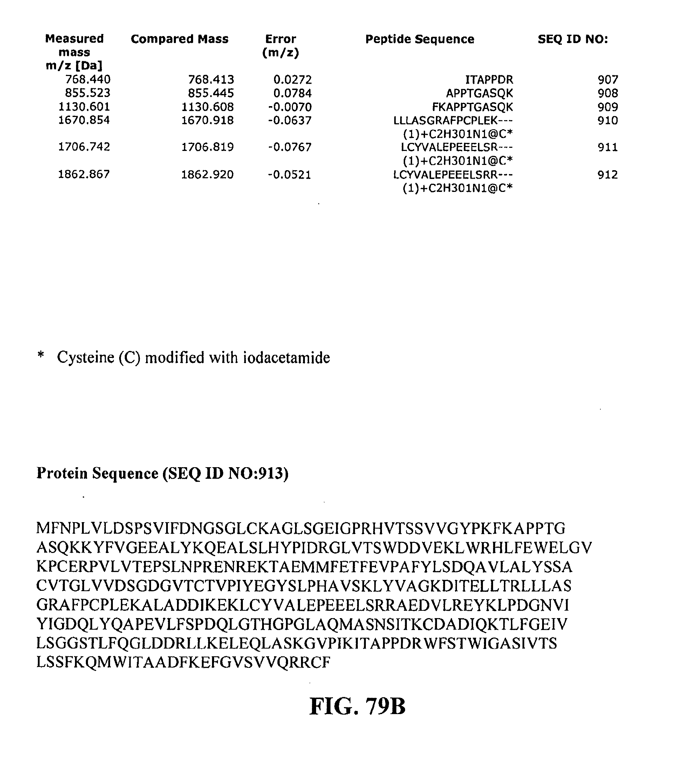

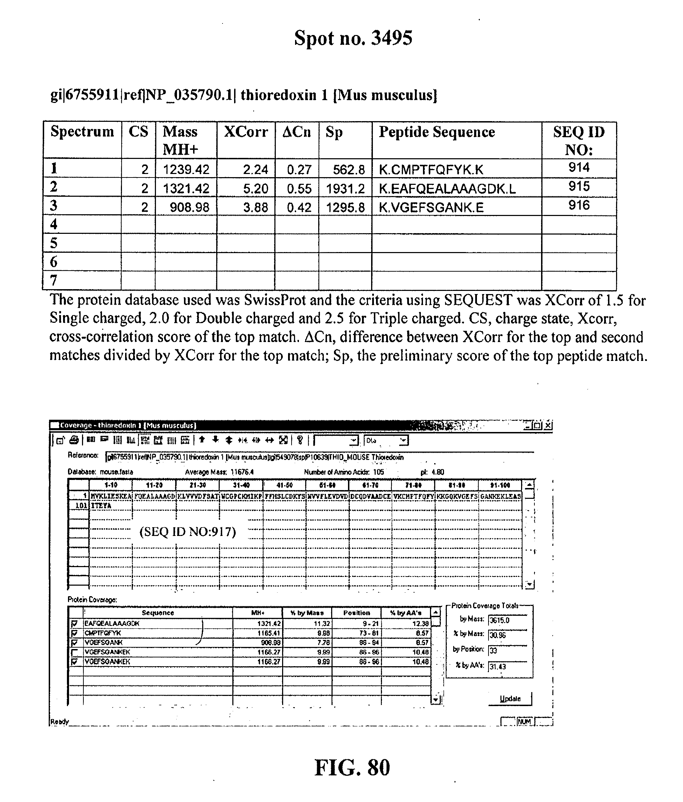

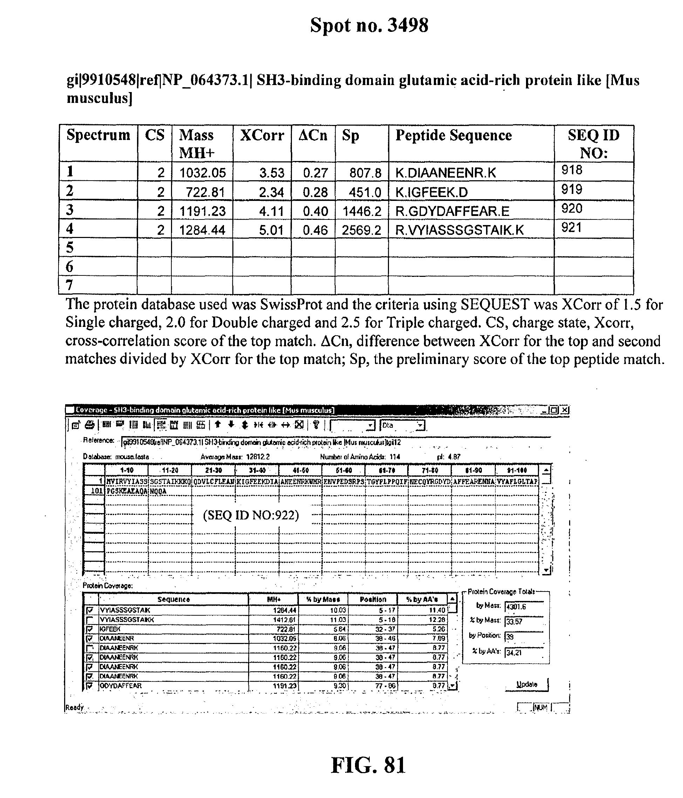

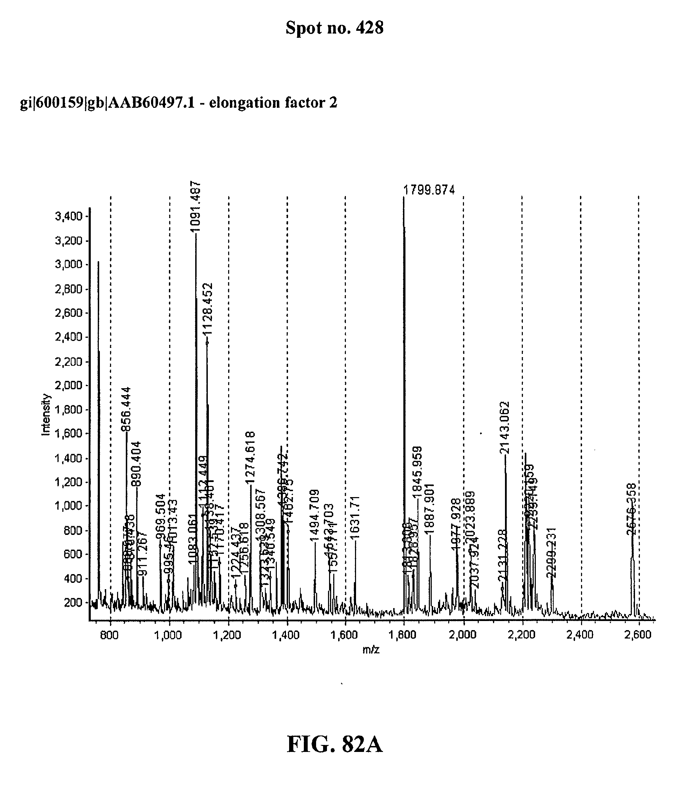

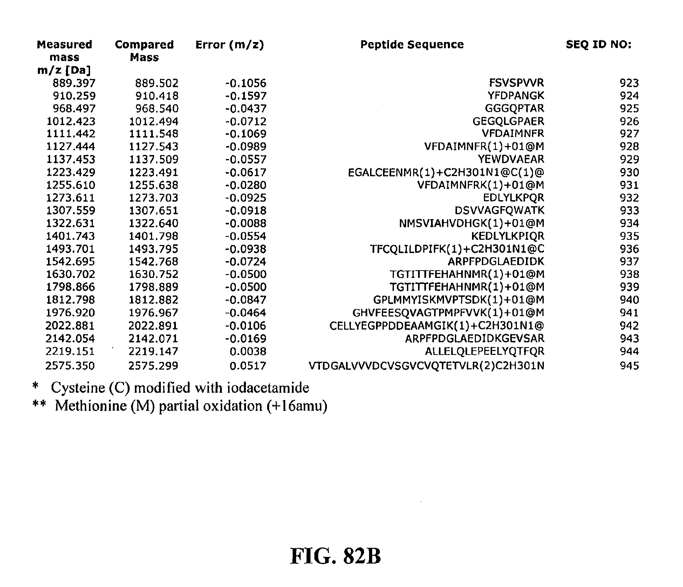

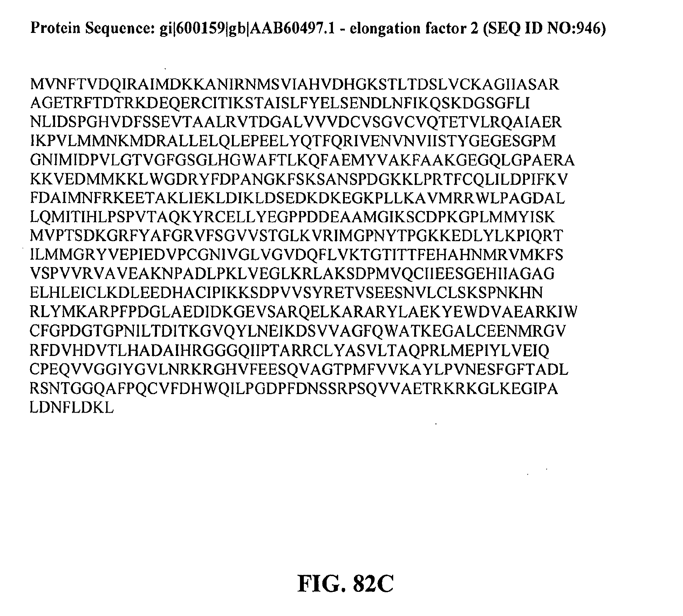

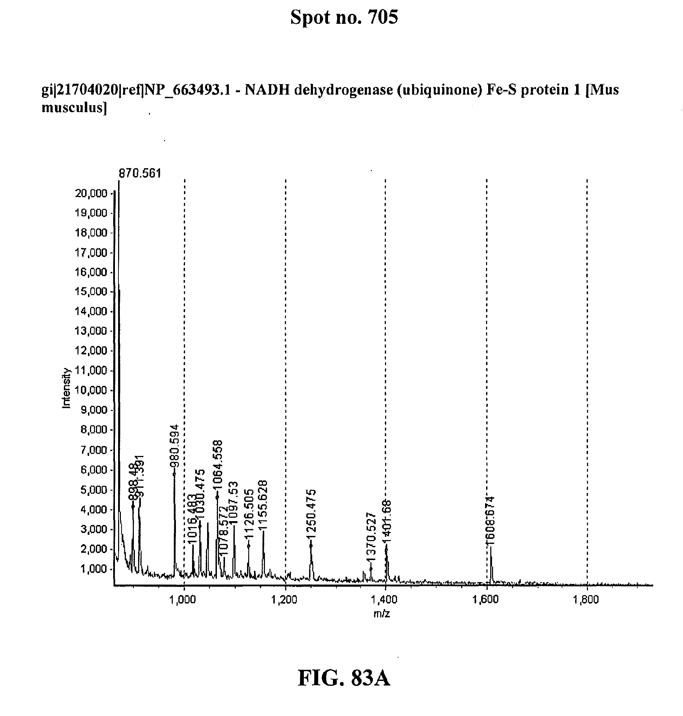

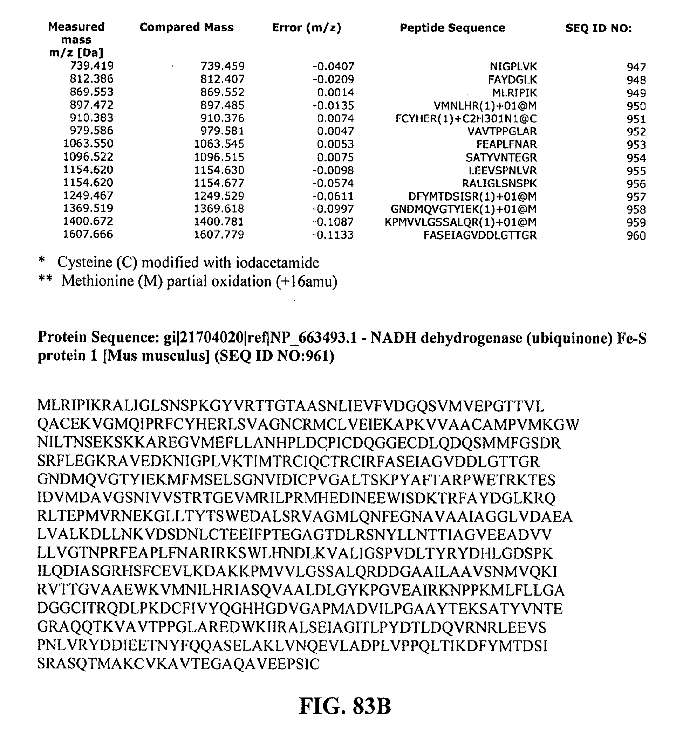

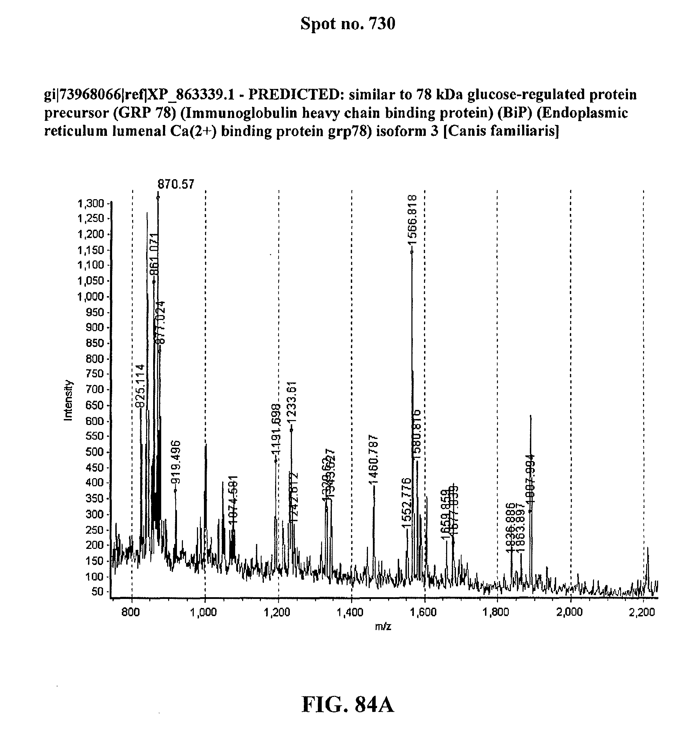

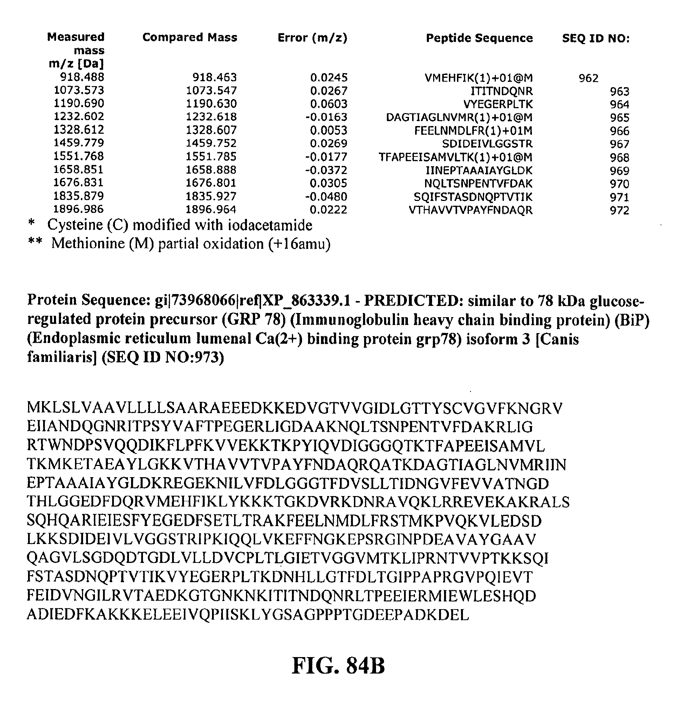

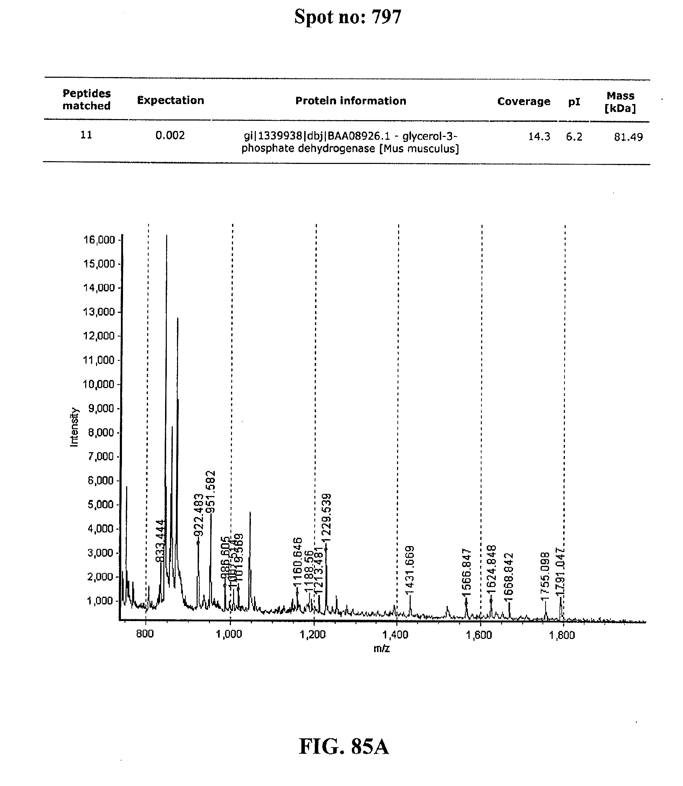

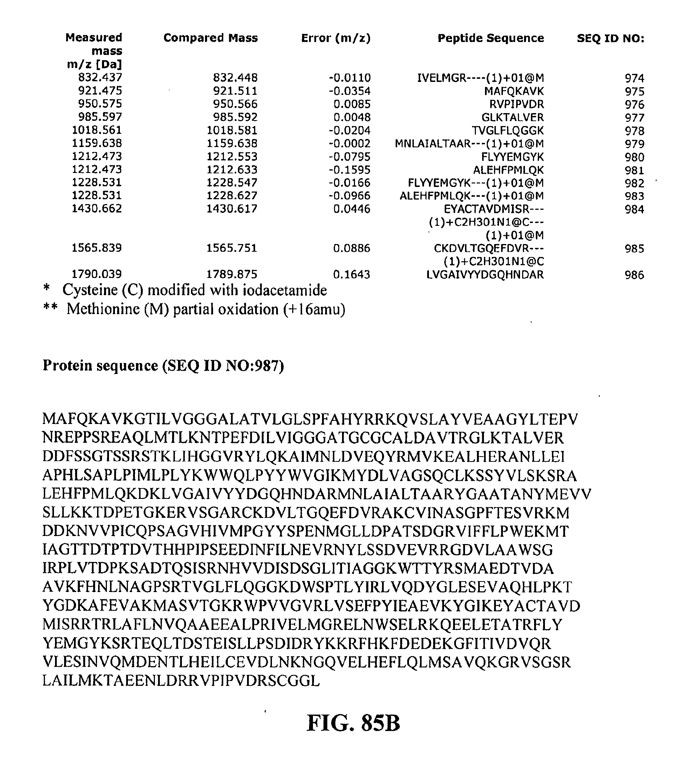

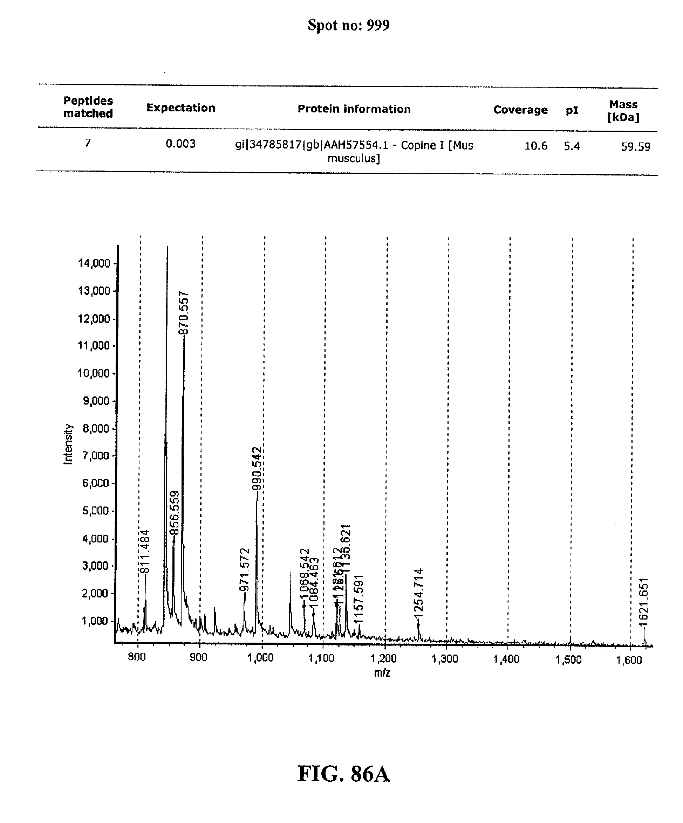

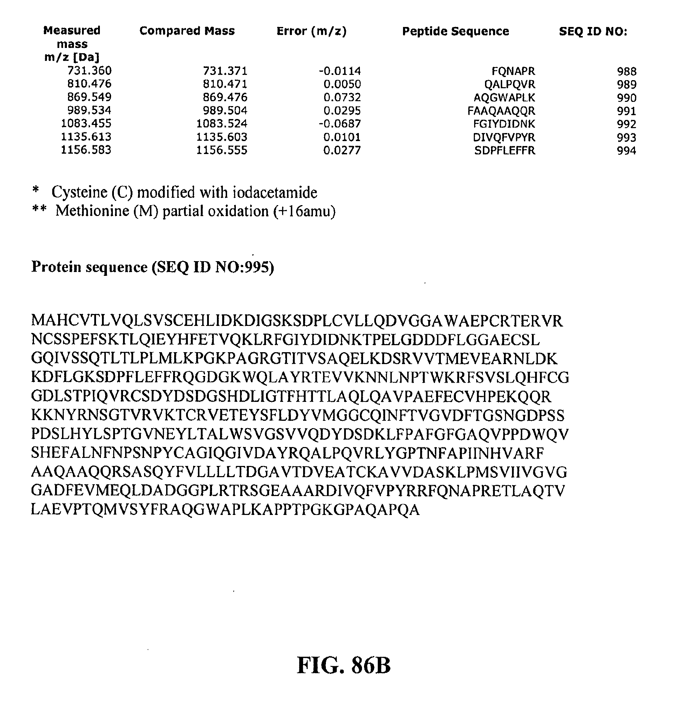

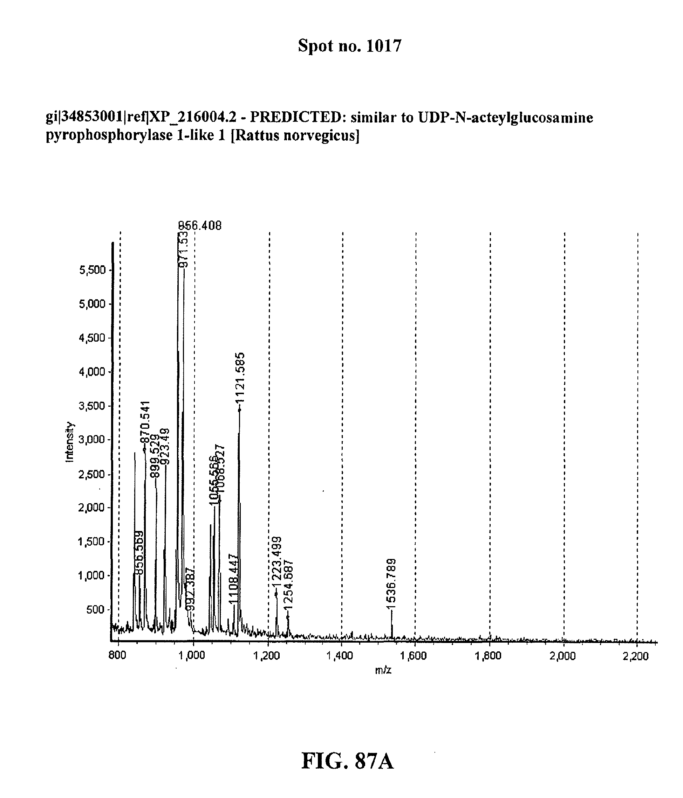

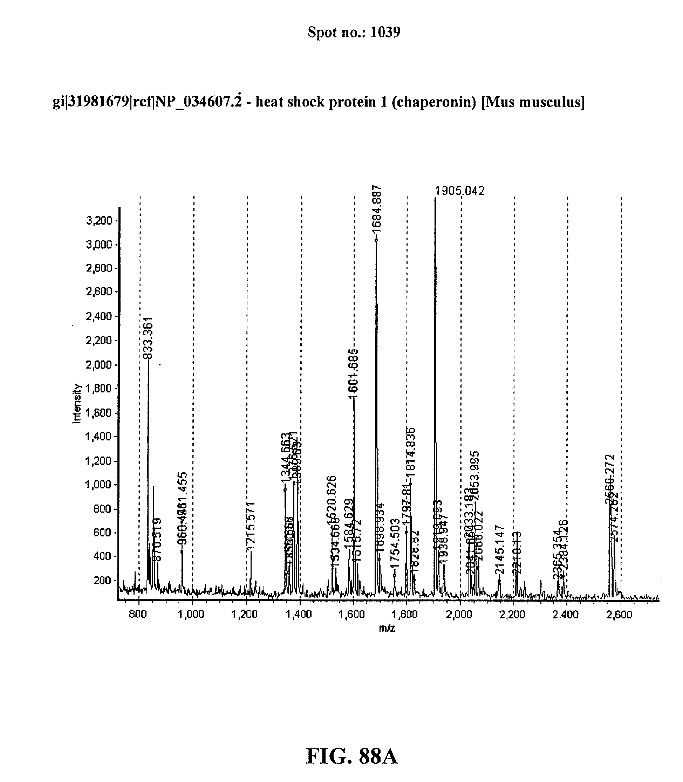

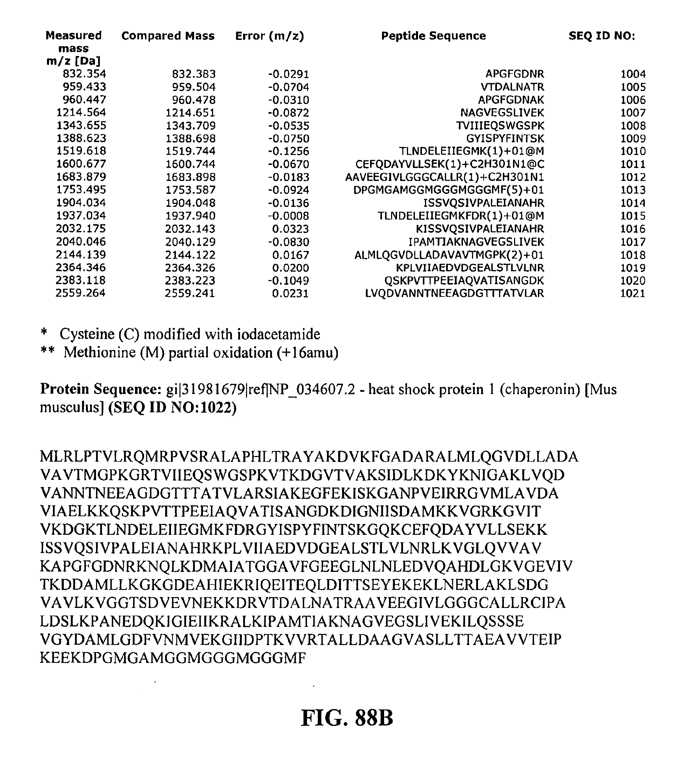

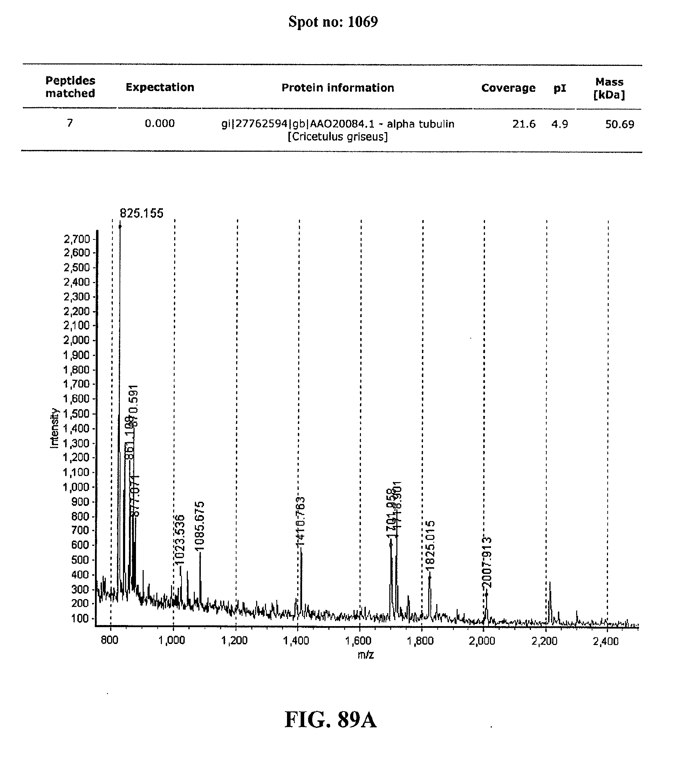

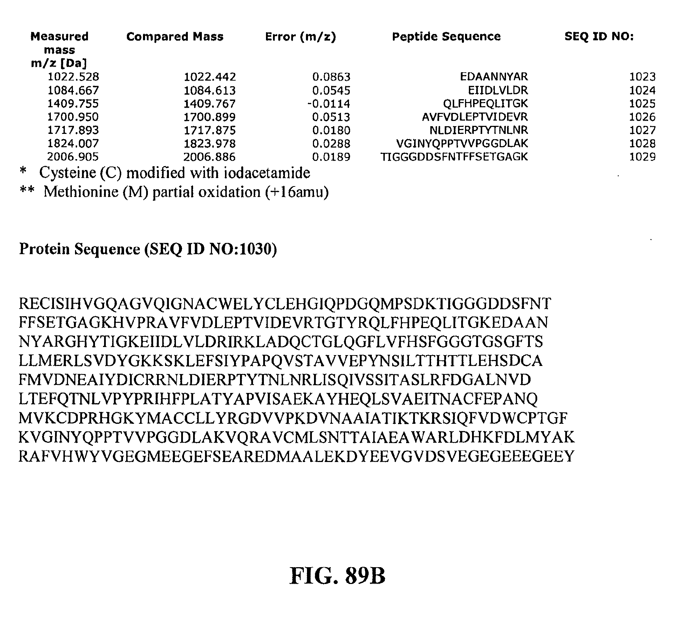

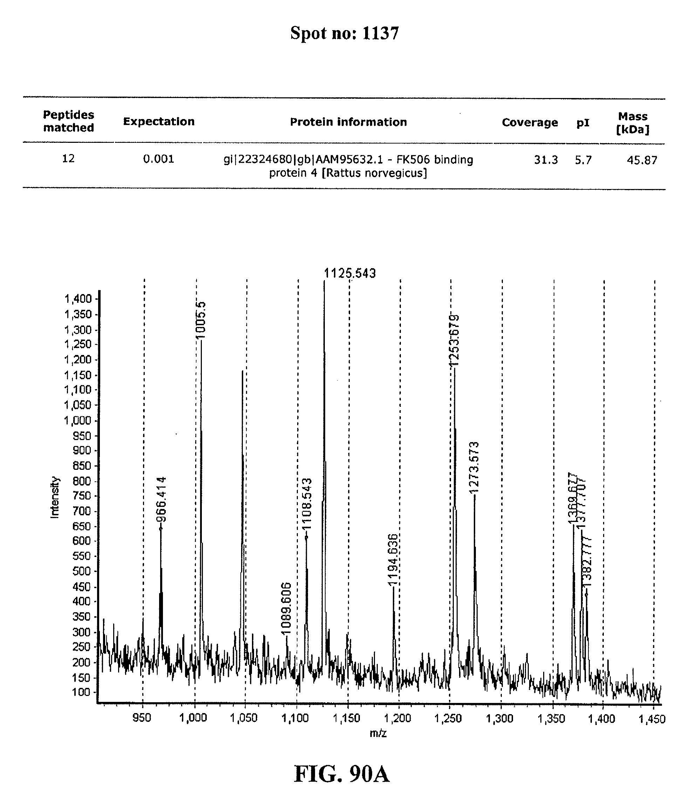

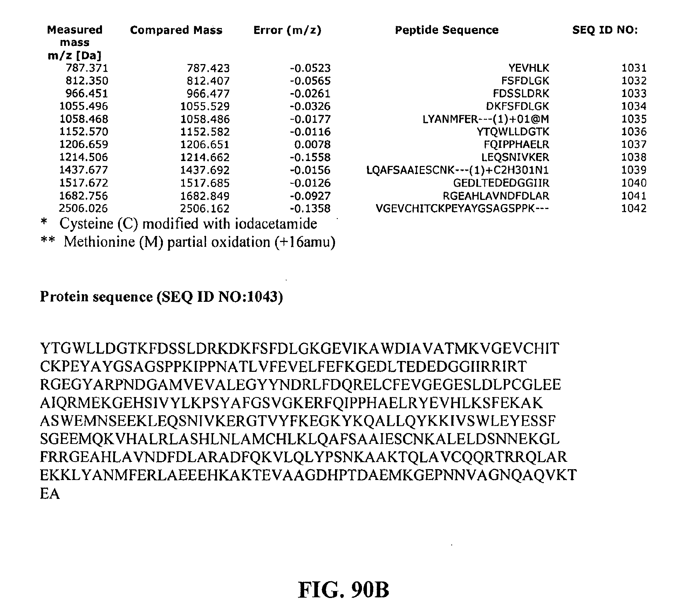

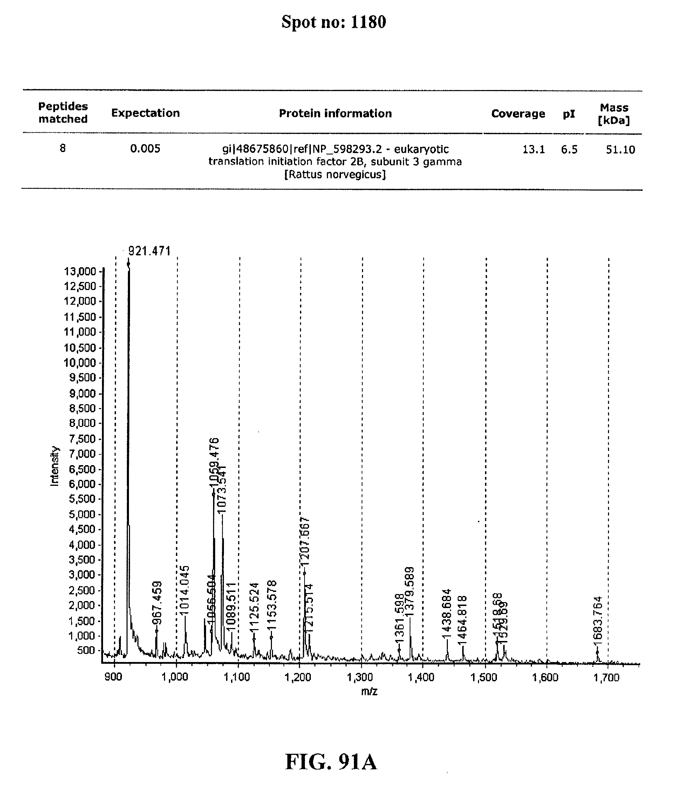

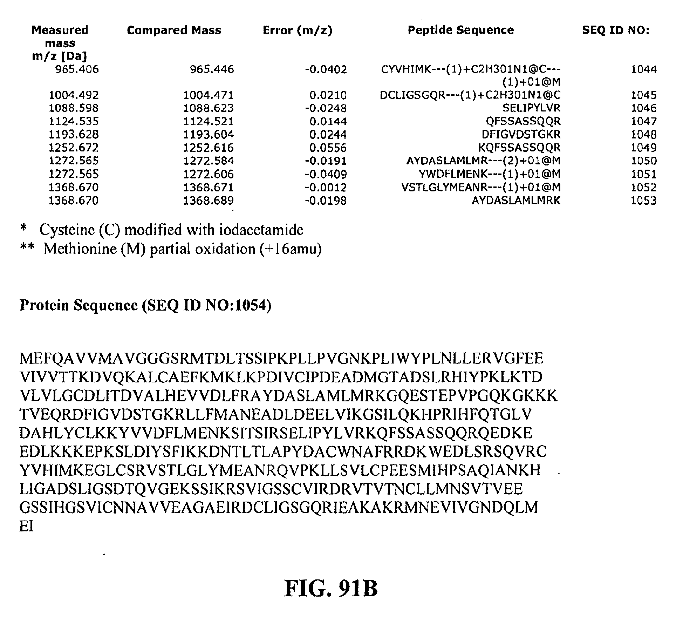

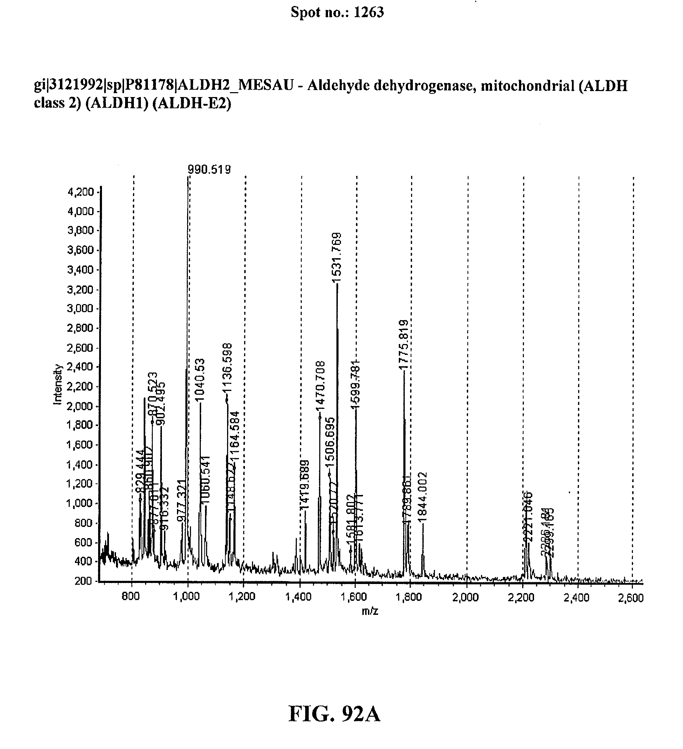

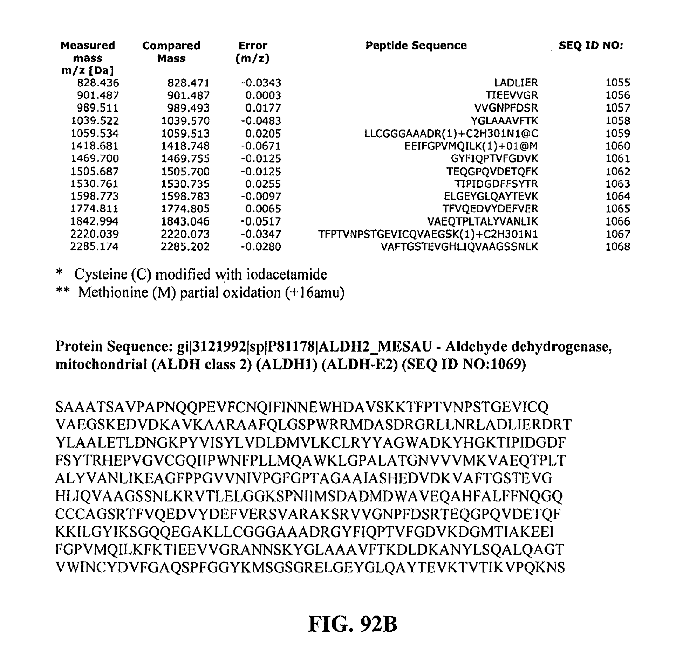

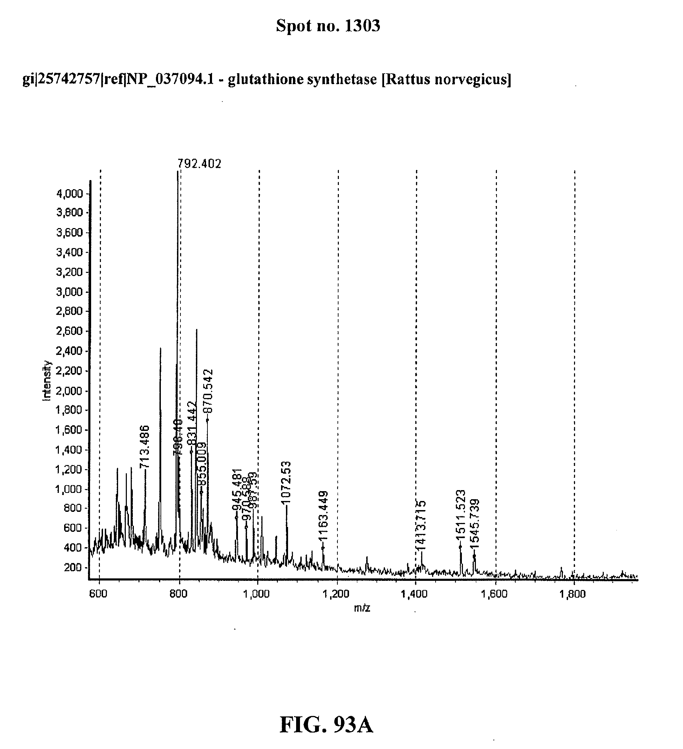

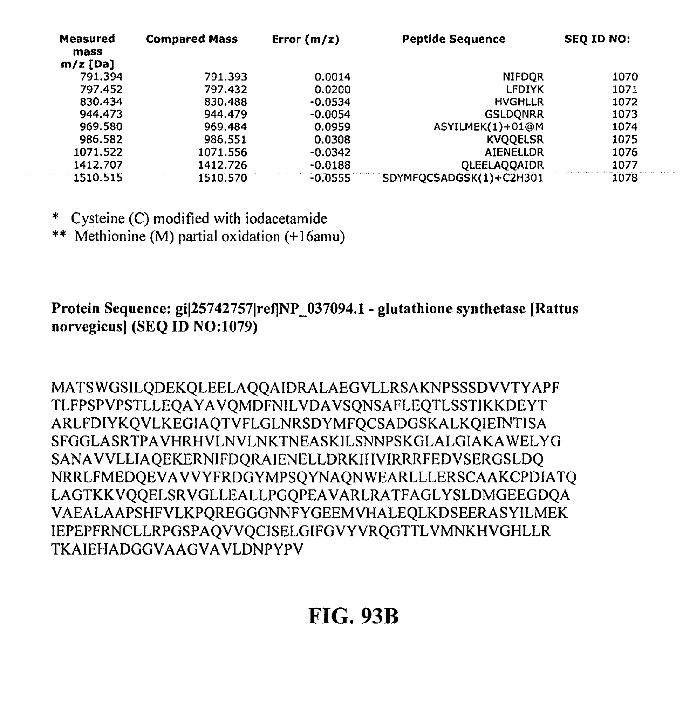

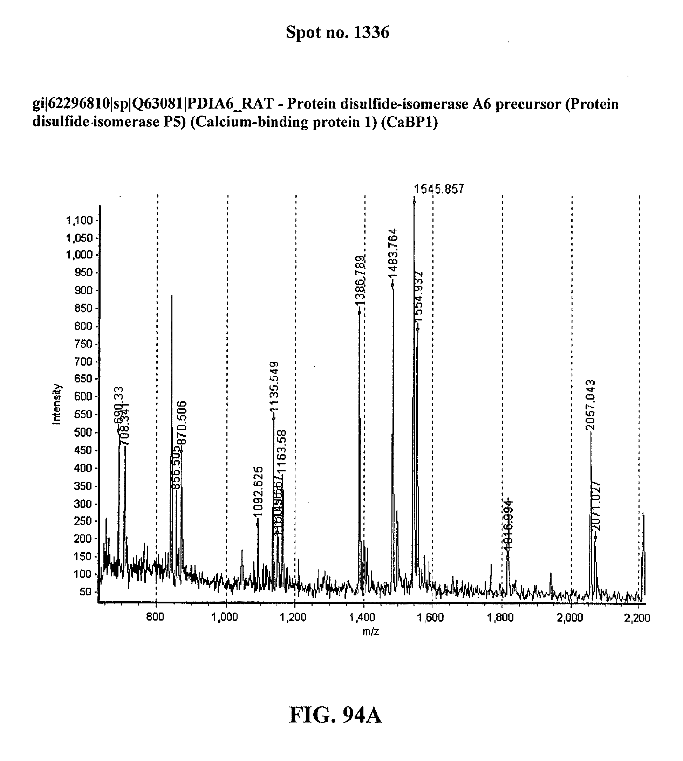

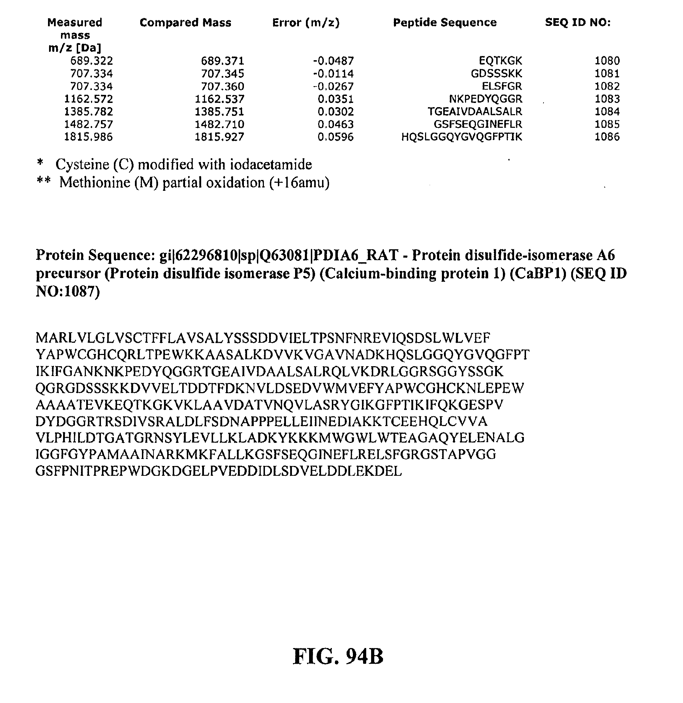

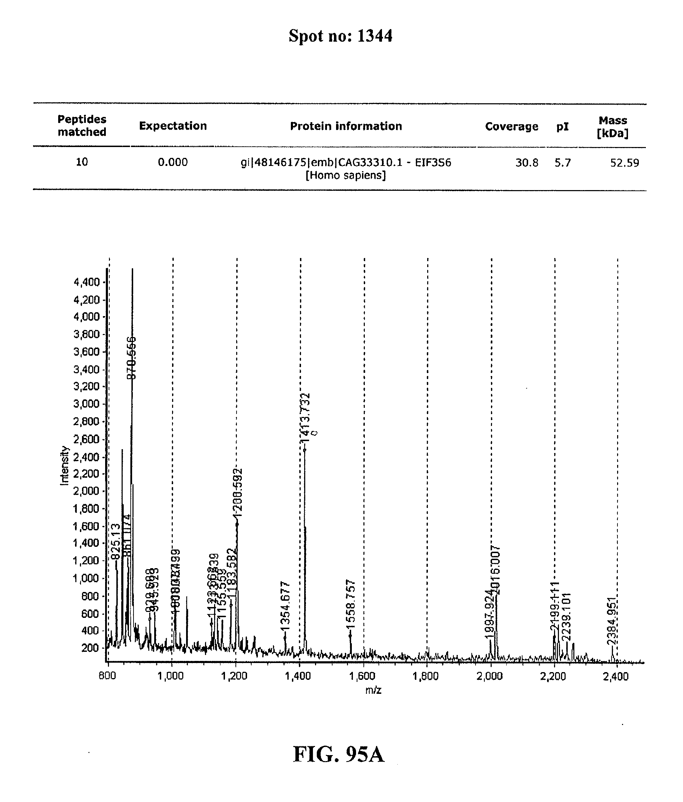

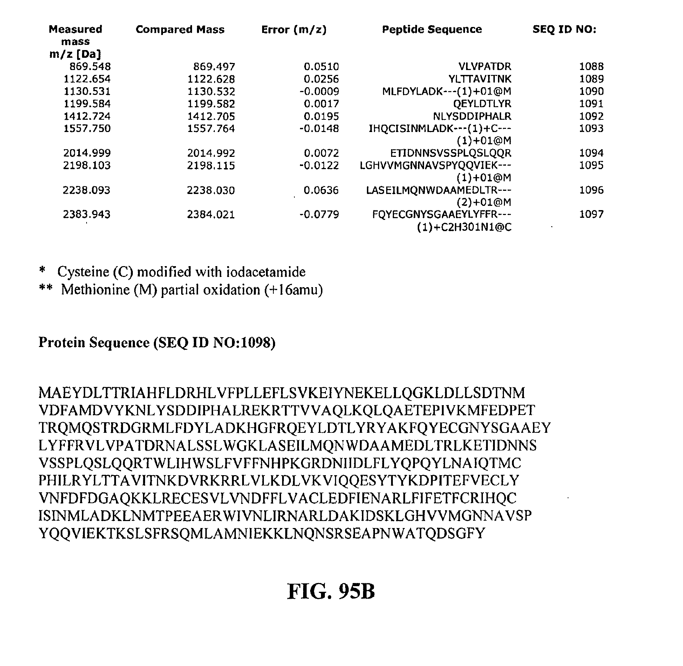

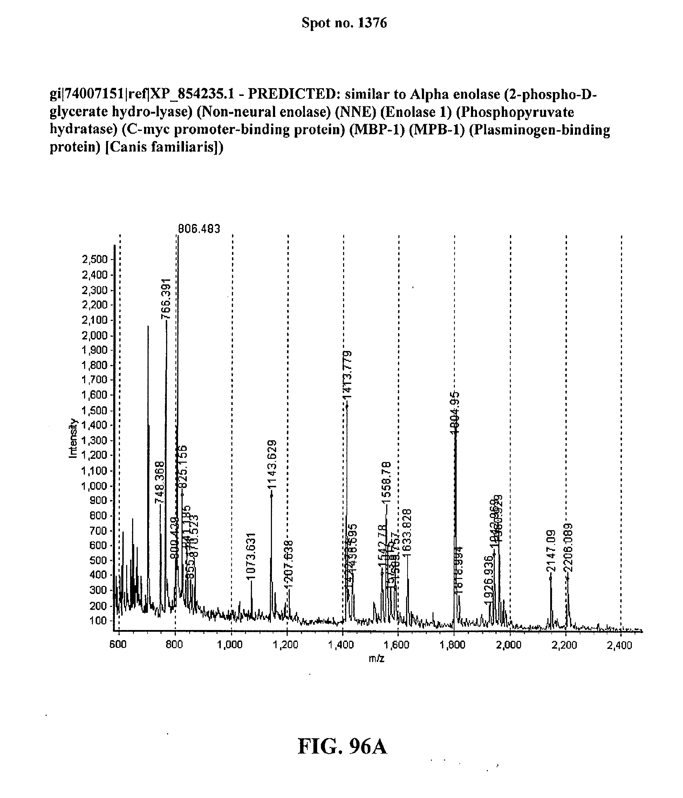

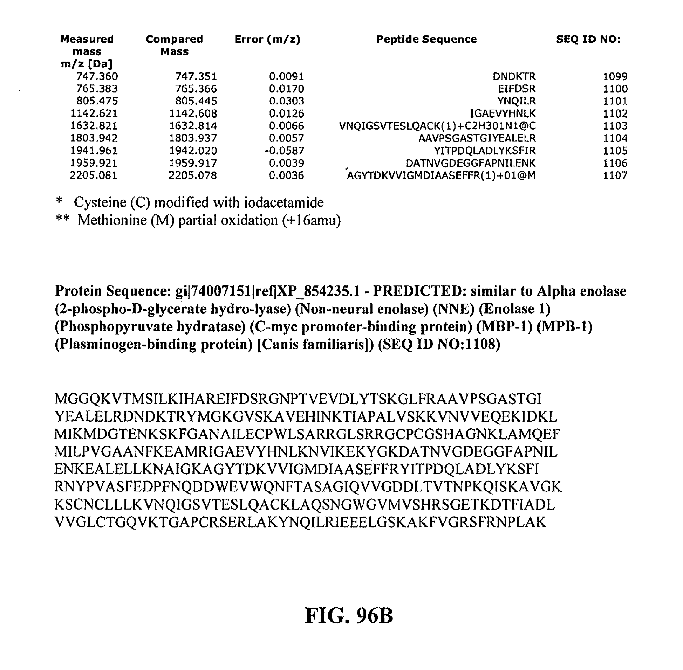

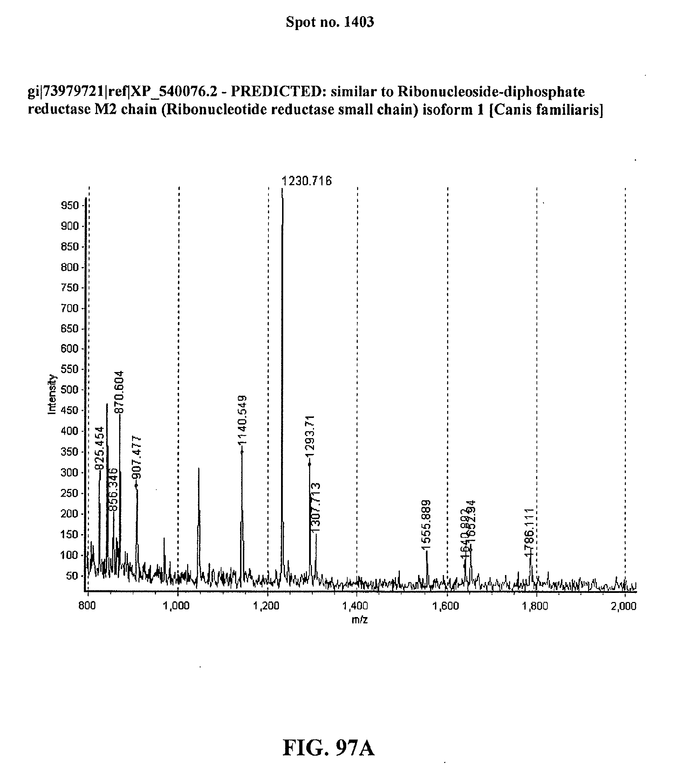

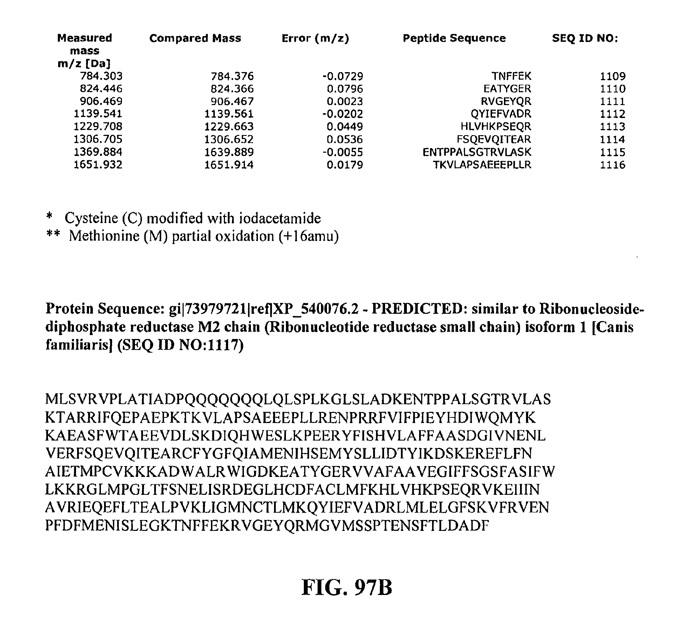

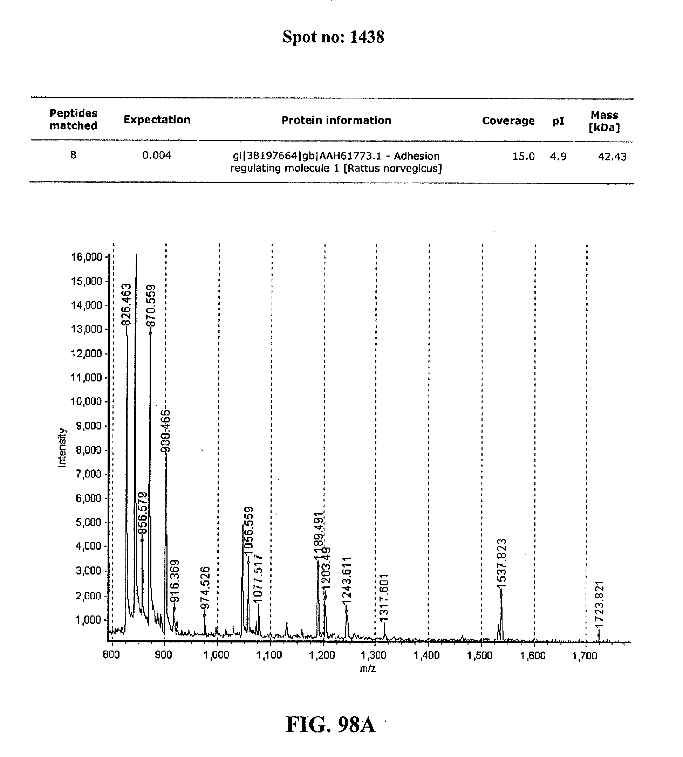

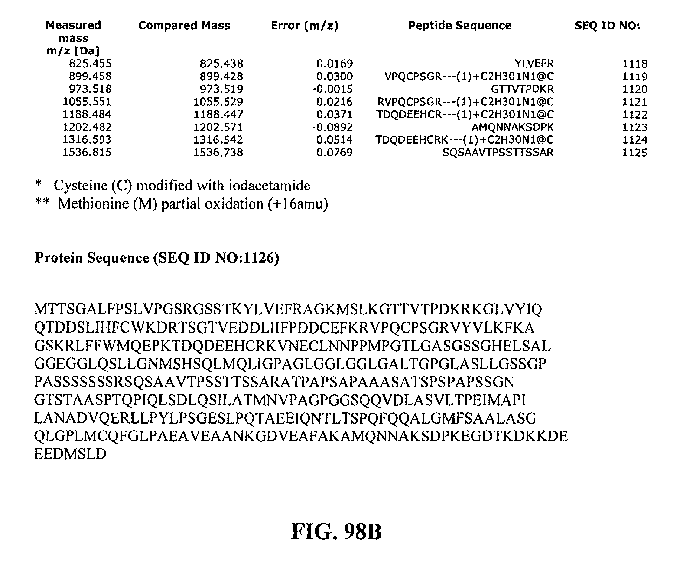

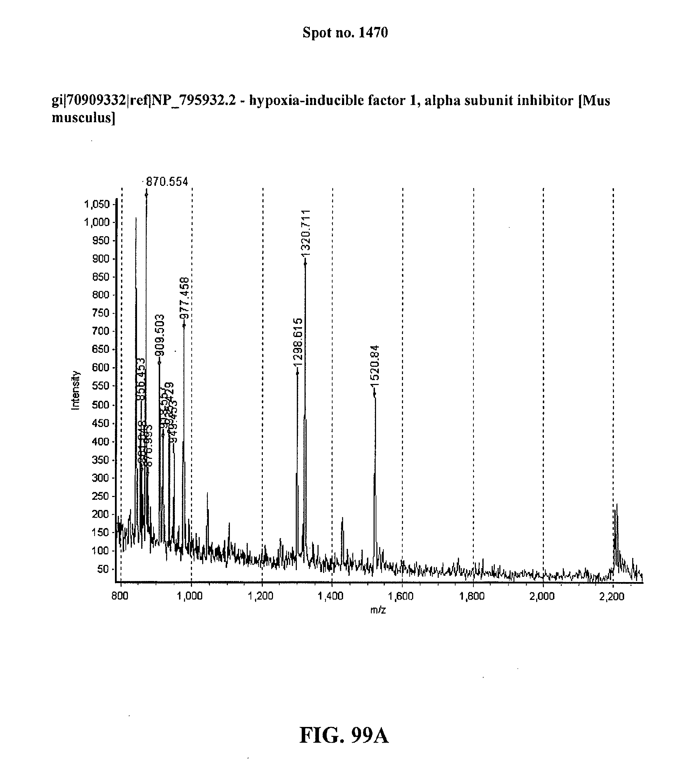

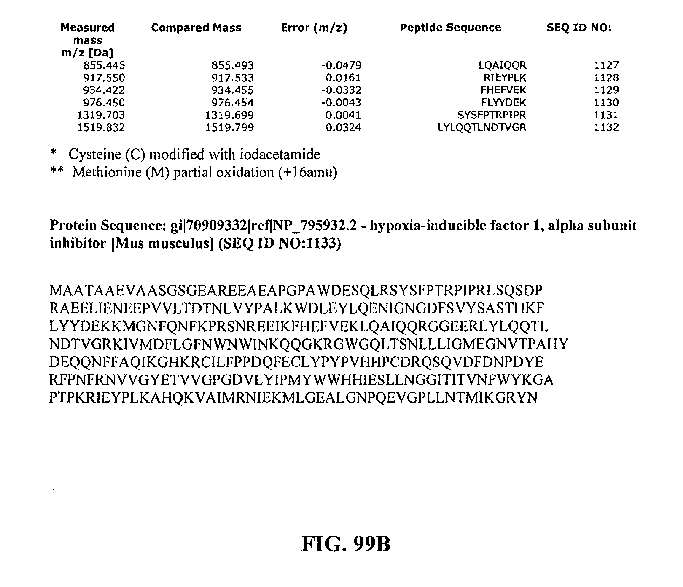

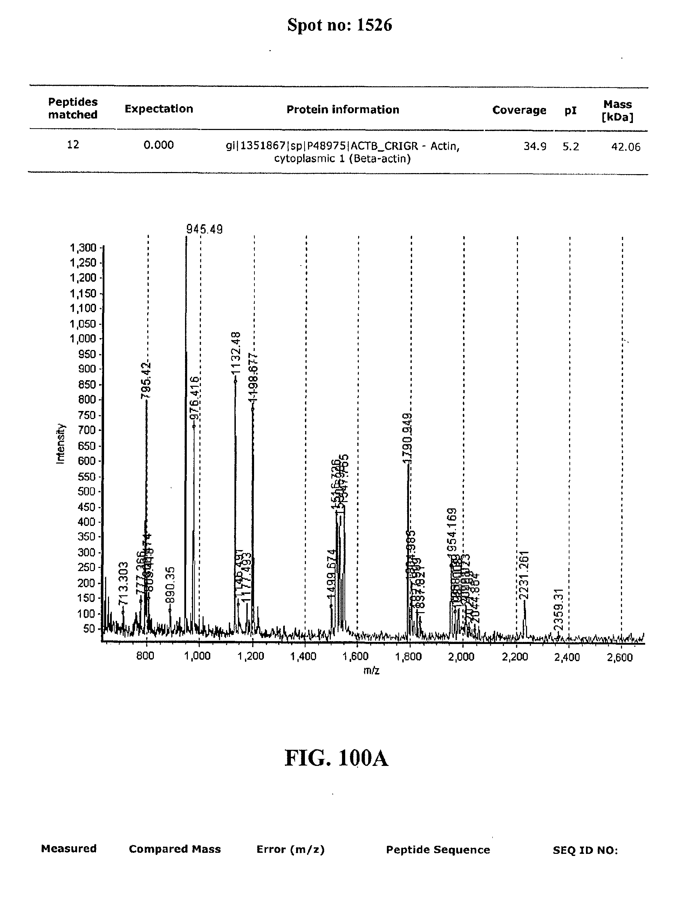

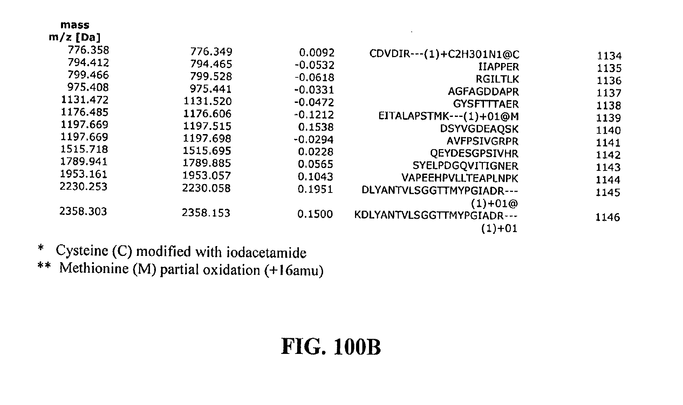

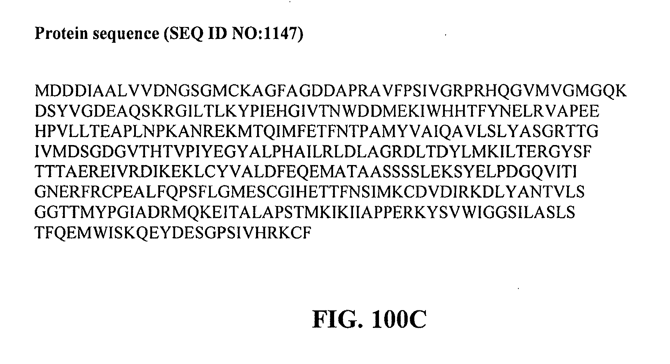

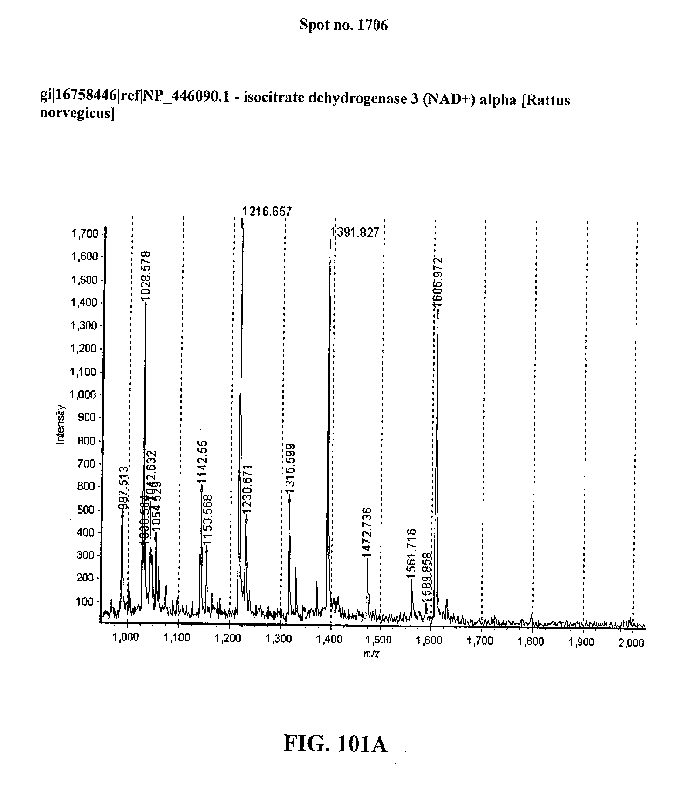

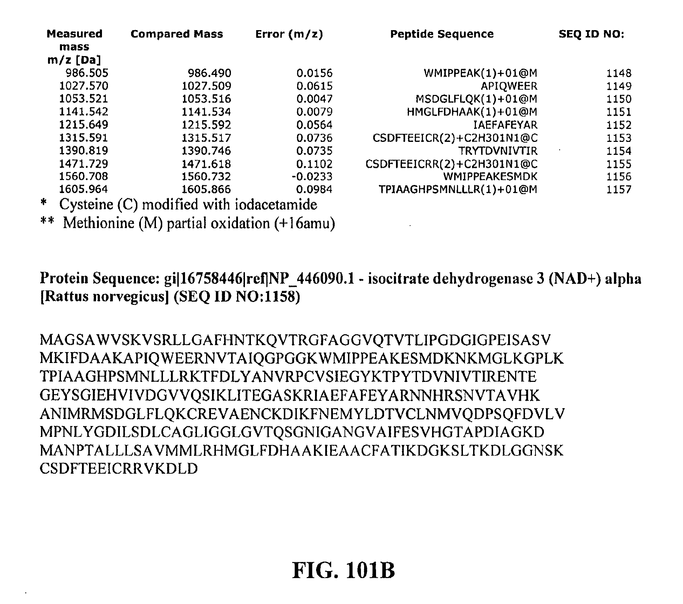

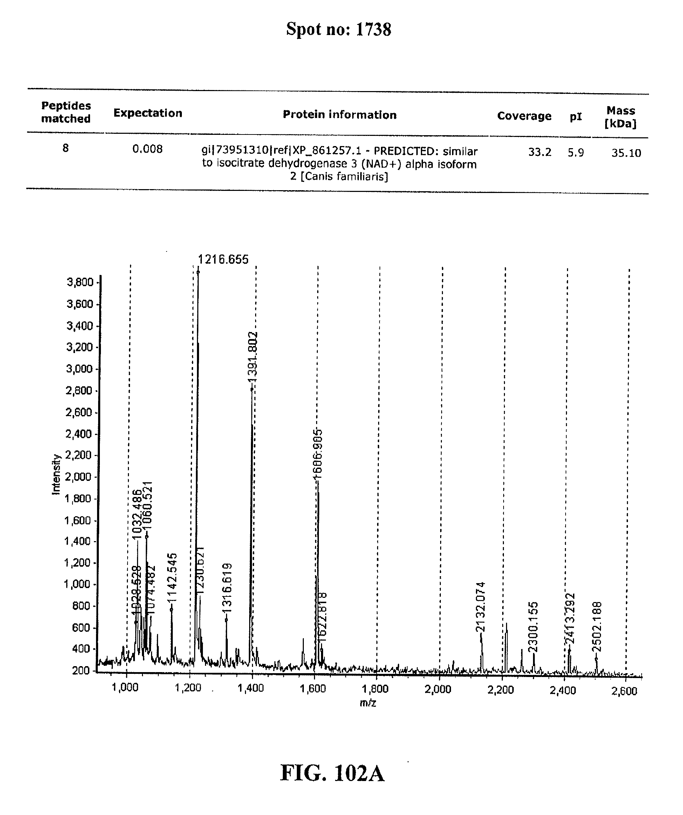

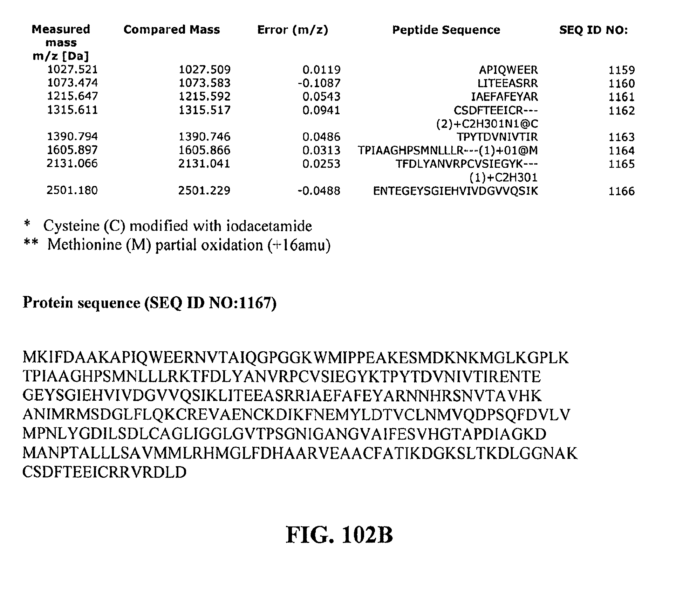

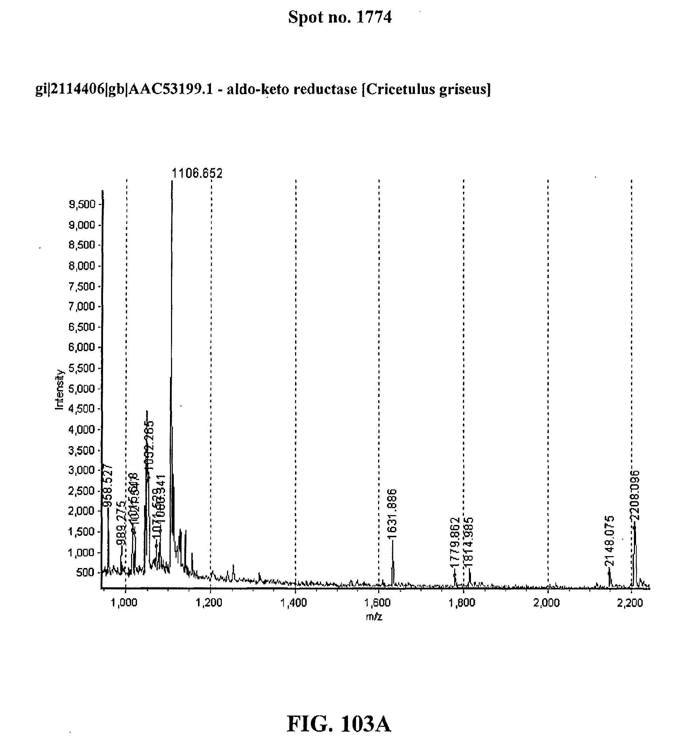

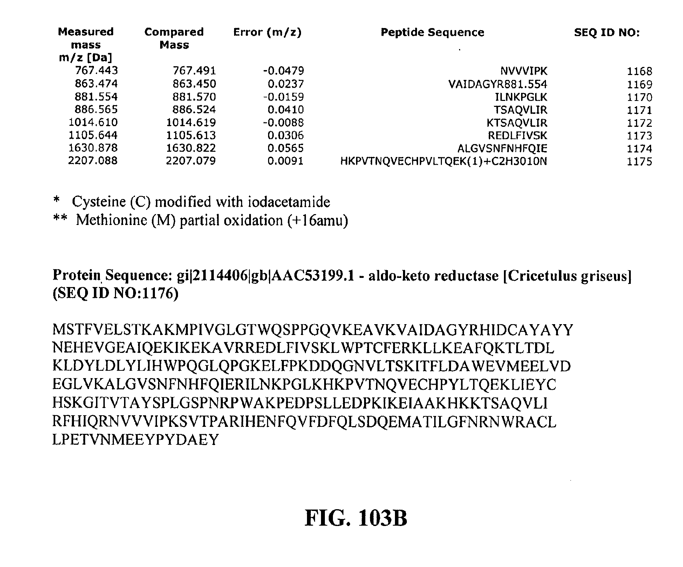

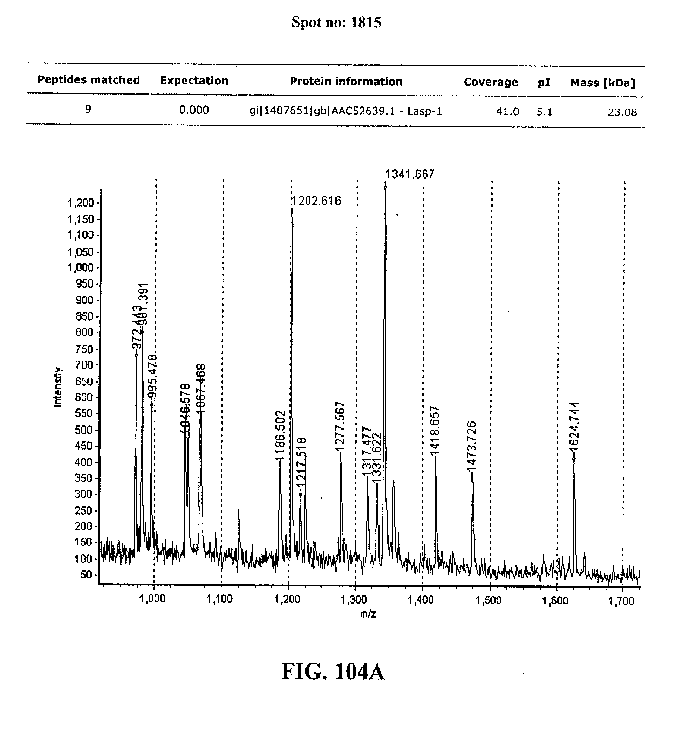

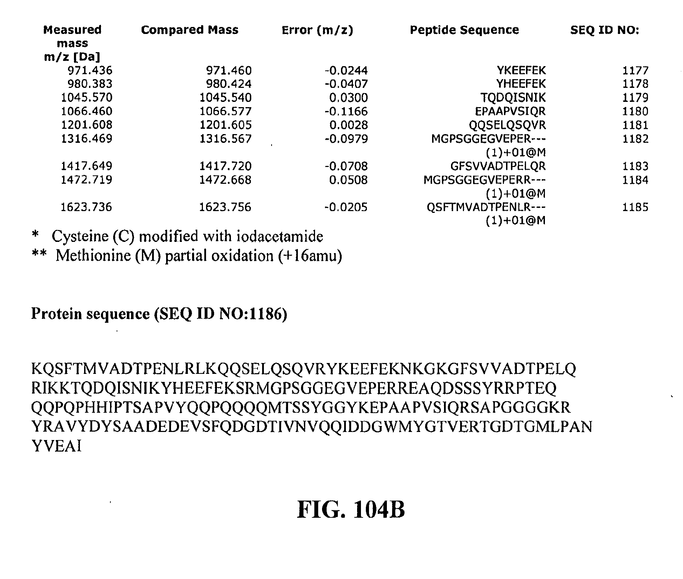

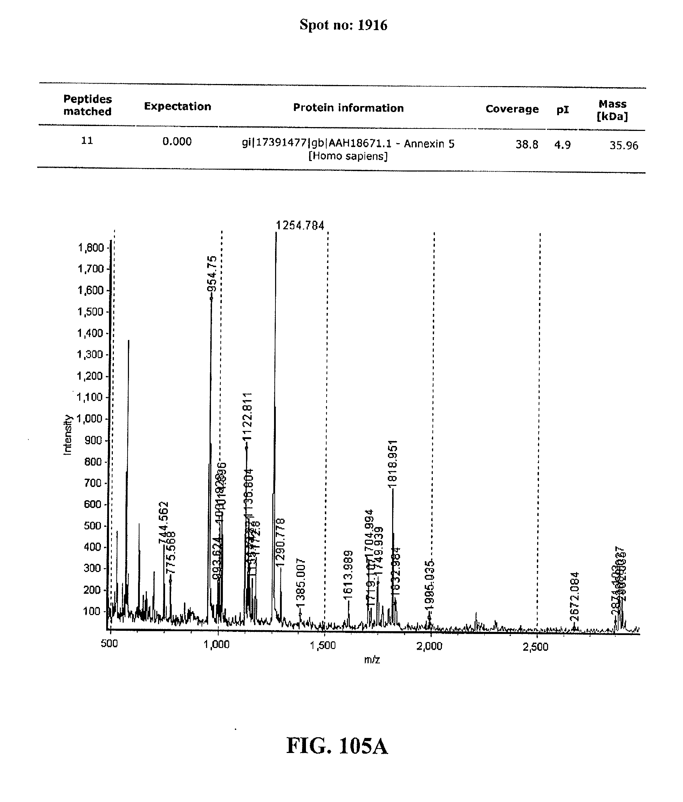

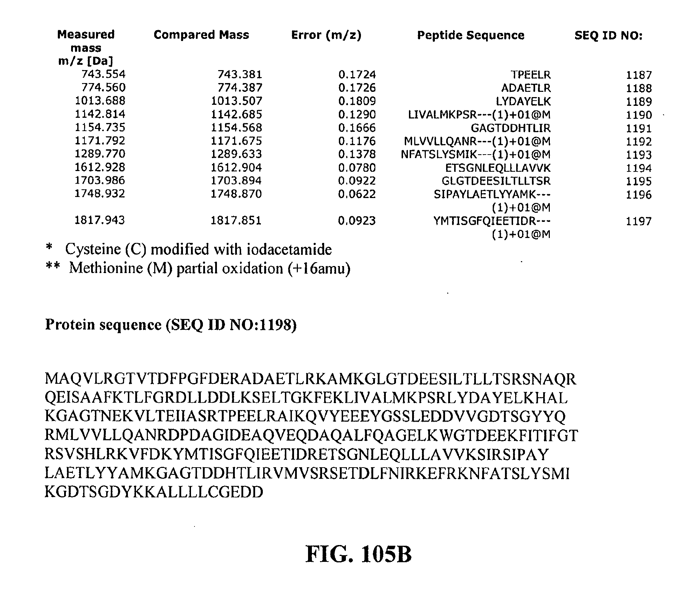

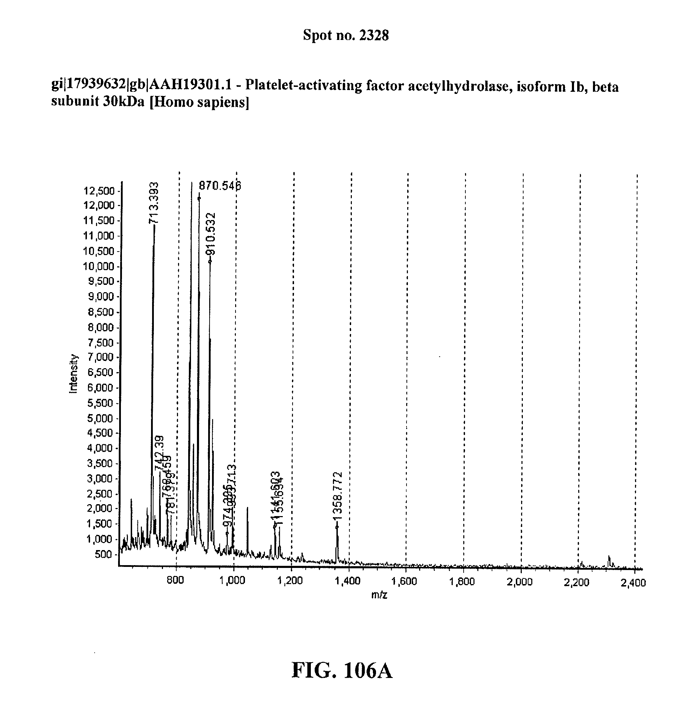

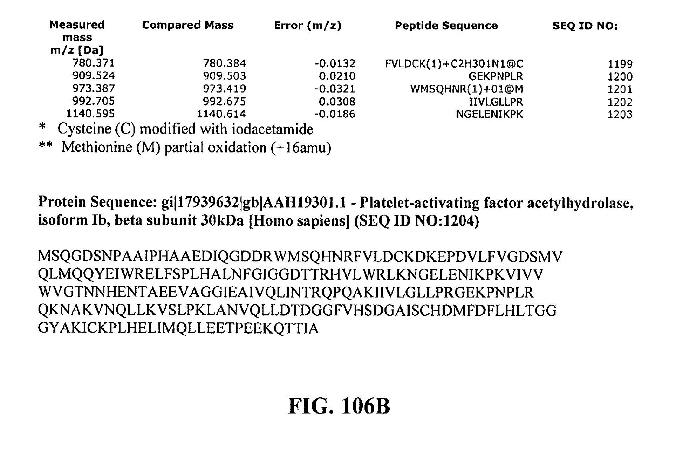

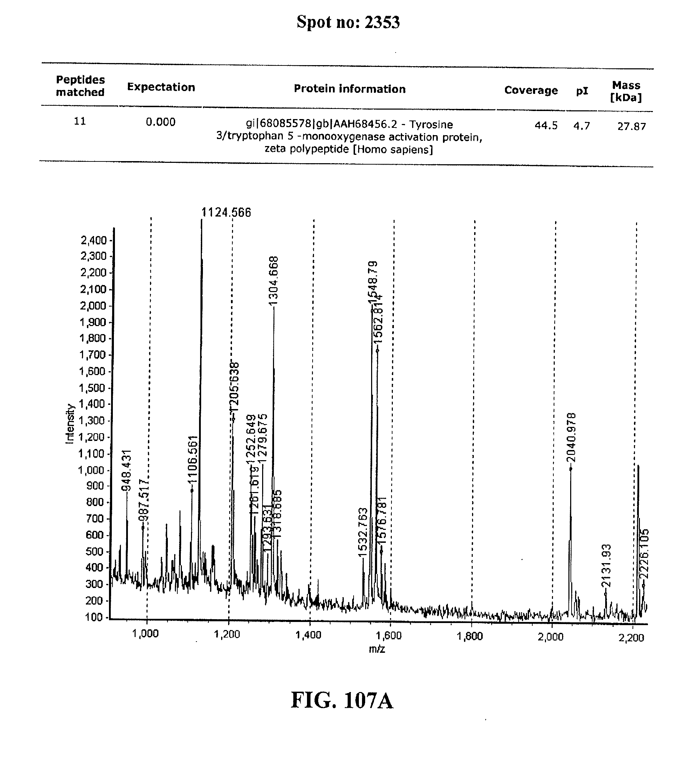

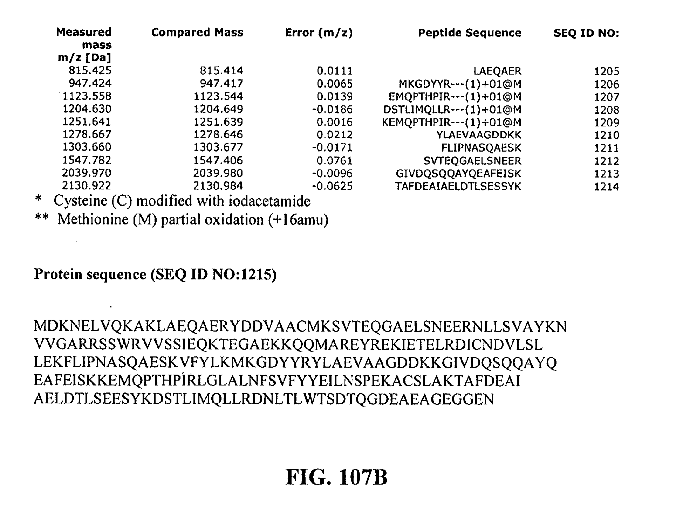

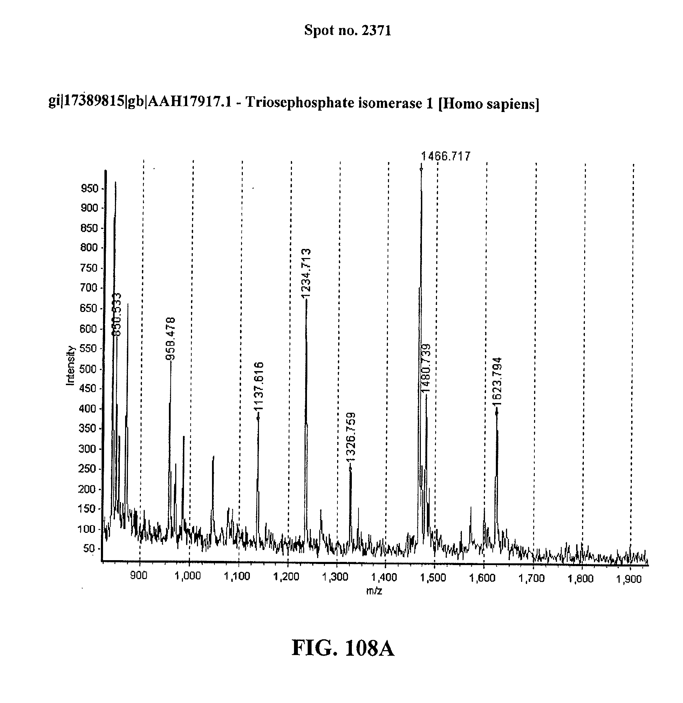

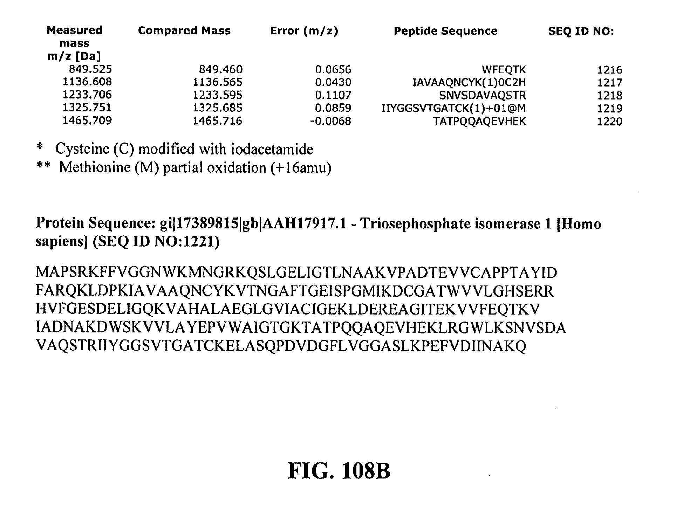

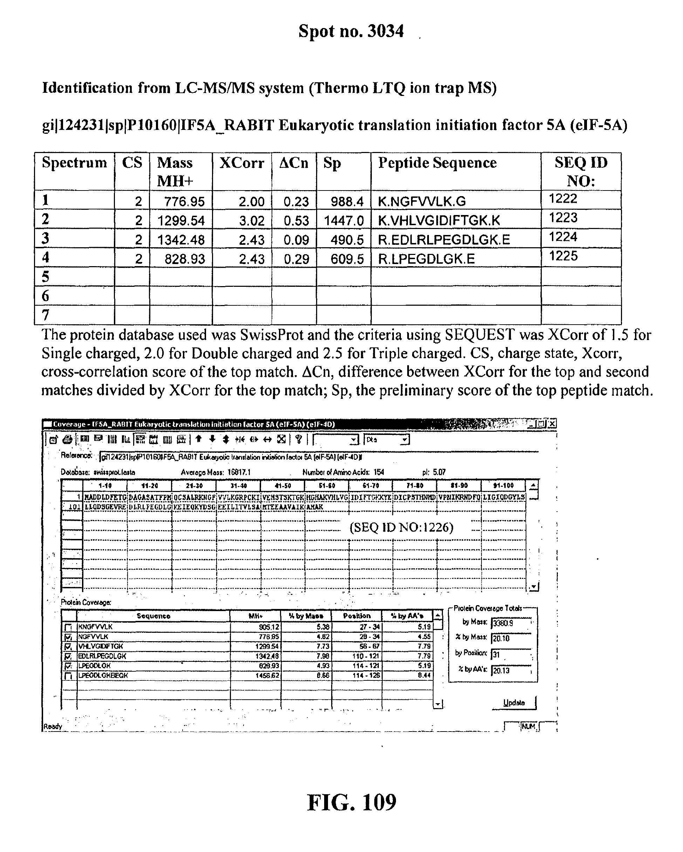

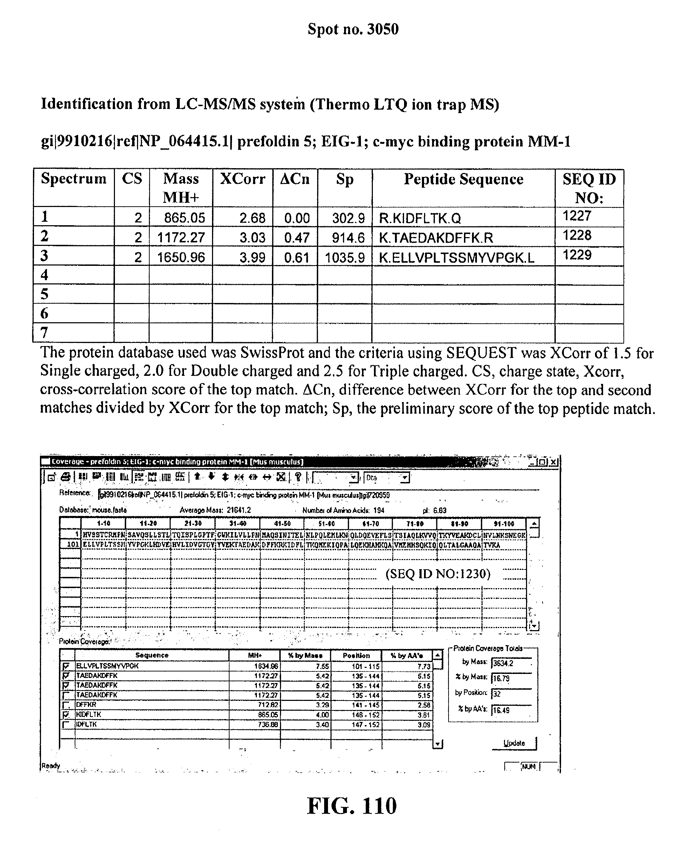

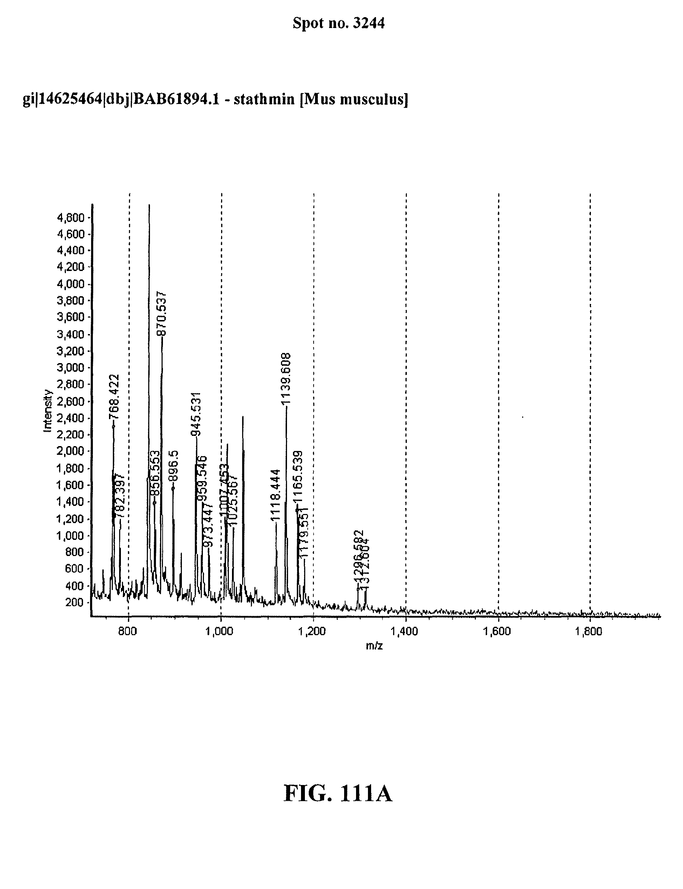

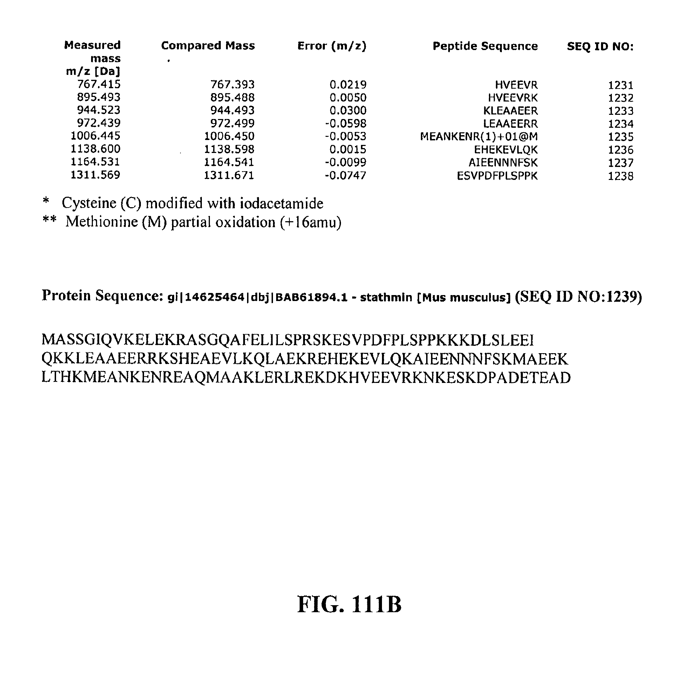

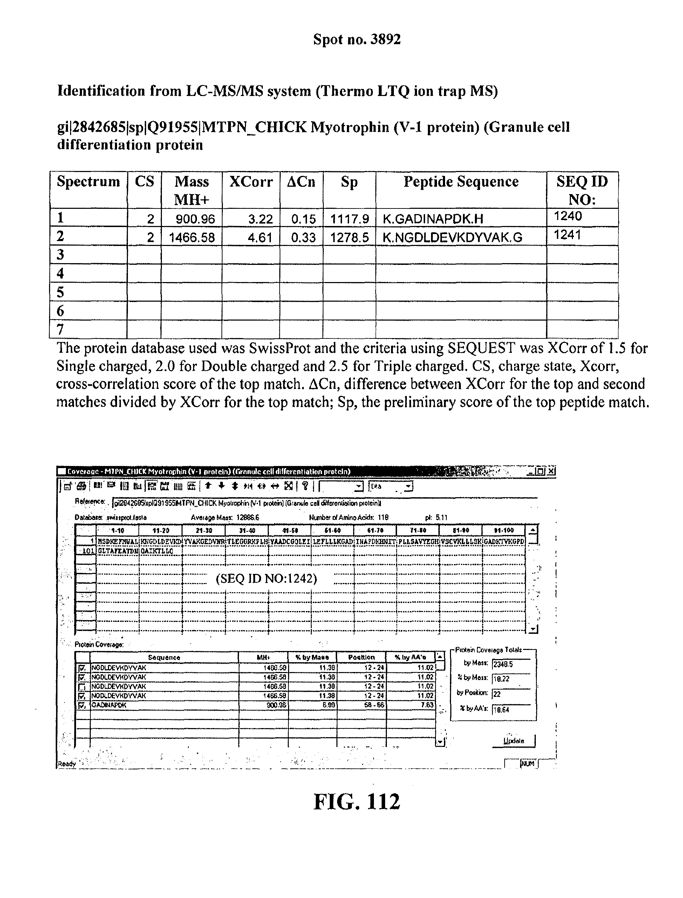

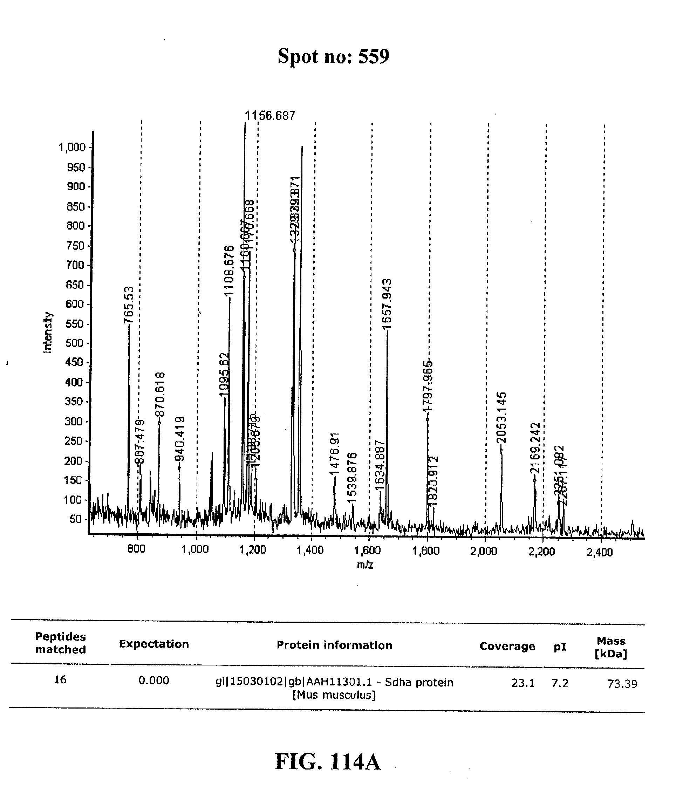

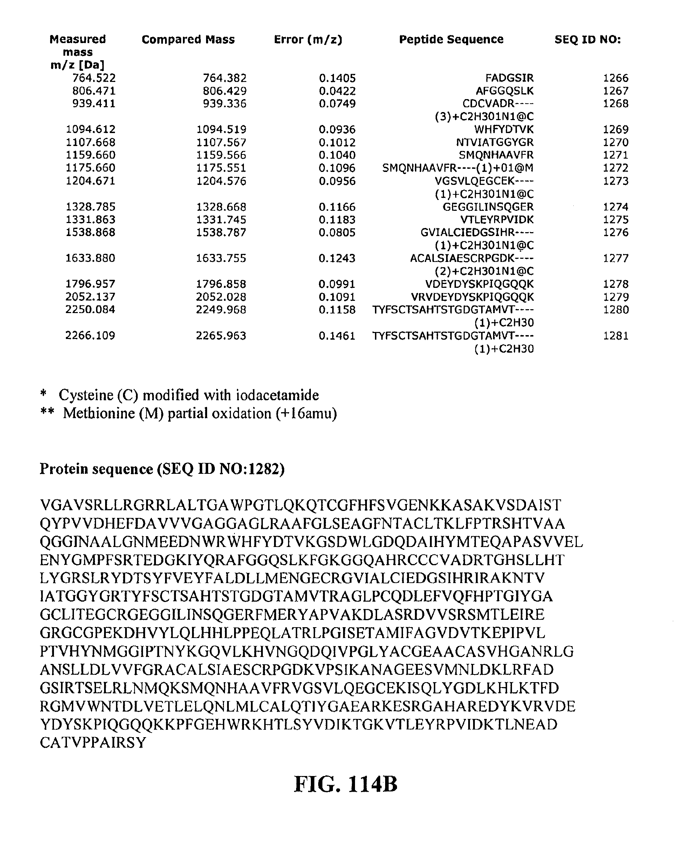

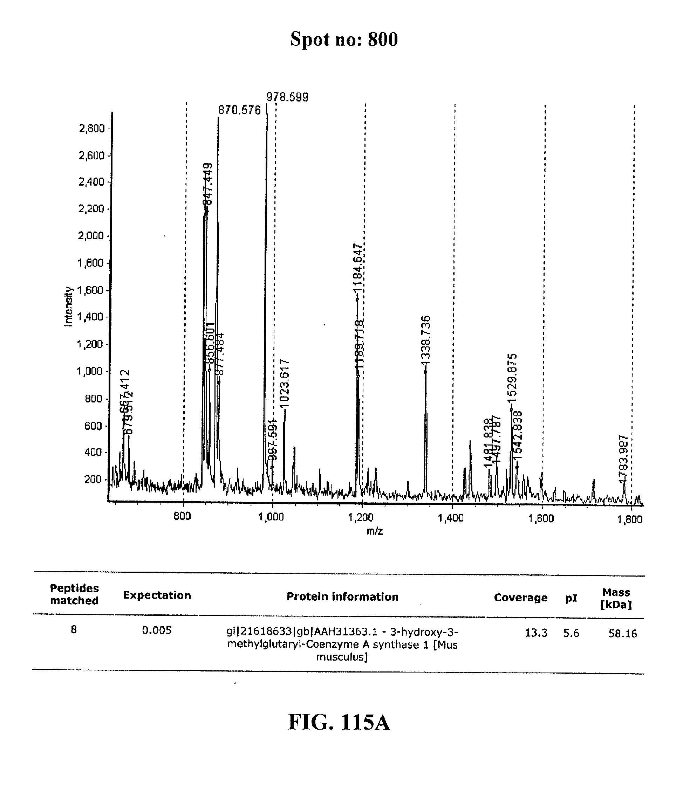

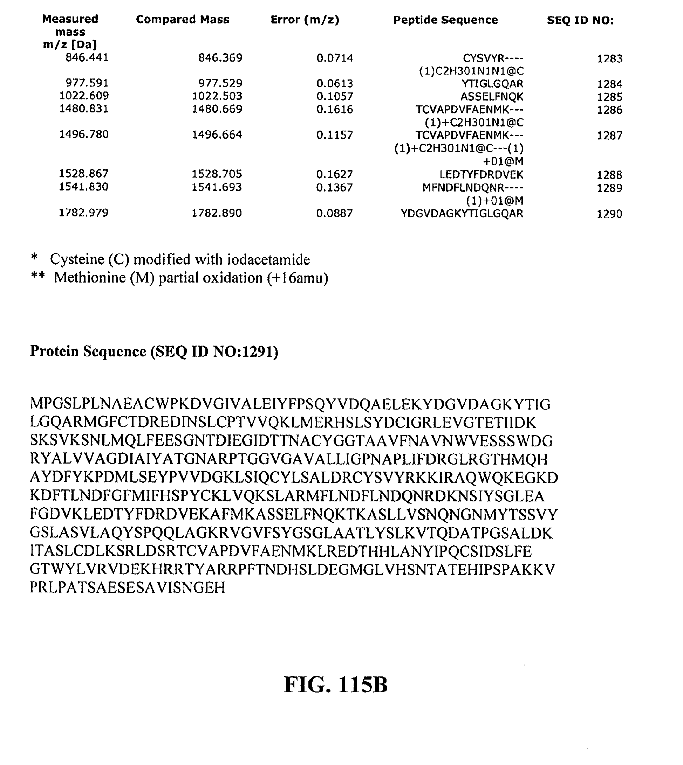

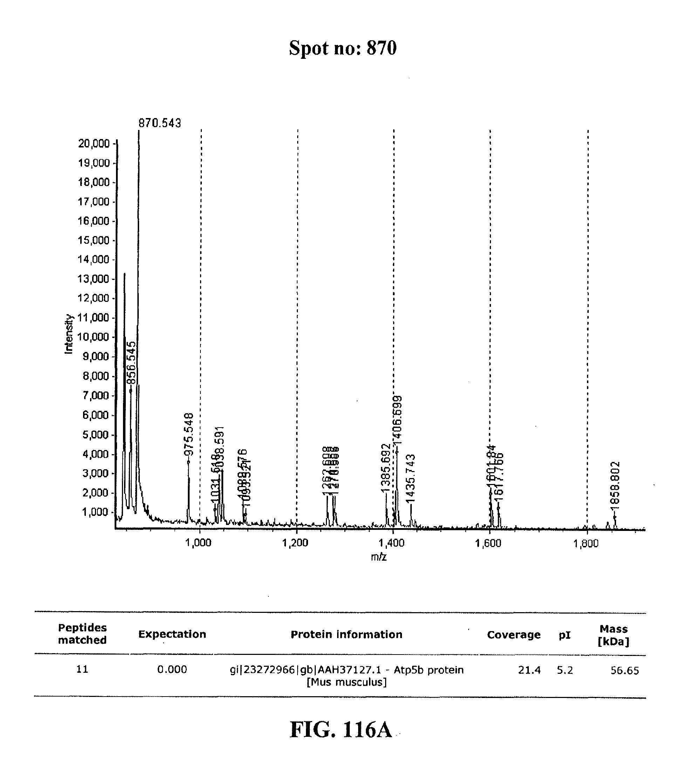

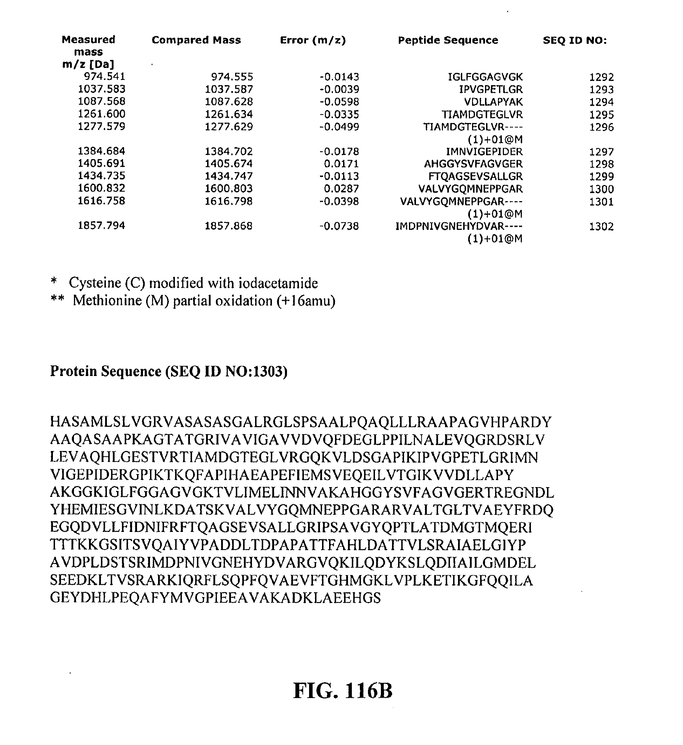

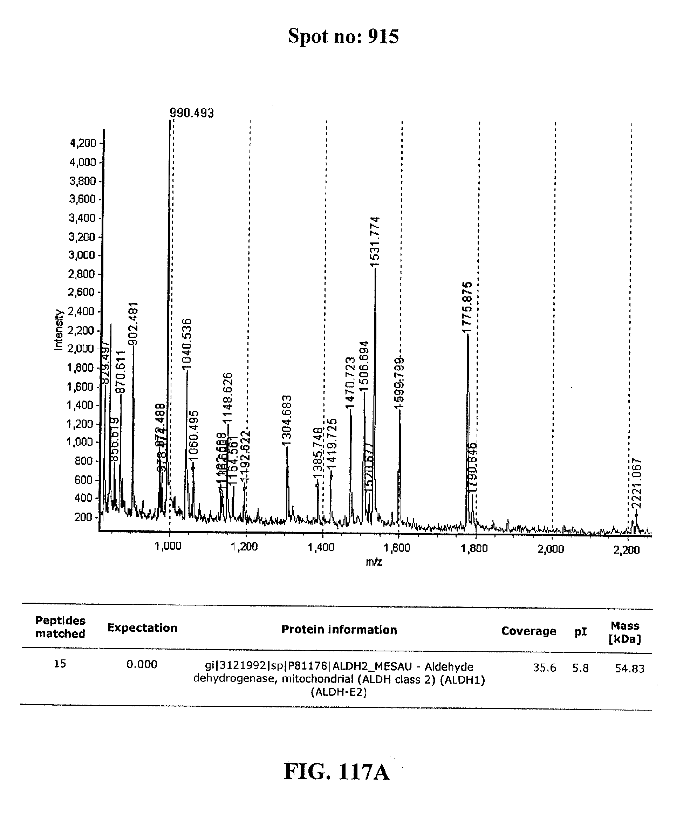

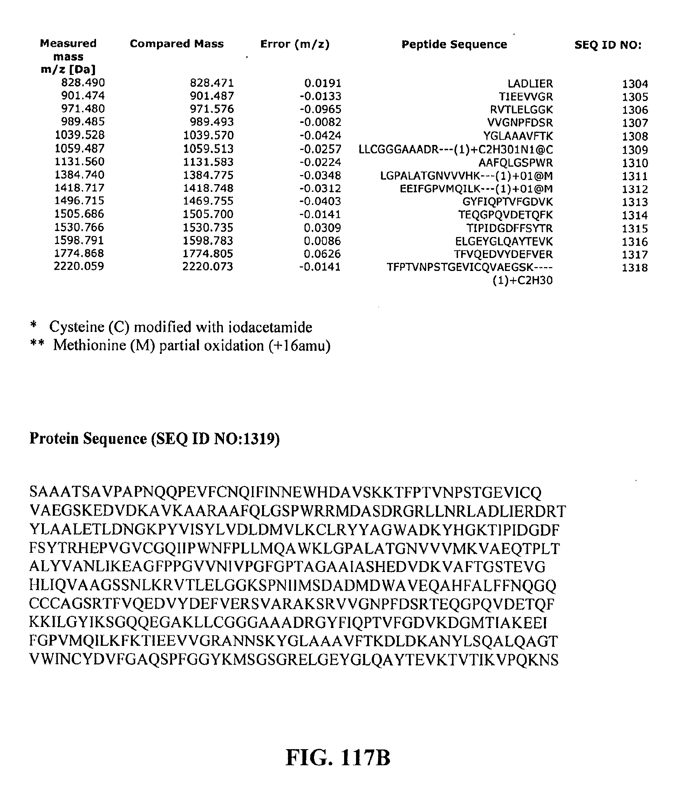

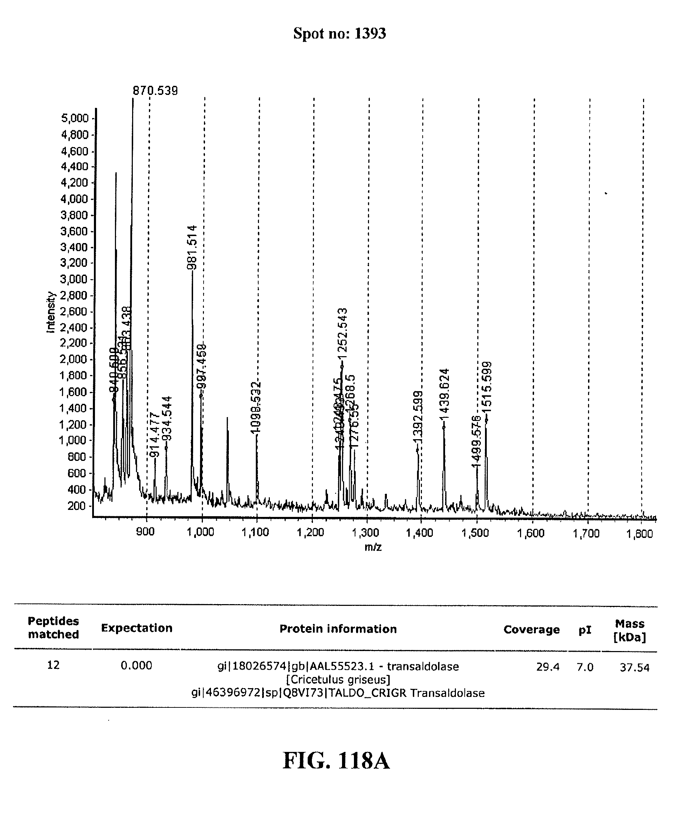

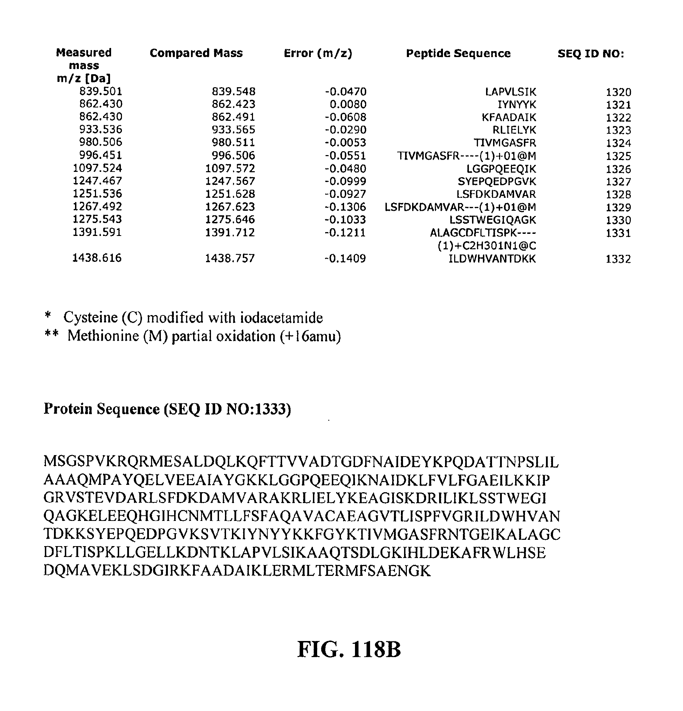

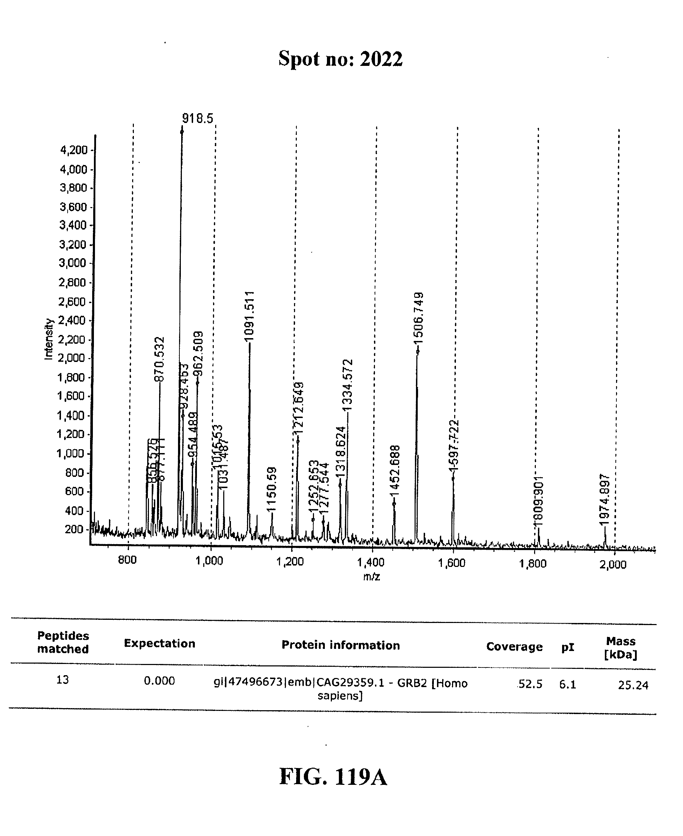

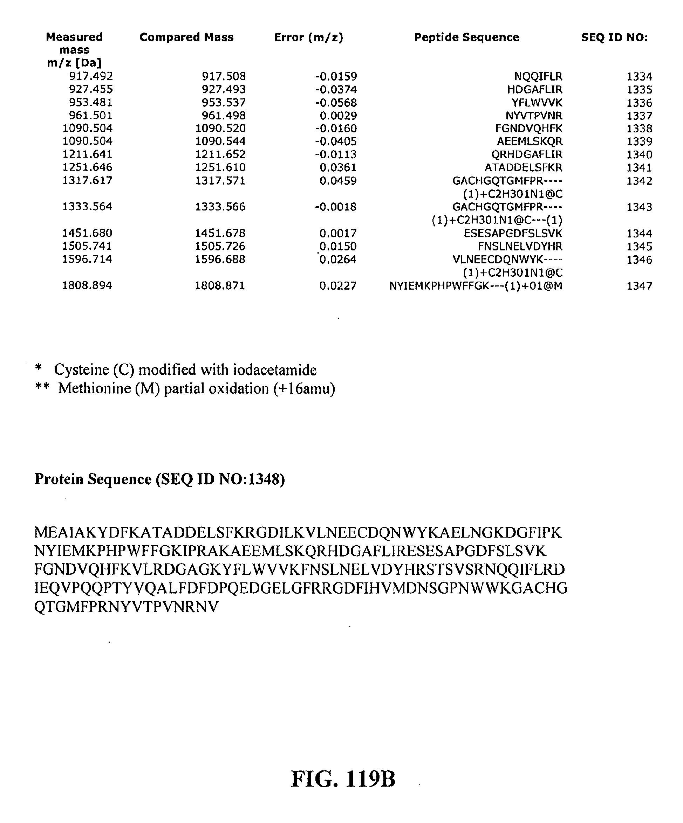

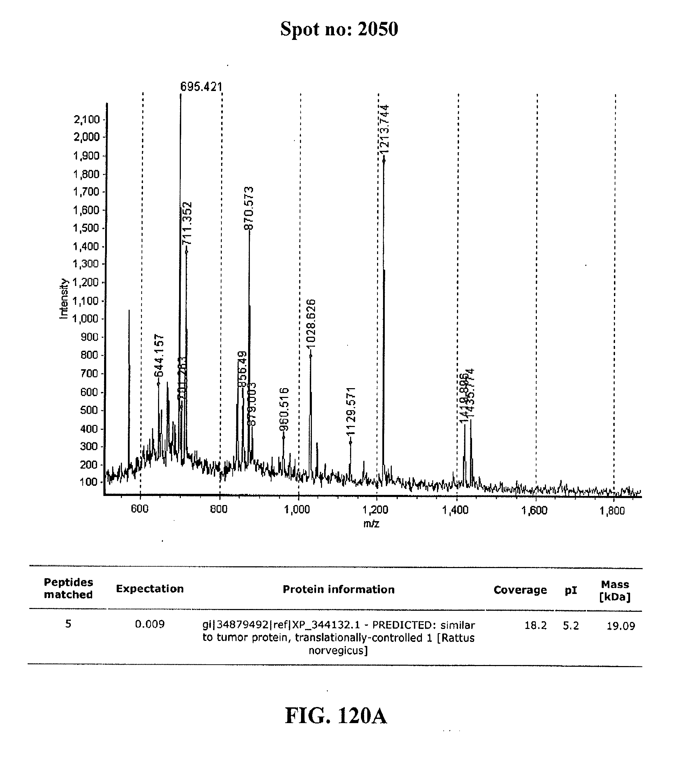

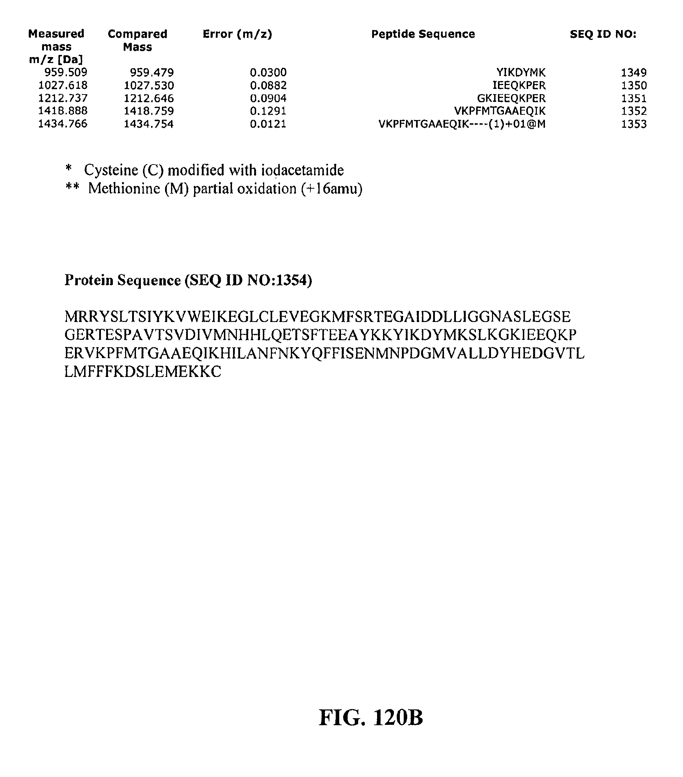

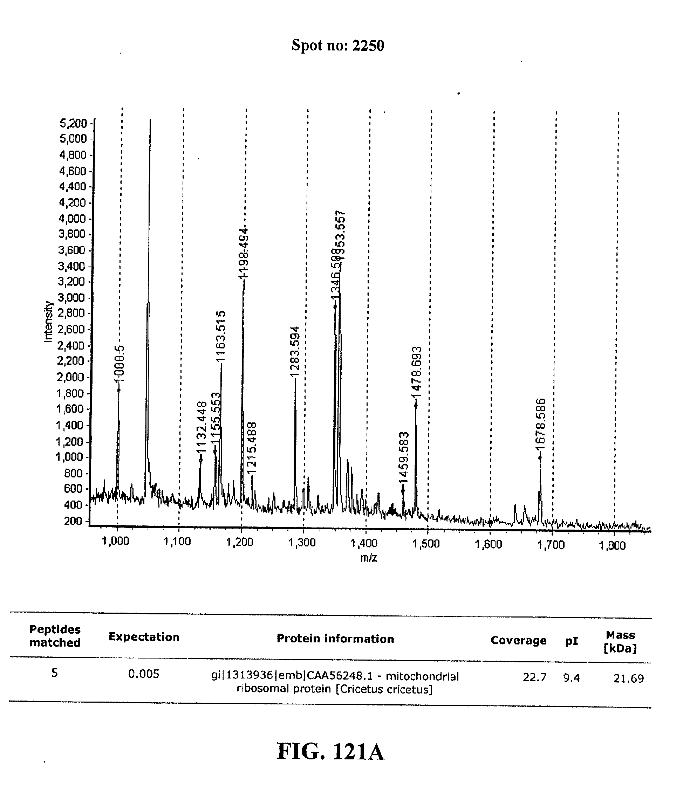

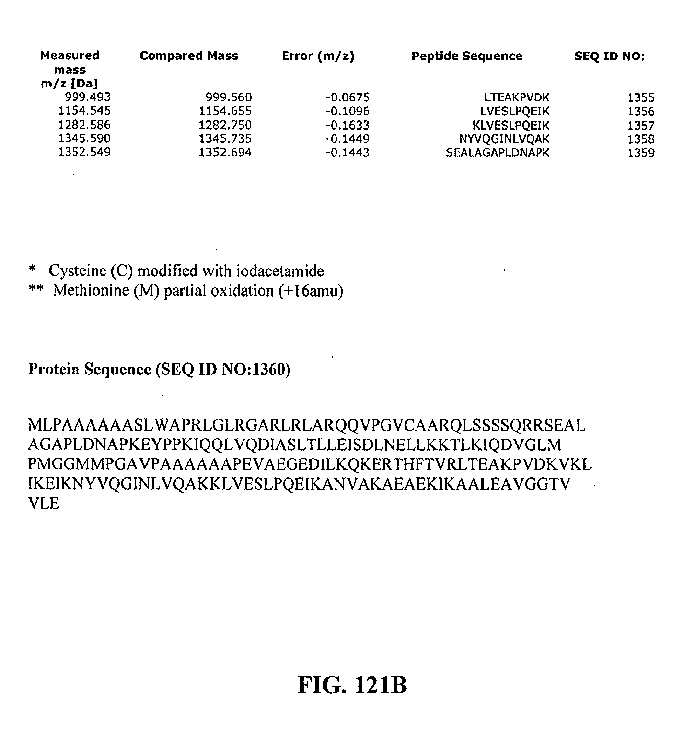

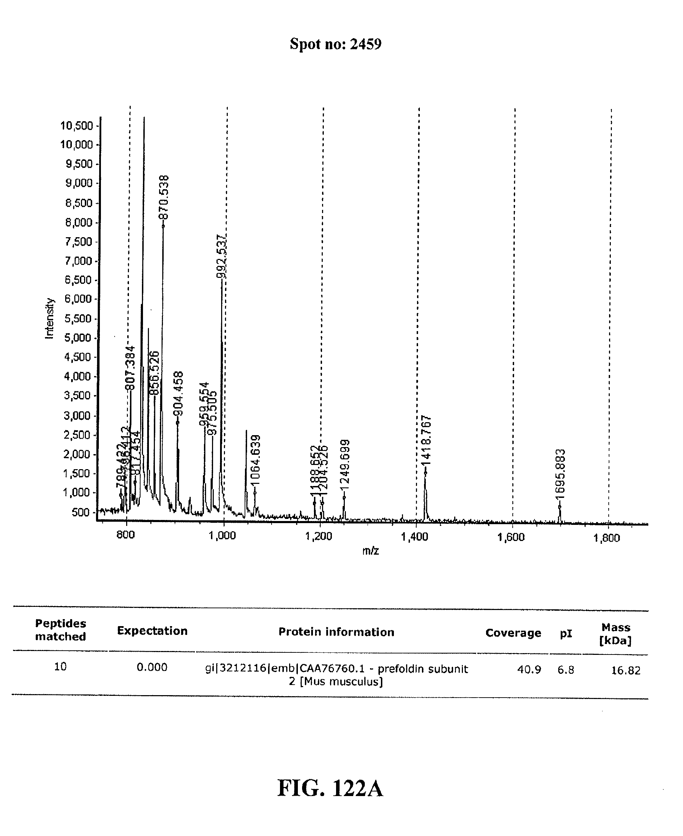

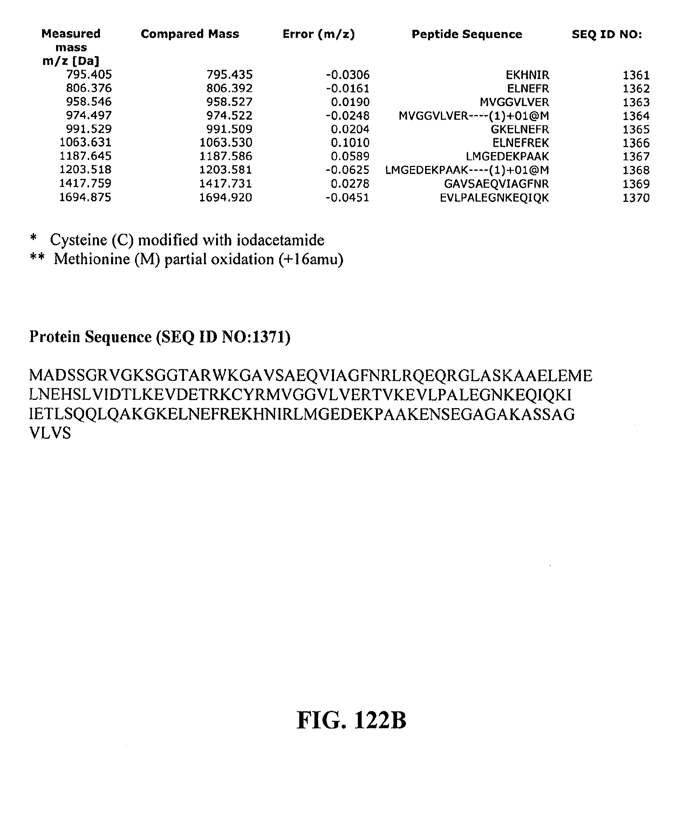

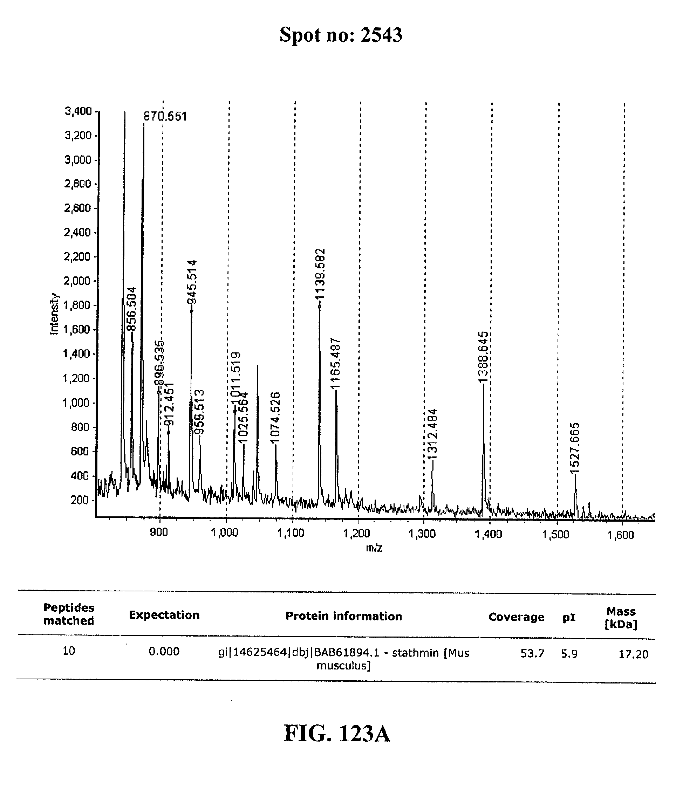

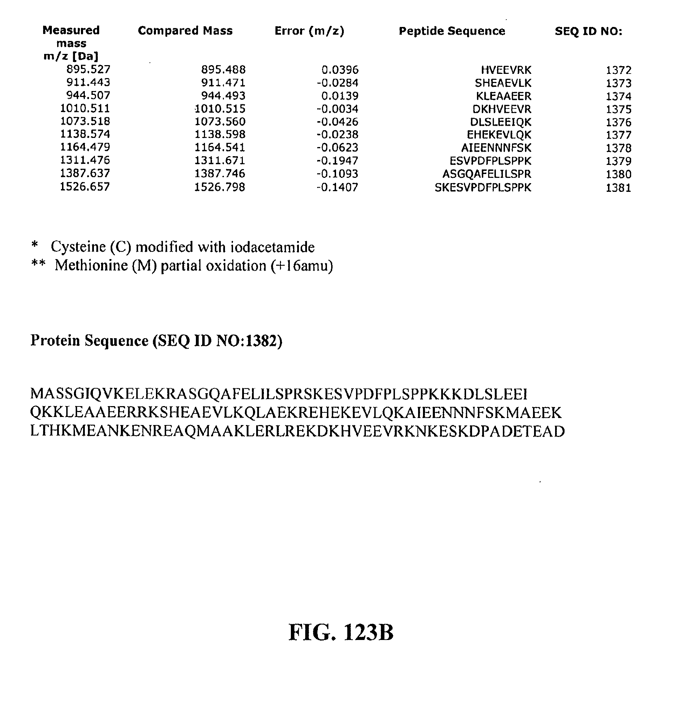

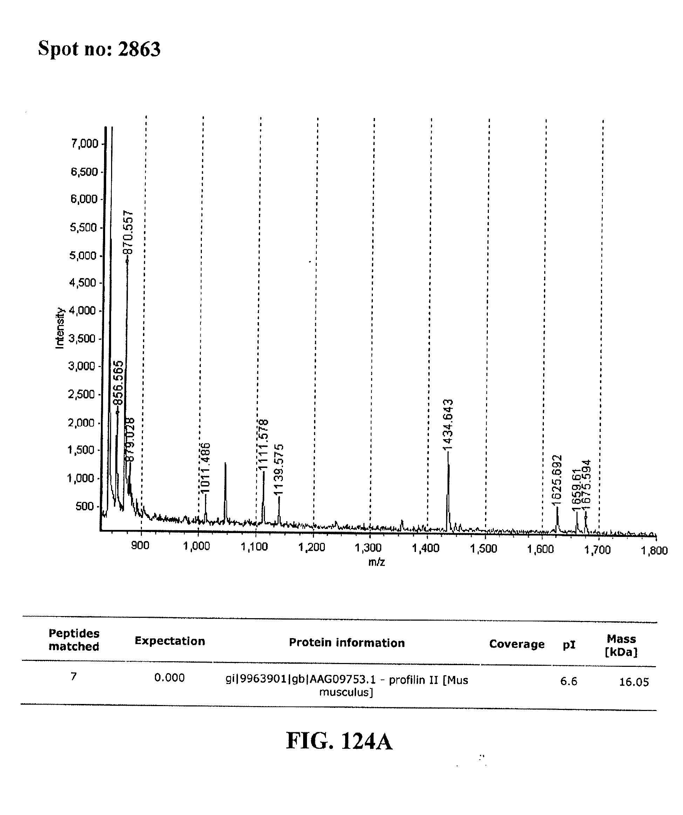

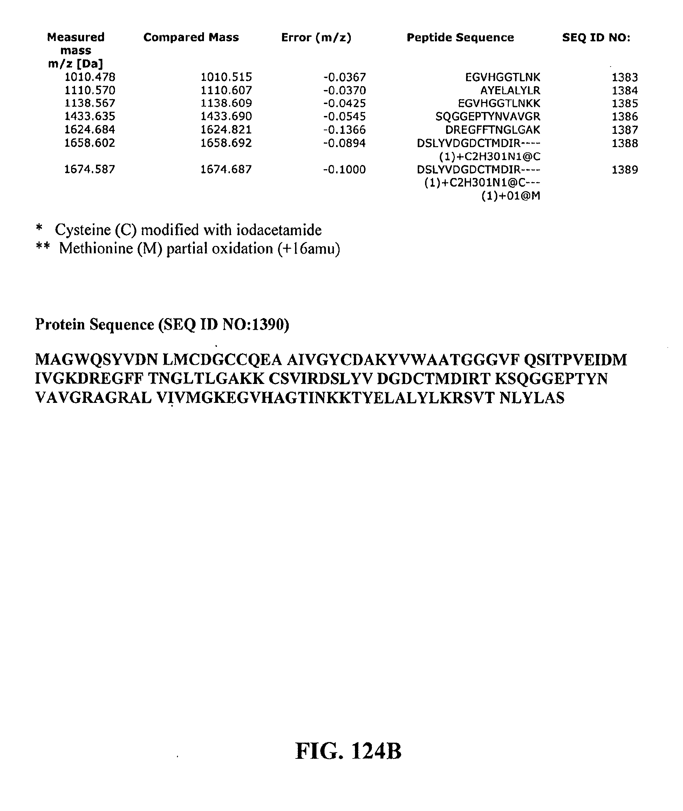

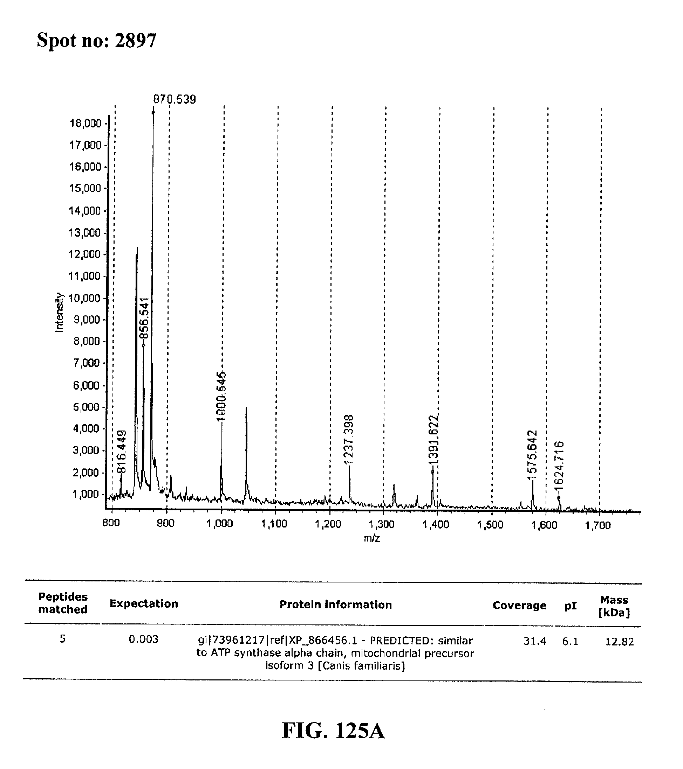

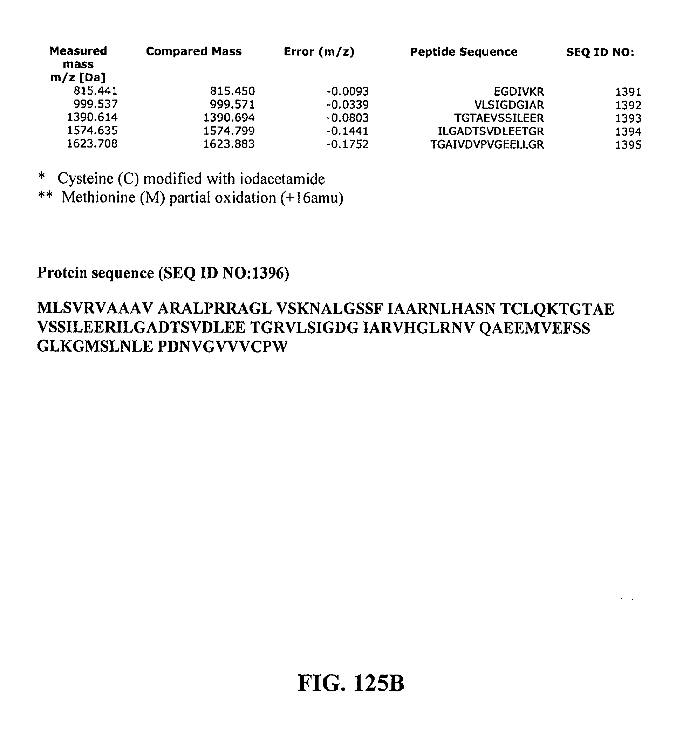

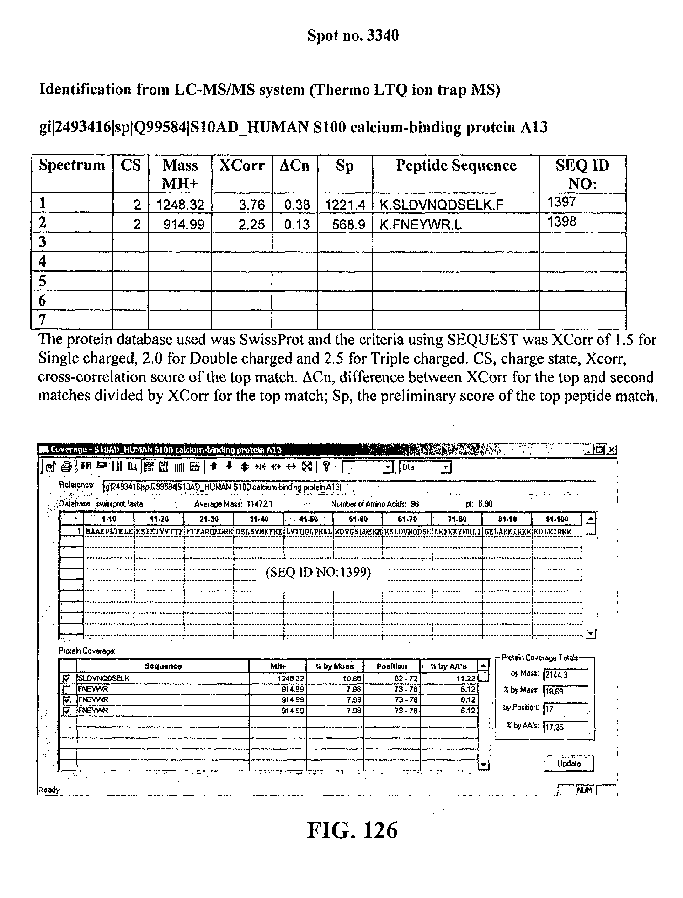

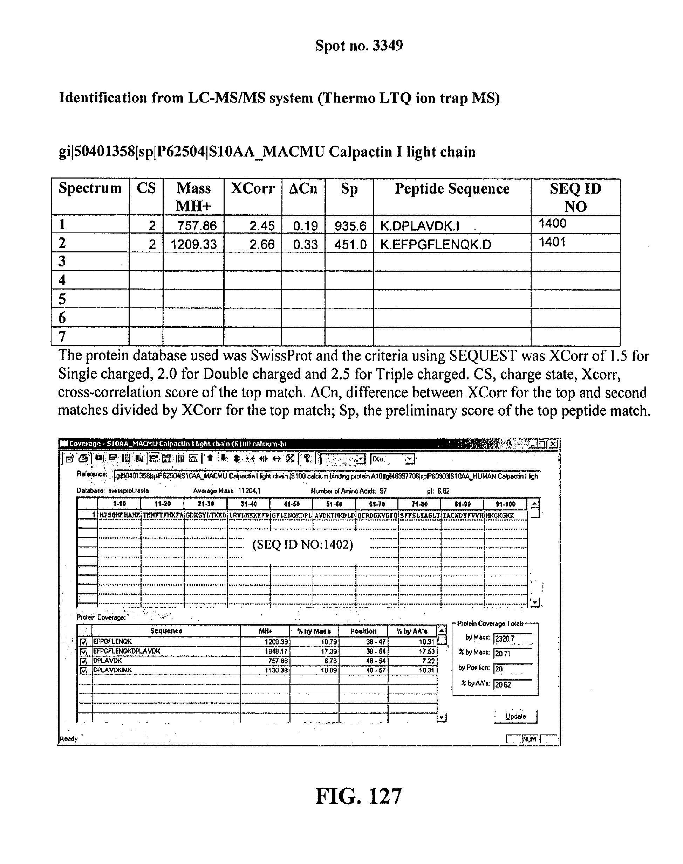

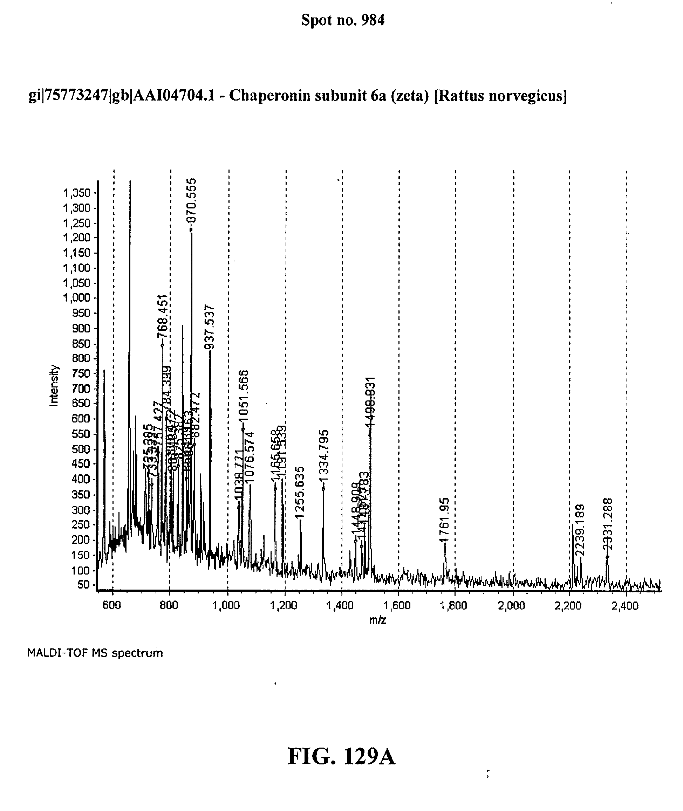

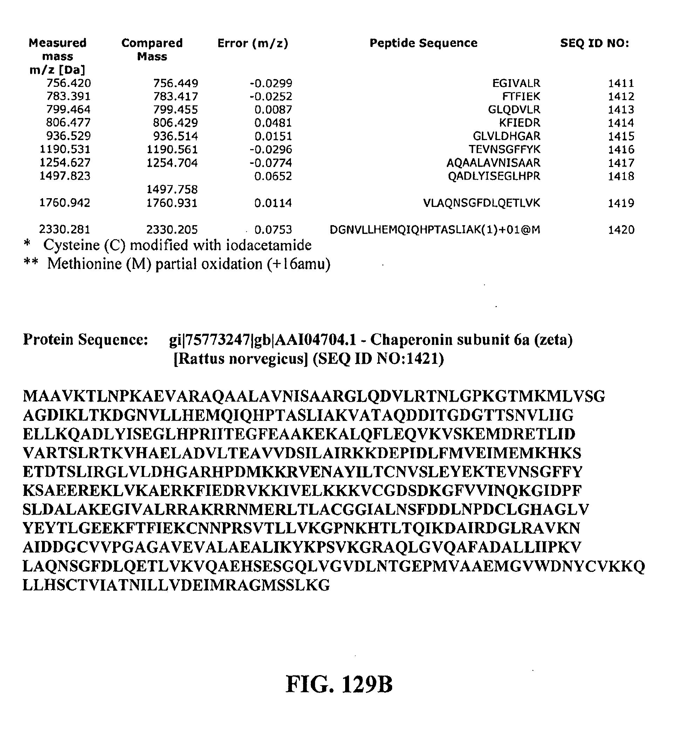

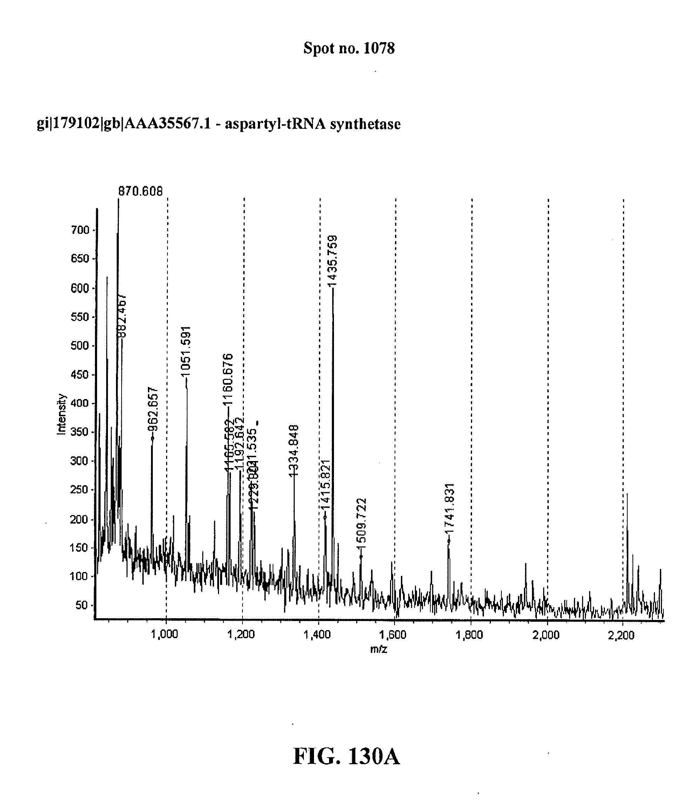

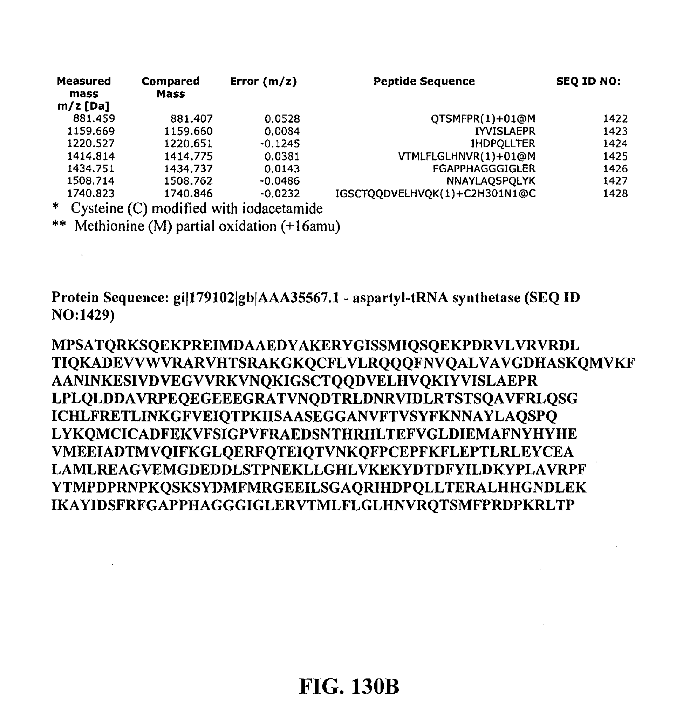

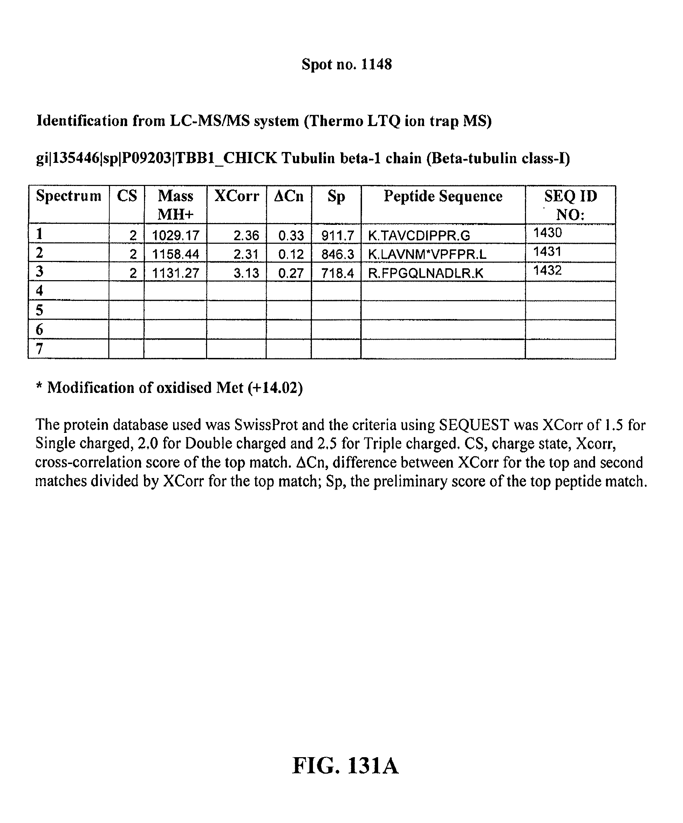

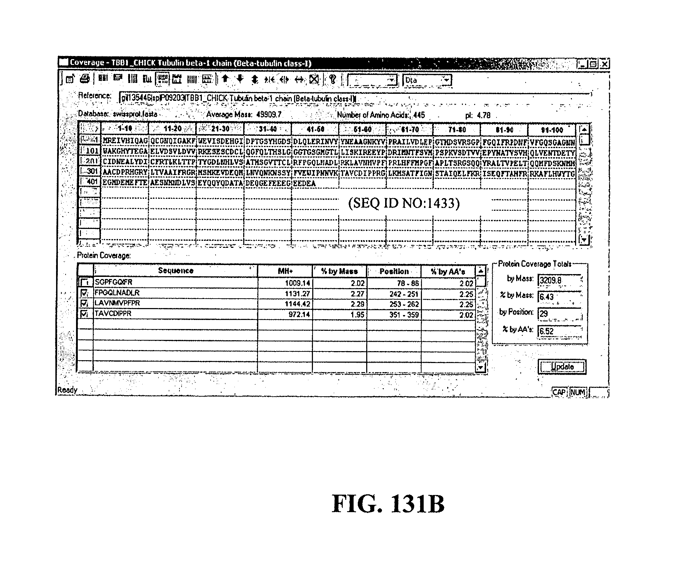

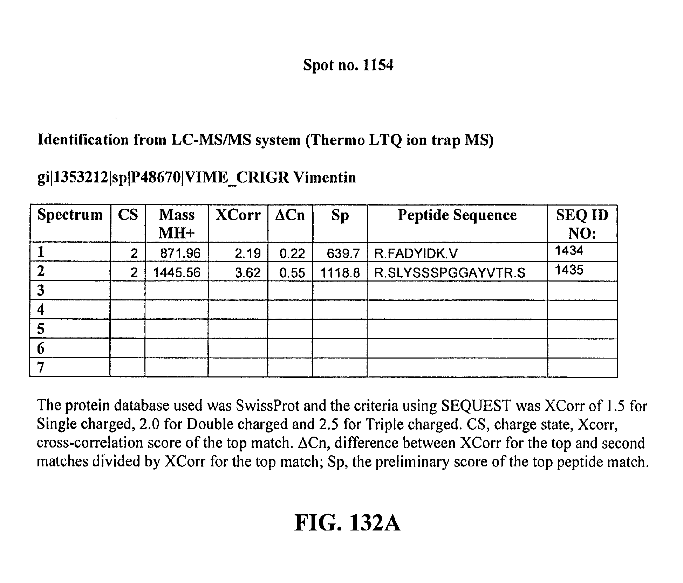

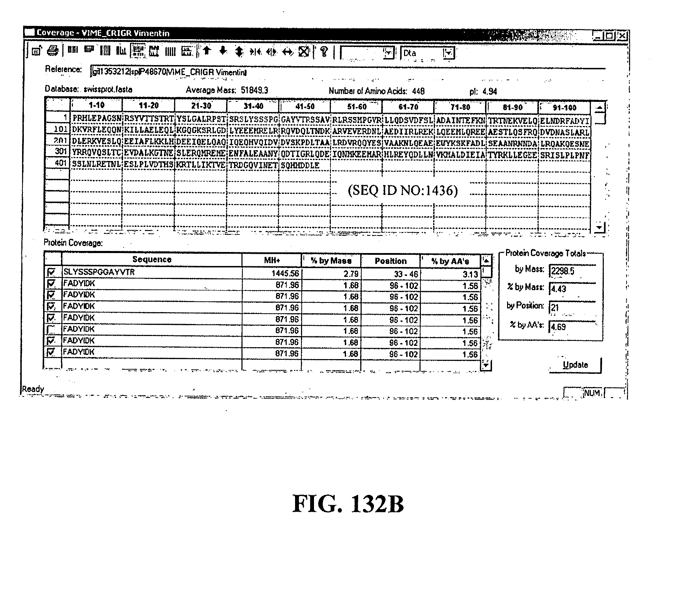

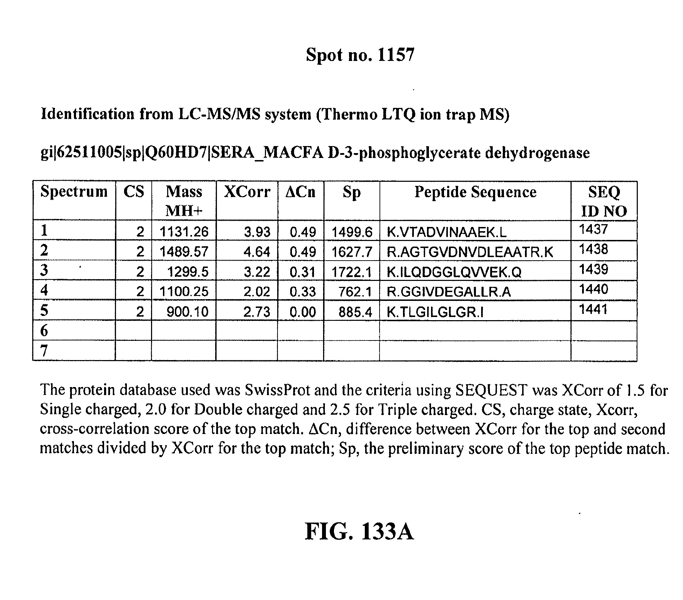

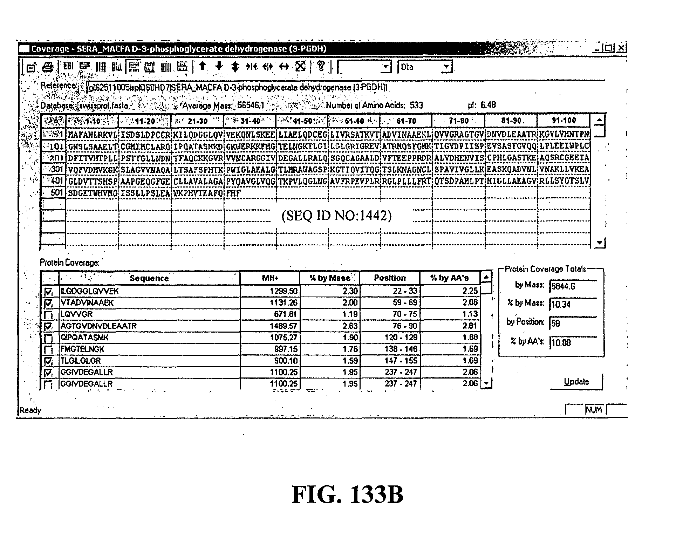

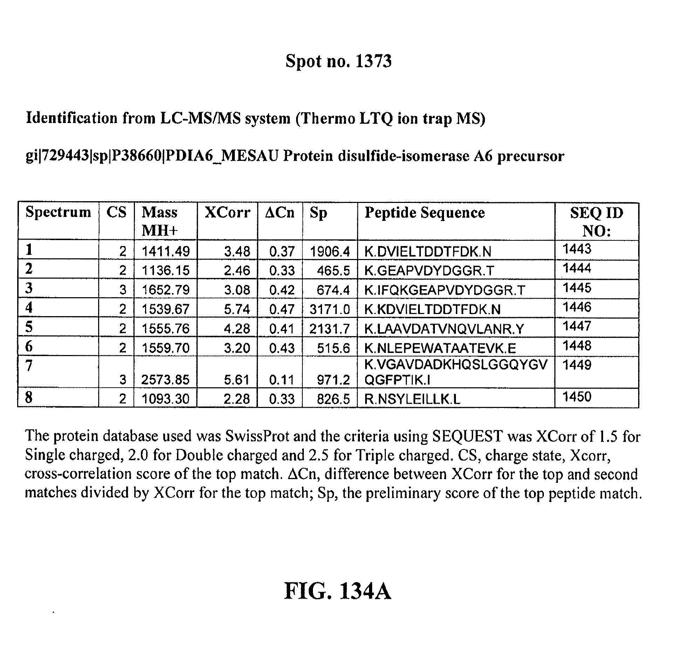

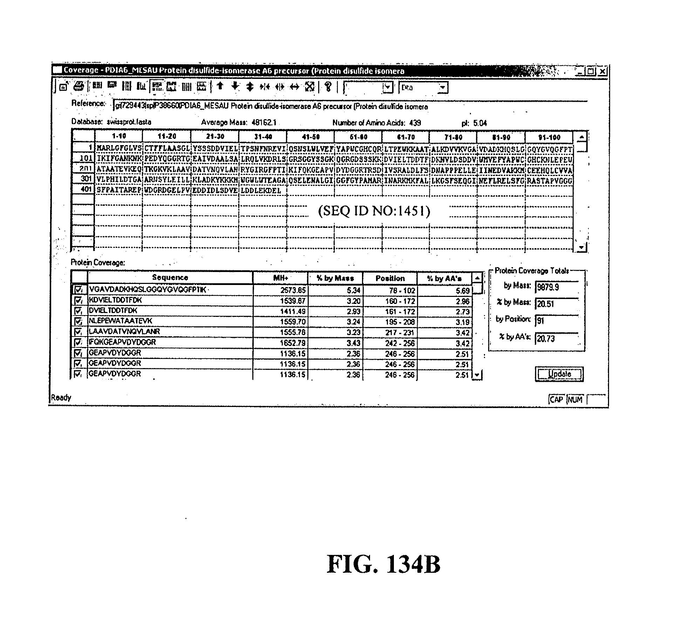

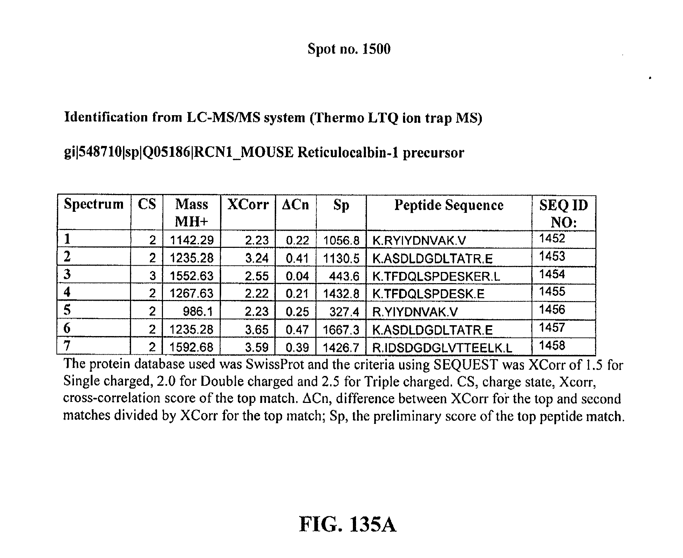

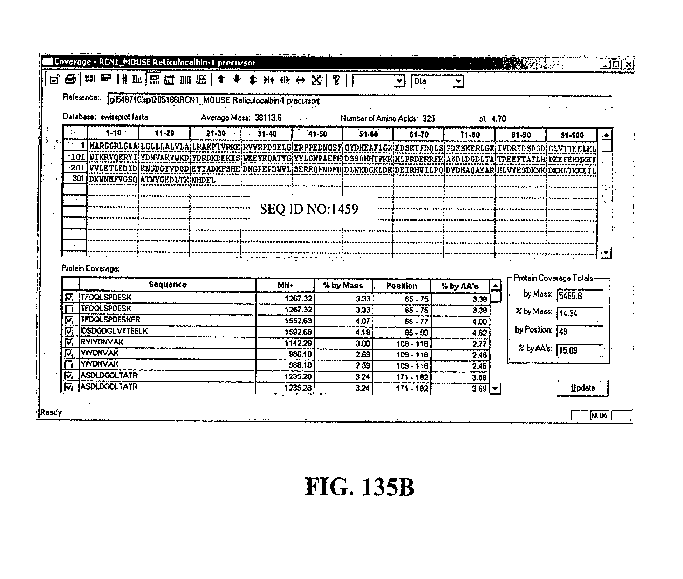

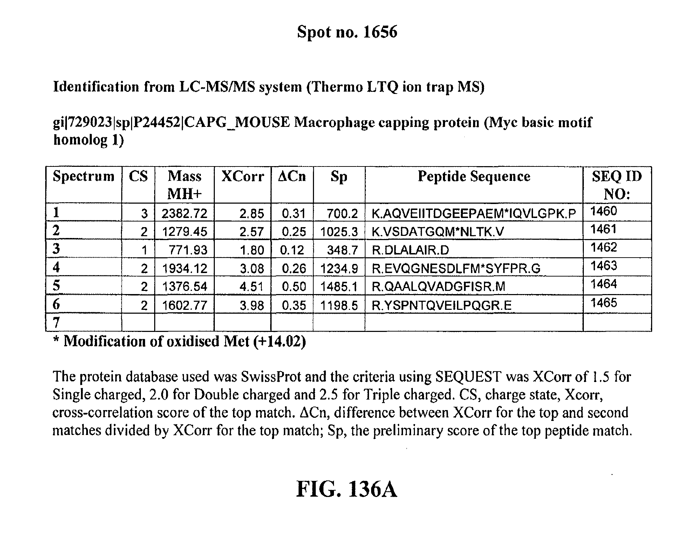

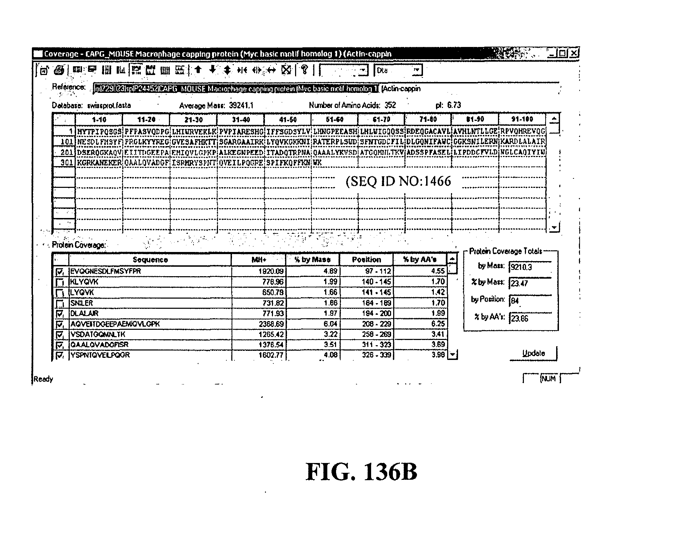

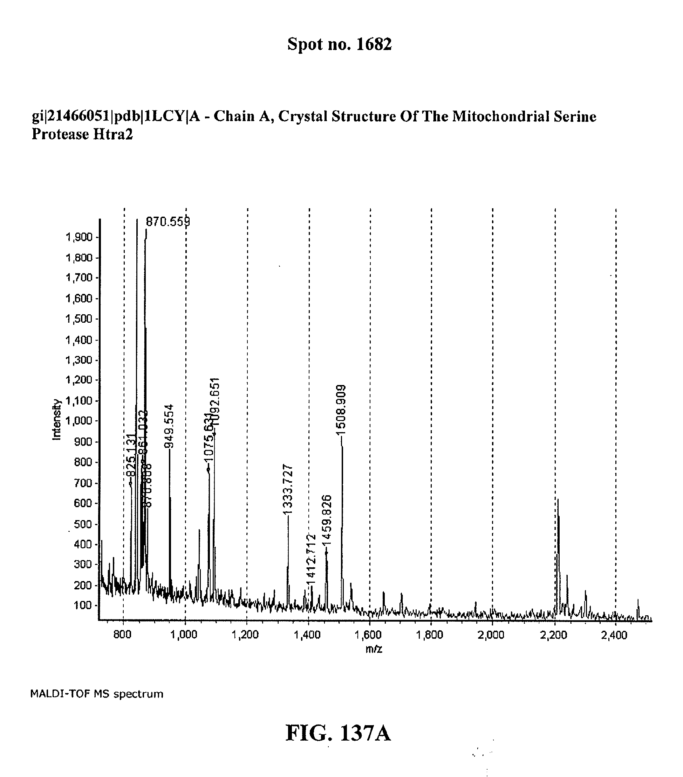

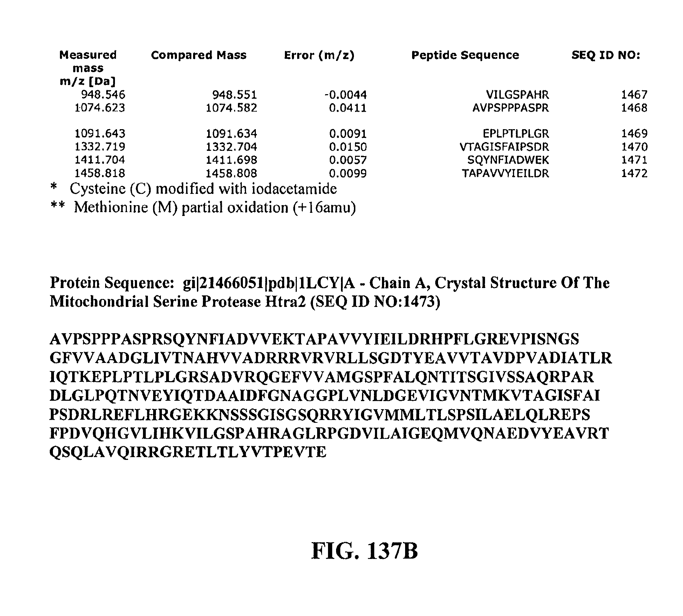

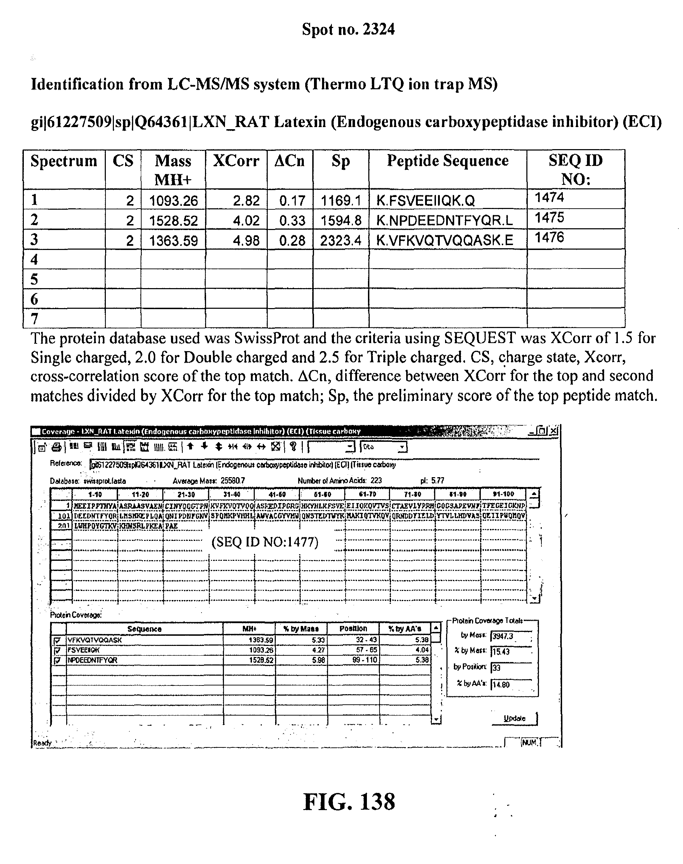

Alternatively, the present invention provides a method of assessing a cell culture phenotype of a cell line. The method including detecting, in a sample from the cell culture, one or more markers indicative of the cell culture phenotype, wherein the markers are selected from the group consisting of peptides selected from Figures 7 through 138, or genes or proteins selected from Tables I through 20 and Tables 24 through 30.

In another aspect, the present invention provides an engineered cell line with an improved cell culture phenotype containing a population of engineered cells, each of which comprises an engineered construct up-regulating or down-regulating one or more proteins identified according to various methods as described above. In particular, the present invention provides an engineered cell line with an improved cellular productivity containing a population of engineered cells, each of which comprises an engineered construct up-regulating or down-regulating one or more genes or proteins selected from Tables 2, 3, and 9 through 12. In some embodiments, the engineered construct is an over-expression construct. In other embodiments, the engineered construct is an interfering RNA construct.

In other embodiments, the present invention provides an engineered cell line with an improved cell growth rate including a population of engineered cells, each of which includes an engineered construct up-regulating or down-regulating one or more genes or proteins selected from Tables 4, 5, 6, 13, 14, 27 and 28. In some embodiments, the engineered construct is an over-expression construct. In other embodiments, the engineered construct is an interfering RNA construct.

In other embodiments, the present invention provides an engineered cell line with an improved peak cell density containing a population of engineered cells, each of which includes an engineered construct up-regulating or down-regulating one or more genes or proteins selected from Tables 8, 15, 16, and 17. In some embodiments, the engineered construct is an over-expression construct. In other embodiments, the engineered construct is an interfering RNA construct.

In other embodiments, the present invention provides an engineered cell line with an improved sustained cell viability containing a population of engineered cells, each of which comprising an engineered construct up-regulating or down-regulating one or more genes or proteins selected from Tables 18 and 26. In some embodiments, the engineered construct is an over-expression construct. In other embodiments, the engineered construct is an interfering RNA construct.

In other embodiments, the present invention provides an engineered cell line with regulated lactate production or consumption containing a population of engineered cells, each of which comprising an engineered construct up-regulating or down-regulating one or more genes or proteins selected from Tables 29 and 30. In some embodiments, the engineered construct is an over-expression construct. In other embodiments, the engineered construct is an interfering RNA construct.

In some embodiments, the present invention provides an improved cell line containing a population of engineered cells, each of which comprising an engineered construct up-regulating or down-regulating one or more genes or proteins selected from Table 20, 24, 25 and 26. In some embodiments, the engineered construct is an over-expression construct. In other embodiments, the engineered construct is an interfering RNA construct.

In yet another aspect, the invention provides a method for expression of a protein of interest using engineered cell lines as described above. The method includes the steps of introducing into an engineered cell line according to any one of the embodiments described above a nucleic acid encoding the protein of interest; and harvesting the protein of interest.

In still another aspect, the invention also provides isolated genes or proteins, or polynucleotides or polypeptides that are of previously undiscovered genes or proteins, and/or are involved with regulating or indicative of cell culture phenotypes of interest. In particular, the invention provides an isolated or recombinant nucleic acid containing a sequence selected from Tables 9, 13, and 15, complements thereof, and subsequences thereof. The present invention also provides an isolated or recombinant protein containing a sequence selected from Tables 2 and 3, or fragments thereof. The invention also provides genetically engineered expression vectors, host cells, and transgenic animals comprising the nucleic acid molecules or proteins of the invention. The invention additionally provides inhibitory polynucleotides, e.g., antisense and RNA interference (RNAi) molecules, to the nucleic acid molecules of the invention or the nucleic acid encoding the proteins of the invention.

Other features, objects, and advantages of the present invention are apparent in the detailed description that follows. It should be understood, however, that the detailed description, while indicating embodiments of the present invention, is given by way of illustration only, not limitation. Various changes and modifications within the scope of the invention will become apparent to those skilled in the art from the detailed description.

### BRIEF DESCRIPTION OF THE DRAWINGS

Figure 1 is a flowchart of an exemplary method for identifying genes and proteins of the invention.
Figure 2 illustrates an exemplary matrix of CHO lines and cellular phenotypes.
Figure 3 depicts an exemplary phenotypic comparison between test cell lines and control cell lines for a "high cell growth rate" phenotype.
Figure 4 illustrates a method of protein expression profiling.
Figures 5 and 6 depict the Cy3 and Cy5 staining patterns on an exemplary gel and provide graphical depictions of the relative abundance of selected proteins. In Figure 5, a protein that appears to be 5-fold upregulated in the Cy5-labeled test cell extract is outlined. In Figure 6, a protein that appears to be 4-fold downregulated in the Cy5-labeled test cell extract is outlined.
Figures 7 through 138 illustrate sequence data and analysis for individual, differentially-expressed proteins.
Figures 139 and 140 schematically depict an unsupervised Pearson Clustering Analysis.
Figure 141 depicts an exemplary method of data analysis using pairwise differences.
Figure 142 depicts an exemplary method of data analysis that does not rely on pairwise differences.
Figures 143-146 depict exemplary evaluations of identified genes in the 3C7 cell line.
Figures 147 and 148 illustrate a 24 well format for assessing the impact of over-expression of identified genes on cellular growth and productivity.

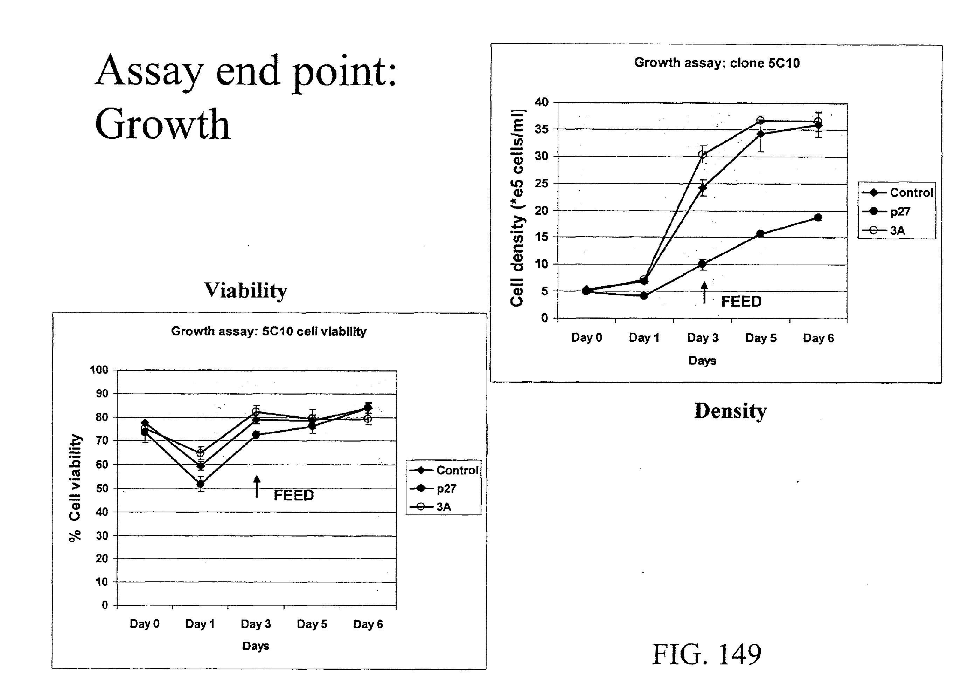

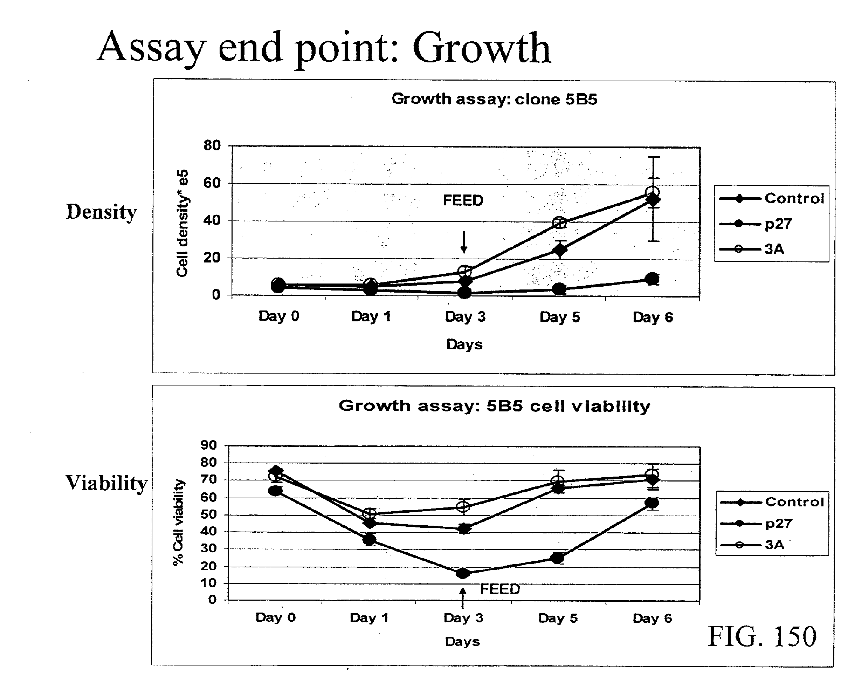

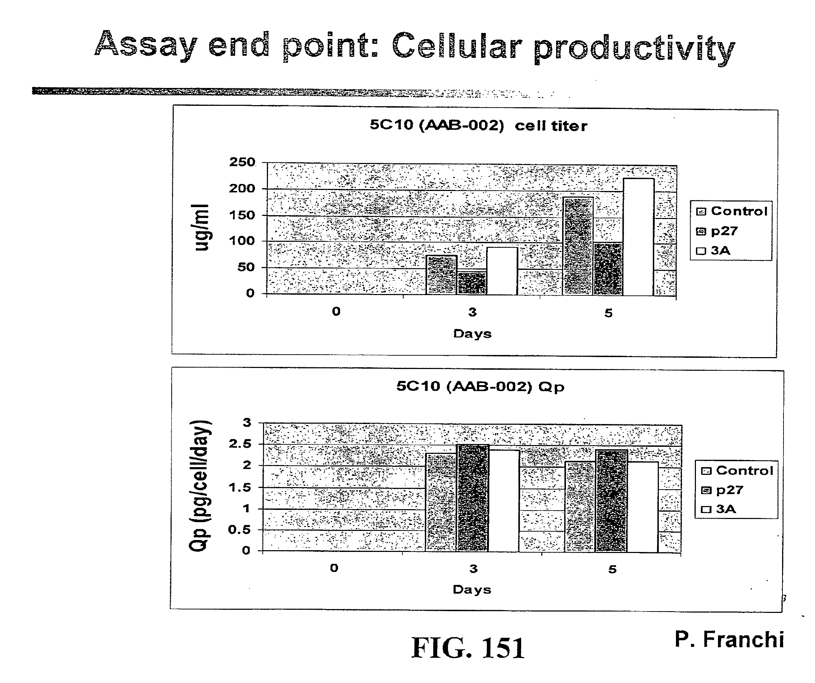

Exemplary results of over-expression of identified genes on cellular growth and productivity are illustrated in Figures 149-151.

### DETAILED DESCRIPTION OF THE INVENTION

The present invention provides systematic methods for identifying genes and proteins that influence cell culture phenotypes of interest. The methods of the invention are based on differential expression profiling analysis of industrially relevant cell culture phenotypes through integrated use of DNA microarray and proteomics analysis. Specifically, the method includes generating a gene or protein expression profile of a sample derived from a test cell line; comparing the gene or protein expression profile to a control profile derived from a control cell line which has a cell culture phenotype distinct from that of the test cell line; and identifying one or more differentially expressed genes or proteins based on the comparison. As used herein, the test cell line and the control cell line can be different cell lines with different genetic background or same cell line grown under different cell culture conditions.

The one or more differentially expressed genes or proteins are candidate genes or proteins that regulate or are indicative of the cell culture phenotype of interest. The identified genes and proteins can be further confirmed and validated. The identified genes or proteins may also be manipulated to improve the cell culture phenotype of interest. Therefore, the present invention represents a significant advance in cell engineering for rational designing of improved cell lines and cell culture conditions.

Various aspects of the invention are described in further detail in the following subsections. The use of subsections is not meant to limit the invention. Each subsection may apply to any aspect of the invention. In this application, the use of "or" means "and/or" unless stated otherwise.

### Cell lines and cell culture phenotypes

The present invention contemplates differential expression profiling analysis and optimization of cell lines derived from a variety of organisms, including, but not limited to, bacteria, plants, fungi, and animals (the latter including, but not limited to, insects and mammals). For example, the present invention may be applied to *Escherichia coli, Spodoptera frugiperda, Nicotiana sp., Zea mays, Lemna sp., Saccharomyces sp., Pichia sp., Schizosaccharomyces sp.,* mammalian cells, including, but not limted to, COS cells, CHO cells, 293 cells, A431 cells, 3T3 cells, CV-1 cells, HeLa cells, L cells, BHK21 cells, HL-60 cells, U937 cells, HEK cells, PerC6 cells, Jurkat cells, normal diploid cells, cell strains derived from in vitro culture of primary tissue, and primary explants. The list of organisms and cell lines are meant only to provide nonlimiting examples.

In particular, the present invention contemplates differential expression profiling analysis of industrially relevant cell lines, such as, for example, CHO cells. CHO cells are a primary host for therapeutic protein production, such as, for example, monoclonal antibody production, receptor productions, and Fc fusion proteins because CHO cells provide fidelity of folding, processing, and glycosylation. CHO cells are also compatible with deep-tank, serum-free culture and have excellent safety records.

The present invention permits an understanding of pathways, genes and proteins that influence desired cell culture phenotypes or characteristics, for example, cell phenotypes that enable highly productive fed-batch processes. Such desired cell phenotypes include, but are not limited to, high cell growth rate, high peak cell density, sustained high cell viability, high maximum cellular productivity, sustained high cellular productivity, low ammonium production, and low lactate production. Desired phenotypes or characteristics may be inherent properties of established cell lines that have certain genomic backgrounds. Desired phenotypes or characteristics may also be conferred to cells by growing the cells in different conditions, e.g., temperatures, cell densities, the use of agents such as sodium butyrate, to be in different kinetic phases of growth (*e.g.*, lag phase, exponential growth phase, stationary phase or death phase), and/or to become serum-independent, *etc.* During the period in which these phenotypes are induced, and/or after these phenotypes are achieved, a pool of target nucleic acid or protein samples can be prepared from the cells and analyzed with the oligonucleotide array to determine and identify which genes demonstrate altered expression in response to a particular stimulus (*e.g.*, temperature, sodium butyrate), and therefore are potentially involved in conferring the desired phenotype or characteristic.

### Preparation of pool of target nucleic acids

In order to conduct gene expression profiling analysis, a pool of target nucleic acids are prepared from a sample derived from a cell line. Any biological sample may be used as a source of target nucleic acids. The pool of target nucleic acids can be total RNA, or any nucleic acid derived therefrom, including each of the single strands of cDNA made by reverse transcription of the mRNA, or RNA transcribed from the double-stranded cDNA intermediate. Methods of isolating target nucleic acids for analysis with an oligonucleotide array or other probes, such as phenol-chloroform extraction, ethanol precipitation, magnetic bead separation, or silica-gel affinity purification, are well known to one of skill in the art.

For example, various methods are available for isolating or enriching RNA. These methods include, but are not limited to, RNeasy kits (provided by Qiagen), MasterPure kits (provided by Epicentre Technologies), charge-switch technology (*see*, *e.g.*, U.S. Published patent application Nos. 2003/0054395 and 2003/0130499), and TRIZOL (provided by Gibco BRL). The RNA isolation protocols provided by Affymetrix can also be employed in the present invention. See, *e.g.*, GeneChip® EXPRESSION ANALYSIS TECHNICAL MANUAL (701021 rev. 3, Affymetrix, Inc. 2002).

Preferably, the pool of target nucleic acids (*i.e.*, mRNA or nucleic acids derived therefrom) should reflect the transcription of gene coding regions. In one example, mRNA is enriched by removing rRNA. Different methods are available for eliminating or reducing the amount of rRNA in a sample. For instance, rRNA can be removed by enzyme digestions. According to the latter method, rRNAs are first amplified using reverse transcriptase and specific primers to produce cDNA. The rRNA is allowed to anneal with the cDNA. The sample is then treated with RNAase H, which specifically digests RNA within an RNA:DNA hybrid.

Target nucleic acids may be amplified before incubation with an oligonucleotide array or other probes. Suitable amplification methods, including, but not limited to, reverse transcription-polymerase chain reaction, ligase chain reaction, self-sustained sequence replication, and in vitro transcription, are well known in the art. It should be noted that oligonucleotide probes are chosen to be complementary to target nucleic acids. Therefore, if an antisense pool of target nucleic acids is provided (as is often the case when target nucleic acids are amplified by *in vitro* transcription), the oligonucleotide probes should correspond with subsequences of the sense complement. Conversely, if the pool of target nucleic acids is sense, the oligonucleotide array should be complementary (*i.e.*, antisense) to them. Finally, if target nucleic acids are double-stranded, oligonucleotide probes can be sense or antisense.

The present invention involves detecting the hybridization intensity between target nucleic acids and complementary oligonucleotide probes. To accomplish this, target nucleic acids may be attached directly or indirectly with appropriate and detectable labels. Direct labels are detectable labels that are directly attached to or incorporated into target nucleic acids. Indirect labels are attached to polynucleotides after hybridization, often by attaching to a binding moiety that was attached to the target nucleic acids prior to hybridization. Such direct and indirect labels are well known in the art. In a preferred embodiment of the invention, target nucleic acids are detected using the biotin-streptavidin-PE coupling system, where biotin is incorporated into target nucleic acids and hybridization is detected by the binding of streptavidin-PE to biotin.

Target nucleic acids may be labeled before, during or after incubation with an oligonucleotide array. Preferably, the target nucleic acids are labeled before incubation. Labels may be incorporated during the amplification step by using nucleotides that are already labeled (e.g., biotin-coupled dUTP or dCTP) in the reaction. Alternatively, a label may be added directly to the original nucleic acid sample (*e.g.*, mRNA, cDNA) or to the amplification product after the amplification is completed. Means of attaching labels to nucleic acids are well known to those of skill in the art and include, but are not limited to, nick translation, end-labeling, and ligation of target nucleic acids to a nucleic acid linker to join it to a label. Alternatively, several kits specifically designed for isolating and preparing target nucleic acids for microarray analysis are commercially available, including, but not limited to, the GeneChip^{®} IVT Labeling Kit (Affymetrix, Santa Clara, Calif.) and the Bioarray™ High Yield™ RNA Transcript Labeling Kit with Fluorescein-UTP for Nucleic Acid Arrays (Enzo Life Sciences, Inc., Farmingdale, N.Y.).

Polynucleotides can be fragmented before being labeled with detectable moieties. Exemplary methods for fragmentation include, but are not limited to, heat or ion-mediated hydrolysis.

### Oligonucleotide arrays

Probes suitable for the present invention includes oligonucleotide arrays or other probes that capable of detecting the expression of a plurality of genes (including previously undiscovered genes) by a cell (or cell line), including known cells or cells derived from an unsequenced organism, and to identify genes (including previously undiscovered genes) and related pathways that may be involved with the induction of a particular cell phenotype, *e.g.*, increased and efficient transgene expression.

Oligonucleotide probes used in this invention may be nucleotide polymers or analogs and modified forms thereof such that hybridizing to a pool of target nucleic acids occurs in a sequence specific manner under oligonucleotide array hybridization conditions. As used herein, the term "oligonucleotide array hybridization conditions" refers to the temperature and ionic conditions that are normally used in oligonucleotide array hybridization. In many examples, these conditions include 16-hour hybridization at 45°C., followed by at least three 10-minute washes at room temperature. The hybridization buffer comprises 100 mM MES, 1 M [Na⁺], 20 mM EDTA, and 0.01% Tween 20. The pH of the hybridization buffer can range between 6.5 and 6.7. The wash buffer is 6XSSPET, which contains 0.9 M NaCl, 60 mM NaH2PO4, 6 mM EDTA, and 0.005% Triton X-100. Under more stringent oligonucleotide array hybridization conditions, the wash buffer can contain 100 mM MES, 0.1 M [Na⁺], and 0.0 1 % Tween 20. *See* also GENECHIP® EXPRESSION ANALYSIS TECHNICAL MANUAL (701021 rev. 3, Affymetrix, Inc. 2002), which is incorporated herein by reference in its entirety.

As is known by one of skill in the art, oligonucleotide probes can be of any length. Preferably, oligonucleotide probes suitable for the invention are 20 to 70 nucleotides in length. Most preferably, suitable oligonucleotide probes are 25 nucleotides in length. In one embodiment, the nucleic acid probes of the present invention have relatively high sequence complexity. In many examples, the probes do not contain long stretches of the same nucleotide. In addition, the probes may be designed such that they do not have a high proportion of G or C residues at the 3' ends. In another embodiment, the probes do not have a 3' terminal T residue. Depending on the type of assay or detection to be performed, sequences that are predicted to form hairpins or interstrand structures, such as "primer dimers," can be either included in or excluded from the probe sequences. In many embodiments, each probe employed in the present invention does not contain any ambiguous base.

Oligonucleotide probes are made to be specific for (e.g., complementary to (i.e., capable of hybridizing to)) a template sequence. Any part of a template sequence can be used to prepare probes. Multiple probes, *e.g.*, 5, 10, 15, 20, 25, 30, or more, can be prepared for each template sequence. These multiple probes may or may not overlap each other. Overlap among different probes may be desirable in some assays. In many embodiments, the probes for a template sequence have low sequence identities with other template sequences, or the complements thereof. For instance, each probe for a template sequence can have no more than 70%, 60%, 50% or less sequence identity with other template sequences, or the complements thereof. This reduces the risk of undesired cross-hybridization. Sequence identity can be determined using methods known in the art. These methods include, but are not limited to, BLASTN, FASTA, and FASTDB. The Genetics Computer Group (GCG) program, which is a suite of programs including BLASTN and FASTA, can also be used. Preferable sequences for template sequences include, but are not limited to, consensus sequences, transgene sequences, and control sequences (*i.e.*, sequences used to control or normalize for variation between experiments, samples, stringency requirements, and target nucleic acid preparations). Additionally, any subsequence of consensus, transgene and control sequences can be used as a template sequence.

In one embodiment, only certain regions (*i.e.*, tiling regions) of consensus, transgene and control sequences are used as template sequences for the oligonucleotide probes used in this invention. One of skill in the art will recognize that protocols that may be used in practicing the invention, *e.g.*, *in vitro* transcription protocols, often result in a bias toward the 3'-ends of target nucleic acids. Consequently, in one embodiment of the invention, the region of the consensus sequence or transgene sequence closest to the 3'-end of a consensus sequence is most often used as a template for oligonucleotide probes. Generally, if a poly-A signal could be identified, the 1400 nucleotides immediately prior to the end of the consensus or transgene sequences are designated as a tiling region. Alternatively, if a poly-A signal could not be identified, only the last 600 nucleotides of the consensus or transgene sequence are designated as a tiling region. However, it should be noted that the invention is not limited to using only these tiling regions within the consensus, transgene and control sequences as templates for the oligonucleotide probes. Indeed, a tiling region may occur anywhere within the consensus, transgene or control sequences. For example, the tiling region of a control sequence may comprise regions from both the 5' and 3'-ends of the control sequence. In fact, the entire consensus, transgene or control sequence may be used as a template for oligonucleotide probes.

An oligonucleotide array suitable for the invention may include perfect match probes to a plurality of consensus sequences (*i.e.*, consensus sequences for multi-sequence clusters, and consensus sequences for exemplar sequences) identified as described above. The oligonucleotide array suitable for the invention may also include perfect match probes to both consensus and transgene sequences. It will be apparent to one of skill in the art that inclusion of oligonucleotide probes to transgene sequences will be useful when a cell line is genetically engineered to express a recombinant protein encoded by a transgene sequence, and the purpose of the analysis is to confirm expression of the transgene and determine the level of such expression. In those cases where the transgene is linked in a bicistronic mRNA to a downstream ORF, such as dihydrofolate reductase (DHFR), the level of transgene expression may also be determined from the level of expression of the downstream sequence. In another embodiment of the invention, the oligonucleotide array further comprises control probes that normalize the inherent variation between experiments, samples, stringency requirements, and preparations of target nucleic acids. Exemplary compositions of each of these types of control probes is described in U.S. Pat. No. 6,040,138 and in U.S. Publication No. 20060010513, the teachings of both of which are incorporated herein in their entirety by reference.

It is well known to one of skill in the art that two pools of target nucleic acids individually processed from the same sample can hybridize to two separate but identical oligonucleotide arrays with varying results. The varying results between these arrays are attributed to several factors, such as the intensity of the labeled pool of target nucleic acids and incubation conditions. To control for these variations, normalization control probes can be added to the array. Normalization control probes are oligonucleotides exactly complementary to known nucleic acid sequences spiked into the pool of target nucleic acids. Any oligonucleotide sequence may serve as a normalization control probe. For example, the normalization control probes may be created from a template obtained from an organism other than that from which the cell line being analyzed is derived. In one embodiment, an oligonucleotide array to mammalian sequences will contain normalization oligonucleotide probes to the following genes: *bio*B, *bio*C, and *bio*D from the organism *Escherichia coli*, *cre* from the organism Bacteriophage PI, and *dap* from the organism *Bacillus subtilis*, or subsequences thereof. The signal intensity received from the normalization control probes are then used to normalize the signal intensities from all other probes in the array. Additionally, when the known nucleic acid sequences are spiked into the pool of target nucleic acids at known and different concentrations for each transcript, a standard curve correlating signal intensity with transcript concentration can be generated, and expression levels for all transcripts represented on the array can be quantified *(see*, *e.g.*, Hill et al. (2001) Genome Biol. 2(12):research0055.1-0055.13).

Due to the naturally differing metabolic states between cells, expression of specific target nucleic acids vary from sample to sample. In addition, target nucleic acids may be more prone to degradation in one pool compared to another pool. Consequently, in another embodiment of the invention, the oligonucleotide array further comprises oligonucleotide probes that are exactly complementary to constitutively expressed genes, or subsequences thereof, that reflect the metabolic state of a cell. Nonlimiting examples of these types of genes are beta-actin, transferrin receptor and glyceraldehyde-3-phosphate dehydrogenase (GAPDH).

In one embodiment of the invention, the pool of target nucleic acids is derived by converting total RNA isolated from the sample into double-stranded cDNA and transcribing the resulting cDNA into complementary RNA (cRNA) using methods described in U.S. Publication No. 20060010513, the teachings of which are incorporated herein in their entirety by reference. The RNA conversion protocol is started at the 3'-end of the RNA transcript, and if the process is not allowed to go to completion (if, for example, the RNA is nicked, *etc.*) the amount of the 3'-end message compared to the 5'-end message will be greater, resulting in a 3'-bias. Additionally, RNA degradation may start at the 5'-end (Jacobs Anderson et al. (1998) EMBO J. 17:1497-506). The use of these methods suggests that control probes that measure the quality of the processing and the amount of degradation of the sample preferably should be included in the oligonucleotide array. Examples of such control probes are oligonucleotides exactly complementary to 3'- and 5'-ends of constitutively expressed genes, such as beta-actin, transferrin receptor and GAPDH, as mentioned above. The resulting 3' to 5' expression ratio of a constitutively expressed gene is then indicative of the quality of processing and the amount of degradation of the sample; i.e., a 3' to 5' ratio greater than three (3) indicates either incomplete processing or high RNA degradation (Auer et al. (2003) Nat. Genet. 35:292-93). Consequently, in a preferred embodiment of the invention, the oligonucleotide array includes control probes that are complementary to the 3'-and 5'-ends of constitutively expressed genes.

The quality of the pool of target nucleic acids is not only reflected in the processing and degradation of the target nucleic acids, but also in the origin of the target nucleic acids. Contaminating sequences, such as genomic DNA, may interfere with well-known quantification protocols. Consequently, in a preferred embodiment of the invention, the array further comprises oligonucleotide probes exactly complementary to bacterial genes, ribosomal RNAs, and/or genomic intergenic regions to provide a means to control for the quality of the sample preparation; These probes control for the possibility that the pool of target nucleic acids is contaminated with bacterial DNA, non-mRNA species, and genomic DNA. Such exemplary control sequences are disclosed in U.S. Publication No. 20060010513, the teaching of which are incorporated herein in their entirety by reference.

In a preferred embodiment of the invention, the oligonucleotide array further comprises control mismatch oligonucleotide probes for each perfect match probe. The mismatch probes control for hybridization specificity. Preferably, mismatch control probes are identical to their corresponding perfect match probes with the exception of one or more substituted bases. More preferably, the substitution(s) occurs at a central location on the probe. For example, where a perfect match probe is 25 oligonucleotides in length, a corresponding mismatch probe will have the identical length and sequence except for a single-base substitution at position 13 (e.g., substitution of a thymine for an adenine, an adenine for a thymine, a cytosine for a guanine, or a guanine for a cytosine). The presence of one or more mismatch bases in the mismatch oligonucleotide probe disallows target nucleic acids that bind to complementary perfect match probes to bind to corresponding mismatch control probes under appropriate conditions. Therefore, mismatch oligonucleotide probes indicate whether the incubation conditions are optimal, i.e., whether the stringency being utilized provides for target nucleic acids binding to only exactly complementary probes present in the array.

For each template, a set of perfect match probes exactly complementary to subsequences of consensus, transgene, and/or control sequences (or tiling regions thereof) may be chosen using a variety of strategies. It is known to one of skill in the art that each template can provide for a potentially large number of probes. As is known, apparent probes are sometimes not suitable for inclusion in the array. This can be due to the existence of similar subsequences in other regions of the genome, which causes probes directed to these subsequences to cross-hybridize and give false signals. Another reason some apparent probes may not be suitable for inclusion in the array is because they may form secondary structures that prevent efficient hybridization. Finally, hybridization of target nucleic acids with (or to) an array comprising a large number of probes requires that each of the probes hybridizes to its specific target nucleic acid sequence under the same incubation conditions.

An oligonucleotide array may comprise one perfect match probe for a consensus, transgene, or control sequence, or may comprise a probeset (i.e., more than one perfect match probe) for a consensus, transgene, or control sequence. For example, an oligonucleotide array may comprise 1, 5, 10, 25, 50, 100, or more than 100 different perfect match probes for a consensus, transgene or control sequence. In a preferred embodiment of the invention, the array comprises at least 11-150 different perfect match oligonucleotide probes exactly complementary to subsequences of each consensus and transgene sequence. In an even more preferred embodiment, only the most optimal probeset for each template is included. The suitability of the probes for hybridization can be evaluated using various computer programs. Suitable programs for this purpose include, but are not limited to, LaserGene (DNAStar), Oligo (National Biosciences, Inc.), MacVector (Kodak/IBI), and the standard programs provided by the GCG. Any method or software program known in the art may be used to prepare probes for the template sequences of the present invention. For example, oligonucleotide probes may be generated by using Array Designer, a software package provided by TeleChem International, Inc (Sunnyvale, Calif.). Another exemplary algorithm for choosing optimal probe sets is described in U.S. Pat. No. 6,040,138, the teachings of which are hereby incorporated by reference. Other suitable means to optimize probesets, which will result in a comparable oligonucleotide array, are well known in the art and may be found in, e.g., Lockhart et al. (1996) Nat. Biotechnol. 14:1675-80 and Mei et al. (2003) Proc. Natl. Acad. Sci. USA 100:11237-42.

The oligonucleotide probes of the present invention can be synthesized using a variety of methods. Examples of these methods include, but are not limited to, the use of automated or high throughput DNA synthesizers, such as those provided by Millipore, GeneMachines, and BioAutomation. In many embodiments, the synthesized probes are substantially free of impurities. In many other embodiments, the probes are substantially free of other contaminants that may hinder the desired functions of the probes. The probes can be purified or concentrated using numerous methods, such as reverse phase chromatography, ethanol precipitation, gel filtration, electrophoresis, or any combination thereof.

More detailed information of making an oligonucleotide array suitable for the present invention and exemplary arrays are disclosed in U.S. Publication No. 20060010513, the disclosures of which are hereby incorporated by reference. As described in U.S. Publication No. 20060010513, a CHO chip microarray suitable for the invention includes 122 array quality control sequences (non-CHO), 732 public hamster sequences, 2835 library-derived CHO sequences, and 22 product/process specific sequences. Additional suitable arrays are described in U.S. Patent No. 6,040,138, the disclosures of which are incorporated by reference.

### Incubation of target nucleic acids with an array to form a hybridization profile

Incubation reactions can be performed in absolute or differential hybridization formats. In the absolute hybridization format, polynucleotides derived from one sample are hybridized to the probes in an oligonucleotide array. Signals detected after the formation of hybridization complexes correlate to the polynucleotide levels in the sample. In the differential hybridization format, polynucleotides derived from two samples are labeled with different labeling moieties. A mixture of these differently labeled polynucleotides is added to an oligonucleotide array. The oligonucleotide array is then examined under conditions in which the emissions from the two different labels are individually detectable. In one embodiment, the fluorophores Cy3 and Cy5 (Amersham Pharmacia Biotech, Piscataway, N.J.) are used as the labeling moieties for the differential hybridization format.

In the present invention, the incubation conditions should be such that target nucleic acids hybridize only to oligonucleotide probes that have a high degree of complementarity. In a preferred embodiment, this is accomplished by incubating the pool of target nucleic acids with an oligonucleotide array under a low stringency condition to ensure hybridization, and then performing washes at successively higher stringencies until the desired level of hybridization specificity is reached. In other embodiments, target nucleic acids are incubated with an array of the invention under stringent or well-known oligonucleotide array hybridization conditions. In many examples, these oligonucleotide array hybridization conditions include 16-hour hybridization at 45 °C., followed by at least three 10-minute washes at room temperature. The hybridization buffer comprises 100 mM MES, 1 M [Na⁺], 20 mM EDTA, and 0.01% Tween 20. The pH of the hybridization buffer can range between 6.5 and 6.7. The wash buffer is 6 X SSPET, which contains 0.9 M NaCl, 60 mM NaH₂PO_{4,} 6 mM EDTA, and 0.005% Triton X-100. Under more stringent oligonucleotide array hybridization conditions, the wash buffer can contain 100 mM MES, 0.1 M [Na⁺], and 0.01% Tween 20. See also GENECHIP® EXPRESSION ANALYSIS TECHNICAL MANUAL (701021 rev. 3, Affymetrix, Inc. 2002), which is incorporated herein by reference in its entirety.

### Differential gene expression profiling analysis

Methods used to detect the hybridization profile of target nucleic acids with oligonucleotide probes are well known in the art. In particular, means of detecting and recording fluorescence of each individual target nucleic acid-oligonucleotide probe hybrid have been well established and are well known in the art, described in, *e.g.*, U.S. Pat. No. 5,631,734, U.S. Publication No. 20060010513, incorporated herein in their entirety by reference. For example, a confocal microscope can be controlled by a computer to automatically detect the hybridization profile of the entire array. Additionally, as a further nonlimiting example, the microscope can be equipped with a phototransducer attached to a data acquisition system to automatically record the fluorescence signal produced by each individual hybrid.

It will be appreciated by one of skill in the art that evaluation of the hybridization profile is dependent on the composition of the array, *i.e.*, which oligonucleotide probes were included for analysis. For example, where the array includes oligonucleotide probes to consensus sequences only, or consensus sequences and transgene sequences only, (*i.e.*, the array does not include control probes to normalize for variation between experiments, samples, stringency requirements, and preparations of target nucleic acids), the hybridization profile is evaluated by measuring the absolute signal intensity of each location on the array. Alternatively, the mean, trimmed mean (*i.e.*, the mean signal intensity of all probes after 2-5% of the probesets with the lowest and highest signal intensities are removed), or median signal intensity of the array may be scaled to a preset target value to generate a scaling factor, which will subsequently be applied to each probeset on the array to generate a normalized expression value for each gene *(see*, *e.g.*, Affymetrix (2000) Expression Analysis Technical Manual, pp. A5-14). Conversely, where the array further comprises control oligonucleotide probes, the resulting hybridization profile is evaluated by normalizing the absolute signal intensity of each location occupied by a test oligonucleotide probe by means of mathematical manipulations with the absolute signal intensity of each location occupied by a control oligonucleotide probe. Typical normalization strategies are well known in the art, and are included, for example, in U.S. Pat. No. 6,040,138 and Hill et al. (2001) Genome Biol. 2(12):research0055.1-0055.13.

Signals gathered from oligonucleotide arrays can be analyzed using commercially available software, such as those provide by Affymetrix or Agilent Technologies. Controls, such as for scan sensitivity, probe labeling and cDNA or cRNA quantitation, may be included in the hybridization experiments. The array hybridization signals can be scaled or normalized before being subjected to further analysis. For instance, the hybridization signal for each probe can be normalized to take into account variations in hybridization intensities when more than one array is used under similar test conditions. Signals for individual target nucleic acids hybridized with complementary probes can also be normalized using the intensities derived from internal normalization controls contained on each array. In addition, genes with relatively consistent expression levels across the samples can be used to normalize the expression levels of other genes.

To identify genes that confer or correlate with a desired phenotype or characteristic, a gene expression profile of a sample derived from a test cell line is compared to a control profile derived from a control cell line that has a cell culture phenotype of interest distinct from that of the test cell line and differentially expressed genes are identified. For example, the method for identifying the genes and related pathways involved in cellular productivity may include the following: 1) growing a first sample of a first cell line with a particular cellular productivity and growing a second sample of a second cell line with a distinct cellular productivity; 2) isolating, processing, and hybridizing total RNA from the first sample to a first oligonucleotide array; 3) isolating, processing, and hybridizing total RNA from the second sample to a second oligonucleotide array; and 4) comparing the resulting hybridization profiles to identify the sequences that are differentially expressed between the first and second samples. Similar methods can be used to identify genes involved in other phenotypes.

Typically, each cell line was represented by at least three biological replicates. Programs known in the art, *e.g.*, GeneExpress 2000 (Gene Logic, Gaithersburg, Md.), were used to analyze the presence or absence of a target sequence and to determine its relative expression level in one cohort of samples (*e.g.*, cell line or condition or time point) compared to another sample cohort. A probeset called present in all replicate samples was considered for further analysis. Generally, fold-change values of 1.2-fold, 1.5-fold or greater were considered statistically significant if the p-values were less than or equal to 0.05.

The identification of differentially expressed genes that correlate with one or more particular cell phenotypes (*e.g.*, cell growth rate, peak cell density, sustained high cell viability, maximum cellular productivity, sustained high cellular productivity, ammonium production or consumption, lactate production or consumption, *etc.*) can lead to the discovery of genes and pathways, including those were previously undiscovered, that regulate or are indicative of the cell phenotypes.

The subsequently identified genes are sequenced and the sequences are blasted against various databases to determine whether they are known genes or unknown genes. If genes are known, pathway analysis can be conducted based on the existing knowledge in the art. Both known and unknown genes are further confirmed or validated by various methods known in the art. For example, the identified genes may be manipulated (*e.g.*, up-regulated or down-regulated) to induce or suppress the particular phenotype by the cells.

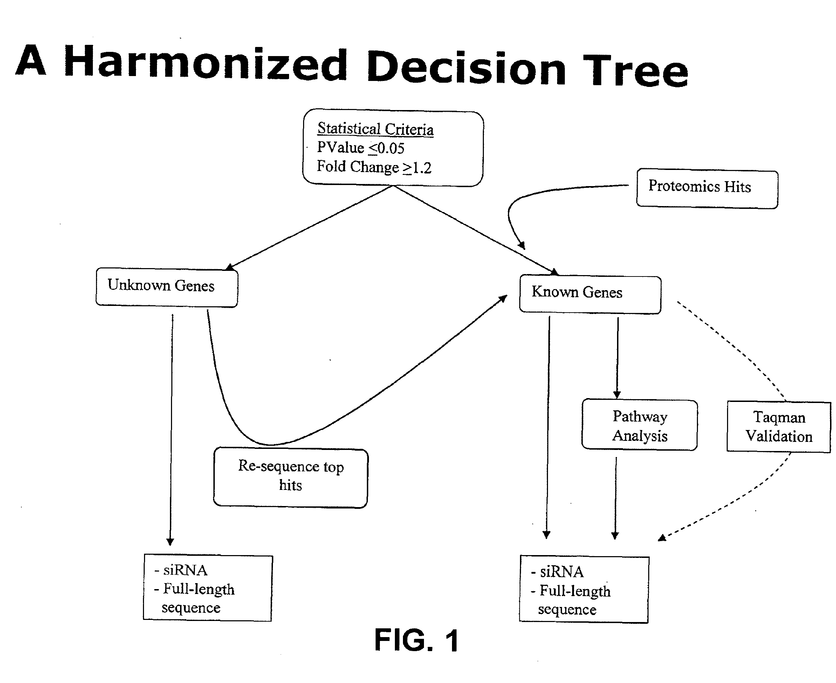

A harmonized decision tree illustrating this process is shown in Figure 1. More detailed identification and validation steps are further described in the Examples and exemplary differentially expressed genes identified using the method of the invention are shown in Tables 9 through 16.

### Differential protein expression profiling analysis

The present invention also provide methods for identifying differentially expressed proteins by protein expression profiling analysis. Protein expression profiles can be generated by any method permitting the resolution and detection of proteins from a sample from a cell line. Methods with higher resolving power are generally preferred, as increased resolution can permit the analysis of greater numbers of individual proteins, increasing the power and usefulness of the profile. A sample can be pre-treated to remove abundant proteins from a sample, such as by immunodepletion, prior to protein resolution and detection, as the presence of an abundant protein may mask more subtle changes in expression of other proteins, particularly for low-abundance proteins. A sample can also be subjected to one or more procedures to reduce the complexity of the sample. For example, chromatography can be used to fractionate a sample; each fraction would have a reduced complexity, facilitating the analysis of the proteins within the fractions.

Three useful methods for simultaneously resolving and detecting several proteins include array-based methods; mass-spectrometry based methods; and two-dimensional gel electrophoresis based methods.

Protein arrays generally involve a significant number of different protein capture reagents, such as antibodies or antibody variable regions, each immobilized at a different location on a solid support. Such arrays are available, for example, from Sigma-Aldrich as part of their Panorama™ line of arrays. The array is exposed to a protein sample and the capture reagents selectively capture the specific protein targets. The captured proteins are detected by detection of a label. For example, the proteins can be labeled before exposure to the array; detection of a label at a particular location on the array indicates the detection of the corresponding protein. If the array is not saturated, the amount of label detected may correlate with the concentration or amount of the protein in the sample. Captured proteins can also be detected by subsequent exposure to a second capture reagent, which can itself be labeled or otherwise detected, as in a sandwich immunoassay format.

Mass spectrometry-based methods include, for example, matrix-assisted laser desorption/ionization (MALDI), Liquid Chromatography/Mass Spectrometry/Mass Spectrometry (LC-MS/MS) and surface enhanced laser desorption/ ionization (SELDI) techniques. For example, a protein profile can be generated using electrospray ionization and MALDI. SELDI, as described, for example, in U.S. Patent No. 6,225,047, incorporates a retention surface on a mass spectrometry chip. A subset of proteins in a protein sample are retained on the surface, reducing the complexity of the mixture. Subsequent time-of-flight mass spectrometry generates a "fingerprint" of the retained proteins.

In methods involving two-dimensional gel electrophoresis, proteins in a sample are generally separated in a first dimension by isoelectric point and in a second dimension by molecular weight during SDS-PAGE. By virtue of the two dimensions of resolution, hundreds or thousands of proteins can be simultaneously resolved and analyzed. The proteins are detected by application of a stain, such as a silver stain, or by the presence of a label on the proteins, such as a Cy2, Cy3, or Cy5 dye. To identify a protein, a gel spot can be cut out and in-gel tryptic digestion performed. The tryptic digest can be analyzed by mass spectrometry, such as MALDI. The resulting mass spectrum of peptides, the peptide mass fingerprint or PMF, is searched against a sequence database. The PMF is compared to the masses of all theoretical tryptic peptides generated in silico by the search program. Programs such as Prospector, Sequest, and MasCot (Matrix Science, Ltd., London, UK) can be used for the database searching. For example, MasCot produces a statistically-based Mowse score indicates if any matches are significant or not. MS/MS can be used to increase the likelihood of getting a database match. CID-MS/MS (collision induced dissociation of tandem MS) of peptides can be used to give a spectrum of fragment ions that contain information about the amino acid sequence. Adding this information to a peptide mass fingerprint allows Mascot to increase the statistical significance of a match. It is also possible in some cases to identify a protein by submitting only a raw MS/MS spectrum of a single peptide.

A recent improvement in comparisons of protein expression profiles involves the use of a mixture of two or more protein samples, each labeled with a different, spectrally-resolvable, charge- and mass-matched dye, such as Cy3 and Cy5. This improvement, called fluorescent 2-dimensional differential in-gel electrophoresis (DIGE), has the advantage that the test and control protein samples are run in the same gel, facilitating the matching of proteins between the two samples and avoiding complications involving non-identical electrophoresis conditions in different gels. The gels are imaged separately and the resulting images can be overlaid directly without further modification. A third spectrally-resolvable dye, such as Cy2, can be used to label a pool of protein samples to serve as an internal control among different gels run in an experiment. Thus, all detectable proteins are included as an internal standard, facilitating comparisons across different gels.

### Engineering cell lines to improve cell phenotypes

As described above, the present invention provides polynucleotide sequences (or subsequences) of genes or polypeptide sequences (or subsequences) of proteins that are differentially expressed in different cell lines or cell samples with at least one distinct cell phenotype. These sequences are collectively referred to as differential sequences. The differential sequences may be used as targets to effect a cell phenotype, particularly a phenotype characterized by increased and efficient production of a recombinant transgene, increased cell growth rate, high peak cell density, sustained high cell viability, high maximum cellular productivity, sustained high cellular productivity, low ammonium production, and low lactate production, *etc.*

More particularly, the invention provides each purified and/or isolated polynucleotide or polypeptide sequence referred to in the relevant Tables that is shown to be a suitable target for regulating a CHO cell phenotype, *i.e.*, is differentially expressed by a first CHO cell line compared to a second CHO cell line, herein designated as "differential CHO sequence." Specifically, as used herein, a differential CHO sequence include a sequence having and/or consisting essentially of a sequence selected from the gene sequences referenced in the Tables, a fragment or a complement thereof. As used herein, a differential CHO sequence also includes a polypeptide sequence selected from the protein sequences referenced in the Tables, or a fragment thereof. As used herein, a differential CHO sequence also includes a polynucleotide sequence encoding a polypeptide sequence selected from the protein sequences referenced in the Tables, a fragment or a complement thereof. A skilled artisan will recognize that the differential CHO sequences of the invention may include novel CHO sequences (as discussed below), known gene sequences that are attributed with a function that is, or was, not obviously involved in transgene expression, and known sequences that previously had no known function but may now be known to function as targets in regulating a CHO cell phenotype.

The present invention contemplates methods and compositions that may be used to alter (*i.e.*, regulate (e.g., enhance, reduce, or modify)) the expression and/or the activity of the genes or proteins corresponding to the differential CHO sequences in a cell or organism. Altered expression of the differential CHO sequences encompassed by the present invention in a cell or organism may be achieved through down-regulating or up-regulating of the corresponding genes or proteins. For example, the differential CHO sequences may be down-regulated by the use of various inhibitory polynucleotides, such as antisense polynucleotides, ribozymes that bind and/or cleave the mRNA transcribed from the genes of the invention, triplex-forming oligonucleotides that target regulatory regions of the genes, and short interfering RNA that causes sequence-specific degradation of target mRNA (*e.g.*, Galderisi et al. (1999) J. Cell. Physiol. 181:251-57; Sioud (2001) Curr. Mol. Med. 1:575-88; Knauert and Glazer (2001) Hum. Mol. Genet. 10:2243-51; Bass (2001) Nature 411:428-29).

The inhibitory antisense or ribozyme polynucleotides suitable for the invention can be complementary to an entire coding strand of a gene of the invention, or to only a portion thereof. Alternatively, inhibitory polynucleotides can be complementary to a noncoding region of the coding strand of a gene of the invention. The inhibitory polynucleotides of the invention can be constructed using chemical synthesis and/or enzymatic ligation reactions using procedures well known in the art. The nucleoside linkages of chemically synthesized polynucleotides can be modified to enhance their ability to resist nuclease-mediated degradation, as well as to increase their sequence specificity. Such linkage modifications include, but are not limited to, phosphorothioate, methylphosphonate, phosphoroamidate, boranophosphate, morpholino, and peptide nucleic acid (PNA) linkages (Galderisi *et al.,* supra; Heasman (2002) Dev. Biol. 243:209-14; Mickelfield (2001) Curr. Med. Chem. 8:1157-70). Alternatively, antisense molecules can be produced biologically using an expression vector into which a polynucleotide of the present invention has been subcloned in an antisense (*i.e.*, reverse) orientation.

In yet another embodiment, the antisense polynucleotide molecule suitable for the invention is an α-anomeric polynucleotide molecule. An α-anomeric polynucleotide molecule forms specific double-stranded hybrids with complementary RNA in which, contrary to the usual β-units, the strands run parallel to each other. The antisense polynucleotide molecule can also comprise a 2'-o-methylribonucleotide or a chimeric RNA-DNA analogue, according to techniques that are known in the art.

The inhibitory triplex-forming oligonucleotides (TFOs) suitable for the present invention bind in the major groove of duplex DNA with high specificity and affinity (Knauert and Glazer, supra). Expression of the genes of the present invention can be inhibited by targeting TFOs complementary to the regulatory regions of the genes (*i.e.,* the promoter and/or enhancer sequences) to form triple helical structures that prevent transcription of the genes.

In one embodiment of the invention, the inhibitory polynucleotides are short interfering RNA (siRNA) molecules. These siRNA molecules are short (preferably 19-25 nucleotides; most preferably 19 or 21 nucleotides), double-stranded RNA molecules that cause sequence-specific degradation of target mRNA. This degradation is known as RNA interference (RNAi) (*e.g.*, Bass (2001) Nature 411:428-29). Originally identified in lower organisms, RNAi has been effectively applied to mammalian cells and has recently been shown to prevent fulminant hepatitis in mice treated with siRNA molecules targeted to Fas MRNA (Song et al. (2003) Nat. Med. 9:347-51). In addition, intrathecally delivered siRNA has recently been reported to block pain responses in two models (agonist-induced pain model and neuropathic pain model) in the rat (Dom et al. (2004) Nucleic Acids Res. 32(5):e49).

The siRNA molecules suitable for the present invention can be generated by annealing two complementary single-stranded RNA molecules together (one of which matches a portion of the target mRNA) (Fire et al., U.S. Pat. No. 6,506,559) or through the use of a single hairpin RNA molecule that folds back on itself to produce the requisite double-stranded portion (Yu et al. (2002) Proc. Natl. Acad. Sci. USA 99:6047-52). The siRNA molecules can be chemically synthesized (Elbashir et al. (2001) Nature 411:494-98) or produced by *in vitro* transcription using single-stranded DNA templates (Yu *et al.,* supra). Alternatively, the siRNA molecules can be produced biologically, either transiently (Yu *et al.*, supra; Sui et al. (2002) Proc. Natl. Acad. Sci. USA 99:5515-20) or stably (Paddison et al. (2002) Proc. Natl. Acad. Sci. USA 99:1443-48), using an expression vector(s) containing the sense and antisense siRNA sequences. Recently, reduction of levels of target mRNA in primary human cells, in an efficient and sequence-specific manner, was demonstrated using adenoviral vectors that express hairpin RNAs, which are further processed into siRNAs (Arts et al. (2003) Genome Res. 13:2325-32).

The siRNA molecules targeted to the differential CHO sequences of the present invention can be designed based on criteria well known in the art (e.g., Elbashir et al. (2001) EMBO J. 20:6877-88). For example, the target segment of the target mRNA should begin with AA (preferred), TA, GA, or CA; the GC ratio of the siRNA molecule should be 45-55%; the siRNA molecule should not contain three of the same nucleotides in a row; the siRNA molecule should not contain seven mixed G/Cs in a row; and the target segment should be in the ORF region of the target mRNA and should be at least 75 bp after the initiation ATG and at least 75 bp before the stop codon. siRNA molecules targeted to the polynucleotides of the present invention can be designed by one of ordinary skill in the art using the aforementioned criteria or other known criteria.

Down-regulation of the genes or proteins of the present invention in a cell or organism may also be achieved through the creation of cells or organisms whose endogenous genes corresponding to the differential CHO sequences of the present invention have been disrupted through insertion of extraneous polynucleotides sequences (*i.e.*, a knockout cell or organism). The coding region of the endogenous gene may be disrupted, thereby generating a nonfunctional protein. Alternatively, the upstream regulatory region of the endogenous gene may be disrupted or replaced with different regulatory elements, resulting in the altered expression of the still-functional protein. Methods for generating knockout cells include homologous recombination and are well known in the art (*e.g.*, Wolfer et al. (2002) Trends Neurosci. 25:336-40).

The expression or activity of the CHO differential sequences may also be altered by up-regulating the genes or proteins corresponding to the CHO differential sequences of the invention. Up-regulation includes providing an exogenous nucleic acid (*e.g.*, an over-expression construct) encoding a protein or gene of interest or a variant retaining its activity or providing a factor or a molecule indirectly enhancing the protein activity. The variant generally shares common structural features with the protein or gene of interest and should retain the activity permitting the improved cellular phenotype. The variant may correspond to a homolog from another species (*e.g.* a rodent homolog; a primate homolog, such as a human homolog; another mammalian homolog; or a more distant homolog retaining sequence conservation sufficient to convey the desired effect on cellular phenotype). In some cases, the variant may retain at least 70%, at least 80%, at least 90%, or at least 95% sequence identity with the CHO sequence or with a known homolog. In certain embodiments, the variant is a nucleic acid molecule that hybridizes under stringent conditions to the CHO nucleic acid sequence or to the nucleic acid sequence of a known homolog.

For example, the isolated polynucleotides corresponding to the differential CHO sequences of the present invention may be operably linked to an expression control sequence such as the pMT2 and pED expression vectors for recombinant production of differentially expressed genes or proteins of the invention. General methods of expressing recombinant proteins are well known in the art.

The expression or activity of the differentially expressed genes or proteins of the present invention may also be altered by exogenous agents, small molecules, pharmaceutical compounds, or other factors that may be directly or indirectly modulating the activity of the genes or proteins of the present invention. As a result, these agents, small molecules, pharmaceutical compounds, or other factors may be used to regulate the phenotype of CHO cells, e.g., increased production of a recombinant transgene, increased cell growth rate, high peak cell density, sustained high cell viability, high maximum cellular productivity, sustained high cellular productivity, low ammonium production, and low lactate production, *etc.*

Any combinations of the methods of altering gene or protein expression described above are within the scope of the invention. Any combination of genes or proteins affecting different cell phenotypes can be modulated based on the methods described herein and are within the scope of the invention.

### Novel genes or proteins

As described above, the present invention provides differential sequences including sequences newly discovered to be expressed by CHO cells. Accordingly, the present invention provides novel isolated and/or purified polynucleotides that are at least part of previously undiscovered genes. Exemplary novel polynucleotide sequences (or subsequences) of genes that are newly discovered expressed by CHO cells are illustrated in Tables 9, 13, and 15. The present invention also provides isolated and/or purified polypeptides that are at least part of previously undiscovered proteins. Exemplary novel polypeptide sequences (or subsequences) of proteins that are newly discovered expressed by CHO cells are illustrated in Tables 2 and 4. The present invention also provides novel polynucleotides encoding the polypeptides sequences as illustrated in Tables 2 and 4.

Thus, the invention provides each purified and/or isolated polynucleotide sequence selected from Tables 9, 13, and 15 that is, or is part of, a previously undiscovered gene (*i.e.*, a gene that had not been sequenced and/or shown to be expressed by CHO cells) and is verifiably expressed by CHO cells. Alternatively, the invention provides each purified and/or isolated polypeptide sequence selected from Tables 2 and 4 that is, or is part of, a previously undiscovered protein (*i.e.*, a protein that had not been sequenced and/or shown to be expressed by CHO cells) and is verifiably expressed by CHO cells. The invention also provides isolated and/or purified polynucleotide sequence encoding each polypeptides sequence selected from Tables 2 and 4. These sequences are herein collectively designated as "novel CHO sequences." Preferred polynucleotide sequences of the invention include DNA sequences including genomic and cDNA sequences and chemically synthesized DNA sequences, RNA sequences, or other modified nucleic acid sequences. Preferred polypeptide sequences of the invention include amino acid sequences or modified amino acid sequences.

It is part of the invention to provide inhibitory polynucleotides to each novel CHO sequence as described above. Polynucleotides of the present invention also include polynucleotides that hybridize under stringent conditions to novel CHO sequences, or complements thereof, and/or encode polypeptides that retain substantial biological activity of polypeptides encoded by novel CHO sequences of the invention. Polynucleotides of the present invention also include continuous portions of novel CHO sequences comprising at least 21 consecutive nucleotides.

Polynucleotides of the present invention also include polynucleotides that encode any of the amino acid sequences encoded by the polynucleotides as described above, or continuous portions thereof, and that differ from the polynucleotides described above only due to the well-known degeneracy of the genetic code.

The isolated polynucleotides of the present invention may be used as hybridization probes (*e.g.*, as an oligonucleotide array, as described above) and primers to identify and isolate nucleic acids having sequences identical to, or similar to, those encoding the disclosed polynucleotides. Hybridization methods for identifying and isolating nucleic acids include polymerase chain reaction (PCR), Southern hybridization, and Northern hybridization, and are well known to those skilled in the art.

Hybridization reactions can be performed under conditions of different stringencies. The stringency of a hybridization reaction includes the difficulty with which any two nucleic acid molecules will hybridize to one another. Preferably, each hybridizing polynucleotide hybridizes to its corresponding polynucleotide under reduced stringency conditions, more preferably stringent conditions, and most preferably highly stringent conditions. Examples of stringency conditions are shown in Table 1 below: highly stringent conditions are those that are at least as stringent as, for example, conditions A-F; stringent conditions are at least as stringent as, for example, conditions G-L; and reduced stringency conditions are at least as stringent as, for example, conditions M-R.

**Table 1. Stringency Conditions**

| **Stringency Condition** | **Poly-nucleotide Hybrid** | **Hybrid Length (bp)¹** | **Hybridization Temperature and Buffer^{H}** | **Wash Temp. and Buffer^{H}** |
|---|---|---|---|---|
| A | DNA:DNA | >50 | 65°C; 1xSSC -or-42°C; 1xSSC, 50% formamide | 65°C; 0.3xSSC |
| B | DNA:DNA | <50 | T_{B}*; 1xSSC | T_{B}*; 1xSSC |
| C | DNA:RNA | >50 | 67°C; 1xSSC -or-45°C; 1xSSC, 50% formamide | 67°C; 0.3xSSC |
| D | DNA:RNA | <50 | T_{D}*; 1xSSC | T_{D}*; 1xSSC |
| E | RNA:RNA | >50 | 70°C; 1xSSC -or-50°C; 1xSSC, 50% formamide | 70°C; 0.3xSSC |
| F | RNA:RNA | <50 | T_{F}*; 1xSSC | T_{f}*; 1xSSC |
| G | DNA:DNA | >50 | 65°C; 4xSSC -or-42°C; 4xSSC, 50% formamide | 65°C; 1xSSC |
| H | DNA:DNA | <50 | T_{H}*; 4xSSC | T_{H}*; 4xSSC |
| I | DNA:RNA | >50 | 67°C; 4xSSC -or-45°C; 4xSSC, 50% formamide | 67°C; 1xSSC |
| J | DNA:RNA | <50 | T_{J}*; 4xSSC | T_{J}*; 4xSSC |
| K | RNA:RNA | >50 | 70°C; 4xSSC -or-50°C; 4xSSC, 50% formamide | 67°C; 1xSSC |
| L | RNA:RNA | <50 | T_{L}*;2xSSC | T_{L}*;2xSSC |
| ¹: The hybrid length is that anticipated for the hybridized region(s) of the hybridizing polynucleotides. When hybridizing a polynucleotide to a target polynucleotide of unknown sequence, the hybrid length is assumed to be that of the hybridizing polynucleotide. When polynucleotides of known sequence are hybridized, the hybrid length can be determined by aligning the sequences of the polynucleotides and identifying the region or regions of optimal sequence complementarity. | | | | |
| ^{H}: SSPE (1x SSPE is 0.15M NaCl, 10 mM NaH₂PO₄, and 1.25 mM EDTA, pH 7.4) can be substituted for SSC (1x SSC is 0.15M NaCl and 15 mM sodium citrate) in the hybridization and wash buffers. | | | | |
| T_{B}* - T_{R}*: The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tₘ(°C) = 81.5 + 16.6(log₁₀[Na⁺]) + 0.41 (%G + C) - (600/N), where N is the number of bases in the hybrid, and [Na⁺] is the molar concentration of sodium ions in the hybridization buffer ([Na⁺] for I x SSC = 0.165 M). | | | | |

Generally, and as stated above, the isolated polynucleotides of the present invention may also be used as hybridization probes and primers to identify and isolate DNAs homologous to the disclosed polynucleotides. These homologs are polynucleotides isolated from different species than those of the disclosed polynucleotides, or within the same species, but with significant sequence similarity to the disclosed polynucleotides. Preferably, polynucleotide homologs have at least 60% sequence identity (more preferably, at least 75% identity; most preferably, at least 90% identity) with the disclosed polynucleotides. Preferably, homologs of the disclosed polynucleotides are those isolated from mammalian species.

The isolated polynucleotides of the present invention may also be used as hybridization probes and primers to identify cells and tissues that express the polynucleotides of the present invention and the conditions under which they are expressed.

The present invention also contemplates recombinantly express the proteins or polypeptides encoded by the novel CHO sequences. A number of cell types may act as suitable host cells for recombinant expression of the polypeptides encoded by the novel CHO sequences of the invention. Mammalian host cells include, but are not limited to, *e.g.*, COS cells, CHO cells, 293 cells, A431 cells, 3T3 cells, CV-1 cells, HeLa cells, L cells, BHK21 cells, HL-60 cells, U937 cells, HEK cells, PerC6 cells, Jurkat cells, normal diploid cells, cell strains derived from in vitro culture of primary tissue, and primary explants.

Alternatively, it may be possible to recombinantly produce the polypeptides encoded by the novel CHO sequences of the present invention in lower eukaryotes such as yeast or in prokaryotes. Potentially suitable yeast strains include *Saccharomyces cerevisiae*, *Schizosaccharomyces pombe*, *Kluyveromyces* strains, and *Candida* strains. Potentially suitable bacterial strains include *Escherichia coli, Bacillus sublilis*, and *Salmonella typhimurium*. If the polypeptides are made in yeast or bacteria, it may be necessary to modify them by, *e.g.*, phosphorylation or glycosylation of appropriate sites, in order to obtain functionality. Such covalent attachments may be accomplished using well-known chemical or enzymatic methods.

The polypeptides encoded by polynucleotides of the present invention may also be recombinantly produced by operably linking the isolated novel CHO sequences of the present invention to suitable control sequences in one or more insect expression vectors, such as baculovirus vectors, and employing an insect cell expression system. Materials and methods for baculovirus/Sf9 expression systems are commercially available in kit form (*e.g.*, the MaxBac® kit, Invitrogen, Carlsbad, Calif.).

Following recombinant expression in the appropriate host cells, the polypeptides encoded by polynucleotides of the present invention may then be purified from culture medium or cell extracts using known purification processes, such as gel filtration and ion exchange chromatography. Purification may also include affinity chromatography with agents known to bind the polypeptides encoded by the polynucleotides of the present invention. These purification processes may also be used to purify the polypeptides from natural sources.

Alternatively, the polypeptides encoded by the novel CHO sequences of the present invention may also be recombinantly expressed in a form that facilitates purification. For example, the polypeptides may be expressed as fusions with proteins such as maltose-binding protein (MBP), glutathione-S-transferase (GST), or thioredoxin (TRX). Kits for expression and purification of such fusion proteins are commercially available from New England BioLabs (Beverly, Mass.), Pharmacia (Piscataway, N.J.), and Invitrogen (Carlsbad, Calif.), respectively. The polypeptides encoded by polynucleotides of the present invention can also be tagged with a small epitope and subsequently identified or purified using a specific antibody to the epitope. A preferred epitope is the FLAG epitope, which is commercially available from Eastman Kodak (New Haven, Conn.).

The polypeptides encoded by the novel CHO sequences of the present invention may also be produced by known conventional chemical synthesis. Methods for chemically synthesizing the polypeptides encoded by the novel CHO sequences of the present invention are well known to those skilled in the art. Such chemically synthetic polypeptides may possess biological properties in common with the natural, purified polypeptides, and thus may be employed as biologically active or immunological substitutes for the natural polypeptides.

It should be understood that the above-described embodiments and the following examples are given by way of illustration, not limitation. Various changes and modifications within the scope of the present invention will become apparent to those skilled in the art from the present description.

### EXAMPLES

### Example 1. Cell culture

Cells were cultured in serum-free suspension culture in two basic formats, under two basic conditions. One format was small scale, shake flask culture in which cells were cultured in less than 100 ml in a vented tissue culture flask, rotated on an orbiting shaker in a CO₂ incubator. The second format was in bench top bioreactors, 2L or less working volume, controlled for pH, nutrients, dissolved oxygen, and temperature. The two basic culture conditions were ordinary passage conditions of 37C, or fed batch culture conditions. In a basic fed batch culture, the cells are grown for a longer period of time, and shifted to a lower temperature in order to prolong cell viability and extend to the productive phase of the culture.

### Example 2. Classification of CHO cell cultures

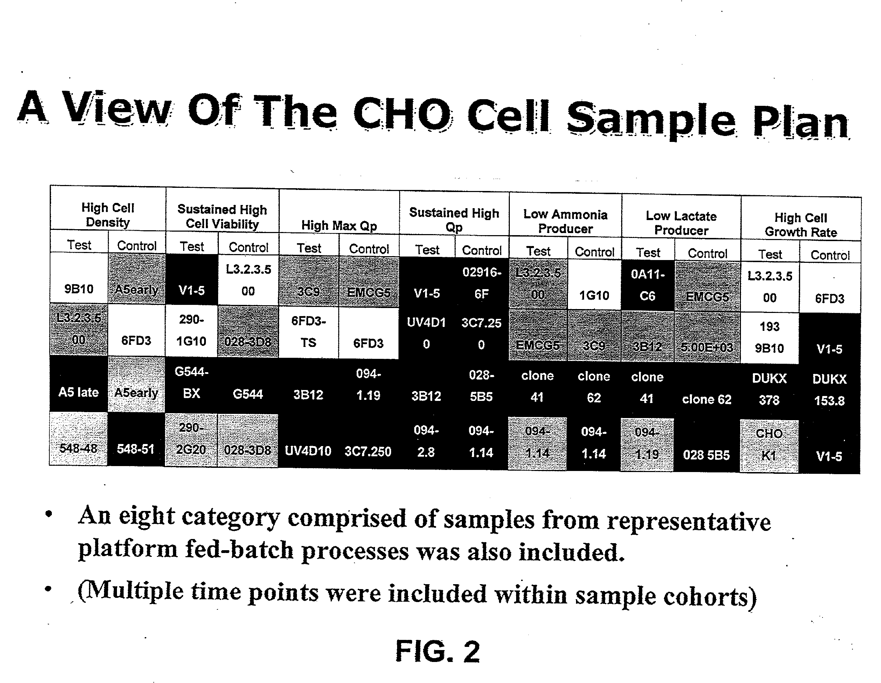

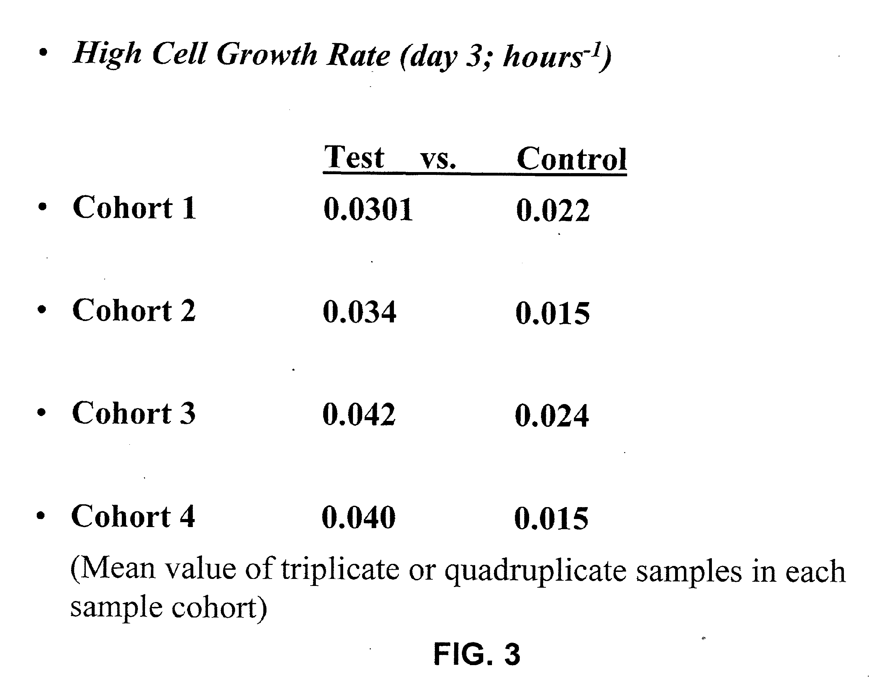

CHO cell lines were categorized based on each of the following phenotypes useful for highly productive fed-batch cell culture processes: high cell growth rate, high peak cell density, sustained high cell viability, high maximum cellular productivity, sustained high cellular productivity, low ammonium production, and low lactate production. A cell sample matrix was generated in which the phenotypic categorieswere populated with the appropriate CHO cell samples taken from shake flask and benchtop bioreactor cultures and included 375 individual samples (including biological triplicates or quadruplicates) and 29 different rCHO lines expressing monoclonal antibodies, cytokines, coagulation factors and Fc:receptor fusion molecules. An exemplary portion of the cell sample matrix is depicted in Figure 2, in which the abbreviation Qp is used for cellular productivity. An exemplary phenotypic comparison between test cell lines and control cell lines for the "high cell growth rate" phenotype is depicted in Figure 3.

### Example 3. Detection of differentially expressed proteins

### Method

Cells were harvested and subjected to standard lysis in 7 M urea, 2 M thiourea, 4% CHAPS, 30 mM Tris, 5 mM magnesium acetate at pH 8.5. 150 µg aliquots of the lysates were analyzed by two-dimensional gel electrophoresis to confirm sample quality using 18 cm immobilized pH gradient isoelectric focusing gradient strips, pH 4-7. The strips were rehydrated overnight with 340 µl of buffer per strip. Samples were loaded at the cathodic end of the strip and subjected to 500 V for 1 hour, 1000 V for 1 hour, and 8000 V for 4 hours and stored at -80°C until the second dimension on 12.5% acrylamide gels. Electrophoresis in the second dimension was performed at 1.5 W per gel for 30 minutes and then a total of 100 W for 5 hours for a Dalt 6 run of 6 large format gels. Proteins were visualized by silver staining to confirm the quality of the proteins in the lysate.

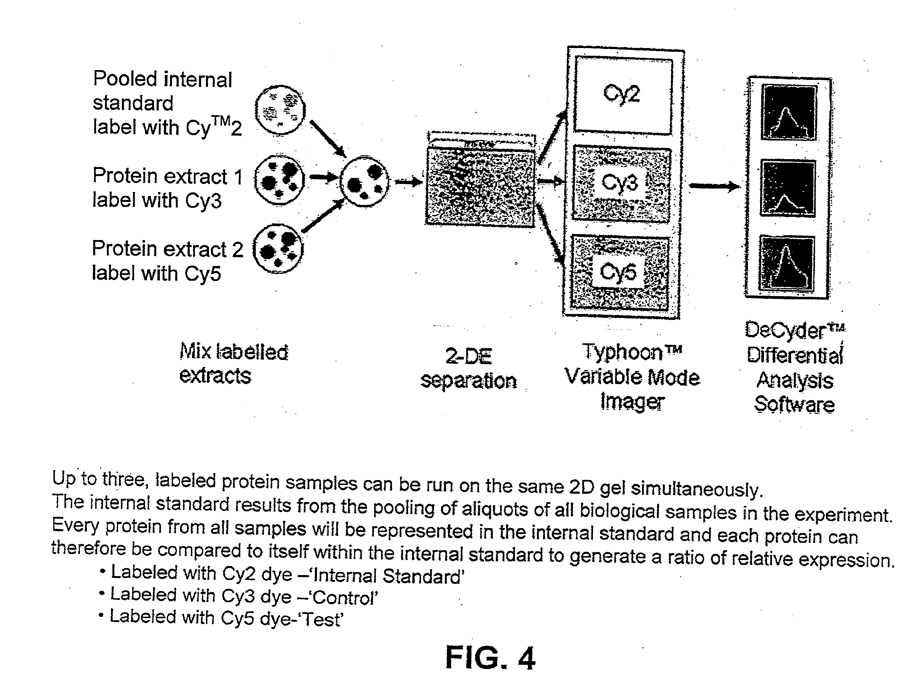

Aliquots of the original lysates were then labeled with fluorescent dyes in preparation for fluorescent 2-dimensional differential in-gel electrophoresis (DIGE), an overview of which is shown in Figure 4. Each comparison of cell cultures was performed four times using duplicate gels for a total of 8 DIGE gels per experiment, using 50 µg each of Cy2-, Cy3-, and Cy5-labeled cell lysates per gel. All cell lysates used in an experiment were pooled and labeled with Cy2 to serve as an internal standard. The control cell lysate was labeled with Cy3 and the test cell lysate is labeled with Cy5. Labeling was performed on ice in the dark for 30 minutes, followed by a 10 minute quenching of the reaction using 10 mM lysine on ice in the dark. The Cy2-, Cy3-, and Cy5-labeled lysates were then pooled and mixed with 2x sample buffer for 15 minutes in the dark on ice.

The samples were applied to immobilized pH gradient isoelectric focusing strips. The strips were rehydrated overnight for about 20 hours. Samples were loaded at the cathodic end of the strip and subjected to 300V/3hr/G, 600V/3hr/S&H, 1000V/3hr/G, 8000V/3hr/G, 8000V/4hr/S&H, and 500V/12hr/S&H. One hour before SDS-PAGE, the strips were subjected to 8000V for one hour. The strips were equilibrated for 15 minutes in SDS buffer + 1% DTT and for 15 minutes in SDS buffer + 2.5% iodoacetamide. The strips were applied to polyacrylamide gels and overlaid with agarose. Electrophoresis through the gels was performed at 1.5 W/gel at 10°C for about 18 hours on a Dalt 12 using 12 large format gels. The gels were scanned on a Typhoon™ 9400 scanner with a variable mode imager; cropped; and imported into DeCyder™ software. Differentially regulated proteins were identified using biological variance analysis (BVA). These proteins were matched to a preparative gel loaded with 400 µg of protein and stained with ruthenium. From the preparative gel, an Ettan Spot Picker was used to pick proteins identified by DIGE as differentially regulated. An Ettan Digestor was used to digest the individual proteins with an overnight trypsin incubation. The resulting peptides were analyzed by mass spectrometry. MALDI is used, particularly for highly abundant samples on gels, for peptide mass fingerprinting.

For lower abundance samples, LC-MS/MS using an MDLC LTQ machine is used. Tryptically digested samples from 2D gel spots were resuspended in 20µL of LC-MS grade water containing 0.1 %TFA and analysed by one-dimensional LC-MS using the Ettan™ MDLC system (GE Healthcare) in high-throughput configuration directly connected to a Finnigan™ LTQ™ (Thermo Electron). Samples were concentrated and desalted on RPC trap columns (Zorbax™ 300SB C18, 0.3mm x 5mm, Agilent Technologies) and the peptides were separated on a nano-RPC column (Zorbax™ 300SB C18, 0.075mm x 100mm, Agilent Technologies) using a linear acetonitrile gradient from 0-65% Acetonitrile (Riedel-de Haën LC-MS grade) over 60 minutes directly into the LTQ via a 10µm nanoESI emitter (Presearch FS360-20-10-CE-20). The LTQ ion trap mass spectrometer was used for MS/MS. A scan time of ~0.15 s (one microscans with a maximum ion injection time of 10ms) over an m/z range of 300-2000 was used followed by MS/MS analysis of the 3 most abundant peaks from each scan which were then excluded for the next 60 seconds followed by MS/MS of the next three abundant peaks which in turn were excluded for 60 seconds and so on. A "collision energy" setting of 35% was applied for ion fragmentation and dynamic exclusion was used to discriminate against previously analysed ions (data dependent analysis).

All buffers used for nanoLC separations contained 0.1 % Formic Acid (Fluka) as the ion pairing reagent. Full scan mass spectra were recorded in profile mode and tandem mass spectra in centroid mode. The peptides were identified using the information in the tandem mass spectra by searching against SWISS PROT database using SEQUEST™. An Xcorr value of >1.5 for singly charged peptides, >2.0 for doubly charged peptide and >2.5 for triply charged peptides was used as statistical cut-off.

### Markers for maximum cellular productivity

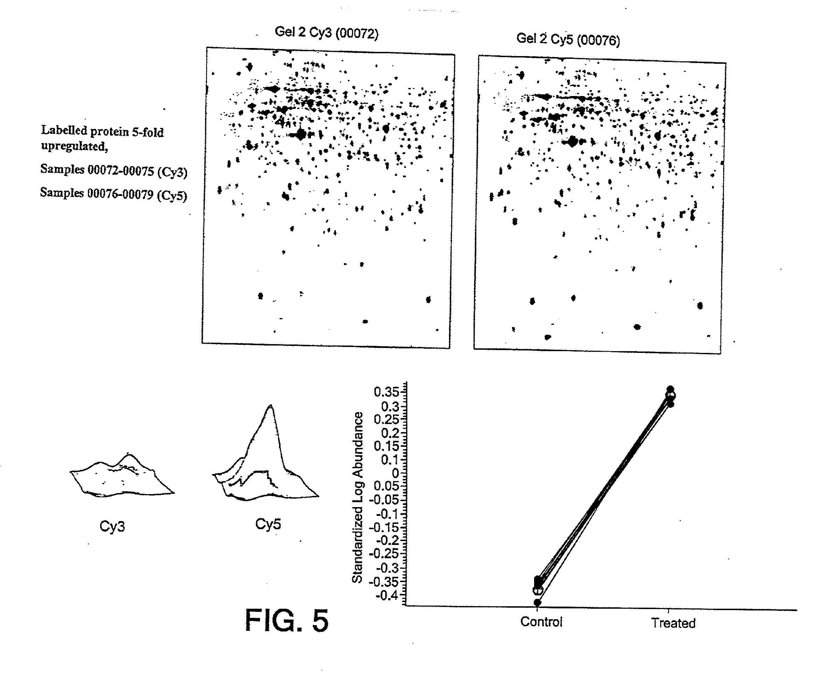

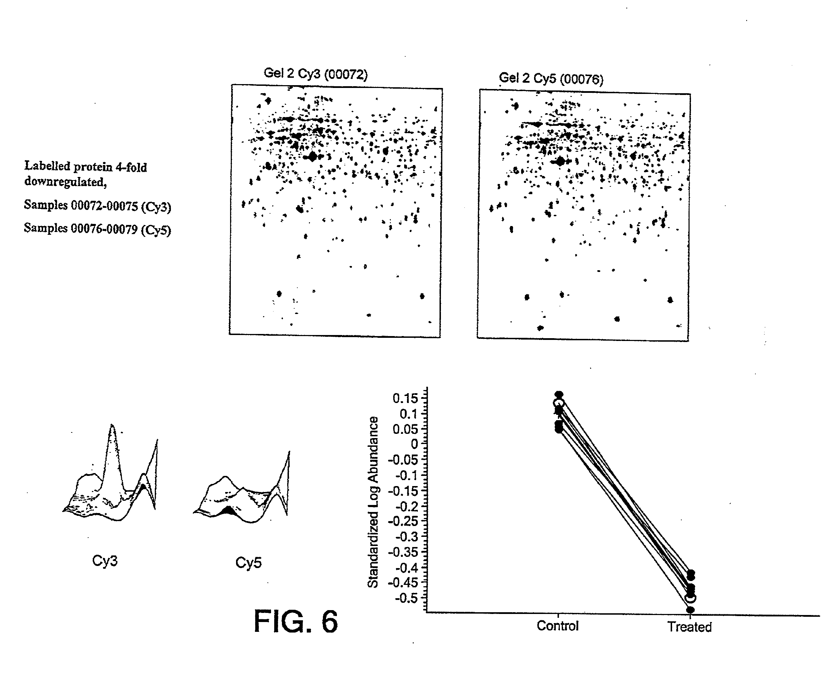

The protein expression profile of four cultures of a cell line overexpressing PACE (furin preproprotein), having a high maximum cellular productivity, was compared to the protein expression profile of four cultures of a control cell line. Approximately 2000 proteins were matched across all 8 gel experiments (involving a total of 24 images). To be considered as a differentially-expressed protein in the DeCyder analysis, a protein must have been identified in all 24; have demonstrated at least a 1.5-fold up- or down-regulation; and have demonstrated a T-test score less than 0.05. 188 proteins were identified as differentially regulated, most with highly significant T-test scores, including several low abundance proteins. Figure 5 depicts the Cy3 and Cy5 staining patterns on an exemplary gel. A protein that appears to be 5-fold upregulated in the Cy5-labeled test cell extract is outlined in the Figure; graphical depictions of the relative abundance of the protein in the Cy5-labeled test cell extract are also shown. A protein that appears to be 4-fold downregulated in the Cy5-labeled test cell extract is outlined in Figure 6 and graphical depictions analogous to those in the previous Figure are shown.

Tables 2 and 3 list several of the spots identified as differentially expressed in the high maximal cellular productivity cell line. For each of the spots listed in the tables, MALDI sequence analysis identified one or two corresponding amino acid sequences. The tables provide, for each spot number, the fold difference in protein levels between the test and control samples, labeled as "Average Ratio"; proteins whose levels are reduced in the test samples are indicated with a negative sign. The tables also provide the p-value that the differences in expression would be the result of random chance and the protein name and accession number corresponding to any identified amino acid sequence. In the MALDI sequence analysis, the molecular weights of the trypsin fragments were compared to predicted molecular weights of trypsin fragments of known sequences. In some cases, in this sequence analysis and in other peptide sequence analyses included in this application, the detected molecular weights are indicative of detection of a modified form of a peptide, such as where cysteine has been modified with iodacetamide, or where methionine has been partially oxidized. It is understood that this is not necessarily reflective of the initial state of the peptide in the context of the protein in the cell or the cellular milieu. Accordingly, the peptide sequences provided in the sequence listing reflect the unmodified forms of the peptide, and cells engineered to have desirable cellular phenotypes will, in some embodiments, be engineered to regulate genes expressing an amino acid sequence comprising one or more of the peptides.

In the tables, "% coverage" refers to the percentage of the total length of a database sequence for which corresponding trypsin fragments were detected in the experiment. pI and M_{R} refer to the apparent isoelectric point and apparent molecular weight of the protein spot. For some proteins, putative protein functions are also provided in the table.

**Table 2: High Max Qp Prot Proteins Identified as Novel Homologs of Non-Hamster Proteins**

| **Spot no.** | **Average Ratio (Test/Control)** | **p-value** | **gi accession no.** | **Protein name** | **% coverage** | **pl** | **Mr** | **Function** | **Species** |
|---|---|---|---|---|---|---|---|---|---|
| 912 | 1.9 | 3.2x10-12 | gi\|49645 and gi\|62296810 | Protein disulfide-isomerase A6 precursor (Protein disulfide isomerase P5) (Calcium-binding protein 1) (CaBP1) | 18.2 | 5 | 48.54 | Catalyzes the re-arrangement of S-S bonds in proteins / protein folding | Mesocricetus auratus and Rattus norvegicus |
| 105 | -1.87 | 1.4x10-6 | gi\|14250200 | vinculin | 11.4 | 5.8 | 117.3 | | Mus musculus |
| 114 | 1.59 | 5.9x10-5 | gi\|24025637 | heat shock protein 4 | 11.9 | 5.1 | 94.84 | protein folding | Rattus norvegicus |
| 310 | 1.63 | 1.1x10-9 | gi\|51948378 | minichromosome maintenance protein 7 | 13.9 | 5.9 | 81.67 | | Rattus norvegicus |
| 326 | 8.28 | 2x10-12 | gi\|4505579 | furin preproprotein (PACE) | 7.9 | 6 | 87.92 | Endoprotease/ precursor processing activity (release of mature proteins from pro-proteins) | Homo sapiens |
| 381 | 1.5 | 8.90E-09 | gi\|31981237 | thimet oligopeptidase 1 | 18.6 | 5.7 | 78.81 | | Mus musculus |
| 467 | -1.55 | 1.00E-09 | gi\|31981769 | glycerol phosphate dehydrogenase 2, mitochondrial | 8.8 | 6.3 | 81.48 | | Mus musculus |
| 585 | -1.97 | 7.7x10-10 | gi\|1915913 | Ulip2 protein | 23.8 | 6 | 62.55 | | Mus musculus |
| 627 | 1.58 | 5.8x10-9 | gi\|40018616 | chaperonin containing TCP1, subunit 3 (gamma) | 18 | 6.2 | 61.2 | Protein folding | Rattus norvegicus |
| 662 | -3.66 | 3.6x10-15 | gi\|34853001 | PREDICTED: similar to UDP-N-acteylglucosamine pyrophosphorylase 1-like 1 | 14.8 | 5.4 | 57.16 | | Rattus norvegicus |
| 759 | -1.77 | 1.4x10-9 | gi\|52353955 | 3-phosphoglycerate dehydrogenase | 14.8 | 6.1 | 57.37 | | Mus musculus |
| 831 | -1.55 | 7.2x10-8 | gi\|1708472 | Inosine-5'-monophosphate dehydrogenase 1 (IMP dehydrogenase 1) (IMPDH-I) (IMPD 1) | 17.5 | 6.3 | 55.62 | | Mus musculus |
| 899 | 1.58 | 4.4x10-6 | gi\|53237082 | eukaryotic translation initiation factor 3, subunit 5 (epsilon) | 15.2 | 5.2 | 38.09 | Translation initiation | Mus musculus |
| 968 | 1.62 | 6x10-9 | gi\|14010837 | NSFL1 (p97) cofactor (p47) | 42.7 | 5 | 40.66 | | Rattus norvegicus |
| 1082 | -2.5 | 6.9x10-13 | gi\|12842724 | *unnamed protein product* | 16 | 7.7 | 44.45 | | Mus musculus |
| 1126 | 1.52 | 0.00012 | gi\|62296810 | Protein disulfide-isomerase A6 precursor (Protein disulfide isomerase P5) (Calcium-binding protein 1) (CaBP1) | 12.1 | 5 | 48.54 | Catalyses the re-arrangement of S-S bonds in proteins / protein folding | Rattus norveqicus |
| 1129 | -2.12 | 3.1x10-13 | gi\|62296810 | Capg protein | 20.6 | 6.5 | 39.04 | Caps actin filaments/ intermediate filament assembly | Rattus norvegicus |
| 1159 | 1.61 | 1.5x10-6 | gi\|21312564 | calponin 3, acidic | 29.8 | 5.7 | 36.51 | | Mus musculus |
| 1221 | 1.59 | 7.4x10-5 | gi\|54114937 | Eno1 protein (enolase) | 15.3 | 7.8 | 50,18 | | Mus musculus |
| 1265 | 1.66 | 2.6x10-6 | gi\|34870516 | PREDICTED: similar to RIKEN cDNA | 24 | 5.2 | 35.04 | | Rattus norveqicus |
| 1274 | 1.72 | 1.8x10-3 | gi\|3041728 | 60S acidic ribosomal protein P0 (L10E) | 29.8 | 5.7 | 36.51 | | Bos taurus |
| 1295 | 1.57 | 1.5x10-11 | gi\|51262090 | Eef1d protein (eukaryotic translation elongation factor 1-delta) | 24.2 | 4.9 | 31.39 | Protein translation | Mus musculus |
| 1326 | -1.73 | 2.8x10-10 | gi\|47169319 | Chain D, Structure Of Pitp-Alpha Complexed To Phosphatidylinositol | 17.8 | 6.1 | 30.97 | | Homo sapiens |
| 1341 | -1.51 | 8.0x10-10 | gi\|15100179 | malate dehydrogenase (soluble) | 31.5 | 7.7 | 38.91 | | Rattus norvegicus |
| 1352 | 1.55 | 4.5x10-12 | gi\|10442752 | eukaryotic translation elongation factor 1-delta | 21.4 | 4.9 | 31.39 | Protein translation | Mus musculus |
| 1359 | 1.59 | 2x10-15 | gi\|10442752 | eukaryotic translation elongation factor 1-delta | 29.5 | 4.9 | 31.39 | Protein translation | Mus musculus |
| 1412 | -1.62 | 3.3x10-11 | gi\|388923 | purine-nucleoside phosphorylase | 23.9 | 6.5 | 32.57 | 32.57 | Mus spretus |
| 1426 | 1.78 | 1.1x10-3 | gi\|73968592 | PREDICTED: similar to cyclin-dependent kinase 4 isoform 3 | 38.1 | 6.3 | 27.56 | | Canis familiaris |
| 1454 | 1.73 | 3.6x10-10 | gi\|18044897 | Tyms protein (thymidylate synthase) | 19.2 | 5.9 | 35.2 | | Mus musculus |
| 1525 | 2.1 | 2.1x10-12 | gi\|13928824 | tyrosine 3-monooxygenase/tryptophan 5-monooxygenase activation protein | 39.2 | 4.6 | 29.34 | | Rattus norvegicus |
| 1525 | 2.1 | 2.1x10-12 | gi\|73961101 | Tropomyosin | 41.9 | 4.7 | 26.63 | | Canis familiaris |
| 1577 | 1.53 | 8x10-11 | gi\|53733577 and gi\|31982030 | Rho GDP dissociation inhibitor (GDI) alpha | 33.3 | 5.1 | 23.45 | Signal transduction/ cell motility/ cytoskeletal activity | Rattus norvegicus and Mus musculus |
| 1678 | 3.48 | 6.2x10-12 | gi\|14010865 | heat shock 27kDa protein | 35.1 | 6.1 | 22.86 | Chaperone | Rattus norvegicus |
| 1685 | 3.74 | 5.3x10-14 | gi\|14010865 | heat shock 27kDa protein | 26.8 | 6.1 | 22.86 | Chaperone | Rattus norvegicus |
| 1724 | -3.46 | 4.1x10-18 | gi\|34879492 | similar to Translationally controlled tumor protein (TCTP) (p23) (21 kDa polypeptide) (p21) (Lens epithelial protein) | 13.9 | 5.2 | 19.09 | | Rattus norvegicus |
| 1814 | -1.72 | 2.3x10-10 | gi\|817935 | adenine phophoribosyltransferase | 38.3 | 5.7 | 19.61 | | Cricetulus |
| | | | | | | | | | longicaudatus |
| 2000 | -1.8 | 1.3x10-8 | gi\|1083180 | galectin-1 | 37.8 | 5.5 | 15.13 | Unknown function in CHO, may be involved in cell proliferation. In humans, may have role in apoptosis & cell differentiation | Cricetulus griseus |
| 108 | 1.67 | 2.90E-06 | gi\|62087882 | heat shock 70kDa protein 4 isoform a variant | 9.7 | 0 | 5.4 | | Homo sapiens |
| 123 | 1.53 | 0.0011 | gi\|26522952 | alanyl tma synthetase | 17.8 | 0 | 5.3 | | Mesocricetus auratus |
| 256 | -1.87 | 1.70E-07 | gi\|56605726 | eukaryotic translation initiation factor 4B | 13.6 | 0.01 | 5.6 | Translation initiation | Rattus norvegicus |
| 434 | -1.51 | 0.0057 | gi\|42542422 | Heat shock protein 8 | 21.5 | 0 | 5.3 | | Mus musculus |
| 724 | 2.38 | 8.40E-12 | gi\|13097417 | FK506 binding protein 4 | 25.8 | 0.002 | 5.6 | | Mus musculus |
| 1171 | 1.51 | 0.0011 | gi\|56206424 | nucleophosmin 1 | 20.6 | 0.001 | 4.5 | | Mus musculus |
| 1330 | -2.02 | 5.60E-07 | gi\|14249130\| | LIM and SH3 protein 1 (lasp-1) | 39.9 | 0 | 6.6 | | Rattus norvegicus |
| 1358 | -1.52 | 1.30E-09 | gi\|7710036 | heterogeneous nuclear ribonucleoprotein D-like | 21.3 | 0 | 6.9 | | Mus musculus |
| 1459 | -1.51 | 8.10E-09 | gi\|59858367 | annexin 5 | 20.6 | 0.001 | 4.9 | | Bos taurus |
| 1486 | 1.7 | 2.80E-10 | gi\|77377292 | sulfatase modifying factor 2 | 23.4 | 0.006 | 6.6 | | Mus musculus |
| 1532 | -2.19 | 6.70E-09 | gi\|73961099 | PREDICTED: similar to tropomyosin 3 isoform 2 isoform 17 | 29.4 | 0.001 | 4.7 | Intermediate filament | Canis familiaris |
| 1629 | -1.54 | 1.20E-10 | gi\|62647453 | PREDICTED: similar to ribose 5-phosphate isomerase | 21.5 | 0.001 | 7.9 | | Rattus norvegicus |
| 1780 | -1.6 | 9.30E-10 | gi\|32452351 | CAP1 protein | 32.3 | 0.007 | 6.3 | | Mesocricetus auratus |
| 1906 | -1.51 | 3.30E-07 | gi\|14625464 | stathmin | 53.7 | 0 | 5.9 | | Mus musculus |
| 2098 | -2.01 | 6.00E-14 | gi\|51854249 | S100 calcium binding protein A11 (calizzarin) | 21.4 | 0 | 5.6 | | Rattus norvegicus |
| 2130 | -2.61 | 4.20E-12 | gi\|198561 | EGF-binding protein | 19.6 | 0 | 6.9 | Mus musculus | Mus musculus |

**Table 3: High Max Qp Prot Known Hamster Proteins**

| **Spot no.** | **Average Ratio (Test/Control)** | **p-value** | **gi accession no.** | **Protein name** | **% coverage** | **pl** | **Mr** | **Function** | **Species** |
|---|---|---|---|---|---|---|---|---|---|
| 426 | -2 | 3.6x10-7 | gi\31981722 or gi\|90188 | heat shock 70kDa protein 5 (glucose-regulated protein) ordnaK-type molecular chaperone GRP78 precursor - Chinese hamster | 32.1 | 5.1 | 72.53 | Protein complex assembly in ER? | Cricetulus griseus |
| 452 | -1.73 | 3.6x10-11 | gi\|3122170 and gi\|2231704 | Stress-70 protein, mitochondrial precursor (75 kDa glucose regulated protein) (GRP 75) / 70 kDa heat shock protein precursor | 15.8 | 5.9 | 74 | Chaperone | Cricetulus griseus |
| 719 | 3.54 | 7.6x10-14 | gi\|123332 | Hydroxymethylglutaryl-CoA synthase, cytoplasmic (HMG-CoA synthase) (3-hydroxy-3-methylglutaryl coenzyme A synthase) | 11.5 | 5.4 | 57.93 | Cholesterol biosynthesis | Cricetulus griseus |
| 729 | 2.78 | 1.6x10-16 | gi\|123332 | Hydroxymethylglutaryl-CoA synthase, cytoplasmic (HMG-CoA synthase) (3-hydroxy-3-methylglutaryl coenzyme A synthase) | 15.8 | 5.4 | 57.93 | Cholesterol biosynthesis | Cricetulus griseus |
| 745 | -1.59 | 3.6x10-8 | gi\|860908 | vimentin | 41.6 | 4.7 | 44.62 | Intermediate filament | Cricetulus griseus |
| 750 | -1.75 | 2.7x10-7 | gi\|860908 | vimentin | 51.6 | 4.7 | 44.62 | Intermediate filament | Cricetulus griseus |
| 778 | -1.65 | 1x10-9 | gi\|860908 | vimentin | 26.6 | 4.7 | 44.62 | Intermediate filament | Cricetulus griseus |
| 867 | -2.33 | 4.6x10-9 | gi\|860908 | vimentin | 26.6 | 4.7 | 44.62 | Intermediate filament | Cricetulus griseus |
| 947 | -2.02 | 7.2x10-7 | gi\|860908 | vimentin | 45.5 | 4.7 | 44.62 | Intermediate filament | Cricetulus griseus |
| 1308 | -2.38 | 2.70E-14 | gi\|2114406 | aldo-keto reductase | 23.7 | 6.2 | 36.61 | | Cricetulus griseus |

Sequence data for identified proteins are provided in Figures 7 through 59. Each figure provides, for a particular protein spot from the DIGE, the spectrum of molecular weights detected in the tryptic digest; the corresponding protein database match or matches, including the number of peptides matched to the predicted tryptic peptides for the protein database entry, the accession number, name, and species of the protein from the database entry, the percent coverage, the isoelectric point and mass; for each molecular weight matched with a predicted mass of a predicted peptide, the measured mass, the predicted (compared) mass, the difference between the two, and the corresponding peptide sequence; and the full length sequence of the protein from the database entry.

### Markers for high cell growth rate

The protein expression profile of PA DUKX 378, having a high cell growth rate, was compared to the protein expression profile of PA DUKX 153.8. Tables 4 and 5 list several of the spots identified as differentially expressed in the high maximal cellular productivity cell line. For each of the spots listed in the tables, MALDI sequence analysis identified matches to a corresponding amino acid sequence from Chinese hamsters or from another species. The tables provide, for each spot number, the fold difference in protein levels between the test and control samples, labeled as "Average Ratio"; proteins whose levels are reduced in the test samples are indicated with a negative sign. The tables also provide: the p-value (statistical significance); and the protein name, accession number, and species corresponding to any identified amino acid sequence.

**Table 4: High Cell Growth Rate Proteins Identified as Novel Homologs of Non-Hamster Proteins**

| **Decyder Master no.** | **Average Ratio (Test/Control)** | **p-value** | **Accession no.** | **Protein name** | **Mass Spec Identification** | **% coverage** | **p*I*** | **Mr** | **Expecta ncy value -MALDI** | **No. peptides used for LC-MS/MS ID** |
|---|---|---|---|---|---|---|---|---|---|---|
| | | | | | | | | | | |
| 310 | 1.67 | 5.1x10-5 | gi\|17865351 | valosin-containing protein | MALDI ID | 30.3 | 5.1 | 90.02 | 0 | |
| 314 | -1.6 | 3.8x10-4 | gi\|600159 | elongation factor 2 | MALDI ID | 23.1 | 6.4 | 96.26 | 0 | |
| 440 | 1.64 | 0.0024 | gi\|38371758 | alpha glucosidase II alpha subunit isoform 1 | MALDI ID | 12.3 | 5.9 | 86.27 | 0 | |
| 610 | 2.18 | 1.5x10-5 | gi\|21411235 | NADH dehydrogenase (ubiquinone) Fe-S protein 1, 75kDa, precursor | MALDI ID | 23.2 | 5.8 | 80.45 | 0 | |
| 624 | 1.54 | 2.7x10-4 | gi\|73968066 | PREDICTED: similar to 78 kDa glucose-regulated protein precursor (GRP 78) (Immunoglobulin heavy chain binding protein) (BiP) (Endoplasmic reticulum lumenal Ca(2+) binding protein grp78) isoform 3 | MALDI ID | 30.3 | 5.1 | 65.46 | 0 | |
| 636 | 1.56 | 3.5x10-6 | gi\|73968066 | PREDICTED: similar to 78 kDa glucose-regulated protein precursor (GRP 78) (Immunoglobulin heavy chain binding protein) (BiP) (Endoplasmic reticulum lumenal Ca(2+) binding protein grp78) isoform 3 | MALDI ID | 26.3 | 5.1 | 65.46 | | |
| 703 | 1.65 | 9.5x10-4 | gi\|42542422 | heat shock 70kDa protein 8 (Hsc70-ps1) | MALDI ID | 34.8 | 5.3 | 71.08 | 0 | |
| 708 | 1.52 | 0.0035 | gi\|56385 | heat shock 70kDa protein 8 (Hsc70-ps1) | MALDI ID | 37.8 | | | 0 | |
| 894 | 1.56 | 0.0016 | gi\|24025637 | heat shock 70kDa protein 4 | MALDI ID | 9.6 | 5.1 | 94.84 | 0.001 | |
| 990 | 1.61 | 2.7x10-10 | gi\|2745838 | Hsp70/Hsp90 organizing protein | MALDI ID | 27.8 | 6.4 | 63.26 | 0 | |
| 1033 | 1.63 | 2.7x10-7 | gi\|16508150 | ERP57 protein (glucose regulated protein/ protein disulphide isomerase) | MALDI ID | 25.7 | 6 | 57.23 | 0 | |
| 1139 | 1.59 | 3.8x10-6 | gi\|52353955 | 3-phosphoglycerate dehydrogenase | MALDI ID | 16.9 | 6.1 | 57.37 | 0 | |
| 1172 | -1.67 | 3.2x10-5 | gi\|73993723 | PREDICTED: similar to Serine/threonine protein phosphatase 2A, 55 kDa regulatory subunit B, alpha isoform (PP2A, subunit B, B-alpha isoform) (PP2A, subunit B, B55-alpha isoform) (PP2A, subunit B, PR55-alpha isoform) (PP2A, subunit B, R2-alpha isoform)... isoform 9 | MALDI ID | 19.1 | 5.7 | 53.46 | 0.001 | |
| 1284 | 1.77 | 0.003 | gi\|21618633 | 3-hydroxy-3-methylglutaryl-Coenzyme A synthase 1 | MALDI ID | 21 | 5.6 | 58.16 | 0 | |
| 1452 | 1.54 | 0.012 | gi\|51710798 | PREDICTED: similar to tubulin, beta, 2 | MALDI ID | 8.3 | 4.7 | 50.28 | 0.001 | |
| 1541 | -1.68 | 3.2x10-9 | gi\|381964 | actin-related protein | MALDI ID | 39.6 | 6.3 | 42.67 | 0 | |
| 1806 | 1.52 | 2.9x10-4 | gi\|433308\| | capping protein alpha | MALDI ID | 20.4 | 5.6 | 32.96 | 0.002 | |
| 1880 | -1.62 | 2.2x10-4 | gi\|18026574 | transaldolase | MALDI ID | 25.8 | 7 | 37.54 | 0 | |
| 1959 | -2.23 | 5.3x10-8 | gi\|18026574 | transaldolase | MALDI ID | 40.4 | 7 | 37.54 | 0 | |
| 2326 | -1.69 | 6.80E-11 | gi\|73962540 | PREDICTED: similar to Proteasome activator complex subunit 2 (Proteasome activator 28-beta subunit) (PA28beta) (PA28b) (Activator of multicatalytic protease subunit 2) (11S regulator complex beta subunit) (REG-beta) isoform 1 | MALDI ID | 30.1 | 5.5 | 27.5 | 0 | |
| 2445 | -1.88 | 1.7x10-6 | gi\|61862114 | PREDICTED: similar to SET protein (Phosphatase 2A inhibitor I2PP2A) (I-2PP2A) (Template activating factor I) (TAF-I) (Liver regeneration related protein LRRGR00002) (Ab1-115), partial/ (SET beta isoform) | MALDI ID | 32.1 | 4.1 | 29.5 | 0.001 | |
| 2564 | 1.65 | 2.4x10-7 | gi\|14010865 | heat shock 27kDa protein 1 | MALDI ID | 34.1 | 6.1 | 22.86 | 0 | |
| 2733 | 1.52 | 7.1x10-9 | gi\|13386316 | actin related protein M2 | MALDI ID | 12.7 | 5 | 42.05 | 0.002 | |
| 3495 | 1.5 | 1.90E-06 | gi\|6755911\| gi\|549078\| sp\|P10639\| | Thioredoxin I | LC-MS/MS ID | 31.43 | 4.8 | 11.68 | | 3 |
| 3498 | 1.5 | 5.10E-05 | gi\|12230575\| sp\|O75368\| | SH3 domain-binding glutamic acid-rich-like protein | LC-MS/MS ID | 34.21 | 4.87 | 12.81 | | 4 |

**Table 5: High Cell Growth Rate Known Hamster Proteins**

| **Spot no.** | **Average Ratio (Test/Control)** | **p-value** | **gi accession no.** | **Protein name** | **Species** |
|---|---|---|---|---|---|
| 314 | -1.6 | 3.8x10-4 | gi\|600159 | elongation factor 2 | Cricetulus griseus |
| 990 | 1.61 | 2.7x10-10 | gi\|2745838 | Hsp70/Hsp90 organizing protein | Cricetulus griseus |
| 1033 | 1.63 | 2.7x10-7 | gi\|16508150 | ERP57 protein (glucose regulated protein/ protein disulphide isomerase) | Cricetulus griseus |
| 1880 | -1.62 | 2.2x10-4 | gi\|18026574 | transaldolase | Cricetulus griseus |
| 1959 | -2.23 | 5.3x10-8 | gi\|18026574 | transaldolase | Cricetulus griseus |

**Table 6 HCGR3 List**

| **Spot no.** | **Average Ratio (Test/Control)** | **p-value** | **Accession no.** | **Protein name** | **Mass Spec Identification** | **% coverage** | **pI** | **Mr** | **Expectancy value -MALDI** | **No. peptides used for LC-MS/MS ID** |
|---|---|---|---|---|---|---|---|---|---|---|
| | | | | | | | | | | |
| 428 | 3.57 | 1.70E-08 | gi\|600159 | elongation factor 2 | MALDI ID | 29.8 | 6.4 | 96.26 | 0 | |
| 447 | 3.67 | 4.10E-08 | gi\|600159 | elongation factor 2 | MALDI ID | 28.3 | 6.4 | 96.26 | 0 | |
| 705 | 1.84 | 3.6x10-15 | gi\|21704020 | NADH dehydrogenase (ubiquinone) Fe-S protein 1 | MALDI ID | 17.2 | 5.5 | 80.76 | 0 | |
| 716 | 2.32 | 5.90E-06 | gi\|73968066 | PREDICTED: similar to 78 kDa glucose-regulated protein precursor (GRP 78) (Immunoglobulin heavy chain binding protein) (BiP) (Endoplasmic reticulum lumenal Ca(2+) binding protein grp78) isoform 3 | MALDI ID | 30 | 5.1 | 65.46 | 0 | |
| 730 | 1.76 | 0.024 | gi\|73968066 | PREDICTED: similar to 78 kDa glucose-regulated protein precursor (GRP 78) (Immunoglobulin heavy chain binding protein) (BiP) (Endoplasmic reticulum lumenal Ca(2+) binding protein grp78) isoform 3 | MALDI ID | 26.1 | 5.1 | 65.46 | 0 | |
| 797 | -1.87 | 5.8x10-15 | gi\|1339938 | glycerol-3-phosphate dehydrogenase | MALDI ID | 14.3 | 6.2 | 81.49 | 0.002 | |
| 999 | -1.58 | 1.6x10-14 | gi\|34785817 | Copine I | MALDI ID | 10.6 | 5.4 | 59.59 | 0.003 | |
| 1017 | -1.7 | 1.8x10-15 | gi\|34853001 | similar to UDP-N-acteylglucosamine pyrophosphorylase 1-like I | MALDI ID | 11.6 | 5.4 | 57.16 | 0.005 | |
| 1039 | 1.61 | 7.50E-15 | gi\|31981679 | heat shock protein 1 (chaperonin) | MALDI ID | 41.2 | 5.7 | 61.11 | 0 | |
| 1069 | -1.64 | 3.1x10-9 | gi\|27762594 | alpha tubulin | MALDI ID | 21.6 | 4.9 | 50.69 | 0 | |
| 1137 | 1.58 | 9.9x10-14 | gi\|22324680 | FK506 binding protein 4 | MALDI ID | 31.3 | 5.7 | 45.87 | 0.001 | |
| 1180 | 1.54 | 3.9x10-14 | gi\|48675860 | eukaryotic translation initiation factor 2B, subunit 3 gamma | MALDI ID | 13.1 | 6.5 | 51.1 | 0.005 | |
| 1263 | 1.61 | 3.4x10-11 | gi\|3121992 | Aldehyde dehydrogenase, mitochondrial (ALDH class 2) (ALDH1) (ALDH-E2) | MALDI ID | 37 | 5.8 | 54.83 | 0 | |
| 1303 | -1.62 | 1.3x10-13 | gi\|25742757 | glutathione synthetase | MALDI ID | 16.2 | 5.5 | 52.61 | 0 | |
| 1336 | -1.64 | 3x10-10 | gi\|62296810 | Protein disulfide-isomerase A6 precursor (Protein disulfide isomerase P5) (Calcium-binding protein 1) (CaBP1) | MALDI ID | 15.2 | 5 | 48.55 | 0 | |
| 1344 | 1.9 | 4.9x10-15 | gi\|48146175 | EIF3S6 (eukaryotic initiation factor 3, subunit 6) | MALDI ID | 30.8 | 5.7 | 52.59 | 0 | |
| 1376 | 1.92 | 1.7x10-12 | gi\|74007151 | PREDICTED: similar to Alpha enolase (2-phospho-D-glycerate hydro-lyase) (Non-neural enolase) (NNE) (Enolase 1) (Phosphopyruvate hydratase) (C-myc promoter-binding protein) (MBP-1) (MPB-1) (Plasminogen-binding protein) | MALDI ID | 32.3 | 9.4 | 44.26 | 0.009 | |
| 1393 | 1.54 | 9.6x10-12 | gi\|74007151 | PREDICTED: similar to Alpha enolase (2-phospho-D-glycerate hydro-lyase) (Non-neural enolase) (NNE) (Enolase 1) (Phosphopyruvate hydratase) (C-myc promoter-binding protein) (MBP-1) (MPB-1) (Plasminogen-binding protein) | MALDI ID | 32.5 | 9.4 | 44.26 | 0 | |
| 1403 | -1.54 | 1.70E-09 | gi\|73979721 | PREDICTED: similar to Ribonucleoside-diphosphate reductase M2 chain (Ribonucleotide reductase small chain) isoform 1 | MALDI ID | 20.6 | 5.3 | 45.73 | 0.003 | |
| 1438 | -1.6 | 1.3x10-10 | gi\|38197664 | Adhesion regulating molecule 1 | MALDI ID | 15 | 4.9 | 42.43 | 0.004 | |
| 1470 | -1.82 | 1.00E-10 | gi\|70909332 | hypoxia-inducible factor 1, alpha subunit inhibitor | MALDI ID | 14.9 | 5.6 | 40.39 | 0.008 | |
| 1526 | -1.59 | 8.9x10-16 | gi\|1351867 sp\|P48975\| | Actin, cytoplasmic I (Beta-actin) | MALDI ID | 34.9 | 5.2 | 42.06 | 0 | |
| 1706 | -1.82 | 3.10E-13 | gi\|16758446 | isocitrate dehydrogenase 3 (NAD+) alpha | MALDI ID | 24 | 6.5 | 40.05 | 0.001 | |
| 1738 | -1.61 | 1.3x10-15 | gi\|73951310 | PREDICTED: similar to isocitrate dehydrogenase 3 (NAD+) alpha isoform 2 | MALDI ID | 33.2 | 5.9 | 35.1 | 0.008 | |
| 1774 | -1.7 | 6.7x10-16 | gi\|2114406 | aldo-keto reductase | MALDI ID | 23.1 | 6.2 | 36.61 | 0.002 | |
| 1815 | -1.51 | 1.2x10-8 | gi\|1407651 | Lasp-1 | MALDI ID | 41 | 5.1 | 23.08 | 0 | |
| 1916 | -1.68 | 1.6x10-15 | gi\|17391477 | Annexin 5 | MALDI ID | 38.8 | 4.9 | 35.96 | 0 | |
| 2328 | -1.67 | 0 | gi\|17939632 | Platelet-activating factor acetylhydrolase, isoform Ib, beta subunit 30kDa | MALDI ID | 17.5 | 5.6 | 25.72 | 0.003 | |
| 2353 | -1.6 | 4.8x10-10 | gi\|68085578 | Tyrosine 3/tryptophan 5 -monooxygenase activation protein, zeta polypeptide | MALDI ID | 44.5 | 4.7 | 27.87 | 0 | |
| 2371 | 1.55 | 6x10-12 | gi\|17389815 | Triosephosphate isomerase I | MALDI ID | 22.1 | 6.4 | 26.91 | 0.001 | |
| 3034 | -1.69 | 3.70E-11 | gi\|124231\| sp\|P10160\| | Eukaryotic translation initiation factor 5A (elF-5A) (elF-4D) | LC-MS/MS ID | 20.13 | 5.07 | 16.82 | | 4 |
| 3050 | 1.58 | 1.50E-09 | gi\|9910216 AND gi\|73921733\| sp\|Q5RAY0\| | Prefoldin subunit 5 | LC-MS/MS ID | 16.49 | 6.83 | 21.64 | | 3 |
| 3244 | 1.79 | 2.5x10-14 | gi\|14625464 | stathmin | MALDIID | 36.9 | 5.9 | 17.2 | 0.007 | 3 |
| 3892 | 1.56 | 8.30E-10 | gi\|2842685\| sp\|Q91955 | Myotrophin (V-1 protein) (Granule cell differentiation protein) | LC-MS/MS ID | 18.64 | 5.11 I | 12.87 | | 2 |
| ***Statistics used in Decyder analysis,* +/- *1.5 fold change, t-test < 0.05*** | | | | | | | | | | |

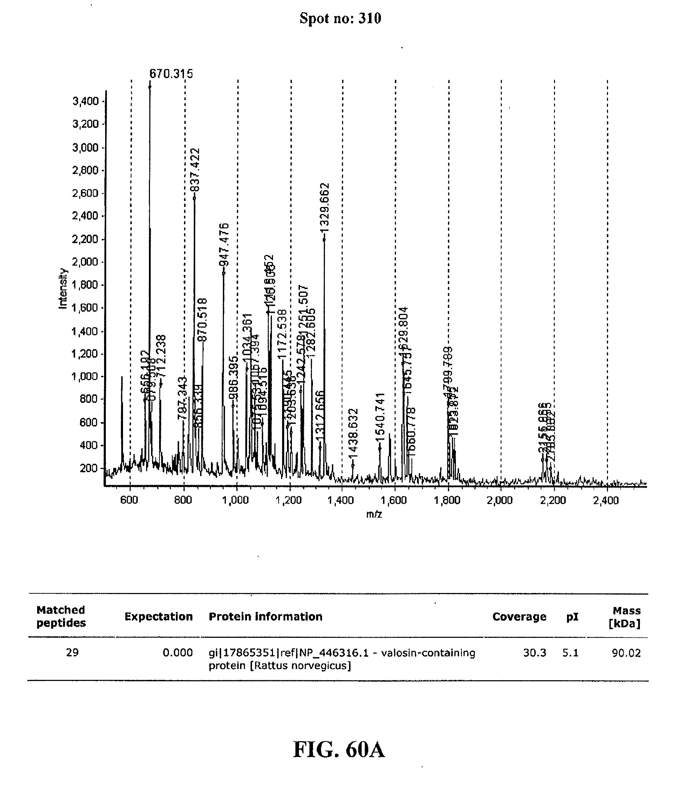

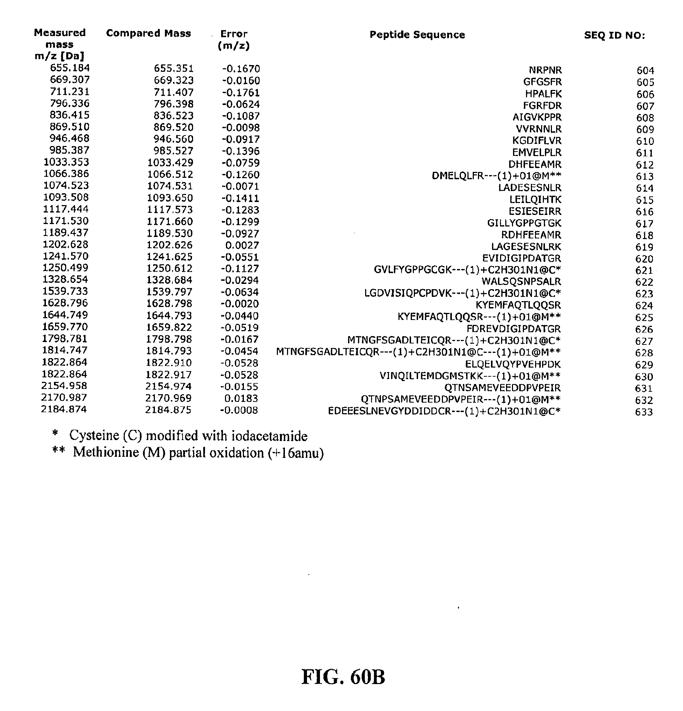

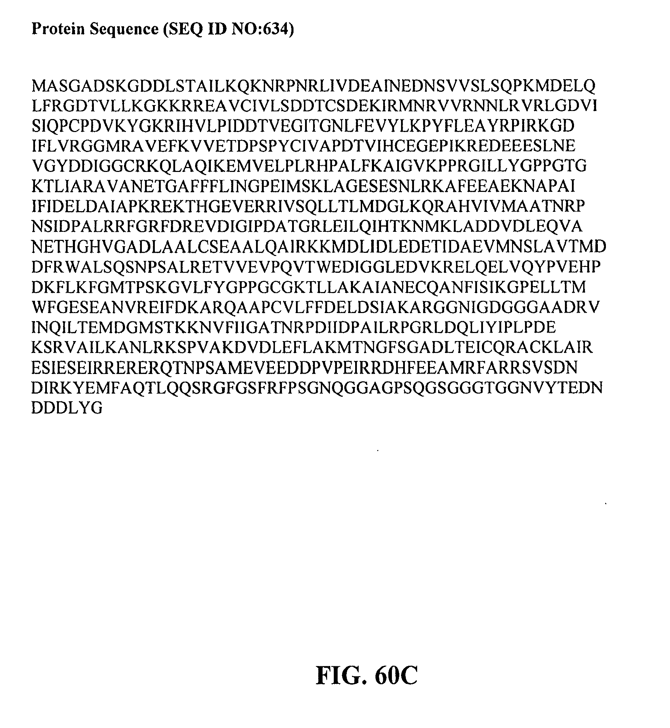

Sequence data for identified proteins are provided in Figures 60 through 112. Each figure provides, for a particular protein spot from the DIGE, the spectrum of molecular weights detected in the tryptic digest; the corresponding protein database match or matches, including the number of peptides matched to the predicted tryptic peptides for the protein database entry, the accession number, name, and species of the protein from the database entry, the percent coverage, the isoelectric point and mass; for each molecular weight matched with a predicted mass of a predicted peptide, the measured mass, the predicted (compared) mass, the difference between the two, and the corresponding peptide sequence; and the full length sequence of the protein from the database entry.

### Example 4. Proteins differentially expressed in cells with sustained high cell viability or high peak cell density

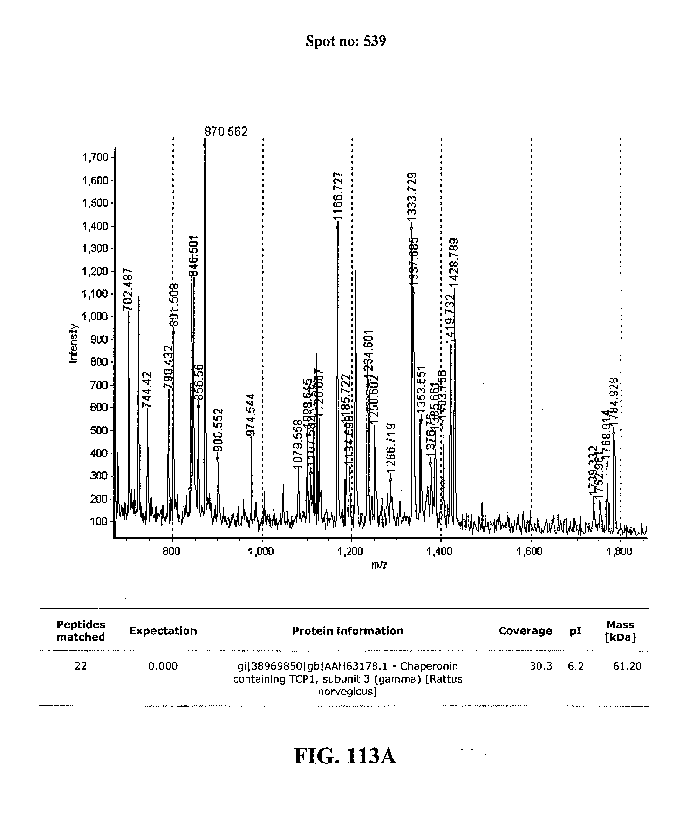

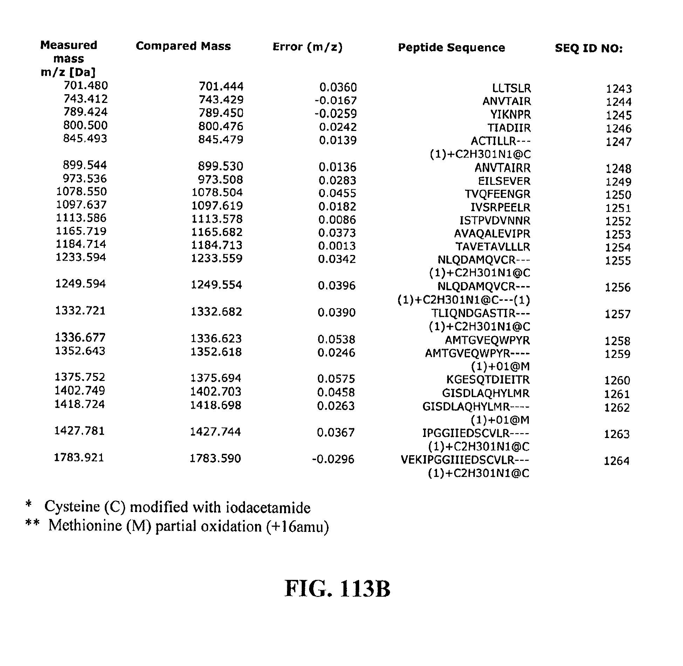

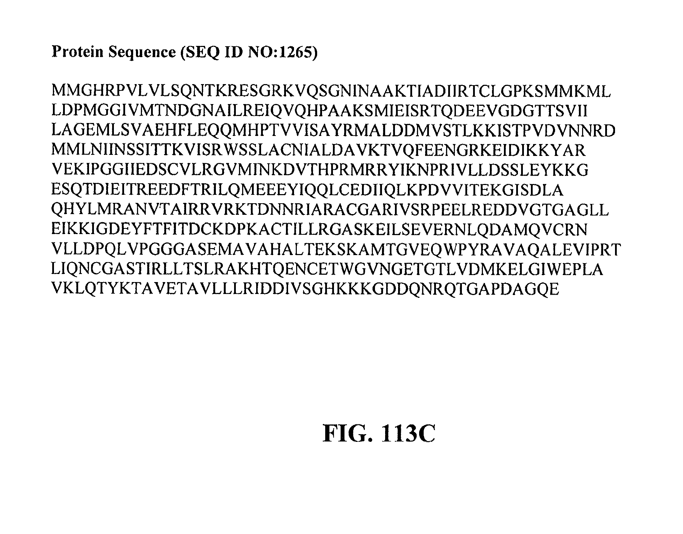

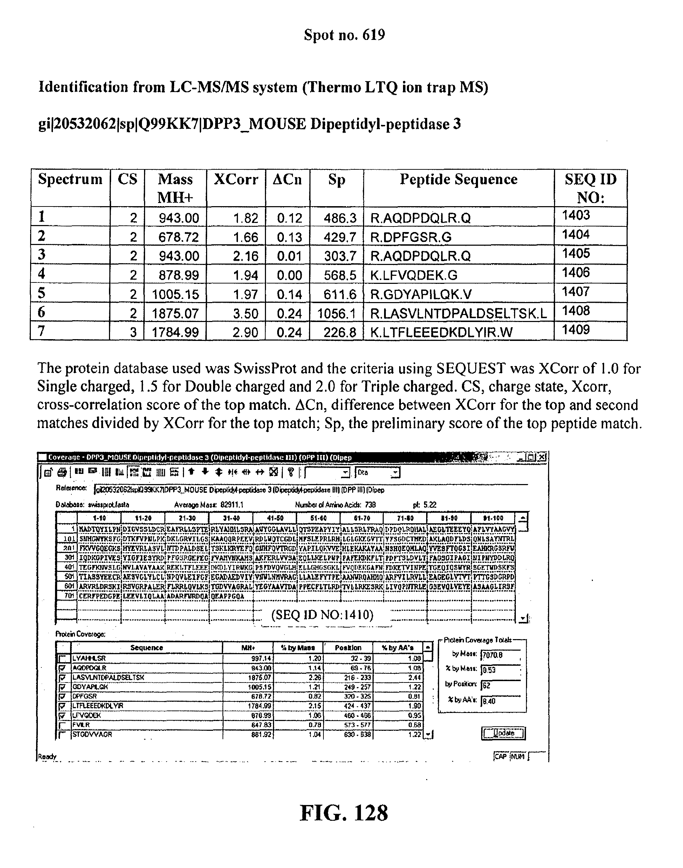

Table 7 lists several of the spots identified as differentially expressed in the cells with sustained high cell viability using methods as described in Example 3. Sequence data for the identified proteins are provided in Figures 113 through 127. Table 8 lists several of the spots identified as differentially expressed in the cells with high peak cell density using similar methods; corrresponding sequence data are shown in Figures 128 through 138. The tables provide, for each spot number, the fold difference in protein levels between the test and control samples, labeled as "Average Ratio"; proteins whose levels are reduced in the test samples are indicated with a negative sign. The tables also provide the p-value that the differences in expression would be the result of random chance and the protein name and accession number corresponding to any identified amino acid sequence. The resulting peptides were analyzed by mass spectrometry. MALDI is used, particularly for highly abundant samples on gels, for peptide mass fingerprinting. For lower abundance samples, LC-MS/MS using an MDLC LTQ machine is used. In the MALDI sequence analysis, the molecular weights of the trypsin fragments were compared to predicted molecular weights of trypsin fragments of known sequences. In the tables, "% coverage" refers to the percentage of the total length of a database sequence for which corresponding trypsin fragments were detected in the experiment. pI and M_{R} refer to the apparent isoelectric point and apparent molecular weight of the protein spot.

**Table 7. Differentially expressed proteins in cells with sustained high cell viability (Statistics used in Decyder analysis, +/- 1.5 fold change, t-test < 0.05)**

| **Spot no.** | **Average Ratio (Test/Control)** | **p-value** | **Accession no.** | **Protein name** | **Mass Spec Identification** | **% coverage** | **p*I*** | **Mr** | **Expect ancy value-MALDI** | **No. peptides used for LC-MS/MS ID** |
|---|---|---|---|---|---|---|---|---|---|---|
| | | | | | | | | | | |
| 539 | 1.93 | 1.90E-05 | gi\|38969850 | Chaperonin containing TCP I, subunit 3 (gamma) | MALDI ID | 30.3 | 6.2 | 61.2 | 0 | |
| 559 | -1.53 | 0.03 | gi\|15030102 | Sdha protein (succinate dehydrogenase) | MALDI ID | 23.1 | 7.2 | 73.39 | 0 | |
| 800 | 2.89 | 2.90E-10 | gi\|21618633 | 3-hydroxy-3 methylglutaryl-Coenzyme A synthase 1 | MALDI ID | 13.3 | 5.6 | 58.16 | 0.005 | |
| 870 | -1.84 | 0.014 | gi\|23272966\| | Atp5b protein | MALDI ID | 21.4 | 5.2 | 56.65 | 0 | |
| 915 | -1.54 | 0.04 | gi\|3121992\| sp\|P81178\| | Aldehyde dehydrogenase, mitochondrial (ALDH class 2) | MALDI ID | 35.6 | 5.8 | 54.83 | 0 | |
| 1393 | -1.61 | 0.0082 | gi\|18026574 gi\|46396972\| sp\|Q8VI73\| | transaldolase | MALDI ID | 29.4 | 7 | 37.54 | 0 | |
| 2022 | 1.53 | 0.00054 | gi\|47496673 | GRP2 (Growth factor receptor-bound protein 2) | MALDI ID | 52.5 | 6.1 | 25.24 | 0 | |
| 2050 | -1.58 | 0.023 | gi\|34879492 | PREDICTED: similar to tumor protein, translationally-controlled 1 | MALDI ID | 18.2 | 5.2 | 19.09 | 0.009 | |
| 2250 | -1.63 | 0.01 | gi\|1313936 | mitochondrial ribosomal protein | MALDI ID | 22.7 | 9.4 | 21.69 | 0.005 | |
| 2459 | 2.08 | 0.0021 | gi\|3212116 | prefoldin subunit 2 | MALDI ID | 40.9 | 6.8 | 16.82 | 0 | |
| 2543 | 1.72 | 2.80E-05 | gi\|14625464 | stathmin | MALDI ID | 53.7 | 5.9 | 17.2 | 0 | |
| 2863 | 1.72 | 0.013 | gi\|9963901 | profilin II | MALDI ID | 29.5 | 6.6 | 16.05 | 0 | |
| 2897 | 1.77 | 0.0013 | gi\|73961217 | PREDICTED: similar to ATP synthase alpha chain, mitochondrial precursor isoform 3 | MALDI ID | 31.4 | 6.1 | 12.82 | 0.003 | |
| 3340 | -7.54 | 7.90E-13 | gi\|2493416\| sp\|Q99584 | S100 calcium-binding protein A13 | LC-MS/MS ID | 17.35 | 5.9 | 11.47 | | 2 |
| 3349 | -1.55 | 0.00011 | gi\|50401358\| sp\|P62504\| | Calpactin I light chain (S100 calcium-binding protein A10) | LC-MS/MS ID | 20.62 | 6.82 | 11.2 | | 2 |

**Table 8 HCD3 Protein List All test samples vs all control samples (day 3, 5, 7), (Filters - 1.2 fold up/down regulation, t-test < 0.01, 2-way anova <0.01, Decyder statistical analysis)**

| **Spot no.** | **Fold Change (All Test/All Control)** | **Accession number** | **Protein Name** | **Mass Spec Identification** | **% coverage** | **pI** | **Mr** | **Expect ancy value -MALDI** | **No. peptides used for LC-MS/MS ID** |
|---|---|---|---|---|---|---|---|---|---|
| | | | | | | | | | |
| 619 | -1.34 | gi\|20532062\| sp\|Q99KK7\| | Dipeptidyl-peptidase 3 (Dipeptidyl-peptidase III) | LC-MS/MS ID | 8.4 | 5.22 | 82.29 | | 7 |
| 984 | 1.2 | gi\|75773247\| | Chaperonin subunit 6a (zeta) | MALDI ID | 20.3 | 6.6 | 58.46 | 0.007 | |
| 1078 | 1.2 | gi\|179102\| | aspartyl-tRNA synthetase | MALDI ID | 16.4 | 6.2 | 57.57 | 0.005 | |
| 1148 | -1.23 | gi\|135446\| sp\|P09203\| | Tubulin beta-1 chain (Beta-tubulin class-I) | LC-MS/MS ID | 6.52 | 4.78 | 49.91 | | 3 |
| 1154 | -1.25 | gi\|1353212\| sp\|P48670\| | Vimentin | LC-MS/MS ID | 4.69 | 4.94 | 51.85 | | 2 |
| 1157 | 1.3 | gi\|62511005\| sp\|Q60HD7\| | D-3-phosphoglycerate dehydrogenase | LC-MS/MS ID | 10.88 | 6.48 | 56.55 | | 5 |
| 1373 | -1.24 | gi\|729443\| sp\|P38660\| | Protein disulfide-isomerase A6 precursor | LC-MS/MS ID | 20.73 | 5.04 | 48.16 | | 8 |
| 1382 | -1.24 | gi\|729443\| sp\|P38660\| | Protein disulfide-isomerase A6 precursor | LC-MS/MS ID | | 5.04 | 48.16 | | |
| 1500 | -1.23 | gi\|548710\|sp\|Q05 186\| | Reticulocalbin-1 precursor | LC-MS/MS ID | 15.08 | 4.7 | 38.11 | | 7 |
| 1656 | 1.24 | gi\|729023\| sp\|P24452 | Macrophage capping protein (Myc basic motif homolog 1) | LC-MS/MS ID | 23.86 | 6.73 | 39.24 | | 6 |
| 1682 | 1.32 | gi\|21466051 | Chain A, Crystal Structure Of The Mitochondrial Serine Protease Htra2 | MALDI ID | 20.9 | 6.1 | 34.95 | 0 | |
| 2324 | -1.31 | gi\|61227509\| sp\|Q64361\| | Latexin (Endogenous carboxypeptidase inhibitor) | LC-MS/MS ID | 14.8 | 5.77 | 25.58 | | 3 |

### Example 5. mRNA expression profiling

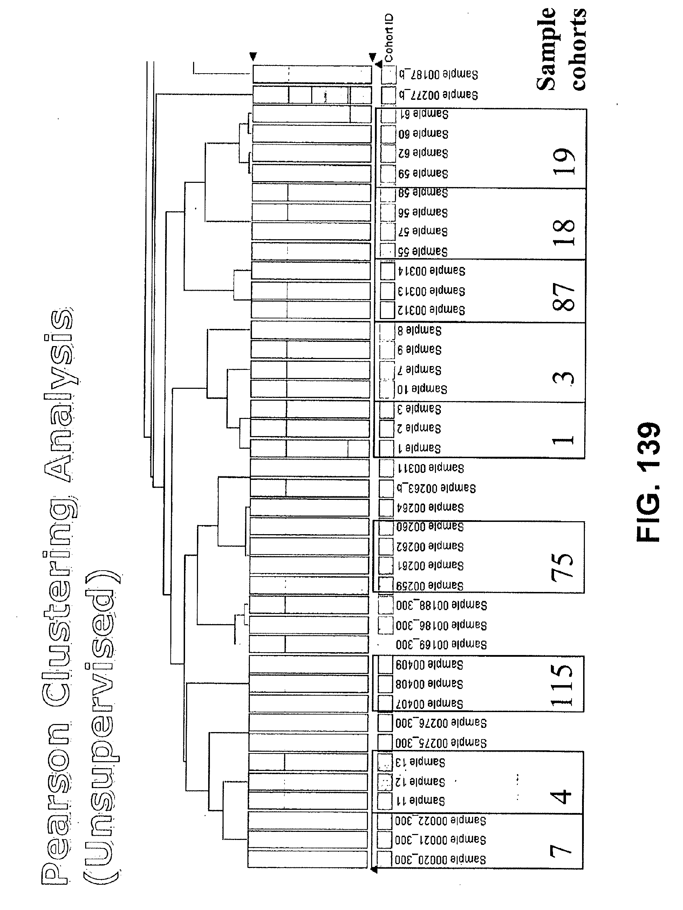

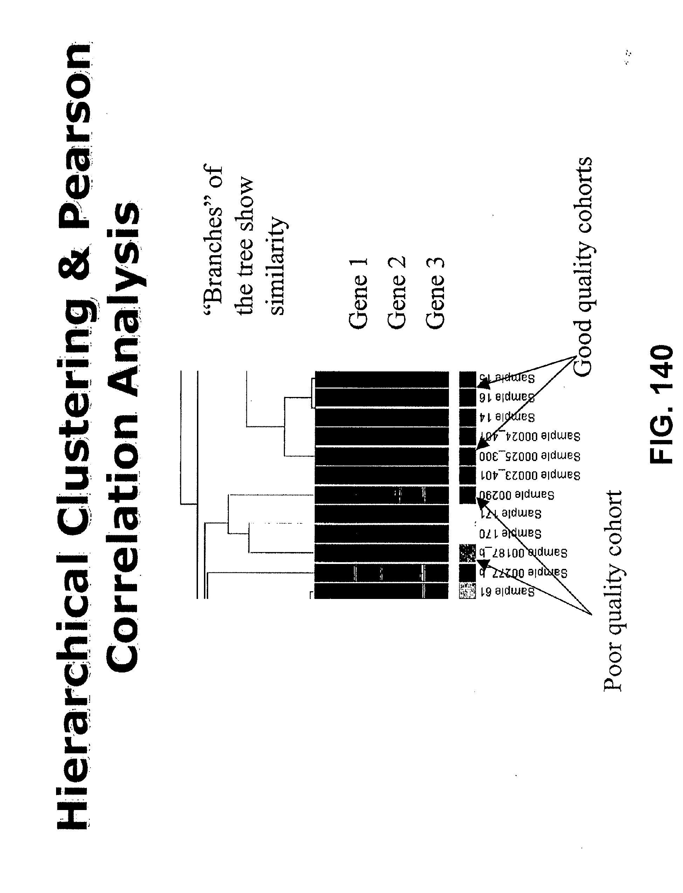

RNA samples from test and control CHO cell lines were obtained and analyzed on a microchip containing probes for CHO mRNA sequences as described in U.S. Patent Application Publication US2006/0010513, the complete contents of which are herein incorporated by reference. The hybridization cocktail was spiked with a fragmented cRNA standard to generate a standard curve using labeled, fragmented cRNA of control sequences at known concentrations, permitting normalization of the data and assessment of chip sensitivity and saturation. The scan data were quality controlled using the 3'/5' ratio of β-actin and GAPDH, the signal intensity and consistency, and the percent present. Generally, data normalization was performed using software tools Affy 5.0 and Genesis 2.0; or dChiP (see Li et al. (2001) Proc. Natl. Acad. Sci. USA 98:31-36 and Li et al. (2001) Genome Biol. 2:0032.1-0032.11) and Genespring. A P_{Value} less than or equal to 0.05 and a fold-change minimum between the test and control lines of 1.2 was required before a gene would be further considered. An unsupervised Pearson Clustering Analysis is depicted in Figures 139 and 140.

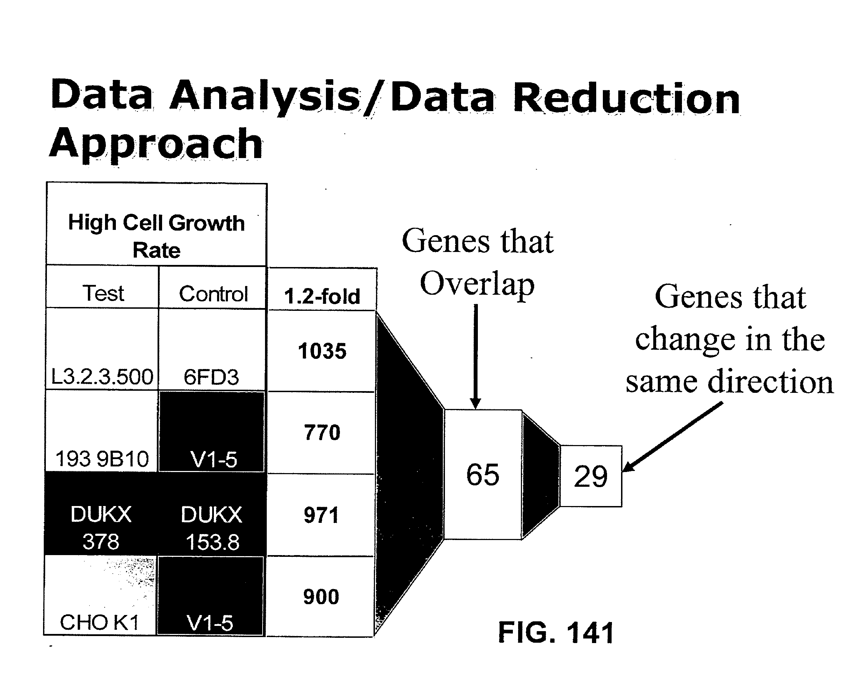

An exemplary method of data analysis is depicted in Figure 141. Pairs of test and control cell lines for the high cell growth rate were compared and mRNA expression patterns meeting the 1.2-fold difference requirement were identified. Of those, the 65 genes that were differentially expressed in each of four different pairs of test and control cell lines were identifed. Of the 65, 29 were either consistently up-regulated or consistently down-regulated in the test cell lines; these were given a higher priority for further analysis.

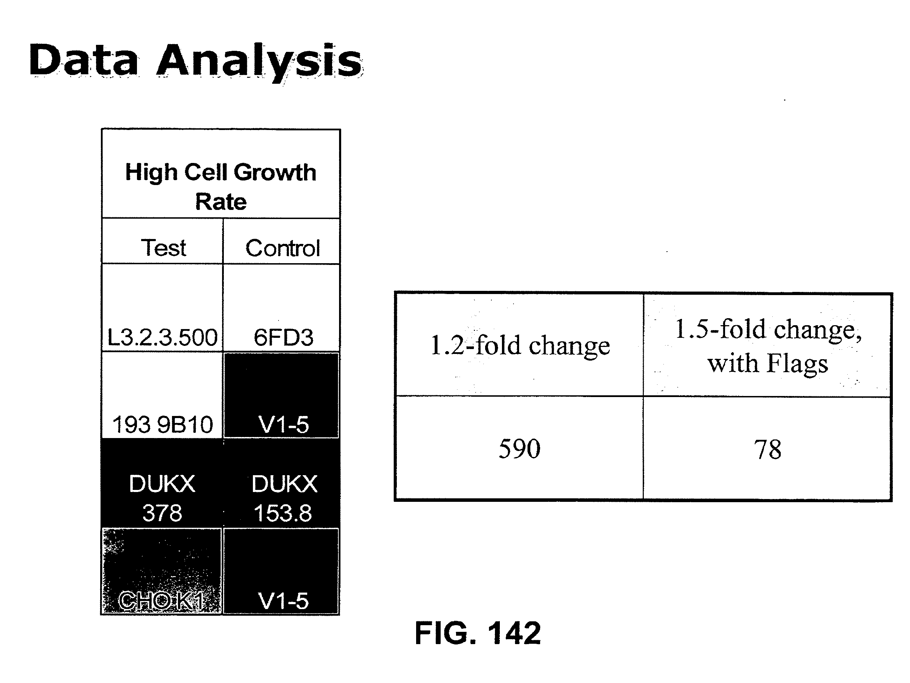

An exemplary method of data analysis that does not rely on pairwise differences is depicted in Figure 142. 590 genes were identified whose average expression levels in the high cell growth rate test CHO cell lines as a group were at least 1.2-fold higher than the average expression in the group of control CHO cell lines. When a 1.5-fold difference in expression was required and additional, more stringent statistical analysis was applied, 78 genes passed the criteria; these were given a higher priority for further analysis.

### Example 6. Genes differentially expressed in cells with high maximum cellular productivity

A summary of nucleic acids identified as differentially expressed in cells with high maximum cellular productivity is provided in Tables 9 and 10. For each nucleic acid, a qualifier name, symbol, and title are provided, as well as whether the nucleic acid is up-regulated or down-regulated in the cells with higher maximum cellular productivity. For nucleic acids with human or mouse homologs in the Unigene database, the table provides Unigene ID numbers and statistics relating to the comparison, including e-values, percent sequence identities between the CHO sequence and the Unigene databank entries, and percent coverage ("% QC").

Nucleic acids encoding proteins associated with the endoplasmic reticulum (ER) or the Golgi complex may contribute to cellular productivity, particularly for the production of a secreted protein. Table 11 summarizes nucleic acids that are differentially expressed by a factor of at least 1.2 in cells overexpressing PACE and encode an ER-associated protein. Table 12 summarizes nucleic acids that are differentially expressed by a factor of at least 1.2 in cells overexpressing PACE and encode a Golgi-associated protein.

**Table 9: High Max Qp NA Unknown CHO Sequences**

| Qualifier List | Symbol | Title | Human Unigene ID | eValue | %ID | %QC | Mouse Unigene ID | eValue | %ID | %QC | |
|---|---|---|---|---|---|---|---|---|---|---|---|
| WAN0088NU_at (SEQ ID NO:1478) | LMNA | Lamin A/C | Hs.491359 | 3E-23 | 85.7143 | 23.0503 | Mm.243014 | 0 | 92.04947 | 98.0936 | down |
| WAN0088OT_at (SEQ ID NO:1479) | NA | WAN0088OT 10595D-F11 | #N/A | | | | #N/A | | | | down |
| WAN0088PY_at (SEQ ID NO:1480) | DDX5 | DEAD (Asp-Glu_Ala-Asp) box polypeptide 5 | Hs.416922 | 0.001 | 91.6667 | 6.2069 | Mm.220038 | 3.00E-16 | 93.05556 | 12.4138 | down |
| WAN0088T3_at (SEQ ID NO:1481) | ANXA1 | Annexin A1 | Hs.494173 | 6E-76 | 87.372 | 71.1165 | Mm.248360 | 1E-114 | 92.13115 | 74.0291 | down |
| WAN0088ZP_at (SEQ ID NO:1482) | PAWR | PRKC, apoptosis, WT1, regulator | Hs.406074 | 4E-10 | 91.5254 | 11.1111 | Mm.336104 | 9E-53 | 91.62562 | 38.2298 | up |
| WAN00895Y_f_at (SEQ ID NO:1483) | DQ390542.2 | Mitochondrial cytochrome b | #N/A | | | | Mm.369891 | 0.001 | 91.42857 | 19.5531 | down |
| WAN008C42_at (SEQ ID NO:1484) | CD36 | CD36 antigen | Hs.120949 | 1.00E-05 | 90.9091 | 3.05132 | Mm.18628 | 7.00E-19 | 85.71429 | 9.2233 | down |
| WAN008CJ1_at (SEQ ID NO:1485) | ERP70 | Protein disulfide isomerase-associated 4 | Hs.93659 | 1E-120 | 89.6277 | 81.9172 | Mm.2442 | 1E-170 | 94.57364 | 84.3137 | up |
| WAN008DT7_at (SEQ ID NO:1486) | GSTO1 | Glutathione S-transferase omega 1 | Hs.190028 | 5E-65 | 83.9437 | 60.8919 | Mm.378931 | 1E-102 | 87.27273 | 66.0377 | down |
| WAN008EA0_at (SEQ ID NO:1487) | VCP | Valosin-containing protein | Hs.529782 | 0 | 90.7273 | 99.6377 | Mm.379457 | 0 | 95.47101 | 100 | up |
| WAN008F1I_at (SEQ ID NO:1488) | SHOC2 | Soc-2 suppressor of clear homolog (C. elegans) | Ks.104315 | 2E-18 | 85.0575 | 31.0714 | Mm.228669 | 1 E-36 | 90.37037 | 24.1071 | down |
| WAN008F2S_at (SEQ ID NO:1489) | NA | WAN008F2S 11165A-F02 | #N/A | | | | #N/A | | | | down |
| WAN013HX8_x_at (SEQ ID NO:1490) | EIF4A2 | Eukaryotic translation initiation factor 4A, isoform 2 | Hs.478553 | 0 | 90.824 | 98.3425 | Mm.260084 | 0 | 92.50936 | 98.3425 | up |
| WAN013HXR_x_at (SEQ ID NO:1491) | STAT6 | signal transducer and activator of transcription 6 | #N/A | | | | Mm.336898 | 2.00E-19 | 89.62264 | 7.89278 | down |
| WAN013l1P_at (SEQ ID NO:1492) | HNRPA2B1 | Heterogeneous nuclear ribonucleoprotein A2/B1 | Hs.487774 | 0 | 97.2222 | 90.9474 | Mm.155896 | 0 | 96.52778 | 90.9474 | down |
| WAN01311T_x_at (SEQ ID NO:1493) | DQ390542.2 | Mitochondrial NADH dehydrogenase subunit 2 | #N/A | | | | #N/A | | | | down |
| WAN013l66_f_at (SEQ ID NO:1494) | Vim | Vimentin (Vim), mRNA hypoxanthine | Hs.533317 | 1E-126 | 92.2559 | 57.6699 | Mm.268000 | 1E-131 | 91.84953 | 61.9417 | down |
| WAN013lA0_at (SEQ ID NO:1495) | HPRT1 | phosphoribosyltransferase 1 (Lesch-Nylan syndrome) | Hs.412707 | 2.00E-66 | 83.0986 | 17.6727 | Mm.299381 | 4.00E-70 | 83.10811 | 18.4194 | down |
| WAN013IAB_x_at (SEQ ID NO:1496) | TP53 | Tumor protein p53 (Li-Fraumeni syndrome) | Hs.408312 | 1E-150 | 82.4477 | 48.8592 | #N/A | 1 E-133 | 81.32045 | 48.8592 | down |

**Table 10: High Max Qp NA Known CHO Sequences**

| Qualifier List | Symbol | Title | Human Unigene ID | eValue | %ID | %QC | Mouse Unigene ID | eValue | %ID | %QC | Direction of change (test vs control) |
|---|---|---|---|---|---|---|---|---|---|---|---|
| AF325501_at (SEQ ID NO:1497) | LY96 | Lymphocyte antigen 96 | Hs.69328 | 2E-17 | 79.2727 | 74.3243 | Mm.116844 | 1 E-78 | 85.20548 | 98.6486 | down |
| D45419_at (SEQ ID NO:1498) | Hcfc1 | Host cell factor C1 | Hs.83634 | 1E-22 | 84.8649 | 32.7434 | Mm.248353 | 1E-123 | 85.99291 | 99.823 | down |
| K00924_at (SEQ ID NO:1499) | VIM | Vimentin | Hs.533317 | 5E-44 | 92.9134 | 56.4444 | Mm.268000 | 3E-47 | 94.35484 | 55.1111 | down |
| L00176_at (SEQ ID NO:1500) | HMGCR | 3-hydroxy-3-methylglutaryl-Coenzyme A reductase | Hs.11899 | 6E-54 | 87.9808 | 57.9387 | Mm.316652 | 4E-82 | 94.47236 | 55.4318 | up |
| L18986_at (SEQ ID NO:1501) | LAMP1 | Lysosomal-associated membrane protein 1 | Hs.494419 | 1E-82 | 96.7136 | 16.2595 | Mm.16716 | 1E-160 | 87.11864 | 45.0382 | down |
| U48852_at (SEQ ID NO:1502) | CRELD2 | Cysteine-rich with EGF-like domains 2 | Hs.211282 | 1E-109 | 81.7694 | 55.1367 | Mm.292567 | 0 | 88.79936 | 91.7221 | up |

**Table 11: CHO Sequences Differentially Expressed in PACE Overexpressing Cells and Encoding ER-Associated Proteins**

| Qualifier List | Symbol | Title | Human Unigene ID | eValue | %ID | %QC | Mouse Unigene ID | eValue | %ID | %QC | Fold change | Direction of change |
|---|---|---|---|---|---|---|---|---|---|---|---|---|
| WAN008DRM_at (SEQ ID NO:1503) | EPHX1 | Epoxide hydrolase 1, microsomal (Ephx1) | Hs.89649 | 9E-85 | 87.987 | 60.392 | Mm.9075 | 1E-113 | 91.223 | 62.549 | 1.319 | up |
| WAN0088T7_at (SEQ ID NO:1504) | Cyp51 | Cytochrome P450, family 51, subfamily A, polypeptide 1 (CYP51A1) | Hs.417077 | 1E-132 | 86.879 | 98.051 | Mm.46044 | 1E-152 | 88.515 | 98.441 | 1.297 | up |
| WAN008ELH_at (SEQ ID NO:1505) | RPN1 | Ribophorin I | Hs.518244 | 0 | 90.519 | 99.643 | Mm.188544 | 0 | 92.335 | 100.000 | 1.295 | up |
| WAN0088K7_x_at (SEQ ID NO:1506) | HSPA5 | Heat shock 70kDa protein 5 (glucose-regulated protein, 78kDa) | | 0 | 0.000 | 0.000 | Mm.330160 | 0.000009 | 100.000 | 6.923 | 3.401 | up |
| L00176_at (SEQ ID NO:1500) | HMGCR | 3-hydroxy-3-methylglutaryl-Coenzyme A reductase | Hs.11899 | 7E-54 | 87.981 | 57.939 | Mm.316652 | 3E-82 | 94.472 | 55.432 | 2.551 | up |
| L00178_at (SEQ ID NO:1507) | HMGCR | 3-hydroxy-3-methylglutaryl-Coenzyme A reductase | Hs.11899 | 3E-47 | 90.062 | 42.819 | Mm.316652 | 1 E-57 | 93.038 | 42.021 | 2.033 | up |
| L00169_at (SEQ ID NO:1508) | HMGCR | 3-hydroxy-3-methylglutaryl-Coenzyme A reductase | Hs.11899 | 2E-19 | 89.535 | 33.992 | Mm.316652 | 7E-27 | 94.872 | 30.830 | 2.020 | up |
| L00180_at (SEQ ID NO:1509) | HMGCR | 3-hydroxy-3-methylglutaryl-Coenzyme A reductase | Hs.11899 | 5E-35 | 86.310 | 68.016 | Mm.316652 | 1E-49 | 90.244 | 66.397 | 1.988 | up |
| L00181_at (SEQ ID NO:1510) | HMGCR | 3-hydroxy-3-methylglutaryl-Coenzyme A reductase | Hs.11899 | 1E-37 | 89.781 | 32.697 | Mm.316652 | 3E-52 | 93.617 | 33.652 | 1.976 | up |
| L00171_at (SEQ ID NO:1511) | HMGCR | 3-hydroxy-3-methylglutaryl-Coenzyme A reductase | Hs.11899 | 5E-36 | 91.525 | 33.908 | Mm.316652 | 5E-41 | 93.220 | 33.908 | 1.934 | up |
| L00170_x_at (SEQ ID NO:1512) | HMGCR | 3-hydroxy-3-methylglutaryl-Coenzyme A reductase | Hs.11899 | 7E-27 | 89.091 | 70.968 | Mm.316652 | 6E-54 | 94.815 | 87.097 | 1.718 | up |
| L00173_at (SEQ ID NO:1513) | HMGCR | 3-hydroxy-3-methylglutaryl-Coenzyme A reductase | Hs.11899 | | 2E-33 85.882 | 30.466 | Mm.316652 | 8E-78 | 89.919 | 44.444 | 1.621 | up |
| L00182_at (SEQ ID NO:1514) | HMGCR | 3-hydroxy-3-methylglutaryl-Coenzyme A reductase | Hs.11899 | | 3E-65 94.012 | 48.688 | Mm.316652 | 2E-65 | 94.479 | 47.522 | 1.546 | up |
| AF380341_at (SEQ ID NO:1515) | CANX | Calnexin | Hs.567968 | 1E-101 | 93.359 | 67.016 | Mm.248827 | 1E-114 | 95.000 | 68.063 | 3.165 | up |
| WAN013l86_x_at (SEQ ID NO:1516) | CANX | Calnexin | Hs.567968 | 1E-111 | 86.053 | 97.187 | Mm.248827 | 1E-169 | 92.072 | 100.000 | 1.508 | up |
| WAN008ES3_at (SEQ ID NO:1517) | CANX | Calnexin | Hs.567968 | 5E-18 | 88.421 | 22.565 | Mm.248827 | 1E-114 | 92.562 | 86.223 | 1.376 | up |
| WAN008EHW_at (SEQ ID NO:1518) | OPRS1 | Opioid receptor, sigma 1 | Hs.522087 | 1E-141 | 87.776 | 95.777 | Mm.29025 | 1E-163 | 89.349 | 97.313 | 1.618 | up |
| X15652_at (SEQ ID NO:1519) | NSF | N-ethylmaleimide-sensitive factor Homocysteine-inducible, endoplasmic reticulum stress-inducible, ubiquitin-like domain member 1 | Hs.431279 | 0 | 89.899 | 99.331 | Mm.260117 | 0 | 94.649 | 100.000 | 1.346 | up |
| WAN0088XH_at (SEQ ID NO:1520) | HERPUD1 | | Hs.146393 | 7E-79 | 87.417 | 68.481 | Mm.29151 | 1 E-1 50 | 91.463 | 92.971 | 1.247 | up |
| WAN008EED_at (SEQ ID NO:1521) | Sc5d | Sterol-C5-desaturase (fungal ERG3, delta-5-desaturase) homolog (S. cerevisae) | Hs.287749 | 2E-42 | 85.446 | 40.727 | Mm.32700 | 1E-98 | 87.705 | 69.981 | 2.387 | up |
| WAN008CT8_at (SEQ ID NO:1522) | AP2M1 | Adaptor-related protein complex 2, mu 1 subunit | Hs.518460 | 0 | 94.384 | 80.803 | Mm.18946 | 0 | 95.484 | 81.152 | 1.385 | up |
| WAN008CJ1_at (SEO ID NO:1485) | ERP70 | Protein disulfide isomerase-associated 4 | Hs.93659 | 1E-120 | 89.628 | 81.917 | Mm.2442 | 1E-170 | 94.574 | 84.314 | 2.985 | up |
| WAN013I5F_at (SEQ ID NO:1523) | SIAT8D | ST8 alpha-N-acetyl-neuraminide alpha-2,8-sialyltransferase 4 | Hs.308628 | 0 | 90.621 | 61.296 | Mm.306228 | 0 | 90.673 | 90.283 | 1.370 | up |
| WAN0088XZ_at (SEQ ID NO:1524) | RTN3 | Reticulon 3 | Hs.473761 | 1E-167 | 91.126 | 83.848 | Mm.246990 | 0 | 92.545 | 99.819 | 1.546 | up |
| WAN01319D_at (SEQ ID NO:1525) | HYOU1 | Hypoxia up-regulated 1 | Hs.277704 | 4E-72 | 85.498 | 26.417 | Mm.116721 | 1E-122 | 92.236 | 25.698 | 1.695 | up |
| WAN008DUB_at (SEQ ID NO:1526) | ROH11 | Retinol dehydrogenase 11 (Rdh11) | Hs.226007 | 1E-77 | 84.840 | 81.385 | Mm.291799 | 3E-89 | 91.373 | 55.195 | 1.414 | up |
| WAN008ELW_f_at (SEQ ID NO:1527) | Sec13I1 | SEC13-like 1 (S. cerevisiae) | Hs.166924 | 8E-22 | 93.151 | 87.952 | Mm.29296 | 8E-24 | 92.500 | 96.386 | 1.302 | up |
| WAN013I30_at (SEQ ID NO:1528) | TRA1 | Tumor rejection antigen (gp96) 1 | Hs.192374 | 0 | 90.545 | 100.000 | Mm.87773 | 0 | 93.273 | 100.000 | 2.849 | up |
| WAN013HWO_x_at (SEQ ID NO:1529) | TRA1 | Tumor rejection antigen (gp96) 1 | Hs.192374 | 6E-51 | 85.106 | 94.000 | Mm.87773 | 2E-66 | 87.805 | 98.400 | 2.092 | up |
| WAN0088ZO_x_at (SEQ ID NO:1530) | SYNCRIP | Synaptotagmin binding, cytoplasmic RNA interacting protein | Hs.472056 | 1E-50 | 94.615 | 70.652 | Mm.32874 | 3E-56 | 95.556 | 73.370 | 2.198 | up |
| WAN00894J_at (SEQ ID NO:1531) | ZMPSTE24 | Zinc metallopeptidase (STE24 homolog, yeast) | Hs.591501 | 1E-146 | 88.727 | 93.922 | Mm.34399 | 0 | 92.277 | 99.020 | 1.484 | up |
| WAN013I4D_at (SEQ ID NO:1532) | TAP2 | Transporter 2, ATP-binding cassette, sub-family B (MDR/TAP) | Hs.502 | 5E-33 | 83.258 | 46.331 | Mm.14814 | 1E-111 | 88.950 | 75.891 | -1.914 | down |
| AF323965_at (SEQ ID NO:1533) | CYP11A1 | Cytochrome P450, family 11, subfamily A, polypeptide I | Hs.303980 | 1 E-1 75 | 82.511 | 86.034 | Mm.302865 | 0 | 91.004 | 88.349 | -1.492 | down |
| WAN013HX5_at (SEQ ID NO:1534) | MGST1 | Microsomal glutathione S-transferase 1 | Hs.389700 | 3E-28 | 78.987 | 81.950 | Mm.14796 | 1E-152 | 89.394 | 95.851 | -1.483 | down |
| AJ298842_at (SEQ ID NO:1535) | Dyt1 | Torsin family 1, member A (torsin A) | Hs.534312 | 2E-89 | 87.209 | 58.703 | Mm.154994 | 1E-153 | 94.366 | 60.580 | -1.251 | down |
| AF004831_at (SEQ ID NO:1536) | SPTLC1 | Serine palmitoyltransferase, long chain base subunit 1 | Hs.90458 | 1E-18 | 88.889 | 6.767 | Mm.240336 | 5E-84 | 89.441 | 24.211 | -2.201 | down |
| WAN013I4M_at (SEQ ID NO:1537) | ENTPD5 | Ectonucleoside triphosphate diphosphohydrolase 5 | Hs.131555 | 2E-48 | 90.341 | 32.653 | Mm.10211 | 8E-44 | 88.298 | 34.879 | -1.618 | down |
| WAN013I65_at (SEQ ID NO:1538) | DPAGT1 | Dolichyl-phosphate (UDP-N-acetylglucosamine) N-acetylglucosaminephosphotransferase 1 (GlcNAc-1-P transferase) | Hs.524081 | 0 | 90.962 | 39.334 | Mm.18353 | 1E-178 | 90.267 | 39.637 | -1.455 | down |
| WAN0088KG_at | PPGB | Protective protein for beta- | Hs.517076 | 1E-115 | 87.589 | 72.870 | Mm.359633 | 1E-149 | 90.931 | 72.870 | -3.26 | down |
| (SEQ ID NO:1539) WAN0088TG_at | | galactosidase (galactosialidosis) | | | | | | | | | | |
| (SEQ ID NO:1540) | SRP72 | Signal recognition particle 72kDa | Hs.237825 | 1E-58 | 89.041 | 50.812 | Mm.296976 | 1E-119 | 92.401 | 76.334 | -1.416 | down |
| WAN013I39_at (SEQ ID NO:1541) | GGA2 | Golgi associated, gamma adaptin ear containing, ARF binding protein 2 | Hs.460336 | 7E-30 | 84.454 | 46.667 | Mm.29619 | 1E-147 | 93.333 | 79.412 | -1.474 | down |
| WAN008CUO_at (SEQ ID NO:1542) | GGA2 | Golgi associated, gamma adaptin ear containing, ARF binding protein 2 | Hs.460336 | 4E-31 | 89.344 | 25.957 | Mm_29619 | 1E-146 | 90.364 | 99.362 | -1.253 | down |

**Table 12: CHO Sequences Differentially Expressed in PACE Overexpressing Cells and Encoding Golgi-Associated Proteins**

| **Qualifier List** | **Symbol** | **Title** | **Human Unigene ID** | **eValue** | **%ID** | **%QC** | **Mouse Unigene ID** | **eValue** | **%ID** | **%QC** | **Fold change** | **Direction of change** |
|---|---|---|---|---|---|---|---|---|---|---|---|---|
| WAN0088ZC_at (SEQ ID NO:1543) | PSEN1 | Presenilin 1 (Alzheimer disease 3) | Hs.592324 | 5E-82 | 89.161 | 86.145 | Mm.998 | 5E-78 | 88.153 | 86.446 | 1.255 | up |
| WAN014IYT_at (SEQ ID NO:1544) | FURIN | Furin (paired basic amino acid cleaving enzyme) | Hs.513153 | 0 | 99.922 | 93.193 | Mm.5241 | 0 | 88.701 | 89.718 | 2.500 | up |
| WAN008CMC_x_at (SEQ ID NO:1545) | MAPRE1 | Microtubule-associated protein, RP/EB family, member 1 | Hs.472437 | 2E-49 | 94.964 | 88.535 | Mm.143877 | 9E-50 | 94.964 | 88.535 | 1.242 | up |
| WAN008D2C_at (SEQ ID NO:1546) | Csnk2a2 | Casein kinase II, alpha 2, polypeptide (Csnk2a2) | Hs.82201 | 1E-141 | 89.293 | 91.497 | Mm.51136 | 0 | 95.158 | 99.261 | 1.272 | up |
| WAN008E72_x_at (SEQ ID NO:1547) | GDI2 | GDP dissociation inhibitor 2 | Hs.299055 | 6E-25 | 86.139 | 100.000 | Mm.153226 | 1 E-74 | 95.545 | 100.000 | -1.455 | down |
| WAN01318H_x_at (SEQ ID NO:1548) | APP | Amyloid beta (A4) precursor protein (protease nexin-II, Alzheimer disease) | Hs.434980 | 1 E-1 02 | 84.192 | 99.646 | Mm.277585 | 1E-162 | 87.788 | 100.000 | -1.902 | down |
| AF030413_at (SEQ ID NO:1549) | APP | Amyloid beta (A4) precursor protein (protease nexin-II, Alzheimer disease) | Hs.434980 | 6E-90 | 93.421 | 100.000 | Mm.277585 | 2E-97 | 94.737 | 100.000 | -1.851 | down |
| WAN013I12_at (SEQ ID NO:1550) | VDP | Vesicle docking protein p115 | Hs.292689 | 4E-12 | 85.227 | 15.385 | Mm.15868 | 9E-42 | 85.259 | 43.881 | -1.588 | down |
| WAN013HUW_at (SEQ ID NO:1551) | ARL1 | ADP-ribosylation factor-like 1 | Hs.372616 | 2E-88 | 91.339 | 51.313 | Mm.291247 | 1E-150 | 90.798 | 98.788 | -1.687 | down |
| WAN008CLK_at (SEQ ID NO:1552) | Rab6 | RAB6, member RAS oncogene family | Hs.503222 | | 2E-55 88.477 | 48.214 | Mm.28650 | 1E-163 | 92.276 | 97.619 | -1.292 | down |
| WAN0088X9_at (SEQ ID NO:1553) | RAB34 | RAB34, member RAS oncogene family | Hs.301853 | 1 E-1 08 | 89.174 | 66.730 | Mm.275864 | 1E-161 | 92.157 | 87.262 | -1.374 | down |
| WAN013HZH_at (SEQ ID NO:1554) | M6PRBP1 | Mannose-6-phosphate receptor binding protein 1 | Hs.140452 | 3E-18 | 76.364 | 47.826 | Mm.311696 | 1E-107 | 81.239 | 98.261 | -1.342 | down |

### Example 7. Genes differentially expressed in cells with high cellular growth rate

A summary of nucleic acids identified as differentially expressed in cells with high cellular growth rate is provided in Tables 13 and 14. For each nucleic acid, a qualifier name, symbol, and title are provided, as well as whether the nucleic acid is up-regulated or down-regulated in the cells with higher maximum cellular productivity. For nucleic acids with human or mouse homologs in the Unigene database, the table provides Unigene ID numbers and statistics relating to the comparison, including e-values, percent sequence identities between the CHO sequence and the Unigene databank entries, and percent coverage ("% QC").

**Table 13: High Cell Growth Rate NA Unknown CHO Sequences**

| **Qualifier List** | **Symbol** | **Title** | **Human Unigene ID** | **eValue** | **%ID** | **%QC** | **Mouse Unigene ID** | **eValue** | **%ID** | **%QC** | |
|---|---|---|---|---|---|---|---|---|---|---|---|
| /AN0088JV_at SEQ ID NO:1555) | TRIB3 | Tribbles homolog 3 (Drosophila) | Hs.516826 | 4E-62 | 81.778 | 86.37 | Mm.276018 | 1E-158 | 88.845 | 98.08 | down |
| AN0088PT_at SEQ ID NO:1556) | Psmc1 | Protease (prosome, macropain) 26S subunit, ATPase 1 | Hs.356654 | 0 | 92.292 | 99.59 | Mm.157105 | 0 | 94.561 | 99.17 | down |
| AN0088XH_at SEQ ID NO:1557) | HERPUD1 | Homocysteine-inducible, endoplasmic reticulum stress-inducible, ubiquitin-like domain member 1 | Hs.146393 | 7E-79 | 87.417 | 68.48 | Mm.29151 | 1E-144 | 91.463 | 92.97 | down |
| AN008BSH_at SEQ ID NO:1558) | CAT | Catalase | Hs.502302 | 6E-16 | 89.286 | 38.71 | Mm.4215 | 3E-41 | 89.247 | 85.71 | down |
| AN008CM1_x_at SEQ ID NO:1559) | DQ390542.2 | Mitochondrial 12S ribosomal RNA | #N/A | | | | #N/A | 0 | 0 | 0 | up |
| AN008CWC_x_at SEQ ID NO:1560) | NA | WAN008CWC 10603C-F10 | #N/A | | | | #N/A | | | | up |
| AN008D2Q_at SEQ ID NO:1561) | Eif4b | Eukaryotic translation initiation factor 4B (Eif4b) | #N/A | 6E-49 | 92.949 | 29.38 | Mm.290022 | 1E-129 | 91.316 | 71.56 | up |
| AN008D5V_x_at SEQ ID NO:1562) | Gosr2 | Golgi SNAP receptor complex member 2, mRNA (cDNA clone MGC:6437 IMAGE:3601627) | Hs.463278 | | | | Mm.195451 | 1E-08 | 90.196 | 43.59 | down |
| AN008D6J_at SEQ ID NO:1563) | HMGA2 | High mobility group AT-hook 2 | Hs.505924 | 4E-62 | 94.805 | 33.33 | Mm.157190 | 1E-130 | 90.444 | 97.4 | down |
| AN008DGD_at SEQ ID NO:1564) | Apip2 | Amyloid beta (A4) precursor-like protein 2 (Aplp2) | #N/A | | | | Mm.19133 | 7E-69 | 93.088 | 44.47 | down |
| AN008DJ9_at SEQ ID NO:1565) | SLC1A4 | Solute carrier family 1 (glutamate/neutral amino acid transporter), member 4 | Hs.323878 | 2E-39 | 86.932 | 39.46 | Mm.6379 | 1E-121 | 88.614 | 90.58 | down |
| AN008DSE_at SEQ ID NO:1566) | SLC1A4 | Solute carrier family 1 (glutamate/neutral amino acid transporter), member 4 | Hs.323878 | 2E-82 | 86.89 | 60.18 | Mm.6379 | 1E-117 | 90.643 | 62.75 | down |
| AN008E2E_at SEQ ID NO:1567) | PSMC4 | Proteasome (prosome, macropain) 26S subunit, ATPase, 4 | Hs.211594 | 1E-131 | 89.95 | 100 | Mm.29582 | 1E-141 | 90.955 | 100 | down |
| AN008E8M_at SEQ ID NO:1568) | HADHB | Hydroxyacyl-Coenzyme A dehydrogenase/3-ketoacyl-Coenzyme A thiolase/enoyl-Coenzyme A hydratase (trifunctional protein), beta subunit | Hs.515848 | 1E-114 | 88.191 | 85.78 | Mm.291463 | 1E-162 | 91.256 | 96.12 | down |
| AN008EBJ_at SEQ ID NO:1569) | Triobp | TRIO and F-actin binding protein | Hs.533030 | 6E-89 | 87.613 | 61.87 | Mm.123714 | 1E-175 | 90.707 | 92.52 | down |
| AN008EFS_at SEQ ID NO:1570) | TXNRD1 | Thioredoxin reductase 1 | Hs.434367 | 6E-18 | 86.777 | 22.08 | Mm.210155 | 2E-54 | 88.393 | 40.88 | down |
| AN008EGV_at SEQ ID NO:1571) | GDI2 | GDP dissociation inhibitor 2 | Hs.299055 | 0 | 92.897 | 94.86 | Mm.8070 | 0 | 94.019 | 94.86 | down |
| AN008EMQ_at SEQ ID NO:1572) | KPNA3 | Karyopherin alpha 3 (importin alpha 4) | Hs.527919 | 1E-144 | 91.853 | 100 | Mm.25548 | 0 | 96.334 | 100 | down |
| AN008ERL_at SEQ ID NO:1573) | ETFA | TYRO3P protein tyrosine kinase pseudogene | Hs.39925 | 1E-135 | 95.57 | 87.05 | | 1E-162 | 95.868 | 100 | down |
| AN008ETP_at SEQ ID NO:1574) | AADACL1 | Arylacetamide deacetylase-like 1 | Hs.444099 | 2E-72 | 86.942 | 97.65 | Mm.24576 | 8E-89 | 88.926 | 100 | down |
| AN008EX2_x_at SEQ ID NO:1575) | IFRD1 | Interferon-related developmental regulator 1 | Hs.7879 | 7E-39 | 90.299 | 100 | Mm.168 | 6E-63 | 97.761 | 100 | down |
| AN013HUM_at SEQ ID NO:1576) | EHD4 | EH-domain containing 4 | Hs.143703 | 1E-95 | 92.771 | 59 | Mm.132226 | 1E-132 | 89.286 | 99.53 | down |
| AN013HWG_at SEQ ID NO:1577) | DQ390542.2 | Mitochondrial NADH dehydrogenase subunit 5 | Hs.550202 | 5.00E-08 | 85 | 6.525 | #N/A | | | | up |
| AN013HX4_at SEQ ID NO:1578) | ESD | Esterase D/formylglutathione hydrolase | Hs.432491 | 1E-155 | 87.617 | 93.51 | Mm.38055 | 0 | 91.902 | 93.16 | down |
| AN013HYO_at SEQ ID NO:1579) | RPL11 | Ribosomal protein L11 | Hs.388664 | 0 | 90.522 | 100 | Mm.276856 | 0 | 91.473 | 99.81 | up |
| AN013I0W_at SEQ ID NO:1580) | TAPBP | TAP binding protein (tapasin) | Hs.370937 | 2E-57 | 80.633 | 93.23 | Mm.154457 | 1E-149 | 86.885 | 95.31 | down |
| AN013I0X_at SEQ ID NO:1581) | GSS | Glutathione synthetase | Hs.82327 | 3E-96 | 90.444 | 56.13 | Mm.252316 | 1E-129 | 95.189 | 55.75 | down |
| AN013I1G_at SEQ ID NO:1582) | SLC25A20 | Solute carrier family 25 (carnitine/acylcarnitine translocase), member 20 | Hs.13845 | 1E-137 | 86.706 | 87.65 | Mm.29666 | 0 | 92.354 | 86.43 | down |
| AN013I38_at SEQ ID NO:1583) | Pkm2 | Pyruvate kinase, muscle, mRNA (cDNA clone MGC: 11908 IMAGE:3598842) | Hs.198281 | 3E-63 | 90.521 | 38.36 | Mm.216135 | 0 | 92.99 | 88.18 | down |
| AN013I8K_at SEQ ID NO:1584) | DQ390542.2 | Mitochondrial cytochrome b | #N/A | | | | #N/A | | | | up |

**Table 14: High Cell Growth Rate NA Known CHO Sequences**

| **Qualifier List** | **Symbol** | **Title** | **Human Unigene ID** | **eValue** | **%ID** | **%QC** | **Mouse Unigene ID** | **eValue** | **%ID** | **%QC** | |
|---|---|---|---|---|---|---|---|---|---|---|---|
| AF081143_at (SEQ ID NO:1585) | RPS18 | Ribosomal protein S18 | Hs.546290 | 1 E-78 | 90.717 | 98.34 | Mm.324762 | 4E-93 | 92.946 | 100 | up |
| U62588_x_at (SEQ ID NO:1586) | SDC1 | Syndecan 1 | Hs.224607 | 1E-32 | 93 | 53.19 | Mm.2580 | 7E-48 | 90.85 | 81.38 | down |
| X51747_at (SEQ ID NO:1587) | HSPB1 | Heat shock 27kDa protein 1 | Hs.520973 | 1E-101 | 87.368 | 50.53 | Mm.13849 | 0 | 91.952 | 66.09 | up |

### Example 8. Genes differentially expressed in cells with high peak cell density

A summary of nucleic acids identified as differentially expressed in cells with high peak cell density is provided in Tables 15, 16, and 17. For each nucleic acid, a qualifier name, symbol, and title are provided, as well as whether the nucleic acid is up-regulated or down-regulated in the cells with higher maximum cellular productivity. For nucleic acids with human or mouse homologs in the Unigene database, the table provides Unigene ID numbers and statistics relating to the comparison, including e-values, percent sequence identities between the CHO sequence and the Unigene databank entries, and percent coverage ("% QC").

| | | | | | | | | | | | |
|---|---|---|---|---|---|---|---|---|---|---|---|
| ***Table 15: High Cell Density Unknown CHO Sequences*** | | | | | | | | | | | |

| **Qualifier List** | **Symbol** | **Title** | **Human Unigene ID** | **eValue** | **%ID** | **%QC** | **Mouse Unigene ID** | **eValue** | **%ID** | **%QC** | **Direction of change (test vs control)** |
|---|---|---|---|---|---|---|---|---|---|---|---|
| WAN008819_x_at (SEQ ID | | | | | | | | | | | |
| NO:1588) WAN0088PR_at | CCNA2 | cyclin A2 | Hs.85137 | 100E-55 | 87.6494 | 20.291 | Mm.4189 | 4.00E-60 | 89.423 | 16.815 | up |
| (SEQ ID NO:1589) | CCPG1 | Phosphatidylinositol glycan, class B | Hs.285051 | 2E-08 | 87.5 | 11.429 | Mm.268475 | 3E-34 | 90.244 | 21.964 | down |
| WAN0088Q6_at (SEQ ID | | | | | | | | | | | |
| NO:1590) WAN0088S8_at | Hist1h2bn | Histone 1, H2bn Solute carrier family 29 | Hs.534368 | 1E-153 | 93.7158 | 63.432 | Mm.261676 | 0 | 95.134 | 71.231 | down |
| (SEQ ID NO:1591) | SLC29A1 | (nucleoside transporters), member 1 | Hs.25450 | 3E-35 | 81.3559 | 76.129 | Mm.29744 | 6E-97 | 86.098 | 88.172 | up |
| WAN0088T2_at (SEQ ID NO:1592) | ATF4 | Activating transcription factor 4 (tax-responsive enhancer element B67) | Hs.496487 | 1E-158 | 88.5397 | 97.83 | Mm.641 | 0 | 91.714 | 96.022 | up |
| WAN0088X2_at (SEQ ID NO:1593) | PEO1 | Progressive external ophthalmoplegia 1 | Hs.22678 | 1E-141 | 88.651 | 94.534 | Mm.105585 | 7E-78 | 90.678 | 47.773 | up |
| WAN008BRK_at (SEQ ID NO:1594) | Tmsb4x | Thymosin, beta 4, X chromosome | Hs.522584 | 1E-153 | 93.617 | 71.756 | Mm. 142729 | 0 | 95.34 | 98.282 | down |
| WAN008BSG_x_at (SEQ ID NO:1595) | TRAM1 | Translocation associated membrane protein I | Hs.491988 | 7E-29 | 89.9225 | 36.236 | Mm.28765 | 5E-44 | 91.787 | 58.146 | up |
| WAN008CHP_x_at (SEQ ID NO:1596) | NA | WAN008CHP 105990-H02 | #N/A | | | | #N/A | | | | up |
| WAN008CM7_x_at (SEQ ID NO:1597) | MRPL51 | Mitochondrial ribosomal protein L51 | Hs.55847 | 0.0002 | 82.0225 | 25.356 | Mm.354426 | 6E-13 | 84.466 | 29.345 | up |
| WAN008CQP_at (SEQ ID NO:1598) | AATF | Apoptosis antagonizing transcription factor | Hs.195740 | 6E-73 | 83.7587 | 99.309 | Mm.257482 | 8E-99 | 85.615 | 99.309 | up |
| WAN008CX9_at (SEQ ID NO:1599) | ISGF3G | Interferon-stimulated transcription factor 3, gamma 48kDa | Hs.1706 | 2E-64 | 83.4225 | 81.481 | Mm.2032 | 1E-119 | 88.424 | 88.453 | up |
| WAN008CXC_at (SEQ ID NO:1600) | ATP6V0A1 | ATPase, H+ transporting, lysosomal V0 subunit a isoform I | Hs.463074 | 0 | 93.2927 | 99.394 | Mm.340818 | 0 | 94.343 | 100 | down |
| WAN008D2S_at (SEQ ID NO:1601) | BPY2IP1 | BPY2 interacting protein I | Hs.66048 | 6E-15 | 84.0336 | 20.951 | Mm.248559 | 1E-101 | 86.99 | 69.014 | down |
| WAN008D3Z_at (SEQ ID NO:1602) | GALNT7 | UDP-N-acetyl-alpha-D-galactosamine:polypeptide N-acetylgalactosaminyltransferase 7 (GalNAc-T7) | Hs.127407 | 1E-135 | 88.8668 | 100 | Mm.62886 | 1E-150 | 90.855 | 100 | down |
| WAN008D55-rc_at (SEQ ID NO:1603) | LAMB1 | Laminin, beta 1 | Hs.489646 | 1E-155 | 87.8229 | 97.482 | Mm.172674 | 1E-161 | 91.667 | 77.698 | 1up, 1down |
| WAN008D5V_x_at (SEQ ID NO:1562) | Gosr2 | Golgi SNAP receptor complex member 2, mRNA (cDNA clone MGC:6437 IMAGE:3601627) | Hs.463278 | | | | Mm.195451 | 1E-08 | 90.196 | 43.59 | down |
| WAN008D6R_at (SEQ ID NO:1604) | TMED4 | Transmembrane emp24 protein transport domain containing 4 | Hs.510745 | IE-111 | 91.0828 | 73.709 | Mm.254495 | 1E-140 | 92.412 | 86.62 | down |
| WAN008DFT_at (SEQ ID NO:1605) | ABHD6 | Abhydrolase domain containing 6 | Hs.476454 | 3E-17 | 83.2168 | 26.335 | Mm.181473 | 9E-53 | 87.124 | 42.91 | up |
| WAN008DGZ_at (SEQ ID NO:1606) | SLC7A6OS | Solute carrier family 7, member 6 opposite strand | Hs.334848 | 2E-79 | 84.2342 | 79.428 | Mm.269029 | 1E-139 | 89.862 | 77.639 | up |
| WAN008D17_at (SEQ ID NO:1607) | FBXO42 | F-box protein 42 | #N/A | | | | Mm.28865 | 2E-23 | 86.957 | 68.452 | down |
| WAN008DIA_at (SEQ ID NO:1608) | U2AF1 | U2(RNU2) small nuclear RNA auxiliary factor 1 | Hs.365116 | 1E-170 | 90.5544 | 97.4 | Mm.311063 | 0 | 95.4 | 100 | up |
| WAN008DJ8_f_at (SEQ ID NO:1609) | Ubc | Ubiquitin C, mRNA (cDNA clone IMAGE:2645223) | Hs.378821 | 1E-22 | 87.8049 | 24.848 | Mm.331 | 2E-25 | 88.618 | 24.848 | down |
| WAN008DMI_at (SEQ ID NO:1610) | ACSL5 | Acyl-CoA synthetase long-chain family member 5 | Hs.11638 | 1E-118 | 85 | 96.601 | #N/A | 0 | 89.946 | 99.642 | up |
| WAN008DMJ_at (SEQ ID NO:1611) | NAB2 | NGFI-A binding protein 2 (EGR1 binding protein 2) | Hs.159223 | 1E-176 | 89.5717 | 100 | Mm.336898 | 0 | 92.683 | 99.255 | up |
| WAN008DQE_at (SEQ ID NO:1612) | YES1 | V-yes-1 Yamaguchi sarcoma viral oncogene homolog I | Hs.194148 | 0 | 94.8529 | 100 | #N/A | 0 | 95.588 | 100 | up |
| WAN008DS9_at (SEQ ID NO:1613) | CFL2 | Cofilin 2 (muscle) | Hs.180141 | 1E-113 | 89.0187 | 90.87 | Mm.276826 | 1E-132 | 92.982 | 84.713 | down |
| WAN008DWJ_at (SEQ ID NO:1614) | USP1 | Similar to ubiquitin specific protease 1 | Hs.35086 | 0 | 92.9476 | 97.018 | Mm.371692 | 0 | 94.182 | 96.491 | up |
| WAN008DZF_at (SEQ ID NO:1615) | AL033326 | Expressed sequence AL033326 | #N/A | 1E-92 | 87.0558 | 99.747 | Mm.182145 | 1E-156 | 92.658 | 100 | down |
| WAN008E06_at (SEQ ID NO:1616) | Rabep2 | Rabaptin, RAB GTPase binding effector protein 2 | Hs.555978 | 2E-92 | 85.4722 | 76.34 | Mm.35467 | 0 | 91.37 | 98.521 | down |
| WAN008E1M_f_at (SEQ ID NO:1617) | CD36 | CD36 antigen | Hs.120949 | 0.00001 | 90.9091 | 3.0513 | Mm.18628 | 7.00E-19 | 85.714 | 9.2233 | down |
| WAN008E2Q_at (SEQ ID NO:1618) | GSPT1 | G1 to S phase transition 1 | Hs.528780 | 0 | 93.6957 | 100 | Mm.325827 | 0 | 95.87 | 100 | up |
| WAN008E5L_at (SEQ ID NO:1619) | SLC1A5 | Solute carrier family 1 (neutral amino acid transporter), member 5 | Hs.515494 | 8E-42 | 84.1667 | 45.627 | Mm.1056 | 1E-115 | 87.671 | 83.27 | up |
| WAN008E9N_at (SEQ ID NO:1620) | KLHL7 | Kelch-like 7 (Drosophila) | Hs.385861 | 1E-150 | 89.4422 | 99.406 | Mm.273768 | 0 | 93.111 | 89.109 | down |
| WAN008EBP_at (SEQ ID NO:1621) | Sqstm1 | Sequestosome I | Hs.529892 | 0 | 93.4066 | 97.849 | Mm.40828 | 0 | 93.407 | 97.849 | down |
| ***WAN008EH5_at*** (SEQ ID NO:1622) | ***PRNP*** | ***Prion protein (p27-30) (Creutzfeld-Jakob disease, Gerstmann-Strausler-Scheinker syndrome, fatal familial insomnia**)* | ***Hs.472010*** | 9E-45 | 86.802 | 34.806 | ***Mm.648*** | ***4E-92*** | ***89.815*** | ***57.24*** | ***down*** |
| WAN008EID_at (SEQ ID NO:1623) | TRIB3 | Tribbles homolog 3 (Drosophila) | Hs.516826 | 1E-12 | 92.7273 | 12.195 | Mm.276018 | 1E-51 | 89.773 | 39.024 | up |
| WAN008EJY_at (SEQ ID NO:1624) | NA | WAN008EJY 11232A-H04 | #N/A | | | | #N/A | | | | up |
| WAN008EKK_at (SEQ ID NO:1625) | PSMA8 | Proteasome (prosome, macropain) subunit, alpha type, 8 | Hs.464813 | 1E-104 | 91.6667 | 98.63 | Mm.87277 | 2E-89 | 90.182 | 94.178 | down |

| **Qualifier List** | **Symbol** | **Title** | **Human Unigene ID** | **evalue** | **%ID** | **%QC** | **Mouse Unigene ID** | **eValue** | **%ID** | **%QC** | **Direction of change (test vs control)** |
|---|---|---|---|---|---|---|---|---|---|---|---|
| WAN008ELE_at (SEQ ID NO:1626) | PSAT1 | Phosphoserine aminotransferase I | Hs.494261 | 7E-27 | 93.1034 | 16.171 | Mm.289936 | 5E-70 | 87.54 | 58.178 | up |
| WAN008EM4_at (SEQ ID NO:1627) | ARHGAP18 | Rho GTPase activating protein 18 | Hs.486458 | 1E-109 | 85.3516 | 97.897 | Mm.356496 | 1E-147 | 87.763 | 100 | down |
| WAN008END_at (SEQ ID NO:1628) | SCYL1 | SCY1-like 1 (S. cerevisiae) | Hs.238839 | 2E-61 | 83.3333 | 74.844 | Mm.276063 | 0 | 92.292 | 99.792 | down |
| WAN008EOB_at (SEQ ID NO:1629) | NOL1 | Nucleolar protein 1,120kDa | Hs.534334 | 8E-48 | 89.8058 | 42.474 | Mm.29203 | 1E-120 | 87.248 | 92.165 | up |
| WAN008EQM_at (SEQ ID NO:1630) | NA | RC WAN008EQM 11232D-D11 | #N/A | | | | #N/A | | | | down |
| WAN008ERB_at (SEQ ID NO:1631) | PCBP1 | Poly(rC) binding protein I | Hs.2853 | 0 | 96.7611 | 99.396 | Mm.274146 | 0 | 97.586 | 100 | up |
| WAN008ERI_at (SEQ ID NO:1632) | FNBP3 | Formin binding protein 3 | Hs.298735 | 4E-81 | 96.6102 | 98.883 | Mm.257474 | 2E-94 | 99.441 | 100 | down |
| WAN008ERO_at (SEQ ID NO:1633) | SNAG 1 | Sorting nexin associated golgi protein 1 | Hs.432755 | 7E-30 | 89.1156 | 28.215 | Mm.33721 | 3E-40 | 90.511 | 26.296 | up |
| WAN008ERP_at (SEQ ID NO:1634) | LEPREL1 | Leprecan-like 1 | Hs.374191 | 1E-45 | 87.1245 | 92.829 | Mm.326869 | 1E-68 | 88.983 | 94.024 | down |
| WAN008EUO_at (SEQ ID NO:1635) | LPL | Lipoprotein lipase | Hs.180878 | 2E-82 | 87.5 | 74.109 | Mm.1514 | 1E-113 | 91.667 | 74.109 | down |
| WAN008EY0_at (SEQ ID NO:1636) | C330017I15Rik | RIKEN cDNA C330017115 gene | Hs.520619 | 1E-179 | 90.2111 | 99.049 | Mm.58660 | 1E-163 | 88.783 | 100 | up |
| WAN008F1P_x_at (SEQ ID NO:1637) | NA | WAN008F1P 11165A-A01 | #N/A | | | | #N/A | | | | down |
| WAN013HVJ_at (SEQ ID NO:1638) | Rn.75246 | Similar to RIKEN cDNA 2310045A20 | #N/A | 7E-78 | 83.6683 | 71.583 | #N/A | 1E-118 | 87.112 | 75.36 | down |
| WAN013HVL_at (SEQ ID | UGDH | UDP-glucosedehydrogenase | Hs.28309 | 1E-160 | 89.3805 | 97.835 | Mm.344831 | 1E-160 | 89.231 | 98.485 | up |

| **Qualifier List** | **Symbol** | **Title** | **Human Unigene ID** | **eValue** | **%ID** | **%QC** | **Mouse Unigene ID** | **evalue** | **%ID** | **%QC** | **Direction of change (test vs control)** |
|---|---|---|---|---|---|---|---|---|---|---|---|
| NO:1639) WAN013HW0_x_at | | | | | | | | | | | |
| (SEQ ID NO:1640) | DQ390542.2 | Mitochondrial NADH dehydrogenase subunit I | Hs.326475 | 7E-23 | 82.5688 | 16.257 | #N/A | | | | up |
| WAN013HWB_at (SEQ ID N0:1641) | APLP2 | Amyloid beta (A4) precursor-like protein 2 | Hs.370247 | 3E-08 | 85.5263 | 9.7812 | Mm.19133 | 1E-161 | 95 | 46.332 | down |
| WAN013HX8_f_at (SEQ ID NO:1490) | EIF4A2 | Eukaryotic translation initiation factor 4A, isoform 2 | Hs.478553 | 1E-155 | 95.8199 | 100 | Mm.260084 | 1E-155 | 95.82 | 100 | down |
| WAN013HZ3_at (SEQ ID NO:1642) | ARMCX3 | Armadillo repeat containing, X-linked 3 | Hs.172788 | 4E-09 | 91.8367 | 9.9796 | Mm.67949 | 4E-29 | 82.684 | 47.047 | down |
| WAN013HZK_at (SEQ ID NO:1643) | NA | Cluster includes WAN008DS2 11228C-H04 | #N/A | | | | #N/A | 7E-10 | 88.372 | 17.587 | up |
| WAN013HZP_at (SEQ ID NO:1644) | Eif4g2 | Eukaryotic translation initiation factor 4, gamma 2 | Hs.183684 | 1E-179 | 97.7465 | 72.449 | Mm.185453 | 0 | 99-718 | 72.449 | up |
| WAN013I01_at (SEQ ID NO:1645) | MCFD2 | Multiple coagulation factor deficiency 2 | Hs.293689 | 4E-59 | 89.5604 | 52 | Mm.30251 | 2E-35 | 84.699 | 52.286 | down |
| WAN013I05_at (SEQ ID NO:1646) | Abcb6 | ATP-binding cassette, sub-family B (MDR/TAP), member 6 | Hs.107911 | 1E-154 | 87.1508 | 100 | Mm.28663 | 0 | 91.806 | 100 | up |
| WAN013115_at (SEQ ID NO:1647) | SUCLG2 | Succinate-CoA ligase, GDP-forming, beta subunit | Hs.186512 | 1E-157 | 85.8195 | 100 | Mm.292637 | 0 | 88.398 | 100 | up |
| WAN013I1U_x_at (SEQ ID NO:1648) | DQ390542.2 | Mitochondrial NADH dehydrogenase subunit 4 | Hs.571926 | 0.00002 | 92.1053 | 7.7079 | #N/A | 0.00001 | | 92.105 7.7079 | up |
| WAN013I2F_at (SEQ ID NO:1649) | THBD | Thrombomodulin | Hs.2030 | 1E-18 | 88.764 | 18.053 | Mm.24096 | 9E-93 | 85.714 | 92.292 | up |
| WAN013I2K_at (SEQ ID NO:1650) | TMEFF1 | Transmembrane protein with EGF-like and two follistatin-like domains 1 | Hs.336224 | 8E-93 | 91.0781 | 100 | Mm.130982 | 7E-86 | 89.963 | 100 | down |
| WAN013I2L_at (SEQ ID NO:1651) | SLC7A5 | solute carrier family 7 (cationic amino acid transporter, y+ system), member 5 | Hs.513797 | 9.00E-07 | 100 | 2.2523 | Mm.27943 | 1.00E-07 | 92 | 3.7538 | up |

| **Qualifier List** | **Symbol** | **Title** | **Human Unigene ID** | **evalue** | **%ID** | **%QC** | **Mouse Unigene ID** | **evalue** | **%ID** | **%QC** | **Direction of change (test vs control)** |
|---|---|---|---|---|---|---|---|---|---|---|---|
| WAN01312T_at (SEQ ID NO:1652) | CBX5 | Chromobox homolog 5 (HP1 alpha homolog, Drosophila) | Hs.349283 | 1E-142 | 91.8635 | 72.023 | Mm.262059 | 1E-168 | 94.751 | 72.023 | up |
| WAN013I3P_at (SEQ ID NO:1653) | CAMLG | Calcium modulating ligand | Hs.529846 | 1E-147 | 86.7021 | 99.296 | #N/A | 1E-172 | 88.612 | 98.944 | up |
| WAN013I61_at (SEQ ID NO:1654) | Nppb | Natriuretic peptide precursor type B | Hs.219140 | | | | Mm.2740 | 5E-30 | 88.281 | 23.146 | down |
| WAN013I6C_at (SEQ ID NO:1655) | SLCI6A1 | Solute carrier family 16 (monocarboxylic acid transporters), member 1 | Hs.75231 | 2E-26 | 84.472 | 12.697 | Mm.9086 | 1E-110 | 87.24 | 30.284 | up |
| WAN013I6E_x_at (SEQ ID NO:1656) | GSTP1 | Glutathione S-transferase pi | Hs.523836 | 1E-129 | 81.9905 | 85.889 | #N/A | 0 | 87.577 | 88.467 | down |
| WAN013I6J_s_at (SEQ ID NO:1657) | CAD | Carbamoyl-phosphate synthetase 2, aspartate transcarbamylase, and dihydroorotase | Hs.377010 | 0 | 91.1552 | 99.461 | Mm.305535 | 0 | 93.502 | 99.461 | up |
| WAN013I6P_x_at (SEQ ID NO:1658) | ABCB1 | ATP-binding cassette sub-family B (MDR/TAP), member 1 | Hs.489033 | 0 | 87.7104 | 28.592 | Mm.146649 | 0 | 89.771 | 33.646 | down |
| WAN013I8B_at (SEQ ID NO:1659) | Akr1a4 | Aldo-keto reductase family 1, member A4 (aldehyde reductase) | Hs.474584 | 0 | 91.5371 | 99.314 | Mm.30085 | 0 | 91.71 | 99.314 | down |
| WAN013I8V_at (SEQ ID NO:1660) | NCL | Nucleolin | Hs.79110 | IE-111 | 90.0585 | 67.059 | Mm.154378 | 1E-137 | 93.275 | 67.059 | up |
| WAN013I8X_at (SEQ ID NO:1661) | HSPD1 | Heat shock 60kDa protein 1 (chaperonin) | Hs.113684 | 0 | 90.308 | 99.775 | Mm.1777 | 0 | 93.388 | 99.775 | Up |
| WAN013I9F_at (SEQ ID NO:1662) | HSPA9B | Heat shock protein 9A | Hs.184233 | 3E-29 | 92.233 | 18.693 | Mm.209419 | 2E-72 | 90.688 | 44.828 | Up |
| WAN013I9G_at (SEQ ID NO:1663) | SLC3A2 | Solute carrier family 3 (activators of dibasic and neutral amino acid transport), member 2 | Hs.502769 | 1E-105 | 84.8544 | 38.064 | Mm.4114 | | | | |
| WAN013I9Z_at (SEQ ID NO:1664) | GNAS | guanine nucleotide binding protein, alpha stimulating | Hs.125898 | | | | Mm.125770 | 0 | 94.403 | 41.104 | Down |

**Table 16: High Cell Density Known CHO Sequences**

| **Qualifier List** | **Symbol** | **Title** | **Human Unigene ID** | **evalue** | **%ID** | **%QC** | **Mouse Unigene ID** | **evalue** | **%ID** | **%QC** | **Direction of change (test vs control)** |
|---|---|---|---|---|---|---|---|---|---|---|---|
| AB020230_at (SEQ ID NO:1665) | INTRON | delta-sarcoglycan intron 1 | #N/A | 3E-08 | 91.6667 | 7.5 | #N/A | 2E-17 | 92.537 | 10.469 | Down |
| AF022945-*c_f_at (SEQ ID NO:1666) | Thbd | Thrombomodulin | Hs.2030 | | | | Mm.24096 | 1E-13 | 89.552 | 65.049 | Up |
| AF081141_at (SEQ ID NO:1667) | CCL2 | Chemokine (C-C motif) ligand 2 | Hs.303649 | 1E-12 | 97.6744 | 9.0147 | Mm.290320 | 6E-41 | 91.045 | 28.092 | Down |
| AF113614_at (SEQ ID NO:1668) | TLR2 | Toll-like receptor 2 | Hs.519033 | 1E-101 | 86.2651 | 80.897 | Mm.87596 | 1E-167 | 92.326 | 83.821 | Down |
| AF320819_at (SEQ ID NO:1669) | BSG | Basigin (OK blood group) | Hs.501293 | 1E-25 | 90.099 | 17.119 | Mm.726 | 4E-92 | 89.145 | 51.525 | Down |
| M27838_s_at (SEQ ID NO:1670) | ASNS | Asparagine synthetase | Hs.489207 | 0 | 89.072 | 100 | Mm.2942 | 0 | 91.847 | 100 | Up |
| M76730_at (SEQ ID NO:1671) | Col5a1 | Procollagen, type V, alpha 1 | Hs.210283 | 6E-52 | 91.358 | 30.975 | Mm.7281 | 1E-110 | 96.443 | 48.375 | Down |
| U29167 at (SEQ ID NO:1672) | TPM2 | Tropomyosm 2 (beta) | Hs.300772 | 0 | 92.8311 | 88.781 | Mm.646 | 0 | 94 743 | 90.291 | Up |
| U42430_at (SEQ ID NO:1673) | CD36 | CD36 antigen (collagen type I receptor, thrombospondin receptor) | Hs.120949 | 6E-43 | 86.3636 | 34.256 | Mm.18628 | 2E-57 | 88.073 | 37.716 | Down |
| U62588_x_at (SEQ ID NO:1586) | SDC1 | Syndecan 1 | Hs.224607 | 1E-32 | 93 | 53.191 | Mm.2580 | 7E-48 | 90.85 | 81.383 | Down |
| Y00365_at (SEQ ID NO:1674) | HMGB1 | High-mobility group box 1 | Hs.434102 | 1E-102 | 93.9799 | 23.396 | Mm.313345 | 1 E-1 45 | 88.841 | 53.991 | Up |

**Table 17: Control vs. Test HCD4**

| 1.5f Up | | | | | | | | | | | | |
|---|---|---|---|---|---|---|---|---|---|---|---|---|
| WAN0088WZ-rc_at (SEQ ID NO:1675) | 1.736111 | APC | Adenomatosis polyposis coli | WAN0088WZ-rc_at Blast Report | Hs. 158932 | 2E-50 | 84.75177 | 99.29577 | Mm.7883 | 2E-80 | 89.70588 | 95.77465 |
| AF052840_x_at (SEQ ID NO:1676) | 273224 | NA | AF052840 Mesocricetus auratus clone ut3 retroviral-like pol protein (pol) mRNA, partial cds. | AF052840_x_at Blast Report | #N/A | 0 | 0 | 0 | #N/A | 0 | 0 | 0 |
| AF306800_at (SEQ ID NO: 1677) | 1.610306 | NA | AF306800 Cricetutus griseus clone cos7-1 intrachromosomal telomeric-like sequence. | AF306800_at Blast Report | #N/A | 0.00006 | 9428571 | 2.571639 | #N/A | 0.000008 | 94.44444 | 2.645114 |
| WAN008ED8_at (SEQ ID NO:1678) | 1.519757 | AVPI1 | Arginine vasopressin-induced 1 | WAN008ED8_at Blast Report | Hs.23918 | 3E-44 | 84.27419 | 49.8994 | Mm.30060 | 1E-133 | 88.2716 | 97-78872 |
| AY011521_x_at (SEQ ID NO:1679) | 1.564945 | BMI1 | B lymphoma Mo-MLV insertion region (mouse) | AY011521_x_at Blast Report | Hs.496613 | 1E-118 | 96.40288 | 100 | Mm.289584 | 1E-121 | 95.68345 | 100 |
| WAN013I3P_at (SEQ ID NO:1653) | 1.745201 | CAMLG | Calcium modulating ligand | WAN013I3P_at Blast Report | Hs.529846 | 1E-147 | 86.70213 | 99 29577 | #N/A | 1E-172 | 88 6121 | 98 94366 |
| WAN008CI3_at (SEQ ID NO:1680) | 1.811594 | CCNJ | Cyclin J | WAN008CI3_at Blast Report | Hs.596479 | 1E-145 | 93.71429 | 77.60532 | Mm.309 | 1E-105 | 88.85714 | 77.60532 |
| D11437_at (SEQ ID NO:1681) | 1.506024 | Cyp2c55 | Cytochrome P450, family 2, subfamily c. polypep6de 55 | D11437_at Blast Report | #N/A | 2E-73 | 84.0617 | 68.12609 | Mm.142581 | 0 | 91.66667 | 94.57093 |
| X81405_at (SEQ ID NO:1682) | 1.587302 | EN2 | Engrailed homolog 2 | X81405_at Blast Report | Hs.134989 | 5E-69 | 92 59259 | 81.11588 | Mm.4298 | 7E-30 | 92.63158 | 40.77253 |
| U67146_at (SEQ ID NO:1683) | 1.680672 | EEF1E1 | Eukaryotic translation elongation factor 1 epsilon 1 | U67146_at Blast Report | Hs.631818 | 1E-152 | 88.62275 | 63.90306 | Mm.36683 | 0 | 89.70381 | 90.43367 |
| WAN008E2Q_at (SEQ ID NO:1618) | 3 521127 | GSPT1 | G1 to S phase transition 1 | WAN008E2Q_at Blast Report | Hs 528780 | 0 | 9369565 | 100 | Mm.325827 | 0 | 95.86957 | 100 |
| WAN008CWV_at (SEQ ID NO:1684) | 1.66113 | HDGF | Hepatoma-derived growth factor (high-mobility group protein 1-like) | WAN008CWV_a t Blast Report | Hs 506748 | 3E-75 | 91.48936 | 43.04029 | Mm.292208 | 2E-75 | 91.48936 | 43.04029 |
| U43278_at (SEQ ID NO:1685) | 1.626016 | Msr1 | Macrophage scavenger receptor 1 | U43278_at Blast Report | Hs.632045 | 0 | 0 | 0 | Mm.239291 | 7E-64 | 87.5 | 92.8839 |
| Z30972_ at (SEQ ID NO:1686) | 209205 | PPARG | Peroxisome proliferative activated receptor, gamma | Z30972_at Blast Report | Hs. 162646 | 6E-70 | 91.62562 | 46.88222 | Mm.3020 | 1E-78 | 93.17073 | 47.34411 |
| WAN008DRG_at (SEQ ID NO:1687) | 1.607717 | PGM1 | Phosphoglucomulase 1 | WAN008DRG_a t Blast Report | Hs. 1869 | 1E-164 | 90.88937 | 99.78355 | Mm.2325 | 0 | 93.23144 | 99.1342 |
| WAN008EWS_at (SEQ lD N0: 1 688) | 1.66113 | PLAA | PhospholipaseA2-activating^{p}rotein | WAN008EWS_a t Blast Report | Hs.27182 | 0 | 93.45133 | 100 | Mm.22724 | 0 | 93.45133 | 100 |
| WAN008DUZ_at (SEQ ID NO:1689) | 1.538462 | POP7 | Processing of precursor 7, ribonuclease P subunit (S cerevisiae) | WAN008DUZ_at Blast Report | Hs 416994 | 6E-52 | 89 50276 | 35.21401 | Mm.290242 | 9E-62 | 91.71271 | 35.21401 |
| WAN0088X2_at (SEQ ID NO-. 1593) | 3.322259 | PEO1 | Progressive external ophthalmoplegia 1 | WAN0088X2_at Blast Report | Hs 22678 | 1E-141 | 88.65096 | 94.53441 | Mm.105585 | 7E-78 | 90.67797 | 47.77328 |
| WAN008CWW_at (SEQ ID NO:1690) | 2.849003 | PHTF2 | Putative homeodomain transcription factor 2 | WAN008CWW_ at Blast Report | Hs.203965 | 1E-12 | 86.2069 | 21.75 | Mm.86410 | 1E-17 | 86.86869 | 24.75 |
| WAN008EY3_at (SEQ ID NO:1691) | 1.652893 | 5430407P10 Rik | RIKEN cDNA 5430407P10 gene | WAN008EY3_at Blast Report | #N/A | 0.00005 | 85.48387 | 18.12865 | Mm.133542 | 1E-96 | 88.20059 | 99.12281 |
| WAN008D6O_at (SEQ ID NO.1692) | 1.672241 | Strbp | Spermatid pennuclear RNA binding protein | WAN008D6O_at Blast Report | Hs.645506 | 9E-62 | 89.7561 | 67.65677 | Mm237095 | 3E-94 | 95.34884 | 70.9571 |
| WAN008DXC_at (SEQ ID NO:1693) | 2.816901 | TXNDC11 | Thioredoxin domain containing 11 | WAN008DXC_at Blast Report | Hs.313847 | 0 | 94.0678 | 98.95178 | Mm.291015 | 0 | 94.54927 | 100 |
| WAN008DWJ_at (SEQ ID NO:1614) | 3.636364 | USP1 | Ubiquitin specific peptidase 1 | WAN008DWJ_a t Blast Report | Hs.35086 | 0 | 92.94756 | 97.01754 | Mm.371692 | 0 | 94.90909 | 96.49123 |
| WAN013HVL_at (SEQ ID NO:1639) | 1.760563 | UGDH | UDP-glucose dehydrogenase | WAN013HVL_at Blast Report | Hs.572518 | 1E-165 | 90.06772 | 95.88745 | Mm.344831 | 1E-158 | 89.01099 | 98.48485 |
| WAN008CS2_at (SEQ ID NO:1694) | 1.569859 | VKORC1L1 | Vitamin K epoxide reductase complex, subunit 1-like 1 | WAN008CS2_at Blast Report | Hs.427232 | 1E-168 | 91.89189 | 96.73203 | Mm.288718 | 0 | 97.28507 | 96 2963 |
| X02950_at (SEQ ID NO:1695) | 1.547988 | NA | X02950 Hamster alpha-A crystallin gene 5 part (exons 1-3) | X02950_at Blast Report | #N/A | 0 | 0 | 0 | #N/A | 5E-08 | 86.88525 | 10.87344 |
| X56207_at (SEQ ID NO:1696) | 1.531394 | NA | X56207 Hamster gene for myosin heavy chain, exons 1 & 2 | X56207_at Blast Report | #N/A | 0 | 0 | 0 | #N/A | 0.00005 | 92.10526 | 6.713781 |
| WAN008DKJ_x_at (SEQ ID NO.1697) | 1.54321 | Zip297b | Zinc finger protein 297B | WAN008DKJ_x_ at Blast Report | #N/A | 1E-27 | 91.57895 | 90.47619 | Mm.44186 | 2E-24 | 89.69072 | 92.38095 |
| | | | | | | | | | | | | |
| 1.5F Down | | | | | | | | | | | | |
| AB003732_f_at (SEQ ID NO:1698) | 1.542 | NA | AB003732 Cricetulus griseus gene for polyubiquitin, complete cds. | AB003732_f_at Blast Report | #N/A | 2E-14 | 100 | 10.44776 | #N/A | 1E-35 | 88.96104 | 38.30846 |
| WAN008EB0_at (SEQ ID NO:1699) | 2.179 | ACOT7 | Acyl-CoA thioesterase 7 | WAN008EB0_at Blast Report | Hs.126137 | 1E-49 | 88.64865 | 50.40872 | Mm.296191 | 3E-70 | 93.04813 | 50.95368 |
| AF284090_s_at (SEQ ID NO:1700) | 1.508 | ADK | Adenosine kinase | AF284090_s_at Blast Report | Hs.584739 | 2E-42 | 88.41463 | 98.79518 | Mm.188734 | 7E-63 | 93.37349 | 100 |
| AJ286821_at (SEQ ID NO:1701) | 1.629 | NA | AJ286821 Mesocricetus auratus partial mRNA for protein tyrosine phosphatase (ptp gene) | AJ286821_at Blast Report | #N/A | 0 | 0 | 0 | #N/A | 2E-22 | 85.82677 | 34.23181 |
| WAN0013I8B_at (SEQ ID NO:1659) | 1.542 | AKR1A1 | Aldo-keto reductase family 1, member A1 (aldehyde reductase) | WAN013I8B_at Blast Report | Hs.474584 | 1E-170 | 87 56567 | 97.94168 | #N/A | 0 | 9153713 | 99.31389 |
| WAN013IAG_at (SEQ ID NO·1702) | 65 | Areg | Amphiregulin | WAN013IAG_at Blast Report | Hs.270833 | 2E-14 | 84.21053 | 25 | Mm.8039 | 3E-49 | 89.20455 | 38.59649 |
| WAN008DGD_at (SEQ ID NO:1564) | 1.61 | Aplp2 | Amyloid beta (A4) precursor-like protein 2 | WAN008DGD_a t Blast Report | Hs.370247 | 0 | 0 | 0 | Mm.19133 | 6E-69 | 93.08756 | 44.46721 |
| WAN008EMP_at (SEQ ID NO:1703) | 1.709 | CAP1 | CAP, adenylate cyclase-associated protein 1 (yeast) | WAN008EMP_a t Blast Report | Hs.370581 | 1E-56 | 93.40659 | 35.20309 | Mm.8687 | 1E-88 | 92.01389 | 55.706 |
| WAN013I0Y_at (SEQ ID NO.1704) | 1.523 | CAPG | Capping protein (actin filament), gelsolin-like | WAN013I0Y_at Blast Report | Hs.516155 | 1E-112 | 87.1134 | 72.52336 | Mm.18626 | 1E-153 | 89.65517 | 81.30841 |
| AF081141_at (SEQ ID NO:1667) | 21.04 | CCL2 | Chemokine (C-C motif) ligand 2 | AF081141_at Blast Report | Hs.303649 | 3E-13 | 90.625 | 13.41719 | Mm 290320 | 5E-41 | 91.04478 | 28.09224 |
| WAN013I8F_at (SEQ ID NO:1705) | 2.19 | MA | Cluster includes AF308456 Cricetulus griseus intracellular adhesion molecule 1 (ICAM1) mRNA, complete cds. | WAN013I8F_at Blast Report | #N/A | 0 | 0 | 0 | #N/A | 4E-07 | 91.80328 | 4.552239 |
| WAN013I8Y_at (SEQ ID NO:1706) | 1.528 | NA | Cluster includes M23159 Chinese hamster DHFR-coamplified protein mRNA, partial cds, clone 2BE2121. | WAN013I8Y_at Blast Report | #N/A | 0 | 84.15233 | 70.65972 | #N/A | 0 | 89.18919 | 70.65972 |
| WAN013HWP_x_at (SEQ ID NO:1707) | 1.59 | NA | Cluster includes WAN008CUN 10602C-E01 | WAN013HWP_x _at Blast Report | #N/A | 0 | 0 | 0 | #N/A | 8E-76 | 92.82297 | 45.93407 |
| WAN013HZK_at (SEQ ID NO:1643) | 2.279 | NA | Cluster includes WAN018DS2 11228C-H04 | WAN013HZK_at Blast Report | #N/A | 0 | 0 | 0 | #N/A | 6E-10 | 88.37209 | 17.58691 |
| WAN013I73_at (SEQ ID NO:1708) | 1.564 | NA | Cluster includes X61958 C.longicaudatus mRNA for thrombin receptor | WAN013I73_at Blast Report | #N/A | 0 | 0 | 0 | #N/A | 4E-45 | 88.54167 | 42.01313 |
| WAN008EHM_at (SEQ ID NO:1709) | 2.033 | CLU | Clusterin | WAN0D8EHM_a t Blast Report | Hs.436657 | 1E-71 | 91.38756 | 36.60245 | Mm.200608 | 1E-91 | 90.74074 | 47.28546 |
| WAN013HWY_at (SEQ ID NO:1710) | 1.658 | CCDC80 | Coiled-coil domain containing 80 | WAN013HWY_a t Blast Report | Hs.477128 | 6E-92 | 86 55914 | 76.07362 | Mm.181074 | 1E-171 | 90.57377 | 99.7955 |
| WAN008CST_at (SEQ ID NO:1711) | 1.581 | COPS2 | COP9 constitutive photomorphogenic homolog subunit 2 (Arabidopsis) | WAN008CST_at Blast Report | Hs.369614 | 0 | 94.16058 | 100 | Mm.3596 | 0 | 96.89781 | 100 |
| U71399_at (SEQ ID NO:1712) | 1.593 | Cyb5 | Cytochrome b-5 | U713999_at Blast Report | #N/A | 4E-62 | 85 09934 | 60.4 | Mm.31018 | 1E-103 | 90.9396 | 59.6 |
| WAN0088LE_at (SEQ ID NO:1713) | 1.52 | DSTN | Destrin (actin depolymerizing factor) | WAN0088LE_at Blast Report | Hs.304192 | 1E-169 | 90.38855 | 87.47764 | Mm.28919 | 0 | 93.04511 | 95.16995 |
| WAN008EAZ_at (SEQ ID NO:1714) | 1.597 | DPP3 | Dipeptidyl-peptidase 3 | WAN008EAZ_at Blast Report | Hs.502914 | 1E-161 | 90.9292 | 96.17021 | Mm.234769 | 0 | 94.68085 | 100 |
| WAN008CYZ_at (SEQ ID NO:1715) | 1.929 | EFNB2 | Ephrin-82 | WAN008CYZ_at Blast Report | Hs.149239 | 2E-27 | 85.81081 | 25.69444 | Mm.209813 | 1E-102 | 89.6648 | 62.15278 |
| WAN013HYK_at (SEQ ID NO:1716) | 3.18 | EPS8 | Epidermal growth factor receptor pathway substrate 8 | WAN013HYK_at Blast Report | Hs.591160 | 2E-15 | 89.28571 | 14.50777 | Mm.235346 | 2E-13 | 93.75 | 13.81693 |
| AF046870_at (SEQ ID NO:1717) | 1 536 | Efemp2 | Epidermal growth factor-containing fibulin-like extracellular matrix protein 2 | AF046870_at Blast Report | #N/A | 0 | 88.50856 | 92 4642 | Mm.276367 | 0 | 91.79567 | 97.36247 |
| WAN013I3F_at (SEQ ID NO:1718) | 1 816 | ETHE1 | Ethylmalonic encephatopathy 1 | WAN013I3F_at Blast Report | Hs.7486 | 1E-111 | 84.33515 | 98.21109 | Mm.29553 | 0 | 90.87657 | 100 |
| WAN008EUG_at (SEQ ID NO.1719) | 1.621 | EXOC6 | Exocyst complex component 6 | WAN008EUG_a t Blast Report | Hs.292097 | 2E-97 | 89.27445 | 95.06061 | Mm.24865 | 1E-124 | 93.44262 | 92.42424 |
| AF061256_at (SEQ ID NO:1720) | 1.971 | FOLR1 | Folate receptor 1 (adult) | AF061256_at Blast Report | Hs 73769 | 1E-124 | 83.96825 | 61.76471 | Mm.2135 | 0 | 89.23077 | 76.47059 |
| WAN008EMJ_at (SEQ ID NO:1721) | 1.873 | GPC6 | Glypican 6 | WAN008EMJ_at Blast Report | Hs.4444329 | 7E-64 | 92.55319 | 37.4502 | Mm.234129 | 3E-58 | 90.37433 | 37251 |
| WAN0088O4_at | 1 556 | IMPDH1 | IMP (inosine monophosphate | WAN0088O4_at | Hs.534808 | 1E-170 | 91 64835 | 89 39096 | Mm 260707 | 0 | 91 28713 | 99 21415 |

| (SEQ ID NO:1722) | | | dehydrogenase 1 | Blast Report | | | | | | | | |
|---|---|---|---|---|---|---|---|---|---|---|---|---|
| WAN013I3K_at (SEQ ID NO:1723) | 1.872 | IDH1 | Isocitrate dehydrogenase 1 (NADP+), soluble | WAN013I3K_at Blast Report | Hs.11223 | 0 | 91.04478 | 99.44341 | Mm.9925 | 0 | 93.47015 | 99.44341 |
| WAN008E63_at (SEQ ID NO:1724) | 1.733 | JAK1 | Janus kinase 1 (a protein tyrosine kinase) | WAN008E63_at Blast Report | Hs.207538 | 1E-142 | 89 83834 | 82.79159 | Mm.289657 | 0 | 94.53303 | 83.93881 |
| WAN008E9N_at (SEQ ID NO: 1620) | 1.542 | KLHL7 | Kelch-like 7 (Drosophila) | WAN008E9N_at Blast Report | Hs.385861 | 1E-150 | 89.44223 | 99.40594 | #N/A | 0 | 93.11111 | 89.10891 |
| WAN008EZV_at (SEQ ID NO:1725) | 1.777 | KIF1B | Kinesin family member 1B | WAN008EZV_at Blast Report | Hs.97858 | 1E-176 | 89 38053 | 98.77622 | Mm.402393 | 1E-14 | 89.18919 | 12.93706 |
| AF093673_at (SEQ ID NO:1726) | 1.703 | LLN | layilin | AF093673_at Blast Report | #N/A | 1E-158 | 88.54962 | 38.67159 | #N/A | 1E-149 | 90.2439 | 33.28413 |
| M96676_at (SEQ ID NO:1727) | 1.542 | LGALS1 | Lectin, galactoside-binding, soluble, 1 (galectin 1) | M96676_at Blast Report | Hs.445351 | 1E-122 | 88.94472 | 100 | Mm.43831 | 1E-131 | 89.94975 | 100 |
| WAN008ERP_at (SEQ ID NO:1634) | 1.673 | LEPREL1 | Leprecan-like 1 | WAN008ERP_at Blast Report | Hs.374191 | 1E-45 | 87.12446 | 92.82869 | Mm.326869 | 1E-68 | 88.98305 | 94.0239 |
| WAN008EUO_at (SEQ ID NO:1635) | 2.105 | LPL | lipoprotein lipase | WAN008EUO_a t Blast Report | HS.180878 | 2E-82 | 87.5 | 74.10926 | Mm.1514 | 1E-114 | 91.66667 | 74.10926 |
| L18986_at (SEQ ID NO:1501) | 1.619 | LAMP1 | Lysosomal-associated membrane protein 1 | L18986_at Blast Report | Hs.494419 | 4E-58 | 85.66176 | 20.76336 | Mm.16716 | 1E-157 | 86.94915 | 45.03817 |
| WAN013I8P_at (SEQ ID NO:1728) | 1.818 | LAMP2 | Lysosomal-associated membrane protein 2 | WAN013I8P_at Blast Report | Hs.496684 | 1E-133 | 86.23853 | 93.32192 | Mm.486 | 0 | 91.20287 | 95.37671 |
| AF306662_at (SEQ ID NO:1729) | 2.002 | MMP14 | Matrix metallopeptidase 14 (membrane-inserted) | AF306662_at Blast Report | Hs.2399 | 0 | 89.72603 | 99.82906 | Mm.280175 | 0 | 90.08547 | 100 |
| WAN008DBL_at (SEQ ID NO:1730) | 1568 | NDUFB9 | NADH dehydrogenase (ubiquinone) 1 beta subcomplex, 9, 22kDa | WAN008DBL_at Blast Report | Hs. 15977 | 1E-120 | 85.76923 | 93.86282 | Mm.322294 | 1E-180 | 90.67961 | 92.96029 |
| WAN008BSC_at (SEQ ID NO:1731) | 1706 | NRD1 | Nardilysin (N-arginine dibasic convertase) | WAN008BSC_at Blast Report | Hs 584782 | 1E-113 | 90 82569 | 60 78067 | Mm 274950 | 1E-131 | 93.00912 | 61.15242 |
| WAN013I62_at (SEQ ID NO:1732) | 1.607 | ODC1 | Ornithine decarboxylase 1 | WAN013I62_at Blast Report | Hs.467701 | 1E-178 | 85.99222 | 56.64952 | Mm.34102 | 0 | 91.76788 | 54.44526 |
| WAN008DXO_at (SEQ ID NO:1733) | 2.241 | OSBPL9 | Oxysterol binding protein-like 9 | WAN008DXO_a t Blast Report | Hs.21938 | 0 | 92.9368 | 98.89706 | Mm.366315 | 0 | 93.93382 | 100 |
| AB014875_at (SEQ ID NO:1734) | 1.787 | PLS3 | plastin 3 (T isoform) | AB014875_at Blast Report | Hs.496622 | 1E-159 | 92 | 31.59851 | Mm.28777 | 1E-175 | 90.32847 | 40.74349 |
| AF221841_at (SEQ ID NO.1735) | 1.564 | Mm 379870 | PREDICTED: Mus musculus similar to Peroxiredoxin 1 (Thioredoxin peroxidase 2) (Thioredoxin-dependent peroxide reductase 2) (Osteoblast specific factor 3) (OSF-3) (Macrophage 23 kDa stress protein) (LOC545161), mRNA | AF221841_at Blast Report | #N/A | 0 | 9097889 | 100 | #N/A | 0 | 93.56725 | 98.46449 |
| WAN008DSZ_at (SEQ ID NO.1736) | 1 522 | PPP1R7 | Protein phosphatase 1, regulatory subunit 7 | WAN008DSZ_at Blast Report | Hs.36587 | 1E-125 | 89.08189 | 100 | Mm.88704 | 1E-163 | 93.05211 | 100 |
| AB056121_at (SEQ ID NO:1737) | 2.595 | S100B | S100 calcium binding protein, beta (neural) | AB056121_at Blast Report | Hs.422181 | 6E-85 | 89.28571 | 83.58209 | Mm.235998 | 1E-108 | 90.14925 | 100 |
| WAN008DSW_at (SEQ ID NO:1738) | 1.732 | STAT3 | Signal transducer and activator of transcription 3 (acute-phase response factor) | WAN008DSW_a t Blast Report | Hs.463059 | 0 | 91 91729 | 97.25777 | Mm.249934 | 0 | 94.11765 | 99.45155 |
| WAN013I2Q_at (SEQ ID NO:1739) | 1.694 | SSU72 | SSU72 RNA polymerase II CTD phosphatase homolog (S. cerevisiae) | WAN013I2Q_at Blast Report | Hs.30026 | 1E-132 | 91.57303 | 71.91919 | Mm.294770 | 1E-171 | 91.47982 | 90.10101 |
| WAN008D44_at (SEQ ID NO:1740) | 1.854 | SCP2 | Sterol carrier protein 2 | WAN008D44_at Blast Report | Hs.476365 | 1E-115 | 89.53168 | 64.3617 | Mm.379011 | 1E-132 | 91.46006 | 64.3617 |
| WAN008EKU_at (SEQ ID NO:1741) | 1.503 | TAX1BP1 | Tax1 (human T-cell leukemia virus type I) binding protein 1 | WA008EKU_at Blast Report | Hs.34576 | 1E-120 | 89.5122 | 99.51456 | Mm.431979 | 0 | 0 | 0 |
| WAN013I4X_at (SEQ ID NO:1742) | 1.676 | Timp2 | Tissue inhibitor of metalloproteinase 2 | WAN013I4X_at Blast Report | Hs.633514 | 0 | 0 | 0 | Mm.206505 | 0 | 94.2623 | 100 |
| AF113614_at (SEQ ID NO.1668) | 1 823 | TLR2 | Toll-like receptor 2 | AF113614_at Blast Report | Hs.519033 | 1E-101 | 86.26505 | 80.89669 | Mm.87596 | 1E-167 | 92.32558 | 83.82066 |
| WAN008DM2_at (SEQ ID NO:1743) | 1.541 | TRAPPC3 | Trafficking protein particle complex 3 | WAN008DM2_at Blast Report | Hs.523131 | 1E-129 | 93.18885 | 69.3133 | Mm.8392 | 1E-130 | 93.18885 | 69.3133 |
| WAN013I8T_at (SEQ ID NO_{.}1744) | 1.877 | Mm.392113 | Transcribed locus, moderately similar to XP_426592.1 PREDICTED: similar to tubulin, alpha 2; tubulin alpha 2 [Gallus gallus] | WAN013I8T_at Blast Report | #N/A | 0 | 90 74941 | 79.65418 | #N/A | 0 | 94.36202 | 94.3097 |
| WAN0088TW_at (SEQ ID NO:1745) | 1.618 | TCEB3 | Transcription elongation factor B (SIII), polypeptide 3 (110kDa, elongin A) | WAN0088TW_at Blast Report | Hs.584806 | 1E-173 | 89.36567 | 98.52941 | Mm.27663 | 0 | 93.09701 | 98.52941 |
| WAN008CZR_at (SEQ ID NO.1746) | 1.522 | TMED1 | Transmembrane emp24 protein transport domain containing 1 | WAN008CZR_at Blast Report | Hs.515139 | 1E-139 | 87.57515 | 90.72727 | Mm.196618 | 0 | 94.89194 | 92.54545 |
| WAN008D2L_at (SEQ ID NO:1747) | 2.049 | TMEM50A | Transmembrane protein 50A | WAN008D2L_at Blast Report | Hs 523054 | 2E-76 | 85.79882 | 80.09479 | Mm.88349 | 1E-63 | 86 92308 | 61 61137 |
| WAN013I4D_at (SEQ ID NO:1532) | 2.02 | TAP2 | Transporter 2, ATP-binding cassette, sub-family B (MDR/TAP) | WAN013I4D_at Blast Report | Hs.502 | 5E-33 | 8325792 | 46.33124 | Mm.14814 | 1E-111 | 88.95028 | 75.89099 |
| U22818_at (SEQ ID NO.1748) | 1.582 | NA | U22818 Cricetulus griseus SRD-2 mutant sterol regulatory element binding protein-2 (SREBP-2) MRNA, complete cds. | U22818_at Blast Report | #N/A | 0 | 0 | 0 | #N/A | 1E-35 | 87.81726 | 15.91276 |
| AF004368_at (SEQ ID NO.1749) | 1.618 | UGP2 | UDP-glucose pyrophosphorylase 2 | AF004368_at Blast Report | Hs.516217 | 1E-116 | 91.77215 | 53.92491 | Mm.28877 | 1E-147 | 90.86538 | 70.98976 |
| WAN008D7G_at (SEQ ID NO:1750) | 1.52 | VAMP3 | Vesicle-associated membrane protein 3 (cellubrevin) | WAN008D7G_at Blast Report | Hs.66708 | 4E-07 | 97.56098 | 7.400722 | Mm.273930 | 9E-19 | 91.86047 | 15.52347 |
| WAND13HVO_at (SEQ ID NO:1751) | 1.541 | YBX1 | Y box binding protein 1 | WAN013HVO_a t Blast Report | Hs.473583 | 0 | 93.86139 | 99.40945 | Mm.258204 | 0 | 95.54455 | 99.40945 |

### Example 9. Genes differentially expressed in cells with sustained high cell viability

Bcl-xL is a powerful inhibitor of cell death. Cells overpressing Bcl-xL demonstrate sustained high cell viability. Tables **18 and 19** summarize nucleic acids that are differentially expressed by a factor of at least 1.2 in cells overexpressing Bcl-xL. Samples were taken at multiple time points for comparison. Table **18** summarizes nucleic acids that are differentially expressed by a factor of at least 1.2 at day 5. Table **19** summarizes nucleic acids that are differentially expressed by a factor of at least 1.2 at a stage later than day 5.

**Table 18: d5 comparison**

| **DOWN (Originally 173)** | | | | | | | | | | | | |
|---|---|---|---|---|---|---|---|---|---|---|---|---|
| **Qualifier List** | **Fold Change** | **Symbol** | **Title** | **Human Unigene ID** | **eValue** | **%ID** | **%QC** | **Mouse Unigene ID** | **eValue** | **%ID** | **%QC** | |
| WAN008DUG_at (SEQ ID NO:1752) | 1.582 | Hibadh | 3-hydroxyisobutyrate dehydrogenase | WAN008DUG_at Blast Report | Hs.406758 | 0.00000002 | 90.78947 | 1374322 | Mm 286458 | 3E-43 | 91.55844 | 27.8481 |
| WAN008EB0_at (SEQ ID NO:1699) | 1.796 | ACOT7 | Acyl-CoA thioesterase 7 | WAN008EB0_at Blast Report | Hs.126137 | 1E-49 | 88.64865 | 50.40872 | Mm.296191 | 3E-70 | 93.04813 | 50.95368 |
| WAN00CT8_at (SEQ ID NO:1522) | 1.533 | AP2M1 | Adaptor-related protein complex 2, mu 1 subunit | WAN008CT8_at Blast Report | Hs.518460 | 0 | 94.38445 | 80 80279 | Mm.18946 | 0 | 95.48387 | 81.15183 |
| AF120325_x_at (SEQ ID NO:1753) | 2.101 | NA | AF120325 Cricetulus gnseus class I beta tubulin gene, complete cds. | AF120325_x_at Blast Report | #N/A | 0 | 87.58085 | 83 83948 | #N/A | 0 | 91.625 | 86.7679 |
| WAN008EKP_at (SEQ ID NO.1754) | 1.752 | ATP5B | ATP synthase, H+ transporting, mitochondrial F1 complex, beta polypeptide | WAN008EKP_at Blast Report | Hs.406510 | 0 | 93.25397 | 99 80198 | Mm. 238973 | 0 | 93.2 | 99.0099 |
| WAN008F20_x_at (SEQ ID NO:1755) | 1 544 | BCCIP | BRCA2 and CDKN1A interacting protein | WAN008F20_x_at Blast Report | Hs.370292 | 1E-26 | 85.31073 | 81.56682 | Mm 389983 | 5E-21 | 89.77273 | 40.553 |
| WAN008DPO_at (SEQ ID NO:1756) | 1.636 | BTBD1 | BTB (POZ) domain containing 1 | WAN008DPO_at Blast Report | Hs.459149 | 3E-32 | 844 | 50.2008 | Mm.71103 | 7E-41 | 87.08134 | 41.96787 |
| WAN013I3P_at (SEQ ID NO.1653) | 1.804 | CAMLG | Calcium modulating ligand | WAN013I3P_at Blast Report | Hs.529846 | 1E-147 | 86.70213 | 9929577 | #N/A | 1E-172 | 88.6121 | 98.94366 |
| WAN008DSX_at (SEQ ID NO:1757) | 3 055 | CALM1 | Calmodulin 1 (phosphorylase kinase, delta) | WAN008DSX_at Blast Report | Hs.282410 | 7E-51 | 94.07895 | 38 48101 | Mm.285993 | 6E-84 | 90.80882 | 68.86076 |
| WAN013HVK_at (SEQ ID NO:1758) | 1.581 | Arpp19 | CAMP-regulated phosphoprotein 19 | WAN013HVK_at Blast Report | #N/A | 1E-164 | 88.38028 | 97.93103 | Mm.247837 | 0 | 94.3761 | 98.10345 |
| WAN008ECX_at (SEQ ID NO.1759) | 1.628 | Cd151 | CD151 antigen | WAN008ECX_at Blast Report | #N/A | 9E-85 | 86.36364 | 65.0647 | Mm.30246 | 1E-155 | 94.85714 | 64.69501 |
| WAN008D27_at (SEQ ID NO:1760) | 1.57 | CLTA | Clathrin, right polypeptide (Lca) | WAN008D27_at Blast Report | Hs.522114 | 0 | 94.06308 | 99.26335 | Mm.298875 | 0 | 94.83395 | 99.81584 |
| WAN008DS9_at (SEQ ID NO:1613) | 1.583 | CFL2 | Cofilin 2 (muscle) | WAN00DS9_at Blast Report | Hs 180141 | 1E-113 | 89.01869 | 9087049 | Mm.276826 | 1E-132 | 92 98246 | 84.71338 |
| AF022941_x_at (SEQ ID NO.1761) | 2.041 | Cirbp | Cold inducible RNA binding protein | AF022941_x_at Blast Report | Hs.634522 | 9E-27 | 93.65079 | 6961326 | Mm.17898 | 1E-52 | 96.26866 | 74.03315 |
| WAN008EEB_at (SEQ ID NO:1762) | 1 779 | CORO1B | Coronin, actin binding 1B | WAN008EEB_at Blast Report | Hs6191 | 2E-95 | 90.34483 | 55 98456 | Mm.276859 | 1E-128 | 94.61279 | 57.33591 |
| WAN0088J2_at (SEQ ID NO:1763) | 1.63 | CUEDC2 | CUE domain containing 2 | WAN0088J2_at Blast Report | Hs.500874 | 1E-145 | 87 9017 | 98.8785 | Mm.218848 | 1E-156 | 88.18011 | 99.62617 |
| WAN0088PY_at (SEQ ID NO:1480) | 1.609 | Ddx5 | DEAD (Asp-Glu-Ala-Asp) box polypeptide 5 | WAN0088PY_at Blast Report | Hs 279806 | 0 | 0 | 0 | Mm.220038 | 00000002 | 92 85714 | 9.230769 |
| WAN013I1H_at (SEQ ID NO:1764) | 1.601 | D19Bwg1357e | DNA segment, Chr 19, Brigham & Women's Genetics 1357 expressed | WAN013I1H_at Blast Report | #N/A | 1E-167 | 86.99473 | 99.64974 | Mm.261027 | 0 | 88.61646 | 100 |
| WAN008DVF_at (SEQ ID NO:1765) | 1.509 | DNAJC7 | DnaJ (Hsp40) homolog, subfamily C, member 7 | WAN008DVF_at Blast Report | Hs.500156 | 1E-143 | 89.33333 | 100 | Mm.258140 | 0 | 95.11111 | 100 |
| WAN013I3F_at (SEQ ID NO:1718) | 1.544 | ETHE1 | Ethylmalonic encephalopathy 1 | WAN013I3F_at Blast Report | Hs.7486 | 1E-111 | 84.33515 | 98.21109 | Mm.29553 | 0 | 90.87657 | 100 |
| WAN013HZ5_at (SEQ ID NO:1766) | 1.592 | EIF2S2 | Eukaryotic tanslation initiation factor 2, subunit 2 beta, 38kDa | WAN013HZ5_at Blast Report | Hs 429180 | 0 | 93.29004 | 83.69565 | Mm.377134 | 0 | 96.31236 | 83.51449 |
| WAH008E8R_at (SEQ ID NO:1767) | 1.522 | EIF3S1 | Eukaryotic translation initiation factor 3, subunit 1 alpha, 35kDa | WAN008E8R_at Blast Report | Hs.404056 | 1E-122 | 93.13725 | 7116279 | Mm.27695 | 1E-140 | 95.46926 | 71.86047 |
| WAN013HZP_at SEQ ID NO:1644) | 1.532 | Eif4g2 | Eukaryotic translation initiation factor 4, gamma 2 | WAN013HZP_at Blast Report | Hs.183684 | 1E-178 | 97.74648 | 72.44898 | Mm.185453 | 0 | 99.71831 | 72.44898 |
| WAN013IA4_at (SEQ ID NO:1768) | 1.97 | ETF1 | Eukaryotic translation termination factor 1 | WAN013IA4_at Blast Report | Hs 483494 | 1E-174 | 92.89827 | 98.11676 | Mm.329353 | 0 | 95.2919 | 100 |
| WAN008E82_at (SEQ ID NO:1769) | 1.522 | FbxI11 | F-box and leucine-rich repeat protein 11 | WAN008E82_at Blast Report | Hs 124147 | 8E-57 | 87.68657 | 64.57831 | Mm.31941 | 1E-162 | 93.9759 | 100 |
| D43757_at (SEQ ID NO:1770) | 1.581 | FGA | fibrinogen alpha chain | D43757_at Blast Report | Hs.351593 | 6E-15 | 85.04673 | 19.31408 | Mm.88793 | 3E-40 | 82.95455 | 47.65343 |
| WAN008E72_x_at (SEQ ID NO:1547) | 2.339 | GDI2 | GDP dissociation inhibitor 2 | WAN008E72_x_at Blast Report | Hs 299055 | 6E-25 | 86.13861 | 100 | Mm.153226 | 9E-72 | 94.05941 | 100 |
| WAN008EXR_at (SEQ ID NO:1771) | 1574 | GPI | Glucose phosphate isomerase | WAN008EXR_at Blast Report | Hs 466471 | 9E-81 | 86.35015 | 100 | #N/A | 1E-103 | 89.22156 | 99.10979 |
| WAN008EQH_at (SEQ ID NO:1772) | 1.813 | GLUD1 | Glutamate dehydrogenase 1 | WAN008EQH_at Blast Report | Hs.500409 | 5E-15 | 94.54545 | 12.22222 | Mm.10600 | 1E-57 | 90.26549 | 50.22222 |
| WAN008BR0_at (SEQ ID NO:1773) | 1.564 | GOT2 | Glutamic-oxaloacetic transaminase 2, mitochondrial (aspartate aminotransferase 2) | WAN008BR0_at Blast Report | Hs 599470 | 0.000001 | 83.95062 | 15 0838 | Mm.230169 | 6E-26 | 88.80597 | 24.95345 |
| WAN008CFZ_at (SEQ ID NO:1774) | 1.601 | HSBP1 | Heat shock factor binding protein 1 | WAN008CFZ_at Blast Report | Hs 250899 | 6E-18 | 90 | 14.76015 | Mm.358714 | 6E-29 | 91.17647 | 18.81919 |
| WAN013I1P_at (SEQ ID NO:1492) | 1.587 | HNRPA2B1 | Heterogeneous nuclear ribonucleoprotein A2/B1 | WAN013I1P_at Blast Report | Hs.487774 | 0 | 97.22222 | 90.94737 | Mm.155896 | 0 | 96.52778 | 90.94737 |
| Y00385_at (SEQ ID NO:1674) | 1.895 | HMGB1 | High-mobility group box 1 | Y00365_at Blast Report | Hs.434102 | 1E-104 | 93.97993 | 23.39593 | Mm.207047 | 1E-145 | 88.84058 | 53.99061 |
| WAN008EKL_at (SEQ ID NO:1775) | 1512 | HBP1 | HMG-box transcription factor 1 | WAN008EKL_at Blast Report | Hs162032 | 1E-120 | 89.60784 | 98.4556 | Mm.390461 | 0 | 91.68279 | 99.80695 |
| WAN013I8N_at (SEQ ID NO:1776) | 1709 | IMPDH2 | IMP (inosine monophosphate) dehydrogenase 2 | WAN013I8N_at Blast Report | Hs.476231 | 0 | 90.28974 | 95.36968 | Mm.6065 | 0 | 93.18358 | 96.41524 |
| WAN008809_at ' (SEQ ID NO:1777) | 2.845 | Itgb1 | Integrin beta 1 (fibronectin receptor beta) | WAN0088O9_at Blast Report | Hs.295626 | 1E-13 | 90.32258 | 16.48936 | Mm.263396 | 4E-55 | 9049774 | 39.1844 |
| AF180918_at (SEQ ID NO:1778) | 1.742 | KLHL5 | Keich-like 5 (Drosophila) | AF180918_at Blast Report | Hs.272251 | 6E-21 | 88.57143 | 19.77401 | Mm.10281 | 5E48 | 86.2069 | 49.15254 |
| WAN008E26_x_at (SEQ ID NO:1779) | 2.466 | KLHL7 | Keich-like 7 (Drosophila) | WAN008E26_x_at Blast Report | Hs.385861 | 7E-11 | 90.90909 | 57.89474 | #N/A | 6E-13 | 87.77778 | 78.94737 |
| WAN008BNG_at (SEQ ID NO:1780) | 1.568 | LRRC28 | Leucine nch repeat containing 28 | WAN008BNG_at Blast Report | Hs.578684 | 4E-90 | 93.77778 | 45.91837 | Mm.31247 | 1E-85 | 92.88889 | 45.91837 |
| WAN008EKF_at (SEQ ID NO:1781) | 1.623 | Lass2 | Longevity assurance homolog 2 (S cerevisiae) | WAN008EKF_at Blast Report | Hs.643565 | 1E-151 | 89.65517 | 98.7234 | Mm.181009 | 0 | 94.20601 | 99.14894 |
| L18986_at (SEQ ID NO:1501) | 1.584 | LAMP1 | Lysosomal-associated membrane protein 1 | L18986_at Blast Report | Hs.494419 | 4E-58 | 85 66176 | 2076336 | Mm.16716 | 1E-157 | 86.94915 | 45.03817 |
| WAN008F1L_at (SEQ ID NO:1782) | 1.676 | Mxi1 | Max interacting protein 1 | WAN008F1L_at Blast Report | Hs.501023 | 1E-127 | 90.23355 | 84.86486 | Mm.2154 | 1E-131 | 88.44765 | 99.81982 |
| WAN008ESO_at (SEQ ID NO:17883) | 1.757 | MPP6 | Membrane protein, palmitoylated 6 (MAGUK p55 subfamily member 6) | WAN008ESO_at Blast Report | Hs.533355 | 0 | 92 12598 | 99 60784 | Mm.41288 | 0 | 94.88189 | 99.60784 |
| J00061_at (SEQ ID NO: 1784) | 1.861 | MT1 | metallothionein I | J00061_at Blast Report | #N/A | 3E-46 | 87.70053 | 66.31206 | Mm.192991 | 2E-64 | 91.89189 | 65.60284 |
| X79864_at (SEQ ID NO: 1785) | 1.741 | MRPL12 | Mitochondrial ribosomal protein L12 | X79864_at Blast Report | Hs.109059 | 4E-44 | 85 26786 | 38.75433 | Mm.133851 | 1E-107 | 85.79336 | 93.77163 |
| WAN008E43_at (SEQ ID NO 1786) | 1 583 | MRPL30 | Mitochondrial ribosomal protein L30 | WAN008E43_at Blast Report | Hs.590896 | 6E-86 | 87.12575 | 59.53654 | Mm.26614 | 1E-130 | 89.42308 | 74.1533 |
| WAN008DKS_at (SEQ ID NO:1787) | 1.615 | MAPK8IP1 | Mitogen-activated protein kinase 8 interacting protein 1 | WAN008DKS_at Blast Report | Hs.234249 | 1E-116 | 92.16867 | 68.73706 | Mm.2720 | 0 | 93.39019 | 97.10145 |
| WAN008CQE_at (SEQ ID NO 1788) | 1747 | NDUFB6 | NADH dehydrogenase (ubiquinone) 1 beta subcomplex, 6, 17kDa | WAN008CQE_at Blast Report | Hs.493668 | 5E-40 | 87.28324 | 33 7232 | Mm.1103 | 1E-128 | 87.47475 | 96.49123 |
| WAN008EE0_x_at (SEQ ID NO 1789) | 1.671 | NDUFS1 | NADH dehydrogenase (ubiquinone) Fe-S protein 1, 75kDa (NADH-coenzyme Q reductase) | WAN008EE0_x_at Blast Report | Hs.471207 | 0.00002 | 88.23529 | 13.31593 | Mm.290791 | 4E-26 | 84.47205 | 42.03655 |
| WAN013I17_at (SEQ ID NO.1790) | 2237 | NID1 | Nidogen 1 | WAN013I17_at Blast Report | Hs 356624 | 1E-101 | 83.47826 | 83.03249 | Mm.4691 | 9E-99 | 91.66667 | 45.48736 |
| WAN0088NY_at (SEQ ID NO:1791) | 1.61 | NSMCE1 | Non-SMC element 1 homolog (S. cerevisiae) | WAN0088BNY_at Blast Report | Hs.284295 | 1E-129 | 92.30769 | 84.0796 | Mm.4467 | 1E-146 | 94.3787 | 84.0796 |
| WAN0088KK_x_at (SEQ ID NO:1792) | 1.779 | Nfe2I2 | Nuclear factor, erythroid derived 2, like 2 | WAN0088KK_x_at Blast Report | Hs.155396 | 1E-30 | 95.58824 | 38.96848 | Mm.1025 | 4E-75 | 91.12903 | 71.06017 |
| WAN013I62_at (SEQ ID NO:1732) | 1.843 | ODC1 | Ornithine decarboxylase 1 | WAN013I62_at Blast Report | Hs.467701 | 1E-178 | 85.99222 | 56.64952 | Mm.34102 | 0 | 91.76788 | 54.44526 |
| WAN013I2X_at (SEQ ID NO:1793) | 2.366 | PPIG | Peptidylprolyl isomerase G (cyclophilin G) | WAN013I2X_at Blast Report | Hs.470544 | 1E-129 | 88 28633 | 98 08511 | Mm.11815 | 0 | 93.76344 | 98.93617 |
| AB041733_at (SEQ ID NO:1794) | 1 693 | PEX12 | Peroxisomal biogenesis factor 12 | AB041733_at Blast Report | Hs.591190 | 1E-39 | 92.56198 | 9.173616 | Mm.102205 | 4E-75 | 86.98413 | 23.88173 |
| WAN008DUC_at (SEQ ID NO:1795) | 1.657 | PHF14 | PHD finger protein 14 | WAN008DUC_at Blast Report | Hs 159918 | 8E-86 | 94 63415 | 99 51456 | Mm 212411 | 4E-74 | 92.19512 | 99.51456 |
| AB004109_at (SEQ ID NO:1796) | 2124 | PTDSS2 | Phosphatidylserine synthase 2 | AB004109_at Blast Report | Hs.12851 | 0 | 91.24767 | 40.40632 | Mm.293591 | 0 | 89.98288 | 87.88563 |
| WAN0088ZP_at (SEQ ID NO:1482) | 1.519 | Pawr | PRKC, apoptosis, WT1, regulator | WAN0088ZP_at Blast Report | Hs 643130 | 4E-10 | 91 52542 | 11.11111 | Mm.391419 | BE-53 | 91.62562 | 38.22976 |
| WAN008E7A_at (SEQ ID NO:1797) | 1.93 | PGRMC1 | Progesterone receptor membrane component 1 | WAN008E7A_at Blast Report | Hs 90061 | 1E-164 | 9143426 | 98.04688 | Mm.9052 | 1E-170 | 91.63347 | 98.04688 |
| WAN0088KG_at | 1.642 | PPGB | Protective protein for beta- | WAN0088KG_at Blast | Hs 517076 | 1E-115 | 87.5895 | 72.86957 | Mm.359633 | 1E-149 | 90.93079 | 72.86957 |

| (SEQ ID NO:1539) | | | galactosidase (galactosialidosis) | Report | | | | | | | | |
|---|---|---|---|---|---|---|---|---|---|---|---|---|
| WAN0088XS_at (SEQ ID NO:1798) | 1.678 | PKN2 | Protein kinase N2 | WAN0088XS_at Blast Report | Hs.440833 | 8E-13 | 85.04673 | 34 96732 | Mm.244236 | 7E-12 | 84.40367 | 35.62092 |
| WAN0088YU_at (SEQ ID NO:1799) | 1.556 | PPP4C | Protein phosphatase 4 (formerly X), catalytic subunit | WAN0088YU_at Blast Report | Hs.534338 | 1E-107 | 94.02985 | 89.63211 | Mm.41998 | 1E.107 | 94.02985 | 89.63211 |
| WAN0086X9_at (SEQ ID NO:1553) | 1.501 | RAB34 | RAB34, member RAS oncogene family | WAN0088X9_at Blast Report | Hs.301853 | 1E-108 | 89.17379 | 66.73004 | Mm.275864 | 1E-161 | 92.15686 | 87.26236 |
| WAN008CLK_at (SEQ ID NO:1552) | 1.56 | RAB6A | RAB6A, member RAS oncogene family | WAN008CLK_at Blast Report | Hs.12152 | 2E-55 | 88.47737 | 4821429 | #N/A | 1E-161 | 92.07317 | 97.61905 |
| AB015640_at (SEQ ID NO:1800) | 1.554 | RANBP9 | RAN binding protein 9 | AB015640_at Blast Report | Hs.306242 | 0 | 95 | 100 | Mm.148781 | 0 | 97.95455 | 100 |
| WAN013HVD_at (SEQ ID NO:1801) | 1.569 | RHOA | Ras homolog gene family, member A | WAN013HVD_at Blast Report | Hs.247077 | 0 | 95.8042 | 100 | Mm.757 | 0 | 96.32867 | 100 |
| WAN008DRK_at (SEQ ID NO:1802) | 1.738 | RFC4 | Replication factor C (activator 1) 4, 37kDa | WAN008DRK_at Blast Report | Hs.591322 | 0.000005 | 86.66667 | 11.07011 | Mm.386835 | 6E-29 | 90.37037 | 24.90775 |
| WAN008DWV_x_at (SEQ ID NO:1803) | 1.717 | Rmnd5a | Required for meiotic nuclear division 5 homolog A (S cerevisiae) | WAN008DWV_x_at Blast Report | Hs.75277 | 3E-59 | 96.83544 | 44.2577 | Mm.28474 | 1E-133 | 95.2381 | 100 |
| WAN013I1O_at (SEQ ID NO:1804) | 1.543 | RNH1 | Ribonuclease/angiogenin inhibitor 1 | WAN013I10_at Blast Report | Hs.530687 | 1E-18 | 83.45324 | 27.8 | Mm.279485 | 1E-91 | 88.23529 | 61.2 |
| WAN008DC4_at (SEQ ID NO:1805) | 2743 | RpI14 | Ribosomal protein L14 | WAN008DC4_at Blast Report | Hs.446522 | 4E-99 | 88.52941 | 68.41046 | Mm289810 | 1E-154 | 90.58296 | 89.73843 |
| WAN013I36_f_at (SEQ ID NO:1806) | 1 889 | RPSA | Ribosomal protein SA | WAN013I36_f_at Blast Report | Hs.449909 | 1E-14 | 89.41176 | 85 85859 | Mm 4071 | 3E-20 | 90 81633 | 98.9899 |
| WAN008CKA_at (SEQ ID NO:1807) | 1.518 | 1110002B05Rik | RIKEN cDNA 1110002B05 gene | WAN008CKA_at Blast Report | #N/A | 1E-112 | 87.2 | 100 | Mm 292775 | 1E-147 | 90 | 100 |
| WAN008EQG_at (SEQ ID NO:1808) | 1.678 | SAPS3 | SAPS domain family, member 3 | WAN008EQG_at Blast Report | Hs 503022 | 2E-57 | 87.26708 | 87.02703 | Mm.284686 | 1E-145 | 95 40541 | 100 |
| AF004831_at (SEQ ID NO:1536) | 1.999 | SPTLC1 | Senne palmitoyltransferase, long chain base subunit 1 | AF004831_at Blast Report | Hs 90458 | 1E-18 | 88.88889 | 6.766917 | Mm 240336 | 5E-84 | 89.44099 | 24.21053 |
| WAN008EE3_at (SEQ ID NO:1809) | 1 521 | SARS | Seryl-tRNA synthetase | WAN008EE3_at Blast Report | Hs 531176 | 1E-116 | 9080119 | 100 | Mm 28688 | 1E-136 | 9317507 | 100 |
| WAN0088TG_at (SEQ ID NO: 1540) | 1.997 | SRP72 | Signal recognition particle 72kDa | WAN0088TG_at Blast Report | Hs237825 | 1E-58 | 89.0411 | 50.81206 | Mm 296976 | 1E-119 | 92.40122 | 76.33411 |
| S79122_x_at (SEQ ID NO:1810) | 2.103 | SON | SON DNA binding protein | S79122_x_at Blast Report | Hs 517262 | 4E-47 | 98.44961 | 75.88235 | Mm.46401 | 2E-32 | 87.64706 | 100 |
| WAN008EFY_at (SEQ ID NO:1811) | 1.597 | SPG21 | Spastic paraplegia 21 (autosomal recessive, Mast syndrome) | WAN008EFY_at Blast Report | Hs 242458 | 1E-128 | 90.45093 | 71.94656 | Mm.272475 | 1E-136 | 91.44385 | 71.37405 |
| WAN008CSS_at (SEQ ID NO.1812) | 1.546 | SART1 | Squamous cell carcinoma antigen recognised by T cells | WAND08CSS_at Blast Report | Hs 502883 | 0 | 90.3169 | 98.2699 | Mm.34562 | 0 | 94.80969 | 100 |
| AF039202_at (SEQ ID NO:1813) | 1.603 | STIP1 | Stress-induced-phosphoprotein 1 (Hsp70/Hsp90-organizing protein) | AF039202_at Blast Report | Hs 337295 | 0 | 88.80208 | 61.39089 | Mm.258633 | 0 | 93.61979 | 61.39089 |
| WAN013I25_at (SEQ ID NO:1814) | 1.88 | Stx4a | Syntaxin 4A (placental) | WAN013I25_at Blast Report | #N/A | 3E-81 | 88.88525 | 56.27306 | Mm.24867 | 1E-165 | 88.17006 | 99.8155 |
| WAN013HX6_at (SEQ ID NO:1815) | 1 998 | TAX1BP1 | Tax1 (human T-cell leukemia virus type I) binding protein 1 | WAN013HX6_at Blast Report | Hs 34576 | 1E-111 | 8672986 | 4149459 | Mm 431979 | 1E-124 | 89.42065 | 39 03638 |
| WAN0088ST_at (SEQ ID NO:1816) | 1.521 | TBC1D15 | TBC1 domain family, member 15 | WAN0088ST_at Blast Report | Hs.284630 | 5E-18 | 84.31373 | 52.44216 | Mm.22252 | 3E-67 | 90.7563 | 61.18252 |
| WAN008D32_at (SEQ ID NO:1817) | 1.687 | TSPAN6 | Tetraspanin 6 | WAN008D32_at Blast Report | Hs 43233 | 4E-12 | 100 | 9.87013 | Mm.46701 | 1E-57 | 88.70968 | 64.41558 |
| WAN013I98_at (SEQ ID NO:1818) | 1.517 | TST | Thiosulfate sulfurtransferase (rhodanese) | WAN013I98_at Blast Report | Hs.474783 | 0 | 84.09332 | 87.23404 | Mm.15312 | 0 | 90.04329 | 85 47641 |
| WAN013HUQ_x_at (SEQ ID NO: 1819) | 1.674 | THOC5 | THO complex 5 | WAN013HUQ_x_at Blast Report | Hs.75361 | 3E-48 | 90.50633 | 96.34146 | Mm.28969 | 7E-66 | 94.47853 | 99 39024 |
| L00365_at (SEQ ID NO:1820) | 1.673 | TK1 | Thymidine kinase 1, soluble | L00365_at Blast Report | Hs.515122 | 6E-24 | 90.32258 | 70.45455 | Mm.2661 | 4E-48 | 94.4 | 94.69697 |
| WAN008EKQ_at (SEQ ID NO:1821) | 1.528 | TIAL1 | TIA1 cytotoxic granule-associated RNA binding protein-like 1 | WAN008EKQ_at Blast Report | Hs.501203 | 1E-127 | 92.2043 | 100 | Mm 242072 | 1E-164 | 95.16129 | 100 |
| WAN013I4X_at (SEQ ID NO:1742) | 1.957 | Timp2 | Tissue inhibitor of metalloproteinase 2 | WAN013I4X_at Blast Report | Hs.633514 | 0 | 0 | 0 | Mm.206505 | 0 | 94.2623 | 100 |
| WAN013IA5_at (SEQ ID NO:1822) | 1.625 | TOP2B | Topoisomerase (DNA) II beta 180kDa | WAN013IA5_at Blast Report | Hs.475733 | 0 | 94.43414 | 100 | Mm.130362 | 0 | 98.7013 | 100 |
| WAN0088TW_at (SEQ ID NO:1745) | 1.537 | TCEB3 | Transcription elongation factor B (SIII), polypeptide 3 (110kDa, elongin A) | WAN0088TW_at Blast Report | Hs.584806 | 1E-173 | 89.36567 | 98.52941 | Mm.27663 | 0 | 93.09701 | 98.52941 |
| WAN008DE8_at (SEQ ID NO:1823) | 1.553 | TIMM23 | Translocase of inner mitochondrial membrane 23 homolog (yeast) | WAN008DE8_at Blast Report | Hs.524308 | 1E-106 | 89.75904 | 100 | Mm.303703 | 1E-127 | 92.66055 | 98.49398 |
| WAN008CSZ_at (SEQ ID NO:1824) | 1.896 | Tloc1 | Translocation protein 1 | WAN008CSZ_at Blast Report | Hs.592561 | 4E-22 | 86.41975 | 43.31551 | Mm.26017 | 1E-125 | 93.18182 | 62.7451 |
| WAN008EFO_at (SEQ ID NO:1825) | 1.59 | TM9SF2 | Transmembrane 9 superfamily member 2 | WAN008EFO_at Blast Report | Hs.130413 | 1E-153 | 90.94203 | 100 | Mm 275191 | 0 | 95.47101 | 100 |
| WAN008D2L_at (SEQ ID NO:1747) | 1.6 | TMEM50A | Transmembrane protein 50A | WAN008D2L_at Blast Report | Hs.523054 | 2E-76 | 85.79882 | 80.09479 | Mm 88349 | 1 E-63 | 86.92308 | 61.61137 |
| U29167_at (SEQ ID NO:1672) | 1.592 | TPM2 | Tropomyosin 2 (beta) | U29167_at Blast Report | Hs.300772 | 0 | 92.83111 | 88 78101 | Mm 646 | 0 | 94.74313 | 90.29126 |
| WAN013IAB_x_at (SEQ ID NO:1496) | 1.578 | TP53 | Tumor protein p53 (Li-Fraumeni syndrome) | WAN013IAB_x_at Blast Report | Hs.408312 | 1E-150 | 82.44767 | 4885917 | #N/A | 1E-133 | 81.32045 | 48.85917 |
| WAN008EUV_x_at (SEQ ID NO:1826) | 2.038 | Tpt1 | Tumor protein, translationally-controlled 1 | WAN008EUV_x_at Blast Report | Hs.374596 | 7E-19 | 88.76404 | 3617886 | Mm 297482 | 1E-65 | 88.21138 | 100 |
| WAN008E9O_at (SEQ ID NO:1827) | 217 | TSG101 | Tumor susceptibility gene 101 | WAN008E9O_at Blast Report | Hs.523512 | 1E-49 | 86.78414 | 43.074 | Mm 241334 | 1E-97 | 95 55556 | 42 6945 |
| WAN008DK3_at (SEQ ID NO:1828) | 1.574 | YWHAQ | Tyrosine 3-monooxygenase/tryptophan 5-monooxygenase activation protein, theta polypeptide | WAN008DK3_at Blast Report | Hs 74405 | 1E-98 | 89.41799 | 100 | Mm.289630 | 1E-151 | 94.17989 | 100 |
| WAN008DK1_at | 1.778 | UQCRC1 | Ubiquinol-cytochrome c | WAN008DK1_at Blast | Hs 119251 | 3E-69 | 85.66879 | 64 87603 | Mm.335460 | 1E-110 | 91.0828 | 64.87603 |

| (SEQ ID NO:1829) | | | reductase core protein I | Report | | | | | | | | |
|---|---|---|---|---|---|---|---|---|---|---|---|---|
| AF004368_at (SEQ ID NO:1749) | 1.703 | UGP2 | UDP-glucose pyrophosphorylase 2 | AF004368_at Blast Report | Hs.516217 | 1E-116 | 91.77215 | 53.92491 | Mm.28877 | 1E-147 | 90.86538 | 70.98976 |
| WAN008DB7G_at (SEQ ID NO:1750) | 1.595 | VAMP3 | Vesicle-associated membrane protein 3 (cellubrevin) | WAN008D7G_at Blast Report | Hs.66708 | 0.0000004 | 97.56098 | 7.400722 | Mm273930 | 9E-19 | 91.86047 | 15.52347 |
| WAND13I7T_at (SEQ ID NO:1830) | 1.691 | HRAS | V-Ha-ras Harvey rat sarcoma viral oncogene homolog | WAN013I7T_at Blast Report | Hs.37003 | 2E-36 | 89.92806 | 24.5583 | #N/A | 1E-171 | 92.13251 | 85.33569 |
| X63416_at (SEQ ID NO:1831) | 1.675 | MOS | V-mos Moloney marine sarcoma viral oncogene homolog | X63416_at Blast Report | Hs.533432 | 4E-28 | 862069 | 27.30697 | Mm.317339 | 1E-157 | 100 | 52.9 1902 |
| S74024at (SEO ID NO:1832) | 1.772 | XPA | Xeroderma pigmentosum, complementation group A | S74024_at Blast Report | Hs.591907 | 4E-54 | 87.73585 | 96 36364 | Mm.247036 | 6E-64 | 89.09091 | 100 |
| WAND008906_at (SEQ ID NO:1833) | 1.532 | Zfp259 | Zinc finger protein 259 | WAN008906_at Blast Report | #N/A | 1E-162 | 901354 | 94 86239 | Mm.17519 | 0 | 92.84404 | 100 100 |
| WAN008CO7_at (SEQ ID NO:1834) | 1.701 | Zfp222 | Zinc finger protein 622 | WAN008CO7_at Blast Report | #N/A | 1E-166 | 89.8 | 88 33922 | Mm.29145 | 0 | 91.56194 | 98.40989 |
| | | | | | | | | | | | | |

| **UP (Originally 78)** | | | | | | | | | | | | |
|---|---|---|---|---|---|---|---|---|---|---|---|---|
| **Qualifier List** | **Fold Change** | **Symbol** | **Title** | **Human Unigene ID** | **eValue** | **%ID** | **%QC** | **Mouse Unigene ID** | **evalue** | **%ID** | **%QC** | |
| L00178_at (SEQ ID NO:1 507) | 1.828153565 | Hmgcr | 3-hydroxy-3methylglutaryl-Coenzyme A reductase | Hs.643495 | 3E-47 | 90.0621118 | 42.81915 | Mm.316652 | 1E-57 | 93.037975 | 42.02128 | |
| L00327_x_at (SEQ ID NO: 1835) | 2475247525 | HMGCS1 | 3-hydroxy-3-methylglutaryl-Coenzyme A synthase 1 (soluble) | Hs.397729 | 8E-51 | 95 23809524 | 97.67442 | Mm.61526 | 2E-41 | 93220339 | 91.47287 | |
| WAN013HXI_at (SEQ ID NO:1836) | 1.538461538 | ACAT2 | Acetyl-Coenzyme A acetyltransferase 2 (acetoacetyl Coenzyme A thiolase) | Hs.571037 | IE-122 | 83.06010929 | 98.74101 | Mm.229342 | 0 | 88321168 | 98 56115 | |
| AF022944_at (SEQ ID NO 1837) | 1 834862385 | NA | AF022944 Cncetulus griseus DNA damage inducible done similar to eukaryotic initiation factor 5 (elF-5) cDNA, partial sequence | #N/A | 1E-34 | 93.2038835 | 59.53757 | #N/A | 2E-44 | 97 087379 | 59.53757 | |
| WAN013HTZ_at (SEQ ID NO:1838) | 4.464285714 | BCL2L1 | BCL2-like 1 | Hs.516966 | 0 | 93.20754717 | 99.43715 | Mm.238213 | 0 | 93.962264 | 99.43715 | |
| WAN008CI5_at (SEQ ID NO:1839) | 1 550387597 | CDC20 | CDC20 cell division cycle 20 homolog (S cerevisiae) | Hs 524947 | 1E-105 | 89 21282799 | 68.6 | Mm 289747 | 1E-142 | 93.586006 | 68.6 | |
| M29895_at (SEQ ID NO:1840) | 1.508295626 | CHI3L1 | Chitinase 3-like 1 (cartilage glycoprotein-39) | Hs.382202 | 5E-26 | 86.82170543 | 54.66102 | Mm.38274 | 9E-17 | 93.442623 | 25.84746 | |
| X81405_at (SEQ ID NO:1682) | 1.552795031 | EN2 | Engrailed homolog 2 | Hs.134989 | 5E-69 | 92.59259259 | 81.11588 | Mm.4298 | 7E-30 | 92.631579 | 40.77253 | |
| D11452_at (SEQ ID NO:1841) | 1.503759398 | GIP | Gastnc inhibitory polypeptide | Hs.1454 | 3E-35 | 94.84536082 | 17.41472 | Mm.248452 | 4E-95 | 87.257618 | 64.81149 | |
| WAN013I0X_at (SEQ ID NO. 1581) | 2008032129 | GSS | Glulathione synthetase | Hs.82327 | 1E-98 | 90.78498294 | 56.13027 | Mm.252316 | 1E-129 | 95.189003 | 55.74713 | |
| WAN008F1C_x_at (SEQ ID NO 1842) | 1.706484642 | GSK3A | Glycogen synthase kinase 3 aloha | Hs.466828 | 2E-16 | 81.9209D395 | 58.22368 | Mm.294664 | 6E-34 | 84.11215 | 70.39474 | |
| WAN0088K7_x_at (SEQ ID NO 1506) | 3.558718861 | Hspa5 | Heat shock 70kD protein 5 (glucose-regulated protein) | Hs.605502 | 0 | 0 | 0 | Mm.330160 | 0.000008 | 100 | 6.923077 | |
| WAN013HWO_x_at (SEQ ID NO 1529) | 2141327623 | HSP90B1 | Heat shock protein 90kDa beta (Grp94), member 1 | Hs.192374 | 2E-48 | 84.68085106 | 94 | Mm 87773 | 2E-66 | 87 804878 | 98.4 | |
| L38710_ x_at (SEQ ID NO 1843) | 1.697792869 | HSD3B1 | Hydroxy-delta-5-steroid dehydrogenase, 3 beta- and steroid delta-isomerase 1 | Hs.364941 | 2E-41 | 80.44554455 | 29.51059 | Mm.140811 | 1E-134 | 90.024331 | 30.02191 | |
| WAN013I9D_at (SED ID NO 1525) | 1.582278481 | HYOU1 | Hypoxia up-regulated 1 | Hs.277704 | 4E-72 | 85.49848943 | 26.4166 | Mm. 116721 | 1E-122 | 92.236025 | 25.69832 | |
| D89285_at (SEQ ID NO:1844) | 1.677852349 | ITIH1 | Inter-alpha (globulin) inhibitor H1 | Hs.420257 | 2E-80 | 82.95218295 | 80.97643 | Mm.3227 | 1E-127 | 87.366167 | 78.61953 | |
| WAN0088SX_x_at (SEQ ID NO:1845) | 1 76366843 | MGEA5 | Meningioma expressed antigen 5 (hyaluronidase) | Hs.500842 | 0.00006 | 84.93150685 | 67.59259 | Mm.122725 | 9E-15 | 90.410959 | 67.59259 | |
| AB028638_at (SEQ ID NO:1846) | 1.680672269 | PDGFB | Platelet-derived growth factor beta polypeptide (simian sarcoma viral (v-sis) oncogene homolog) | Hs.1976 | 4E-68 | 83.68421053 | 84.63252 | Mm.144089 | 1E-169 | 91.759465 | 100 | |
| WAN008CWW_at (SEQ ID NO:1690) | 1.703577513 | PHTF2 | Putative homeodomain transcription factor 2 | Hs.203965 | 1E-12 | 86.20689655 | 21.75 | Mm.86410 | 1E-17 | 86.868687 | 24.75 | |
| WAN008EIS_at (SEQ ID NO 1847] | 1.782531194 | RORB | RAR-related orphan receptor B | Hs.494178 | 0 | 92.87054409 | 98.15838 | Mm.234641 | 0 | 94.814815 | 99.44751 | |
| WAN008DXF_x_at (SEQ ID NO:1848) | 1.623376623 | SENP5 | SUMO1/sentnn specific peptidase 5 | Hs.240770 | 1E-33 | 97.5308642 | 96.42857 | Mm.152890 | 9E-36 | 97.619048 | 100 | |
| WAN008D13_at (SEQ ID NO:1849) | 1.642036125 | TXNRD1 | Thioredoxin reductase 1 | Hs.567352 | 2E·91 | 88.74598071 | 64.2562 | Mm.210155 | 1E-118 | 90.168539 | 73.55372 | |
| AF022945-rc_f_at (SEQ ID NO:1666) | 2 577319588 | Thbd | Thrombomodulin | Hs.2030 | 0 | 0 | 0 | Mm.24096 | 1E-13 | 89.552239 | 65.04854 | |
| L22614_at (SEQ ID NO:1850) | 1.798561151 | THBS1 | Thrombospondin 1 | Hs.1 64226 | 3E-62 | 88.80407125 | 29.26284 | Mm.4159 | 1E-129 | 91.759465 | 33.43261 | |
| L19142_f_at SEQ ID NO 1851) | 2.222222222 | TFRC | Transferrin receptor (p90, CD71) | Hs 529618 | 1E-106 | 87 12871287 | 70 26087 | Mm 28683 | 0 | 91474245 | 97 91304 | |
| AY012002_at (SEQ ID NO:1852) | 1.703577513 | TYR | Tyrosinase (oculocutaneous albinism IA) | Hs.503555 | 1E-83 | 85.34031414 | 92.27053 | Mm.238127 | 1E-153 | 91.545894 | 100 | |
| WAN0088P2_at (SEQ ID NO:1853) | 1.519756839 | UAP1 | UDP-N-acteylglucosamme pyrophosphorylase 1 | Hs 492859 | 1E-130 | 86 73267327 | 91.98543 | Mm.27969 | 1E-177 | 90 018832 | 96 72131 | |
| AF271265_at (SEQ ID NO 1854) | 162601626 | UCP3 | Uncoupling protein 3 (mitochondrial, proton camer) | Hs.101337 | 3E-69 | 87.22627737 | 47 32297 | Mm.6254 | 1E.118 | 95.131086 | 46.11399 | |
| WAN008ERE_x_at (SEQ ID NO:1855) | 1.788908766 | WDR67 | WD repeat domain 67 | Hs 492716 | 9E-36 | 86.12716763 | 98.29545 | Mm.390835 | 2E-57 | 91.32948 | 98.29545 | |

**Table 19: late comparison**

| Down (Originally 26) | | | | | | | | | | | |
|---|---|---|---|---|---|---|---|---|---|---|---|
| Qualifier List (SEQ ID NO:) | Fold Change | Symbol | Title | Human Unigene ID | eValue | %ID | %QC | Mouse Unigene ID | evalue | %ID | %QC |
| WAN008EB0_at (SEQ ID NO.1699) | 1.656 | ACOT7 | Acyl-CoA thioesterase 7 | Hs.125137 | 1E-49 | 88.64865 | 50.40872 | Mm.296191 | 3E-70 | 93.04813 | 50.95368 |
| BI431005_x_at (SEQ ID NO:1856) | 1.513 | CCDC67 | Coiled-coil domain containing 67 | Hs.436625 | 2E-22 | 8251366 | 89.26829 | Mm.32237 | 2E-60 | 90.20619 | 94.63415 |
| WAN0088O9_at (SEQ ID NO:1777) | 1.661 | Itgb1 | Integrin beta 1 (fibronectin receptor beta) | Hs.295626 | 1E-13 | 9032258 | 16.48936 | Mm.263396 | 4E-55 | 90.49774 | 39.1844 |
| M99691_at (SEQ ID NO:1857) | 1713 | NA | M99691 Hamster retroviral sequence mRNA. | #N/A | 0 | 0 | 0 | #N/A | 0 | 0 | 0 |
| J00061_at (SEQ ID NO.1784) | 1.682 | MT1 | metallothionein I | #N/A | 3E-46 | 87.70053 | 66.31206 | Mm.192991 | 2E-64 | 91.89189 | 65.60284 |
| AY029611_f_at (SEQ ID NO:1858) | 1.569 | OPN1 SW | Opsin 1 (cone pigments), short-wave-sensitive (color blindness, tritan) | Hs.592258 | 0.00000005 | 83.72093 | 91.48936 | Mm.56987 | 5E-25 | 90.42553 | 100 |
| U21937_at (SEQ ID NO:1859) | 1.723 | Kcnj6 | Potassium inwardly-rectifying channel, subfamily J, member 6 | Hs.50927 | 0.000001 | 84.93151 | 13.27273 | Mm.328720 | 2E-19 | 86 36364 | 20 |
| WAN0088KG_at (SEQ ID NO:1539) | 2.203 | PPGB | Protective protein for beta-galactosidase (galactosialidosis) | Hs.517076 | 1E-115 | 87.5895 | 72.86957 | Mm.359633 | 1E-149 | 90.93079 | 72.86957 |
| WAN008DC4_at (SEQ ID NO:1805) | 2.003 | Rpl14 | Ribosomal protein L14 | Hs.446522 | 4E-99 | 8852941 | 68.41046 | Mm.289810 | 1E-154 | 90.58296 | 89.73843 |
| WAN013136_f_at (SEQ ID NO:1806) | 1619 | RPSA | Ribosomal protein SA | Hs.449909 | 1E-14 | 89 41176 | 85.85859 | Mm.4071 | 3E-20 | 90.81633 | 98.9899 |
| M87540_at (SEQ ID NO:1860) | 1589 | Scn1a | Sodium channel, voltage-gated, type I, alpha | Hs.22654 | 0 | 9489051 | 100 | Mm.365737 | 2E-86 | 85.67708 | 93.43066 |
| U66490_at (SEQ ID NO:1861) | 1.574 | STAR | Steroidogenic acute regulator | Hs.521535 | 6E-88 | 87.27811 | 26.02002 | Mm.293314 | 1E-120 | 90.9621 | 26.40493 |
| WAN013I4X_at (SEQ ID NO: 1742) | 1.55 | Timp2 | Tissue inhibitor of metalloproteinase 2 | Hs.633514 | 0 | 0 | 0 | Mm 206505 | 0 | 94.2623 | 100 |
| WAN008DE8_at (SEQ ID NO:1823) | 1.552 | TIMM23 | Translocase of inner mitochondrial membrane 23 homolog (yeast) | Hs.524308 | 1E-106 | 89 75904 | 100 | Mm.303703 | 1E-127 | 92.66055 | 98.49398 |
| WAN013I7T_at (SEQ ID NO.1830) | 1.524 | HRAS | V-Ha-ras Harvey rat sarcoma viral oncogene homolog | Hs.37003 | 2E-36 | 89 92806 | 24.5583 | #N/A | 1E-171 | 92.13251 | 85.33569 |
| | | | | | | | | | | | |
| | | | | | | | | | | | |

| UP (Originally 170) (SEQ ID NO:) | | | | | | | | | | | |
|---|---|---|---|---|---|---|---|---|---|---|---|
| Qualifier List (SEQ ID NO:) | Fold Change | Symbol | Title | Human Unigene ID | evalue | %ID | %QC | Mouse Unigene ID | eValue | %ID | %QC |
| L00169_at | 2.832861 | Hmgcr | 3-hydroxy-3-methylglutaryl- | Hs.643495 | 2E-19 | 89.53488 | 33.99209 | Mm.316652 | 7E-27 | 9487179 | 30.83004 |

| (SEQ ID NO:1508) | | | Coenzyme A reductase | | | | | | | | |
|---|---|---|---|---|---|---|---|---|---|---|---|
| L00332_at (SEQ ID NO 1862) | 2.849003 | HMGCS1 | 3-hydroxy-3-methylglutaryl-Coenzyme A synthase 1 (soluble) | Hs.397729 | 3E-41 | 90.57971 | 95.83333 | Mm.61526 | 1E-42 | 92.1875 | 88.88889 |
| WAN008BQY_at (SEQ ID NO:1863) | 1.960784 | PFKFB4 | 6-phosphofructo-2-kinase/fructose-2, 6-biphosphatase 4 | Hs.476217 | 8E-87 | 89.32384 | 98.59649 | Mm.132391 | 1E-113 | 92.98246 | 100 |
| WAN013HXI_at (SEQ ID NO:1836) | 1 724138 | ACAT2 | Acetyl-Coenzyme A acetyltransferase 2 (acetoacetyl Coenzyme A thiolase) | Hs 571037 | 1E-122 | 83.06011 | 98.74101 | Mm 229342 | 0 | 88.32117 | 100 98 56115 |
| AF022944_at (SEQ ID NO:1837) | 1.642036 | NA | AF022944 Cricetulus griseus DNA damage inducible clone similar to eukaryotic initiation factor 5 (elF-5) cDNA, partial sequence. | #N/A | 1E-34 | 93.20388 | 59.53757 | #N/A | 2E-44 | 97.08738 | 59.53757 |
| WAN008DJ2_at (SEQ ID NO:1864) | 2061856 | Anapc1 | Anaphase promoting complex subunit 1 | Hs.436527 | 1E-152 | 89.30818 | 96.95122 | Mm.277408 | 0 | 94.96855 | 96.95122 |
| WAN008DNR_at (SEQ ID NO:1865) | 1.694915 | ANKRD32 | Ankynn repeat domain 32 | Hs.556673 | 1E-67 | 87.64479 | 64.75 | Mm.209730 | 1E-117 | 89.43089 | 92.25 |
| WAN008EKW_at (SEQ ID NO:1866) | 1776199 | RERE | Arginine-glutamic acid dipeptide (RE) repeats | Hs 463041 | 1E-147 | 9025845 | 97 85992 | Mm 291274 | 0 | 93 77432 | 100 |
| WAN008BSS_at (SEQ ID NO:1867) | 1.636661 | ATAD2 | ATPase family, AAA domain containing 2 | Hs.370834 | 7E-51 | 86.74699 | 61.63366 | Mm.221758 | 8E-71 | 91.34615 | 51.48515 |
| WAN013I05_at (SEQ ID NO:1646) | 1.577287 | ABCB6 | ATP-binding cassette, sub-family B (MDR/TAP), member 6 | Hs.107911 | 1E-154 | 87.15084 | 100 | Mm.28663 | 0 | 91.80633 | 100 |
| WAN008DGC_at (SEQ ID NO:1868) | 1.623377 | BAIAP2L1 | BAI1-associated protein 2-like 1 | Hs.584939 | 2E-34 | 83.69099 | 100 | Mm.18814 | 3E-75 | 90.98712 | 100 |
| WAN013HTZ_at (SEQ ID NO: 1838) | 6.17284 | BCL2L1 | BCL2-like 1 | Hs.516966 | 0 | 93.20755 | 99.43715 | Mm.238213 | 0 | 93.96226 | 99.43715 |
| WAN00BCYH_at (SEQ ID NO:1869) | 1.639344 | CALD1 | Caldesmon 1 | Hs.490203 | 1E-152 | 89.07216 | 85.53792 | Mm.308134 | 0 | 92.76896 | 100 |
| WAN008BSH_at (SEQ ID NO:1558) | 1.529052 | CAT | Catalase | Hs.502302 | 6E-16 | 89.28571 | 38.70968 | Mm.4215 | 3E-41 | 89 24731 | 85.71429 |
| WAN008CI5_at (SEQ ID NO:839) | 1.858736 | CDC20 | CDC20 cell division cycle 20 homolog (S cerevisiae) | Hs.524947 | 1E-105 | 89.21283 | 68.6 | Mm.289747 | 1E-142 | 93.58601 | 68.6 |
| WAN013I2T_at (SEQ ID NO:1652) | 1.531394 | CBX5 | Chromobox homolog 5 (HP1 alpha homolog. Drosophila) | Hs.632724 | 1E-142 | 91.86352 | 72.02268 | Mm.262059 | 1E-168 | 94.75066 | 72.02268 |
| WAN008D17_at (SEQ ID NO: 1870) | 1.531394 | COPA | Coatomer protein complex, subunit alpha | Hs 162121 | 0 | 92.98597 | 96.14644 | Mm.30041 | 0 | 94 32485 | 98.45857 |
| WAN013HWY_at (SEQ ID NO:1710) | 3 968254 | CCDC80 | Coiled-coil domain containinq 80 | Hs 477128 | 6E-92 | 86.55914 | 76.07362 | Mm.181074 | 1E-171 | 90 57377 | 99.7955 |
| WAN008CI3_at (SEQ ID NO:1680) | 155521 | CCNJ | Cyclin J | Hs.596479 | 1E-145 | 93.71429 | 77.60532 | Mm.309 | 1E-105 | 88 85714 | 77.60532 |
| U66494_at (SEQ ID NO:1871) | 1.552795 | CYP17A1 | Cytochrome P450, family 17. subfamily A, polypeptide 1 | Hs.438016 | 4E-47 | 81.94842 | 62.21034 | Mm.1262 | 1E-70 | 82 58929 | 79.8574 |
| WAN00893H_at SEQ ID NO:1872) | 1.594896 | DDX23 | DEAD (Asp-Glu-Ala-Asp) box polypeptide 23 | Hs.130098 | 1E-163 | 90.41667 | 97.36308 | Mm.45725 | 1E-173 | 91.09731 | 97.9716 |
| WAN013HUL_at (SEQ ID NO.1873) | 1.605136 | DDX3X | DEAD (Asp-Glu-Ala-Asp) box polypeptide 3, X-linked | Hs.380774 | 1E-78 | 93.75 | 42.27642 | Mm289662 | 2E-88 | 91.76955 | 49.39024 |
| WAN0088WF_at (SEQ ID NO:1874) | 1.814882 | DHX36 | DEAH (Asp-Glu-Ala-His) box polypeptide 36 | Hs.446270 | 2E-97 | 92.07547 | 55.67227 | Mm.224233 | 1E-113 | 94.40299 | 56.30252 |
| WAN0088OR_at (SEQ ID NO.1875) | 1.74216 | EIF5B | Eukaryotic translation initiation factor 5B | Hs.158688 | 2E-61 | 91.17647 | 41.04628 | Mm.260943 | 2E-71 | 89.30041 | 48.89336 |
| WAN008DNP_at (SEQ ID NO:1876) | 1 776199 | XPO1 | Exportin 1 (CRM1 homolog, yeast) | Hs.370770 | 0 | 91.08527 | 98.47328 | Mm.217547 | 0 | 94.84733 | 100 |
| WAN008D0K_at (SEQ ID NO:1877) | 1.538462 | AA408296 | Expressed sequence AA408296 | #N/A | 1E-110 | 86.77494 | 96.85393 | Mm.173758 | 1E-143 | 89.86486 | 99.77528 |
| WAN008CPJ_at (SEQ ID NO:1878) | 1 538462 | Fdft1 | Farnesyl diphosphate farnesyl transferase 1 | Hs.593928 | 1E-123 | 85.09874 | 99.64222 | Mm.425927 | 0 | 0 | 0 |
| WAN013I1W_at (SEQ ID NO:1879) | 1.52207 | FDPS | Farnesyl diphosphate synthase (farnesyl pyrophosphate synthetase, dimethylallytranstransferase, geranyltranstransferase) | Hs.335918 | 1E-146 | 86.11632 | 100 | Mm.39472 | 0 | 90.78947 | 99.81238 |
| WAN008CQY_at (SEQ ID NO 1880) | 1 589825 | FBXL11 | F-box and leucine-rich repeat protein 11 | Hs.124147 | 0 | 93.30544 | 88.3549 | Mm.31941 | 0 | 97.57914 | 99.26063 |
| WAN008CQH_at (SEQ ID NO:1881) | 1.923077 | FTSJ3 | FtsJ homolog 3 (E. coli) | Hs.463785 | 1E-82 | 88.44884 | 84.63687 | Mm.29795 | 1E.110 | 91.69329 | 87.43017 |
| WAN0088Y2_at (SEQ ID NO:1882) | 1.529052 | Ggnbp2 | Gametogenetin binding protein 2 | #N/A | 0 | 90 99265 | 100 | Mm.356653 | 0 | 93.56618 | 100 |
| WAN013I0X_at (SEQ ID NO:1581) | 1.831502 | GSS | Glutathione synthetase | Hs.82327 | 1E-98 | 90.78498 | 56.13027 | Mm.252316 | 1E-129 | 95.189 | 55.74713 |
| WAN008DXA_at (SEQ ID NO:1883) | 1 669449 | GPD2 | Glycerol-3-phosphate dehydrogenase 2 (mitochondrial) | Hs.512382 | 1E-111 | 86.72769 | 98.6456 | Mm.3711 | 1E-159 | 91.15646 | 99.54853 |
| AF307847 _at (SEQ ID NO:1884) | 1.776199 | GAB1 | GRB2-associated binding protein 1 | Hs.80720 | 7E-98 | 85.99034 | 76.52495 | Mm.277409 | 1E-136 | 89.57346 | 78.0037 |
| WAN008EAH_at (SEQ ID NO:1885) | 1.508296 | GTPBP4 | GTP binding protein 4 | Hs.215766 | 1E-126 | 88.42795 | 88.07692 | Mm.41800 | 0 | 95.41485 | 88.07692 |
| WAN0088K7_x_at (SEQ ID NO:1506) | 2.463054 | Hspa5 | Heat shock 70kD protein 5 (glucose-regulated protein) | Hs.605502 | 0 | 0 | 0 | Mm.330160 | 0.000008 | 100 | 6.923077 |
| WAN013HWO_x_at (SEQ ID NO:1529) | 2.10084 | HSP90B1 | Heat shock protein 90kDa beta (Grp94), member 1 | Hs.192374 | 2E-48 | 84 68085 | 94 | Mm.87773 | 2E-66 | 87 80488 | 98.4 |
| WAN008DQ1_at (SEQ ID NO:1886) | 1.6 | Herpud2 | HERPUD family member 2 | Hs.599851 | 3E-31 | 95 34884 | 26 95925 | Mm.142843 | 1E.106 | 90 28213 | 100 |
| WAN008EVU_x_at (SEQ ID NO:1887) | 1.610306 | HNRPK | Heterogeneous nuclear ribonucleoprotein K | Hs.522257 | 1E-129 | 92.9878 | 59.63636 | Mm.142872 | 1E-170 | 90.5838 | 96.54545 |
| L38709_at (SEQ ID NO:1888) | 1.706485 | HSD38 | Hydroxy-delta-5-steroid dehydrogenase, 3 beta- and steroid delta-isomerase | #N/A | 2E-41 | 82 53425 | 29.46519 | #N/A | 1E-151 | 85 82803 | 63.37033 |
| WAN013I9D_at (SEQ ID NO·1525) | 1.712329 | HYOU1 | Hypoxia up-regulated 1 | Hs.277704 | 4E-72 | 85 49849 | 26.4166 | Mm.116721 | 1E-122 | 92.23602 | 25.69832 |
| WAN008CF7_at (SEQ ID NO:1889) | 1.760563 | IVNS1ABP | Influenza virus NS1A binding protein | Hs.497183 | 0 | 9327434 | 100 | Mm.33764 | 0 | 97.34513 | 100 |
| D89285_at (SEQ ID NO. 1844) | 1 712329 | ITIH1 | Inter-alpha (globulin) inhibitor H1 | Hs 420257 | 2E-80 | 82 95218 | 80 97643 | Mm 3227 | 1E-127 | 87.36617 | 78.61953 |
| WAN008EX2_x_at (SEQ ID NO1575) | 1.650165 | Ifrd1 | Interferon-related developmental regulator 1 | Hs.7879 | 7E-39 | 90.29851 | 100 | Mm.168 | 5E-63 | 97.76119 | 100 |
| AF046210_at (SEQ ID NO:1890) | 1.706485 | IL10 | Interleukin 10 | Hs.193717 | 3E-65 | 84.14986 | 92.53333 | Mm.874 | 1E-134 | 91.37466 | 98.93333 |
| X56067_at (SEQ ID NO1891) | 1 724138 | IAPP | Islet amyloid polypeptide | Hs.46835 | 3E-17 | 83.45324 | 20 65379 | Mm 415 | 7E-91 | 88.03681 | 48.43982 |
| WAN008D55-rc_at (SEQ ID NO.1603) | 1.77305 | Lamb1 _predicted | Laminin, beta 1 (predicted) | #N/A | 1E-155 | 87.82288 | 97.48201 | #N/A | 1E-161 | 91 66667 | 77.69784 |
| WAN00895S_at (SEQ ID NO:1892) | 1.52439 | LRRC59 | Leucine nch repeat containing 59 | Hs.370927 | 0 | 89 7482 | 100 | Mm.172720 | 0 | 92.6259 | 100 |
| AF306662_at (SEQ ID NO.1729) | 2.008032 | MMP14 | Matrix metallopeptidase 14 (membrane-inserted) | Hs.2399 | 0 | 89.72603 | 99.82906 | Mm.280175 | 0 | 90.08547 | 100 |
| WAN013I96_at (SEQ ID NO:1893) | 1.533742 | MDM2 | Mdm2, transformed 3T3 cell double minute 2, p53 binding protein (mouse) | Hs.567303 | 5E-83 | 86.03896 | 51.41903 | Mm.22670 | 2E-89 | 87.10592 | 53.08848 |
| WAN008EQD_at (SEQ ID NO:1894) | 1.718213 | Mx2 | Myxovirus (influenza virus) resistance 2 | Hs.926 | 2E-29 | 80.31746 | 75 | Mm.14157 | 2E-99 | 85.95238 | 100 |
| WAN0088IR_at (SEQ ID NO:1895) | 1.615509 | NQO1 | NAD(P)H dehydrogenase, quinone 1 | Hs.406515 | 1E-42 | 87.57062 | 62.76596 | Mm.252 | 4E-87 | 90.73359 | 91.84397 |
| WAN008DWL_at (SEQ ID NO:1896) | 1.5625 | NEK2 | NIMA (never in mitosis gene a)-relaled kinase 2 | Hs.153704 | 1E-71 | 87.38739 | 58.42105 | Mm.33773 | 1E-152 | 89.13934 | 85.61404 |
| WAN013I9Q_f_at (SEQ ID NO.1897) | 1.66113 | NKX6-1 | NK6 transcription factor related, locus 1 (Drosophila) | Hs.546270 | 0.00002 | 89.3617 | 10 | Mm.193072 | 5E-26 | 95.89041 | 15.53191 |
| WAN008ECD_at (SEQ ID NO1898) | 1697793 | NARG1 | NMDA receptor regulated 1 | Hs.555985 | 0 | 94.43299 | 99.58932 | Mm.275281 | 0 | 96.70103 | 99.58932 |
| WAN008BT6_at (SEQ ID NO:1899) | 1.54321 | NVL | Nuclear VCP-like | Hs.497867 | 1E-86 | 86.31285 | 70.47244 | Mm.263464 | 1E-156 | 91.42212 | 87.20472 |
| WAN008E89_at (SEQ ID NO:1900) | 1.612903 | Nup160 | Nucleoporin 160 | Hs.645358 | 0 | 92.35182 | 98.12383 | Mm.24532 | 0 | 94 3609 | 99.81238 |
| WAN008EXG_at (SEQ ID NO:1901) | 1.547988 | NUP98 | Nucleoporin 98kDa | Hs.524750 | 1E-108 | 88.66499 | 95.43269 | Mm.215288 | 1E-145 | 91.10577 | 100 |
| WAN008EVI-rc_at (SEQ ID NO:1902) | 2.325581 | Pparbp | Peroxisome proliferator activated receptor binding protein | Hs.643754 | 5E-35 | 90.83333 | 51.06383 | Mm.12926 | 2E-76 | 90.6383 | 100 |
| WAN008DHY_at (SEQ ID NO:1903) | 1.515152 | PIK4CA | Phosphatidylinositol 4-kinase, catalytic, alpha polypeptide | Hs.529438 | 1E-172 | 88.44133 | 99.82517 | Mm.5718 | 0 | 92.43243 | 97.02797 |
| WAN008CYA_at (SEQ ID NO:1904) | 1.577287 | PIR | Pirin (iron-binding nuclear protein) | Hs.495728 | 1E-111 | 86.87783 | 76.60312 | Mm.293463 | 1E-110 | 89.9705 | 58 75217 |
| WAN013I81_at (SEQ ID NO:1905) | 1.644737 | POLD1 | Polymerase (DNA directed), delta 1, catalytic subunit 125kDa | Hs.279413 | 0 | 86.30952 | 98.31748 | Mm.16549 | 0 | 91.73372 | 100 |
| WAN008BQZ_at (SEQ ID NO:1906) | 1.757469 | KCTD7 | Potassium channel tetramerisation domain containing 7 | Hs.546627 | 2E-58 | 91.01124 | 36.47541 | Mm.55812 | IE-116 | 97.05882 | 48.77049 |
| WAN008CQA_at (SEQ ID NO:1907) | 1.642036 | PKN2 | Protein kinase N2 | Hs.440833 | 0 | 91.79389 | 100 | Mm.244236 | 0 | 93.70229 | 100 |
| WAN013HXP_at (SEQ ID NO:1908) | 1.515152 | PPP1R12A | Protein phosphatase 1, regulatory (inhibitor) subunit 12A | Hs.49582 | 0 | 93.40463 | 100 | Mm.207499 | 3E-18 | 89.61039 | 13 72549 |
| WAN008E7E_at (SEQ ID NO:1909) | 2 03666 | PTAR1 | Protein prenyltransferase alpha subunit repeat containing 1 | Hs.494100 | 1E-125 | 87.72321 | 92.37113 | Mm.32215 | 1E-169 | 91.32321 | 95.05155 |
| WAN008EXW_at (SEQ ID NO:1910) | 1.550388 | PPFIA1 | Protein tyrosine phosphatase, receptor type, f polypeptide (PTPRF), interacting protein (liprin), alpha 1 | Hs.530749 | 4E-61 | 85.56338 | 89.87342 | Mm.272809 | 1E-133 | 93.98734 | 100 |
| WAN013I0K_at (SEQ ID NO.1911) | 1.610306 | PRPF8 | PRP8 pre-mRNA processing factor 8 homolog (S. cerevisiae) | Hs.181368 | 0 | 88.03419 | 98.81757 | Mm.3757 | 0 | 91.83673 | 99.32432 |
| WAN008E6L_at (SEQ ID NO·1912) | 1.533742 | NA | RC WAN008E6L 11230B-F04 | #N/A | 0 | 0 | 0 | #N/A | 2E-13 | 88.15789 | 17.92453 |
| WAN008BR5_at (SEQ ID NO:1913) | 1 851852 | RCBTB2 | Regulator of chromosome condensation (RCC1) and BTB (POZ) domain containing protein 2 | Hs.25447 | 1E-68 | 89.42731 | 43 90716 | Mm.280068 | IE-166 | 90.17682 | 98.45261 |
| WAN008DAW_x_at (SEQ ID NO:1914) | 1.512859 | RPN1 | Ribophorin I | Hs.518244 | 1E-13 | 91.66667 | 32.43243 | Mm.188544 | 8E-26 | 91.30435 | 49.72973 |
| WAN013HVB_at (SEQ ID NO:1915) | 1.569859 | RNF10 | Ring finger protein 10 | Hs.442798 | 1E-180 | 92.22462 | 80 66202 | Mm.30051 | 1E-158 | 89 56159 | 83.44948 |
| WAN0088WO_at (SEQ ID NO:1916) | 1.976285 | RBM5 | RNA binding motif protein 5 | Hs.439480 | 1E-123 | 93.44262 | 62.37219 | Mm.259197 | 1E-134 | 90.17857 | 91.61554 |
| WAND08DX4_at (SEQ ID NO:1917) | 1.506024 | AHCYL1 | S-adenosylhomocysteine hydrolase-like 1 | Hs.485365 | 2E-38 | 93.85965 | 34.65046 | Mm.220328 | 8E-49 | 97.3913 | 34.95441 |
| WAN008CR3_at (SEQ ID NO:1918) | 1.851852 | SCYL2 | SCY1-like 2 (S.cerevisiae) | Hs.506481 | 1E-120 | 90.2507 | 99.44598 | Mm.27651 | 1E-116 | 90.05682 | 97.50693 |
| M74776_at (SEQ ID NO.1919) | 1.745201 | SERPINA6 | Serpin peptidase inhibitor, clade A (alpha-1 antiproteinase, antitrypsin), member 6 | Hs.532635 | 0.000006 | 88.46154 | 9 42029 | Mm.290079 | 8E-10 | 82.20339 | 21.37681 |
| WAN008EHX_at (SEQ ID NO·1920) | 1 594896 | Setd8 | SET domain containing (lysine methyltransferase) 8 | Hs.572262 | 1E-141 | 86.65448 | 100 | Mm.137966 | 0 | 89.76234 | 100 |
| WAN008DL0_at (SEQ ID NO:1921) | 1.66113 | SRP68 | Signal recognition particle 68kDa | Hs.514495 | 1E-170 | 89.1791 | 96.40288 | Mm.29655 | 0 | 92.85714 | 98.20144 |
| (SEQ ID NO:1922) | 1 694915 | SMYD5 | SMYD family member 5 | Hs 631882 | 1E-85 | 87.19512 | 9732938 | Mm.219946 | 1E-106 | 89.93902 | 97.32938 |
| WAN013HU2_at (SEQ ID NO:1923) | 1.515152 | SHOC2 | Soc-2 suppressor of clear homolog (C. elegans) | Hs.104315 | 1E-161 | 90.12605 | 94.82072 | Mm.228669 | 1E-123 | 86.17234 | 99.40239 |
| WAN008DZS_at (SEQ ID NO:1924) | 1.620746 | SLC4A7 | Solute carrier family 4, sodium bicarbonate cotransporter, member 7 | Hs.250072 | 0 | 90.19608 | 100 | Mm.258893 | 0 | 95.18717 | 100 |
| WAN008CMM_at (SEQ ID NO:1925) | 1 73813 | SF3B2 | Splicing factor 3b, subunit 2, 145kDa | Hs.406423 | 1E-142 | 88.4696 | 84.87544 | Mm.196532 | 0 | 91.23435 | 99.46619 |
| WAN013HVW_at (SEQ ID NO:1926) | 1 988072 | Scd1 | Stearoyl-Coenzyme A desaturase 1 | #N/A | 0 | 0 | 0 | Mm.193096 | 8E-07 | 88.67925 | 9.330986 |
| U22819_s_at (SEQ ID NO:1927) | 2 207506 | SREBF2 | Sterol regulatory element binding transcription factor 2 | Hs.443258 | 1E-118 | 89 83516 | 99.45355 | Mm.38016 | 1E-133 | 92.39437 | 96.99454 |
| WAN008E8B_at (SEQ ID NO:1928) | 1.512859 | TAOK2 | TAO kinase 2 | Hs.291623 | 0 | 91.35135 | 100 | Mm 259634 | 0 | 95.13514 | 100 |
| WAN008DMR_f_at (SEQ ID NO:1929) | 1.506024 | Taok3 | TAO kinase 3 | #N/A | 1E-141 | 90.97744 | 100 | Mm 248296 | IE-180 | 95 20202 | 99.24812 |
| WAN008DIO_at (SEQ ID NO: 1930) | 1.824818 | TIPARP | TCDD-inducible poly(ADP-ribose) polymerase | Hs.12813 | 1E-114 | 88.65979 | 69.90991 | Mm.246398 | 1E-180 | 89.8917 | 99.81982 |
| WAN013HV0_at (SEQ ID NO:1931) | 3021148 | TXNRD1 | Thioredoxin reductase 1 | Hs.567352 | 5E-94 | 80 21583 | 96 02763 | Mm 210155 | 1E-105 | 8429561 | 74 78411 |
| AF022945-rc_f_at (SEQ ID NO.1666) | 1.592357 | Thbd | Thrombomodulin | Hs.2030 | 0 | 0 | 0 | Mm.24096 | 1E-13 | 89.55224 | 65.04854 |
| WAN013I3R_at (SEQ ID NO:1932) | 2.881844 | THRAP4 | Thyroid hormone receptor associated protein 4 | Hs.462983 | 2E-63 | 89.15094 | 37.65542 | Mm.246493 | 6E-89 | 91.80328 | 43.33925 |
| WAN008DOW_at (SEQ ID NO:1933) | 2.717391 | TRIP12 | Thyroid hormone receptor interactor 12 | Hs.591633 | 3E-45 | 87.30159 | 91.74757 | Mm.209265 | 1E-74 | 92.23301 | 100 |
| L19142_f_at (SEQ ID NO:1851) | 2 624672 | TFRC | Transferrin receptor (p90, CD71) | Hs.529618 | 1E-128 | 85.6102 | 68.28358 | Mm.28683 | 0 | 90.125 | 99.50249 |
| WAN008CZR_at (SEQ ID NO:1746) | 1.512859 | TMED1 | Transmembrane emp24 protein transport domain containing 1 | Hs.515139 | 1E-139 | 87.57515 | 90.72727 | Mm.196618 | 0 | 94.89194 | 92.54545 |
| WAN008D6R_at (SEQ ID NO:1604) | 1.519757 | TMED4 | Transmembrane emp24 protein transport domain containing 4 | Hs.510745 | 1E-110 | 91.23377 | 72.30047 | Mm.254495 | 1E-145 | 92.41192 | 86.61972 |
| WAN008EVD_at (SEQ ID NO.1934) | 1.526718 | UBE3B | Ubiquitin protein ligase E3B | Hs.374067 | 1E-116 | 89.88764 | 78.76106 | Mm.28792 | 1E-133 | 91.85393 | 78.76106 |
| WAN008E4F_at (SEQ ID NO:1935) | 1.893939 | USP9X | Ubiquitin specific peptidase 9, X-linked | Hs 77578 | 1E-180 | 91.45129 | 100 | Mm.242646 | 0 | 94 63221 | 100 |
| WAN013HVL_at (SEQ ID NO:1639) | 1.579779 | UGDH | UDP-glucose dehydrogenase | Hs.572518 | 1E-165 | 90.06772 | 95.88745 | Mm.344831 | 1E-158 | 89.01099 | 98.48485 |
| WAN0088P2_at (SEQ ID NO:1853) | 1.923077 | UAP1 | UDP-N-acteylglucosamine pyrophosphorylase 1 | Hs.492859 | IE-130 | 86.73267 | 91.98543 | Mm.27969 | 1E-177 | 90.01883 | 96.72131 |
| WAN008EH0_at (SEQ ID NO 1936) | 2.024291 | YES1 | V-yes-1 Yamaguctu sarcoma viral oncogene homolog 1 | Hs.194148 | 1E-81 | 92.79279 | 100 | Mm.4558 | 1E-86 | 93.69369 | 100 |
| WAN008EC3_at (SEQ ID NO:1937) | 1 519757 | WDFY3 | WD repeat and FYVE domain containing 3 | Hs.480116 | 4E-31 | 89 34426 | 24.64646 | Mm 332522 | 1E-57 | 87 86408 | 41 61616 |
| WAN008ERE_x_at (SEQ ID NO:1855) | 2.336449 | WDR67 | WD repeat domain 67 | Hs.492716 | 9E-36 | 86.12717 | 98.29545 | Mm.390835 | 2E-57 | 91 32948 | 98.29545 |
| WAN008E97_at (SEQ ID NO:1938) | 1.569859 | WDR76 | WD repeat domain 76 | Hs.250154 | 6E-20 | 83.01887 | 52.82392 | #N/A | 1E-37 | 87.5 | 53.15615 |
| X56207_at (SEQ ID NO:1696) | 1.703578 | NA | X56207 Hamster gene for myosin heavy chain, exons 1 & 2 | #N/A | 0 | 0 | 0 | #N/A | 0.00005 | 92.10526 | 6.713781 |
| X65592_at (SEQ ID NO.1939) | 1.54321 | NA | X65592 C.griseus DNA sequence for interstitial telomere associated sequence 1 | #N/A | 0 | 0 | 0 | #N/A | 0 | 0 | 0 |
| WAN008DKJ_x_at (SEQ ID NO:1697) | 1 615509 | Zfp297b | Zinc finger protein 297B | #N/A | 1 E-27 | 91 57895 | 90 47619 | Mm.44186 | 2E-24 | 89 69072 | 92.38095 |

### Example 10. Platform analysis

Four cell lines were analyzed from the Platform Process category that exhibit a desired metabolic phenotype when cultured in fed batch culture. That is, the cell lines maintain high viability, and consume lactate and ammonia late in fed batch culture. Multiple time points were collected for each cell line grown in fed batch culture. The time points from each cell line were examined by ANOVA analysis to monitor the changes in gene expression over the course of the culture. The gene lists from each cell line were compared, and those that were in common between all 4 cell lines were identified. Exemplary nucleic acid sequences are listed in Table 20.

**Table 20: Platform Analysis**

| **Qualifier List** | **Symbol** | **Title** | **Human Unigene ID** | **evalue** | **%ID** | **%QC** | **Mouse Unigene ID** | **evalue** | **%ID** | **%QC** |
|---|---|---|---|---|---|---|---|---|---|---|
| AF022941_x_at (SEQ ID NO:1761) | Cirbp | Cold inducible RNA binding protein | Hs.634522 | 9E-27 | 93.65079 | 69.61326 | Mm. 17898 | 1E-52 | 96.26866 | 74.03315 |
| AF081141_at (SEQ ID NO:1667> | CCL2 | Chemokine (C-C motif) ligand 2 | Hs.303649 | 3E-13 | 90.625 | 13.41719 | Mm.290320 | 5E-41 | 91.04478 | 28.09224 |
| AF254572_at (SEQ ID NO:1940) | ORC1L L | Origin recognition complex, subunit 1-like (yeast) | Hs.17908 | 0 | 85.62005 | 63.06156 | Mm.294154 | 0 | 89.31624 | 97.33777 |
| l00366_x_at (SEQ ID NO:1941) | TK1 | Thvmidine kinase 1, soluble | Hs.515122 | 4E-18 | 89.87342 | 84.94624 | Mm.2661 | 1E-16 | 88.75 | 86.02151 |
| M12329_at (SEQ ID NO:1942) | NA | M12329 Chinese hamster alpha-tubulin III mRNA, complete cds. | #N/A | 0 | 93.01676 | 54.28355 | #N/A | 0 | 96.47391 | 53.75284 |
| M80243-rc_at (SEQ ID NO:1943) | BIRC5 | Baculoviral lAP repeat-containinq 5 (survivin) | Hs.514527 | 4E-38 | 92.37288 | 20.34483 | Mm.8552 | 1E-36 | 93.45794 | 18.44828 |
| U11790_at (SEQ ID NO:1944) | KIF2C | Kinesin family member 2C | Hs.69360 | 0 | 89.25714 | 66.43888 | Mm.247651 | 0 | 92.51055 | 71.98178 |
| U48852_at (SEQ lD NO:1502) | CRELD2 | Cysteine-rich with EGF-like domains 2 | Hs.211282 | 1E-109 | 81.76944 | 55.13673 | Mm.292567 | 0 | 88.79936 | 91.7221 |
| WAN0088J9_x_at (SEQ ID NO:1588) | NA | WAN0088J9 10595A-E01 | #N/A | 0 | 0 | 0 | #N/A | 0 | 0 | 0 |
| WAN0088K2_at (SEQ ID NO:1945) | DUSP16 | Dual specificity phosphatase 16 | Hs.536535 | 0.00002 | 84.44444 | 15.98579 | Mm.3994 | 4E-21 | 87.5 | 22.73535 |
| WAN00880N_at (SEQ ID NO:1946) | ATAD2 | ATPase family, AAA domain containing 2 | Hs.370834 | 2E-45 | 83.7037 | 59.08096 | Mm.221758 | 9E-71 | 87.31884 | 60.39387 |
| WAN0088Q6_at (SEQ ID NO:1590) | NA | WAN0088Q6 10595D-A09 | #N/A | 1 E-153 | 93.71585 | 63.43154 | #N/A | 0 | 94.43155 | 74.69671 |
| WAN0088S8_at (SEQ ID NO:1591) | SLC29A1 | Solute carrier family 29 (nucleoside transporters), member 1 | Hs.25450 | 3E-35 | 81.35593 | 76.12903 | Mm.29744 | 5E-97 | 86.09756 | 88.17204 |
| WAN0088T7_at (SEQ ID NO:1504) | Cyp51 | Cytochrome P450, family 51 | #N/A | 1E-132 | 86.87873 | 98.05068 | Mm.46044 | 1E-152 | 88.51485 | 98.44055 |
| WAN0088U6_at (SEQ ID NO:1947) | SPAG5 | Sperm associated antigen 5 | Hs.514033 | 1 E-1 08 | 84.43649 | 98.07018 | Mm.24250 | 1 E-153 | 87.12522 | 99.47368 |
| WAN0088X5_at (SEQ ID NO:1948) | MAD2L1 | MAD2 mitotic arrest deficient-like 1 (yeast) | Hs.591697 | 1E-111 | 87.4092 | 93.01802 | Mm.290830 | 1 E-153 | 90.95128 | 97.07207 |
| WAN008906_at (SEQ ID NO:1833) | Zfp259 | Zinc finger protein 259 | #N/A | 1E-162 | 90.1354 | 94.86239 | Mm.17519 | 0 | 92.84404 | 100 |
| WAN00893Z_at (SEQ ID NO:1949) | NA | WAN00893Z 10599B-D03 | #N/A | 8E-33 | 85.87571 | 30.62284 | #N/A | 3E-77 | 88.25503 | 51.55709 |
| WAN008BNE_x_at (SEQ ID NO:1950) | NA | WAN008BNE 11233D-H09 | #N/A | 0 | 0 | 0 | #N/A | 0 | 0 | 0 |
| WAN0088NO_at (SEQ ID NO:1951) | 2810025M15Rik | RIKEN cDNA 2810025M15 gene | #N/A | 9E-08 | 83.90805 | 15.90494 | Mm.286863 | 1E-146 | 88.09524 | 92.13894 |
| WAN008BRX_at (SEQ ID NO:1952) | RETSAT | Retinol saturase (all-trans-retinol 13,14-reductase) | Hs.440401 | 5E-74 | 83.52403 | 80.18349 | Mm.305108 | 0 | 91.37615 | 100 |
| WAN008BSS_at (SEQ ID NO:1867) | ATAD2 | ATPase family, AAA domain containing 2 | Hs.370834 | 7E-51 | 86.74699 | 61.63366 | Mm.221758 | 8E-71 | 91.34615 | 51.48515 |
| WAN008CI5_at (SEQ ID NO:1839) | CDC20 | CDC20 cell division cycle 20 homolog (S. cerevisiae) | Hs.524947 | 1E-105 | 89.21283 | 68.6 | Mm.289747 | 1E-142 | 93.58601 | 68.6 |
| WAN008CLU_at (SEQ ID NO:1953) | Emp1 | Epithelial membrane protein 1 | Hs.436298 | 0 | 0 | 0 | Mm.182785 | 3E-28 | 90.16393 | 21.66963 |
| WAN008CRT_at (SEQ ID NO:1954) | ALG14 | Asparagine-linked glycosylation 14 homolog (yeast) | Hs.408927 | 4E-47 | 88.39779 | 32.43728 | Mm.269881 | 5E-51 | 88.77005 | 33.51254 |
| WAN008CS2_at (SEQ ID NO:1694) | VKORC1L1 | Vitamin K epoxide reductase complex, subunit 1-like 1 | Hs.427232 | 1E-168 | 91.89189 | 96.73203 | Mm.288718 | 0 | 97.28507 | 96.2963 |
| WAN008CSG_at (SEQ ID NO:1955) | Mthfd1 | Methylenetetrahydrofolate dehydrogenase (NADP+ dependent), methenyltetrahydrofolate cyclohydrolase, formyltetrahydrofolate synthase | Hs.614936 | 1E-147 | 86.8705 | 100 | Mm.29584 | 0 | 90.57971 | 99.28058 |
| WAN008CT2_at (SEQ ID NO:1956) | NA | WAN008CT2 10602B-C08 | #N/A | 2E-98 | 92.39544 | 47.0483 | #N/A | 1E-112 | 94.05204 | 48.12165 |
| WAN008CTA_at (SEQ ID NO:1957) | NOLC1 | Nucleolar and coiled-body phosphoprotein 1 | Hs.523238 | 1E-101 | 89.12387 | 59.63964 | Mm.402190 | 3E-28 | 89.90826 | 19.63964 |
| WAN008CVX_at (SEQ ID NO:1958) | CDC20 | CDC20 cell division cycle 20 homolog (S. cerevisiae) | Hs.524947 | 1E-169 | 90.6639 | 85.15901 | Mm.289747 | 0 | 92.30769 | 87.27915 |
| WAN008CX4_at (SEQ ID NO:1959) | MCM5 | MCM5 minichromosome maintenance deficient 5, cell division cycle 46 (S. cerevisiae) | Hs.517582 | 1E-152 | 87.10247 | 100 | Mm.5048 | 0 | 91.48936 | 99.64664 |
| WAN008CXZ_at (SEQ ID NO:1960) | UMPS | Uridine monophosphate synthetase (orotate phosphoribosyl transferase and orotidine-5'-decarboxylase) | Hs.2057 | 1E-135 | 86.13139 | 99.63636 | Mm.13145 | 0 | 91.43898 | 99.81818 |
| WAN008CYY_at (SEQ ID NO:1961) | BUB1 B | BUB1 budding uninhibited by benzimidazoles 1 homolog beta (yeast) | Hs.631699 | 4E-34 | 81.32184 | 73.10924 | Mm.29133 | 2E-71 | 84.59384 | 75 |
| WAN008CZP_at (SEQ ID NO:1962) | NA | WAN008CZP 10604A-A08 | #N/A | 3E-29 | 90.65421 | 21.44289 | #N/A | 7E-50 | 82.69231 | 62.52505 |
| WAN008D06_at (SEQ ID NO:1963) | MCM4 | MCM4 minichromosome maintenance deficient 4 (S. cerevisiae) | Hs.460184 | 1E-159 | 87.97814 | 98.21109 | Mm.1500 | 0 | 92.98561 | 99.46333 |
| WAN008D31_at (SEQ ID NO:1964) | Lss | Lanosterol synthase | Hs.596543 | 1E-80 | 85.30184 | 68.27957 | Mm.55075 | 1E-150 | 91.52542 | 74.01434 |
| WAN008D7X_at (SEQ ID NO:1965) | NA | WAN008D7X 11164B-D06 | #N/A | 4E-16 | 88.23529 | 16.73228 | #N/A | 1E-109 | 91.23377 | 60.62992 |
| WAN008DBR_at (SEQ ID NO:1966) | LUC7L | LUC7-like (S. cerevisiae) | Hs.16803 | 0 | 93.66197 | 100 | Mm.386921 | 0 | 95.07042 | 100 |
| WAN008DGK_at (SEQ ID NO:1967) | CHAF1A | Chromatin assembly factor 1, subunit A (p150) | Hs.79018 | 1E-83 | 91.32231 | 57.89474 | Mm.391010 | 1E-101 | 90.84746 | 70.57416 |
| WAN008DK1_at (SEQ ID NO:1829) | UQCRC1 | Ubiquinol-cytochrome c reductase core protein I | Hs.119251 | 3E-69 | 85.66879 | 64.87603 | Mm.335460 | 1E-110 | 91.0828 | 64.87603 |
| WAN008DMP_at (SEQ ID NO:1968) | EWSR1 | Ewinq sarcoma breakpoint region 1 | Hs.374477 | 1E-157 | 90.52863 | 94.19087 | Mm.142822 | 0 | 92.98246 | 94.60581 |
| WAN008DO3_at (SEQ ID NO:1969) | ACIN1 | Apoptotic chromatin condensation inducer 1 | Hs.124490 | 2E-54 | 89.2562 | 62.85714 | Mm.297078 | 2E-59 | 84.94318 | 91.42857 |
| WAN008DRM_at (SEQ ID NO:1503) | EPHX1 | Epoxide hydrolase 1, microsomal (xenobiotic) | Hs.89649 | 9E-85 | 87.98701 | 60.39216 | Mm.9075 | 1E-113 | 91.22257 | 62.54902 |
| WAN008OWL_at (SEQ ID NO:1896) | NEK2 | NIMA (never in mitosis gene a)-related kinase 2 | Hs.153704 | 1E-71 | 87.38739 | 58.42105 | Mm.33773 | 1E-152 | 89.13934 | 85.61404 |
| WAN008DXL_at (SEQ ID NO:1970) | NA | WAN008DXL 11229A-C02 | #N/A | 5E-58 | 89.74359 | 44.72477 | #N/A | 2E-83 | 94.92386 | 45.18349 |
| WAN008DZY_at (SEQ ID NO:1971) | MCM7 | MCM7 minichromosome maintenance deficient 7 (S. cerevisiae) | Hs.438720 | 3E-99 | 88.37209 | 100 | Mm.241714 | 1E-123 | 91.27907 | 100 |
| WAN008E3C_at (SEQ ID NO:1972) | Ptma | Prothymosin alpha | Hs.459927 | 2E-67 | 93.83886 | 44.98934 | Mm.19187 | 1E-148 | 92.74005 | 91.04478 |
| WAN008E3O_at (SEQ ID NO:1973) | LINCR | Likely ortholog of mouse lung-inducible Neutralized-related C3HC4 RING domain protein | Hs.149219 | 3E-19 | 84.61538 | 38.0117 | Mm.389110 | 3E-76 | 85.33724 | 99.7076 |
| WAN008E4X_at (SEQ ID NO:1974) | NA | WAN008E4X 11230A-D06 | #N/A | 0 | 0 | 0 | #N/A | 0 | 0 | 0 |
| WAN008E4Z_at (SEQ ID NO:1975) | Nup153 | Nucleoporin 153 | Hs.601591 | 1E-169 | 89.96063 | 92.53188 | Mm.255398 | 0 | 93.75 | 99.08925 |
| WAN008E5L_at (SEQ ID NO:1619) | SLC1A5 | Solute carrier family 1 (neutral amino acid transporter), member 5 | Hs.631582 | 8E-42 | 84.16667 | 45.62738 | Mm.1056 | 1E-115 | 87.67123 | 83.26996 |
| WAN008E65_at (SEQ ID NO:1976) | ERP29 | Endoplasmic reticulum protein 29 | Hs.75841 | 1E-164 | 91.04803 | 79.79094 | Mm.154570 | 1E-171 | 90.98532 | 83.10105 |
| WAN008E6I_at (SEQ ID NO:1977) | NA | WAN008E6I 11230B-F07 | #N/A | 1E-43 | 88.95706 | 42.22798 | #N/A | 3E-76 | 85.38682 | 90.41451 |
| WAN008EED_at (SEQ ID NO:1521) | Sc5d | Sterol-C5-desaturase (fungal ERG3, delta-5-desaturase) homolog (S. cerevisae) | #N/A | 2E-42 | 85.44601 | 40.72658 | Mm.32700 | 9E-99 | 87.70492 | 69.98088 |
| WAN008EJV_at (SEQ ID NO:1978) | Racgap1 | Rac GTPase-activating protein 1 | Hs.645513 | 1E-103 | 86.39618 | 93.31849 | Mm.273804 | 1E-133 | 89.31116 | 93.76392 |
| WAN008EK5-rc_f_at (SEQ ID NO:1979) | NA | WAN008EK5 11232A-G08 | #N/A | 2E-35 | 92.66055 | 26.65037 | #N/A | 6E-44 | 94.78261 | 28.11736 |
| WAN008EML_at (SEQ ID NO:1980) | PBK | PDZ binding kinase | His.104741 | 5E-52 | 89.50276 | 39.09287 | Mm.24337 | 3E-80 | 89.78102 | 59.17927 |
| WAN008EMN_at (SEQ ID NO:1981) | NA | WAN008EMN 11232B-E01 | #N/A | 0 | 0 | 0 | #N/A | 0 | 0 | 0 |
| WAN008EP0_at (SEQ ID NO:1982) | NA | WAN008EP0 11232C-B07 | #N/A | 0 | 0 | 0 | #N/A | 0.00003 | 95.12195 | 10.90426 |
| WAN008ET3_at (SEQ ID NO:1983) | NA | WAN008ET3 11233A-C09 | #N/A | 3E-47 | 85.65574 | 50.30928 | #N/A | 1E-133 | 89.27739 | 88.45361 |
| WAN008ETA_at (SEQ ID NO:1984) | Usp40 | Ubiquitin specific peptidase 40 | Hs.96513 | 0 | 0 | 0 | Mm.80484 | 3E-46 | 84.72222 | 50.08696 |
| WAN008EXF_at (SEQ ID NO:1985) | KIF11 | Kinesin family member 11 | Hs.8878 | 4E-28 | 86.86131 | 27.56539 | Mm.42203 | 3E-24 | 91.86047 | 17.30382 |
| WAN008F1A_at (SEQ ID NO:1986) | CYC1 | Cytochrome c-1 | Hs.289271 | 1E-124 | 86.82008 | 89.34579 | Mm.29196 | 0 | 92.42424 | 98.69159 |
| WAN013HV4_at (SEQ ID NO:1987) | NA | Cluster includes WAN008F09 10599A-D09 | #N/A | 5E-09 | 97.2973 | 7.07457 | #N/A | 5E-20 | 86.92308 | 24.8566 |
| WAN013HVE_at (SEQ ID NO: 1988) . | NARS | Asparaginyl-tRNA synthetase | Hs.465224 | 1E-104 | 85.0211 | 79.5302 | Mm.29192 | 0 | 92.22904 | 82.04698 |
| WAN013HW1_at (SEQ ID NO:1989) | Eef1d | Eukaryotic translation elongation factor 1 delta (guanine nucleotide exchange protein) | Hs.333388 | 1E-115 | 84.05797 | 99.45946 | Mm.258927 | 0 | 91.24088 | 98.73874 |
| WAN013HW5_at (SEQ ID NO:1990) | RPL10A | Ribosomal protein L10a | Hs.546269 | 1E-164 | 89.09465 | 98.98167 | Mm.336955 | 0 | 91.85336 | 100 |
| WAN013HWL_at (SEQ ID NO:1991) | EBP | Emopamil binding protein (sterol isomerase) | Hs.632801 | 6E-21 | 84.17266 | 24.86583 | Mm.27183 | 2E-46 | 91.9708 | 24.50805 |
| WAN013HX8_x_at (SEQ ID NO:1490) | EIF4A2 | Eukaryotic translation initiation factor 4A, isoform 2 | Hs.518475 | 2E-75 | 94.08602 | 68.50829 | Mm.260084 | 0 | 92.50936 | 98.34254 |
| WAN013HXG_at (SEQ ID NO:1992) | NA | Cluster includes WAN008CY6 10604A-H03 | #N/A | 1E-103 | 88.0814 | 62.54545 | #N/A | 1E-118 | 89.14286 | 63.63636 |
| WAN013HZA_at (SEQ ID NO:1993) | CSE1L | CSE1 chromosome segregation 1-like (yeast) | Hs.90073 | 1E-180 | 88.51351 | 100 | Mm.22417 | 0 | 93.07432 | 100 |
| WAN013103_at (SEQ ID NO:1994) | RPL8 | Ribosomal protein L8 | Hs.178551 | 1E-166 | 88.00705 | 97.92746 | Mm.30066 | 0 | 92.91883 | 100 |
| WAN013I06_at (SEQ ID NO:1995) | NA | Cluster includes WAN008EOQ 11229C-H06 | #N/A | 1E-111 | 85.15284 | 84.34622 | #N/A | 1E-143 | 87.71552 | 85.4512 |
| WAN01310L_at (SEQ ID NO:1996) | SND1 | Staphylococcal nuclease domain containing 1 | Hs.122523 | 1E-156 | 87.89683 | 99.40828 | #N/A | 0 | 91.51874 | 100 |
| WAN013I2L_at (SEQ ID NO:1651) | NA | Cluster includes WAN0088QX 105988-F05 | #N/A | 0 | 0 | 0 | #N/A | 0 | 0 | 0 |
| WAN013I2T_at (SEQ ID NO:1652) | CBX5 | Chromobox homolog 5 (HP1 alpha homolog, Drosophila) | Hs.632724 | 1E-142 | 91.86352 | 72.02268 | Mm.262059 | 1E-168 | 94.75066 | 72.02268 |
| WAN013I3N_at (SEQ ID NO:1997) | NA | Cluster includes WAN00893W 10599B-D08 | #N/A | 6E-29 | 87.31343 | 40.36145 | #N/A | 7E-76 | 92.57426 | 60.84337 |
| WAN01315T_at (SEQ ID NO:1998) | CCNB1 | Cyclin B1 | Hs.23960 | 1 E-93 | 85.30259 | 28.11994 | Mm.260114 | 1E-110 | 87.17949 | 28.44408 |
| WAN013I6G_at (SEQ ID NO:1999) | NA | Cluster includes M12252 Chinese hamster alpha-tubulin I mRNA, complete cds. | #N/A | 0 | 0 | 0 | #N/A | 0 | 0 | 0 |
| WAN013181_at (SEQ ID NO:1905) | POLD1 | Polymerase (DNA directed), delta 1, catalytic subunit 125kDa | Hs.279413 | 0 | 86.30952 | 98.31748 | Mm. 16549 | 0 | 91.73372 | 100 |
| WAN013I8D_at (SEQ ID NO:2000) | PARP1 | Poly (ADP-ribose) polymerase family, member 1 | Hs.177766 | 2E-43 | 85.57692 | 35.01684 | Mm.277779 | 1E-102 | 87.78055 | 67.50842 |
| WAN01318J_at (SEQ ID NO:2001) | CCNB2 | Cyclin B2 | Hs.194698 | 1E-173 | 86.9258 | 44.39216 | Mm.22592 | 0 | 90.70946 | 46.43137 |
| WAN013I8N_at (SEQ ID NO:1776) | IMPDH2 | IMP (inosine monophosphate) dehydrogenase 2 | Hs.476231 | 0 | 90.28974 | 95.36968 | Mm.6065 | 0 | 93.18358 | 96.41524 |
| WAN013I8R_at (SEQ ID NO:2002) | Rps2 | Ribosomal protein S2 | Hs.356366 | 0 | 90.22298 | 99.14966 | Mm.157452 | 0 | 95.05119 | 99.65986 |
| WAN013I9O_at (SEQ ID NO:2003) | TUBB6 | Tubulin, beta 6 | Hs.193491 | 0 | 92.2528 | 71.39738 | Mm.181860 | 0 | 91.74573 | 76.71033 |
| WAN013I9R_at (SEQ ID NO:2004) | NA | Cluster includes Y08202 C.griseus mRNA for RAD51 protein | #N/A | 1E-104 | 84.573 | 63.46154 | #N/A | 1E-142 | 88.98072 | 63.46154 |
| WAN013IAD_at (SEQ ID NO:2005) | TOP2A | Topoisomerase (DNA) II alpha 170kDa | Hs.156346 | 3E-37 | 80.62678 | 29.52061 | Mm.4237 | 1E-86 | 84.11633 | 37.59462 |
| WAN013IAQ-rc_x_at (SEQ ID NO:2006) | CDKN1A | Cyclin-dependent kinase inhibitor 1A (p21, Cip1) | Hs.370771 | 2E-10 | 100 | 14.1129 | Mm.195663 | 1E-31 | 88.88889 | 50.80645 |
| X83575_at (SEQ ID NO:2007) | KIF23 | Kinesin family member 23 | Hs.270845 | 1E-177 | 92.47788 | 37.07957 | Mm.259374 | 0 | 91.99372 | 52.25595 |
| X83576_at (SEQ ID NO:2292) | KIFC1 | Kinesin family member C1 | Hs.436912 | 0 | 86.77111 | 86.84807 | Mm.335713 | 0 | 90.52369 | 90.92971 |

### Example 11. Target validation: siRNA

The ability of the differentially expressed genes and proteins to affect a cellular phenotype is verified by overexpression of a nucleic acid inhibiting the expression of the relevant gene using methods known in the art. Exemplary methods based on interfering RNA constructs are described below.

### Design and synthesis of siRNA

Typically, targets that are candidates for siRNA mediated gene knockdown are sequenced, and the sequences verified. Full-length cDNA sequence information is preferred (although not required) to facilitate siRNAs design. The target sequence that is a candidate for gene knockdown is compared to gene sequences available on public or proprietary databases (*e.g*., BLAST search). Sequences within the target gene that overlap with other known sequences (for example, 16-17 contiguous basepairs of homology) are generally not suitable targets for specific siRNA-mediated gene knockdown.

siRNAs may be designed using, for example, online design tools, over secure internet connections, such as the one available on the Ambion® website (http://www.ambion.com/techlib/misc/siRNA_finder.html). Alternatively, custom siRNAs may also be requested from Ambion®, which applies the Cenix algorithm for designing effective siRNAs. The standard format for siRNAs is typically 5nmol, annealed and with standard purity in plates. Upon receipt of synthesized siRNAs, the siRNAs are prepared according to the instructions provided by the manufacture and stored at the appropriate temperature (-20°C)

Standard procedures were used for siRNA transfections. Cells to be transfected were typically pre-passaged on the day before transfection to ensure that the cells are in logarithmic growth phase. Typically, an siRNA Fed-Batch assay was used. Exemplary materials, conditions and methods for transfections are as follows.

### Transfection (D0)

Per Spin Tube (50ml)
100uL R1
2uL Transit-TKO transfection reagent (Mirus)
10uL 10uM siRNA
2mL 1e5 cells/mL in AS1 medium

### Following Transfection

37°C: 72 hrs
31°C: 96 hrs
Feed: AQ3 on day 3 (D3)
Sample taken on day 1 (D1), day 3 (D3), day 7 (D7)

### 24 Well Suspension Transfections

For each experiment, 100,000 cells (*e.g.*, 3C7 cells) in 1mL total volume, and 50nM siRNA were used. To make a mix for 3 reactions, 150µL R1 and 70 µL Mirus TKO reagent were mixed and incubated for 10 minutes at room temperature. 15µL of 10µM siRNA was added and the mix was incubated for 10 minutes at room temperature. 57.3µL of the mix was transferred into each of 3 wells. 942.7µL of R5CD1 (containing 100,000 cells) was added and the plate was incubated on rocker at 37°C for 72hrs.

### Spin Tube siRNA Transfection

For each experiment, 100,000 cells (*e.g*., 3C7 cells) in 1mL total volume were used. For each transfection, 100µL R1 and 2µL Mirus TKO reagent were mixed and incubated for 10 minutes at room temperature. 10µL of 10µM siRNA was added and the mix was incubated for 15 minutes at room temperature, mixed occasionally. 1.9mL culture was transferred to each spin tube. siRNA mix (112uL) was added to each spin tube. The culture was initially incubated at 37°C and then the temperature was shifted to 31°C on day 3. Spin tube cultures were shaken rapidly (~250RPM). Samples were taken on days 1, 3, and 7. Cultures were terminated on day 7.

Growth and productivity controls were included on each plate. An exemplary productivity control is DHFR (selectable marker on bicistronic mRNA). Treatment with DHFR siRNA reproducibly decreases amount of antibody in the CM-FcIGEN (antibody production control). An exemplary growth control is CHO1 (kinesin) (see Matuliene et al. (2002) Mol. Cell. Biol. 13:1832-45) (typically, about 20-30% growth inhibition was observed with CHO1 treatment). Other standard controls such as no siRNA treatment (transfection reagents only) and non-targeting siRNA treatment (non-specific siRNA) were also included. Plates were then subjected to cell counting (for example, in a 96-well cell counting instrument) to assess growth and to, for example, an automated 96-well titer assay, to assess productivity. Genes whose modulation, singly or in combination, are sufficient to modify useful cellular phenotypes were thereby validated and such changes can be engineered, singly or in combination, into a mammalian cell line to modify its properties.

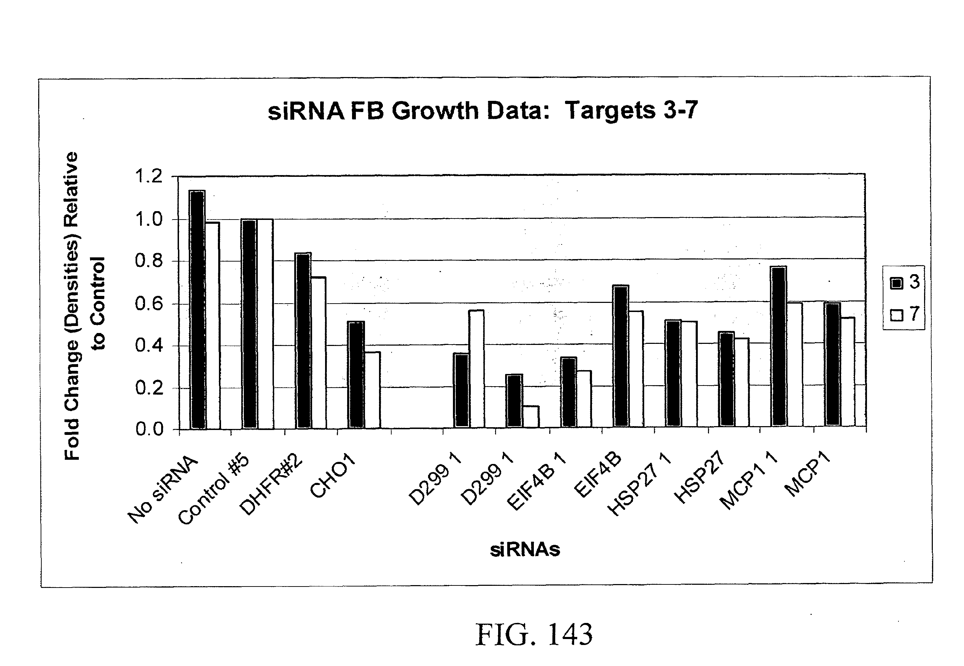

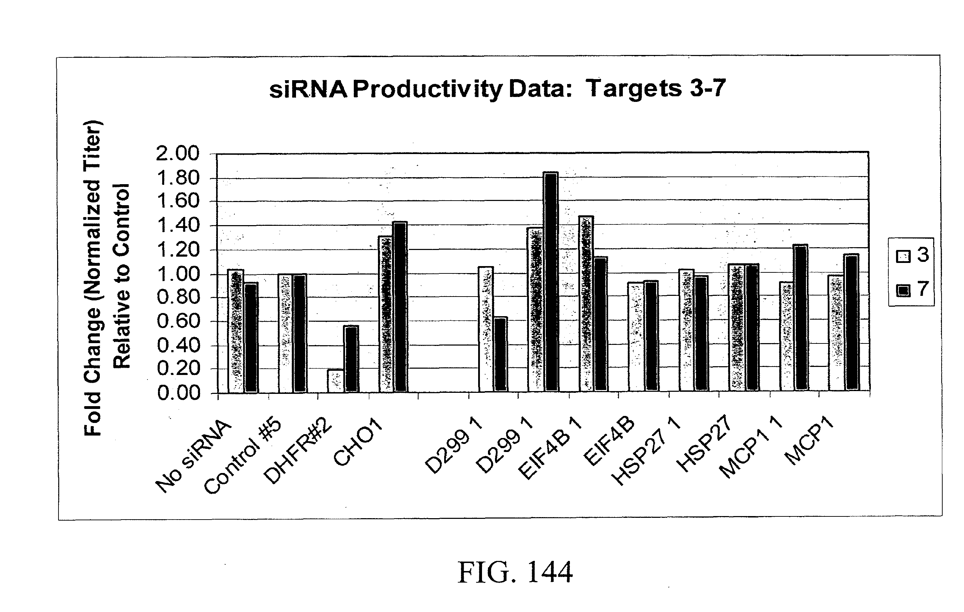

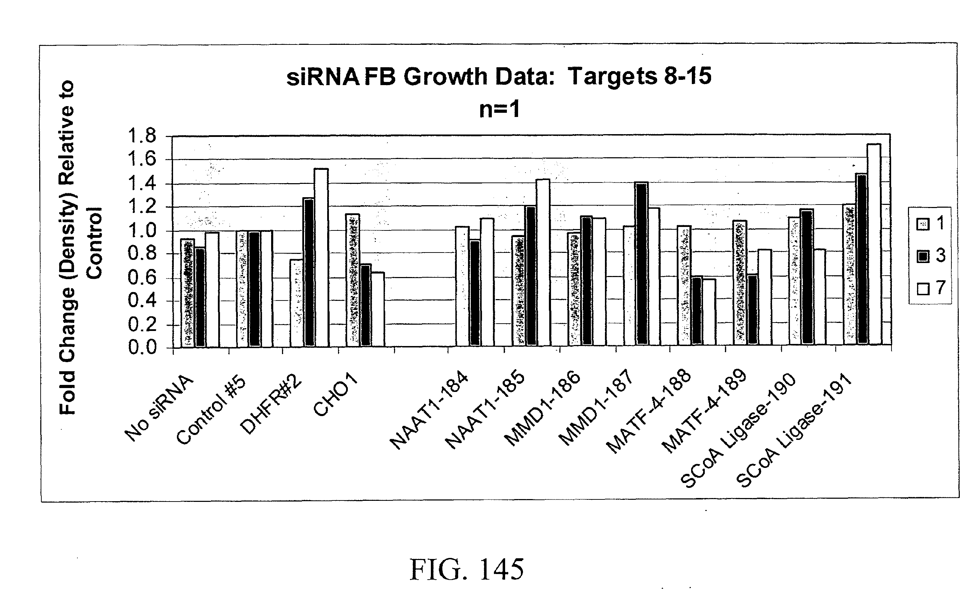

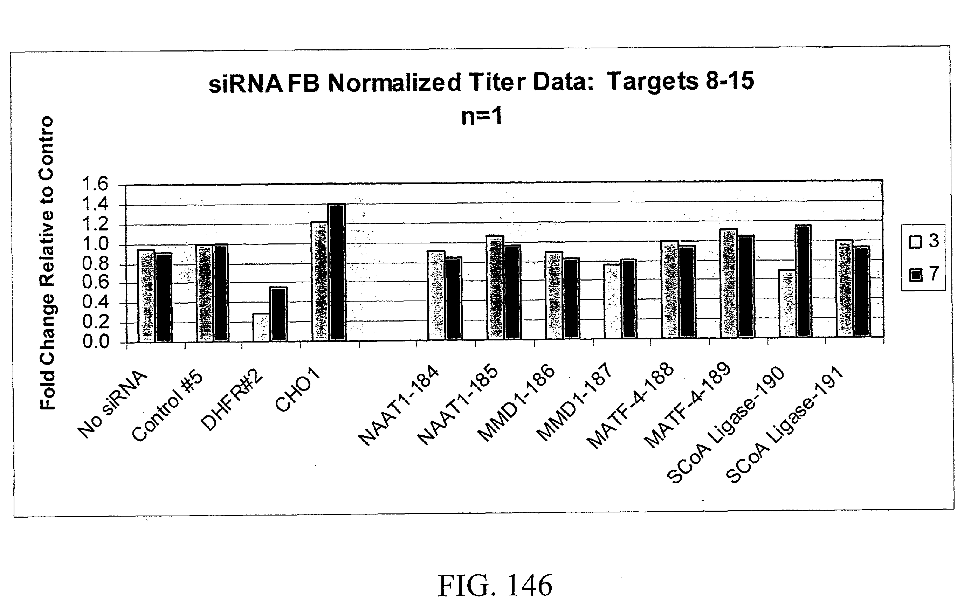

Model cell lines used for the validation purposes and their characteristics are shown in Table 21. Figures 143-146 summarize the evaluation of some of the target genes in the spin tube format in the 3C7 cell line. Target genes evaluated include D299 (WAN013I8K), identified above as elevated in cells with elevated growth rates; EIF4B, identified above as elevated in cells with elevated growth rates; HSP27 (HSPB1), identified above as elevated in cells with elevated growth rates; MCP1 (CCL2), identified above as depressed in cells with high cell density; NAAT1 (SLC1A4), identified above as depressed in cells with elevated growth rates; MMD1 (malate dehydrogenase), identified above as depressed in cells with high maximum cellular productivities; MATF-4 (ATF-4), identified above as elevated in cells with high cell densities; and SCoA Ligase (SUCLG2), identified above as elevated in cells with high cell densities. As shown in Figure 143, for genes identified as elevated in cells with elevated growth rates, inhibition of the gene led to an inhibition of growth relative to the control. Cellular productivity was generally not comparably affected, as shown in Figure 144.

**Table 21. Cell lines and their characteristics**

| **Clone** | **Characteristics** |
|---|---|
| 1.14 | High growth rate; Low Qp |
| 1.18 | Average |
| 2.8 | High Qp; High cell density |
| 2B6 | Low cell density; Low Qp |
| DA-4 | Low cell density; Low GR |
| DD-11 | Average |
| DE-6 | Average |
| 3B12 | High Qp; High cell density |
| 5C10 | Average |
| 5B5 | Low Qp |
| 3C7 | Average |

### Example 12. Target validation: overexpression

The ability of the differentially expressed genes and proteins to affect a cellular phenotype is verified by overexpression of a nucleic acid encoding the expression of the relevant gene using methods known in the art. Exemplary methods are described below.

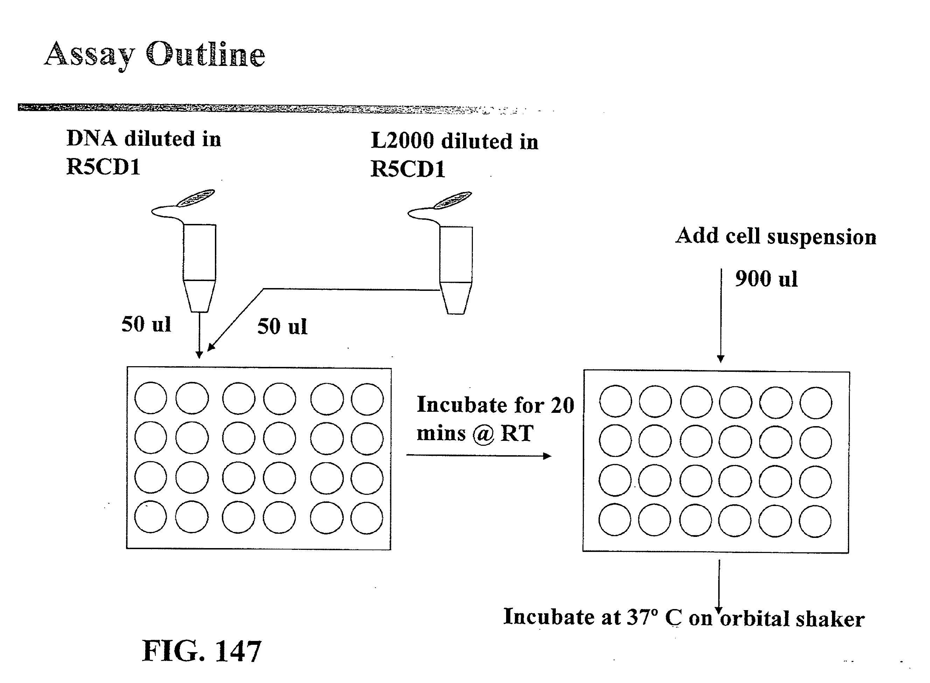

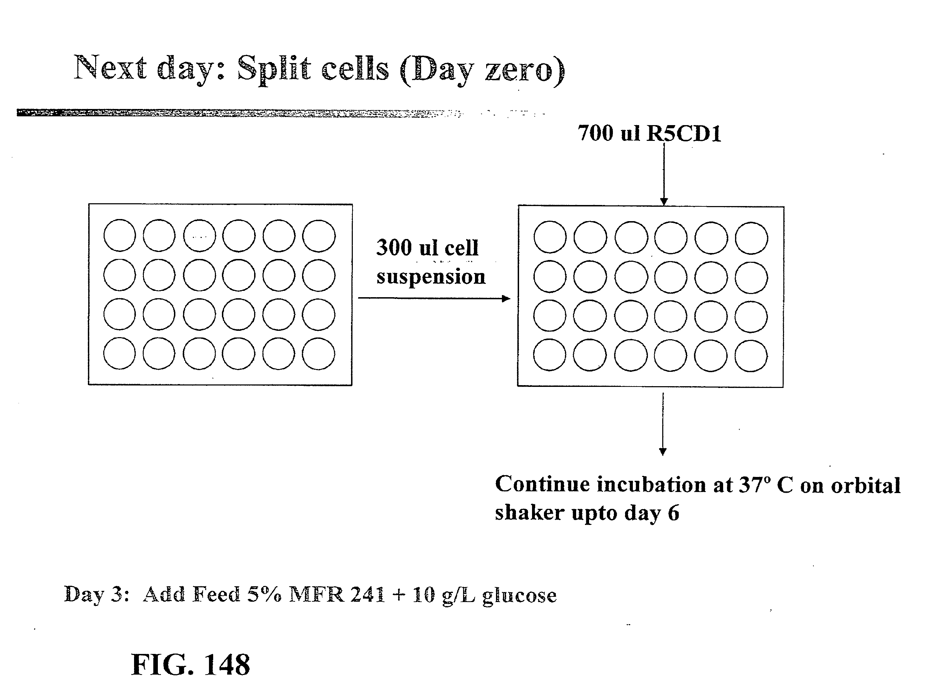

For example, nucleic acids overexpressing specific targets can be introduced into CHO cells by transient transfections and then the impact of over-expression on cellular growth and productivity are monitored. An exemplary protocol, 24 well format, was illustrated in Figures 147 and 148.

Growth and productivity controls are typically used for overexpression assays. For example, positive growth/viability control used in this experiment included Ha-Ras and Bcl-xL. Negative growth control used included p27. Other suitable growth and productivity controls are known in the art and can be used for overexpression assays. Additional standard controls such as no nucleic acid control (transfection reagents only) were also included.

Target genes and the control genes were cloned into the pExpress 1 vector and introduced into various model cell lines as shown in Table 22.

**Table 22. Cell lines for the assay and their characteristics**

| **Clone** | **Characteristics** |
|---|---|
| 1.18 | Middle of the road |
| 5C10 | Middle of the road |
| DE-6 | Middle of the road |
| DD-11 | Middle of the road |
| 1.14 | HCGR, sustained high viability, not sustained high Qp, Not low NH4, Not high cell density |
| 2.8 | High max Qp, sustained high Qp |
| 3B12 | High max Qp, sustained high Qp, high cell density, low lactate |
| DA-4 | Not HCGR, Not high cell density |
| 5B5 | Not sustained high Qp, Not low lactate |
| 2B6 | Not high cell density, Not high max Qp |

The 24 well format was used to distinguish phenotypic effects of transient transfection of various genes on various cell lines. Cellular growth and productivity were determined. Exemplary results are illustrated in Figures 149-151. It was found that results were generally representative and reproducible. Exemplary overexpression results are summarized in Table 23.

**Table 23. Summary of the overexpression assays**

| Gene over-expressed | Cell line tested | Growth phenotype | Productivity phenotype | |
|---|---|---|---|---|
| P27 | | | Titer | Qp, |
| | 1.14 | ↓(---) | NA | NA |
| | 1.18 | ↓(-) | NA | NA |
| | 5C10 | ↓(--) | ↓(--) | ↑(-) |
| | 5B5 | ↓(---) | ↓(--) | ↑(-) |
| | 3B12 | ↓(---) | NA | NA |
| | 2B6 | ↓(---) | NA | NA |
| | 2.8 | ↓(-) | NA | NA |
| | DE-6 | ↓(--) | NA | NA |
| | DD-11 | ↓(--) | NA | NA |
| Bcl-xL | 5B5 | ↑(+++) | ↑(++) | No change |
| H-Ras | 5C10 | ↓(--) | ↓(--) | No change |
| | 5B5 | ↓(---) | ↓(--) | ↓(-) |
| | 3B12 | ↓(--) | NA | NA |
| 3A (EIF4B) | 5C10 | ↑(+) | ↑(+) | No change |
| | 5B5 | ↑(+) | ↑(+) | No change |
| | | | | |
| NA= Not tested or awaiting results | | | | |
| (* * *) Strongly increased or decreased | | | | |
| (* *) Increased or decreased | | | | |
| (*) moderately increased or decreased | | | | |

### Example 13. Engineering cell lines to improve cell phenotypes based on the verified target genes

The verified target genes are used to effect a cell phenotype, particularly a phenotype characterized by increased and efficient production of a recombinant transgene, increased cell growth rate, high peak cell density, sustained high cell viability, high maximum cellular productivity, sustained high cellular productivity, low ammonium production, and low lactate production, *etc.* Exemplary target genes are disclosed above, for example, in Tables 2 through 20 and in Tables 24 through 30.

**Table 24**

| ***High Cell Growth Rate*** | | | | | | | | | | | | |
|---|---|---|---|---|---|---|---|---|---|---|---|---|
| **Qualifier List** | **Symbol** | **Title** | **Human Unigene ID** | **eValue** | **%ID** | **%QC** | **Mouse Unigene ID** | **eValue** | **%ID** | **%QC** | **FC** | **Function** |
| U62588_x_at (SEQ ID NO:1586) | SDC1 | Syndecan 1 | Hs.224607 | 1E-32 | 93 | 53.2 | Mm.2580 | 7E-48 | 91 | 81.4 | down | Adhesion |
| WAN008D2Q_at (SEQ ID NO:1561) | Eif4b | Eukaryotic translation initiation factor 4B (Eif4b) | #N/A | 6E-49 | 93 | 29.4 | Mm.290022 | 1E-129 | 91 | 71.6 | up | translation (initiation) |
| WAN008DJ9_at (SEQ ID NO:1565) | SLC1A4 | Solute carrier family 1 (glutamate/neutral amino acid transporter), member 4 | Hs.323878 | 2E-39 | 87 | 39.5 | Mm.6379 | 1E-121 | 89 | 90.6 | down | serine transporter |
| WAN01310W_at (SEQ ID NO:1580) | TAPBP | TAP binding protein (tapasin) | Hs.370937 | 2E-57 | 81 | 93.2 | Mm.154457 | 1E-149 | 87 | 95.3 | down | ER peptide transporter |
| WAN01310X_at (SEQ ID NO:1581) | GSS | Glutathione synthetase | Hs.82327 | 3E-96 | 90 | 56.1 | Mm.252316 | 1E-129 | 95 | 55.7 | down | glutathione synthesis (protect from oxidative stress) |
| WAN01311G_at (SEQ ID NO:1582) | SLC25A20 | Solute carrier family 25 (carnitine/acylcarnitine translocase), member 20 | Hs.13845 | 1 E-1 37 | 87 | 87.7 | Mm.29666 | 0 | 92 | 86.4 | down | fatty acid translocation across mitochondrial membrane |
| WAN01318K_at (SEQ ID NO:1584) | NA | Cluster includes D29972 Cricetulus griseus Mitochondrial DNA, D-loop region. | #N/A | | | | #N/A | | | | up | |
| X51747_at (SEQ ID NO:1587) | HSPB1 | Heat shock 27kDa protein 1 | Hs.520973 | 1E-101 | 87 | 50.5 | Mm.13849 | 0 | 92 | 66.1 | up | UPR |
| WAN008EEO_x_at (SEQ ID NO:1789) | Ndufs1 | NADH dehydrogenase (ubiquinone) Fe-S protein 1 | Hs.471207 | | | | Mm.290791 | | | | up | electron transport in Mitochondria |

| ***High Cell Density*** | | | | | | | | | | | | |
|---|---|---|---|---|---|---|---|---|---|---|---|---|
| **Qualifier List** | **Symbol** | **Title** | **Human Unigene ID** | **eValue** | **%ID** | **%QC** | **Mouse Unigene ID** | **evalue** | **%ID** | **%QC** | **FC** | **Function** |
| AF022945-rc_f_at (SEQ ID NO:1666) | Thbd | Thrombomodulin | Hs.2030 | | | | Mm.24096 | 1E-13 | 90 | 65 | up | thrombin binding |
| AF081141_at (SEQ ID NO:1667) | CCL2 | Chemokine (C-C motif) ligand 2 | Hs.303649 | 1E-12 | 98 | 9.01 | Mm.290320 | 6E-41 | 91 | 28.1 | down | cytokine; inflammation |
| M27838_s_at (SEQ ID NO:1670) U29167_at | ASNS | Asparagine synthetase | Hs.489207 | 0 | 89 | 100 | Mm.2942 | 0 | 92 | 100 | up | aparagine synthesis |
| (SEQ ID NO:1672) WAN0088X2_at | TPM2 | Tropomyosin 2 (beta) Progressive external ophthalmoplegia | Hs.300772 | 0 | 93 | 88.8 | Mm.646 | 0 | 95 | 90.3 | up | focal adhesion mitochondrial DNA |
| (SEQ ID NO:1593) WAN008CQP_at (SEQ ID NO:1598) | PEO1 AATF | 1 Apoptosis antagonizing transcription factor | Hs.22678 Hs.195740 | 1E-141 6E-73 | 89 84 | 94.5 99.3 | Mm.105585 Mm.257482 | 7E-78 8E-99 | 91 86 | 47.8 99.3 | up | helicase |
| WAN008CX9_at (SEQ ID NO:1599) | ISGF3G | Interferon-stimulated transcription factor 3, gamma 48kDa | Hs.1706 | 2E-64 | 83 | 81.5 | Mm.2032 | 1E-119 | 88 | 88.5 | up | |
| WAN008CXC_at (SEQ ID NO:1600) | ATP6VOA 1 | ATPase, H+ transporting, lysosomal VO subunit a isoform 1 | Hs.463074 | 0 | 93 | 99.4 | Mm.340818 | 0 | 94 | 100 | down | addification of intracellular organelles |
| WAN008D2S_at (SEQ ID NO:1601) | BPY21P1 | BPY2 interacting protein 1 | Hs.66048 | 6E-15 | 84 | 21 | Mm.248559 | 1E-101 | 87 | 69 | down | microtubule binding |
| WAN008D55-rc_at (SEQ ID NO:1603) | LAMB1 | Laminin, beta 1 | Hs.489646 | 1E-155 | 88 | 97.5 | Mm.172674 | 1E-161 | 92 | 77.7 | 1up, 1down | glycoprotein; cell adhesion |
| WAN008D5V_x_at (SEQ ID NO:1562) | Gosr2 | Golgi SNAP receptor complex member 2, mRNA (cDNA clone MGC:6437 IMAGE:3601627) | Hs.463278 | | | | Mm.195451 | 1E-08 | 90 | 43.6 | down | transporter; golgi trafficking |
| WAN008D6R_at (SEQ ID NO:1604) | TMED4 | Transmembrane emp24 protein transport domain containing 4 | Hs.510745 | 1E-111 | 91 | 73.7 | Mm.254495 | 1E-140 | 92 | 86.6 | down | transporter; unknown function |
| WAN008DMI-at (SEQ ID NO:1610) | ACSL5 | Acyl-CoA synthetase long-chain family member 5 | Hs.11638 | 1E-118 | 85 | 96.6 | #N/A | 0 | 90 | 99.6 | up | lipid biosynthesis; fatty acid degradation |
| WAN008DWJ_at (SEQ ID NO:1614) | USP1 | Similar to ubiquitin spedfic protease 1 | Hs.35086 | 0 | 93 | 97 | Mm.371692 | 0 | 94 | 96.5 | up | de-ubiquitinating enzyme |
| WAN008E5L_at (SEQ ID NO:1619) | SLC1A5 | Solute carrier family 1 (neutral amino acid transporter), member 5 | Hs.515494 | 8E-42 | 84 | 45.6 | Mm.1056 | 1E-115 | 88 | 83.3 | up | amino acid transporter |
| WAN008E9N_at (SEQ ID NO:1620) | KLHL7 | Kelch-like 7 (Drosophila) | Hs.385861 | 1E-150 | 89 | 99.4 | Mm.273768 | 0 | 93 | 89.1 | down | unknown function |
| WAN008EBP_at (SEQ ID NO:1621) | Sqstml | Sequestosome 1 | Hs.529892 | 0 | 93 | 97.8 | Mm.40828 | 0 | 93 | 97.8 | down | ubiquitin-associated protein |
| ***WAN008EH5_at (SEQ ID NO:1622)*** | ***PRNP*** | ***Prion protein (p27-30) (Creutzfeld-Jakob disease, Gerstmann-Strausler-Scheinker syndrome, fatal familial insomnia)*** | ***Hs.472010*** | 9E-45 | 87 | 34.8 | ***Mm.648*** | ***4E-92*** | ***90*** | **57.2** | ***down*** | |
| WAN008ELE_at (SEQ ID NO:1626) | PSAT1 | Phosphoserine aminotransferase 1 | Hs.494261 | 7E-27 | 93 | 16_{.}2 | Mm.289936 | 5E-70 | 88 | 58.2 | up | serine biosynthesis |
| WAN008EM4_at (SEQ ID NO:1627) | ARHGAP1 8 | Rho GTPase activating protein 18 | Hs 486458 | 1E-109 | 85 | 97.9 | Mm.356496 | 1E-147 | 88 | 100 | down | unknown function |
| WAN008EOB_at (SEQ ID NO:1629) | NOL1 | Nucleolar protein 1, 120kDa | Hs.534334 | 8E-48 | 90 | 42.5 | Mm.29203 | 1E.120 | 87 | 92.2 | up | cell cycle progression |
| WAN008ERI_at (SEQ ID NO:1632) | FNBP3 | Formin binding protein 3 | Hs.298735 | 4E-81 | 97 | 98.9 | Mm.257474 | 2E-94 | 99 | 100 | down | pre-mRNA processing |
| WAN008ERP_at (SEQ ID NO:1634) | LEPREL1 | Leprecan-like 1 | Hs.374191 | 1E-45 | 87 | 92.8 | Mm.326869 | 1E-68 | 89 | 94 | down | negative regulation of cell proliferation(?) |
| WAN008EUO_at (SEQ ID NO:1635) | LPL | Lipoprotein lipase | Hs.180878 | 2E-82 | 88 | 74.1 | Mm.1514 | 1E-113 | 92 | 74.1 | down | glycerolipid metabolism Homologs in new array, but +/- in most cases, and none of these have any homology Appears to be mitochondrial polycistronic mRNA!!! |
| WAN008F1P_x_at (SEQ ID NO:1637) | NA | WAN008F1P 11165A-A01 | #N/A | | | | #N/A | | | | down | |
| WAN013HW0_x_at (SEQ ID NO:1640) | NA | Cluster includes WAN008C03 10600D.F02 | #N/A | | | | #N/A | | | | up | |
| WAN013HX8_f_at (SEQ ID NO:1490) | EIF4A2 | Eukaryotic translation initiation factor 4A, isoform 2 | Hs.478553 | 1E-155 | 96 | 100 | Mm.260084 | 1E-155 | 96 | 100 | down | translation initiation |
| WAN013l1U_x_at (SEQ ID NO:1648) | NA | Cluster includes WAN008BLL 11233C-H10 | #N/A | 2E-05 | 92 | 7.71 | #N/A | 1E-05 | 92 | 7.71 | up | |
| WAN013l2T_at (SEQ ID NO:1652) | CBX5 | Chromobox homolog 5 (HP1 alpha homolog, Drosophila) | Hs.349283 | 1E-142 | 92 | 72 | Mm.262059 | 1E-168 | 95 | 72 | up | chromatin binding |
| WAN013l6J_s_at (SEQ ID NO:1657) | CAD | Carbamoyl-phosphate synthetase 2, aspartate transcarbamylase, and dihydroorotase | Hs.377010 | 0 | 91 | 99.5 | Mm.305535 | 0 | 94 | 99.5 | up | pyrimidine biosynthesis |
| WAN01318X_at (SEQ ID NO:1661) | HSPD1 | Heat shock 60kDa protein 1 (chaperonin) | Hs.113684 | 0 | 90 | 99.8 | Mm.1777 | 0 | 93 | 99.8 | up | molecular chaperone |
| WAN013l9Z_at (SEQ ID NO:1664) | GNAS | guanine nucleotide binding protein, alpha stimulating | Hs.125898 | | | | Mm.125770 | 0 | 94 | 41.1 | down | cell growth |
| WAN013l9F_at (SEQ ID NO:1662) | HSPA9B | Heat shock protein 9A | Hs.184233 | 3E-29 | 92 | 18.7 | Mm.209419 | 2E-72 | 91 | 44.8 | up | cell proliferation |

| ***High Max Qp*** | | | | | | | | | | | | |
|---|---|---|---|---|---|---|---|---|---|---|---|---|
| **Qualifier List** | **Symbol** | **Title** | **Human Unigene ID** | **eValue** | **%ID** | **%QC** | **Mouse Unigene ID** | **eValue** | **%ID** | **%QC** | **FC** | **Function** |
| gil34853001 | Uap1l1 | PREDICTED. similar to UDP-N-acteylglucosamine | | pyrophosphorylase | 1-like 1 | | Mm.33797 | | | | -2.22 | |

| ***Sustained High Cell Viability*** | | | | | | | | | | | | |
|---|---|---|---|---|---|---|---|---|---|---|---|---|
| **Qualifier List** | **Symbol** | **Title** | **Human Unigene ID** | **eValue** | **%ID** | **%QC** | **Mouse Unigene ID** | **eValue** | **%ID** | **%QC** | **FC** | **Function** |
| AF022942_at (SEQ ID NO:2008) | Cirbp | Cold inducible RNA binding protein | Hs.634522 | 8E-40 | 86 | 86.5 | Mm.17898 | 9E-94 | 95 | 100 | up | |
| AF120325_f_at (SEQ ID NO:1753) | TUBB2B | Tubulin, beta 2B | Hs.300701 | 0 | 89 | 72.7 | #N/A | 0 | 92 | 78.5 | up | |
| M12329_at (SEQ ID NO:1942) | NA | M12329 Chinese hamster alpha-tubulin III mRNA, complete cds. | #N/A | 0 | 93 | 54.3 | #N/A | 0 | 96 | 53.8 | up | |
| M96676_at (SEQ ID NO:1727) | LGALS1 | Lectin, galadoside-binding, soluble, 1 (galectin 1) | Hs.445351 | 1E-122 | 89 | 100 | Mm.43831 | 1E-131 | 90 | 100 | up | |
| WAN0088YL_f_at (SEQ ID NO:2009) | 2700085E 05Rik | RIKEN cDNA 2700085E05 gene | #N/A | 2E-58 | 91 | 96.5 | Mm.249700 | 4E-77 | 94 | 100 | up | |
| WAN008CZP_at (SEQ ID NO:1962) | NA | WAN008CZP 10604A-A08 | #N/A | 3E-29 | 91 | 21.4 | #N/A | 7E-50 | 83 | 62.5 | up | |
| WAN008E65_at (SEQ ID NO:1976) | ERP29 | Endoplasmic reticulum protein 29 | Hs.75841 | 1E-164 | 91 | 79.8 | Mm.154570 | 1E-171 | 91 | 83.1 | up | |
| WAN008940_at (SEQ ID NO:2010) | MRPL37 | Mitochondrial ribosomal protein L37 | Hs.584908 | 5E-68 | 86 | 54.6 | Mm.29517 | 1E-102 | 90 | 60.7 | down | |
| WAN008CQI_at (SEQ ID NO:2011) | GLTSCR2 | Glioma tumor suppressor candidate region gene 2 | Hs.421907 | 1E-113 | 86 | 100 | Mm.277634 | 1E-175 | 91 | 100 | down | |
| WAN008DAG_at (SEQ ID NO:2012) | AARS | Alanyl-tRNA synthetase | Hs.315137 | 1E-101 | 89 | 70.7 | Mm.24174 | 1E-134 | 92 | 74.7 | down | |
| WAN008DSH_at (SEQ ID NO:2013) | MRPL16 | Mitochondrial ribosomal protein L16 Solute carrier family 6 | Hs.530734 | 1 E-37 | 85 | 40 | Mm.203928 | 4E-64 | 90 | 42.4 | down | |
| WAN008DXE_x_at (SEQ ID NO:2014) | SLC6A8 | (neurotransmitter transporter, creatine), member 8 | Hs.540696 | 1 E-59 | 95 | 99.3 | Mm.274553 | 1E-64 | 97 | 99.3 | down | |
| WAN008E2E_at (SEQ ID NO:1567) | PSMC4 | Proteasome (prosome, macropain) 26S subunit, ATPase, 4 | Hs.211594 | 1E-153 | 92 | 100 | Mm.29582 | 1E-141 | 91 | 100 | down | |
| WAN008E5L_at (SEQ ID NO:1619) | SLC1A5 | Solute carrier family 1 (neutral amino acid transporter), member 5 | Hs.631582 | 8E-42 | 84 | 45.6 | Mm.1056 | 1E-115 | 88 | 83.3 | down | |
| WAN008EE3_at (SEQ ID NO:1809) | SARS | Seryl-tRNA synthetase | Hs.531176 | 1E-116 | 91 | 100 | Mm.28688 | 1E-136 | 93 | 100 | down | |
| WAN013HVH_at (SEQ ID NO:2015) | GARS | Glycyl-tRNA synthetase | Hs.404321 | 1 E-1 77 | 88 | 100 | Mm.250004 | 0 | 94 | 100 | down | |
| WAN013l1O_at (SEQ ID NO:1804) | RNH1 | Ribonuclease/angiogenin inhibitor 1 | Hs.530687 | 1E-18 | 83 | 27.8 | Mm.279485 | 1E-91 | 88 | 61.2 | down | |
| WAN013l1Q_at (SEQ ID NO:2016) | TXNL2 | Thioredoxin-like 2 | Hs.42644 | 2E-90 | 89 | 53.5 | Mm.267692 | 1E-126 | 94 | 53.8 | down | |
| WAN008EE5_at (SEQ ID NO:2017) | PANX1 | Pannexin 1 | Hs.591976 | 1 E-125 | 89 | 100 | Mm.142253 | 1E-177 | 94 | 100 | down | |

| ***Sustained High Qp*** | | | | | | | | | | | | |
|---|---|---|---|---|---|---|---|---|---|---|---|---|
| **Qualifier List** | **Symbol** | **Title** | **Human Unigene ID** | **eValue** | **%ID** | **%QC** | **Mouse Unigene ID** | **eValue** | **%ID** | **%QC** | **FC** | **Function** |
| WAN013HUM_at (SEQ ID NO:1576) | EHD4 | EH-domain containing 4 | Hs.143703 | 1 E-95 | 93 | 59 | Mm.132226 | 1E-125 | 89 | 99.5 | up | |
| AB014875_at (SEQ ID NO:1734) | PLS3 | plastin 3 (T isoform) | Hs.496622 | 1E-159 | 92 | 31.6 | Mm.28777 | 1E-175 | 90 | 40.7 | down | |
| WAN0088PY_at (SEQ ID NO:1480) | NA | WAN0088PY 10595D-B07 | Hs.279806 | 0 | 0 | 0 | Mm.220038 | 2E-07 | 93 | 9.23 | down | |
| WAN008CIU_at (SEQ ID NO:2018) | NA | WAN008CIU 10599D-C10 | #N/A | 0 | 0 | 0 | #N/A | 1E-05 | 100 | 5.79 | down | |
| WAN008EY0_at (SEQ ID NO:2019) | NA | WAN008EYO 112338-B05 | #N/A | 0 | 0 | 0 | #N/A | 3E-09 | 89 | 12.4 | down | |
| WAN008CIA_at (SEQ ID NO:2020) | EIF1AY | Eukaryotic translation initiation factor 1A, Y-linked | Hs.461178 | 1E-137 | 91 | 72 | Mm.294623 | 1E-164 | 89 | 99.6 | up | |
| WAN008D60_at (SEQ ID NO:1692) | STRBP | Spermatid perinuclear RNA binding protein | Hs.645506 | 9E-62 | 90 | 67.7 | Mm.237095 | 3E-94 | 95 | 71 | up | |
| WAN008DNJ_at (SEQ ID NO:2021) | Rbmxrt | RNA binding motif protein, X chromosome retrogene | Hs.380118 | 1E-131 | 92 | 71 | Mm.24718 | 0 | 95 | 97.9 | up | |
| WAN008DWF_f_at (SEQ ID NO:2022) | NA | WAN008DWF 11229A-H02 | #N/A | 0 | 0 | 0 | #N/A | 0 | 0 | 0 | up | |
| WAN013HUG_at (SEQ ID NO:2023) | CDKN2C | Cydin-dependent kinase inhibitor 2C (p18, inhibits CDK4) | Hs.525324 | 1E-113 | 95 | 53.7 | Mm-1912 | 1E-142 | 99 | 53.1 | up | |
| WAN00880Y_x_at (SEQ ID NO:2024) | HNRPF | Heterogeneous nuclear ribonucleoprotein F | Hs.558477 | 1E-87 | 99 | 95.8 | Mm.317706 | 1E-98 | 100 | 100 | down | |
| WAN01318H_x_at (SEQ ID NO:1548) | APP | Amyloid beta (A4) precursor protein (protease nexin-II, Alzheimer disease) | Hs.642685 | 1E-77 | 84 | 83.5 | Mm.277585 | 1E-167 | 88 | 100 | down | |
| X53074_f_at (SEQ ID NO:2025) | HPRT1 | Hypoxanthine phosphoribosyliransferase 1 (Lesch-Nyhan syndrome) | Hs.412707 | 5E-35 | 93 | 47.4 | Mm.299381 | 7E-36 | 93 | 47.8 | down | |
| WAN008EJ7_at (SEQ ID NO:2026) | EIF5A | Eukaryotic translation initiation factor 5A | Hs.534314 | 0 | 99 | 100 | Mm.196607 | 0 | 98 | 100 | down | |
| ***Low Ammonia Producer*** | | | | | | | | | | | | |

| **Qualifier List** | **Symbol** | **Title** | **Human Unigene ID** | **eValue** | **%ID** | **%QC** | **Mouse Unigene ID** | **eValue** | **%ID** | **%QC** | **FC** | **Function** |
|---|---|---|---|---|---|---|---|---|---|---|---|---|
| AF180918_at (SEQ ID NO:1778) | KLHL75 | Kelch-like 5 (Drosophila) | Hs.272251 | 6E-21 | 89 | 19.8 | Mm.10281 | 5E-48 | 86 | 49.2 | up | |
| WAN013HW0_x_at (SEQ ID NO:1640) | NA | Cluster includes WAN008CO3 10600D-F02 | #N/A | | 0 | 0 | #N/A | | 0 | 0 | up | |
| WAN01318U_at (SEQ ID NO:2027) | NA | Cluster includes M14311 Chinese Hamster mitochondrial ATPase 6 and URF A6L genes, complete cds. | #N/A | 2E-05 | 92 | 20.3 | #N/A | 0 | 0 | 0 | up | |
| AF100738_at (SEQ ID NO:2028) | SUI1 | Putative translation initiation factor | #N/A | 8E-09 | 85 | 53.4 | #N/A | 2E-11 | 89 | 30.8 | down | |
| L00176_at (SEQ ID NO:1500) | Hmgcr | 3-hydroxy-3-methylglutaryl-Coenzyme A reductase | Hs.643495 | 7E-54 | 88 | 57.9 | Mm.316652 | 3E-82 | 94 | 55.4 | down | |
| L00334_at (SEQ ID NO:2029) | Hmgcs1 | 3-hydroxy-3-methylglutaryl-Coenzyme A synthase 1 | Hs.397729 | 1E-100 | 88 | 36 | Mm.61526 | 1E-178 | 90 | 45.5 | down | |
| M29238_at (SEQ ID NO:2030) | DDIT3 | DNA-damage-inducible transcript 3 | Hs.505777 | 1E-100 | 87 | 76.8 | Mm.110220 | 1E-119 | 89 | 68.9 | down | |
| M60973_at (SEQ ID NO:2031) | GADD45A | Growth arrest and DNA-damage-inducible, alpha | Hs.80409 | 0 | 92 | 76.3 | Mm.389750 | 1E-170 | 91 | 49.2 | down | |
| U29660_s_at (SEQ ID NO:2032) | NA | U29660 Cricetulus griseus hydrogen peroxide-Inducible adapt33A RNA. | #N/A | 0 | 0 | 0 | #N/A | 1E-13 | 85 | 8.15 | down | |
| U48852_at (SEQ ID NO:1502) | CRELD2 | Cysteine-rich with EGF-like domains 2 | Hs.211282 | 1 E-109 | 82 | 55.1 | Mm.292567 | 0 | 89 | 91.7 | down | |
| U67146_at (SEQ ID NO:1683) | EEF1E1 | Eukaryotic translation elongation factor 1 epsilon 1 | Hs.631818 | 1 E-1 52 | 89 | 63.9 | Mm.36683 | 0 | 90 | 90.4 | down | |
| WAN0088II_at (SEQ ID NO:2033) | BNIP2 | BCL2/adenovirus E1B 19kDa interacting protein 2 | Hs.283454 | 1E-154 | 89 | 90.3 | Mm.159777 | 0 | 94 | 96 | down | |
| WAN0088Z9_at (SEQ ID NO:2034) | PLAA | Phospholipase A2-activating protein | Hs.27182 | 1E-148 | 88 | 100 | Mm.22724 | 0 | 94 | 99 | down | |
| WAN008BRV_at (SEQ ID NO:2035) | NA | WAN008BRV 11231C-E04 | #N/A | 1E-49 | 94 | 30.1 | #N/A | 8E-99 | 95 | 55.6 | down | |
| WAN008BT4_at (SEQ ID NO:2036) | NA | WAN008BT4 11231C-A04 | #N/A | 1E-45 | 85 | 84.1 | #N/A | 8E-64 | 86 | 89 | down | |
| WAN008CF7_at (SEQ ID NO:1889) | IVNS1ABP | Influenza virus NS1A binding protein | Hs.497183 | 0 | 93 | 100 | Mm.33764 | 0 | 97 | 100 | down | |
| WAN008CLU_at (SEQ ID NO:1953) | Emp1 | Epithelial membrane protein 1 | Hs.436298 | 0 | 0 | 0 | Mm.182785 | 3E-28 | 90 | 21.7 | down | |
| WAN008CPJ_at (SEQ ID NO:1878) | Fdft1 | Famesyl diphosphate farnesyl transferase 1 | Hs.593928 | 1E-123 | 85 | 99.6 | Mm.425927 | 0 | 0 | 0 | down | |
| WAN008CTB_at (SEQ ID NO:2037) | TIPRL | TIP41, TOR signalling pathway regulator-like (S. cerevisiae) | Hs.209431 | 3E-69 | 91 | 37.7 | Mm.21520 | 1E-134 | 90 | 73.9 | down | |
| WAN008CVX_at (SEQ ID NO:1958) | CDC20 | CDC20 cell division cycle 20 homolog (S. cerevisiae) | Hs.524947 | 1E-169 | 91 | 85.2 | Mm.289747 | 0 | 92 | 87.3 | down | |
| WAN008CW2_at (SEQ ID NO:2038) | SRP54 | Signal recognition particle 54kDa | Hs.167535 | 0 | 92 | 99.6 | Mm.12848 | 0 | 93 | 99.8 | down | |
| WAN008D31_at (SEQ ID NO:1964) | Lss | Lanosterol synthase | Hs.596543 | 1E-80 | 85 | 68.3 | Mm.55075 | 1E-150 | 92 | 74 | down | |
| WAN008D40_at (SEQ ID NO:2039) | NA | WAN008D4O 10604D-C05 | #N/A | 0 | 0 | 0 | #N/A | 2E-12 | 93 | 15.3 | down | |
| WAN008DGF _x_at (SEQ ID NO:2040) | Ddx5 | DEAD (Asp-Glu-Ala-Asp) box polypeptide 5 | Hs.279806 | 6E-23 | 96 | 77.9 | Mm.220038 | 1E-34 | 97 | 100 | down | |
| WAN008DUZ_at (SEQ ID NO:1689) | POP7 | Processing of precursor 7, ribonuclease P subunit (S. cerevisiae) | Hs.416994 | 6E-52 | 90 | 35.2 | Mm.290242 | 9E-62 | 92 | 35.2 | down | |
| WAN008EOW at (SEQ ID NO:2041) | LMAN2 | Lectin, mannose-binding 2 | Hs.75864 | 7E-67 | 87 | 53.8 | Mm.38868 | 1E-130 | 89 | 88.4 | down | |
| WAN008E3R_at (SEQ ID NO:2042) | DDX41 | DEAD (Asp-Glu-Ala-Asp) box polypeptide 41 | Hs.484288 | 1E-176 | 89 | 99.3 | Mm.205045 | 0 | 95 | 100 | down | |
| WAN008E4Z_at (SEQ ID NO:1975) | Nup153 | Nucleoporin 153 | Hs.601591 | 1E-169 | 90 | 92.5 | Mm.255398 | 0 | 94 | 99.1 | down | |
| WAN008EED_at (SEQ ID NO:1521) | Sc5d | Sterol-C5-desaturase (fungal ERG3, delta-5-desaturase) homolog (S. cerevisae) | Mm.32700 | 2E-42 | 85 | 40.7 | Mm.32700 | 9E-99 | 88 | 70 | down | |
| WAN008EXX_at (SEQ ID NO:2043) | CCT4 | Chaperonin containing TCP1, subunit 4 (delta) | Hs.421509 | 1 E-48 | 89 | 55 | Mm.296985 | 3E-54 | 89 | 59.1 | down | |
| WAN008EXZ_at (SEQ ID NO:2044) | NA | WAN008EXZ 11233B-E01 | #N/A | 0 | 0 | 0 | #N/A | 4E-67 | 86 | 68.3 | down | |
| WAN013HUI_at (SEQ ID NO:2045) | HIP2 | Huntingtin interacting protein 2 | Hs.50308 | 0 | 97 | 94.8 | Mm.319512 | 0 | 97 | 99.2 | down | |
| WAN013HX4_at (SEQ ID NO:1578) | ESD | Esterase D/formylglutathione hydrolase | Hs.432491 | 1E-158 | 88 | 93.5 | Mm.38055 | 0 | 92 | 93.2 | down | |
| WAN013HY6_at (SEQ ID NO:1046) | IARS | Isoleucine-tRNA synthetase Proteasome (prosome, macropain) | Hs.445403 | 1E-127 | 86 | 100 | Mm.21118 | 0 | 92 | 99.2 | down | |
| WAN013HYE_at (SEQ ID NO:2047) | Psmd11_p redicted | 26S subunit, non-ATPase, 11 (predicted) | #N/A | 0 | 92 | 100 | #N/A | 0 | 94 | 100 | down | |
| WAN013I88_at (SEQ ID NO:2048) | NA | Cluster includes AF003836 Mesocricetus auratus isopentenyl diphosphate:dimethylallyl diphosphate isomerase mRNA, complete cds. Cluster includes AF044676 Cricetulus | #N/A | 0 | 0 | 0 | #N/A | 8E-32 | 87 | 14 | down | |
| WAN013I8A_at (SEQ ID NO:2049) | NA | griseus glucose-6-phosphate dehydrogenase mRNA, complete cds. | #N/A | 0 | 0 | 0 | #N/A | 7E-38 | 86 | 33.3 | down | |
| WAN013IW_at (SEQ ID NO:2050) | Hspa5 | Heat shock 70kD protein 5 (glucose-regulated protein) | Hs.605502 | 0 | 93 | 95.6 | Mm.330160 | 0 | 97 | 100 | down | |
| WAN013I9H_at (SEQ ID NO: 2051) | HSP9081 | Heat shock protein 90kDa beta (Grp94), member 1 | Hs.192374 | 1E-104 | 89 | 58.5 | Mm.87773 | 1E-120 | 91 | 59 | down | |
| WAN013IAD_at (SEQ ID NO:2005) | TOP2A | Topoisomerase (DNA) II alpha 170kDa | Hs.156346 | 3E-37 | 81 | 29.5 | Mm.4237 | 1E-86 | 84 | 37.6 | down | |
| AF221841 at (SEQ ID NO:1735) | Prdx1 | Peroxiredoxin 1 | Hs.180909 | 0 | 91 | 100 | Mm.30929 | 0 | 94 | 98.5 | up | |
| WAN008DI8_at (SEQ ID NO:2052) | Lmna | Lamin A | #N/A | 0 | 0 | 0 | Mm.243014 | 0 | 93 | 99.8 | up | |
| WAN008DXT_at (SEQ ID NO:2053) | SUCLA2 | Succinate-CoA ligase, ADP-forming, beta subunit | Hs.546323 | 2E-54 | 94 | 34.7 | Mm.38951 | 4E-60 | 96 | 33 | up | |
| *Low Lactate Producer* | | | | | | | | | | | | |
| Qualifier List | Symbol | Title | Human | eValue | %ID | %QC | Mouse | eValue | %ID | %QC | FC | Function |
| | | | Unigene ID | | | | Unigene ID | | | | | |
| AF022942_at (SEQ ID NO:2008) | Cirbp | Cold inducible RNA binding protein | Hs.634522 | 8E-40 | 86 | 86.5 | Mm.17898 | 9E-94 | 95 | 100 | up | |
| M26640_at (SEQ ID NO:2054) | CLU | Clusterin | Hs.436657 | 7E-92 | 83 | 94.6 | Mm.200608 | 0 | 92 | 98.8 | up | |
| WAN00880Y_x_at (SEQ ID NO:2024) | Invs | Inversin | Hs.558477 | 1E-87 | 99 | 95.8 | Mm.317706 | 1E-98 | 100 | 100 | down | |
| WAN008CLU_at (SEQ ID NO:1953) | Emp1 | Epithelial membrane protein 1 | Hs.436298 | | 0 | 0 | Mm.182785 | 3E-28 | 90 | 21.7 | down | |
| WAN008EED_at (SEQ ID NO:1521) | Sc5d | Sterol-C5-desaturase (fungal ERG3, delta-5-desaturase) homolog (S. cerevisae) | #N/A | 2E-42 | 85 | 40.7 | Mm.32700 | 9E-99 | 88 | 70 | down | |
| L00332_at (SEQ ID NO:1862) | HMGCS1 | 3-hydroxy-3-methylglutaryl-Coenzyme A synthase 1 (soluble) | Hs.397729 | 3E-41 | 91 | 95.8 | Mm.61526 | 1E-42 | 92 | 88.9 | down | |
| *Multiple Categories* | | | | | | | | | | | | |
| Qualifier List | Symbol | Title | Human Unigene ID | eValue | %ID | %QC | Mouse Unigene ID | eValue | %ID | %QC | FC | Function |
| WAN0088K2_at (SEQ ID NO:1945) | DUSP16 | Dual specificity phosphatase 16 | Hs.536535 | 2E-05 | 84 | 16 | Mm.3994 | 4E-21 | 88 | 22.7 | down | |
| WAN0088JV_at (SEQ ID NO:1555) | TRIB3 | Tribbles homolog 3 (Drosophila) | Hs.516826 | 4E-62 | 82 | 86.4 | Mm.276018 | 1E-158 | 89 | 98.1 | down | |
| WAN0088OP_at (SEQ ID NO:2055) | Hrb2 | HIV-1 Rev binding protein 2 | #N/A | 1E-141 | 88 | 91.2 | #N/A | 0 | 91 | 99.4 | down | |
| WAN008BSH_at (SEQ ID NO:1558) | CAT | Catalase | Hs.502302 | 6E-16 | 89 | 38.7 | Mm.4215 | 3E-41 | 89 | 85.7 | down | reactive oxygen species |
| WAN008BSL_at (SEQ ID NO:2056) | PAK1IP1 | PAK1 interacting protein 1 | Hs.310231 | 1E-109 | 90 | 86.1 | Mm.24789 | 4E-91 | 86 | 99.7 | down | |
| WAN008CIU_at (SEQ ID NO:2018) | NA | WAN008CIU 10599D-C10 | #N/A | 0 | 0 | 0 | #N/A | 1E-05 | 100 | 5.79 | down | |
| WAN008CZ6_at (SEQ ID NO:2057) | 2010106G 01Rik | RIKEN cDNA2010106G01 gene | #N/A | 1E-125 | 86 | 99.2 | Mm.269928 | 1E-164 | 89 | 100 | down | |
| WAN008E89_at (SEQ ID NO:1900) | Nup160 | Nucleoporin 160 | Hs.645358 | 0 | 92 | 98.1 | Mm.24532 | 0 | 94 | 99.8 | down | |
| WAN008EC4_at (SEQ ID NO:1058) | HIAT1 | Hippocampus abundant transcript 1 | Hs.124156 | 1E-140 | 93 | 66 | Mm.280077 | 1E-155 | 95 | 66 | down | |
| WAN013HX1_at (SEQ ID NO:2059) | SEC13L1 | SEC13-like 1 (S. cerevisiae) | Hs.166924 | 0 | 90 | 99.3 | Mm.29296 | 0 | 90 | 99.3 | down | |
| WAN013HXZ_x_at (SEQ ID NO:2060) | NA | Cluster includes WAN008DCG 11165C-D11 | #N/A | 6E-22 | 91 | 46.1 | #N/A | 5E-30 | 97 | 30.6 | down | |
| WAN013I05_at (SEQ ID NO: 1646) | Abcb6 | ATP-binding cassette, sub-family B (MDR/TAP), member 6 | Hs.107911 | 1E-154 | 87 | 100 | Mm.28663 | 0 | 92 | 100 | down | |
| WAN013I0B_at (SEQ ID NO:2061) | NA | Cluster includes WAN008E5C 11230A-B11 | #N/A | 3E-07 | 83 | 15.6 | #N/A | 6E-22 | 86 | 17.1 | down | |
| WAN013I1D_x_at (SEQ ID NO:2062) | CCNDBP1 | Cyclin D-type binding-protein 1 | Hs.36794 | 2E-67 | 79 | 87 | Mm.7838 | 1E-111 | 82 | 95.1 | down | |
| WAN013I1Z_f_at | NA | Cluster includes WAN0088KD | #N/A | 0 | 0 | 0 | #N/A | 0 | 0 | 0 | up | |
| (SEQ ID NO:2063) | | 10595B-H02 V-maf musculoaponeurotic | | | | | | | | | | |
| WAN013I20_x_at (SEQ ID NO:2064) | MAFG | fibrosarcoma oncogene homolog G (avian) | Hs.252229 | 7E-39 | 83 | 30.8 | Mm.268010 | 2E-50 | 82 | 42.7 | down | |
| WAN013I31_at (SEQ ID NO:2065) | RNF4 | Ring finger protein 4 | Hs.66394 | 8E-67 | 90 | 47.8 | Mm.21281 | 1E-178 | 92 | 95.4 | down | |
| X51747_at (SEQ ID NO:1587) | HSPB1 | Heat shock 27kDa protein 1 | Hs.520973 | 1E-101 | 87 | 50.5 | Mm.13849 | 0 | 92 | 66.1 | up | |
| WAN008DGD_at (SEQ ID NO:1564) | ApIp2 | Amyloid beta (A4) precursor-like protein 2 (ApIp2) | #N/A | | | | Mm.19133 | 7E-69 | 93 | 44.5 | -1.3 | migration; adhesion |
| U42430_at (SEQ ID NO:1673) | CD36 | CD36 antigen (collagen type I receptor, thrombospondin receptor) | Hs.120949 | 6E-43 | 86 | 34.3 | Mm.18628 | 2E-57 | 88 | 37.7 | -1.49 | |
| L00181_at (SEQ ID NO:1510) | Hmgcr | 3-hydroxy-3-methylglutaryl-Coenzyme A reductase | Hs.643495 | 1E-37 | 90 | 32.7 | Mm.316652 | 3E-52 | 94 | 33.7 | down | |
| U22819_s_at (SEQ ID NO:1927) | SREBF2 | Sterol regulatory element binding transcription factor 2 | Hs.443258 | 1E-118 | 90 | 99.5 | Mm.38016 | 1E-133 | 92 | 97 | down | |
| L00366_x_at (SEQ ID NO:1941) | TK1 | Thymidine kinase 1, soluble | Hs.515122 | 4E-18 | 90 | 84.9 | Mm.2661 | 1E-16 | 89 | 86 | up | |

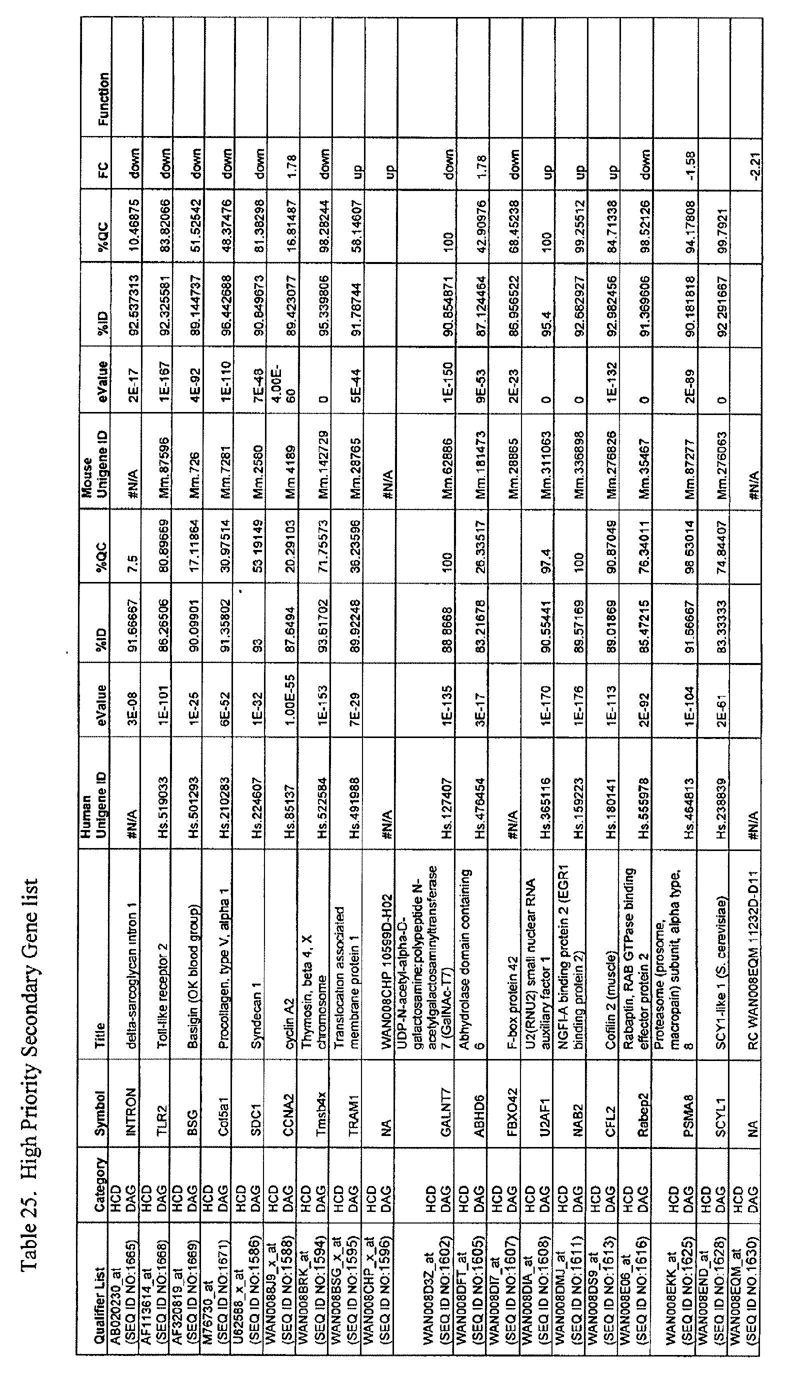

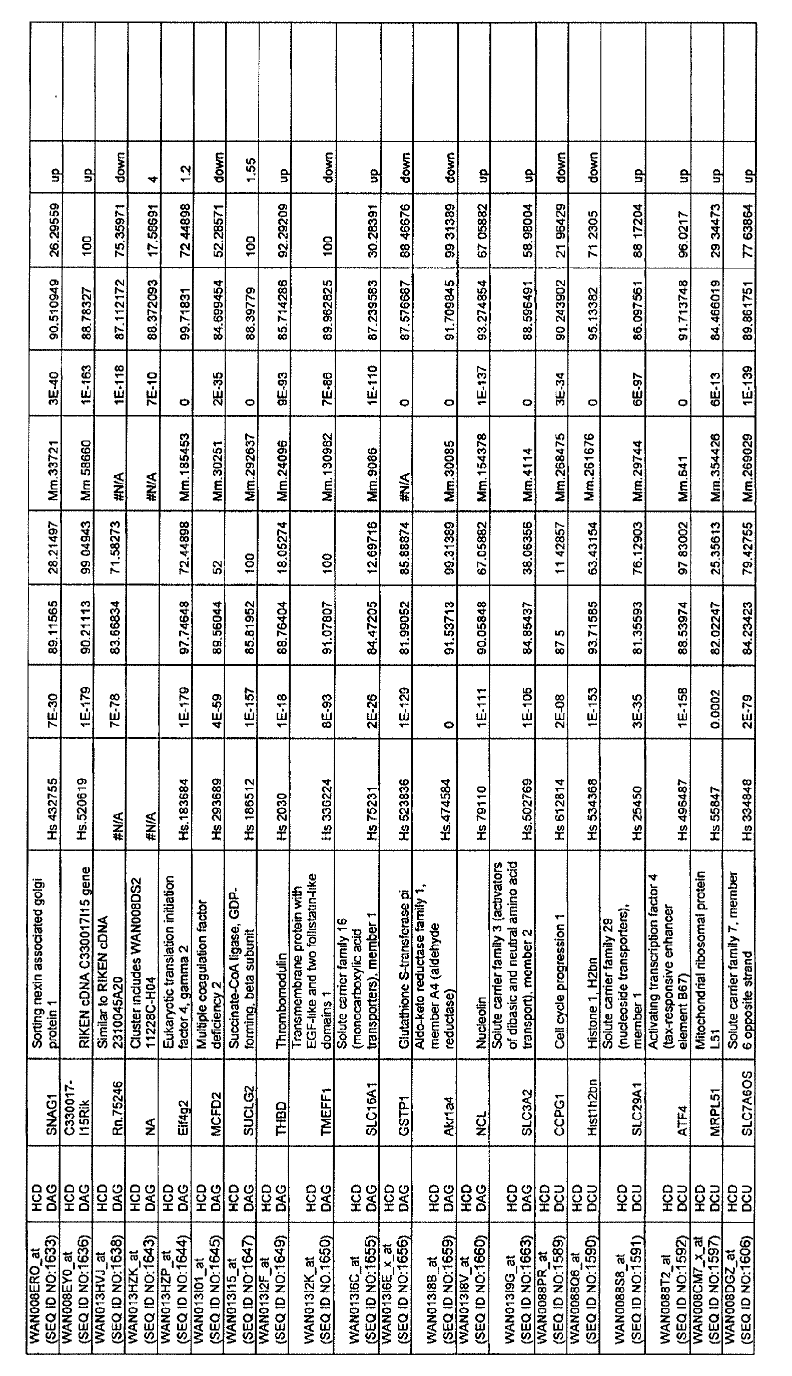

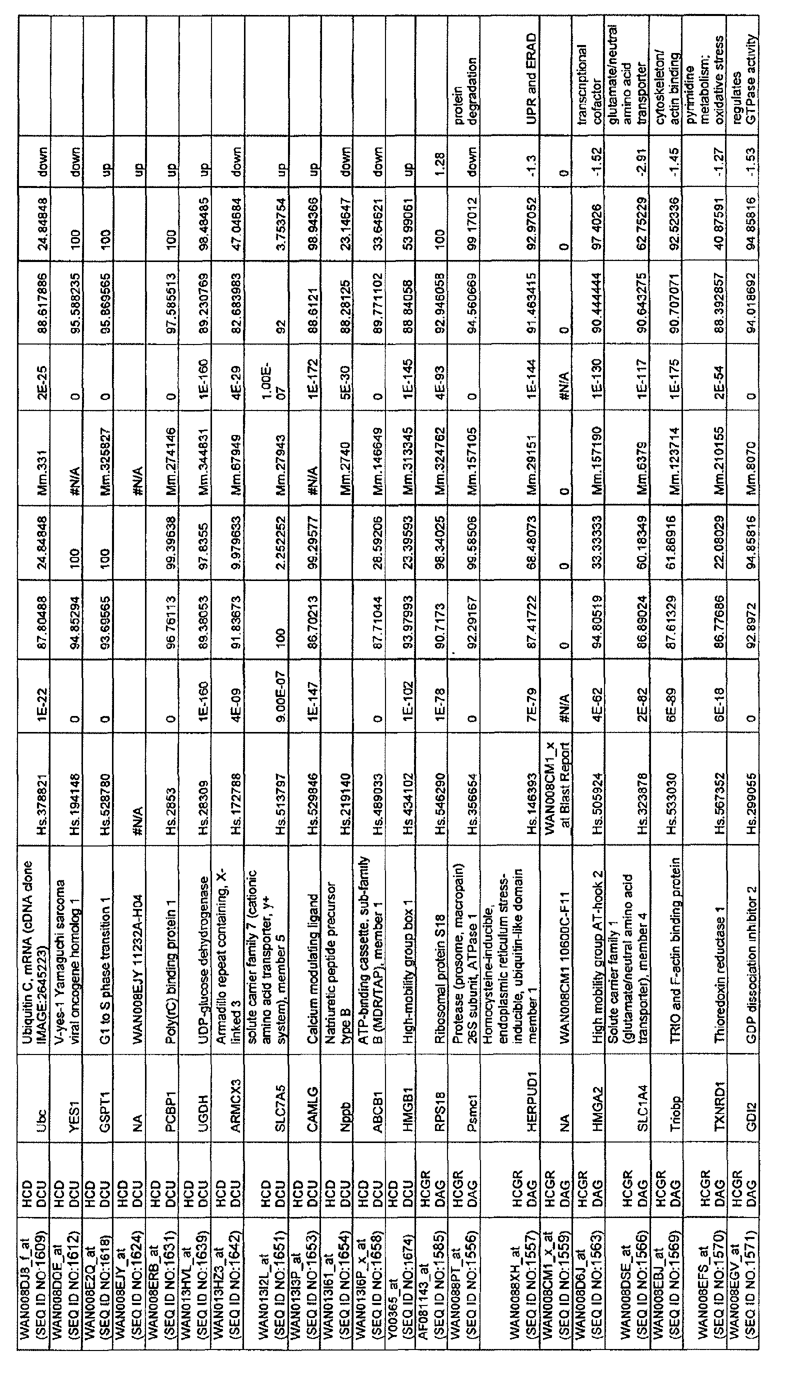

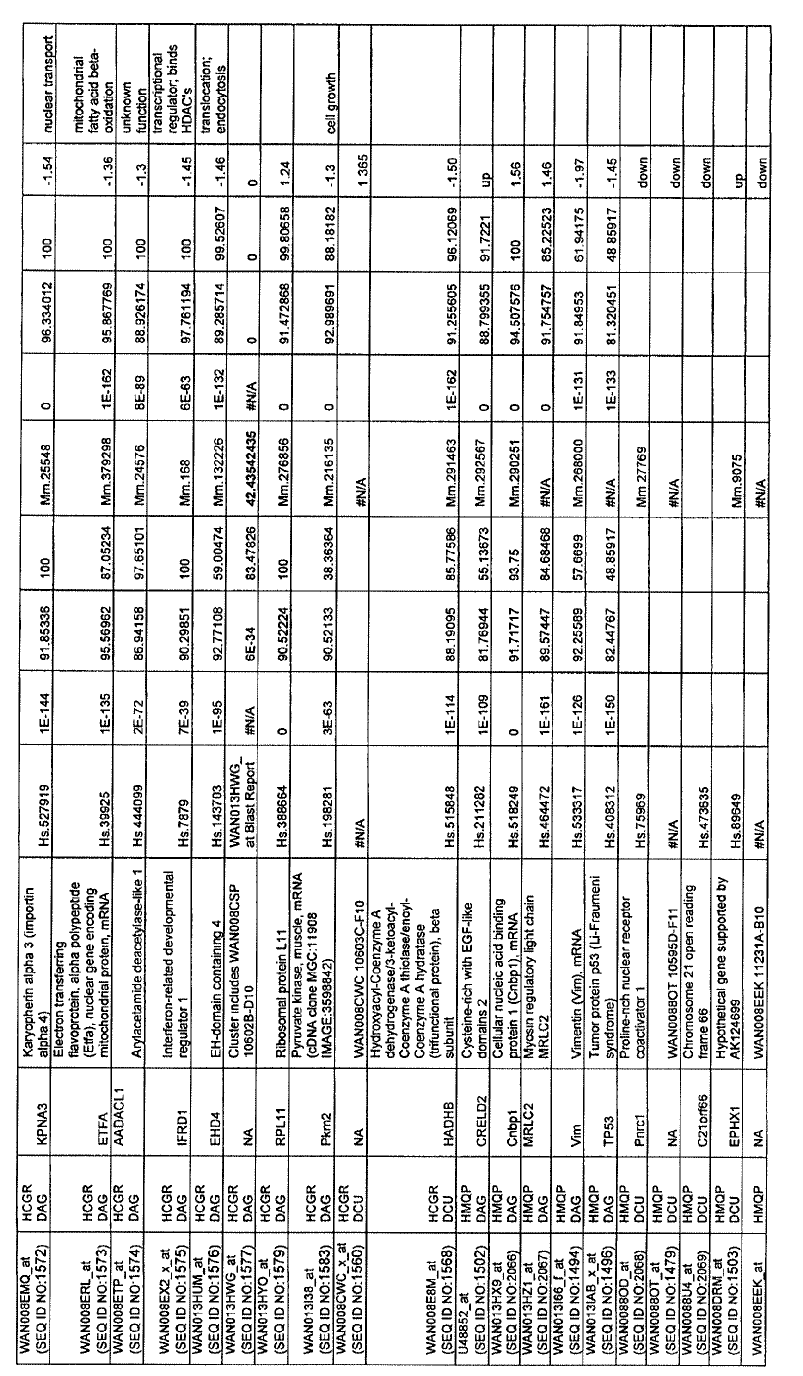

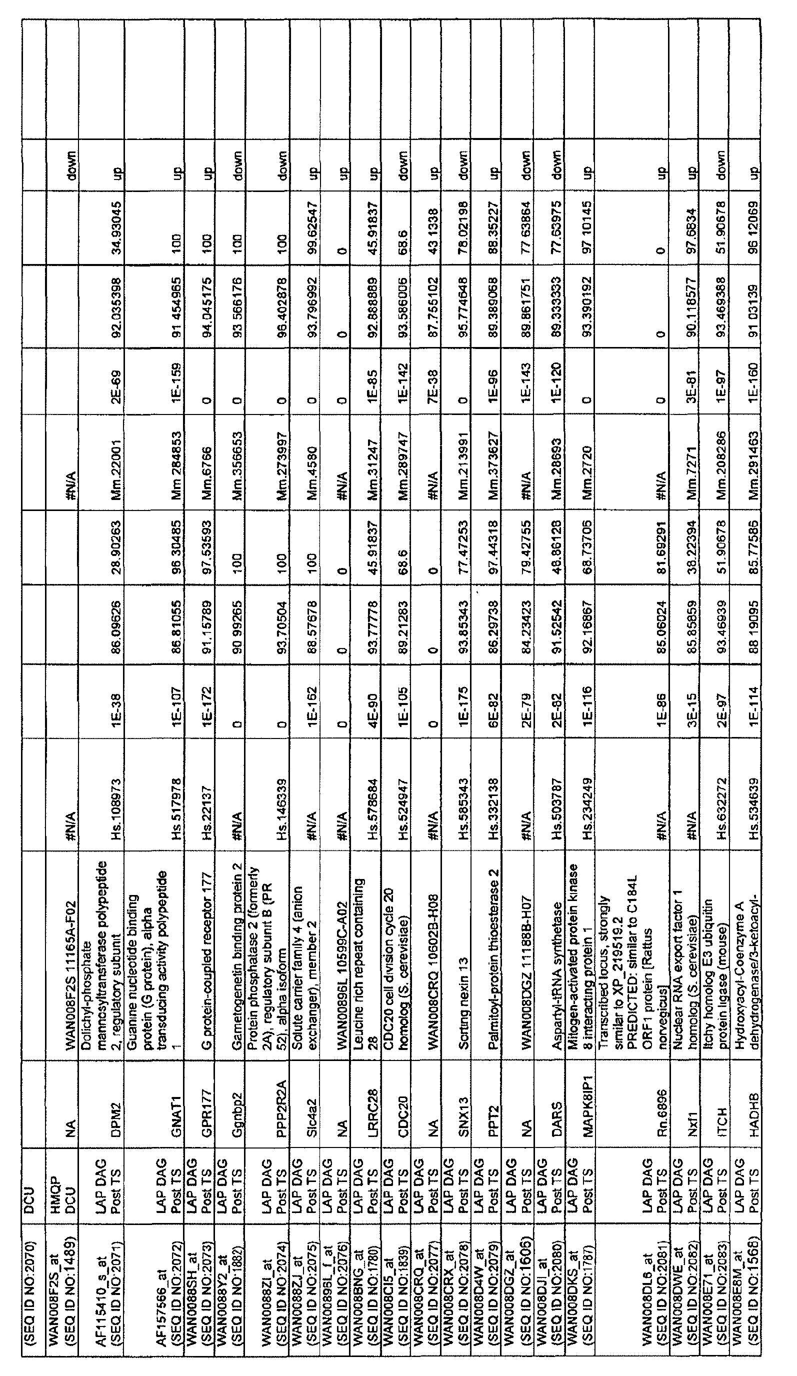

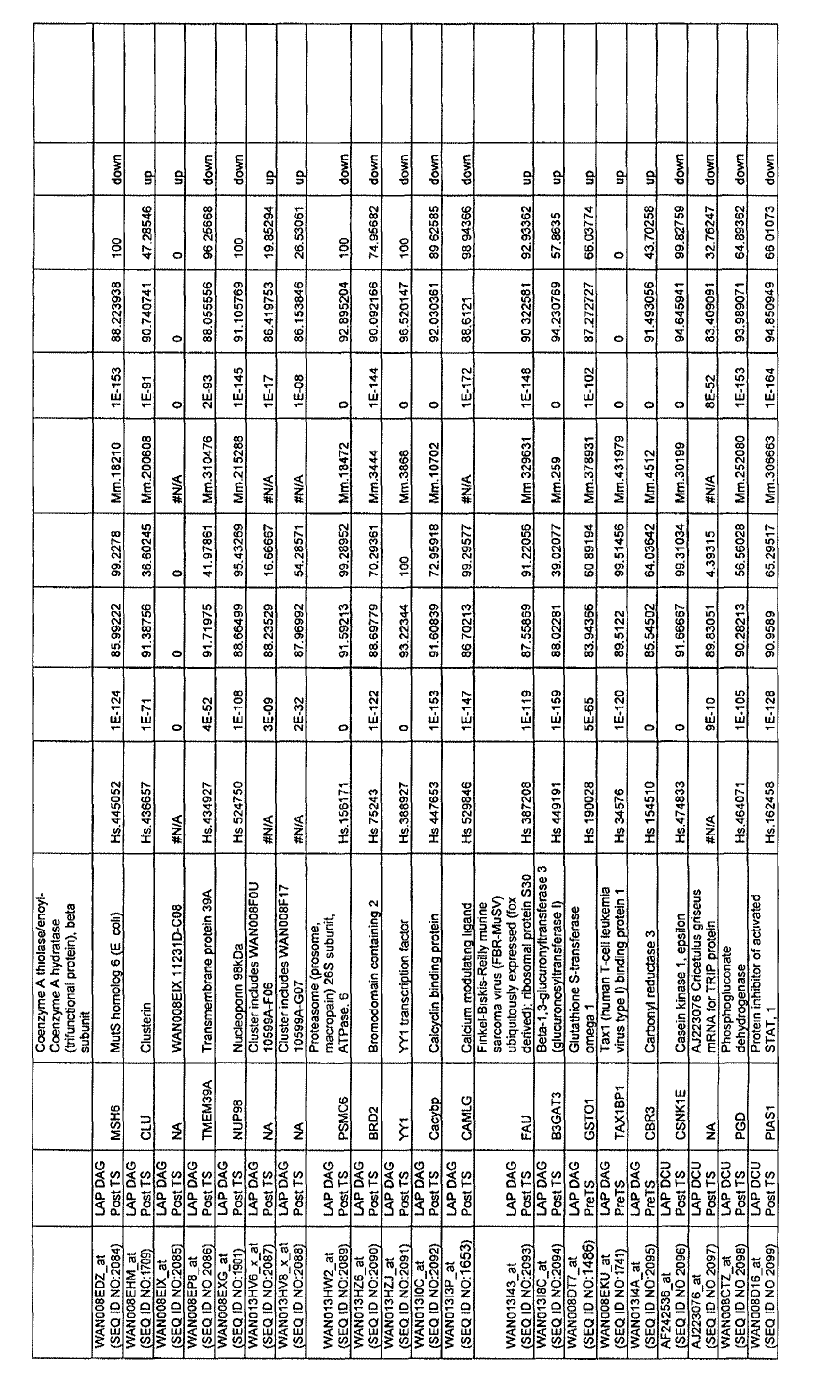

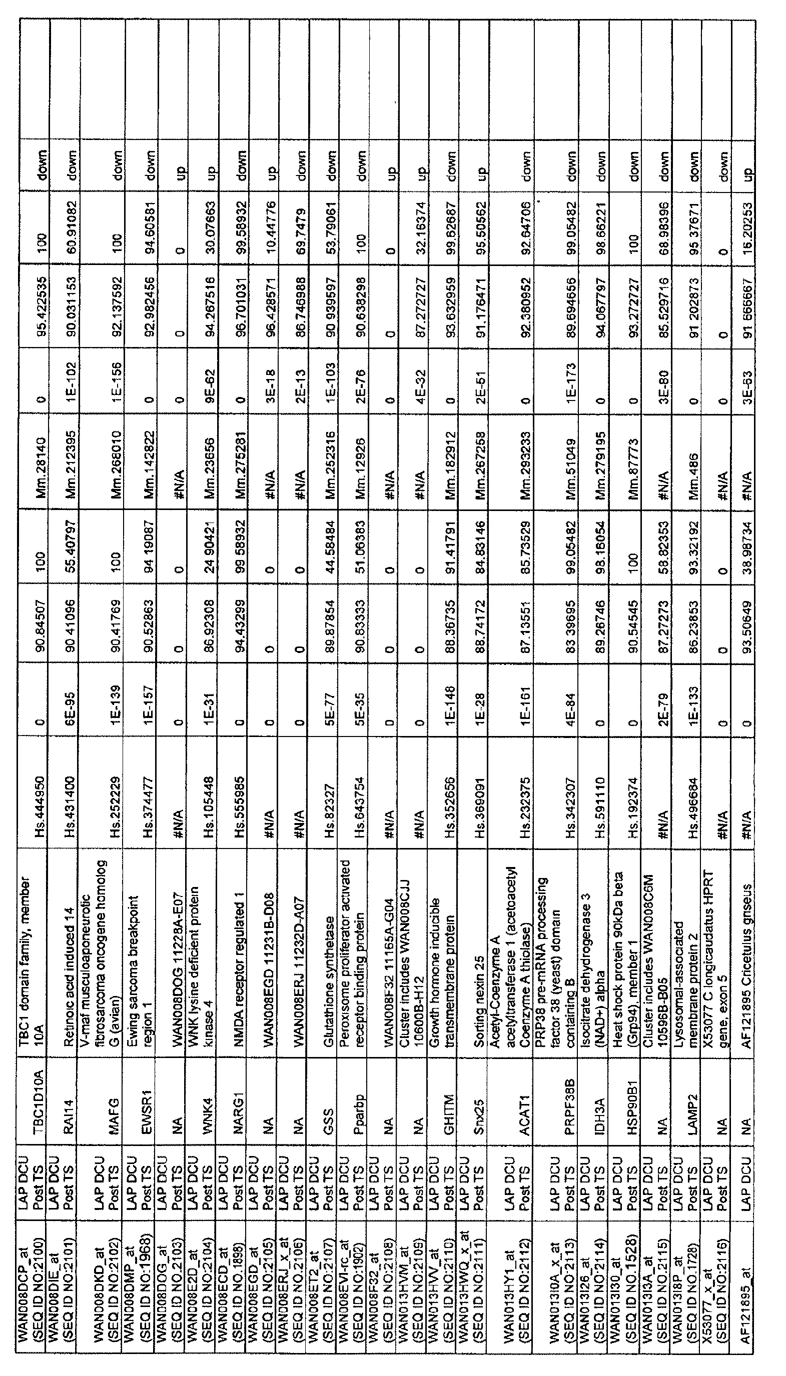

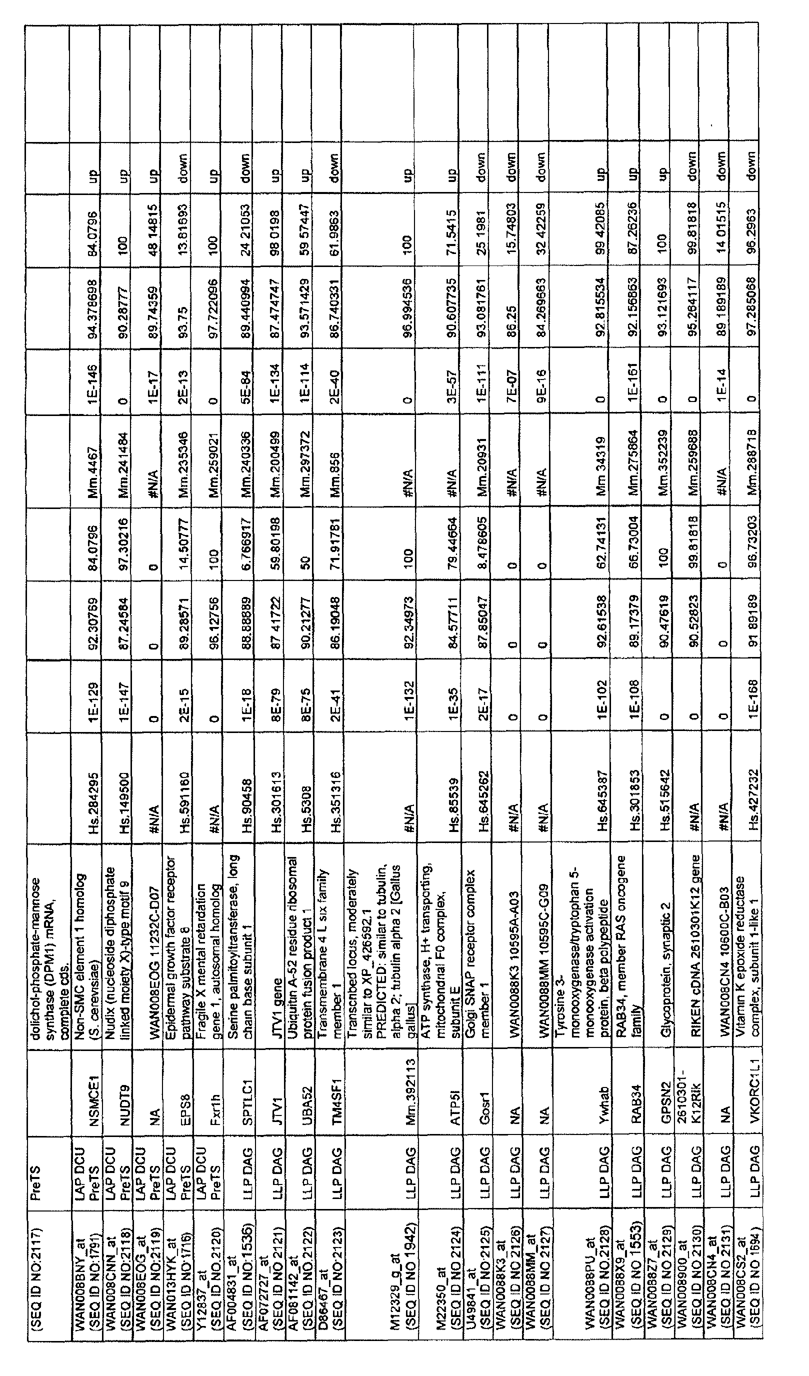

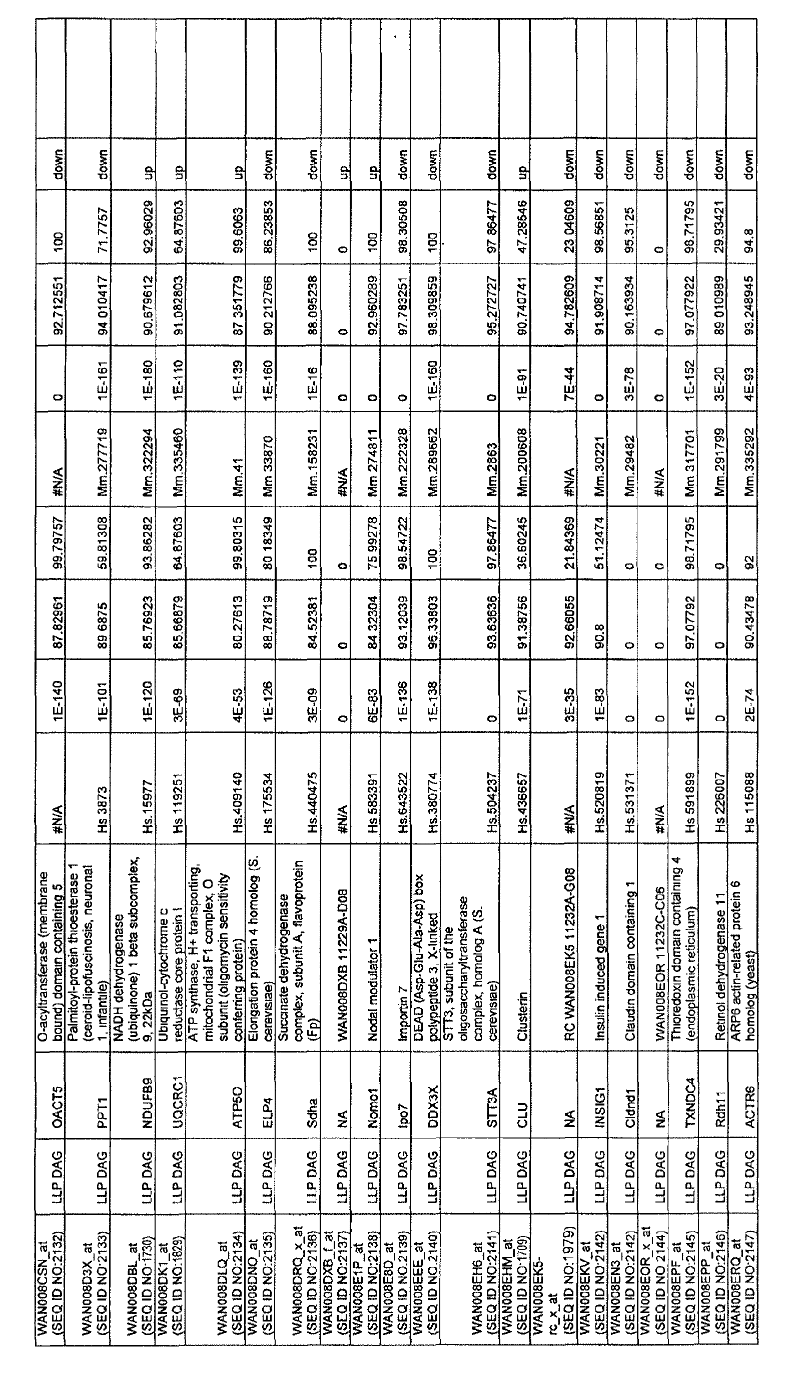

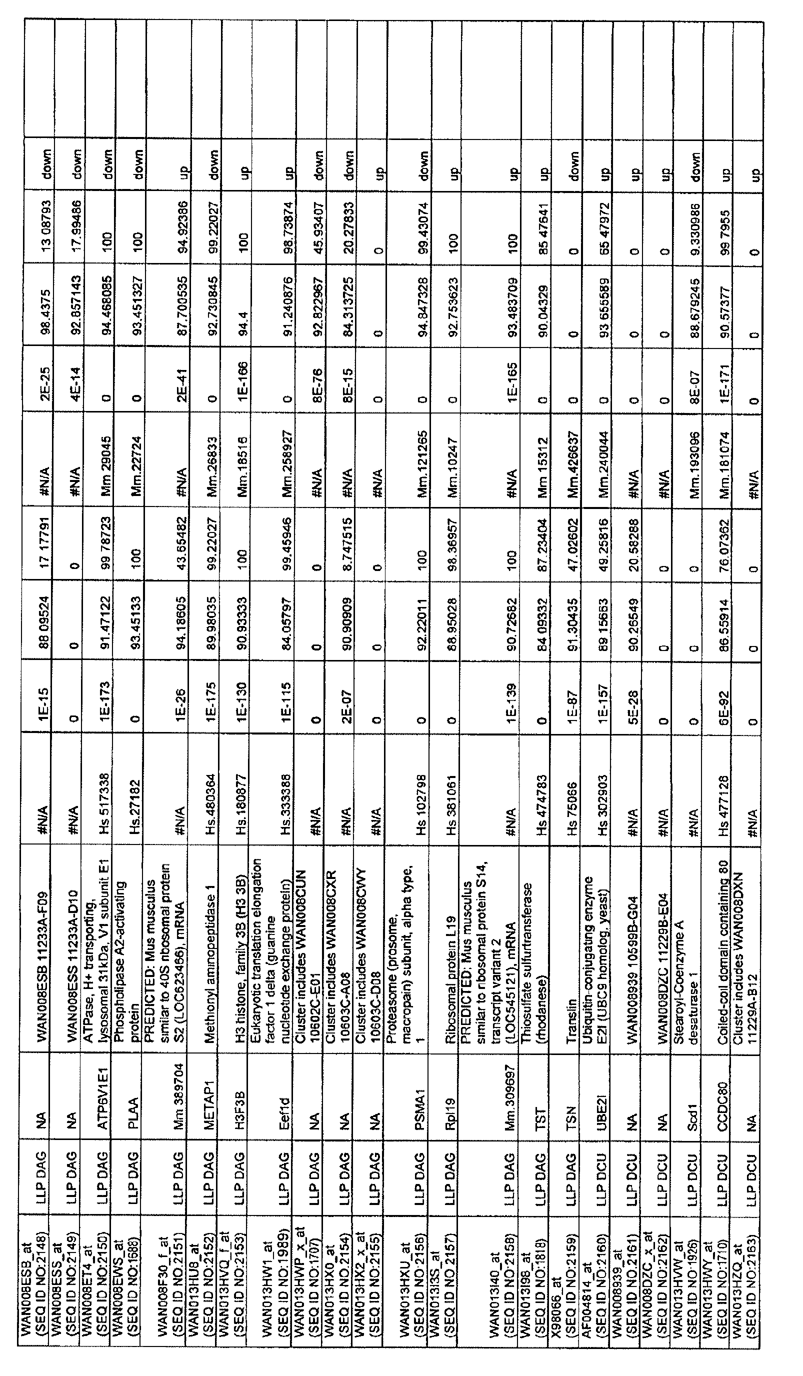

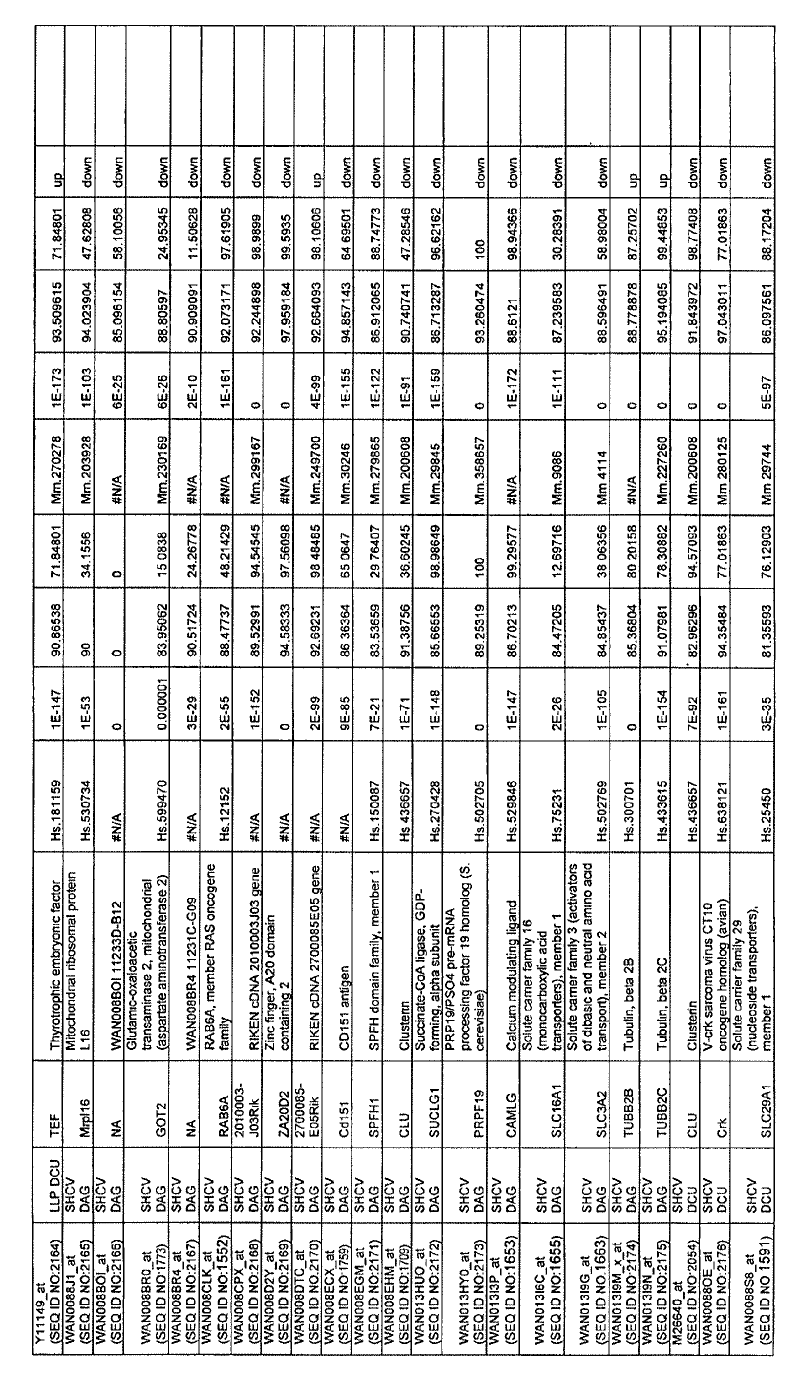

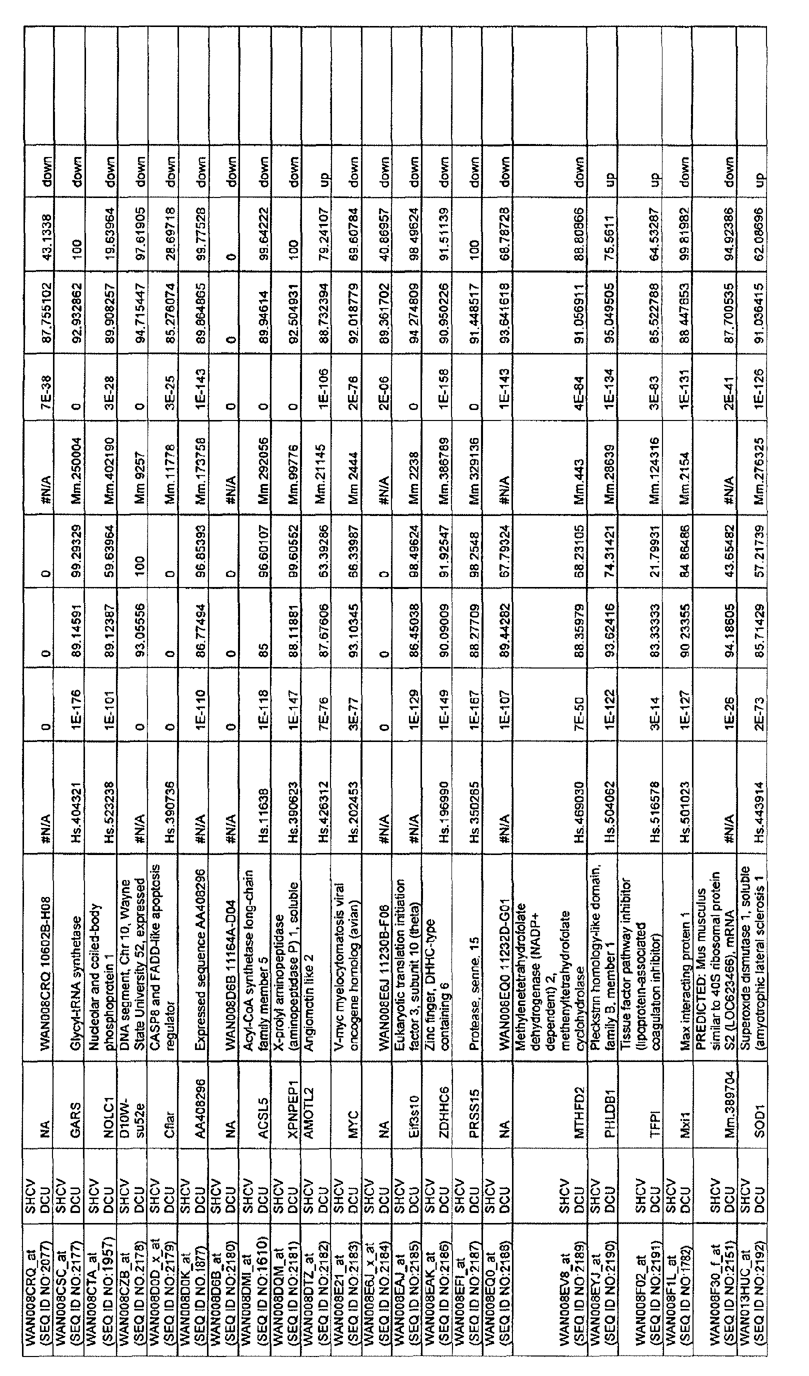

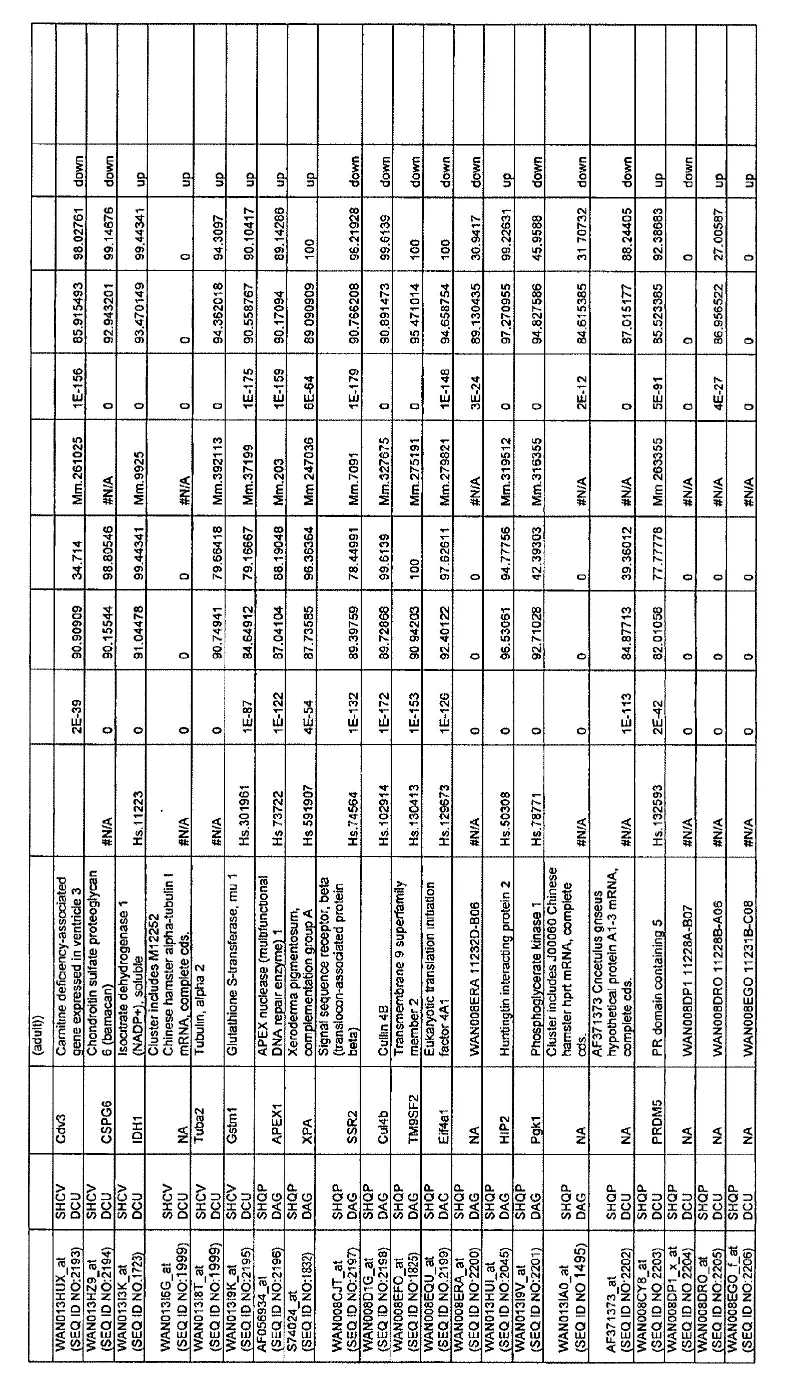

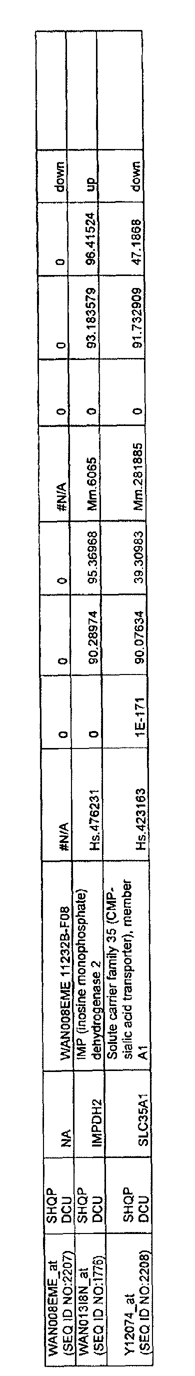

**Table 26. High Priority Gene list 3**

| **Qualifier List** | **Category** | **Symbol** | **Title** | **Human Unigene ID** | **eValue** | **%ID** | **%QC** | **Mouse Unigene ID** | **eValue** | **%ID** | **%QC** | **FC** | **Function** |
|---|---|---|---|---|---|---|---|---|---|---|---|---|---|
| L00176_at (SEQ ID NO:1500) | HMQP DAG | HMGCR | 3-hydroxy-3-methylglutaryl-Coenzyme A reductase | Hs.11899 | 6E-54 | 87.98077 | 57.93872 | Mm.316652 | 4E-82 | 94.47236 | 55.43175 | up | |
| WAN0088ZP_at (SEQ ID NO:1482) | HMQP DAG | PAWR | PRKC, apoptosis. WT1, regulator | Hs 406074 | 4E-10 | 91.52542 | 11.11111 | Mm.336104 | 9E-53 | 91.62562 | 38.22976 | up | |
| WAN008CJ1_at (SEQ ID NO:1485) | HMQP DAG | ERP70 | Protein disulfide isomerase-associated 4 | Hs 93659 | 1E-120 | 89.62766 | 81 91721 | Mm 2442 | 1E-170 | 94 57364 | 84 31373 | up | |
| WAN008EA0_at (SEQ ID NO:1487) | HMQP DAG | VCP | Valosin-containing protein | Hs.529782 | 0 | 90.72727 | 99.63768 | Mm 379457 | 0 | 95.47101 | 100 | up | |
| D45419_at (SEQ ID NO.1498) | HMQP DCU | Hcfc1 | Host cell factor C1 | Hs.83634 | 1E-22 | 84.86486 | 32.74336 | Mm 379457 Mm.248353 | 1E-123 | 95.47101 85.99291 | 99.82301 | down | transcription factor |
| WAN0088NU_at (SEQ ID NO:1478) | HMQP DCU | LMNA | Lamin A/C | Hs.491359 | 3E-23 | 85.71429 | 23.05026 | Mm.243014 | 0 | 92.04947 | 98.09359 | down | |
| WAN008F11_at (SEQ ID NO:1488) | | SHOC2 | Soc-2 suppressor of clear homolog (C. elegans) | Hs.104315 | 2E-18 | 85.05747 | 31.07143 | Nm.228669 | 1E-36 | 90.37037 | 24.10714 | -2.62 | |
| WAN013I1P_at (SEQ ID NO 1492) | | HNRPA2B1 | Heterogeneous nuclear ribonucleoprotein A2/B1 | Hs 487774 | 0 | 97.22222 | 90.94737 | Mm.155896 | 0 | 96.52778 | 90.94737 | -3.49 | pre-mRNA processing |
| WAN008DT7_at (SEQ ID NO: 1486) | | GSTO1 | Glutathione S-transferase omega 1 | Hs.190028 | 5E-65 | 83.94366 | 60.89194 | Mm.378931 | 1E-102 | 87.27273 | 66.03774 | -211 | redox homeostasis; stress response |

**Table 27. HCGR 2+3 Overlap 1.2F UP (10)**

| **Qualifier List** | **Symbol** | **Title** | **Human Unigene ID** | **eValue** | **%ID** | **%QC** | **Mouse Unigene ID** | **eValue** | **%ID** | **%QC** |
|---|---|---|---|---|---|---|---|---|---|---|
| WAN013I0S_at (SEQ ID NO:2209) | 4930548G07Rik | RIKEN cDNA 4930548G07 gene | #N/A | 4E-67 | 87.21804511 | 99.25373134 | Mm.152466 | 1E-104 | 92.91044776 | 100 |
| WAN0088WF_at (SEQ ID NO:1874) | DHX36 | DEAH (Asp-Glu-Ala-His) box polypeptide 36 | Hs.446270 | 2E-97 | 92-0754717 | 55.67226891 | Mm.224233 | 1 E-113 | 94.40298507 | 56.30252101 |
| WAN008EVU_x_at (SEQ ID NO:1887) | HNRPK | Heterogeneous nuclear ribonucleoprotein K | Hs.522257 | 1E-129 | 92.98780488 | 59.63636364 | Mm.142872 | 1E-170 | 90.58380414 | 96.54545455 |
| X51747_at (SEQ ID NO:1587) | HSPB1 | Heat shock 27kDa protein 1 | Hs.520973 | 1E-101 | 87.36842105 | 50.53191489 | Mm.13849 | 0 | 91.95171026 | 66.09042553 |
| WAN008D3S_x_at (SEQ ID NO:2210) | IDE | Insulin-degrading enzyme | Hs.500546 | 0.0000001 | 89.65517241 | 29.14572864 | Mm.28366 | 8E-20 | 90.69767442 | 43.2160804 |
| U62587_at (SEQ ID NO:2211) | MGAT5 | Mannosyl (alpha-1,6-)-glycoprotein beta-1,6-N-acetyl-glucosaminyltransferase | Hs.22689 | 1E-160 | 87.03703704 | 100 | Mm.214682 | 0 | 92.92929293 | 100 |
| AF306662_at (SEQ ID NO:1729) | MMP14 | Matrix metallopeptidase 14 (membrane-inserted) | Hs.2399 | 0 | 89.7260274 | 99.82905983 | Mm.280175 | 0 | 90.08547009 | 100 |
| WAN013I63_at (SEQ ID NO:2212) | RPS15 | Ribosomal protein S15 | Hs.406683 | 1E-109 | 92.15017065 | 61.04166667 | Mm.643 | 1E-108 | 92.5795053 | 58.95833333 |
| WAN008BRK_at (SEQ ID NO:1594) | Tmsb4x | Thymosin, beta 4, X chromosome | Hs.522584 | 1E-153 | 93.26683292 | 76.52671756 | Mm.142729 | 0 | 95.53398058 | 98.28244275 |
| WAN013I9L_x_at (SEQ ID NO:2213) | UBB | Ubiquitin B | Hs.356190 | 3E-22 | 91.17647059 | 21.07438017 | Mm.282093 | 4E-26 | 90.47619048 | 21.69421488 |

**Table 28. HCGR 2+3 Overlap 1.2F DOWN (115)**

| **Qualifier List** | **Symbol** | **Title** | **Human Unigene ID** | **eValue** | **%ID** | **%QC** | **Mouse Uniqene ID** | **eValue** | **%ID** | **%QC** |
|---|---|---|---|---|---|---|---|---|---|---|
| WAN008CPA_at (SEQ ID NO:2214) | 1200007-B05Rik | RIKEN cDNA 1200007B05 gene | #N/A | 9E-84 | 92.79279279 | 61.66666667 | Mm.23896 | 1E-117 | 91.5451895 | 95.27777778 |
| WAN008DFA_at (SEQ ID NO:2215) | 2310042-G06Rik | RIKEN cDNA 2310042G06 gene | #N/A | 1E-163 | 88.51851852 | 97.12230216 | Mm.182294 | 0 | 93.22344322 | 98.20143885 |
| WAN008DI2_at (SEQ ID NO:2216) | 2510049-119Rik | RIKEN cDNA 2510049119 gene | #N/A | 3E-65 | 91.97860963 | 60.12861736 | Mm.28327 | 2E-95 | 92.20779221 | 99.03536977 |
| WAN008D0K_at (SEQ ID NO:1877) | AA408296 | Expressed sequence AA408296 | #N/A | 1E-110 | 86.774942 | 96.85393258 | Mm.173758 | 1E-143 | 89.86486486 | 99.7752809 |
| WAN008CWO_at (SEQ ID NO:2217) | AKAP12 | A kinase (PRKA) anchor protein (qravin) 12 | Hs.371240 | 2E-14 | 85.57692308 | 25.93516209 | Mm.27481 | 2E-37 | 90.44117647 | 33.91521197 |
| WAN01319E_at (SEQ ID NO:2218) | Akr1b8 | Aldo-keto reductase family 1, member B8 | #N/A | 1E-136 | 87.52556237 | 43.00791557 | Mm.5378 | 0 | 89.56989247 | 81.79419525 |
| WAN0088LT_at (SEQ ID NO:2219) | ALAS1 | Aminolevulinate, delta-, synthase 1 | Hs.476308 | 7E-95 | 85.8190709 | 76.5917603 | Mm.290578 | 1E-166 | 91.52892562 | 90.63670412 |
| WAN008DGD_at (SEQ ID NO: 1564) | Aplp2 | synthase 1 Amyloid beta (A4) precursor-like protein 2 | Hs.370247 | 0 | 0 | 0 | Mm.19133 | 6E-69 | 93.0875576 | 44.46721311 |
| WAN013I8H_x_at (SEQ ID NO:1548) | APP | Amyloid beta (A4) precursor protein (peptidase nexin-II, Alzheimer disease) | Hs.642685 | 1 E-77 | 83.89830508 | 83.53982301 | Mm.277585 | 1E-167 | 87.78761062 | 100 |
| WAN0088T2_at (SEQ ID NO:1592) | ATF4 | Activating transcription factor 4 (tax-responsive enhancer element B67) | Hs.496487 | 1E-158 | 88.53974122 | 97.83001808 | Mm.641 | 0 | 91.71374765 | 96.02169982 |
| WAN008DS1_at (SEQ ID NO:2220) | ATXN10 | Ataxin 10 | Hs.475125 | 1E-107 | 87.05035971 | 82.24852071 | Mm.248906 | 1E-170 | 91.32149901 | 100 |
| WAN0088OB_at (SEQ ID NO:2221) | BC017158 | CDNA sequence BC017158 | #N/A | 2E-69 | 84.3575419 | 99.44444444 | Mm.38870 | 1E-142 | 92.7777778 | 100 |
| WAN013HXN_at (SEQ ID NO:2222) | CAT | Catalase | Hs.502302 | 2E-71 | 92.74611399 | 33.80035026 | Mm.4215 | | 90.03831418 | 91.41856392 |
| WAN008DU8_at (SEQ ID NO:2223) | CCM2 | Cerebral cavernous malformation 2 | Hs.148272 | 1 E-139 | 93.10344828 | 75.16198704 | Mm.221271 | 1E-162 | 95.23809524 | 77.10583153 |
| WAN01318J_at (SEQ ID NO:2001) | CCNB2 | Cyclin B2 | Hs.194698 | 1 E-173 | 86.92579505 | 44.39215686 | Mm.22592 | 0 | 90.70945946 | 46.43137255 |
| WAN0088YD_at (SEQ ID NO:2224) | CCS | Copper chaperone for superoxide dismutase | Hs.502917 | 4E-96 | 83.89662028 | 90.4676259 | Mm.426068 | 0.00005 | 96.66666667 | 5.395683453 |
| U42430_at (SEQ ID NO:1673) | CD36 | CD36 molecule (thrombospondin receptor) | Hs.120949 | 6E-43 | 86.36363636 | 34.25605536 | Mm.18628 | 2E-57 | 88.0733945 | 37.71626298 |
| WAN013HYB_at (SEQ ID NO:2225) | CLIC1 | chloride intracellular channel 1 | Hs.414565 | 1E-114 | 87.5 | 98.9010989 | Mm.29524 | 1E-130 | 89.28571429 | 100 |
| WAN008D27_at (SEQ ID NO:1760) | CLTA | Clathrin, light polypeptide (Lca) | Hs.522114 | 0 | 94.06307978 | 99.26335175 | Mm.298875 | 0 | 94.83394834 | 99.81583794 |
| WAN0088NS_at (SEQ ID NO:2226) | CNOT6L | CCR4-NOT transcription complex, subunit 6-like | Hs.591695 | 1E-113 | 97.43589744 | 50.86956522 | Mm.28374 | 1E-106 | 98.13953488 | 46.73913043 |
| WAN008CST_at (SEQ ID NO:1711) | COPS2 | COP9 constitutive photomorphogenic homolog subunit 2 (Arabidopsis) | Hs.369614 | 0 | 94.16058394 | 100 | Mm.3596 | 0 | 96.89781022 | 100 |
| WAN008EEB_at (SEQ ID NO:1762) | CORO1B | Coronin, actin binding protein, 1B | Hs.6191 | 2E-95 | 90.34482759 | 55.98455598 | Mm.276859 | 1E-128 | 94.61279461 | 57.33590734 |
| AY011645_at (SEQ ID NO:2227) | CREM | CAMP responsive element modulator | Hs.200250 | 9E-44 | 94.02985075 | 37.4301676 | Mm.5244 | 1E-149 | 93.82022472 | 99.44134078 |
| WAN013I6I_at (SEQ ID NO:2228) | CRYAB | Crystallin, alpha B | Hs.408767 | 1E-113 | 86.59793814 | 97.97979798 | Mm.178 | 1E-124 | 86.80203046 | 99.49494949 |
| WAN008DS3_at (SEQ ID NO:2229) | CTNNBL1 | Catenin, beta like 1 | Hs.472667 | 1E-167 | 89.05660377 | 99.06542056 | Mm.45193 | 0 | 90.8411215 | 100 |
| WAN013121_at (SEQ ID NO:2230) | Cycs | Cytochrome c, somatic | Hs.437060 | 1E-103 | 89.21832884 | 73.3201581 | Mm.35389 | 0 | 95.86956522 | 90.90909091 |
| M29238_at (SEQ ID NO:2030) | DDIT3 | DNA-damage-inducible transcript 3 | Hs.505777 | 1E-100 | 87.08971554 | 76.80672269 | Mm.110220 | 1E-119 | 89.26829268 | 68.90756303 |
| WAN008ERF_at (SEQ ID NO:2231) | DHCR7 | 7-dehydrocholesterol reductase | Hs.503134 | 2E-27 | 79.9382716 | 70.74235808 | Mm.249342 | 1E-147 | 90.02217295 | 98.47161572 |
| WAN013HUM_at (SEQ ID NO:1576) | EHD4 | EH-domain containing 4 | Hs.143703 | 1E-95 | 92.77108434 | 59.00473934 | Mm.132226 | 1E-125 | 88.57142857 | 99.52606635 |
| WAN008E8R_at (SEQ ID NO:1767) | EIF3S1 | Eukaryotic translation initiation factor 3, subunit 1 alpha, 35kDa | Hs.404056 | 1E-122 | 93.1372549 | 71.1627907 | Mm.27695 | 1E-140 | 95.46925566 | 71.86046512 |
| WAN008EJ7_at (SEQ ID NO:2026) | EIF5A | Eukaryotic translation initiation factor 5A | Hs.534314 | 0 | 98.52941176 | 100 | Mm.196607 | 0 | 97.54901961 | 100 |
| WAN008EC0_at (SEQ ID NO:2232) | EIF5B | Eukaryotic translation initiation factor 5B | Hs.158688 | 5E-85 | 89.28571429 | 97.56097561 | Mm.260943 | 1E-100 | 91.45907473 | 97.90940767 |
| WAN013HYP_at (SEQ ID NO:2233) | FEM1A | Fem-1 homolog a (C.elegans) | Hs.515082 | 9E-84 | 86.66666667 | 58.61456483 | Mm.290813 | 1E-107 | 89.28571429 | 59.68028419 |
| Y12837_at (SEQ ID NO:2120) | Fxr1h | Fragile X mental retardation gene 1, autosomal homolog | #N/A | 0 | 96.12756264 | 100 | Mm.259021 | 0 | 97.72209567 | 100 |
| M60973 at (SEQ ID NO:2031) | GADD45A | Growth arrest and DNA-damage-inducible, alpha | Hs.80409 | 0 | 91.57372986 | 76.34815516 | Mm.389750 | 1E-170 | 90.57692308 | 49.19583728 |
| WAN013139 at (SEQ ID NO:1541) | Gga2 | Golgi associated, gamma adaptin ear containing, ARF binding protein 2 | Hs.460336 | 7E-30 | 84.45378151 | 46.66666667 | Mm.29619 | 1E-147 | 93.33333333 | 79.41176471 |
| WAN013I4Q_at (SEQ ID NO:2234) | GLUL | Glutamate-ammonia ligase (glutamine synthetase) | Hs.518525 | 1E-136 | 88.44221106 | 74.39252336 | Mm.210745 | 1E-135 | 88.11881188 | 75.51401869 |
| WAN013I0X_at (SEQ ID NO:1581) | GSS | Glutathione synthetase | Hs.82327 | 1E-98 | 90.78498294 | 56.1302682 | Mm.252316 | 1E-129 | 95.18900344 | 55.74712644 |
| WAN008EPH_at (SEQ ID NO:2235) | GTF2H4 | General transcription factor IIH, polypeptide 4, 52kDa | Hs.485070 | 1E-111 | 88.97435897 | 87.64044944 | Mm.10182 | 1E-148 | 91.87817259 | 88.53932584 |
| WAN008E8M_at (SEQ ID NO:1568) | HADHB | Hydroxyacyl-Coenzyme A dehydrogenase/3-ketoacyl-Coenzyme A thiolase/enoyl-Coenzyme A hydratase (trifunctional protein), beta subunit | Hs.534639 | 1E-114 | 88.19095477 | 85.77586207 | Mm.291463 | 1E-160 | 91.03139013 | 96.12068966 |
| WAN008EKL_at (SEQ ID NO:1775) | HBP1 | HMG-box transcription factor 1 | Hs.162032 | 1E-120 | 89.60784314 | 98.45559846 | Mm.390461 | 0 | 91.6827853 | 99.80694981 |
| WAN008CWV_at (SEQ ID | HDGF | Hepatoma-derived growth factor (high-mobility group protein 1-like) | Hs.506748 | 3E-75 | 91.4893617 | 43.04029304 | Mm.292208 | 2E-75 | 91.4893617 | 43.04029304 |
| NO:1684) WAN0088XH_at (SEQ ID NO:1557) | HERPUD1 | Homocysteine-inducible, endoplasmic reticulum stress-inducible, ubiquitin-like domain member 1 | Hs.146393 | 7E-79 | 87.41721854 | 68.48072562 | Mm.29151 | 1E-151 | 91.46341463 | 92.97052154 |
| L00180_at (SEQ ID NO:1509) | Hmgcr | 3-hydroxy-3-methylglutaryl-Coenzyme A reductase | Hs.643495 | 5E-35 | 86.30952381 | 68.01619433 | Mm.316652 | 1E-49 | 90.24390244 | 66.39676113 |
| WAN008D6E_x_at (SEQ ID NO:2236) | HMOX2 | Heme oxygenase (decycling) 2 | Hs.284279 | 2E-32 | 94.50549451 | 28.4375 | Mm.272866 | 2E-37 | 95.78947368 | 29.6875 |
| WAN008CLS_at (SEQ ID NO:2237) | HSDL2 | Hydroxysteroid dehydrogenase like 2 | Hs.59486 | 1E-148 | 87.09090909 | 95.65217391 | Mm.272905 | 0 | 93.01919721 | 99.65217391 |
| WAN013I8Z_at (SEQ ID NO:2238) | Hspa8 | Heat shock protein 8 | Hs. 180414 | 0 | 90.56603774 | 69.19060052 | Mm.290774 | 0 | 93.73368146 | 100 |
| WAN008D42_at (SEQ ID NO:2239) | HSPB8 | Heat shock 22kDa protein 8 | Hs.400095 | 1E-16 | 89.02439024 | 15.3271028 | Mm.21549 | 3E-31 | 90.90909091 | 20.56074766 |
| WAN013I26_at (SEQ ID NO:2114) | IDH3A | Isocitrate dehydrogenase 3 (NAD+) alpha | Hs.591110 | 0 | 89.26746167 | 98.16053512 | Mm.279195 | 0 | 94.06779661 | 98.66220736 |
| WAN008EX2_x_at (SEQ ID NO:1575) | Ifrd1 | Interferon-related developmental regulator 1 | Hs.7879 | 7E-39 | 90.29850746 | 100 | Mm.168 | 5E-63 | 97.76119403 | 100 |
| WAN013I0I_at (SEQ ID NO:2240) | INSIG1 | Insulin induced gene 1 | Hs.520819 | 4E-21 | 94.20289855 | 12.94559099 | Mm.30221 | 3E-44 | 86.53846154 | 39.02439024 |
| WAN0088O9_at (SEQ ID NO:1777) | Itgb1 | Integrin beta 1 (fibronectin receptor beta) | Hs.295626 | 1E-13 | 90.32258065 | 16.4893617 | Mm.263396 | 4E-55 | 90.49773756 | 39.18439716 |
| AF081140_at (SEQ ID NO:2241) | Itgb4bp | Integrin beta 4 binding protein | Hs.632277 | 0 | 0 | 0 | Mm.271674 | 5E-56 | 92.02453988 | 52.24358974 |
| X83575_at (SEQ ID NO:2007) | KIF23 | Kinesin family member 23 | Hs.270845 | 1E-177 | 92.47787611 | 37.07957342 | Mm.259374 | 0 | 91.99372057 | 52.2559475 |
| WAN008D29_s_at (SEQ ID NO:2242) | LGALS3 | Lectin, galactoside-binding, soluble, 3 (galectin 3) | Hs.531081 | 3E-41 | 91.8128655 | 31.78438662 | Mm.248615 | 5E-79 | 88.48484848 | 61.33828996 |
| WAN008DBR_at (SEQ ID NO:1966) | LUC7L | LUC7-like (S. cerevisiae) | Hs.16803 | 0 | 93.66197183 | 100 | Mm.386921 | 0 | 95.07042254 | 100 |
| WAN008ECL_at (SEQ ID NO:1243) | MAN2C1 | Mannosidase, alpha, class 2C, member 1 | Hs.26232 | 1 E-134 | 87.68267223 | 96.96356275 | Mm.30110 | 1E-119 | 93.02325581 | 60.93117409 |
| WAN008E7Y_at (SEQ ID NO:2244) | MCM7 | MCM7 minichromosome maintenance deficient 7 (S. cerevisiae) | Hs.438720 | 1E-96 | 84.53389831 | 88.38951311 | Mm.241714 | 1E-171 | 90.98532495 | 89.3258427 |
| WAN013I23_at (SEQ ID NO:2245) | MORF4L2 | Mortality factor 4 like 2 | Hs.326387 | 0 | 89.13043478 | 96.61610268 | Mm.27218 | 0 | 92.6487748 | 100 |
| WAN008CRR_at (SEQ ID NO:2246) | MRPL19 | Mitochondrial ribosomal protein L19 | Hs.44024 | 1E-71 | 86.89655172 | 58 | Mm.276293 | 1E-136 | 87.34939759 | 99.6 |
| WAN013I02_at (SEQ ID NO:2247) | MRPS18A | Mitochondrial ribosomal protein S18A | Hs.520149 | 1E-137 | 85.78767123 | 89.02439024 | Mm.287443 | 0 | 90.92465753 | 89.02439024 |
| WAN008EV8_at (SEQ ID NO:2189) | MTHFD2 | Methylenetetrahydrofolate dehydrogenase (NADP+ dependent) 2, methenyltetrahydrofolate cyclohydrolase | Hs.469030 | 7E-50 | 88.35978836 | 68.23104693 | Mm.443 | 4E-84 | 91.05691057 | 88.80866426 |
| WAN008EQD_at (SEQ ID NO:1894) | Mx2 | Myxovirus (influenza virus) resistance 2 | Hs.926 | 2E-29 | 80.31746032 | -75 | Mm.14157 | 2E-99 | 85.95238095 | 100 |
| WAN008BSP_x_at (SEQ ID NO:2248) | NDFIP1 | Nedd4 family interacting protein 1 | Hs.9788 | 5E-33 | 92.23300971 | 93.63636364 | Mm.102496 | 1E-44 | 95.45454545 | 100 |
| WAN008EDO_at (SEQ ID NO:2249) | NDRG3 | NDRG family member 3 | Hs.437338 | 5E-86 | 88.00904977 | 92.85714286 | Mm.279256 | 3E-92 | 93.41563786 | 51.05042017 |
| WAN013I17_at (SEQ ID NO:1790) | NID1 | Nidogen 1 | Hs.356624 | 1E-101 | 83.47826087 | 83.03249097 | Mm.4691 | 9E-99 | 91.66666667 | 45.48736462 |
| AF182744_at (SEQ ID NO:2250) | NPC1 | Niemann-Pick disease, type C1 | Hs.464779 | 6E-83 | 85.41114058 | 70.20484171 | Mm.3484 | 1E-167 | 92.13483146 | 82.86778399 |
| WAN008EK9_at (SEQ ID NO:2251) | Nup160 | Nucleoporin 160 | Hs.645358 | 1E-159 | 89.2 | 99.60159363 | Mm.24532 | 0 | 94.36619718 | 99.00398406 |
| WAN0088ZP_at (SEQ ID NO:1482) | Pawr | PRKC, apoptosis, WT1, regulator | Hs.643130 | 4E-10 | 91.52542373 | 11.11111111 | Mm.391419 | 8E-53 | 91.62561576 | 38.22975518 |
| WAN008EML_at (SEQ ID NO:1980) | PBK | PDZ binding kinase | Hs.104741 | 5E-52 | 89.50276243 | 39.09287257 | Mm.24337 | 3E-80 | 89.7810219 | 59.17926566 |
| WAN0088X2_at (SEQ ID NO:1593) | PEO1 | Progressive external ophthalmoplegia 1 | Hs.22678 | 1E-141 | 88.6509636 | 94.53441296 | Mm.105585 | 7E-78 | 90.6779661 | 47.77327935 |
| WAN013I38_at (SEQ ID NO:1583) | Pkm2 | Pvruvate kinase, muscle | Hs.534770 | 4E-13 | 92.85714286 | 10.18181818 | Mm.326167 | 0 | 92.42718447 | 93.63636364 |
| WAN0088KG_at (SEQ ID NO:1539) | PPGB | Protective protein for beta-galactosidase (galactosialidosis) | Hs.517076 | 1E-115 | 87.58949881 | 72.86956522 | Mm.359633 | 1E-149 | 90.93078759 | 72.86956522 |
| WAN008968_at (SEQ ID NO:2252) | PPP4R1 | Protein phosphatase 4, regulatory subunit 1 | Hs.464595 | 1E-141 | 86.24338624 | 99.47368421 | Mm.48686 | 0 | 91.34438306 | 95.26315789 |
| WAN008D4W_at (SEQ ID NO:2079) | PPT2 | Palmitoyl-protein thioesterase 2 | Hs.332138 | 6E-82 | 86.29737609 | 97.44318182 | Mm.373627 | 1E-96 | 89.38906752 | 88.35227273 |
| WAN0088ZC_at (SEQ ID NO:1543) | PSEN1 | Presenilin 1 (Alzheimer disease 3) | Hs.592324 | 5E-82 | 89.16083916 | 86.14457831 | Mm.998 | 4E-78 | 88.1533101 | 86.44578313 |
| WAN013HXU_at (SEQ ID NO:2156) | PSMA1 | Proteasome (prosome, macropain) subunit, alpha type, 1 | Hs. 102798 | 0 | 92.22011385 | 100 | Mm.121265 | 0 | 94.84732824 | 99.43074004 |
| WAN013HVR_at (SEQ ID NO:2253) | PSMD4 | Proteasome (prosome, macropain) ' 26S subunit, non-ATPase, 4 | Hs.505059 | 0 | 90.23090586 | 100 | Mm.2261 | 0 | 94.12811388 | 99.82238011 |
| WAN008CLK_at (SEQ ID NO:1552) | RAB6A | RAB6A, member RAS oncogene family | Hs.12152 | 2E-55 | 88.47736626 | 48.21428571 | #N/A | 1E-161 | 92.07317073 | 97.61904762 |
| WAN008CWE_at (SEQ ID NO:2254) | Ralb | V-ral simian leukemia viral oncogene homolog B (ras related) | Hs.469820 | 0 | 0 | 0 | Mm.27832 | 2E-34 | 90.24390244 | 30.37037037 |
| WAN008DNA_at (SEQ ID NO:2255) | RBM28 | RNA binding motif protein 28 | Hs.274263 | 2E-76 | 86.62420382 | 55.9714795 | Mm.40802 | 1E-172 | 90.38112523 | 98.21746881 |
| X61588_at (SEQ ID NO:2256) | RHOG | Ras homolog gene family, member G (rho G) | Hs.501728 | 0 | 90.47619048 | 67.5 | Mm.259795 | 0 | 93.49693252 | 72.76785714 |
| WAN013HVB_at (SEQ ID NO:1915) | RNF10 | Ring finger protein 10 | Hs.442798 | 1E-180 | 92.22462203 | 80.66202091 | Mm.30051 | 1E-158 | 89.56158664 | 83.44947735 |
| WAN008CQ7_at (SEQ ID NO:2257) | ROD1 | ROD1 regulator of differentiation 1 (S. pombe) | Hs.269988 | 4E-84 | 96.31336406 | 39.74358974 | Mm.331640 | 1E-121 | 97.31800766 | 47.8021978 |
| WAN008EE3_at (SEQ ID NO:1809) | SARS | Seryl-tRNA synthetase | Hs.531176 | 1E-116 | 90.80118694 | 100 | Mm.28688 | 1E-136 | 93.17507418 | 100 |
| WAN008ENH_at (SEQ ID NO:2258) | SCAMP5 | Secretory carrier membrane protein 5 | Hs.374180 | 1E-173 | 96.38888889 . | 79.47019868 | Mm.102278 | 0 | 95.58498896 | 100 |
| WAN013I16D_at (SEQ ID NO:2259) | Sdc1 | Syndecan 1 | Hs.224607 | 1E-26 | 90 | 9.615384615 | Mm.2580 | 0 | 88.17073171 | 60.65088757 |
| WAN008EHX_at (SEQ ID NO:1920) | Setd8 | SET domain containing (lysine methyltransferase) 8 | Hs.572262 | 1E-141 | 86.65447898 | 100 | Mm.137966 | 0 | 89.76234004 | 100 |
| WAN008DJ9_at (SEQ ID NO:1565) | SLC1A4 | Solute carrier family 1 (glutamate/neutral amino acid transporter), member 4 | Hs.323878 | 1E-34 | 86.36363636 | 39.46188341 | Mm.6379 | 1E-119 | 88.36633663 | 90.58295964 |
| WAN013I1 G_at (SEQ ID NO:1582) | SLC25A20 | Solute carrier family 25 (carnitine/acylcarnitine translocase), member 20 | Hs. 13845 | 1E-137 | 86.70634921 | 87.65217391 | Mm.29666 | 0 | 92.35412475 | 86.43478261 |
| WAN00895Z_at (SEQ ID NO:2260) | SNAP29 | Synaptosomal-associated protein, 29kDa | Hs.108002 | 2E-84 | 85.95041322 | 100 | Mm.271992 | 3E-88 | 87.87878788 | 100 |
| WAN008EFY_at (SEQ ID NO:1811) | SPG21 | Spastic paraplegia 21 (autosomal recessive, Mast syndrome) | Hs.242458 | 1E-128 | 90.45092838 | 71.94656489 | Mm.272475 | 1E-136 | 91.44385027 | 71.3740458 |
| WAN008EBP_at (SEQ ID NO:1621) | SQSTM1 | Sequestosome 1 | Hs.437277 | 1E-166 | 89.36170213 | 92.65232975 | Mm.40828 | 0 | 93.40659341 | 97.84946237 |
| WAN0088TG_at (SEQ ID NO:1540) | SRP72 | Signal recognition particle 72kDa | Hs.237825 | 1 E-58 | 89.04109589 | 50.81206497 | Mm.296976 | 1E-119 | 92.40121581 | 76.33410673 |
| WAN008EH6_at (SEQ ID NO:2141) | STT3A | STT3, subunit of the oligosaccharyltransferase complex, homolog A (S. cerevisiae) | Hs.504237 | 0 | 93.63636364 | 97.86476868 | Mm.2863 | 0 | 95.27272727 | 97.86476868 |
| WAN013I0W8_at (SEQ ID NO.1580) | TAPBP | TAP binding protein (tapasin) | Hs.370937 | 2E-57 | 80.63314711 | 93.22916667 | Mm.154457 | 1E-149 | 86.8852459 | 95.3125 |
| WAN013HX6_at (SEQ ID NO:1815) | TAX1BP1 | Tax1 (human T-cell leukemia virus type I) binding protein 1 | Hs.34576 | 1E-111 | 86.72985782 | 41.49459194 | Mm.431979 | 1 E-124 | 89.42065491 | 39.03638151 |
| WAN008F02_at (SEQ ID NO:2191) | TFPI | Tissue factor pathway inhibitor (lipoprotein-associated coagulation inhibitor) | Hs.516578 | 3E-14 | 83.33333333 | 21.79930796 | Mm.124316 | 3E-83 | 85.5227882 | 64.53287197 |
| WAN008EAE_at (SEQ ID NO:2261) | Tloc1 | Translocation protein 1 | Hs.592561 | 1E-132 | 92.8358209 | 100 | Mm.26017 | 1E-158 | 96.11940299 | 100 |
| WAN008ER4_at (SEQ ID NO:2262) | TMC6 | Transmembrane channel-like 6 | Hs.632227 | 3E-41 | 84.75336323 | 42.31499051 | Mm.286963 | 1 E-94 | 87.08791209 | 69.07020873 |
| WAN013I1C_at (SEQ ID NO:2263) | Trib3 | Tribbles homolog 3 (Drosophila) | Hs.516826 | 0 | 0 | 0 | Mm.276018 | 5E-22 | 92.20779221 | 13.02876481 |
| WAN008DPJ_at (SEQ ID NO:2264) | Trim26 | Tripartite motif protein 26 | Hs.485041 | 0 | 0 | 0 | Mm.34587 | 7E-10 | 89.18918919 | 14.82965932 |
| WAN008CVL_x_at (SEQ ID NO:2265) | TUBG1 | Tubulin, gamma 1 | Hs.279669 | 8E-64 | 93.90243902 | 96.47058824 | Mm.142348 | 2E-75 | 96.40718563 | 98.23529412 |
| WAN008CYV_at (SEQ ID NO:2266) | TXNDC4 | Thioredoxin domain containing 4 (endoplasmic reticulum) | Hs.591899 | 4E-99 | 93.85245902 | 48.70259481 | Mm.317701 | 1E-142 | 95.76547231 | 61.27744511 |
| WAN0088P2_at (SEQ ID NO:1853) | UAP1 | UDP-N-acteylglucosamine pyrophosphorylase 1 | Hs.492859 | 1E-130 | 86.73267327 | 91.98542805 | Mm.27969 | 1E-177 | 90.01883239 | 96.72131148 |
| WAN008E3E_at (SEQ ID NO.2267) | UBE2G2 | Ubiquitin-conjugating enzyme E2G 2 (UBC7 homolog, yeast) | Hs.529420 | 1E-168 | 90.75630252 | 94.07114625 | Mm.307906 | 0 | 95.20958084 | 99.01185771 |
| WAN008EA0_at (SEQ ID NO:1487) | VCP | Valosin-containing protein | Hs.529782 | 0 | 95.18348624 | 78.98550725 | Mm.262053 | 0 | 95.47101449 | 100 |
| WAN008D19_x_at (SEQ ID NO:2268) | Vps35 | Vacuolar protein sorting 35 | Hs.454528 | 2E-55 | 95.52238806 | 86.4516129 | Mm.296520 | 1E-64 | 97.16312057 | 90.96774194 |
| WAN013I0V_at (SEQ ID NO:2269) | WBP2 | WW domain binding protein 2 | Hs.514489 | 1E-35 | 90.32258065 | 49.20634921 | Mm.284792 | 3E-56 | 87.55364807 | 92.46031746 |
| WAN013I07_at (SEQ ID NO:2270) | WDR43 | WD repeat domain 43 | Hs.169863 | 3E-47 | 92.02898551 | 25.32110092 | Mm.257762 | 1E-63 | 90 | 38.53211009 |
| WAN008ES6_at (SEQ ID NO:2271) | XAB1 | XPA binding protein 1, GTPase | Hs.18259 | 1E-128 | 88.70588235 | 98.60788863 | Mm.348649 | 1E-168 | 92.6713948 | 98.14385151 |
| WAN008DQM_at (SEQ ID NO:2181) | XPNPEP1 | X-prolyl aminopeptidase (aminopeptidase P) 1, soluble | Hs.390623 | 1E-147 | 88.11881188 | 99.60552268 | Mm.99776 | 0 | 92.50493097 | 100 |
| WAN008DNP_at (SEQ ID NO:1876) | XPO1 | Exportin 1 (CRM1 homolog, yeast) | Hs.370770 | 0 | 91.08527132 | 98.47328244 | Mm.217547 | 0 | 94.84732824 | 100 |
| WAN0088PU_at (SEQ ID NO:2128) | Ywhab | Tyrosine 3-monooxygenase/tryptophan 5-monooxygenase activation protein, beta polypeptide | Hs.645387 | 1E-102 | 92.61538462 | 62.74131274 | Mm.34319 | 0 | 92.81553398 | 99.42084942 |

**Table 29. LLP2+4 Test-specific 1.2F UP**

| Qualifier List | Symbol | Title | Human Unigene ID | eValue | %ID | %QC | Mouse Uniqene ID | eValue | %ID | %QC |
|---|---|---|---|---|---|---|---|---|---|---|
| WAN013I76_at (SEQ ID NO:2272) | ERBB2 | V-erb-b2 erythroblastic leukemia viral oncogene homolog 2, neuro/glioblastoma derived oncogene homolog (avian) | Hs.446352 | 7E-61 | 87.23404255 | 51.55393053 | Mm.290822 | 5E-76 | 88.54166667 | 52.65082267 |
| WAN008BXV_at (SEQ ID NO:2273) | FbxI5 | F-box and leucine-rich repeat protein 5 | Hs.645220 | 4E-99 | 94.82758621 | 43.04267161 | Mm.25794 | 0 | 94.37148218 | 98.88682746 |
| WAN013I4Q_at (SEQ ID NO:2234) | GLUL | Glutamate-ammonia ligase (glutamine synthetase) | Hs.518525 | 1E-136 | 88.44221106 | 74.39252336 | Mm.210745 | 1 E-135 | 88.11881188 | 75.51401869 |
| WAN008EMJ_at (SEQ ID NO:1721) | GPC6 | Glypican 6 | Hs.444329 | 7E-64 | 92.55319149 | 37.4501992 | Mm.234129 | 3E-58 | 90.37433155 | 37.25099602 |
| U73375_at (SEQ ID | LGALS3BP | Lectin, galactoside-binding, soluble, 3 binding protein | Hs.514535 | 5E-31 | 82.88288288 | 38.81118881 | Mm.3152 | 2E-84 | 85.48812665 | 66.25874126 |
| WAN013I2D_at (SEQ ID NO:2275) | RAP2C | RAP2C, member of RAS oncogene family | Hs.119889 | 4E-77 | 91.18942731 | 56.75 | Mm.43152 | 2E-86 | 93.24324324 | 55.5 |
| WAN0088ZJ_at (SEQ ID NO:2075) | SIc4a2 | Solute carrier family 4 (anion exchanger), member 2 | #N/A | 1E-162 | 88.57677903 | 100 | Mm.4580 | 0 | 93.79699248 | 99.62546816 |

**Table 30. LLP2+4 Test-specific 1.2F DOWN (39genes)**

| Qualifier List | Symbol | Title | Human Unigene ID | eValue | %ID | %QC | Mouse Unigene ID | eValue | %ID | %QC |
|---|---|---|---|---|---|---|---|---|---|---|
| WAN013I6P_f_at (SEQ ID NO:1658) | Abcb1a | ATP-binding cassette, sub-family B (MDR/TAP), member 1A | #N/A | 6E-40 | 90.90909091 | 80 | Mm.207354 | 1E-51 | 93.29268293 | 99.39393939 |
| WAN008CRT_at (SEQ ID NO:1954) | ALG14 | Asparagine-linked glycosylation 14 homolog (yeast) | Hs.408927 | 4E-47 | 88.39779006 | 32.43727599 | Mm.269881 | 5E-51 | 88.77005348 | 33.5125448 |
| M80243-rc_at (SEQ ID NO:1943) | BIRC5 | Baculoviral IAP repeat-containing 5 (survivin) | Hs.514527 | 4E-38 | 92.37288136 | 20.34482759 | Mm.8552 | 1 E-36 | 93.45794393 | 18.44827586 |
| WAN0088K1_at (SEQ ID NO:2276) | BRD2 | Bromodomain containing 2 | Hs.75243 | 1E-163 | 88.93129771 | 95.09981851 | Mm.3444 | 0 | 90.92558984 | 100 |
| WAN013I8J_at (SEQ ID NO:2001) | CCNB2 | Cyclin B2 | Hs.194698 | 1E-173 | 86.92579505 | 44.39215686 | Mm.22592 | 0 | 90.70945946 | 46.43137255 |
| WAN008CVX_at (SEQ ID NO:1958) | CDC20 | CDC20 cell division cycle 20 homolog (S. cerevisiae) | Hs.524947 | 1E-169 | 90.66390041 | 85.1590106 | Mm.289747 | 0 | 92.30769231 | 87.27915194 |
| WAN008DGK_at (SEQ ID NO:1967) | CHAF1A | Chromatin assembly factor 1, subunit A (p150) | Hs.79018 | 1E-83 | 91.32231405 | 57.89473684 | Mm.391010 | 1E-101 | 90.84745763 | 70.57416268 |
| WAN0088OE_at (SEQ ID NO:2176) | Crk | V-crk sarcoma virus CT10 oncogene homolog (avian) | Hs.638121 | 1E-161 | 94.35483871 | 77.01863354 | Mm.280125 | 0 | 97.04301075 | 77.01863354 |
| WAN008E4R_at (SEQ ID NO:2277) | DHX16 | DEAH (Asp-Glu-Ala-His) box polypeptide 16 | Hs.485060 | 4E-84 | 91.63179916 | 44.25925926 | Mm.390986 | 3E-80 | 90.83333333 | 44.44444444 |
| WAN008DYW_at (SEQ ID NO:2278) | FARSLA | Phenylalanine-tRNA synthetase-like, alpha subunit | Hs-23111 | 1E-126 | 86.45418327 | 90.45045045 | Mm.292517 | 0 | 94.22718808 | 96.75675676 |
| WAN008E40_at (SEQ ID NO:2279) | FEN1 | Flap structure-specific endonuclease 1 | Hs.409065 | 1E-121 | 91.22807018 | 61.40035907 | Mm.2952 | 1 E-136 | 92.77456647 | 62.11849192 |
| WAN013HYG_x_at (SEQ ID NO:2280) | Grb2 | Growth factor receptor bound protein 2 | Hs.444356 | 4E-47 | 80.13937282 | 86.18618619 | Mm.383426 | 1E-89 | 85.07936508 | 94.59459459 |
| WAN008EVU_f_at (SEQ ID NO:1887) | HNRPK | Heterogeneous nuclear ribonucleoprotein K | Hs.522257 | 1E-128 | 92.94478528 | 97.60479042 | Mm.142872 | 1E-133 | 93.11377246 | 100 |
| WAN008EX2_x_at (SEQ ID NO:1575) | Ifrd1 | Interferon-related developmental regulator 1 | Hs.7879 | 7E-39 | 90.29850746 | 100 | Mm.168 | 5E-63 | 97.76119403 | 100 |
| WAN008E3O_at (SEQ ID NO:1973) | LINCR | Likely ortholog of mouse lung-inducible Neutralized-related C3HC4 RING domain protein | Hs.149219 | 3E-19 | 84.61538462 | 38.01169591 | Mm.389110 | 3E-76 | 85.3372434 | 99.70760234 |
| WAN008CVC_at (SEQ ID NO:2281) | Mm.387215 | CDNA, clone:Y0G0110B16, strand:plus, reference:ENSEMBL:Mouse-Transcript-ENST:ENSMUST00000058619, based on BLAT search | #N/A | 1E-119 | 97.95081967 | 100 | Mm.387215 | 1E-117 | 97.54098361 | 100 |
| WAN0088XN_at (SEQ ID NO:2282) | MTHFD1L | Methylenetetrahydrofolate dehydrogenase (NADP+ dependent) 1-like | Hs.591343 | 1E-99 | 90.57239057 | 54.59558824 | Mm.184752 | 1E-126 | 94.27609428 | 54.59558824 |
| WAN008EV8_at (SEQ ID NO:2189) | MTHFD2 | Methylenetetrahydrofolate dehydrogenase (NADP+ dependent) 2, methenyltetrahydrofolate cyclohydrolase | Hs.469030 | 7E-50 | 88.35978836 | 68.23104693 | Mm.443 | 4E-84 | 91.05691057 | 88.80866426 |
| WAN008EOB_at (SEQ ID NO:1629) | NOL1 | Nucleolar protein 1, 120kDa | Hs.534334 | 8E-48 | 89.80582524 | 42.4742268 | Mm.29203 | 1E-120 | 87.24832215 | 92.16494845 |
| WAN008CTA_at (SEQ ID NO:1957) | NOLC1 | Nucleolar and coiled-body phosphoprotein 1 | Hs.523238 | 1E-101 | 89.12386707 | 59.63963964 | Mm.402190 | 3E-28 | 89.90825688 | 19.63963964 |
| WAN013I8D_at (SEQ ID NO:2000) | PARP1 | Poly (ADP-ribose) polymerase family, member 1 | Hs.177766 | 2E-43 | 85.57692308 | 35.01683502 | Mm.277779 | 1E-102 | 87.78054863 | 67.50841751 |
| WAN008DUC_at (SEQ ID NO:1795) | PHF14 | PHD finger protein 14 | Hs.159918 | 8E-86 | 94.63414634 | 99.51456311 | Mm.212411 | 4E-74 | 92.19512195 | 99.51456311 |
| WAN008EBY_at (SEQ ID NO:2283) | PLCG1 | Phospholipase C, gamma 1 | Hs.268177 | 1E-175 | 89.63636364 | 99.45750452 | Mm.44463 | 0 | 92.58589512 | 100 |
| WAN008EHF_at (SEQ ID NO:2284) | POFUT2 | Protein O-fucosyltransferase 2 | Hs.592164 | 5E-77 | 85.84070796 | 61.30198915 | Mm.203556 | 1E-179 | 94.58128079 | 73.41772152 |
| WAN013HY0_at (SEQ ID NO:2173) | PRPF19 | PRP19/PS04 pre-mRNA processing factor 19 homolog (S. cerevisiae) | Hs.502705 | 0 | 89.25318761 | 100 | Mm.358657 | 0 | 93.26047359 | 100 |
| WAN008DQG_at (SEQ ID NO:2285) | PRPF3 | PRP3 pre-mRNA processing factor 3 homolog (S. cerevisiae) | Hs.11776 | 1E-114 | 92.45901639 | 99.02597403 | Mm.279872 | 1E-121 | 93.18181818 | 100 |
| WAN013I0A_x_at (SEQ ID NO:2113) | PRPF38B | PRP38 pre-mRNA processing factor 38 (yeast) domain containing B | Hs.342307 | 4E-84 | 83.39694656 | 99.05482042 | Mm.51049 | 1E-173 | 89.69465649 | 99.05482042 |
| WAN008E7N_at (SEQ ID NO:2286) | RBBP4 | Retinoblastoma binding protein 4 | Hs.16003 | 1E-88 | 89.51048951 | 94.38943894 | Mm.12145 | 1E-113 | 92.07920792 | 100 |
| WAN008EQE_x_at (SEQ ID NO:2287) | RNPEP | Arginyl aminopeptidase (aminopeptidase B) | Hs.497391 | 6E-22 | 85.38461538 | 59.09090909 | Mm.291048 | 7E-45 | 85.45454545 | 100 |
| WAN0088NR_at (SEQ ID NO:2288) | SF3B3 | Splicing factor 3b, subunit 3, 130kDa | Hs.514435 | 1E-131 | 92.39766082 | 81.04265403 | Mm.236123 | 1E-151 | 94.9704142 | 80.09478673 |
| WAN008EPC_at (SEQ ID NO:2289) | SHMT2 | Serine hydroxymethyltransferase 2 (mitochondrial) | Hs.75069 | 1E-143 | 90.43062201 | 99.0521327 | Mm.29890 | 1E-142 | 90.68627451 | 96.68246445 |
| WAN008E5L_at (SEQ ID NO:1619) | SLC1A5 | Solute carrier family 1 (neutral amino acid transporter), member 5 | Hs.631582 | 8E-42 | 84.16666667 | 45.62737643 | Mm.1056 | 1E-115 | 87.67123288 | 83.26996198 |
| WAN0088S8_at (SEQ ID NO:1591) | SLC29A1 | Solute carrier family 29 (nucleoside transporters), member 1 | Hs.25450 | 3E-35 | 81.3559322 | 76.12903226 | Mm.29744 | 5E-97 | 86.09756098 | 88.17204301 |
| WAN008DCP_at (SEQ ID NO:2100) | TBC1D10A | TBC1 domain family, member 10A | Hs.444950 | 0 | 90.84507042 | 100 | Mm.28140 | 0 | 95.42253521 | 100 |
| WAN0088TW_at (SEQ ID NO:1745) | TCEB3 | Transcription elongation factor B (SIII), polypeptide 3 (110kDa, elongin A) | Hs.584806 | 1E-173 | 89.36567164 | 98.52941176 | Mm.27663 | 0 | 93.09701493 | 98.52941176 |
| WAN008CS0_at (SEQ ID NO:2290) | TMEM103 | Transmembrane protein 103 | Hs.311100 | 1E-96 | 84.22131148 | 88.4057971 | Mm.374250 | 1E-152 | 87.96992481 | 96.37681159 |
| WAN008ES6_at (SEQ ID NO:2271) | XAB1 | XPA binding protein 1, GTPase | Hs. 18259 | 1 E-128 | 88.70588235 | 98.60788863 | Mm.348649 | 1E-168 | 92.6713948 | 98.14385151 |
| WAN008EGH_at (SEQ ID NO:2291) | XPOT | Exportin, tRNA (nuclear export receptor for tRNAs) | Hs.85951 | 1E-145 | 87.2659176 | 93.84885764 | Mm-25042 | 1E-150 | 87.64044944 | 93.84885764 |
| WAN008906_at (SEQ ID NO:1833) | Zfp259 | Zinc finger protein 259 | #N/A | 1E-162 | 90.13539652 | 94.86238532 | Mm.17519 | 0 | 92.8440367 | 100 |

Standard cell engineering methods are used to modify target genes to effect desired cell phenotypes. As discussed above, target genes are modified to achieve desired CHO cell phenotypes by interfering RNA, conventional gene knockout or overexpression methods. Typically, knockout methods or stable transfection methods with overexpression constructs are used to engineer modified CHO cell lines. Other suitable methods are discussed in the general description section and known in the art.

The foregoing description of the present invention provides illustration and description, but is not intended to be exhaustive or to limit the invention to the precise one disclosed. Modifications and variations are possible consistent with the above teachings or may be acquired from practice of the invention. Thus, it is noted that the scope of the invention is defined by the claims and their equivalents.

### INCORPORATION BY REFERENCE

All sequence accession numbers, publications and patent documents cited in this application are incorporated by reference in their entirety for all purposes to the same extent as if the contents of each individual publication or patent document was incorporated herein.

In view of the above, it will be appreciated that the invention also encompasses the following items:
1. A method for identifying proteins regulating or indicative of a cell culture phenotype in a cell line, the method comprising:
   generating a protein expression profile of a sample derived from a test cell line;
   comparing the protein expression profile to a control profile derived from a control cell line; and
   identifying one or more differentially expressed proteins based on the comparison,
   wherein the test cell line has a cell culture phenotype distinct from that of the control cell line, and the one or more differentially expressed proteins are capable of regulating or indicating the cell culture phenotype.
2. The method of item 1, wherein the cell line is a Chinese hamster ovary (CHO) cell line.
3. The method of item 1, wherein the cell culture phenotype is a cell growth rate, a cellular productivity, a peak cell density, a sustained cell viability, a rate of ammonia production or consumption, or a rate of lactate production or consumption.
4. The method of item 3, wherein the cell culture phenotype is a maximum cellular productivity.
5. The method of item 3, wherein the cell culture phenotype is a sustained cell viability.
6. The method of item 3, wherein the cell culture phenotype is a peak cell density.
7. The method of item 3, wherein the cell culture phenotype is a cell growth rate.
8. The method of item 1, wherein the protein expression profile is generated by fluorescent two-dimensional differential in-gel electrophoresis.
9. A method for improving a cell line, the method comprising up-regulating one or more proteins identified according to the method of item 1.
10. A method for improving a cell line, the method comprising up-regulating one or more proteins identified according to the method of item 1 by overexpression.
11. A method for improving a cell line, the method comprising down-regulating one or more proteins identified according to the method of item 1.
12. The method of item 11, wherein the method comprises down-regulating one or more proteins identified according to the method of item 1 by RNA interference.
13. A method for improving cellular productivity of a cell line, the method comprising up-regulating one or more proteins identified according to the method of item 1.
14. A method for improving cellular productivity of a cell line, the method comprising down-regulating one or more proteins identified according to the method of item 1.
15. A method for improving cellular productivity of a cell line, the method comprising modulating one or more genes or proteins selected from Tables 2, 3, 9, 10, 11, and 12.
16. A method for improving cellular productivity of a cell line, the method comprising modulating one or more proteins identified according to the method of item 4.
17. A method for improving cell growth rate of a cell line, the method comprising modulating one or more genes or proteins selected from Tables 4, 5, 6, 13, 14, 27 and 28.
18. A method for improving cell growth rate of a cell line, the method comprising modulating one or more proteins identified according to the method of item 7.
19. A method for increasing peak cell density of a cell line, the method comprising modulating one or more genes or proteins selected from Tables 8, 15, 16, and 17.
20. A method for increasing peak cell density of a cell line, the method comprising modulating one or more proteins identified according to the method of item 6.
21. A method for increasing sustained cell viability of a cell line, the method comprising modulating one or more genes or proteins selected from Tables 7, 18 and 19.
22. A method for increasing sustained cell viability of a cell line, the method comprising modulating one or more genes or proteins identified according to the method of item 5.
23. A method for improving a cell line, the method comprising modulating one or more genes selected from Tables 20, 24, 25, and 26.
24. A method for modulating a rate of lactate production or consumption in a cell line, the method comprising modulating one or more genes selected from Tables 29 and 30.
25. A method for improving a cell line, the method comprising up-regulating or down-regulating at least two genes or proteins, wherein a first gene or protein affects a first cell culture phenotype and a second gene or protein affects a second, different cell culture phenotype, wherein the cell culture phenotypes are selected from the group consisting of a cell growth rate, a cellular productivity, a peak cell density, a sustained cell viability, a rate of ammonia production or consumption, or a rate of lactate production or consumption.
26. The method of item 25, further comprising up-regulating or down-regulating a third gene or protein affecting a third cell culture phenotype different from the first and second cell culture phenotypes.
27. A method of assessing a cell culture phenotype of a cell line, the method comprising:
   detecting, in a sample from the cell culture, an expression level of a protein identified according to item 1; and
   comparing the expression level to a reference level,
   wherein the comparison is indicative of the cell culture phenotype.
28. A method of assessing a cell culture phenotype of a cell line, the method comprising detecting, in a sample from the cell culture, one or more markers indicative of the cell culture phenotype, wherein the markers are selected from the group consisting of peptides selected from Figures 7 through 138, proteins selected from Tables 2 through 8, and gene expression products from genes selected from Tables 9 through 20 and 24 through 30.
29. An engineered cell line with an improved cell culture phenotype comprising a population of engineered cells, each of which comprising an engineered construct up-regulating or down-regulating one or more proteins identified according to item 1.
30. An engineered cell line with an improved cellular productivity comprising a population of engineered cells, each of which comprising an engineered construct up-regulating or down-regulating one or more genes or proteins selected from Tables 2, 3, 9, 10, 11, and 12.
31. The engineered cell line of item 30, wherein the engineered construct is an over-expression construct.
32. The engineered cell line of item 30, wherein the engineered construct is an interfering RNA construct.
33. An engineered cell line with an improved cell growth rate comprising a population of engineered cells, each of which comprising an engineered construct up-regulating or down-regulating one or more genes or proteins selected from Tables 4, 5, 6, 13, 14, 27 and 28.
34. The engineered cell line of item 33, wherein the engineered construct is an over-expression construct.
35. The engineered cell line of item 33, wherein the engineered construct is an interfering RNA construct.
36. An engineered cell line with an improved peak cell density comprising a population of engineered cells, each of which comprising an engineered construct up-regulating or down-regulating one or more genes or proteins selected from Tables 8, 15, 16, and 17.
37. The engineered cell line of item 36, wherein the engineered construct is an over-expression construct.
38. The engineered cell line of item 36, wherein the engineered construct is an interfering RNA construct.
39. An engineered cell line with an improved sustained cell viability comprising a population of engineered cells, each of which comprising an engineered construct up-regulating or down-regulating one or more genes or proteins selected from Tables 7, 18 and 19.
40. The engineered cell line of item 39, wherein the engineered construct is an over-expression construct.
41. The engineered cell line of item 39, wherein the engineered construct is an interfering RNA construct.
42. An engineered cell line with modified lactate production or consumption, the engineered cell line comprising a population of engineered cells, each of which comprising an engineered construct up-regulating or down-regulating one or more genes selected from Tables 29 and 30.
43. The engineered cell line of item 42, wherein the engineered construct is an over-expression construct.
44. The engineered cell line of item 42, wherein the engineered construct is an interfering RNA construct.
45. An improved cell line comprising a population of engineered cells, each of which comprising an engineered construct up-regulating or down-regulating one or more genes or proteins selected from Tables 20, 24, 25 and 26.
46. The engineered cell line of item 45, wherein the engineered construct is an over-expression construct.
47. The engineered cell line of item 45, wherein the engineered construct is an interfering RNA construct.
48. A method for expression of a protein of interest, the method comprising the steps of:
   introducing into the engineered cell line of item 29 a nucleic acid encoding the protein of interest; and
   harvesting the protein of interest.
49. An isolated or recombinant nucleic acid comprising a CHO sequence selected from Tables 9, 13, and 15.
50. An isolated or recombinant protein comprising a CHO sequence selected from Tables 2 and 4.

## Claims

1. An engineered cell line with an improved cell culture phenotype comprising a population of engineered cells, each of which comprising an engineered construct up-regulating or down-regulating one or more genes or proteins selected from the group consisting of SLC3A2, PE01, ERP29, CD36, NOL1, RAI14, PFDNS, ATP6VOA1, SUCLA2, SLC16A1, UAP, NSFL1, TRIB3, Eif4b, VCP, and ATF4.

2. The engineered cell line of claim 1, wherein the engineered construct up-regulates the one or more genes or proteins.

3. The engineered cell line of claim 2, wherein the engineered construct is an over-expression construct.

4. The engineered cell line of claim 1, wherein the engineered construct down-regulates the one or more genes or proteins.

5. The engineered cell line of claim 4, wherein the engineered construct is an interfering RNA construct.

6. A method for generating an engineered cell line of claim 1, the method comprising up-regulating or down-regulating one or more genes or proteins selected from the group consisting of SLC3A2, PEO1 ERP29, CD36, NOL1, RAI14, PFDN5, ATP6V0A1, SUCLA2, SLC16A1, UAP, NSFL1, TRIB3, Eif4b, VCP, and ATF4.

7. A method for expression of a protein of interest, the method comprising steps of:
introducing into an engineered cell line of any one of claim 1-5 a nucleic acid encoding the protein of interest; and
harvesting the protein of interest.
